# EUROPEAN PATENT APPLICATION

(11) **EP 4 613 096 A2**
(43) Date of publication of application: **10.09.2025**
(21) Application number: 25185594.6
(22) Date of filing: 26.01.2022
(51) Int. Cl.: A01N 37/00

(54) **ANTIPARASITIC COMPOUNDS**

(30) Priority: 27.01.2021 EP 21153638; 22.12.2021 US 202163292561 P
(62) Divisional of application: 22706735.2
(71) Applicant: Intervet International B.V., 5831 AN Boxmeer (NL); Corteva Agriscience LLC, Indianapolis, IN 46268 (US)
(72) Inventor: LUTZ, Jürgen, 55270 Schwabenheim (DE); HECKEROTH, Anja, Regina, 55270 Schwabenheim (DE); WILLIAMS, Heike, 55270 Schwabenheim (DE); ZOLLER, Hartmut, 55270 Schwabenheim (DE); HUNTER, James Edward, Indianapolis, 46268 (US); LAWLER, Lori Kay, Indianapolis, 46268 (US); TRULLINGER, Tony Kent, Indianapolis, 46268 (US); WALSH, Martin Joseph, Indianapolis, 46268 (US); SCHMITT, Harald, 55270 Schwabenheim (DE); SHEEHAN, John, Gerard, Rahway, 07065-0900 (US); KATZENSTEIN, Joshua M., Rahway, 07065 (US)
(74) Representative: Intervet International B.V.

(57) **Abstract**

The present invention relates to cyclopropylamide compounds that are useful in the treatment of parasitic infestations of animals. The compounds have the following formula (Formula (I))

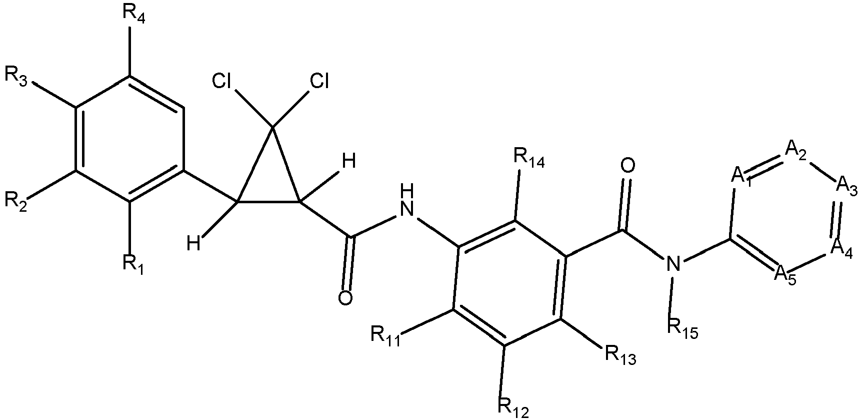

## Description

### FIELD OF THE INVENTION

This invention relates to the use of cyclopropyl amide compounds, compositions and methods for controlling parasitic infestations of animals.

### BACKGROUND OF THE INVENTION

A number of parasites are known to infest animals. These parasites can be great nuisances to both the animals and their owners.

Treatment of animals to prevent infestation by parasites, or to reduce or control the proliferation of these parasites, especially ectoparasites of animals such as ticks in animals is thus important.

The compounds currently available for parasite control and especially ectoparasite and tick control do not always demonstrate good efficacy, sufficiently quick onset of activity, or a long duration of action.

Ectoparasites tend to irritate the animals and can also cause clinical disease and adverse sub-clinical conditions, either by themselves or by carrying vector-transmitted pathogens.

Specifically, ticks parasitize on wild as well as domesticated animals and humans, and are known or suspected to be responsible for the transmission of pathogens including bacteria, viruses and protozoan parasites. Ticks also release toxins which cause inflammation or paralysis in the host. Occasionally, these toxins are fatal to the host.

On the other hand, such ectoparasites seriously impact productivity in the livestock animal industry by reducing weight gain, or milk production, causing inferior quality hide, wool, and meat, and in some cases resulting in mortality. Ectoparasites are also responsible, in part, for the spread of pathogens as vectors and cause discomfort in livestock and companion animals impacting animal welfare.

For example, a parasite which is prevalent among livestock animals, such as cattle, is the tick of the genus *Rhipicephalus,* especially those of the species *R. microplus* (tropical cattle tick), *R. decoloratus* and *R. annulatus.* Ticks such as *R. microplus* (formerly *Boophilus microplus)* are difficult to control because they lay eggs in the pasture where animals graze.

Such limitations include toxicity and safety of existing compounds both for the animal and the user/owner, limited efficacy (potency, onset of activity and duration), narrow spectrum of parasiticide action, bioavailability, pharmacokinetics and pharmacodynamics challenges and resistance issues. Further, certain aspects of the invention overcome at least some limitations in the use of current parasiticides, particularly in providing effective long term, safe control of parasites, that are known to be difficult to treat.

International patent applications WO2016/168056, WO2016/168058, WO 2016/168059 and WO2018/071327 disclose certain cyclopropyl amide compounds as having insecticidal and acaricidal activity and therefore, being useful in controlling agricultural plant pests. Although veterinary use is briefly mentioned, none of these citations exemplify or describe their use of the compounds as claimed herein that address at least some of the needs for its use of such compounds as veterinary medicament as described above. Furthermore, it was found that only a small selection of the compounds as described in these citation actually have an effect on veterinary parasites.

### SUMMARY OF THE INVENTION

The present invention is related in a first aspect to a compound of Formula (I) 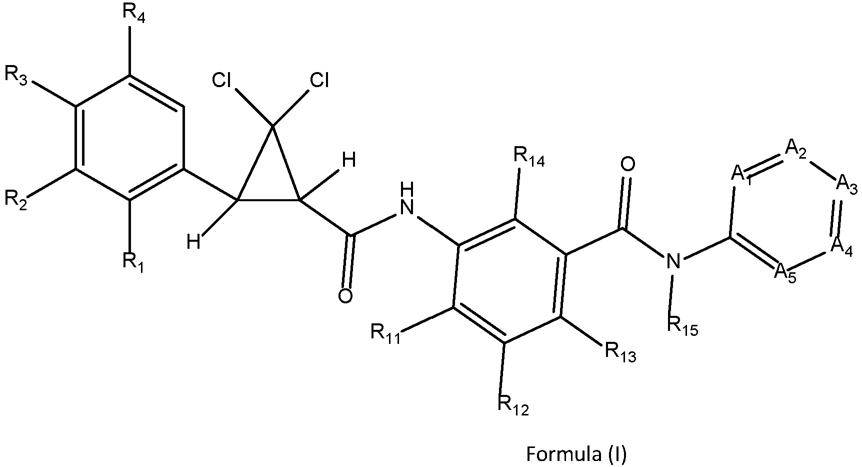
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl
H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.
Suitably the compounds of the invention and any embodiments thereof are for use in a method of treatment or control of a parasitic infestation of an animal,

Suitably the compound and use according to invention and/or any embodiment thereof
**R¹** is selected from the group consisting of H F, and Cl;
**R²** is selected from the group consisting of H, F, and Cl;
**R³** is selected from the group consisting of H, F, and Cl;
**R⁴** is selected from the group consisting of H, F, and Cl;
**R⁵** is selected from the group consisting of H F, Cl, Br, and I;
**R⁶** is selected from the group consisting of H, F, and Cl, Br, and CH₃;
**R⁷** is selected from the group consisting of H F, Cl, Br, and I;
**R⁸** is selected from the group consisting of H, F, Cl, and Br, and CH₃;
**R⁹** is selected from the group consisting of H F, Cl, Br, and I;
**R¹¹** is H;
**R¹²** is selected from the group consisting H, F, CL, CH₃, CF₃;
**R¹³** is selected from the group consisting of H, F, Cl, CF₃ and CH₃;
**R¹⁴** is H or F;
**R¹⁵** is selected from the group consisting of H and CH₃.

Suitably the compound and use according to invention and/or any embodiment thereof
**R¹** is H;
**R²** is selected from the group consisting of H, F, and Cl;
**R³** is selected from the group consisting of H, Cl, F, and Br;
**R⁴** is H, F, Cl;
**R⁵** is selected from the group consisting of H F, and Cl;
**R⁶** is selected from the group consisting of H, F, and Cl, and CH₃;
**R⁷** is selected from the group consisting of H F, and Cl;
**R⁸** is selected from the group consisting of H, F, Cl, and CH₃;
**R⁹** is selected from the group consisting of H F, and Cl;
**R¹¹** is H;
**R¹²** is selected from the group consisting H, F and Cl;
**R¹³** is selected from the group consisting of H, F, and Cl;
**R¹⁴** is H;
**R¹⁵** is selected from the group consisting of H and CH₃.

Surprisingly it has been found that the compounds described in this application can be used as medicaments, especially as veterinary antiparasitic medicaments to treat or control parasite infestation of animals.

The invention is understood to include compounds of the invention and/or embodiments thereof as described in this application for use as a medicament, especially a veterinary medicament, more preferably an antiparasitic veterinary medicament.

The invention is also understood to include the use of compounds of the invention and/or embodiments thereof as described in this application in the manufacture of a medicament for the treatment or control of parasite infestation of animals.

The invention is also understood to include the use of compounds of the invention and/or embodiments thereof as described in this application in the manufacture of a medicament for the protection of animals from parasite infestation.

The compounds of the invention and/or embodiments thereof are suitably used in a method to treat or control an ectoparasite infestation.

Suitably the compounds of the invention and/or embodiments thereof are used in a method, wherein the parasitic infestation is a tick infestation or flea infestation. The compounds are suitably used for a tick infestation.

The compounds of the invention and/or embodiments thereof are suitably used against parasites wherein the parasite is a one host tick or a multi-host tick. Suitably the compounds of the invention are used against *Rhipicephalus microplus.*

In a suitable use or method of the invention and/or any embodiment thereof, the animal is a warm-blooded animal such as a mammal. Suitably, the compounds are also active in a livestock animal, such as a ruminant, bovine animal, or cattle. It was also found that the compounds are active in fish, against a sea lice infestation. Also the compounds are found active in a companion animal, such as a canine animal or a feline animal, especially a dog or cat.

In a suitable use or method of the invention and/or any embodiment thereof, the animal is protected from a parasite infestation. Preferably an existing parasite infestation of the animal is treated or controlled.

In a suitable use or method of the invention and/or any embodiment thereof, the method comprises administering the compound to an animal weekly, bi-weekly, monthly, every 6 weeks, every 2 months or every 3 months.

The invention is also directed to a veterinary composition comprising an effective amount of a compound of Formula (I)-(XCII) as defined in any embodiment described herein and an inert carrier or formulation adjuvant. The composition is suitable for use in the treatment and control of a parasitic infestation of animals such as described herein.

Suitably the veterinary composition is a topical composition, an oral composition, or an injection composition.

### BRIEF DESCRIPTION OF THE FIGURES

Figure 1 shows Plasma concentration of oily solution (Composition A) and volatile solution (Composition B) of compound 453.
Figure 2 shows a comparison of concentrations of Compound 453 in the blood of cattle with no CPE (Composition C) and three different CPEs (Compositions D, E, and F) showing no significant improvement with the inclusion of any CPE.
Figure 3 shows the duration of efficacy >90% against *R. microplus* and the onset of efficacy>90% against *R.microplus* for Composition G, H and Composition of Example 1.
Figure 4: Structures of the compounds 1-480.

### DETAILED DESCRIPTION

This detailed description of preferred embodiments is intended only to acquaint others skilled in the art with Applicants' invention, its principles, and its practical application so that others skilled in the art may adapt and apply the invention in its numerous forms, as they may be best suited to the requirements of a particular use. This detailed description and its specific examples, while indicating preferred embodiments of this invention, are intended for purposes of illustration only.

This invention, therefore, is not limited to the preferred embodiments described in this specification and may be variously modified. In addition, for conciseness purposes and readability only some combinations of embodiments are explicitly described, however it should be understood that other combinations of embodiments are also contemplated.

Briefly, this invention relates to compounds that can generally be used as active ingredient in a medicament for animals. The compounds of the invention and/or embodiments thereof are suitably used in a method to treat or control an ectoparasite infestation and/or to protect animals from parasite infestations.

Surprisingly, it has been found that the compounds of the invention and/or embodiments thereof described herein exhibit superior broad-spectrum efficacy against harmful parasites more rapidly, and over a long duration compared to other similar compounds known in the art when administered to an animal in need thereof. Therefore, these compounds are useful in the methods or uses of the invention and/or embodiments thereof.

In the following text the compounds for use according to the invention and/or embodiments thereof are described as "compounds of the invention" or "compounds" or "compounds of the invention and/or embodiments thereof ( including compounds of Formula (I)" or "compounds of formula (I)" when they are disclosed as compounds per se or as compounds for use/ in the method according to the invention.

The unexpected broad parasiticidal properties of these compounds for use according to the invention and/or embodiments thereof are of considerable significance since there are few agricultural pesticides that can be effectively be used against ectoparasites of animals, let alone against a broad range of animal parasites and in various target species

Compounds of the invention and/or embodiments thereof including compounds of Formula (I)-(XCII) show desirable bioavailability and pharmacokinetics when using different administration routes to administer them as a medicament to animals.. Many of the compounds also provide desirable safety profiles toward the target host animal and/or their owners. In addition, it has been discovered that a single administration of such compounds generally provides potent activity against a broad range of ectoparasites, including difficult to treat parasite infestations, using relevant administration routes while also tending to provide fast onset of activity, and/or long duration of activity, and/or desirable safety profiles. Consequently, these compounds have been shown to be useful as medicaments, especially antiparasitic veterinary medicaments.

The compounds of the invention and/or embodiments thereof show desired beneficial properties, that makes their use in medicaments for animals suitable and it has been further shown that the resistance breaking properties of such compounds are very favorable.

The Examples show that compounds of the invention and/or embodiments thereof including compounds of Formula (I) have broad activity both *in vitro* and *in vivo* against a number of veterinary relevant parasites that affect animals, such as one -host and multi- host ticks, fleas, bed bugs and sea lice that belong to diverse classes of biological organisms that are veterinary relevant parasites. It has been further shown in the Examples that existing parasite infestation with such parasites in different animals (cattle, dogs, fish, guinea pigs) can be successfully treated, while being safe to the animal.

Furthermore, the examples show that the compounds of the invention and/or embodiments thereof act against a broad range of animal parasites, in particular ectoparasites when administered to various target animals using a variety of administration routes.

Consequently, by using the compounds of the invention and/or embodiments thereof as described in this application disadvantages of the prior art can be avoided, because a convenient, safe administration of the cyclopropyl amide compound would be sufficient to achieve the desired effect.

In a first aspect the invention is directed to a compound of Formula (I) 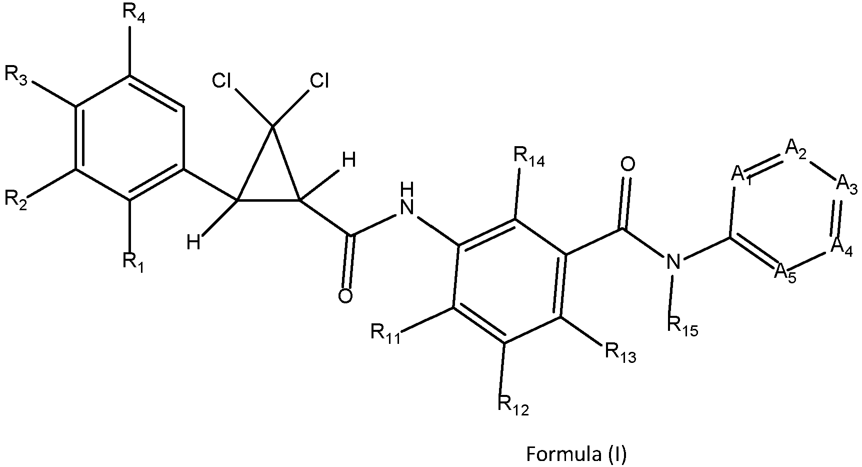
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N;
and wherein no more than 3 of A₁, A₂, A₃, A₄, A₅ are N or N=O;
wherein at least one of R⁵, R⁶, R⁷, R⁸, R⁹, is a substituent selected from the group consisting of F, Cl, Br, and I.

It was found that compounds wherein the ring formed by C-A₁-A₂-A₃-A₄-A₅ is substituted with at least one halogen, these compounds are active against animal ectoparasites. This is in contrast to compounds wherein the ring formed by C-A₁-A₂-A₃-A₄-A₅ is not substituted with at halogen.

The comparative example below shows that similar compounds have been tested in prior art documents WO2016/168059 and WO2018/071327 against a range of agrochemical relevant parasites. Many compounds from WO2016/168059 and WO2018/071327 that were active in plant protection assays, were not active against animal parasites in an assay as described in Example 1. It was found that compounds wherein the ring formed by A₁-A₅ that did not have a halogen substitution were active against agrochemical parasites but not against animal parasites. Compounds wherein the ring formed by A₁-A₅ is substituted by at least one halogen are active against animal parasites.

In the first aspect the compounds according to the invention are further defined as follows
**R¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl
H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.

In an embodiment of the invention and/or embodiments thereof, **R¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy.

In an embodiment of the invention and/or embodiments thereof, **R¹** is selected from the group consisting of H, F, Cl, Br, I, CN, NH₂, NO₂, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl, (C₃-C₆)halocycloalkyl, (C₂-C₆)haloalkenyl, (C₁-C₆)haloalkoxy of H, F, Cl, Br, I, CN, NH₂, NO₂, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl, (C₁-C₆)haloalkoxy.

In an embodiment of the invention and/or embodiments thereof, **R¹** is selected from the group consisting of H, F, Cl, Br, I, CN, NH₂, NO₂, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl of H, F, Cl, Br, I, CN, NH₂, NO₂, (C₁-C₆)alkyl, (C₁-C₆)alkoxy, (C₁-C₆)haloalkyl H, F, CI, Br, I, CN, NH₂, NO₂, (C₁-C₆)alkyl, (C₃-C₆)cycloalkyl, (C₂-C₆)alkenyl, (C₁-C₆)haloalkyl of H, F, Cl, Br, I, CN, NH₂, NO₂, (C₁-C₆)alkyl, (C₁-C₆)haloalkyl.

In an embodiment of the invention and/or embodiments thereof, **R¹** is H.

In an embodiment of the invention and/or embodiments thereof, **R²** is selected from the group consisting H, F, Cl, Br, I, CN, NH₂, NO₂, (C₁-C₆)alkyl, ((C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, S(C₁-C₆)alkyl, S(O)(C₁-C₆)alkyl, S(O)₂(C₁-C₆)alkyl, (C₁-C₆)alkyl-S(O)₂NH₂, (C₁-C₆)haloalkyl-S(O)₂NH₂ and S-(Halo)₅.

In an embodiment of the invention and/or embodiments thereof, **R²** is selected from the group consisting H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, and S-(Halo)₅.

In an embodiment of the invention and/or embodiments thereof, **R²** is selected from the group consisting H, F, Cl, Br, I, CN, (C₁-C₆)alkyl, (C₁-C₆)alkoxy, (C₁-C₆)haloalkyl, (C₁-C₆)haloalkoxy, and S-(Halo)₅.

In an embodiment of the invention and/or embodiments thereof, **R²** is selected from the group consisting of H, F, Cl, Br, CN, (C₁-C₆)alkyl, (C₁-C₆)haloalkyl and S-(Halo)₅.

In an embodiment of the invention and/or embodiments thereof, **R²** is selected from the group consisting of H, F, Cl, Br, CN, CH₃, CHF₂, CF₃, OCHF₂ and S-(Halo)₅.

In an embodiment of the invention and/or embodiments thereof, **R²** is selected from the group consisting of H, F, Cl.

In an embodiment of the invention and/or embodiments thereof, **R²** is selected from the group consisting of F, and Cl.

In an embodiment of the invention and/or embodiments thereof, **R²** is Cl.

In an embodiment of the invention and/or embodiments thereof, **R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆)haloalkoxy.

In an embodiment of the invention and/or embodiments thereof, **R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl.

In an embodiment of the invention and/or embodiments thereof, **R²** is selected from the group consisting of H, F, Cl, Br, (C₁-C₆)alkyl, and (C₁-C₆)haloalkyl.

In an embodiment of the invention and/or embodiments thereof, **R²** is selected from the group consisting of H, F, Cl, and Br.

In an embodiment of the invention and/or embodiments thereof, **R³** is selected from the group consisting of H, F, Cl, Br, I, CN, NH2, NO₂, (C₁-C₆)alkyl, ((C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, S(C₁-C₆)alkyl, S(O)(C₁-C₆)alkyl, S(O)₂(C₁-C₆)alkyl, (C₁-C₆)alkyl-S(O)₂NH₂, (C₁-C₆)haloalkyl-S(O)₂NH₂ and S-(Halo)₅.

In an embodiment of the invention and/or embodiments thereof, **R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy.

In an embodiment of the invention and/or embodiments thereof, R³ is selected from the group consisting of H, F, Cl, Br, I, CH₃, CF₃, CHF₂, OCF₃, OCH₃, CH=CHF, C≡C, CH=CH₂.

In an embodiment of the invention and/or embodiments thereof, **R³** is selected from the group consisting of H, F, Cl, Br, and I.

In an embodiment of the invention and/or embodiments thereof, R³ is selected from the group consisting of H, F, Cl, Br.

In an embodiment of the invention and/or embodiments thereof, R³ is selected from the group consisting of H, F, and Cl.

In an embodiment of the invention and/or embodiments thereof, **R³** is H or F.

In an embodiment of the invention and/or embodiments thereof, **R³** is H.

In an embodiment of the invention and/or embodiments thereof, **R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy.

In an embodiment of the invention and/or embodiments thereof, **R³** is selected from the group consisting of H, F, Cl, Br, I, (C₁-C₆)alkyl, (C₁-C₆)alkoxy, (C₁-C₆)haloalkyl, and (C₁-C₆) haloalkoxy.

In an embodiment of the invention and/or embodiments thereof, **R³** is selected from the group consisting of H, F, Cl, Br, I, (C₁-C₆)haloalkyl, and (C₁-C₆) haloalkoxy.

In an embodiment of the invention and/or embodiments thereof, **R³** is selected from the group consisting of H, F, Cl, Br, and I.

In an embodiment of the invention and/or embodiments thereof, **R⁴** is selected from the group consisting of H, F, Cl, Br, I, CN, NH2, NO₂, (C₁-C₆)alkyl, ((C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, S(C₁-C₆)alkyl, S(O)(C₁-C₆)alkyl, S(O)₂(C₁-C₆)alkyl, (C₁-C₆)alkyl-S(O)₂NH₂, (C₁-C₆)haloalkyl-S(O)₂NH₂ and S-(Halo)₅.

In an embodiment of the invention and/or embodiments thereof, **R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, and S-(Halo)₅.

In an embodiment of the invention and/or embodiments thereof, **R⁴** is selected from the group consisting of H, F, Cl, Br, (C₁-C₆)alkyl, (C₁-C₆)haloalkyl, (C₂-C₆)alkenyl, (C₂-C₆)alkynyl (C₁-C₆)alkoxy, (C₂-C₆)haloalkenyl, and S-(Halo)₅.

In an embodiment of the invention and/or embodiments thereof, R⁴ is selected from the group consisting of H, F, Cl, Br, CH₃, CF₃, OCH₃, CH=CHF, C=C, CH=CH₂ and SF₅.

In an embodiment of the invention and/or embodiments thereof, **R⁴** is selected from the group consisting of H, F, Cl, and Br.

In an embodiment of the invention and/or embodiments thereof, **R⁴** is selected from the group consisting of H, F, and Cl.

In an embodiment of the invention and/or embodiments thereof, **R⁴** is Cl.

In an embodiment of the invention and/or embodiments thereof, **R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy.

In an embodiment of the invention and/or embodiments thereof, **R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl.

In an embodiment of the invention and/or embodiments thereof, **R⁴** is selected from the group consisting of H, F, Cl, Br, (C₁-C₆)alkyl, and (C₁-C₆)haloalkyl.

In an embodiment of the invention and/or embodiments thereof, **R⁴** is selected from the group consisting of H, F, Cl, Br.

In an embodiment of the invention and/or embodiments thereof, **R⁵** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, thiazolyl, thienyl, and triazolyl.

In an embodiment of the invention and/or embodiments thereof, **R⁵** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)haloalkoxy, (C₁-C₆)haloalkyl, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)O(C₁-C₆)alkyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH-C(=O)O(C₁-C₆)alkyl.

In an embodiment of the invention and/or embodiments thereof, **R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁵** is selected from the group consisting of H, F, Cl, Br, I, (C₁-C₆)alkyl, (C₁-C₆)alkoxy, and (C₁-C₆)haloalkyl.

In an embodiment of the invention and/or embodiments thereof, **R⁵** is selected from the group consisting of H, F, Cl, Br, I, CH₃, OCH₃, and CF₃.

In an embodiment of the invention and/or embodiments thereof, **R⁵** is selected from the group consisting of H, F, Cl, Br, and I.

In an embodiment of the invention and/or embodiments thereof, **R⁵** is selected from the group consisting of H, F, and Cl.

In an embodiment of the invention and/or embodiments thereof, **R⁵** is H or F.

In an embodiment of the invention and/or embodiments thereof, **R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰.

In an embodiment of the invention and/or embodiments thereof, **R⁵** is selected from the group consisting of H, F, Cl, CN, NO₂, NH(C₁-C₆)alkyl, and (C₁-C₆)alkyl.

In an embodiment of the invention and/or embodiments thereof, **R⁵** is selected from the group consisting of H, F, Cl, CN and (C₁-C₆)alkyl.

In an embodiment of the invention and/or embodiments thereof, **R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, thiazolyl, thienyl, and triazolyl.

In an embodiment of the invention and/or embodiments thereof, **R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)haloalkoxy, (C₁-C₆)haloalkyl, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)O(C₁-C₆)alkyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH-C(=O)O(C₁-C₆)alkyl.

In an embodiment of the invention and/or embodiments thereof, **R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁶** is selected from the group consisting of H, F, Cl, Br, I, (C₁-C₆)alkyl, (C₁-C₆)alkoxy, (C₁-C₆)haloalkyl, (C₂-C₆)alkenyl, and (C₂-C₆)haloalkenyl.

In an embodiment of the invention and/or embodiments thereof, **R⁶** is selected from the group consisting of H, F, Cl, Br, I, CH₃, OCH₃, CH=CHF, CH=CH, and CF₃.

In an embodiment of the invention and/or embodiments thereof, **R⁶** is selected from the group consisting of H, F, Cl, Br, I, CH₃, OCH₃, CH=CHF, CH=CH, and CF₃.

In an embodiment of the invention and/or embodiments thereof, **R⁶** is selected from the group consisting of H, F, Cl, Br, and CH₃.

In an embodiment of the invention and/or embodiments thereof, **R⁶** is selected from the group consisting of H, F, Cl, and CH₃.

In an embodiment of the invention and/or embodiments thereof, **R⁶** is H or F.

In an embodiment of the invention and/or embodiments thereof, **R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰.

In an embodiment of the invention and/or embodiments thereof, **R⁶** is selected from the group consisting of H, F, NH(C₁-C₆)alkyl, NHC(=O)R¹⁰, and N(C₁-C₆)alkyl-C(=O)IR¹⁰.

In an embodiment of the invention and/or embodiments thereof, **R⁶** is selected from the group consisting of H, F, and NH(C₁-C₆)alkyl.

In an embodiment of the invention and/or embodiments thereof, **R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, thiazolyl, thienyl, and triazolyl.

In an embodiment of the invention and/or embodiments thereof, **R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)haloalkoxy, (C₁-C₆)haloalkyl, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)O(C₁-C₆)alkyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH-C(=O)O(C₁-C₆)alkyl.

In an embodiment of the invention and/or embodiments thereof, **R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁷** is selected from the group consisting of H, F, Cl, Br, I, (C₁-C₆)alkyl, (C₁-C₆)alkoxy, (C₁-C₆)haloalkyl, (C₂-C₆)alkenyl, and (C₂-C₆)haloalkenyl.

In an embodiment of the invention and/or embodiments thereof, **R⁷** is selected from the group consisting of H, F, Cl, Br, I, CH₃, OCH₃, CH=CHF, CH=CH, and CF₃.

In an embodiment of the invention and/or embodiments thereof, **R⁷** is selected from the group consisting of H, F, Cl, Br, I, CH₃, OCH₃, CH=CHF, CH=CH, and CF₃.

In an embodiment of the invention and/or embodiments thereof, **R⁷** is selected from the group consisting of H, F, Cl, Br, and I.

In an embodiment of the invention and/or embodiments thereof, **R⁷** is selected from the group consisting of H, F, and Cl.

In an embodiment of the invention and/or embodiments thereof, **R⁷** is H or F.

In an embodiment of the invention and/or embodiments thereof, **R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl.

In an embodiment of the invention and/or embodiments thereof, **R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, thiazolyl, thienyl, and triazolyl.

In an embodiment of the invention and/or embodiments thereof, **R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)haloalkoxy, (C₁-C₆)haloalkyl, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)O(C₁-C₆)alkyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH-C(=O)O(C₁-C₆)alkyl.

In an embodiment of the invention and/or embodiments thereof, **R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁸** is selected from the group consisting of H, F, Cl, Br, I, (C₁-C₆)alkyl, (C₁-C₆)alkoxy, (C₁-C₆)haloalkyl, (C₂-C₆)alkenyl, and (C₂-C₆)haloalkenyl.

In an embodiment of the invention and/or embodiments thereof, **R⁸** is selected from the group consisting of H, F, Cl, Br, I, CH₃, OCH₃, CH=CHF, CH=CH, and CF₃.

In an embodiment of the invention and/or embodiments thereof, **R⁸** is selected from the group consisting of H, F, Cl, Br, I, CH₃, OCH₃, CH=CHF, CH=CH, and CF₃.

In an embodiment of the invention and/or embodiments thereof, **R⁸** is selected from the group consisting of H, F, Cl, Br, and CH₃.

In an embodiment of the invention and/or embodiments thereof, R⁸ is selected from the group consisting of H, F, Cl, and CH₃.

In an embodiment of the invention and/or embodiments thereof, **R⁸** is H or F.

In an embodiment of the invention and/or embodiments thereof, **R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁸** is selected from the group consisting of H, F, Cl, NH(C₁-C₆)alkyl, and (C₁-C₆)alkyl.

In an embodiment of the invention and/or embodiments thereof, **R⁸** is selected from the group consisting of H, F, and NH(C₁-C₆)alkyl.

In an embodiment of the invention and/or embodiments thereof, **R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, thiazolyl, thienyl, and triazolyl.

In an embodiment of the invention and/or embodiments thereof, **R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)haloalkoxy, (C₁-C₆)haloalkyl, (C₂-C₆)alkenyl, (C₂-C₆)alkynyl, (C₂-C₆)haloalkenyl, (C₃-C₆)cycloalkyl, (C₃-C₆)halocycloalkyl, C(=O)(C₁-C₆)alkyl, C(=O)NH(C₁-C₆)alkyl, C(=O)O(C₁-C₆)alkyl, N((C₁-C₆)alkyl)₂, NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH-C(=O)O(C₁-C₆)alkyl.

Compound and use according to any of the preceding claims, wherein **R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl.

In an embodiment of the invention and/or embodiments thereof, **R⁹** is selected from the group consisting of H, F, Cl, Br, I, (C₁-C₆)alkyl, (C₁-C₆)alkoxy, (C₁-C₆)haloalkyl, (C₂-C₆)alkenyl, and (C₂-C₆)haloalkenyl.

In an embodiment of the invention and/or embodiments thereof, **R⁹** is selected from the group consisting of H, F, Cl, Br, I, CH₃, OCH₃, CH=CHF, CH=CH, and CF₃.

In an embodiment of the invention and/or embodiments thereof, **R⁹** is selected from the group consisting of H, F, Cl, Br, I, CH₃, OCH₃, CH=CHF, CH=CH, and CF₃.

In an embodiment of the invention and/or embodiments thereof, **R⁹** is selected from the group consisting of H, F, Cl, Br, and I.

In an embodiment of the invention and/or embodiments thereof, **R⁹** is selected from the group consisting of H, F, and Cl.

In an embodiment of the invention and/or embodiments thereof, **R⁹** is H or F.

In an embodiment of the invention and/or embodiments thereof, **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-.

In an embodiment of the invention and/or embodiments thereof, **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-.

In an embodiment of the invention and/or embodiments thereof, the ring formed by **R**⁶ and **R⁷** is optionally substituted with one or more substituents of selected from the group consisting of (C₁-C₃)alkyl, F, Cl, Br, I, CN.

In an embodiment of the invention and/or embodiments thereof, the ring formed by **R**⁶ and **R⁷** is optionally substituted with one or more substituents of selected from the group consisting of (C₁-C₃)alkyl, F, and Cl.

In an embodiment of the invention and/or embodiments thereof, the ring formed by **R**⁶ and **R⁷** is optionally substituted with one or more substituents of selected from the group consisting of CH₃, F, and Cl.

In an embodiment of the invention and/or embodiments thereof, the ring formed by **R**⁶ and **R⁷** is not substituted.

In an embodiment of the invention and/or embodiments thereof, **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-.

In an embodiment of the invention and/or embodiments thereof, **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-.

In an embodiment of the invention and/or embodiments thereof, the ring formed by **R⁷** and **R⁸** is optionally substituted with one or more substituents of selected from the group consisting of (C₁-C₃)alkyl, F, Cl, Br, I, and CN.

In an embodiment of the invention and/or embodiments thereof, the ring formed by **R⁷** and **R⁸** is optionally substituted with one or more substituents of selected from the group consisting of (C₁-C₃)alkyl, F, and Cl.

In an embodiment of the invention and/or embodiments thereof, the ring formed by **R⁷** and **R⁸** is optionally substituted with one or more substituents of selected from the group consisting of CH₃, F, and Cl.

In an embodiment of the invention and/or embodiments thereof, the ring formed by **R⁷** and **R⁸** is not substituted.

In an embodiment of the invention and/or embodiments thereof, **R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl.

In an embodiment of the invention and/or embodiments thereof, **R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl.

In an embodiment of the invention and/or embodiments thereof, **R¹¹** is selected from the group consisting of H, F, Cl, Br, I, CN, NH₂, NO₂, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, and (C₁-C₆)alkyl-S(O)₂NH₂.

In an embodiment of the invention and/or embodiments thereof, **R¹¹** is selected from the group consisting of H, F, Cl, Br, I, CN, NH₂, NO₂, and (C₁-C₆)alkyl.

In an embodiment of the invention and/or embodiments thereof, **R¹¹** is selected from the group consisting of H, F, Cl, Br, I, and CH₃.

In an embodiment of the invention and/or embodiments thereof, **R¹¹** is selected from the group consisting of H, F, Cl, and CH₃.

In an embodiment of the invention and/or embodiments thereof, **R¹¹** is H.

In an embodiment of the invention and/or embodiments thereof, **R¹²** is selected from the group consisting of H, F, Cl, Br, I, CN, NH₂, NO₂, (C₁-C₆)alkyl, (C₃-C₆)cycloalkyl, (C₂-C₆)alkenyl (C₂-C₆)alkynyl, (C₁-C₆)alkoxy, (C₁-C₆)haloalkyl, (C₂-C₆)haloalkenyl, (C₁-C₆)haloalkoxy, and (C₁-C₆)alkyl-S(O)₂NH₂.

In an embodiment of the invention and/or embodiments thereof, **R¹²** is selected from the group consisting of H, F, Cl, Br, I, CN, NH₂, NO₂, (C₁-C₆)alkyl, (C₂-C₆)alkenyl; (C₁-C₆)alkoxy, (C₁-C₆)haloalkyl, (C₂-C₆)haloalkenyl, and (C₁-C₆)haloalkoxy.

In an embodiment of the invention and/or embodiments thereof, **R¹²** is selected from the group consisting of H, F, Cl, Br, I, (C₁-C₆)alkyl, (C₁-C₆)haloalkyl.

In an embodiment of the invention and/or embodiments thereof, **R¹²** is selected from the group consisting of H, F, Cl, CH₃ and CF₃.

In an embodiment of the invention and/or embodiments thereof, **R¹²** is H or F.

In an embodiment of the invention and/or embodiments thereof, **R¹²** is H.

In an embodiment of the invention and/or embodiments thereof, **R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, (C₁-C₆)alkyl-S(O)₂NH₂ and triazolyl.

In an embodiment of the invention and/or embodiments thereof, **R¹³** is selected from the group consisting of H, F, Cl, Br, I, CN, NH₂, NO₂, (C₁-C₆)alkyl, (C₂-C₆)alkenyl, (C₁-C₆)alkoxy, (C₁-C₆)haloalkyl, (C₂-C₆)haloalkenyl, and (C₁-C₆)haloalkoxy.

In an embodiment of the invention and/or embodiments thereof, **R¹³** is selected from the group consisting of H, F, Cl, Br, I, (C₁-C₆)alkyl, and (C₁-C₆)haloalkyl.

In an embodiment of the invention and/or embodiments thereof, **R¹³** is selected from the group consisting of H, F, Cl, Br, I, (C₁-C₆)alkyl, (C₁-C₆)haloalkyl.

In an embodiment of the invention and/or embodiments thereof, R¹³ is selected from the group consisting of H, F, Cl, CH₃ and CF₃.

In an embodiment of the invention and/or embodiments thereof, **R¹³** is Cl or F.

In an embodiment of the invention and/or embodiments thereof, **R¹³** is Cl.

In an embodiment of the invention and/or embodiments thereof, **R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy, and (C₁-C₆)alkyl-S(O)₂NH₂.

In an embodiment of the invention and/or embodiments thereof, **R¹⁴** is selected from the group consisting of H, F, Cl, Br, I, CN, NH₂, NO₂, (C₁-C₆)alkyl, (C₂-C₆)alkenyl, (C₁-C₆)alkoxy, (C₁-C₆)haloalkyl, (C₂-C₆)haloalkenyl, and (C₁-C₆)haloalkoxy.

In an embodiment of the invention and/or embodiments thereof, **R¹⁴** is selected from the group consisting of H, F, Cl, Br, I, (C₁-C₆)alkyl, and (C₁-C₆)haloalkyl.

In an embodiment of the invention and/or embodiments thereof, **R¹⁴** is selected from the group consisting of H, F, Cl, CH₃ and CF₃.

In an embodiment of the invention and/or embodiments thereof, **R¹⁴** is selected from the group consisting of H and F.

In an embodiment of the invention and/or embodiments thereof, R¹⁴ is H.

In an embodiment of the invention and/or embodiments thereof, **R¹⁵** is selected from the group consisting of H, (C₁-C₆)alkyl, (C₂-C₆)alkenyl, (C₂-C₆)alkynyl, and (C₁-C₆)haloalkyl.

In an embodiment of the invention and/or embodiments thereof, **R¹⁵** is selected from the group consisting of H, (C₁-C₆)alkyl, and (C₁-C₆)haloalkyl.

In an embodiment of the invention and/or embodiments thereof, **R¹⁵** is selected from the group consisting of H, CH₃, and CF₃.

In an embodiment of the invention and/or embodiments thereof, **R¹⁵** is selected from the group consisting of H, and CH₃.

In an embodiment of the invention and/or embodiments thereof, **R¹⁵** is H.

In an embodiment of the invention and/or embodiments thereof,
**R¹** is selected from the group consisting of H F, and Cl;
**R²** is selected from the group consisting of H, F, and Cl;
**R³** is selected from the group consisting of H, F, and Cl;
**R⁴** is selected from the group consisting of H, F, and Cl;
**R⁵** is selected from the group consisting of H F, Cl, Br, and I;
**R⁶** is selected from the group consisting of H, F, and Cl, Br, and CH₃;
**R⁷** is selected from the group consisting of H F, Cl, Br, and I;
**R⁸** is selected from the group consisting of H, F, Cl, and Br, and CH₃;
**R⁹** is selected from the group consisting of H F, Cl, Br, and I;
**R¹¹** is H;
**R¹²** is selected from the group consisting H, F, CL, CH₃, CF₃;
**R¹³** is selected from the group consisting of H, F, Cl, CF₃ and CH₃;
**R¹⁴** is H or F; and
**R¹⁵** is selected from the group consisting of H and CH₃.

In an embodiment of the invention and/or embodiments thereof,
**R¹** is H;
**R²** is selected from the group consisting of H, F, and Cl;
**R³** is selected from the group consisting of H, Cl, F, and Br;
**R⁴** is H, F, Cl;
**R⁵** is selected from the group consisting of H F, and Cl;
**R⁶** is selected from the group consisting of H, F, and Cl, and CH₃;
**R⁷** is selected from the group consisting of H F, and Cl;
**R⁸** is selected from the group consisting of H, F, Cl, and CH₃;
**R⁹** is selected from the group consisting of H F, and Cl;
**R¹¹** is H;
**R¹²** is selected from the group consisting H, F and Cl;
**R¹³** is selected from the group consisting of H, F, and Cl;
**R¹⁴** is H;
**R¹⁵** is selected from the group consisting of H and CH₃.

In an embodiment of the invention and/or embodiments thereof,
**R¹** is H;
**R²** is selected from the group consisting of H, F, and Cl;
**R³** is selected from the group consisting of H, Cl, F, and Br;
**R⁴** is H, F, Cl;
**R⁵** is selected from the group consisting of H F, and Cl;
**R⁶** is selected from the group consisting of H, F, and CH₃;
**R⁷** is selected from the group consisting of H F, and Cl;
**R⁸** is selected from the group consisting of H, F, and CH₃;
**R⁹** is selected from the group consisting of H F, and Cl;
**R¹¹** is H;
**R¹²** is selected from the group consisting H, and F;
**R¹³** is selected from the group consisting of F and Cl;
**R¹⁴** is H;
**R¹⁵** is selected from the group consisting of H and CH₃.

In an embodiment of the invention and/or embodiments thereof, at least one of R⁵, R⁶, R⁷, R⁸, R⁹, is a substituent selected from the group consisting of F, Cl, and Br.

In an embodiment of the invention and/or embodiments thereof, at least one of R⁵, R⁶, R⁷, R⁸, R⁹, is a substituent selected from the group consisting of F, and Cl.

In an embodiment of the invention and/or embodiments thereof, at least one of R⁵, R⁶, R⁷, R⁸, R⁹, is F.

In an embodiment of the invention and/or embodiments thereof, at least two of R⁵, R⁶, R⁷, R⁸, R⁹, is a substituent selected from the group consisting of F, Cl, and Br.

In an embodiment of the invention and/or embodiments thereof, at least two of R⁵, R⁶, R⁷, R⁸, R⁹, is a substituent selected from the group consisting of F, and Cl.

In an embodiment of the invention and/or embodiments thereof, at least two of R⁵, R⁶, R⁷, R⁸, R⁹, is F.

In an embodiment of the invention and/or embodiments thereof, at least one of R⁵, R⁶, R⁷ is a substituent selected from the group consisting of F, Cl, and Br.

In an embodiment of the invention and/or embodiments thereof, at least one of R⁵, R⁶, R⁷ is a substituent selected from the group consisting of F, and Cl.

In an embodiment of the invention and/or embodiments thereof, at least one of R⁵, R⁶, R⁷ is F.

In an embodiment of the invention and/or embodiments thereof, at least one of R⁵, R⁷ is a substituent selected from the group consisting of F, Cl, and Br.

In an embodiment of the invention and/or embodiments thereof, at least one of R⁵, R⁷ is a substituent selected from the group consisting of F, and Cl.

In an embodiment of the invention and/or embodiments thereof, at least one of R⁵, R⁷ is F.

In an embodiment of the invention and/or embodiments thereof, R⁵ and R⁷ are each a substituent selected from the group consisting of F, Cl, and Br.

In an embodiment of the invention and/or embodiments thereof, R⁵ and R⁷ are each a substituent selected from the group consisting of F, and Cl.

In an embodiment of the invention and/or embodiments thereof, R⁵ and R⁷ are each F.

In an embodiment of the invention and/or embodiments thereof, 0, 1 or 2 of A₁, A₂, A₃, A₄, A₅ is N

In an embodiment of the invention and/or embodiments thereof, the compound is a prodrug and at least one of A₁, A₂, A₃, A₄, A₅ is N=O.

In an embodiment of the invention and/or embodiments thereof, 0, 1 or 2 of A₁, A₂, A₃, A₄, A₅ is N=O

In an embodiment of the invention and/or embodiments thereof,
A₁ is CR₅ or N;
A₂ is CR₆ or N;
A₃ is CR₇ or N;
A₄ is CR₈ or N;
A₅ is CR₉ or N;
wherein no more than 3 of A₁, A₂, A₃, A₄, A₅ are N.

In an embodiment of the invention and/or embodiments thereof,
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N.

In an embodiment of the invention and/or embodiments thereof,
A₁ is CR₅ or N;
A₂ is CR₆ or N
A₃ is CR₇;
A₄ is CR₈;
A₅ is CR₉;
wherein 0 or 1 of A₁, or A₂ are N.

In an embodiment of the invention and/or embodiments thereof, the invention is directed to a compound of formula (II) 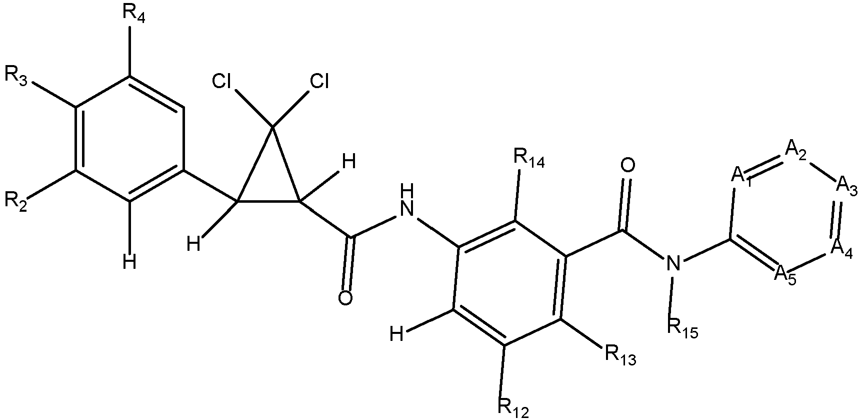 and N-oxides, veterinary acceptable acid addition salts, salt derivatives, solvates, ester derivatives, crystal polymorphs, isotopes, stereoisomers, and tautomers thereof, wherein at least one of R⁵, R⁶, R⁷, R⁸, R⁹, is a substituent selected from the group consisting of F, Cl, Br, and I, wherein R², R³, R⁴, R⁵, R⁶, R⁷, R⁸, R⁹, R¹², R¹³, R¹⁴, R¹⁵, A₁, A₂, A₃, A₄, and A₅ are defined as elsewhere in this description.

In an embodiment of the invention and/or embodiments thereof, the invention is directed to a compound of formula (II) 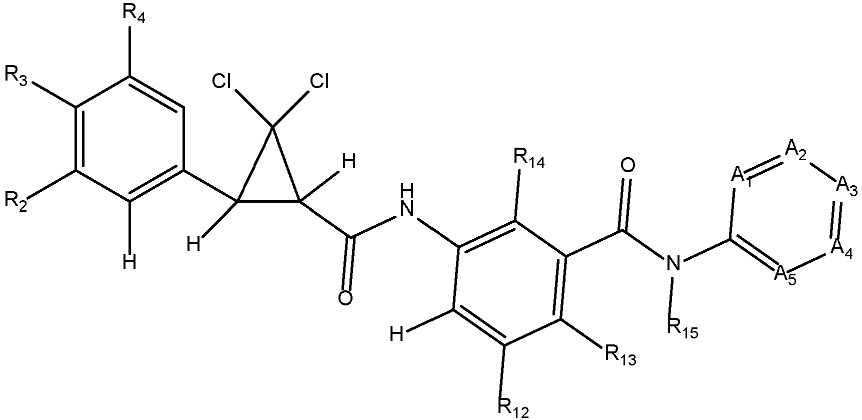
and N-oxides, veterinary acceptable acid addition salts, salt derivatives, solvates, ester derivatives, crystal polymorphs, isotopes, stereoisomers, and tautomers thereof, wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (II) 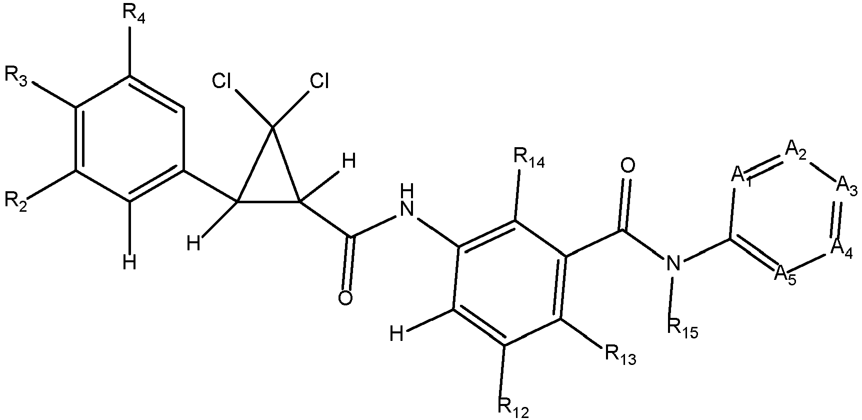
and N-oxides, veterinary acceptable acid addition salts, salt derivatives, solvates, ester derivatives, crystal polymorphs, isotopes, stereoisomers, and tautomers thereof, wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N,
A₂ is CR₆, N=O, or N
A₃ is CR₇, N=O, or N
A₄ is CR₈, N=O or N
A₅ is CR₉, N=O or N
wherein no more than 3 of A₁, A₂, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (II) 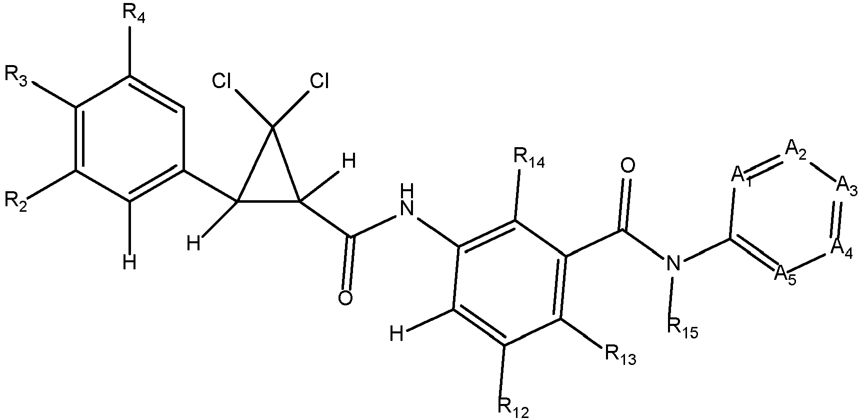
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₃ is CR₇ or N;
A₄ is CR₈ or N;
A₅ is CR₉ or N;
wherein no more than 3 of A₁, A₂, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (II) 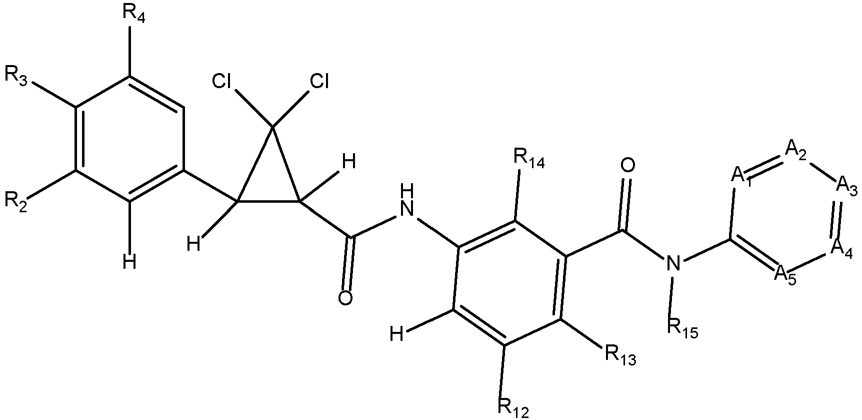
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N;
   **R⁵** is selected from the group consisting of H, F, Cl, CN, NO₂, NH(C₁-C₆)alkyl, and (C₁-C₆)alkyl ;
   **R⁶** is selected from the group consisting of H, F, NH(C₁-C₆)alkyl, NHC(=O)R¹⁰, and N(C₁-C₆)alkyl-C(=O)IR¹⁰ ;
   **R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
   **R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (II) 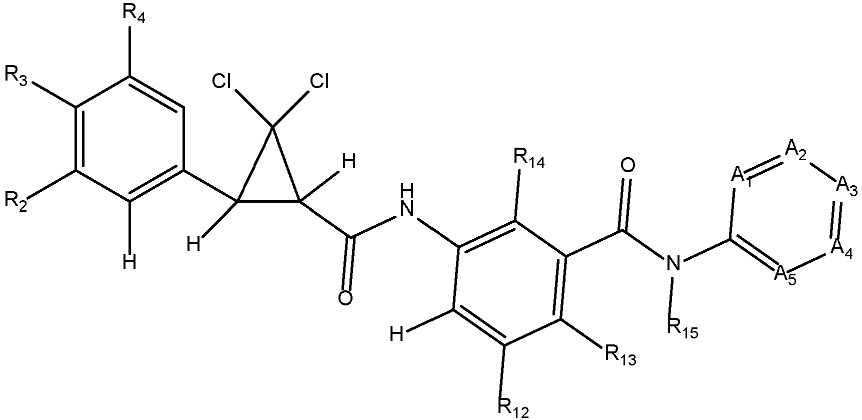
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A2 is CR₆ or N;
A₃ is CR₇;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
   **R⁷** is selected from the group consisting of H, and F ;
   **R⁸** is selected from the group consisting of H, and F ;
   **R⁹** is selected from the group consisting of H and F ;
**R¹²** is H ;
**R¹³** is Cl;
**R¹⁴** is H;
**R¹⁵** is H;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 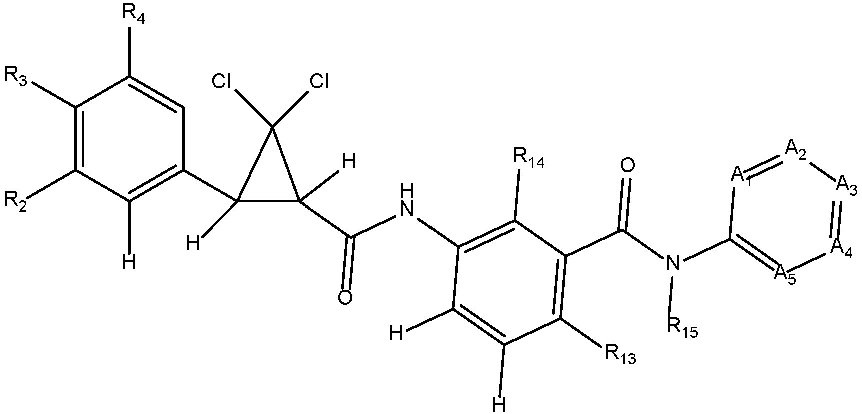
and N-oxides, veterinary acceptable acid addition salts, salt derivatives, solvates, ester derivatives, crystal polymorphs, isotopes, stereoisomers, and tautomers thereof,
wherein
R², R³, R⁴, R⁵, R⁶, R⁷, R⁸, R⁹, R¹³, R¹⁴, R¹⁵, A₁, A₂, A₃, A₄, and A₅ are defined as elsewhere in this description,
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 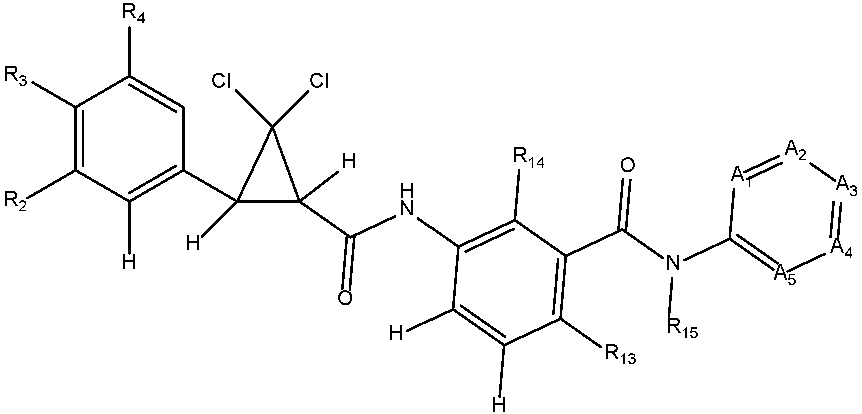
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl
H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 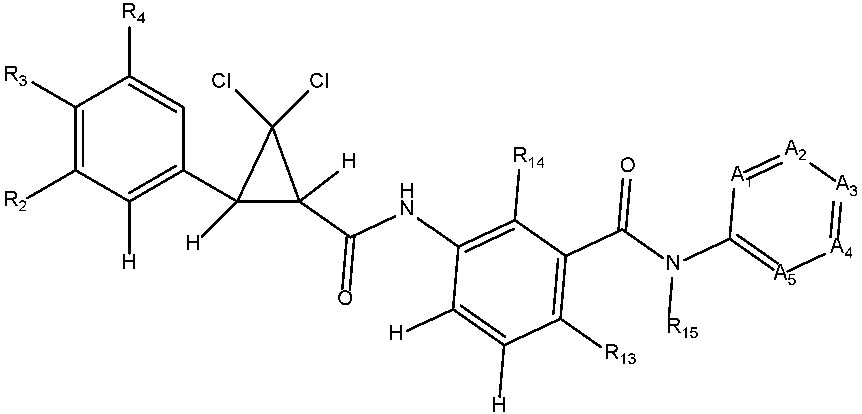
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N,
A₂ is CR₆, N=O, or N
A₃ is CR₇, N=O, or N
A₄ is CR₈, N=O or N
A₅ is CR₉, N=O or N
wherein no more than 3 of A₁, A₂, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃;;
**R¹⁵** is selected from the group consisting of H, CH₃, and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 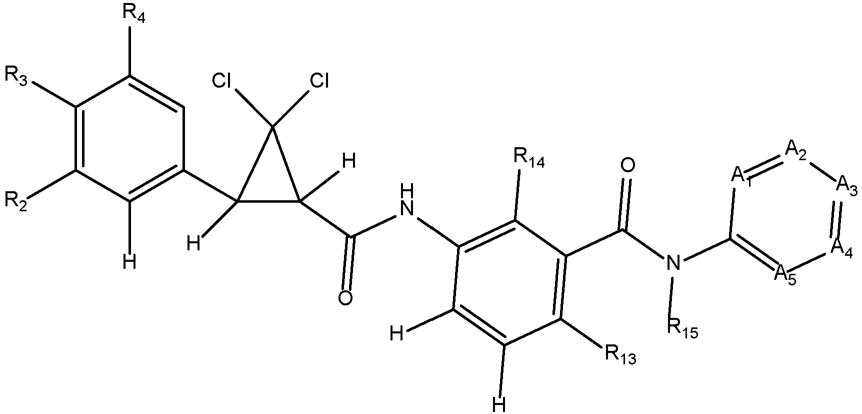
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₃ is CR₇ or N;
A₄ is CR₈ or N;
A₅ is CR₉ or N;
wherein no more than 3 of A₁, A₂, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 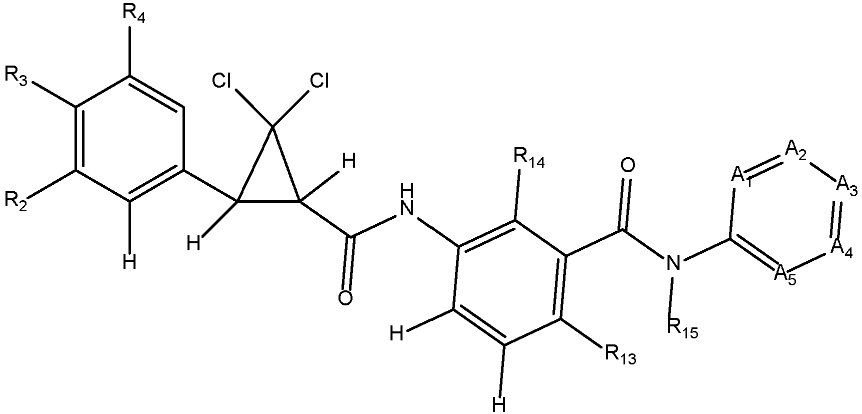
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N;
   **R⁵** is selected from the group consisting of H, F, Cl, CN, NO₂, NH(C₁-C₆)alkyl, and (C₁-C₆)alkyl ;
   **R⁶** is selected from the group consisting of H, F, NH(C₁-C₆)alkyl, NHC(=O)R¹⁰, and N(C₁-C₆)alkyl-C(=O)I_{R}¹⁰ ;
   **R⁷** is selected from the group consisting of H, F, and Cl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
   **R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (III) 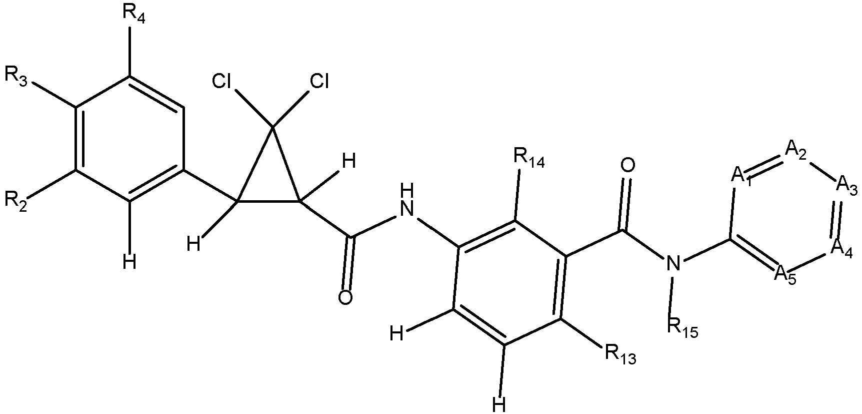
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A2 is CR₆ or N;
A₃ is CR₇;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
**R¹⁴** is H;
**R¹⁵** is H;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (IV) 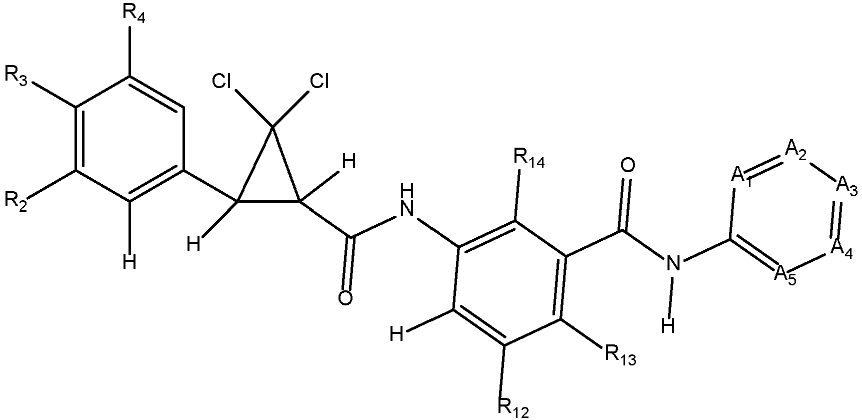
and N-oxides, veterinary acceptable acid addition salts, salt derivatives, solvates, ester derivatives, crystal polymorphs, isotopes, stereoisomers, and tautomers thereof,
wherein
R², R³, R⁴, R⁵, R⁶, R⁷, R⁸, R⁹, R¹², R¹³, R¹⁴, A₁, A₂, A₃, A₄, and A₅ are defined as elsewhere in this description and wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (IV) 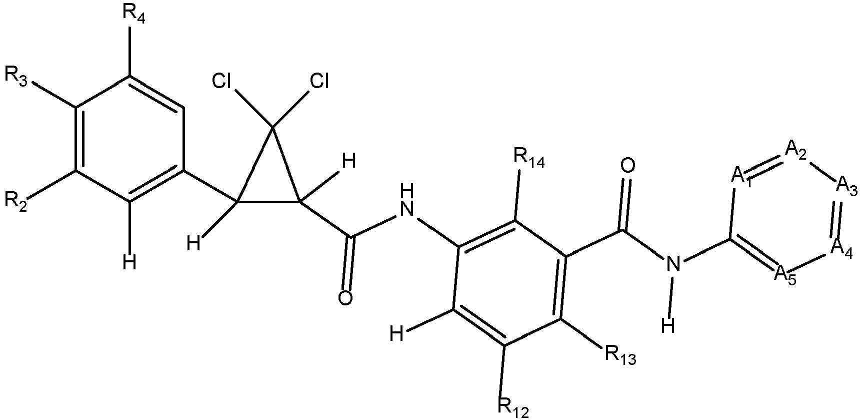
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (IV) 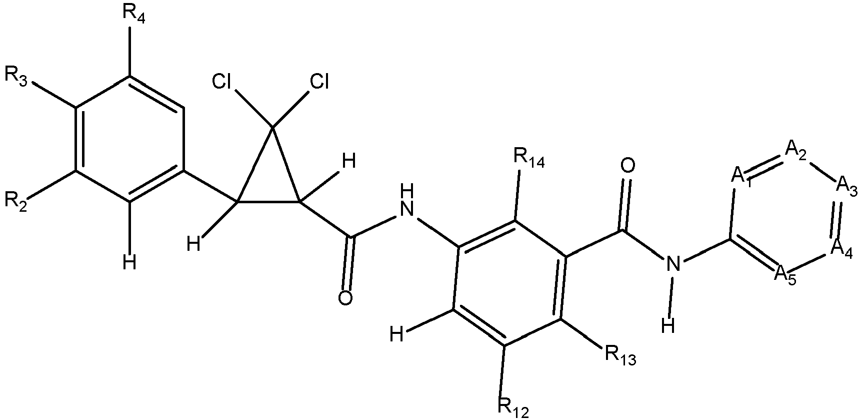
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N,
A₂ is CR₆, N=O, or N
A₃ is CR₇, N=O, or N
A₄ is CR₈, N=O or N
A₅ is CR₉, N=O or N
wherein no more than 3 of A₁, A₂, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (IV) 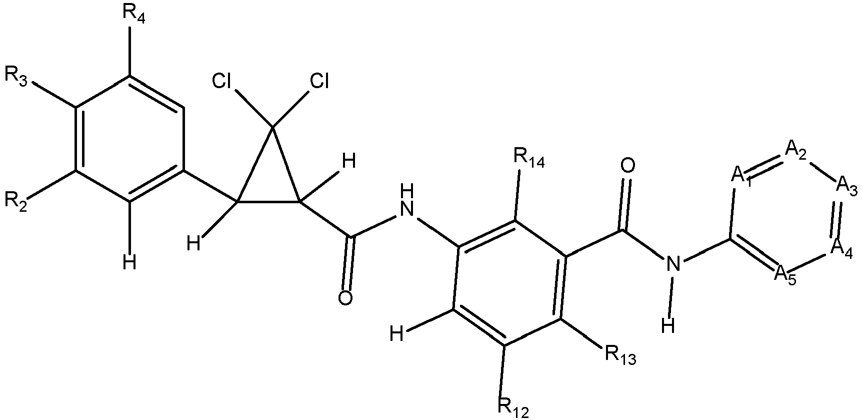
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₃ is CR7 or N;
A₄ is CR₈ or N;
A₅ is CR₉ or N;
wherein no more than 3 of A₁, A₂, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
wherein at least one of R⁵, R⁶, R⁷, R⁸, R⁹, is a substituent selected from the group consisting of F, CI, Br, and I.

In an embodiment of the invention and/or embodiments thereof, the invention is directed to a compound of formula (IV) 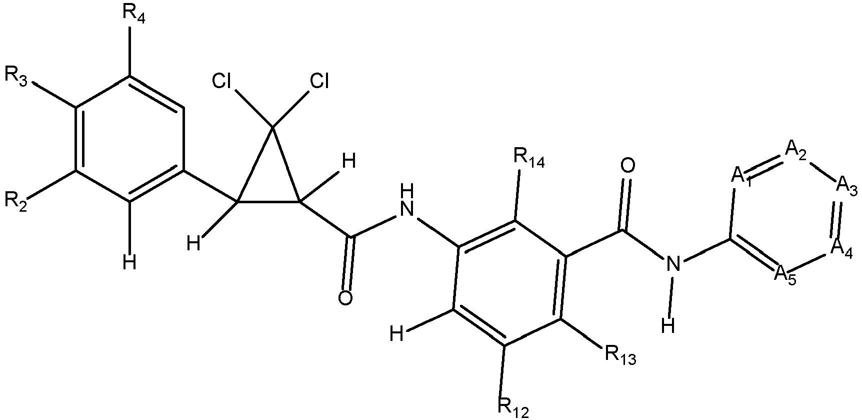
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N;
   **R⁵** is selected from the group consisting of H, F, Cl, CN, NO₂, NH(C₁-C₆)alkyl, and (C₁-C₆)alkyl ;
   **R⁶** is selected from the group consisting of H, F, NH(C₁-C₆)alkyl, NHC(=O)R¹⁰, and N(C₁-C₆)alkyl-C(=O)IR¹⁰ ;
   **R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
   **R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (IV) 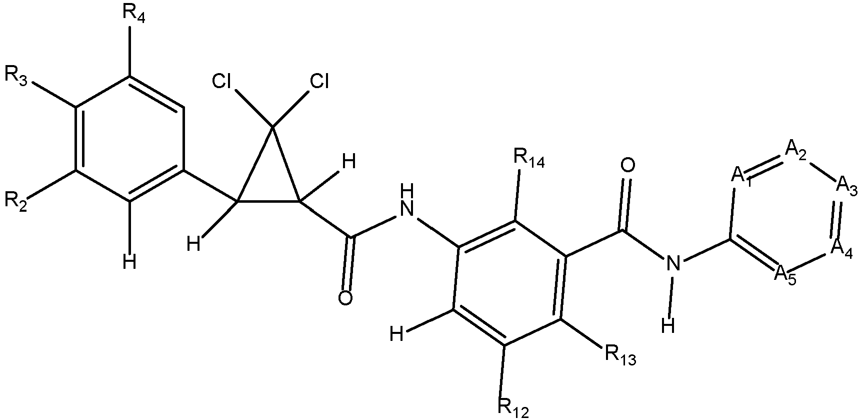
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₂ is CR₅ or N;
A₃ is CR₇;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (V) 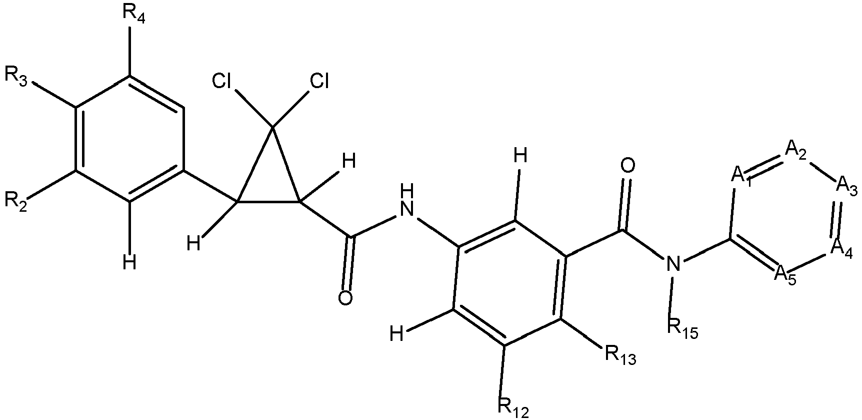
and N-oxides, veterinary acceptable acid addition salts, salt derivatives, solvates, ester derivatives, crystal polymorphs, isotopes, stereoisomers, and tautomers thereof,
wherein
R², R³, R⁴, R⁵, R⁶, R⁷, R⁸, R⁹, R¹², R¹³, R¹⁵, A₁, A₂, A₃, A₄, and A₅ are defined as elsewhere in this description and wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (V) 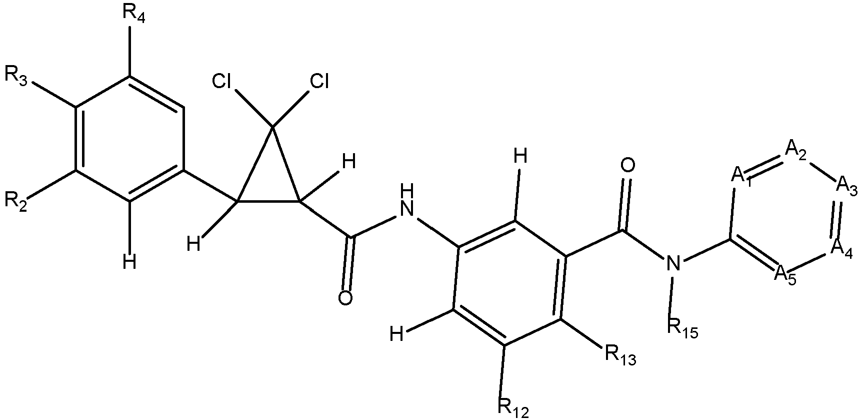
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
R¹⁵ is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (V) 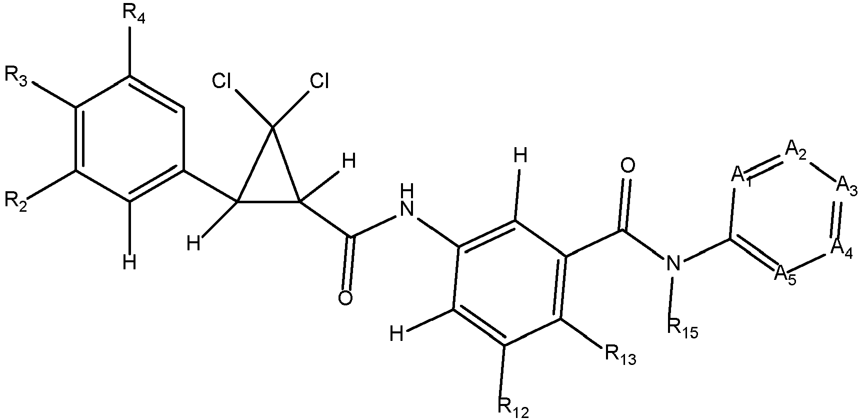
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₃ is CR₇, N=O, or N;
A₄ is CR₈, N=O or N;
A₅ is CR₉, N=O or N;
wherein no more than 3 of A₁, A₂, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or R⁶ and R⁷ together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by R⁶ and R⁷ is optionally substituted with one or more substituents of selected from the group consisting of (C₁-C₃)alkyl, F, Cl, Br, I, CN;
R⁸ is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or R⁷ and R⁸ together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by R⁷ and R⁸ is optionally substituted with one or more substituents of selected from the group consisting of (C₁-C₃)alkyl, F, Cl, Br, I, and CN;
R⁹ is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
R¹⁰ is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
R¹² is selected from the group consisting of H, F, Cl, CH₃ and CF₃;
R¹³ is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁶, R⁷, R⁸, R⁹, is a substituent selected from the group consisting of F, Cl, and Br, and I.

In an embodiment of the invention and/or embodiments thereof, the invention is directed to a compound of formula (V) 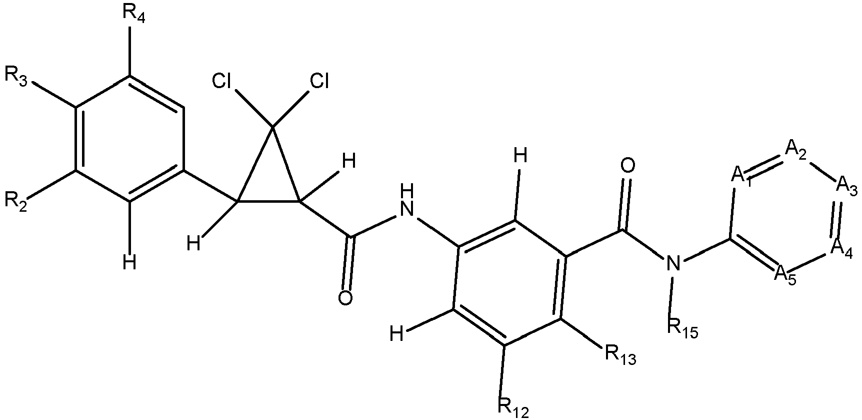
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₃ is CR₇ or N;
A₄ is CR₈ or N;
A₅ is CR₉ or N;
wherein no more than 3 of A₁, A₂, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (V) 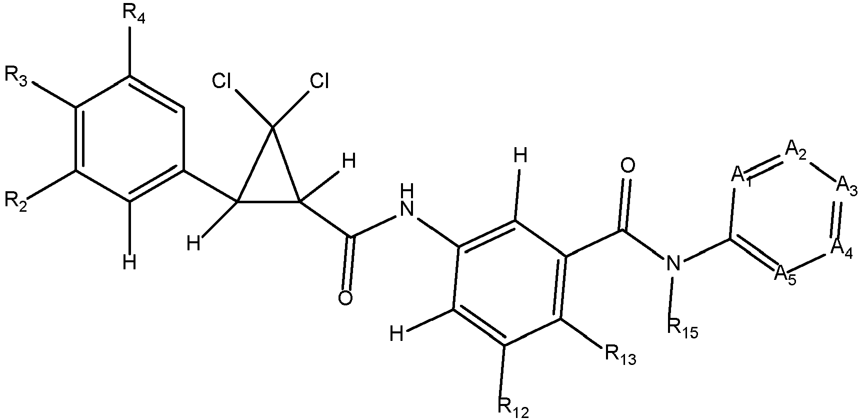
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R**⁶ and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R**⁶ and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-,
and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (V) 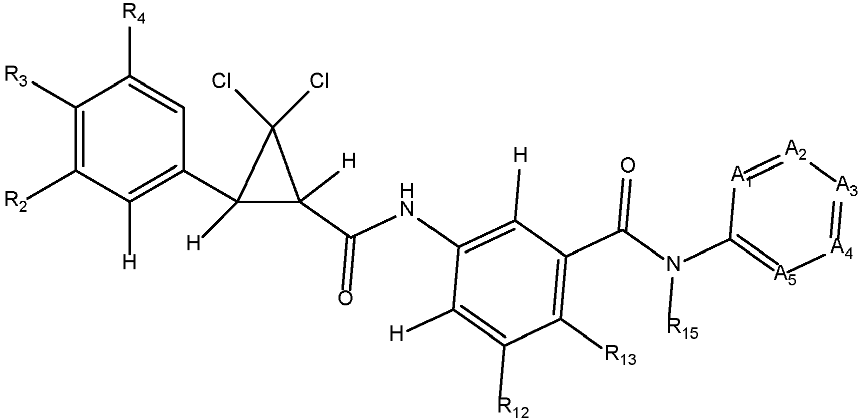
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₂ is CR₆ or N;
A₃ is CR₇;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (VI) 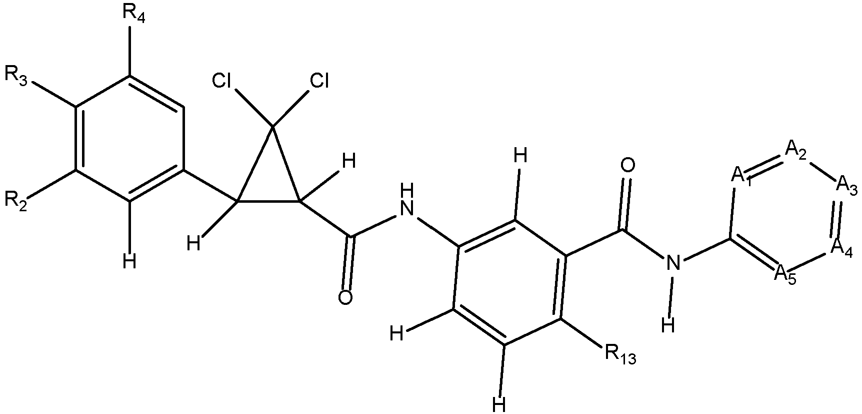
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁷, R⁸, R⁹, R¹³, A₁, A₂, A₃, A₄, and A₅ are defined as elsewhere in this description and wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (VI) 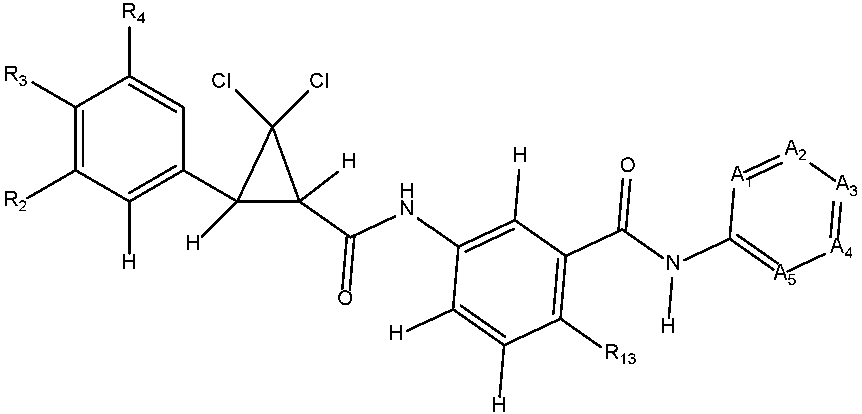
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N ;
wherein no more than 3 of A₁, A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R**⁶ and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (VI) 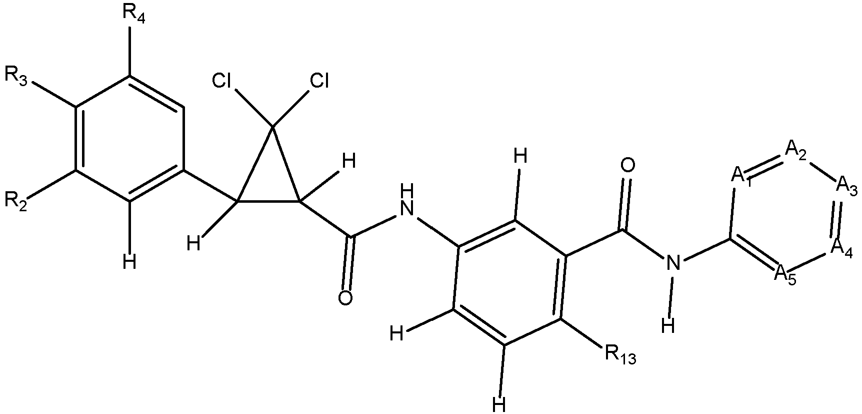
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₃ is CR₇, N=O, or N;
A₄ is CR₈, N=O or N;
A₅ is CR₉, N=O or N;
wherein no more than 3 of A₁, A₂, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
wherein at least one of R⁵, R⁶, R⁷, R⁸, R⁹, is a substituent selected from the group consisting of F, Cl, and Br, and I.

In an embodiment of the invention and/or embodiments thereof, the invention is directed to a compound of formula (VI) 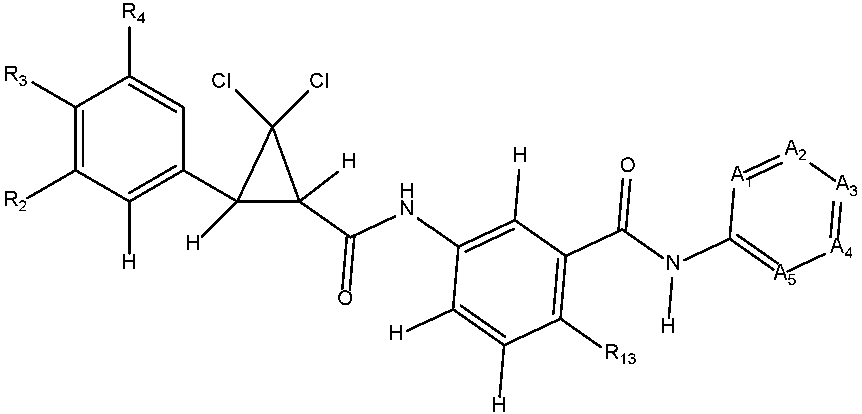
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₃ is CR₇ or N;
A₄ is CR₈ or N;
A₅ is CR₉ or N;
wherein no more than 3 of A₁, A₂, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (VI) 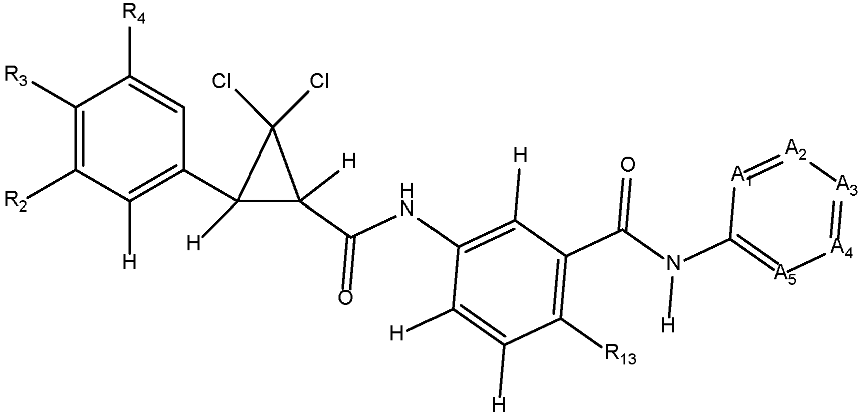
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₃ is CR₇ or N;
A₄ is CR₈ or N;
A₅ is CR₉ or N;
wherein 0 or 1 of A₁, A₂, A₃, A₄, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by R**⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-,
and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (VI) 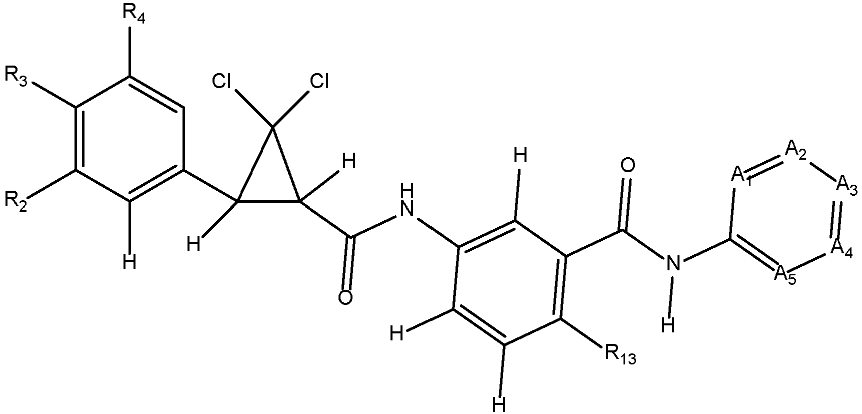
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₂ is CR₆ or N;
A₃ is CR₇;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (VII) 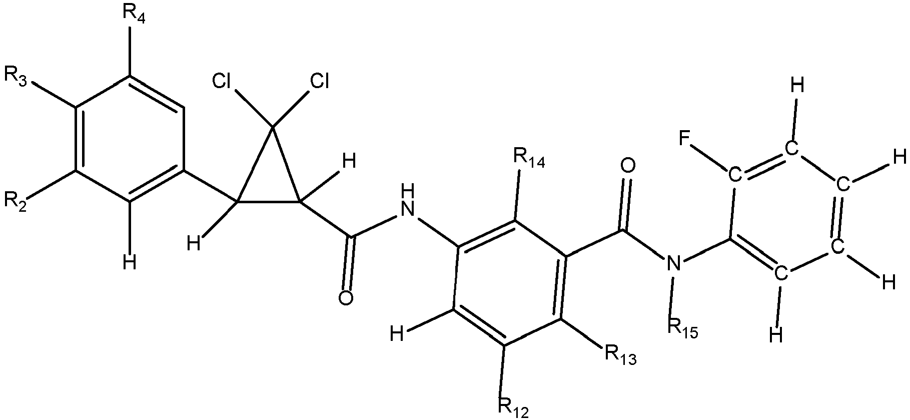
and N-oxides, veterinary acceptable acid addition salts, salt derivatives, solvates, ester derivatives, crystal polymorphs, isotopes, stereoisomers, and tautomers thereof,
wherein
wherein R², R³, R⁴, R¹², R¹³, R¹⁴, R¹⁵, are defined as elsewhere in this description.

In an embodiment of the invention and/or embodiments thereof, the invention is directed to a compound of formula (VII) 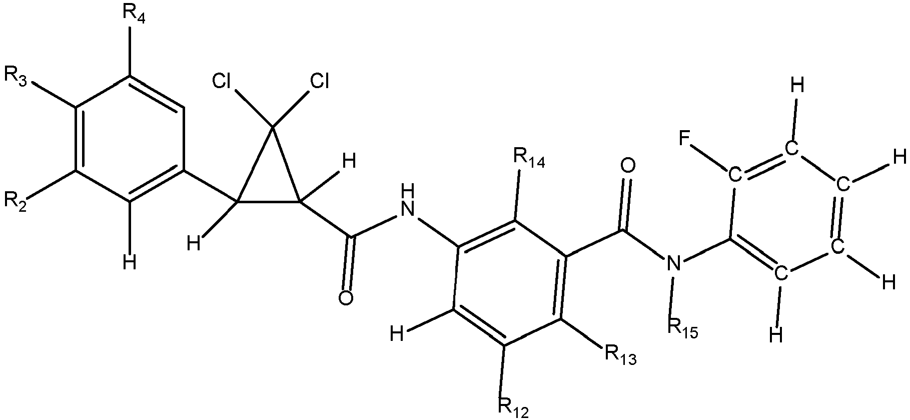
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (VII) 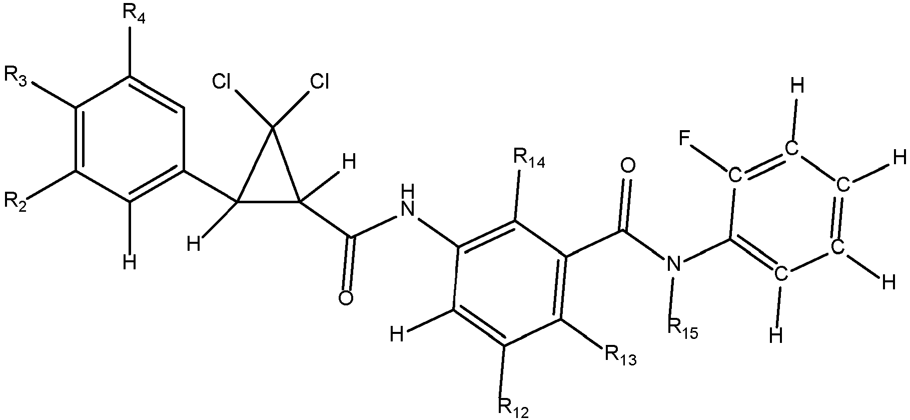
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (VII) 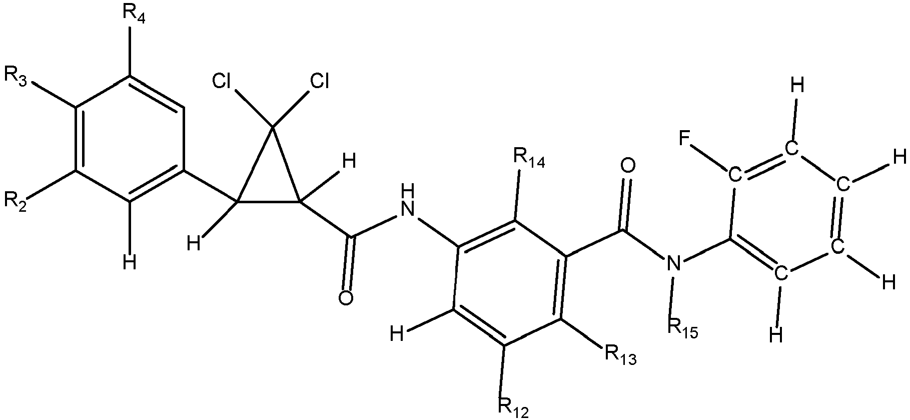
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃I.

In an embodiment of the invention and/or embodiments thereof, the invention is directed to a compound of formula (VII) 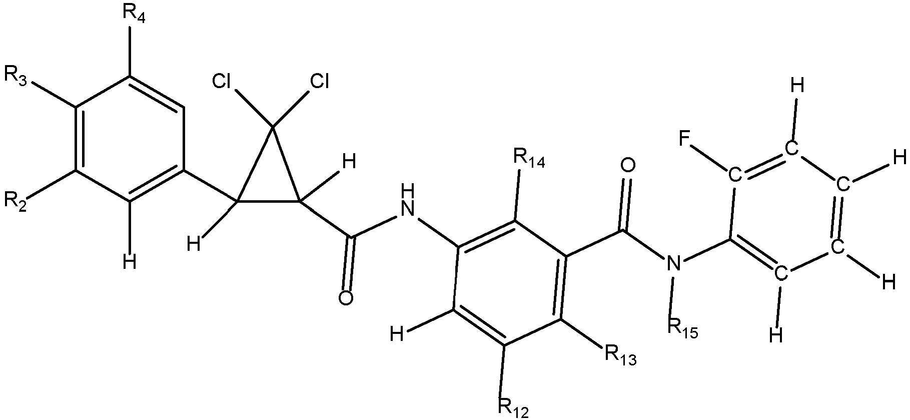
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (VII) 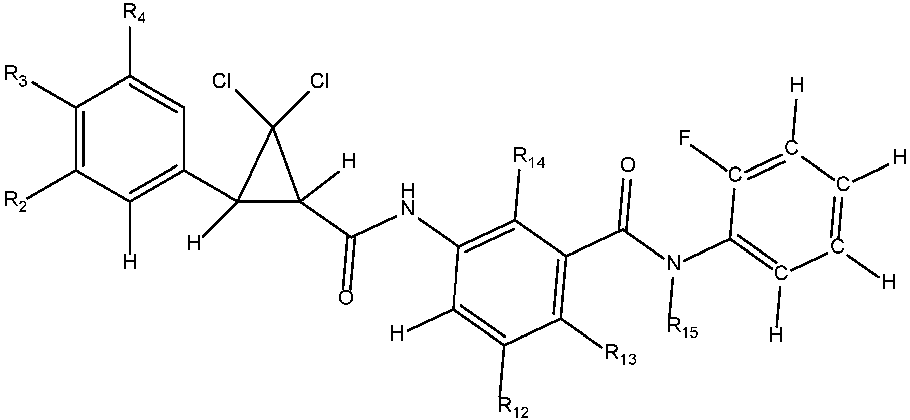
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H;
**R¹⁵** is H.

In an embodiment of the invention and/or embodiments thereof, the invention is directed to a compound of formula (VIII) 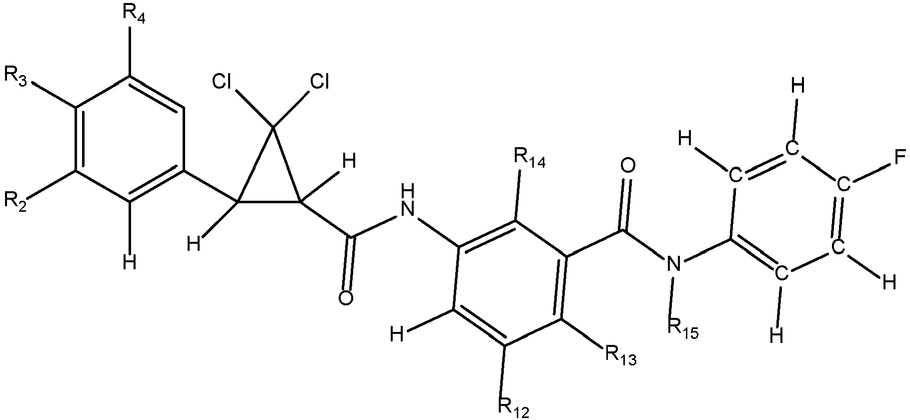
and N-oxides, veterinary acceptable acid addition salts, salt derivatives, solvates, ester derivatives, crystal polymorphs, isotopes, stereoisomers, and tautomers thereof,
wherein
wherein R², R³, R⁴, R¹², R¹³, R¹⁴, and R¹⁵, are defined as elsewhere in this description.

In an embodiment of the invention and/or embodiments thereof, the invention is directed to a compound of formula (VIII) 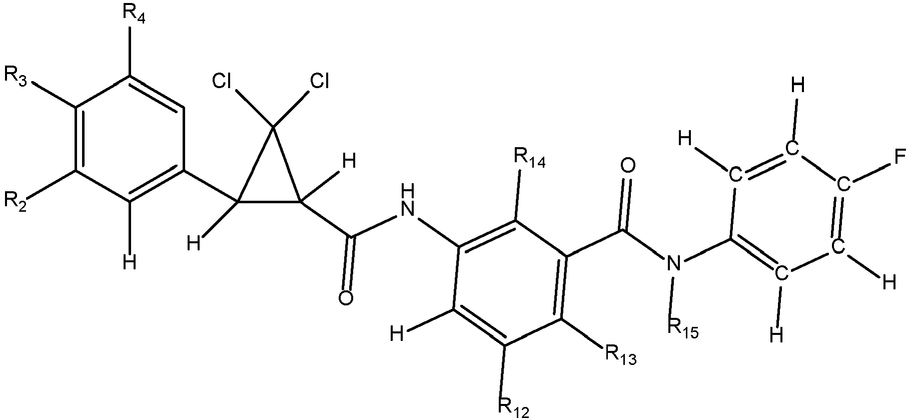
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (VIII) 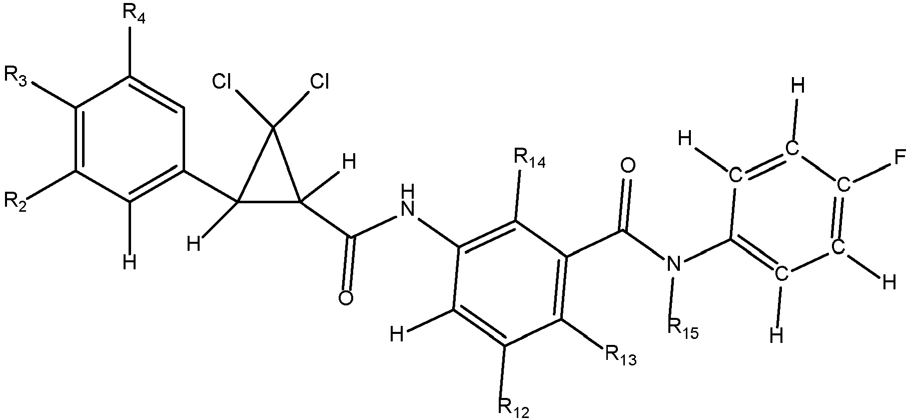
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (VIII) 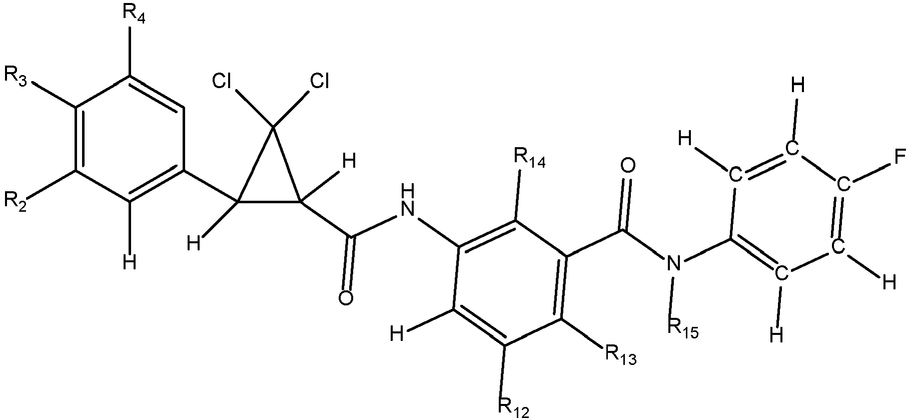
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (VIII) 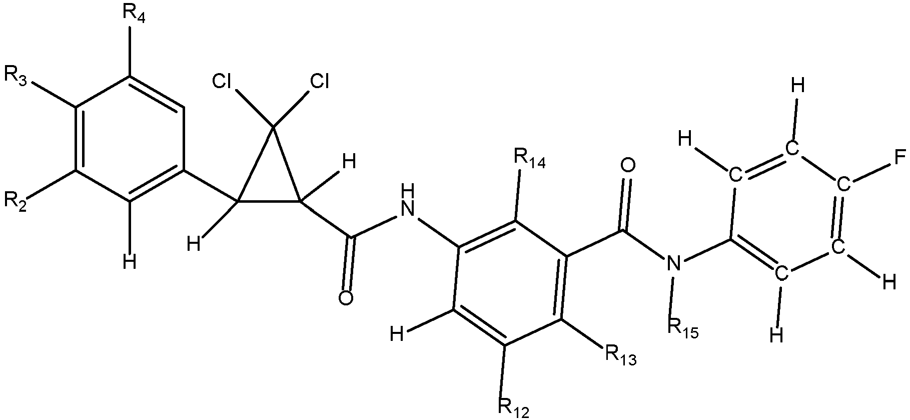
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (VIII) 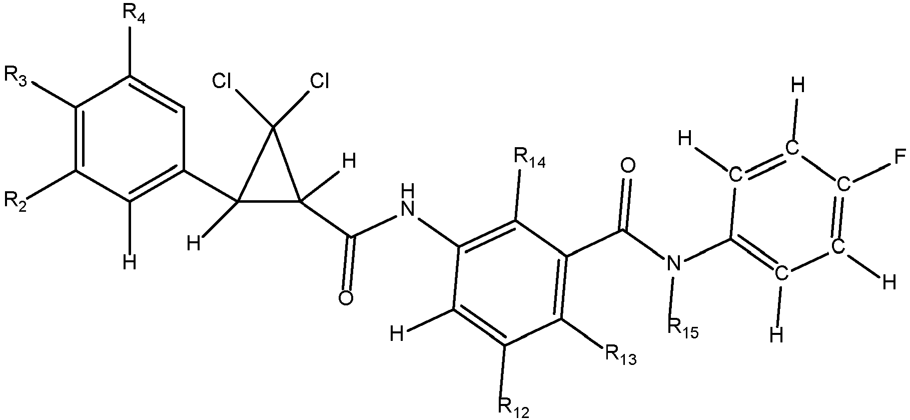
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H;
**R¹⁵** is H.

In an embodiment of the invention and/or embodiments thereof, the invention is directed to a compound of formula (IX) 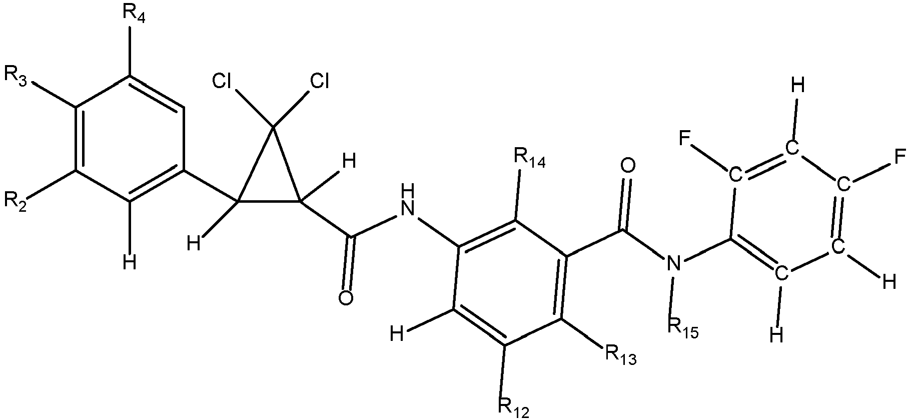
and N-oxides, veterinary acceptable acid addition salts, salt derivatives, solvates, ester derivatives, crystal polymorphs, isotopes, stereoisomers, and tautomers thereof,
wherein
R², R³, R⁴, R¹², R¹³, R¹⁴, and R¹⁵ are defined as elsewhere in this description.

In an embodiment of the invention and/or embodiments thereof, the invention is directed to a compound of formula (IX) 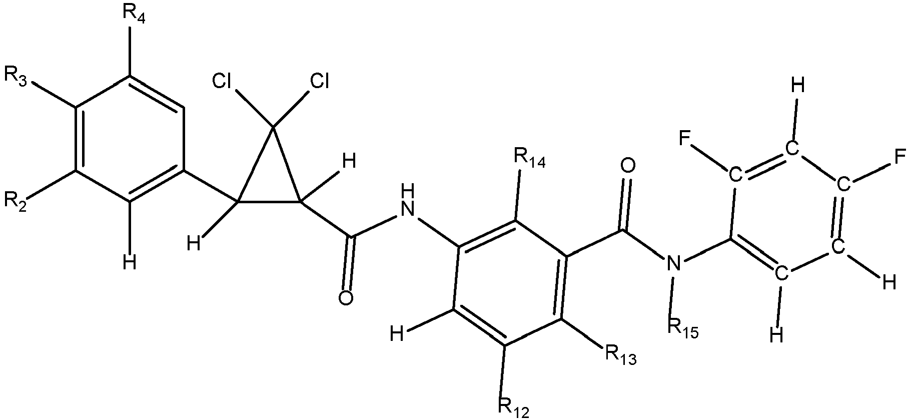
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (IX) 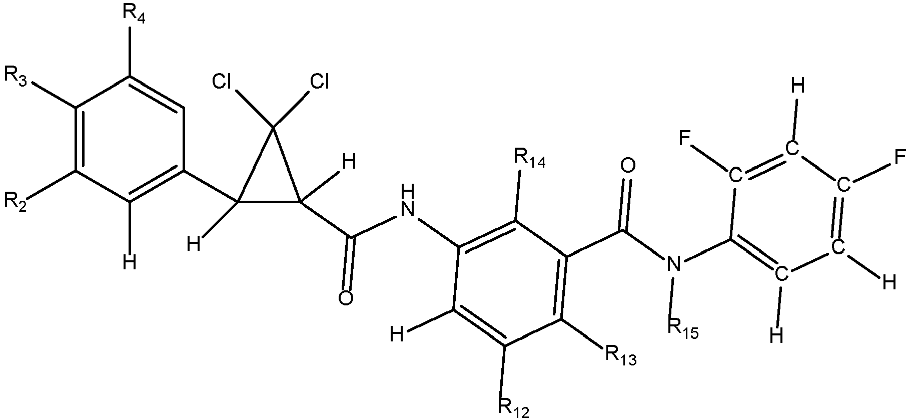
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (IX) 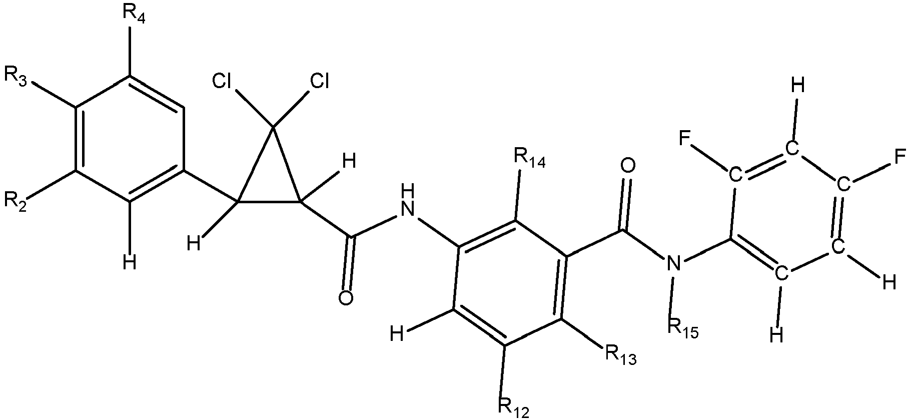
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
R³ is selected from the group consisting of H, F, Cl, Br, I, (C₁-C₆)alkyl, (C₁-C₆)alkoxy, (C₁-C₆)haloalkyl, and (C₁-C₆) haloalkoxy ;
R⁴ is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (IX) 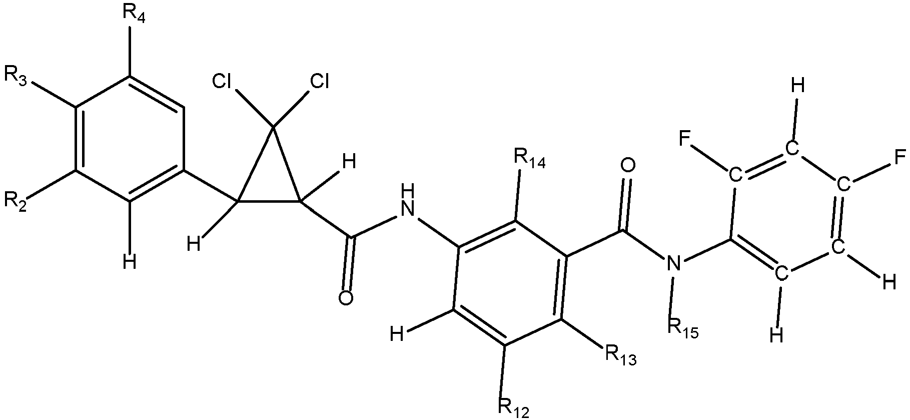
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (IX) 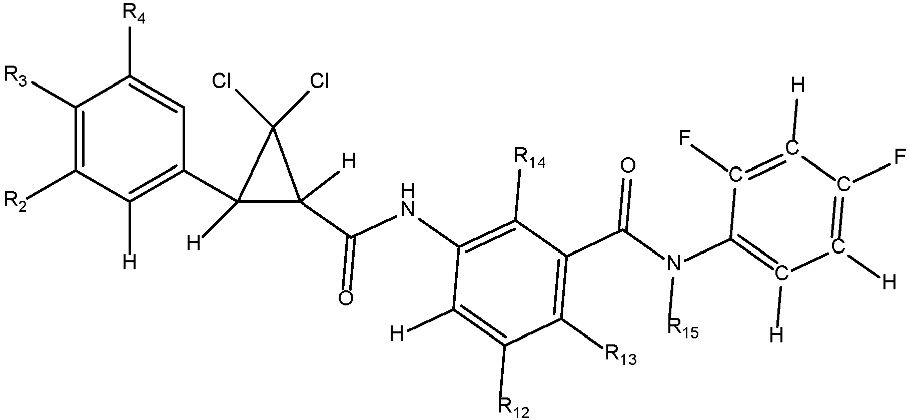
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H;
**R¹⁵** is H.

In an embodiment of the invention and/or embodiments thereof, the invention is directed to a compound of formula (X) 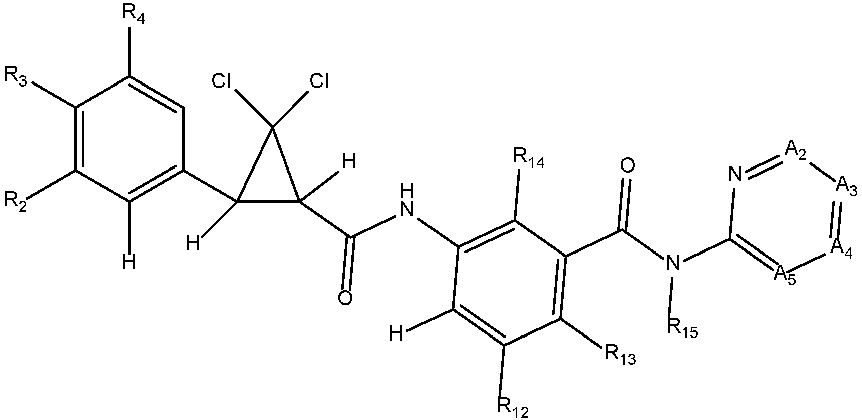
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁷, R⁸, R⁹, R¹², R¹³, R¹⁴, R¹⁵, A₂, A₃, A₄, and A₅ are defined as elsewhere in this description and wherein at least one of R⁶, R⁷, R⁸, R⁹, is a substituent selected from the group consisting of F, Cl, Br, and I.

In an embodiment of the invention and/or embodiments thereof, the invention is directed to a compound of formula (X) 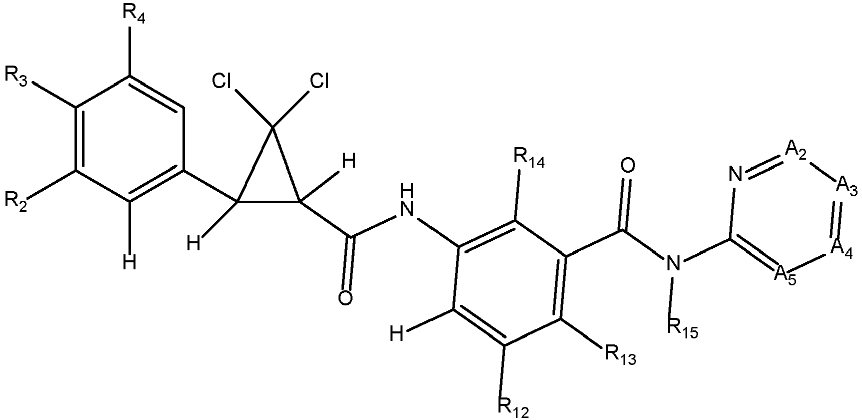
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (X) 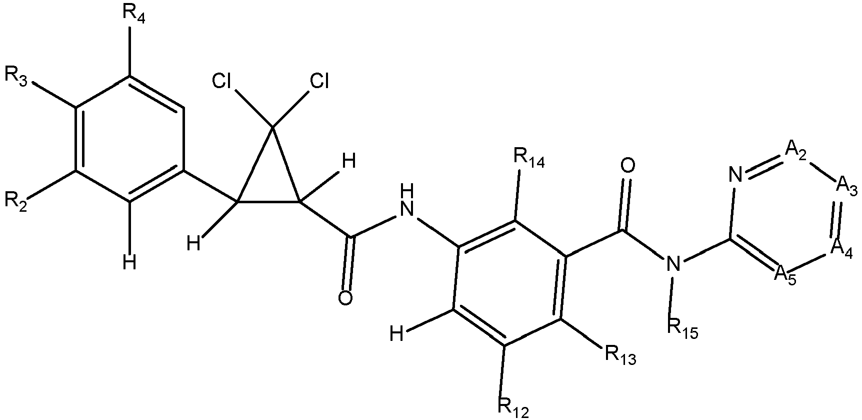
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, and Br, and I.

In an embodiment of the invention and/or embodiments thereof, the invention is directed to a compound of formula (X) 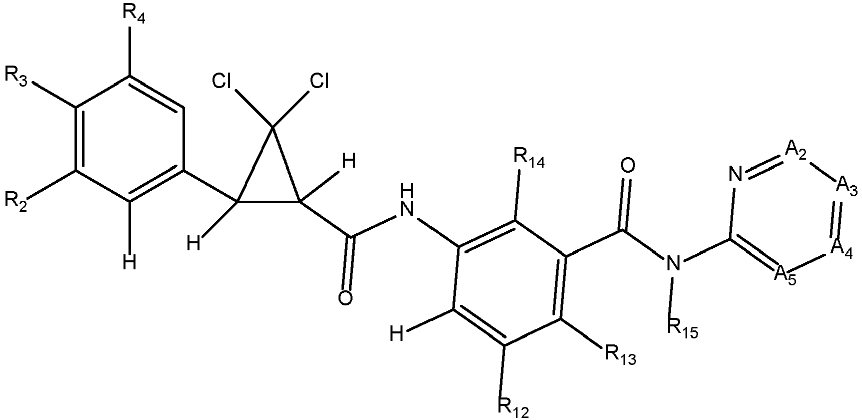
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₃ is CR₇ or N;
A₄ is CR₈ or N;
A₅ is CR₉ or N;
wherein no more than 2 of A₂, A₃, A₄, A₅ are N;
R**⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (X) 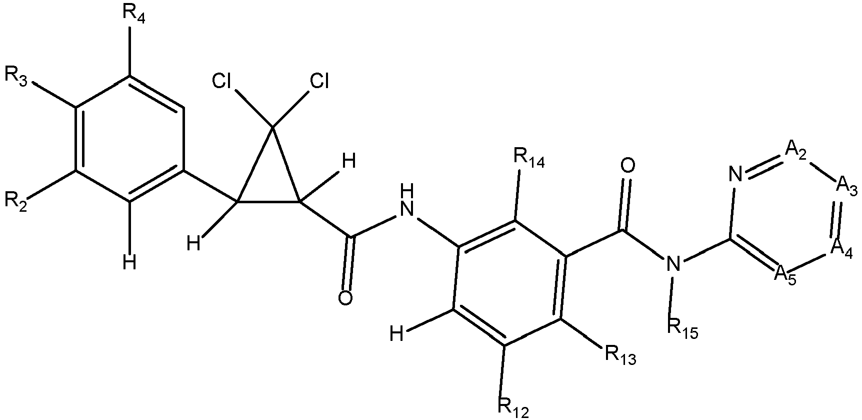
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₂ is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃;
wherein at least one of R⁶, R⁷, R⁸, R⁹, is a substituent selected from the group consisting of F, and Cl.

In an embodiment of the invention and/or embodiments thereof, the invention is directed to a compound of formula (X) 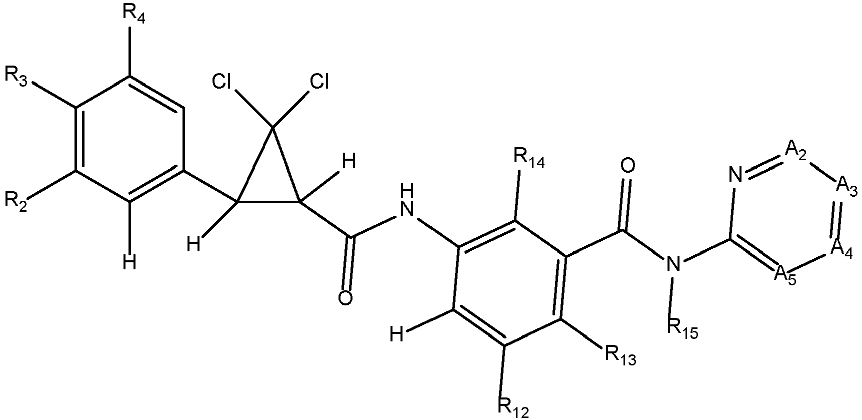
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A2 is CR₆ or N;
A₃ is CR₇;
A₄ is CR₈;
A₅ is CR₉;
wherein 0 or 1 of A₂ are N;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H
wherein at least one of R⁶, R⁷, R⁸, R⁹, is a substituent selected from the group consisting of F, and Cl.

In an embodiment of the invention and/or embodiments thereof, the invention is directed to a compound of formula (XI) 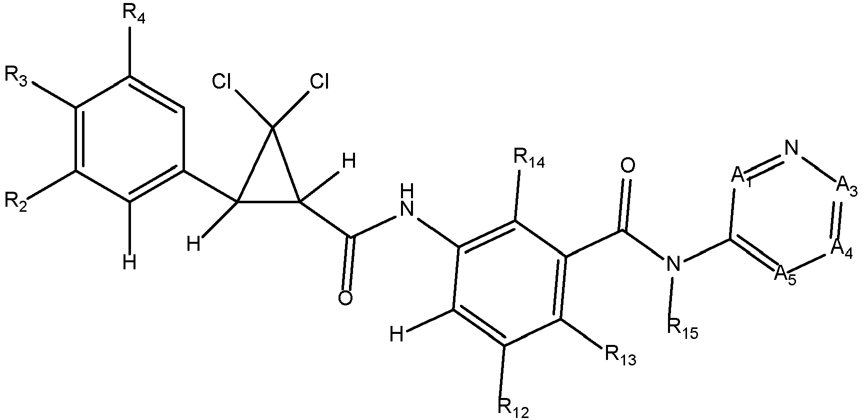
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁷, R⁸, R⁹, R¹², R¹³, R¹⁴, R¹⁵, A₁, A₃, A₄, and A₅ are defined as elsewhere in this description and wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XI) 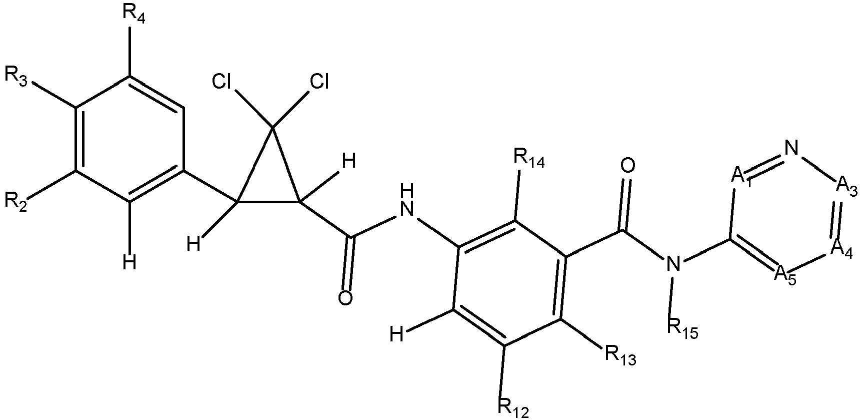
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₃ is CR₇, N=O, or N;
A₄ is CR₈, N=O or N;
A₅ is CR₉, N=O or N ;
wherein no more than 2 of A₁, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, CI, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XI) 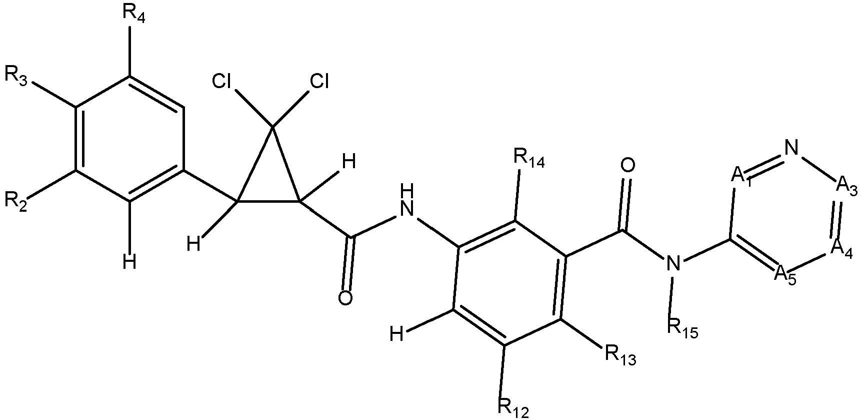
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₃ is CR₇, N=O, or N;
A₄ is CR₈, N=O or N;
A₅ is CR₉, N=O or N;
wherein no more than 2 of A₁, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
R¹² is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁷, R⁸, R⁹, is a substituent selected from the group consisting of F, Cl, and Br, and I.

In an embodiment of the invention and/or embodiments thereof, the invention is directed to a compound of formula (XI) 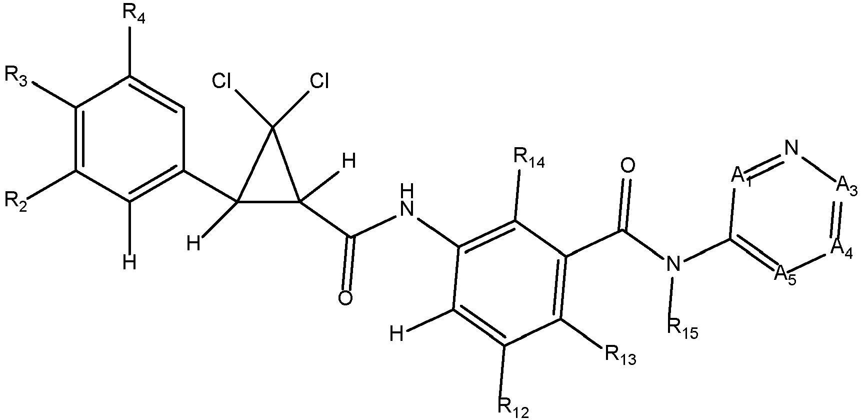
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₃ is CR₇ or N;
A₄ is CR₈ or N;
A₅ is CR₉ or N;
wherein no more than 2 of A₁, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
**R⁸** is selected from the group consisting of H, F, Cl, NH(C₁-C₆)alkyl, and (C₁-C₆)alkyl ;
or R⁷ and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XI) 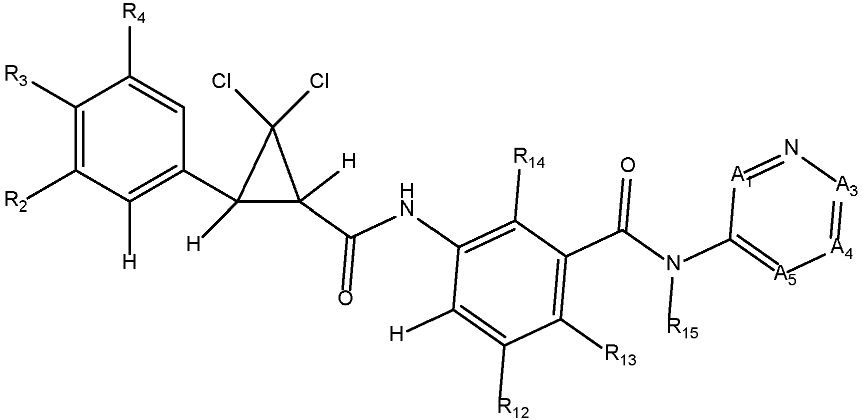
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₃ is CR₇ or N;
A₄ is CR₈ or N;
A₅ is CR₉ or N;
wherein 0 or 1 of A₁, A₃, A₄, A₅ are N;
**R⁵** is selected from the group consisting of H, F, Cl, CN, NO₂, NH(C₁-C₆)alkyl, and (C₁-C₆)alkyl ;
**R⁷** is selected from the group consisting of H, F, and Cl ;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃;
wherein at least one of R⁵, R⁷, R⁸, R⁹, is a substituent selected from the group consisting of F, and Cl.

In an embodiment of the invention and/or embodiments thereof, the invention is directed to a compound of formula (XI) 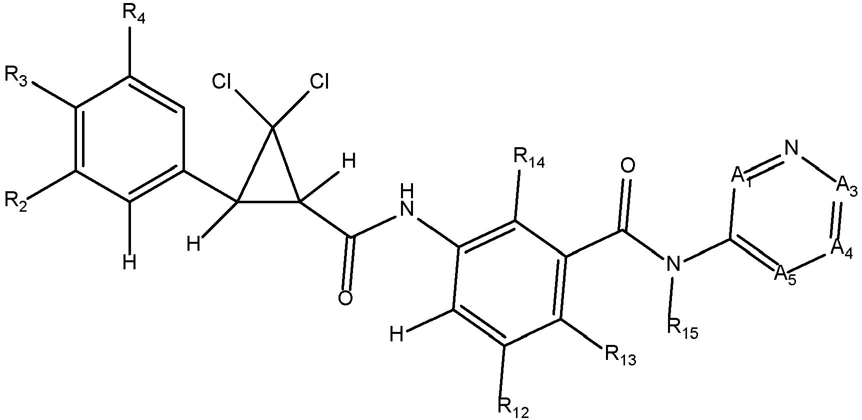
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₃ is CR₇;
A₄ is CR₈;
A₅ is CR₉;
wherein 0 or 1 of A₁ are N;
**R⁵** is selected from the group consisting of H, F, Cl, CN and (C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H
wherein at least one of R⁵, R⁷, R⁸, R⁹, is a substituent selected from the group consisting of F, and Cl.

In an embodiment of the invention and/or embodiments thereof, the invention is directed to a compound of formula (XII) 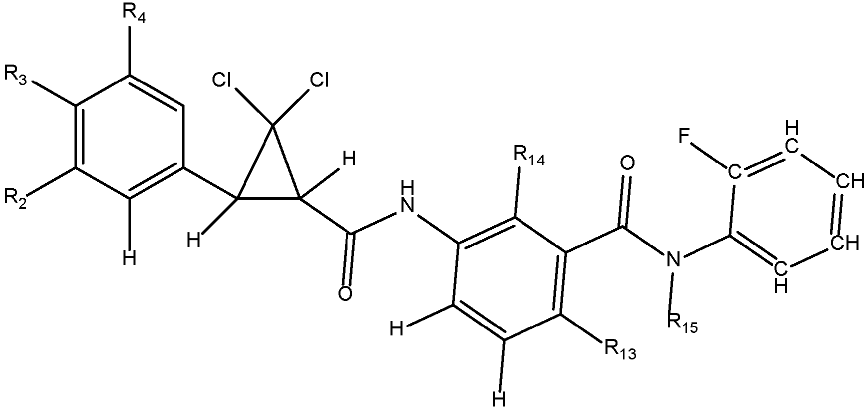
and N-oxides, veterinary acceptable acid addition salts, salt derivatives, solvates, ester derivatives, crystal polymorphs, isotopes, stereoisomers, and tautomers thereof,
wherein R², R³, R⁴, R¹³, R¹⁴, R¹⁵, are defined as elsewhere in this description.

In an embodiment of the invention and/or embodiments thereof, the invention is directed to a compound of formula (XII) 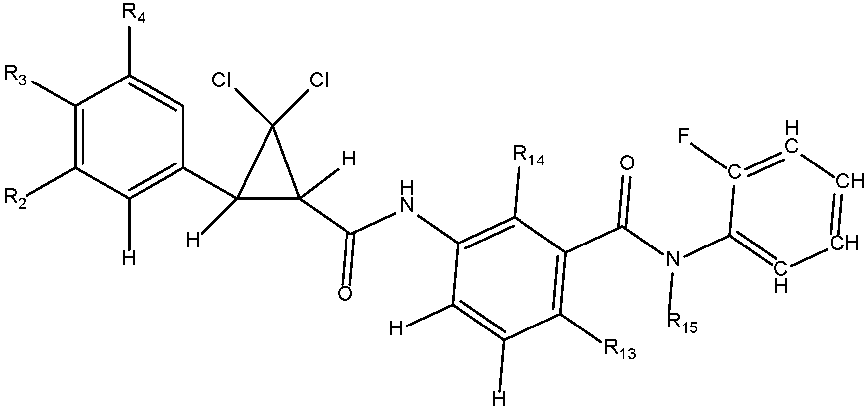
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XII) 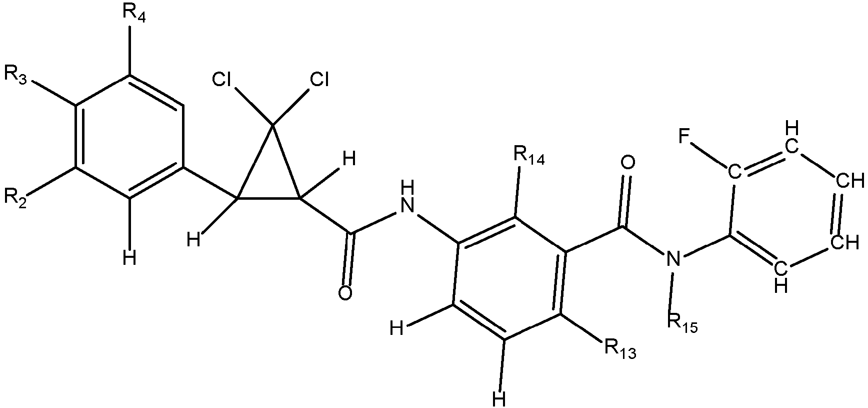
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XII) 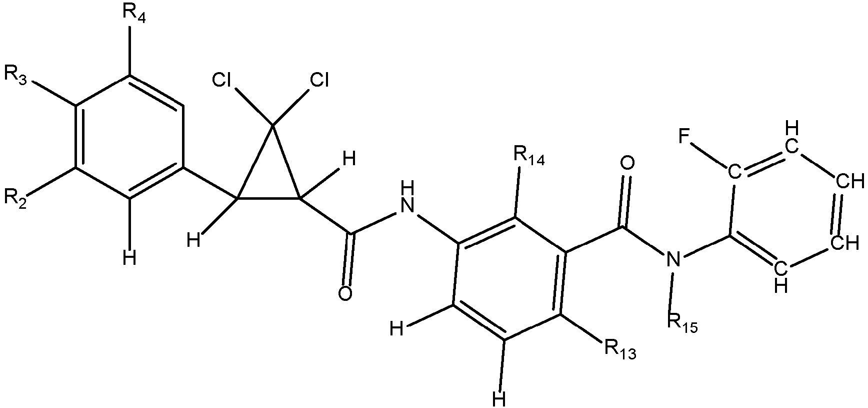
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XII) 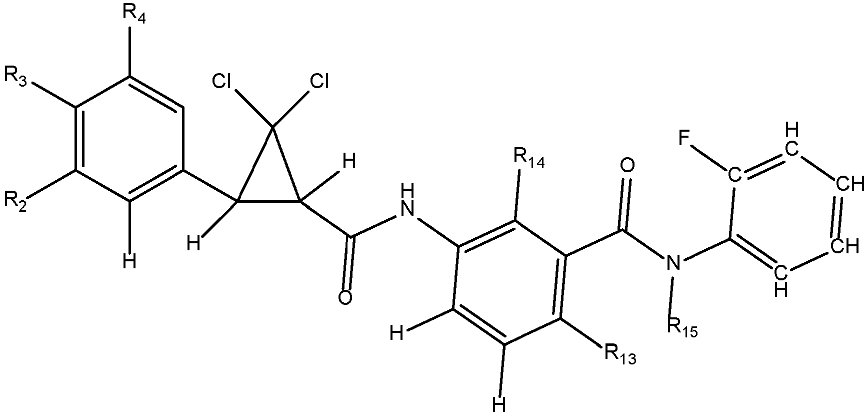
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XII) 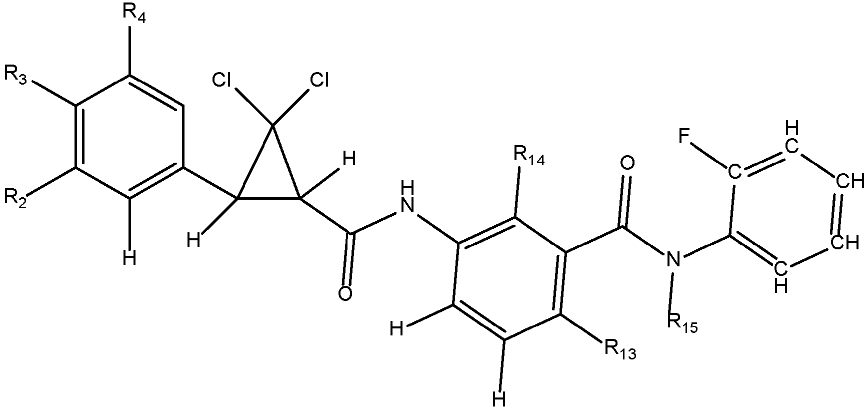
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl;
**R¹⁴** is H;
**R¹⁵** is H.

In an embodiment of the invention and/or embodiments thereof, the invention is directed to a compound of formula (XIII) 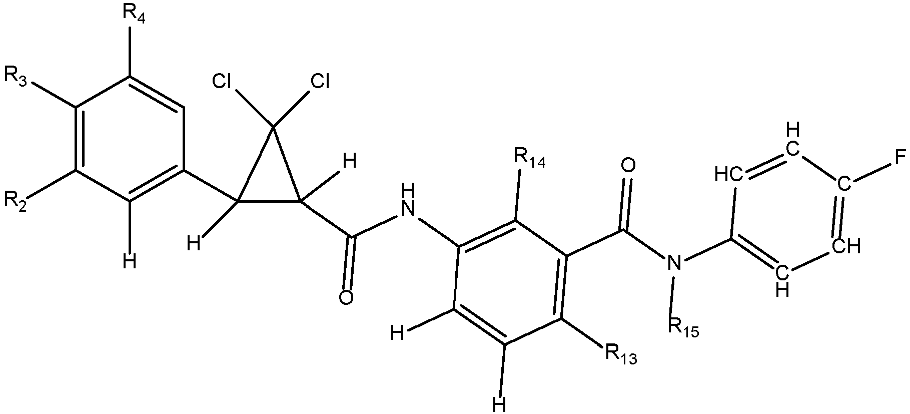
and N-oxides, veterinary acceptable acid addition salts, salt derivatives, solvates, ester derivatives, crystal polymorphs, isotopes, stereoisomers, and tautomers thereof,
wherein R², R³, R⁴, _{,} R¹³, R¹⁴, R¹⁵, are defined as elsewhere in this description.

In an embodiment of the invention and/or embodiments thereof, the invention is directed to a compound of formula (XIII) 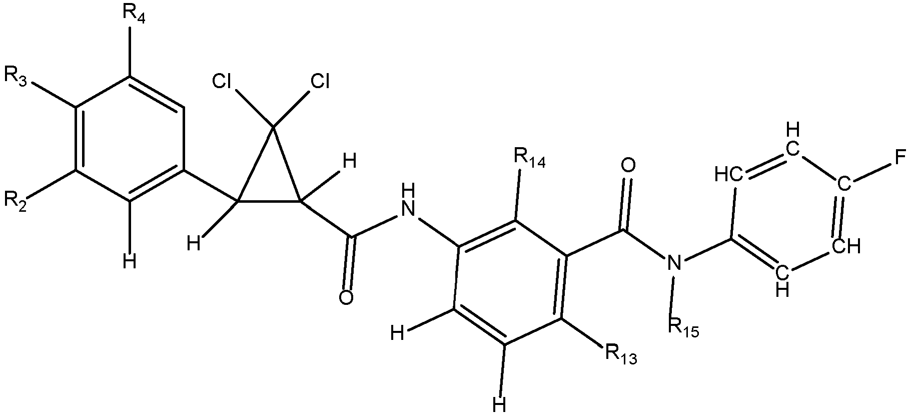
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
R³ is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
R⁴ is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
R¹³ is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
R¹⁴ is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
R¹⁵ is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XIII) 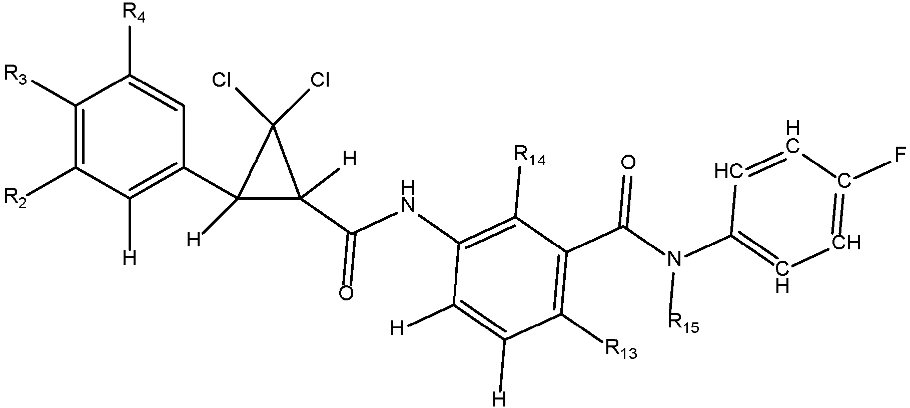
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
R³ is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
R⁴ is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃ ;
R¹⁴ is selected from the group consisting of H, F, Cl, CH₃ and CF₃ ;
R¹⁵ is selected from the group consisting of H, CH₃, and CF₃.

In an embodiment of the invention and/or embodiments thereof, the invention is directed to a compound of formula (XIII) 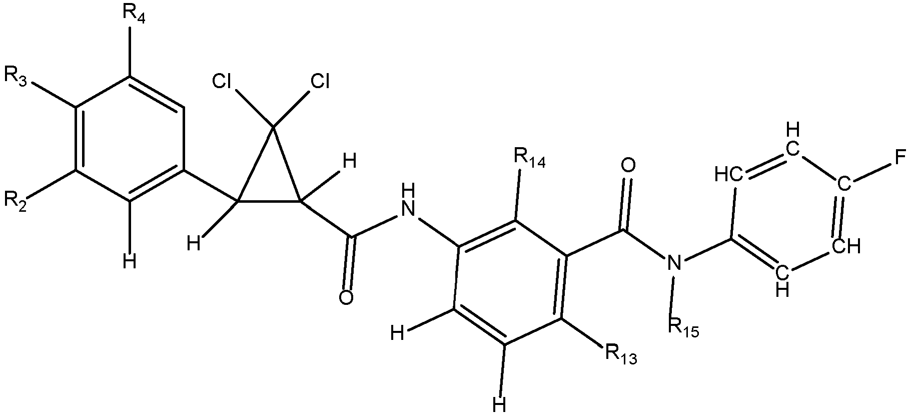
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XIII) 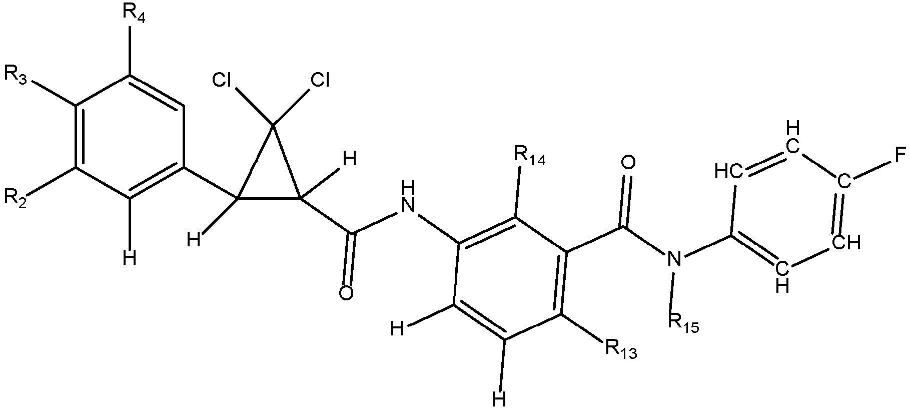
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XIII) 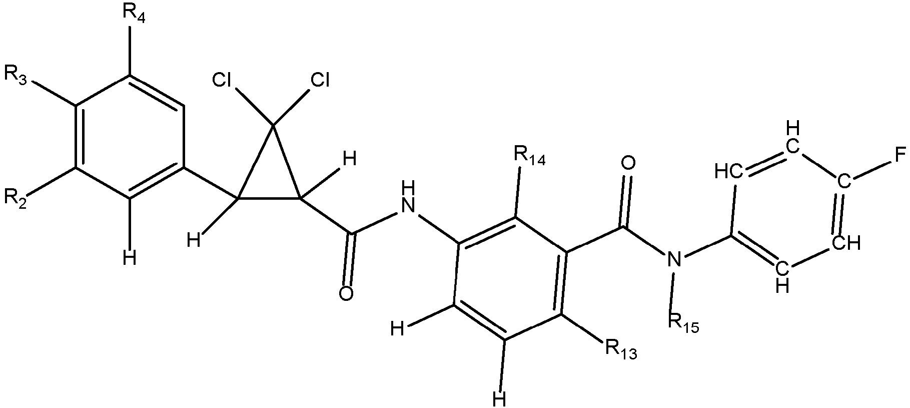
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl;
**R¹⁴** is H;
**R¹⁵** is H.

In an embodiment of the invention and/or embodiments thereof, the invention is directed to a compound of formula (XIV) 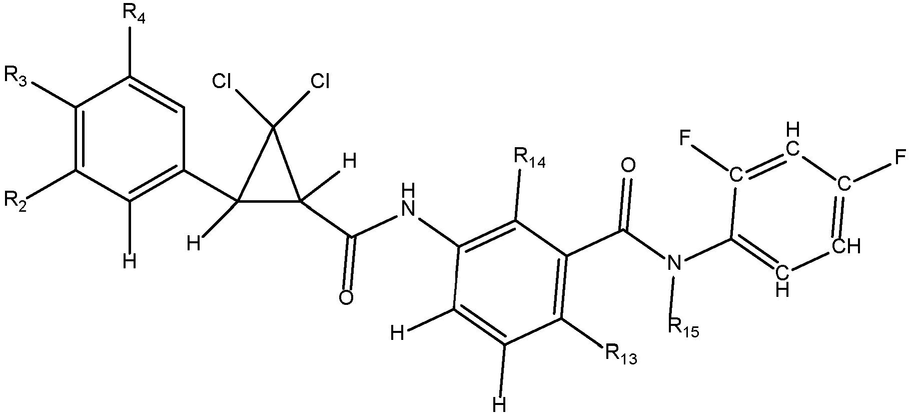
and N-oxides, veterinary acceptable acid addition salts, salt derivatives, solvates, ester derivatives, crystal polymorphs, isotopes, stereoisomers, and tautomers thereof,
wherein R², R³, R⁴, R¹³, R¹⁴, R¹⁵, are defined as elsewhere in this description.

In an embodiment of the invention and/or embodiments thereof, the invention is directed to a compound of formula (XIV) 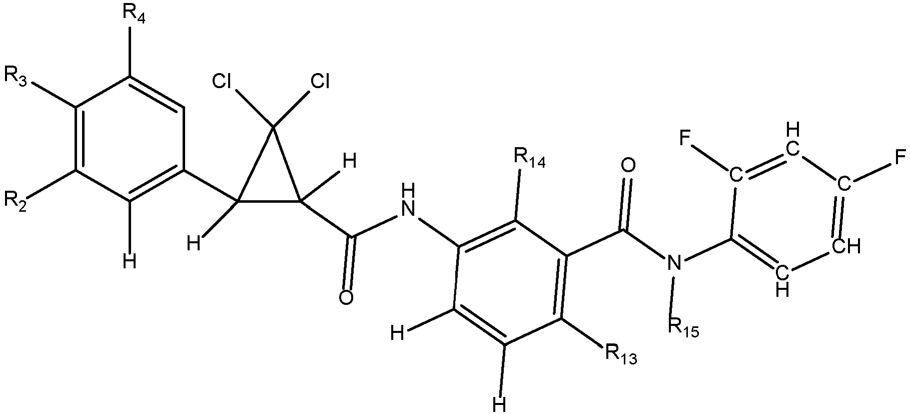
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XIV) 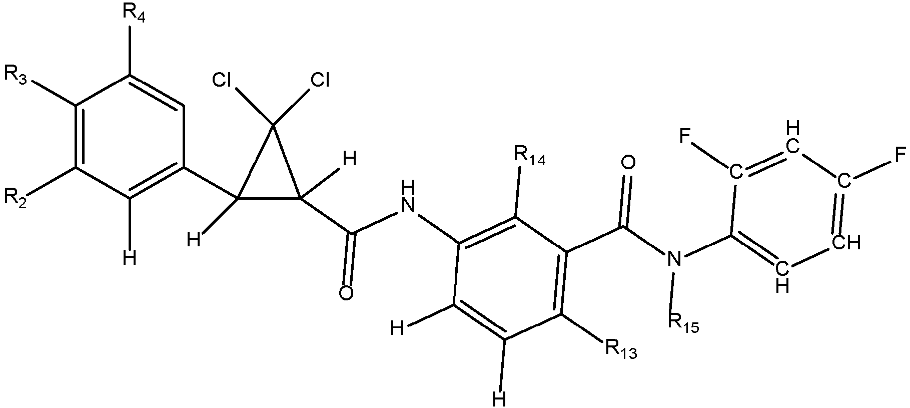
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XIV) 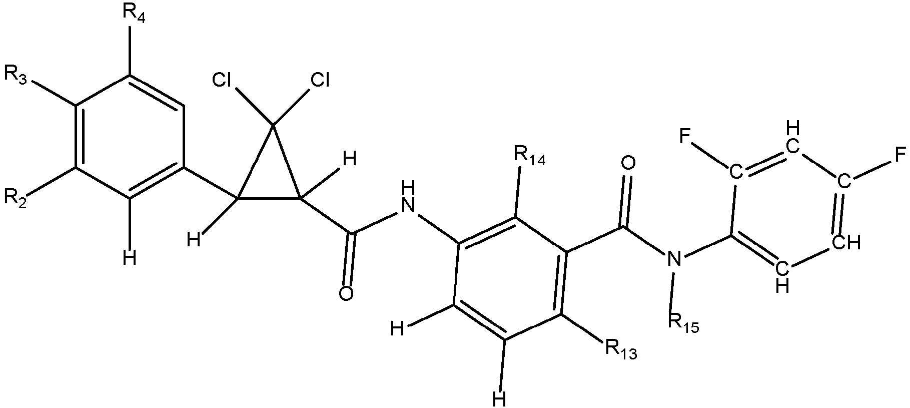
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XIV) 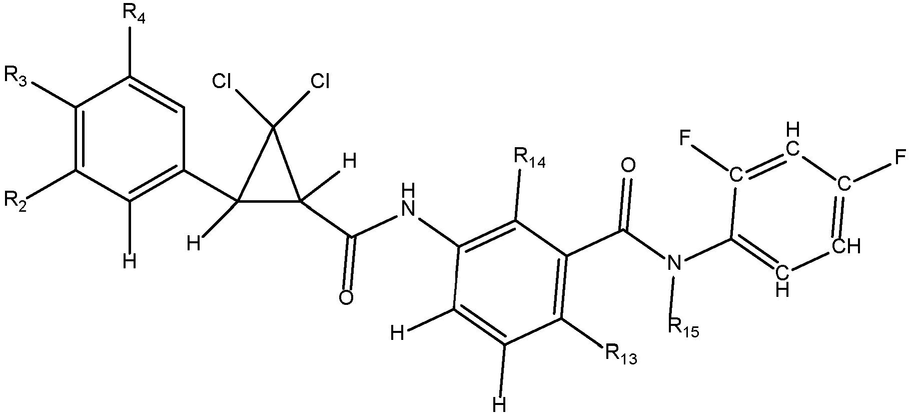
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XIV) 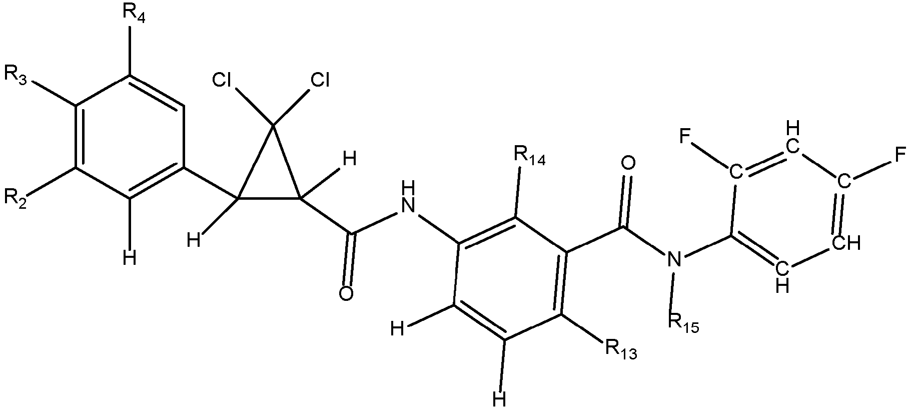
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl;
**R¹⁴** is H;
**R¹⁵** is H.

In an embodiment of the invention and/or embodiments thereof, the invention is directed to a compound of formula (XV) 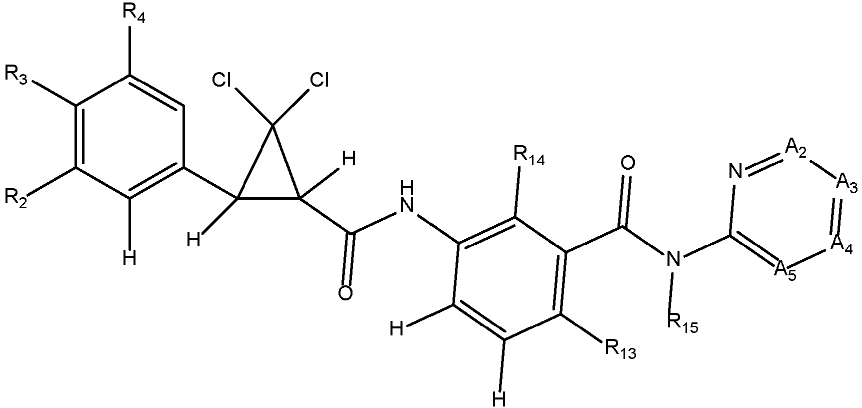
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹³, R¹⁴, R¹⁵, A₂, A₃, A₄, and A₅ are defined as elsewhere in this description and wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XV) 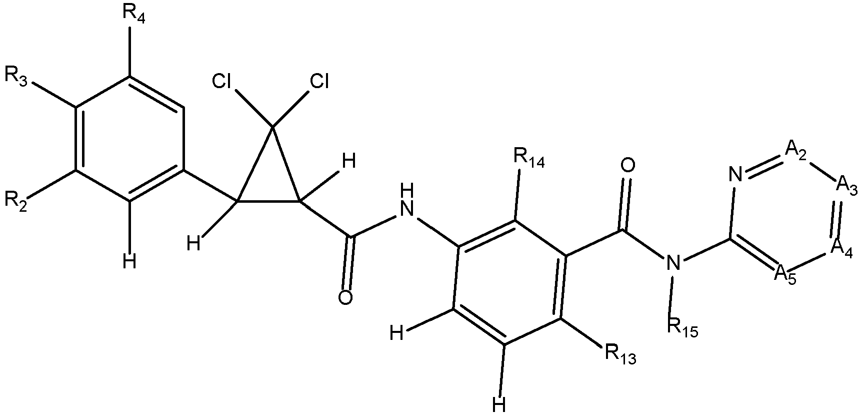
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴ is** selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XV) 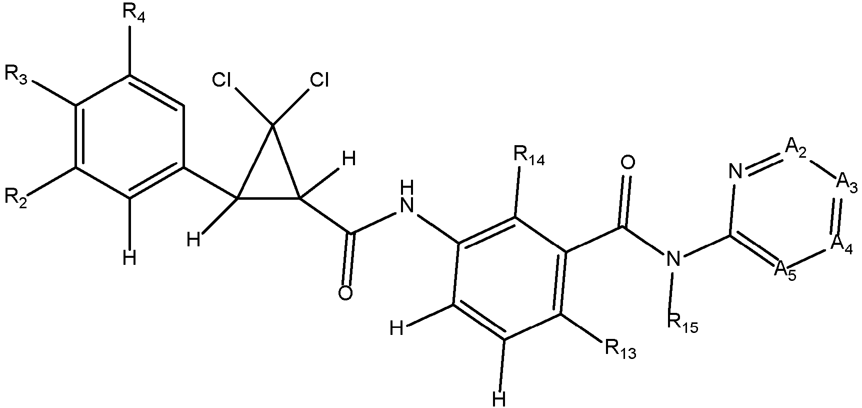
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R**⁷ and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R**⁷ and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, and Br, and I.

In an embodiment of the invention and/or embodiments thereof, the invention is directed to a compound of formula (XV) 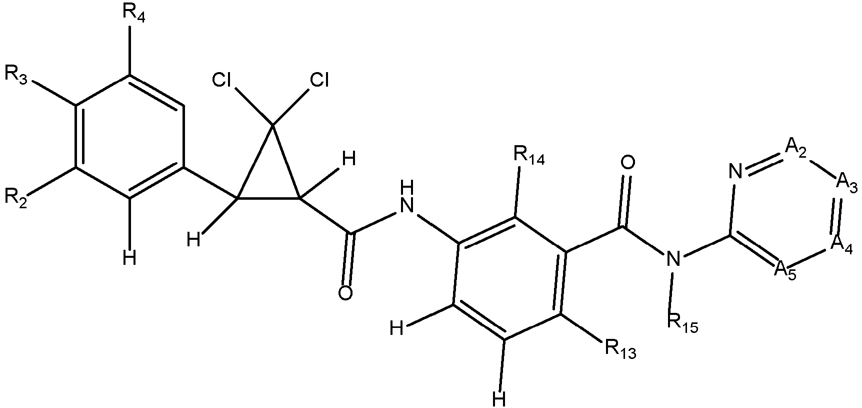
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A2 is CR₆ or N;
A₃ is CR₇ or N;
A4 is CR₈ or N;
A₅ is CR₉ or N;
wherein no more than 2 of A₂, A₃, A₄, A₅ are N;
**R⁶ is** selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R**⁶ and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹² is** selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XV) 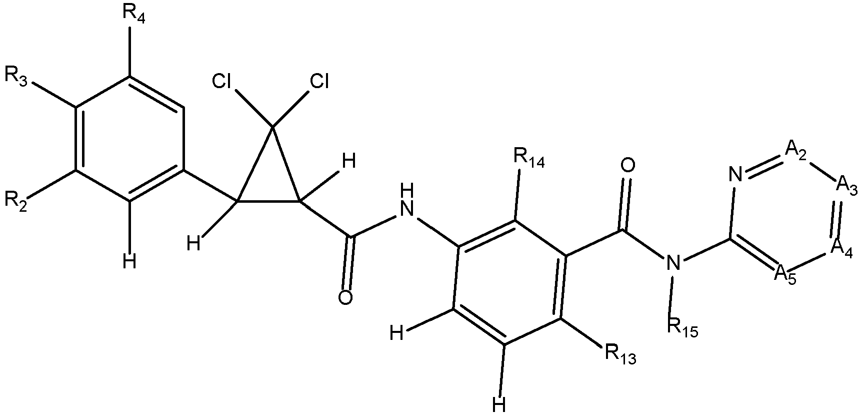
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₂ is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
**or R**⁶ and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R**⁶ and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃;
wherein at least one of R⁶, R⁷, R⁸, R⁹, is a substituent selected from the group consisting of F, and Cl.

In an embodiment of the invention and/or embodiments thereof, the invention is directed to a compound of formula (XV) 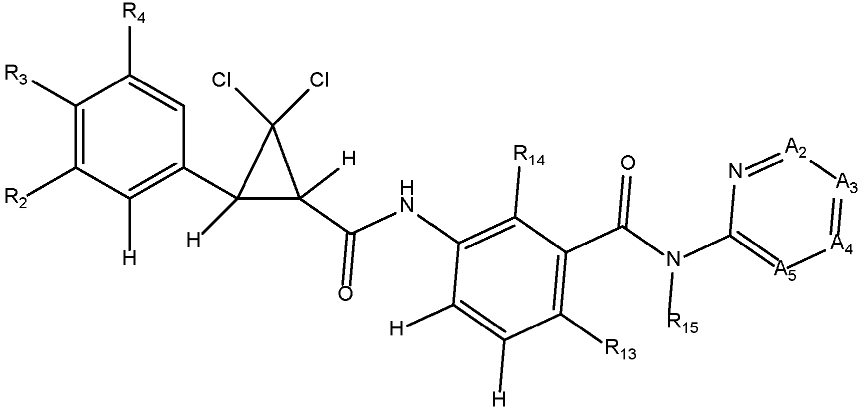
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A2 is CR₆ or N;
A₃ is CR₇;
A₄ is CR₈;
A₅ is CR₉;
**R⁶** is selected from the group consisting of H, F, and NH(C₁-C₆)alkyl ;
**R⁷** is selected from the group consisting of H, and F ;
**R⁸** is selected from the group consisting of H, and F ;
R⁹ is selected from the group consisting of H and F ;
**R¹²** is H ;
**R¹³** is Cl;
**R¹⁴** is H;
**R¹⁵** is H
wherein at least one of R⁶, R⁷, R⁸, R⁹, is a substituent selected from the group consisting of F, and Cl.

In an embodiment of the invention and/or embodiments thereof, the invention is directed to a compound of formula (XVI) 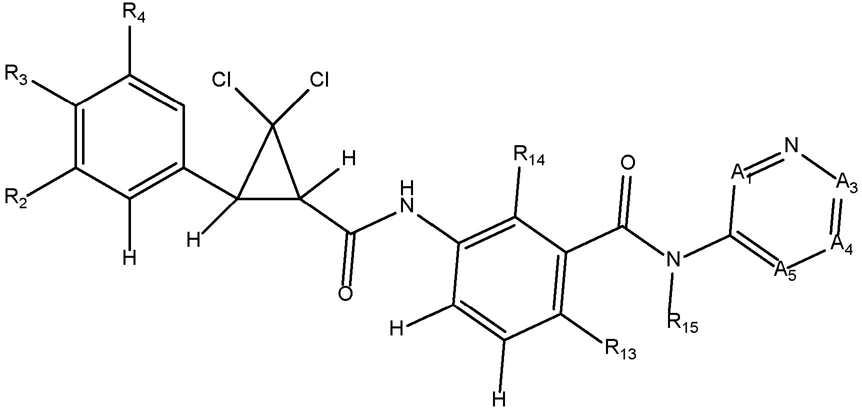
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁷, R⁸, R⁹, R¹³, R¹⁴, R¹⁵, A₁, A₃, A₄, and A₅ are defined as elsewhere in this description and wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XVI) 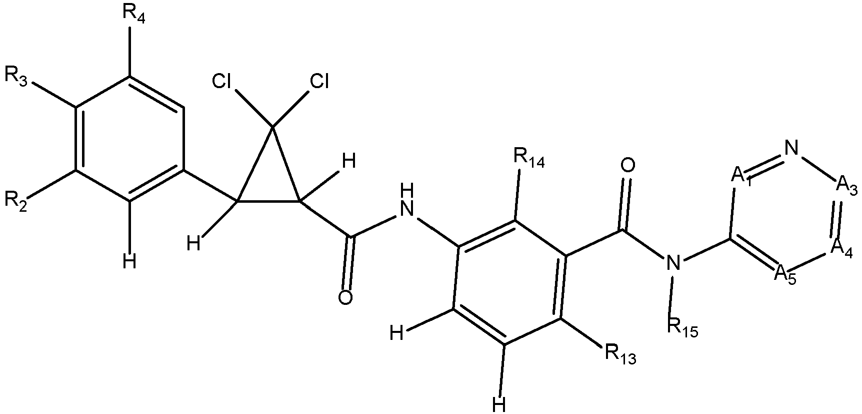
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₃ is CR₇, N=O, or N;
A₄ is CR₈, N=O or N;
A₅ is CR₉, N=O or N ;
wherein no more than 2 of A₁, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
R¹⁰ is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
R¹³ is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XVI) 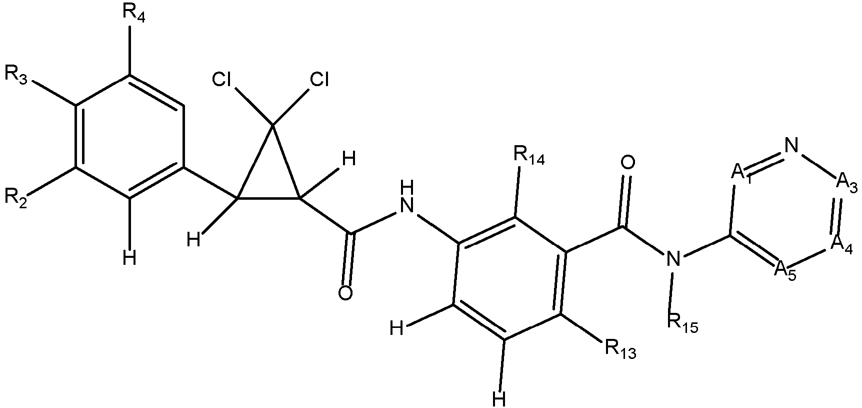
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₃ is CR₇, N=O, or N;
A₄ is CR₈, N=O or N;
A₅ is CR₉, N=O or N;
wherein no more than 2 of A₁, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁷, R⁸, R⁹, is a substituent selected from the group consisting of F, Cl, and Br, and I.

In an embodiment of the invention and/or embodiments thereof, the invention is directed to a compound of formula (XVI) 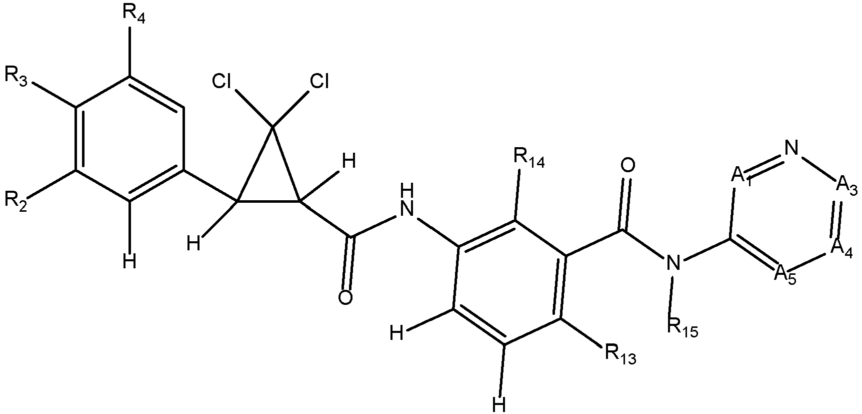
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₃ is CR₇ or N;
A₄ is CR₈ or N;
A₅ is CR₉ or N;
wherein no more than 2 of A₁, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XVI) 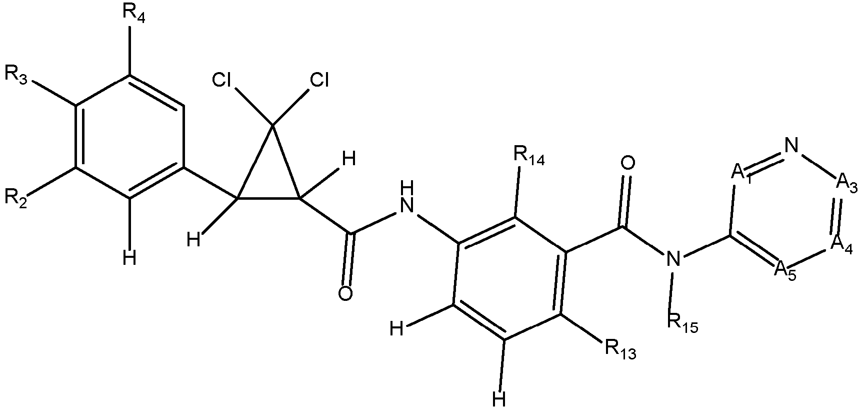
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₃ is CR₇ or N;
A₄ is CR₈ or N;
A₅ is CR₉ or N;
wherein 0 or 1 of A₁, A₃, A₄, A₅ are N;
**R⁵** is selected from the group consisting of H, F, Cl, CN, NO₂, NH(C₁-C₆)alkyl, and (C₁-C₆)alkyl ;
**R⁷** is selected from the group consisting of H, F, and Cl ;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (XVI) 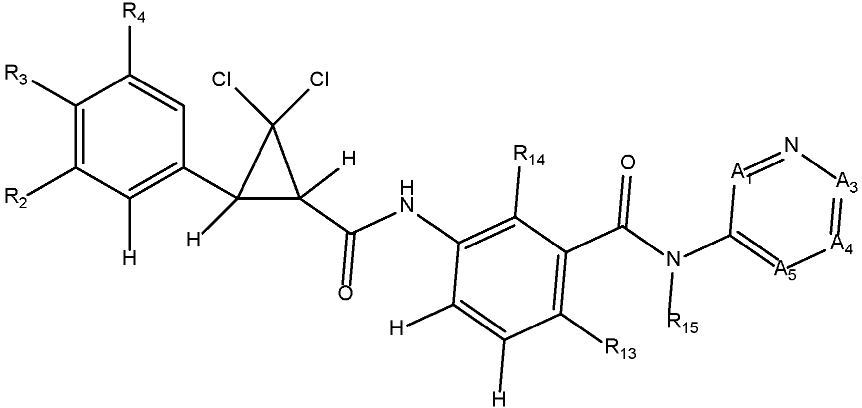
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₃ is CR₇;
A₄ is CR₈;
A₅ is CR₉;
**R⁵** is selected from the group consisting of H, F, Cl, CN and (C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
**R¹⁴** is H;
**R¹⁵** is H
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (XVII) 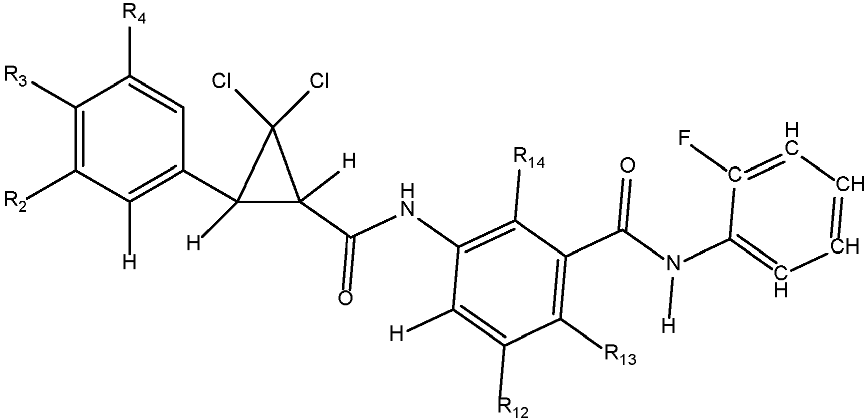
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁷, R⁸, R⁹, R¹², R¹³, and R¹⁴ are defined as elsewhere in this description.

In an embodiment of the invention and/or embodiments thereof, the invention is directed to a compound of formula (XVII) 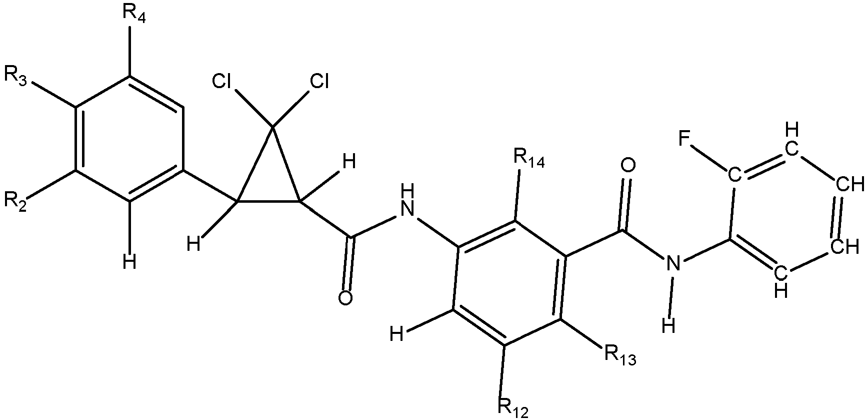
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XVII) 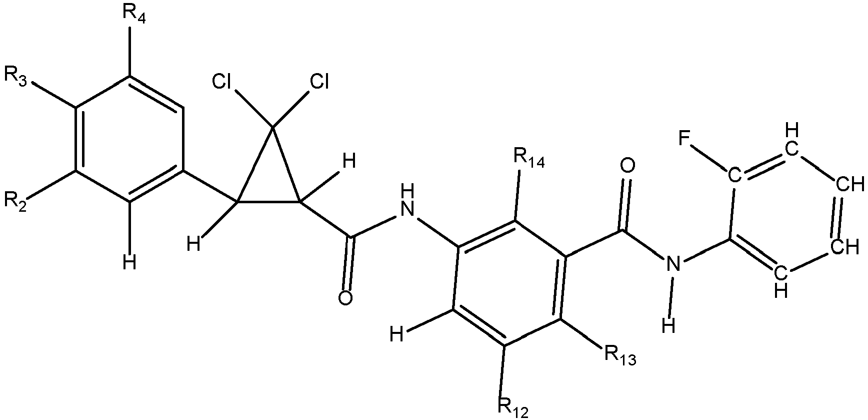
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XVII) 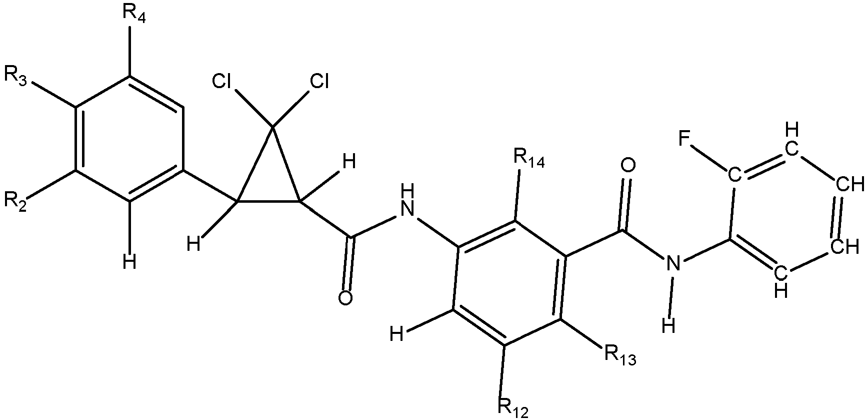
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XVII) 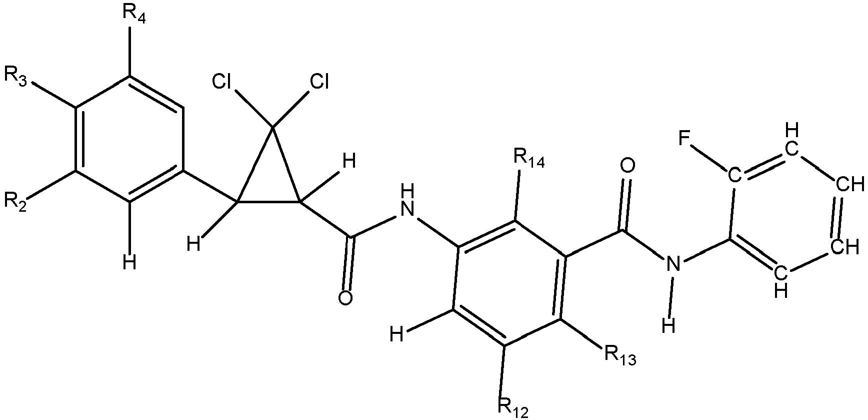
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F.

In an embodiment of the invention and/or embodiments thereof, the invention is directed to a compound of formula (XVII) 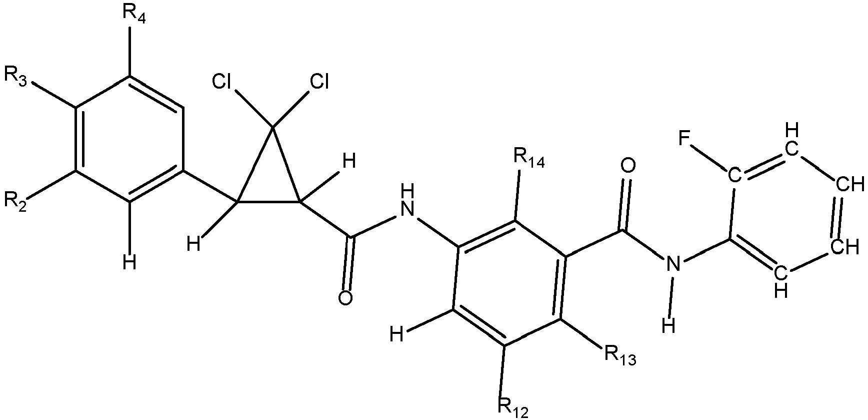
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H.

In an embodiment of the invention and/or embodiments thereof, the invention is directed to a compound of formula (XVIII) 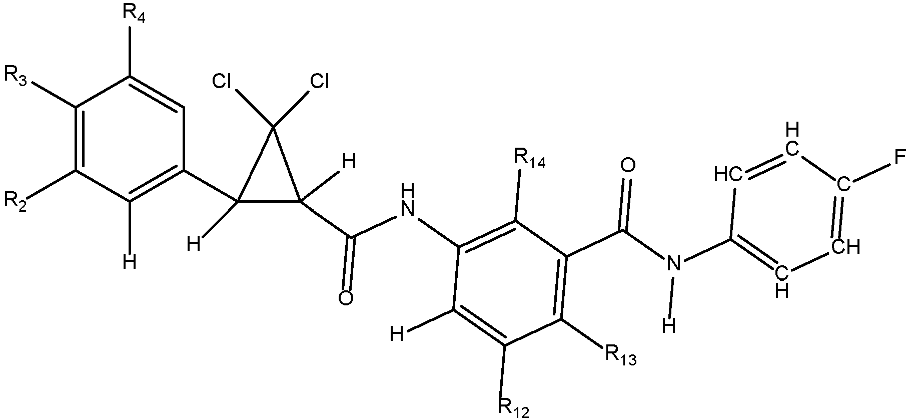
and N-oxides, veterinary acceptable acid addition salts, salt derivatives, solvates, ester derivatives, crystal polymorphs, isotopes, stereoisomers, and tautomers thereof,
wherein R², R³, R⁴, R¹², R¹³, R¹⁴, are defined as elsewhere in this description.

In an embodiment of the invention and/or embodiments thereof, the invention is directed to a compound of formula (XVIII) 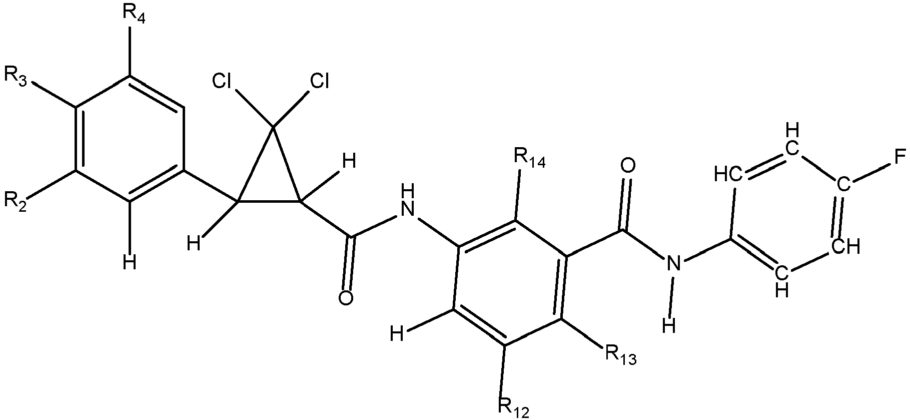
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XVIII) 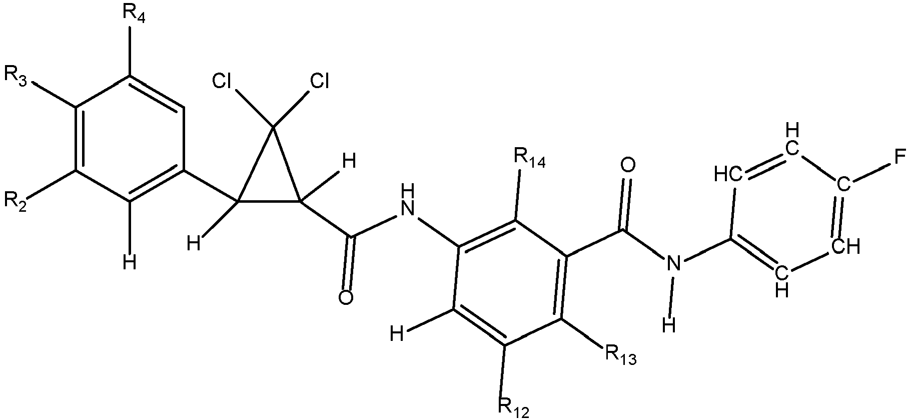
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XVIII) 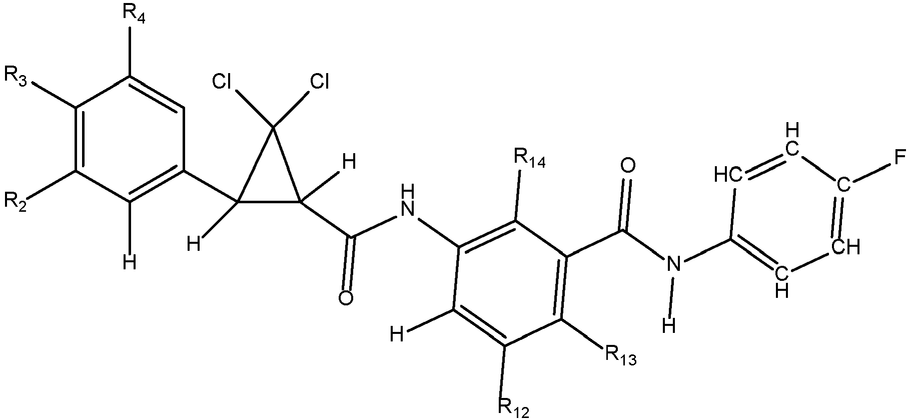
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XVIII) 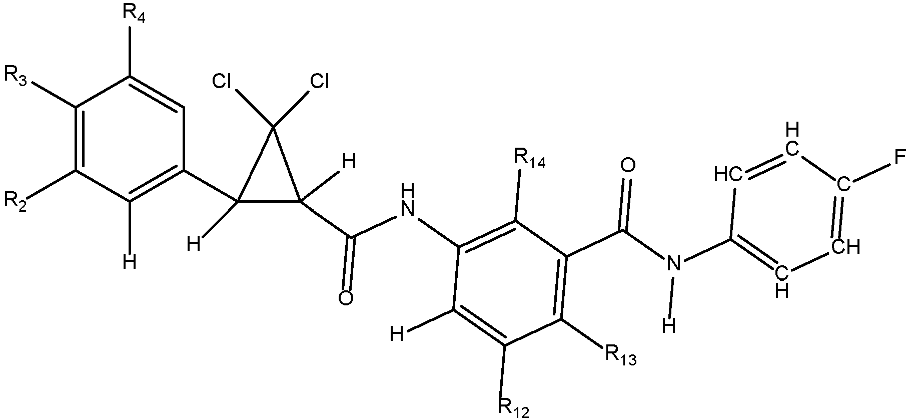
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F.

In an embodiment of the invention and/or embodiments thereof, the invention is directed to a compound of formula (XVIII) 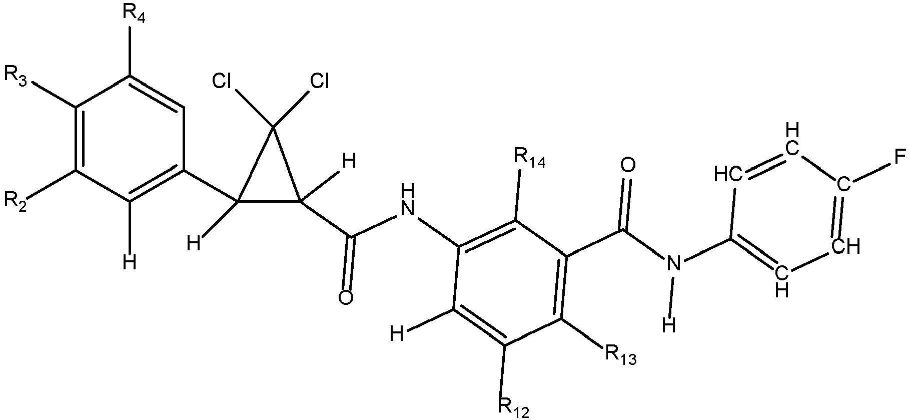
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H.

In an embodiment of the invention and/or embodiments thereof, the invention is directed to a compound of formula (XIX) 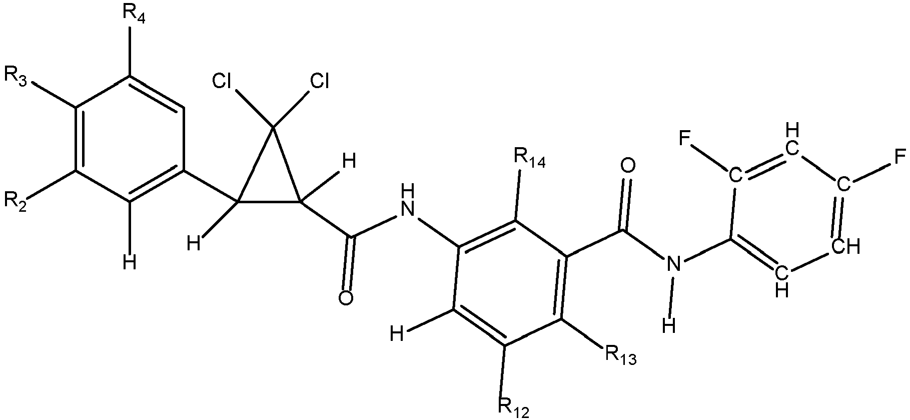
and N-oxides, veterinary acceptable acid addition salts, salt derivatives, solvates, ester derivatives, crystal polymorphs, isotopes, stereoisomers, and tautomers thereof,
wherein R², R³, R⁴, R¹², R¹³, and R¹⁴ are defined as elsewhere in this description.

In an embodiment of the invention and/or embodiments thereof, the invention is directed to a compound of formula (XIX) 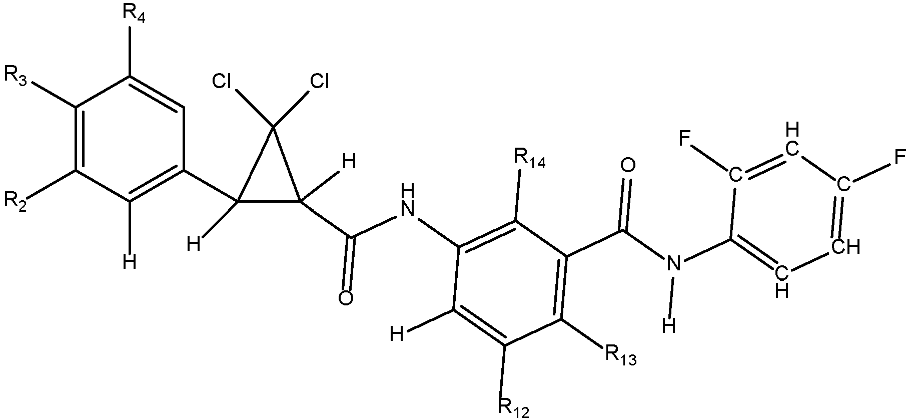
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XIX) 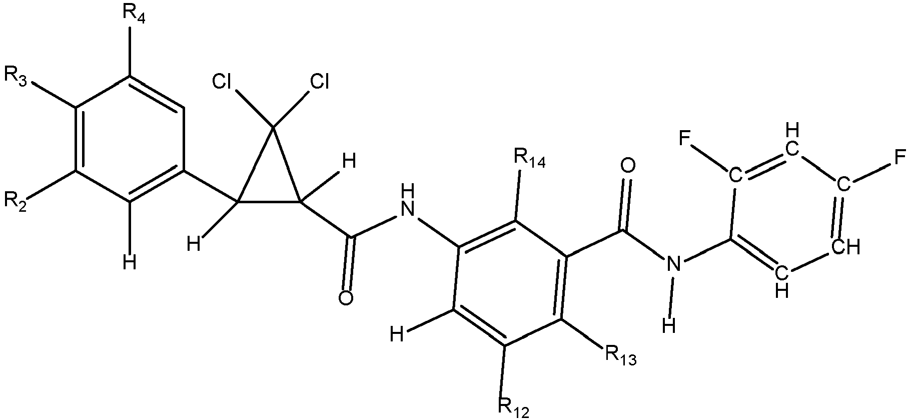
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XIX) 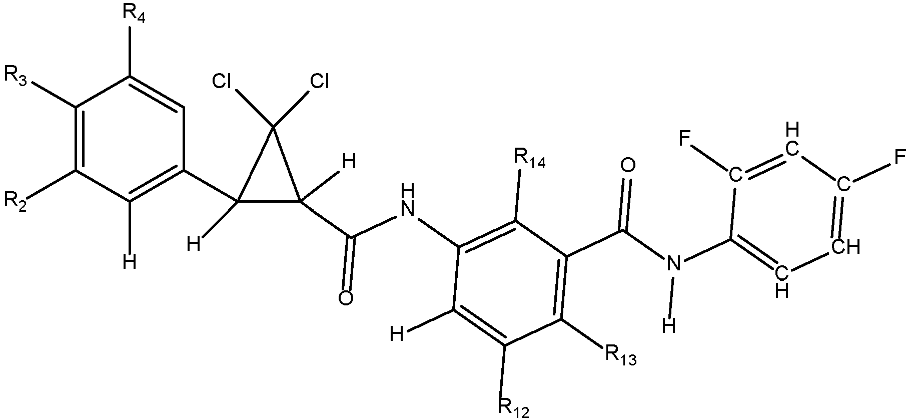
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF₃ ;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XIX) 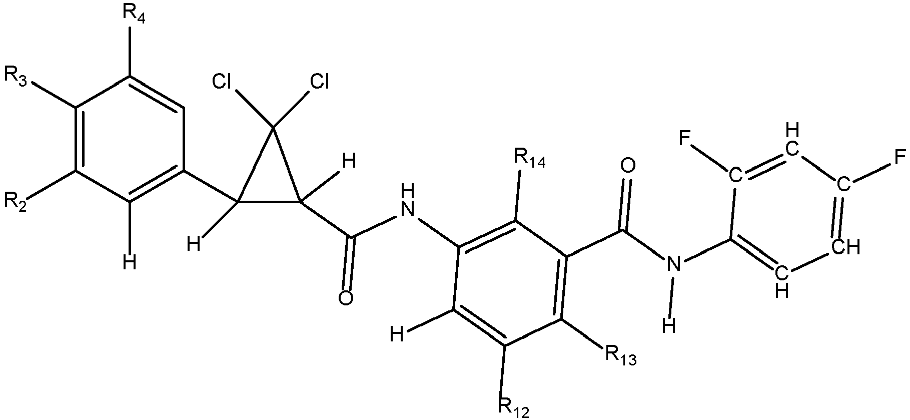
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁴** is selected from the group consisting of H and F.

In an embodiment of the invention and/or embodiments thereof, the invention is directed to a compound of formula (XIX) 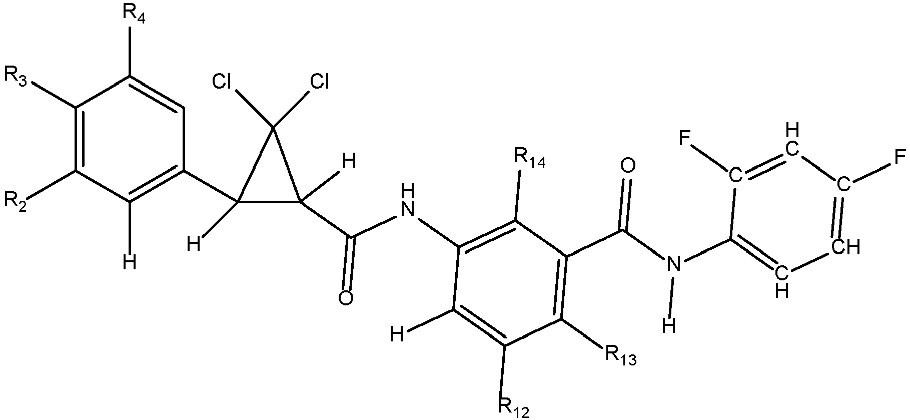
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁴** is H.

In an embodiment of the invention and/or embodiments thereof, the invention is directed to a compound of formula (XX) 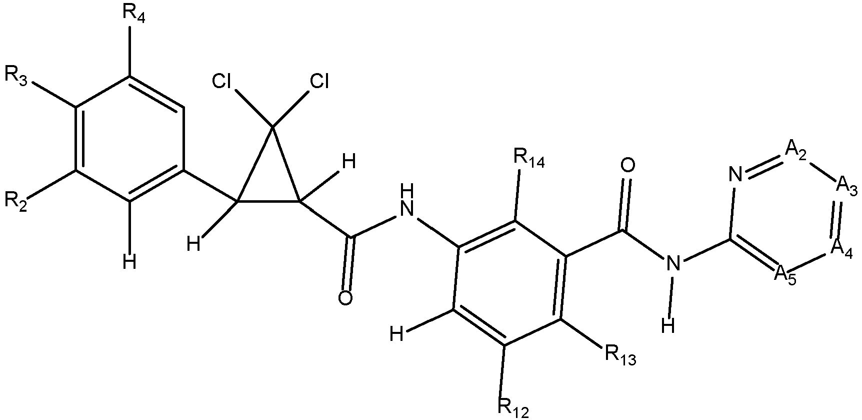
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹², R¹³, R¹⁴, A₂, A₃, A₄, and A₅ are defined as elsewhere in this description and wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XX) 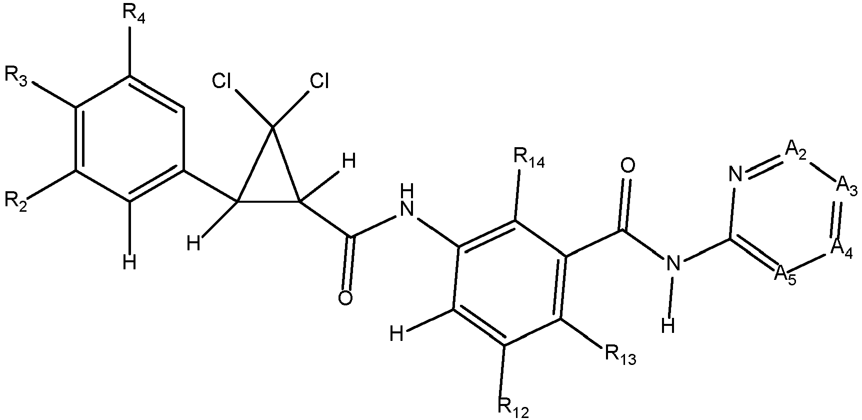
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XX) 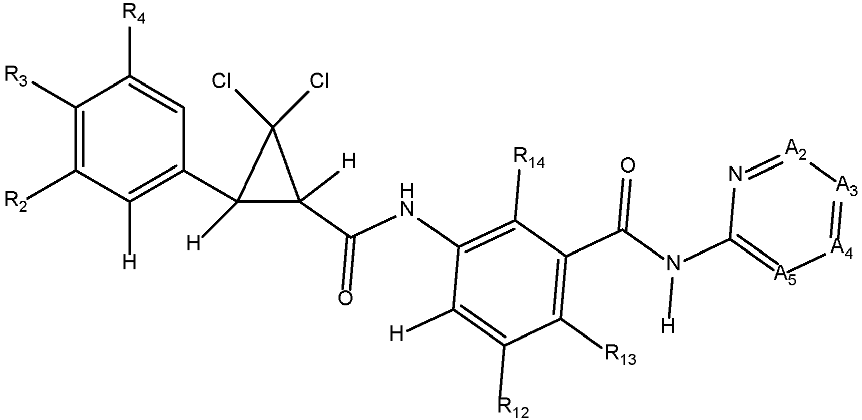
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
wherein at least one of R⁵, R⁶, R⁷, R⁸, R⁹, is a substituent selected from the group consisting of F, Cl, and Br, and I.

In an embodiment of the invention and/or embodiments thereof, the invention is directed to a compound of formula (XX) 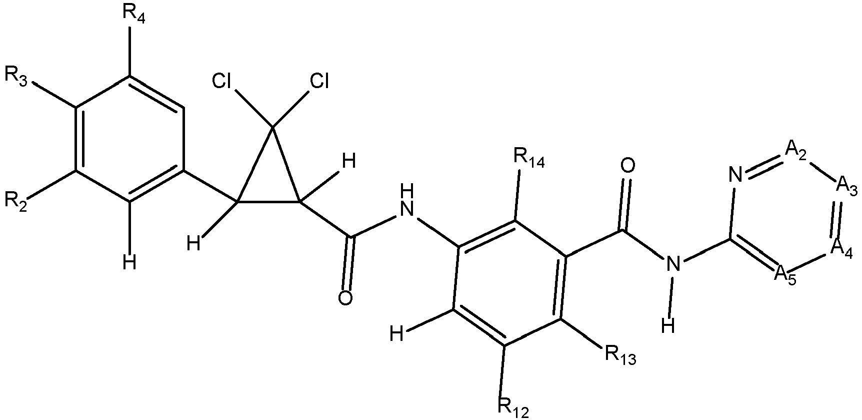
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₃ is CR₇ or N;
A₄ is CR₈ or N;
A₅ is CR₉ or N;
wherein no more than 2 of A₂, A₃, A₄, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by R**⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
wherein at least one of R⁵, R⁶, R⁷, R⁸, R⁹, is a substituent selected from the group consisting of F, Cl, Br, and I.

In an embodiment of the invention and/or embodiments thereof, the invention is directed to a compound of formula (XX) 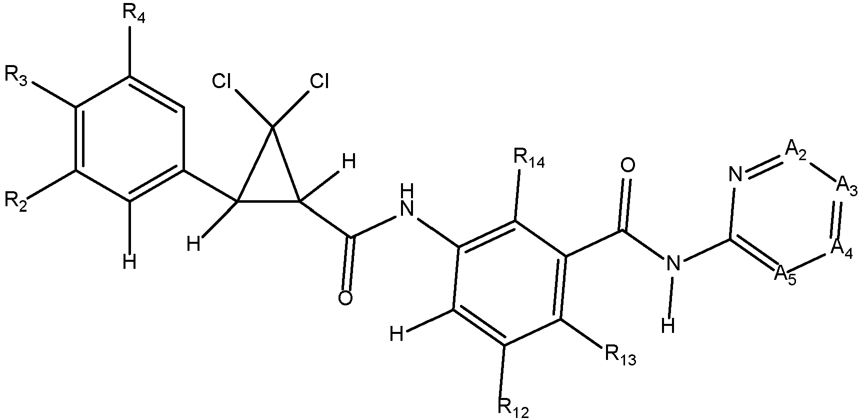
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₂ is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (XX) 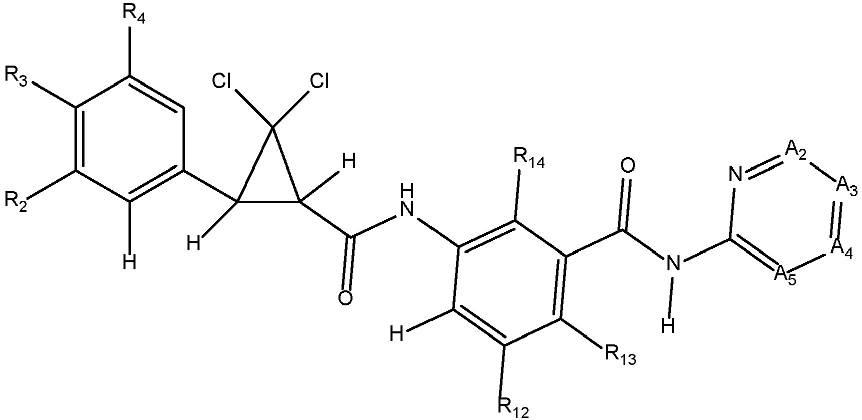
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A2 is CR₆ or N;
A₃ is CR₇;
A₄ is CR₈;
A₅ is CR₉;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (XXI) 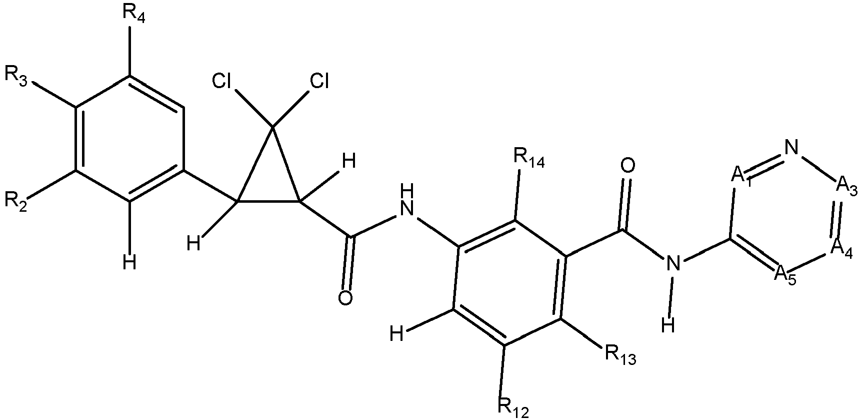
**and N-oxides,** veterinary acceptable acid addition salts, salt derivatives, solvates, ester derivatives, crystal polymorphs, isotopes, stereoisomers, and tautomers thereof,
wherein R², R³, R⁴, R⁵, R⁷, R⁸, R⁹, R¹², R¹³, R¹⁴, A₁, A₃, A₄, and A₅ are defined as elsewhere in this description and wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXI) 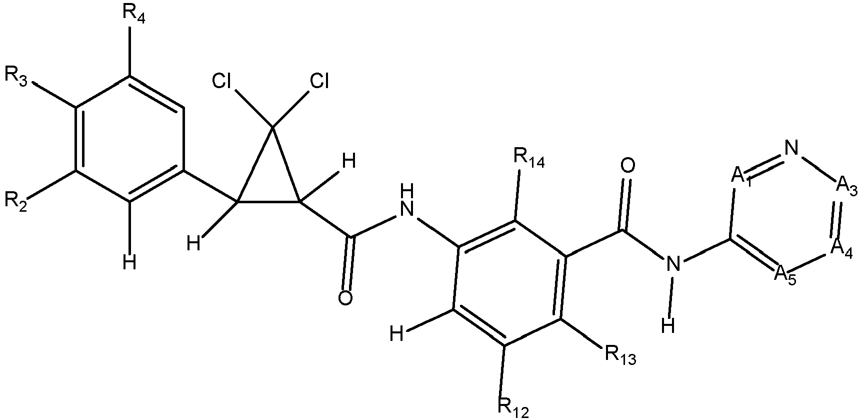
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₃ is CR₇, N=O, or N;
A₄ is CR₈, N=O or N;
A₅ is CR₉, N=O or N ;
wherein no more than 2 of A₁, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXI) 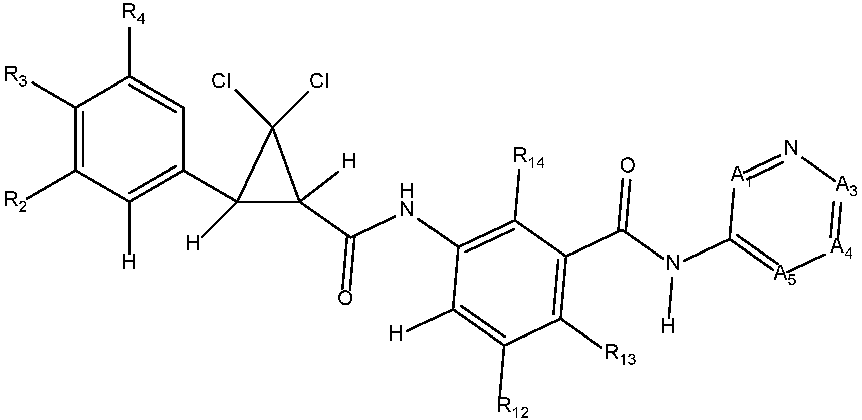
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₃ is CR₇, N=O, or N;
A₄ is CR₈, N=O or N;
A₅ is CR₉, N=O or N;
wherein no more than 2 of A₁, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
wherein at least one of R⁵, R⁷, R⁸, R⁹, is a substituent selected from the group consisting of F, Cl, and Br, and I.

In an embodiment of the invention and/or embodiments thereof, the invention is directed to a compound of formula (XXI) 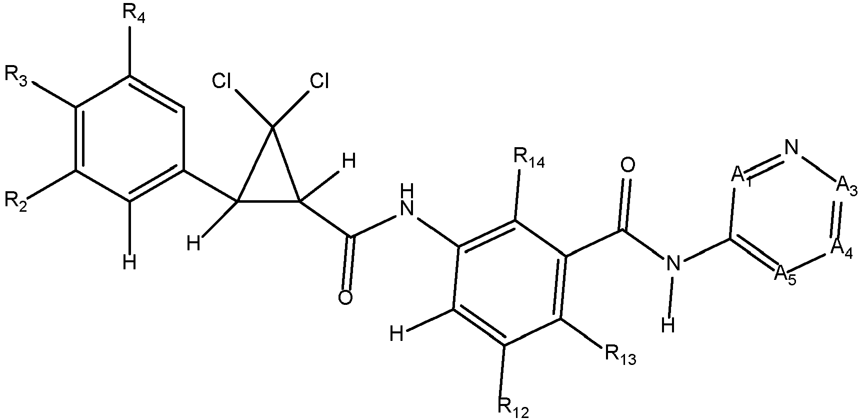
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₃ is CR₇ or N;
A₄ is CR₈ or N;
A₅ is CR₉ or N;
wherein no more than 2 of A₁, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXI) 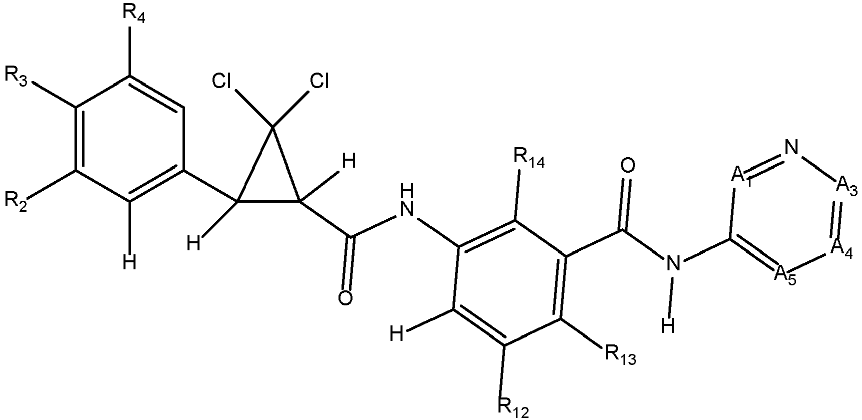
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₃ is CR₇ or N;
A₄ is CR₈ or N;
A₅ is CR₉ or N;
wherein 0 or 1 of A₁, A₃, A₄, A₅ are N;
**R⁵** is selected from the group consisting of H, F, Cl, CN, NO₂, NH(C₁-C₆)alkyl, and (C₁-C₆)alkyl ;
**R⁷** is selected from the group consisting of H, F, and Cl ;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹²** is H or F ;
**R¹³** is Cl or F ;
**R¹⁴** is selected from the group consisting of H and F ;
wherein at least one of R⁵, R⁷, R⁸, R⁹, is a substituent selected from the group consisting of F, and Cl.

In an embodiment of the invention and/or embodiments thereof, the invention is directed to a compound of formula (XXI) 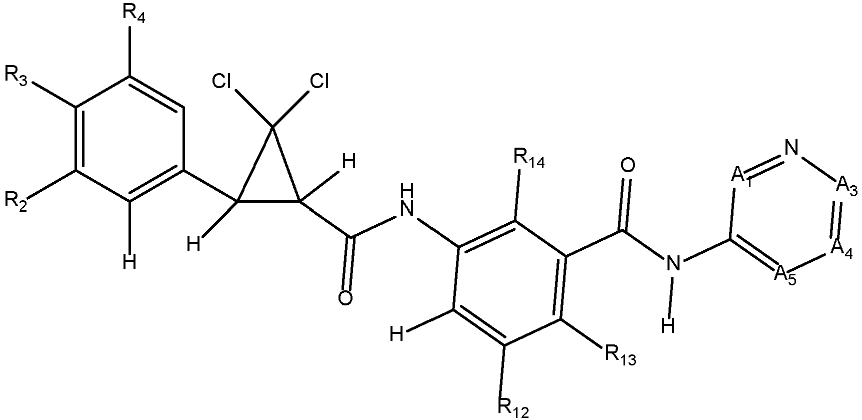
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
A₁ is CR₅ or N;
A₃ is CR₇;
A₄ is CR₈;
A₅ is CR₉;
**R⁵** is selected from the group consisting of H, F, Cl, CN and (C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
wherein at least one of R⁵, R⁶, R⁷, R⁸, R⁹, is a substituent selected from the group consisting of F, and Cl.

In an embodiment of the invention and/or embodiments thereof, the invention is directed to a compound of formula (XXII) 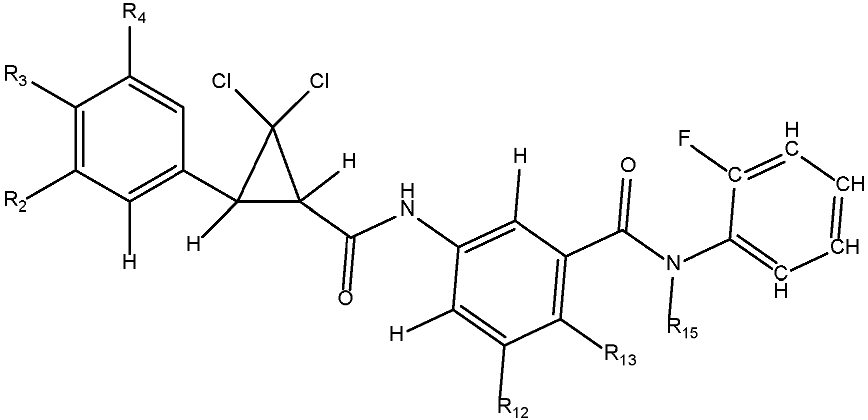
and N-oxides, veterinary acceptable acid addition salts, salt derivatives, solvates, ester derivatives, crystal polymorphs, isotopes, stereoisomers, and tautomers thereof,
wherein R², R³, R⁴, R¹², R¹³, and R¹⁵ are defined as elsewhere in this description.

In an embodiment of the invention and/or embodiments thereof, the invention is directed to a compound of formula (XXII) 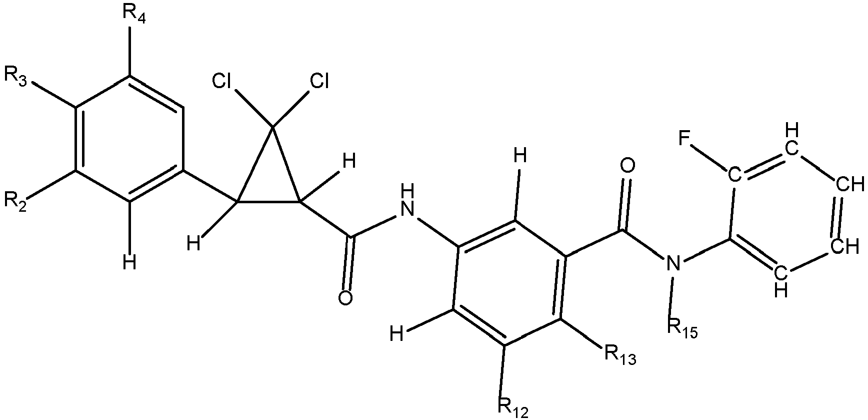
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXII) 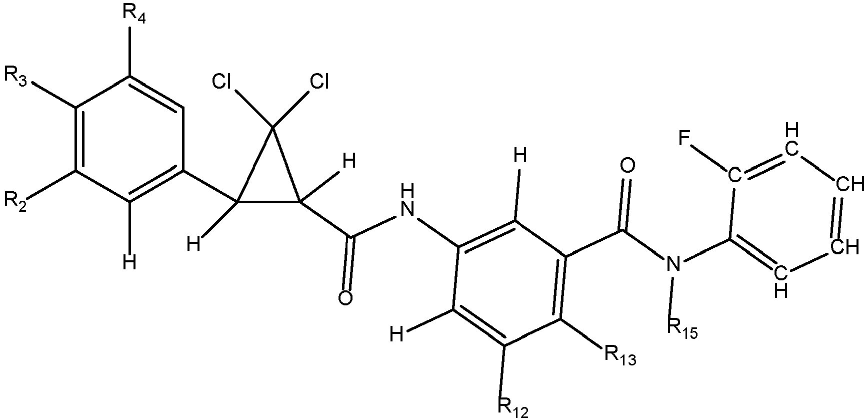
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF_{3 ;}
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXII) 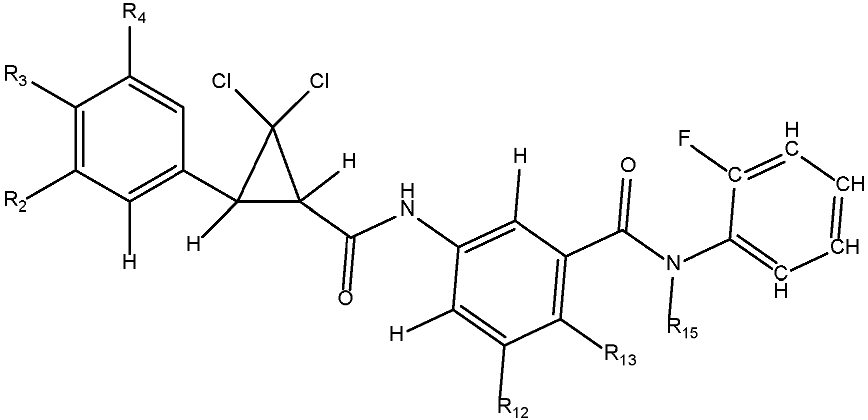
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF_{3 ;}
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXII) 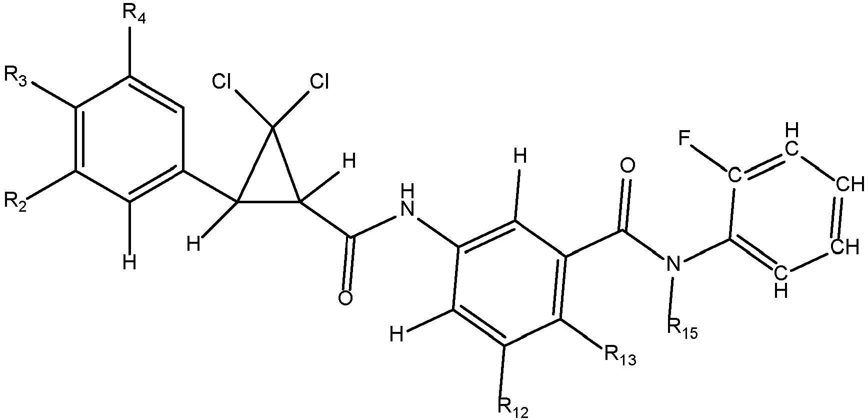
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XXII) 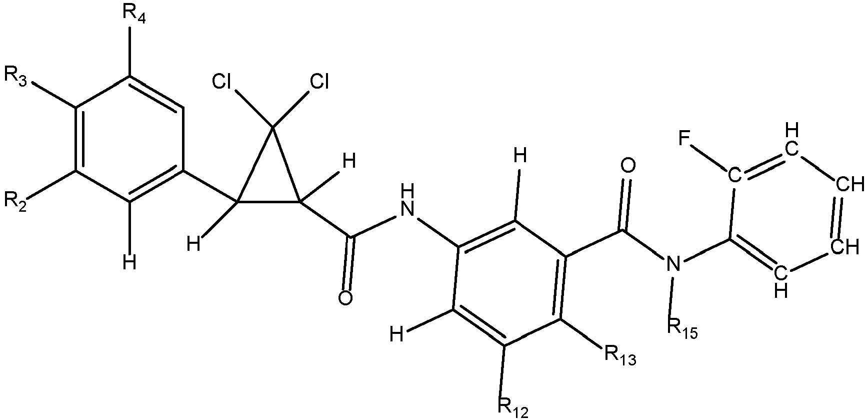
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R¹²** is H ;
**R¹³** is Cl;
**R¹⁵** is H.

In an embodiment of the invention and/or embodiments thereof, the invention is directed to a compound of formula (XXIII) 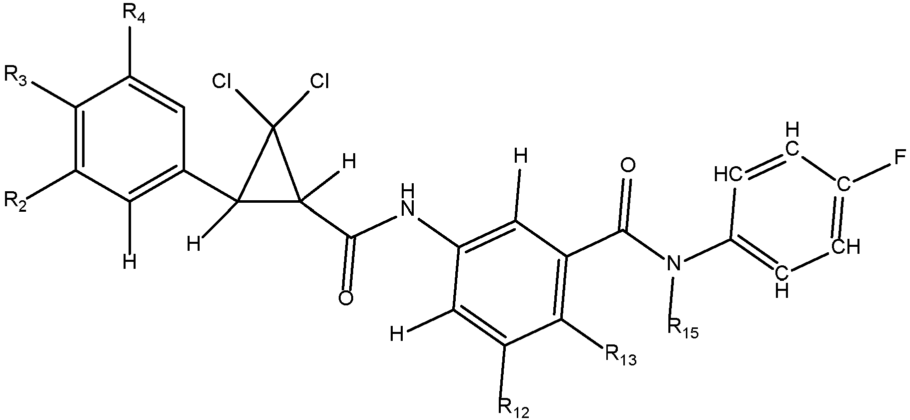
and N-oxides, veterinary acceptable acid addition salts, salt derivatives, solvates, ester derivatives, crystal polymorphs, isotopes, stereoisomers, and tautomers thereof,
wherein R², R³, R⁴, R¹², R¹³, and R¹⁵ are defined as elsewhere in this description.

In an embodiment of the invention and/or embodiments thereof, the invention is directed to a compound of formula (XXIII) 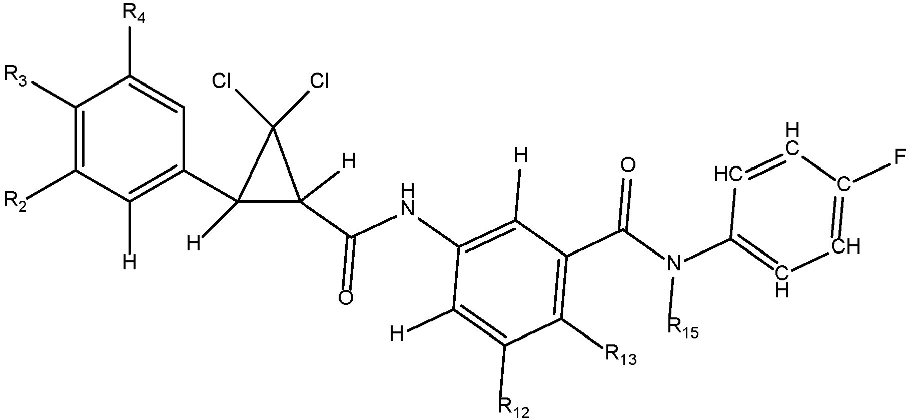
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXIII) 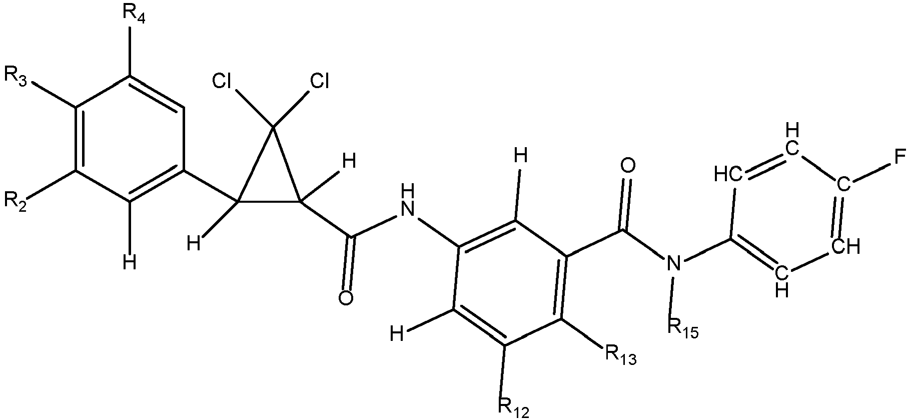
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF_{3 ;}
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXIII) 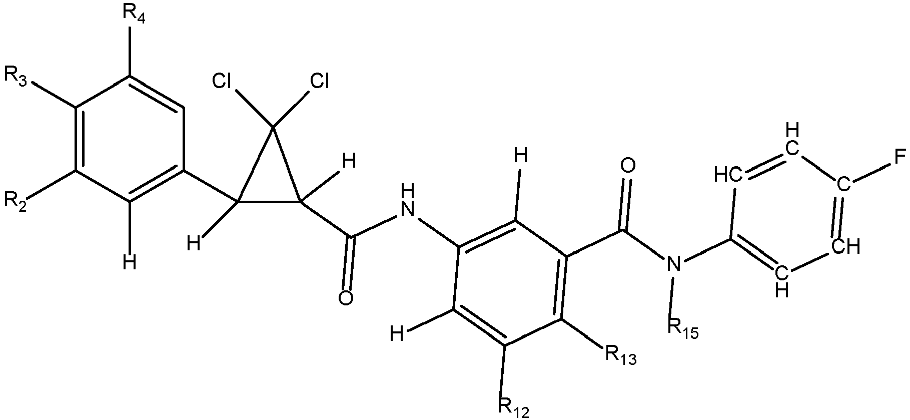
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF_{3 ;}
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXIII) 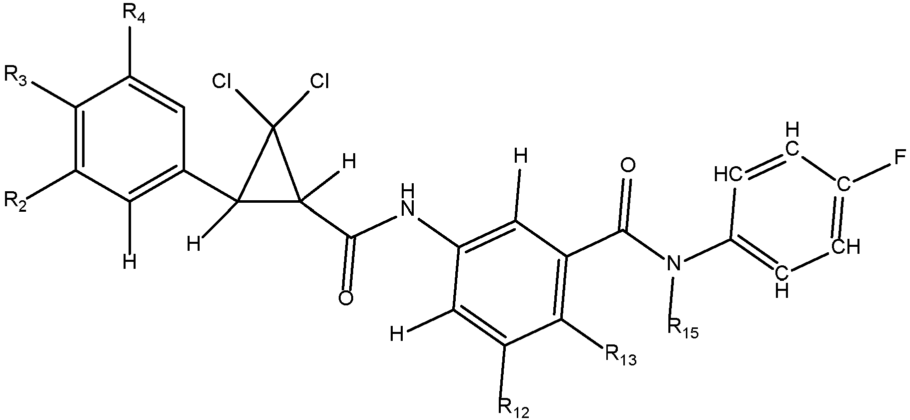
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XXIII) 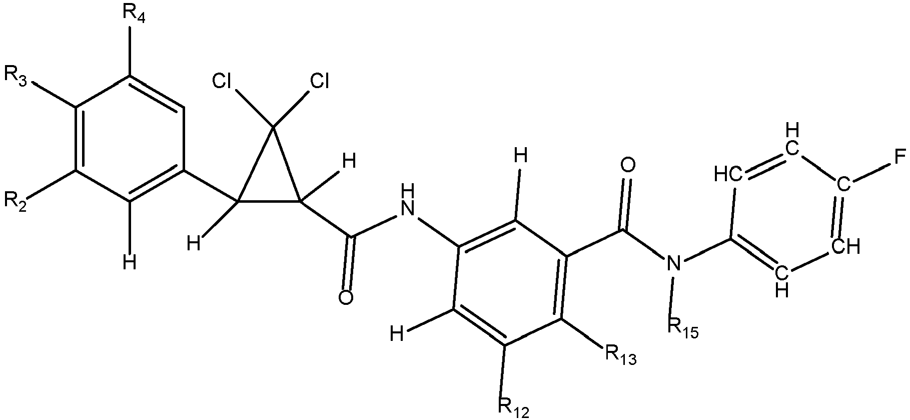
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁵** is H.

In an embodiment of the invention and/or embodiments thereof, the invention is directed to a compound of formula (XXIV) 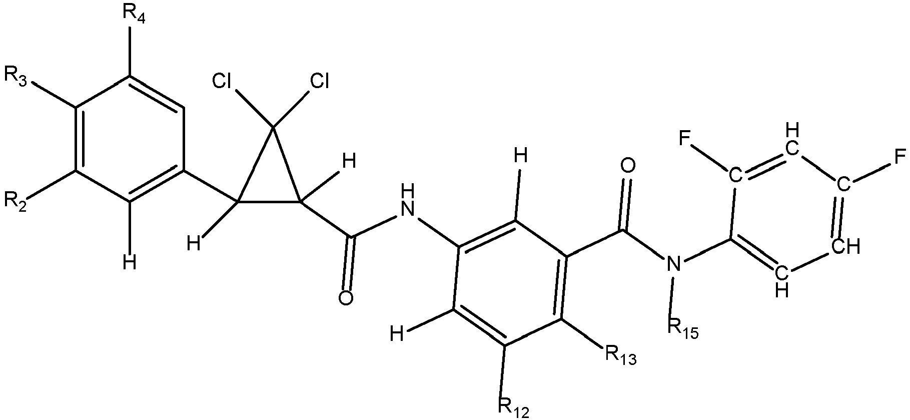
and N-oxides, veterinary acceptable acid addition salts, salt derivatives, solvates, ester derivatives, crystal polymorphs, isotopes, stereoisomers, and tautomers thereof,
wherein R², R³, R⁴, R¹², R¹³, and R¹⁵ are defined as elsewhere in this description.

In an embodiment of the invention and/or embodiments thereof, the invention is directed to a compound of formula (XXIV) 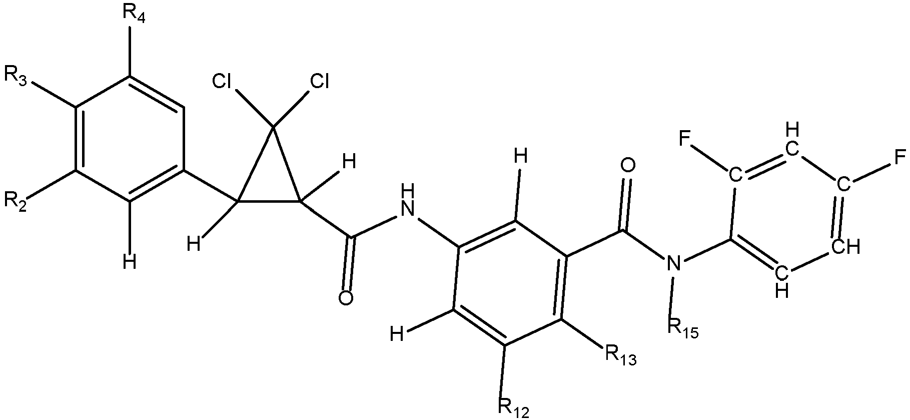
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXIV) 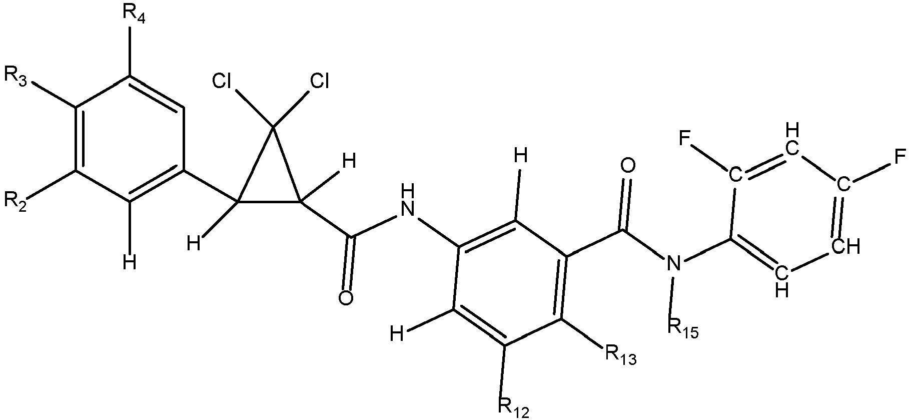
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXIV) 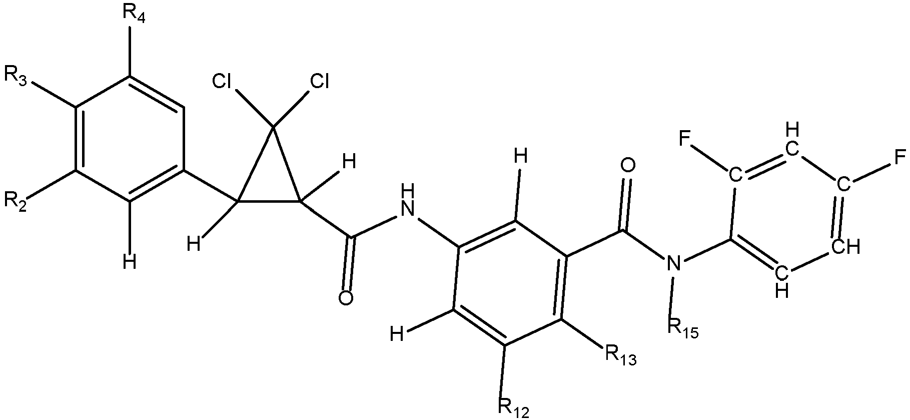
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXIV) 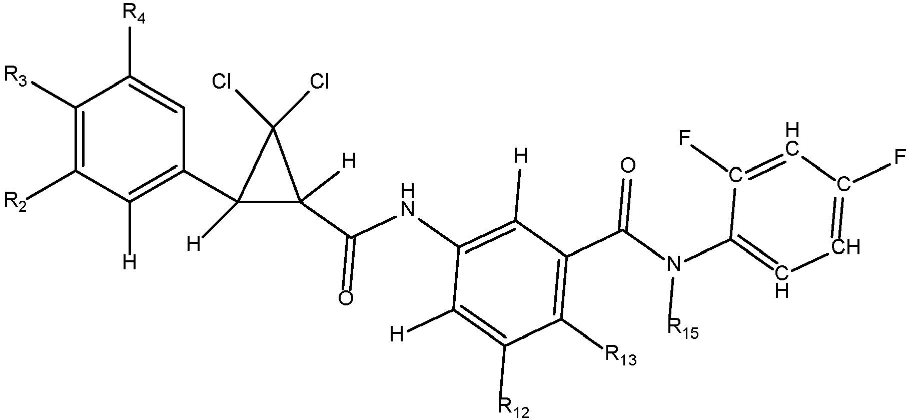
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XXIV) 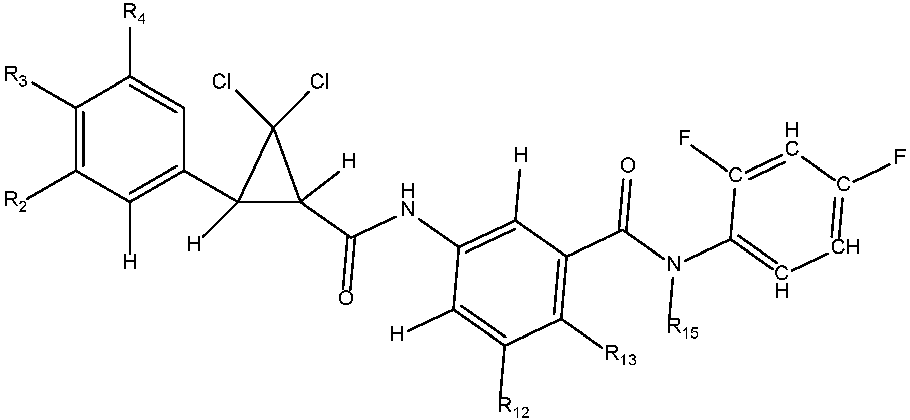
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹²** is H ;
**R¹³** is Cl;
**R¹⁵** is H.

In an embodiment of the invention and/or embodiments thereof, the invention is directed to a compound of formula (XXV) 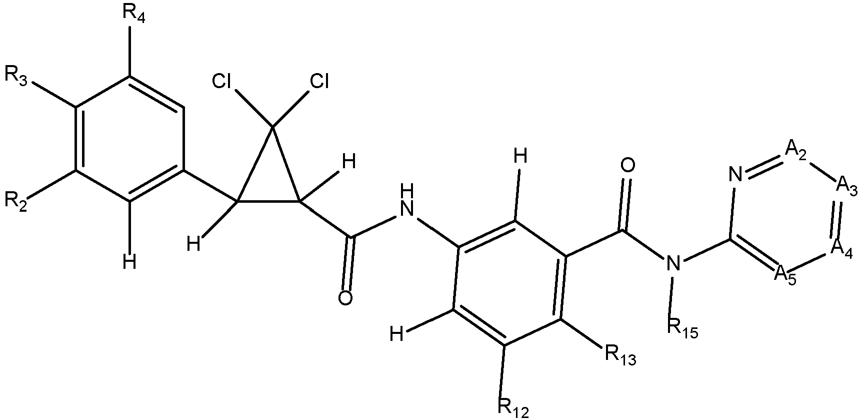
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹², R¹³, R¹⁵, A₂, A₃, A₄, and A₅ are defined as elsewhere in this description and wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXV) 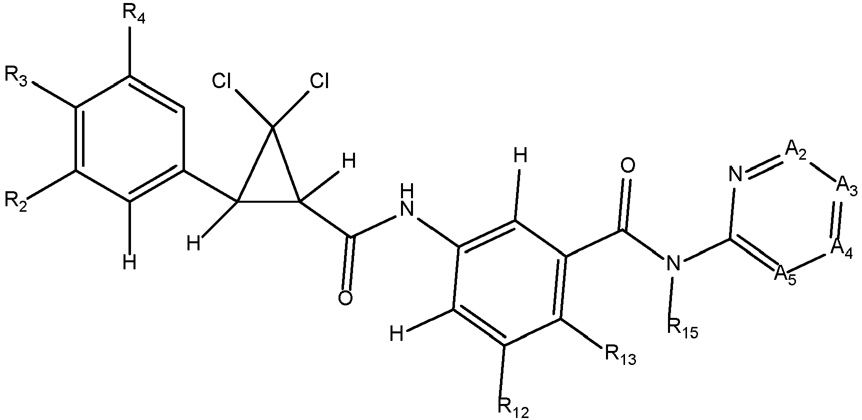
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R**⁷ and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXV) 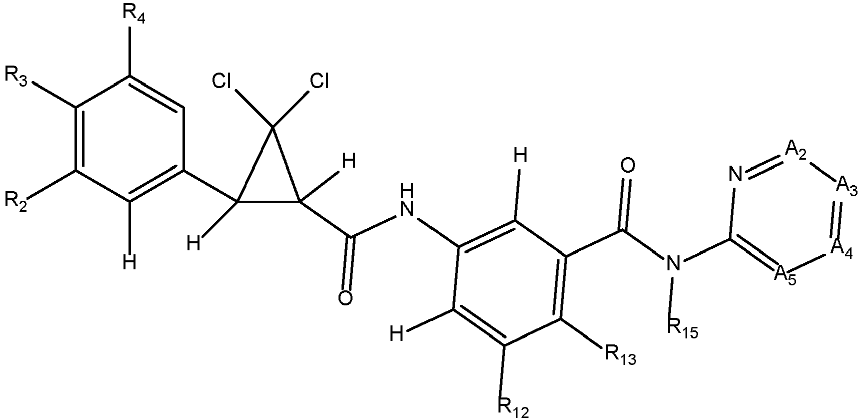
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((Ci-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, and Br, and I.

In an embodiment of the invention and/or embodiments thereof, the invention is directed to a compound of formula (XXV) 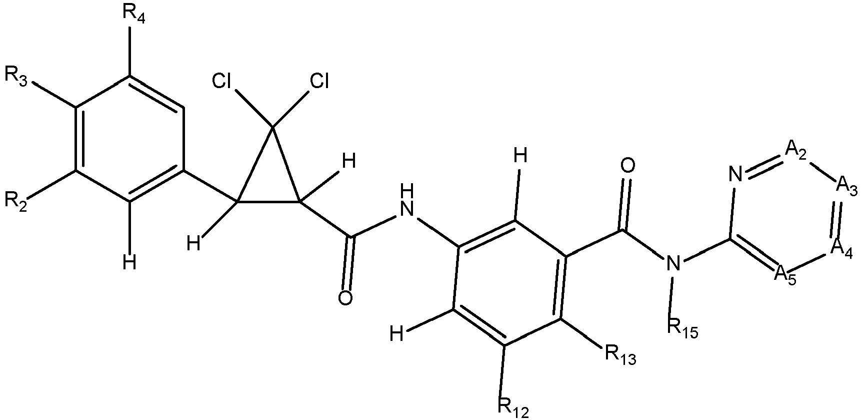
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₃ is CR₇ or N;
A₄ is CR₈ or N;
A₅ is CR₉ or N;
wherein no more than 2 of A₂, A₃, A₄, A₅ are N;
R⁶ is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R**⁷ and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXV) 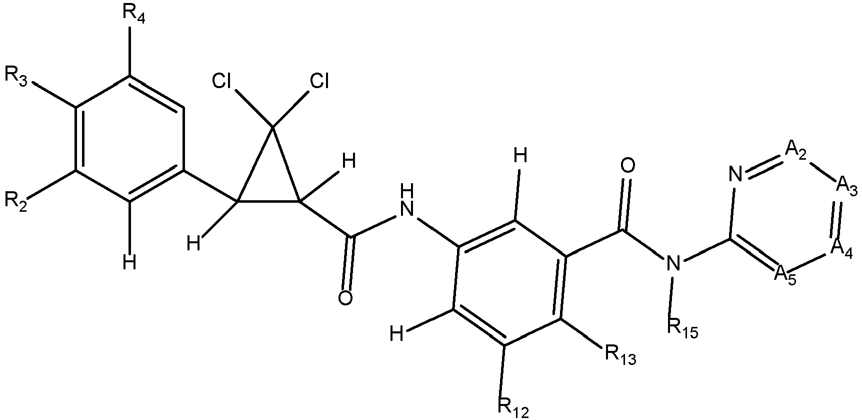
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₂ is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃;
wherein at least one of R⁶, R⁷, R⁸, R⁹, is a substituent selected from the group consisting of F, and Cl.

In an embodiment of the invention and/or embodiments thereof, the invention is directed to a compound of formula (XXV) 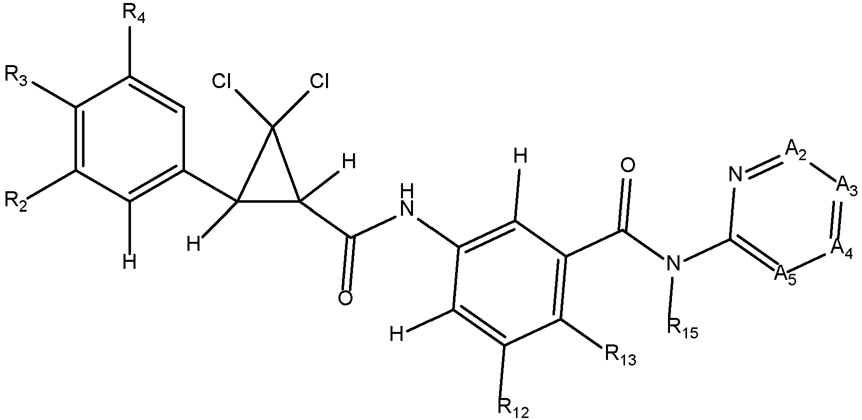
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A2 is CR₆ or N;
A₃ is CR₇;
A₄ is CR₈;
A₅ is CR₉;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁵** is H
wherein at least one of R⁶, R⁷, R⁸, R⁹, is a substituent selected from the group consisting of F, and Cl.

In an embodiment of the invention and/or embodiments thereof, the invention is directed to a compound of formula (XXVI) 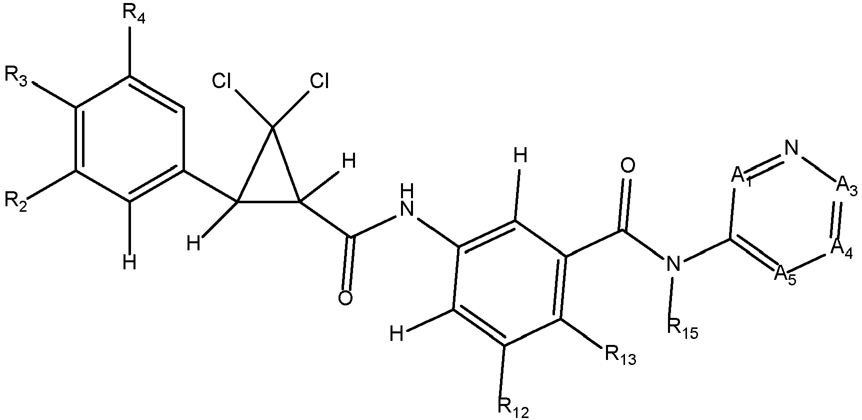
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁷, R⁸, R⁹, R¹², R¹³, R¹⁴, R¹⁵, A₁, A₃, A₄, and A₅ are defined as elsewhere in this description and wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXVI) 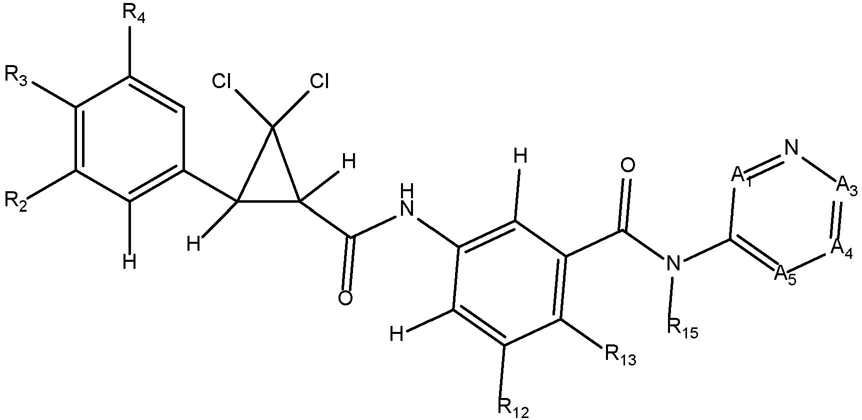
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₃ is CR₇, N=O, or N;
A₄ is CR₈, N=O or N;
A₅ is CR₉, N=O or N ;
wherein no more than 2 of A₁, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXVI) 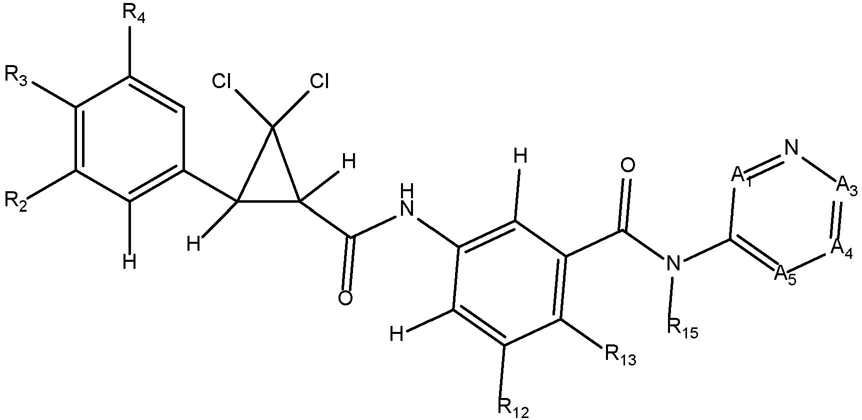
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₃ is CR₇, N=O, or N;
A₄ is CR₈, N=O or N;
A₅ is CR₉, N=O or N;
wherein no more than 2 of A₁, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃ ;
wherein at least one of R⁵, R⁷, R⁸, R⁹, is a substituent selected from the group consisting of F, Cl, and Br, and I.

In an embodiment of the invention and/or embodiments thereof, the invention is directed to a compound of formula (XXVI) 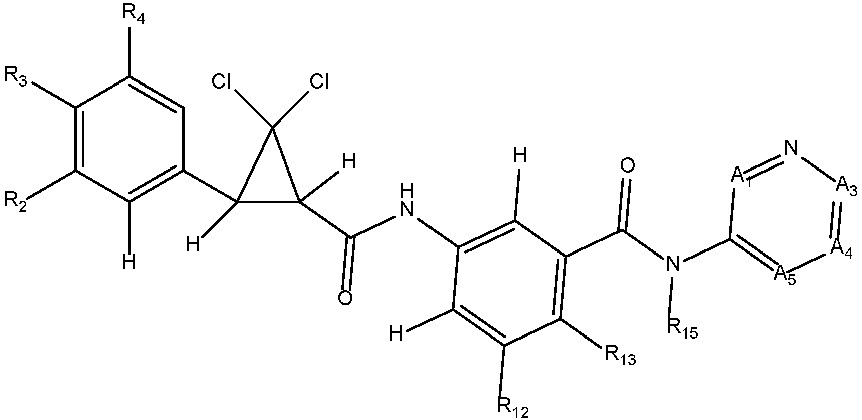
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₃ is CR₇ or N;
A₄ is CR₈ or N;
A₅ is CR₉ or N;
wherein no more than 2 of A₁, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
**R⁸** is selected from the group consisting of H, F, Cl, NH(C₁-C₆)alkyl, and (C₁-C₆)alkyl ;
or **R**⁷ and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by R⁷ and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃ ;
**R¹³** is selected from the group consisting of H, F, Cl, CH₃ and CF₃ ;
**R¹⁵** is selected from the group consisting of H, CH₃, and CF₃ ;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXVI) 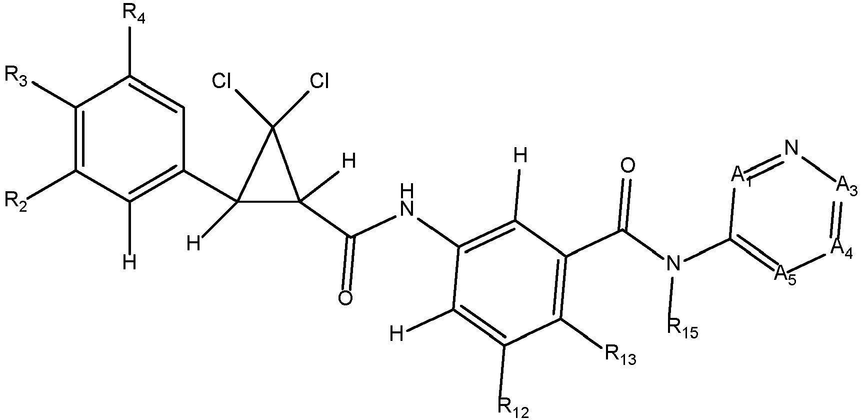
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₁ is CR₅ or N;
A₃ is CR₇ or N;
A4 is CR₈ or N;
A₅ is CR₉ or N;
wherein 0 or 1 of A₁, A₃, A₄, A₅ are N;
**R⁵** is selected from the group consisting of H, F, Cl, CN, NO₂, NH(C₁-C₆)alkyl, and (C₁-C₆)alkyl ;
**R⁷** is selected from the group consisting of H, F, and Cl ;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹²** is H or F ;
R¹³ is Cl or F ;
**R¹⁵** is selected from the group consisting of H, and CH₃;
wherein at least one of R⁵, R⁷, R⁸, R⁹, is a substituent selected from the group consisting of F, and Cl.

In an embodiment of the invention and/or embodiments thereof, the invention is directed to a compound of formula (XXVI) 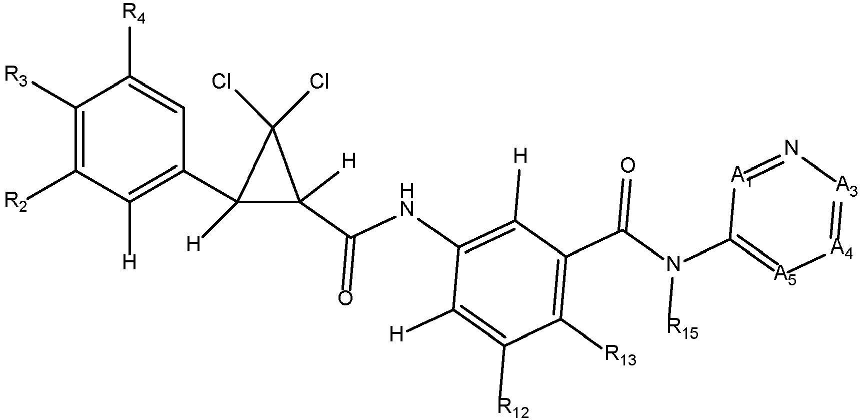
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₃ is CR₇;
A₄ is CR₈;
A₅ is CR₉;
**R⁵** is selected from the group consisting of H, F, Cl, CN and (C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁵** is H
wherein at least one of R⁵, R⁷, R⁸, R⁹, is a substituent selected from the group consisting of F, and Cl.

In an embodiment of the invention and/or embodiments thereof, the invention is directed to a compound of formula (XXVII) 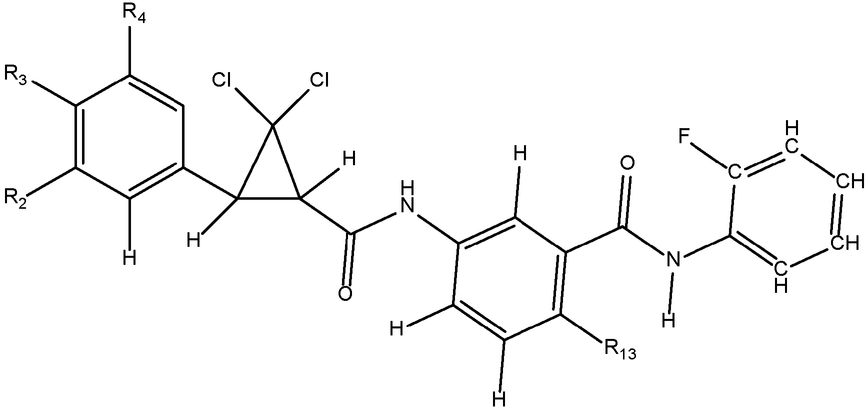
and N-oxides, veterinary acceptable acid addition salts, salt derivatives, solvates, ester derivatives, crystal polymorphs, isotopes, stereoisomers, and tautomers thereof,
wherein R², R³, R⁴, and R¹³ are defined as elsewhere in this description.

In an embodiment of the invention and/or embodiments thereof, the invention is directed to a compound of formula (XXVII) 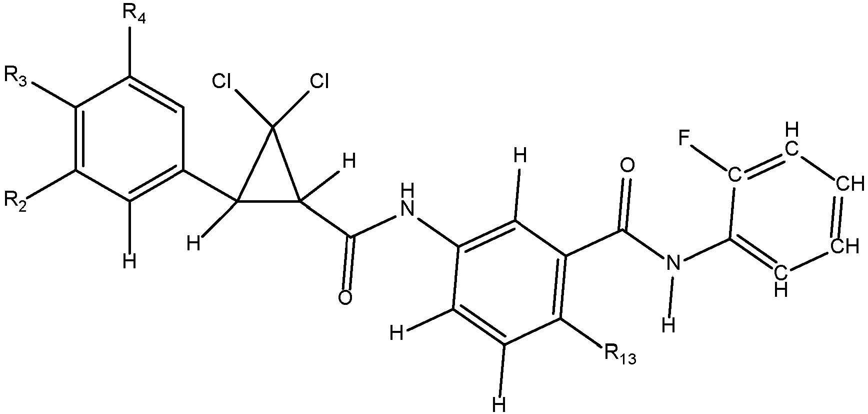
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XXVII) 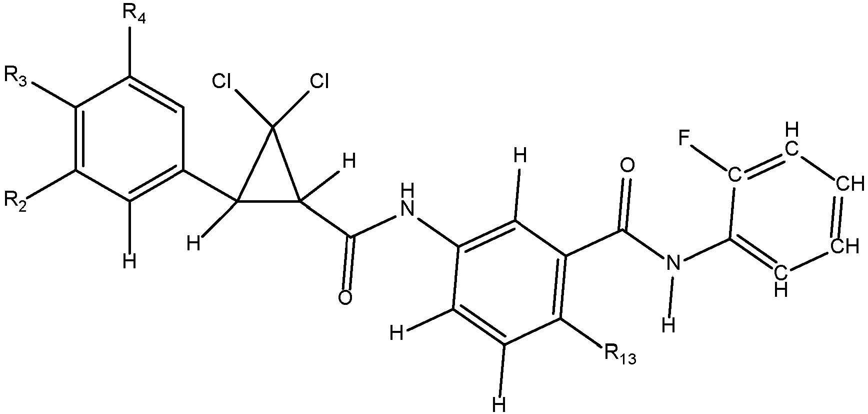
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃.

In an embodiment of the invention and/or embodiments thereof, the invention is directed to a compound of formula (XXVII) 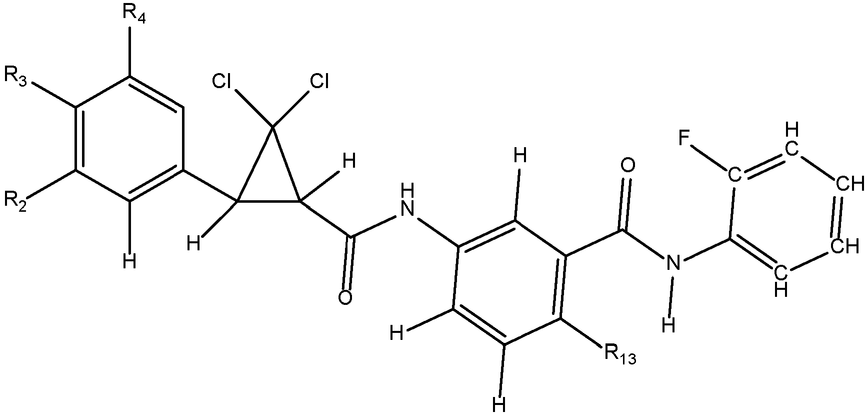
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃.

In an embodiment of the invention and/or embodiments thereof, the invention is directed to a compound of formula (XXVII) 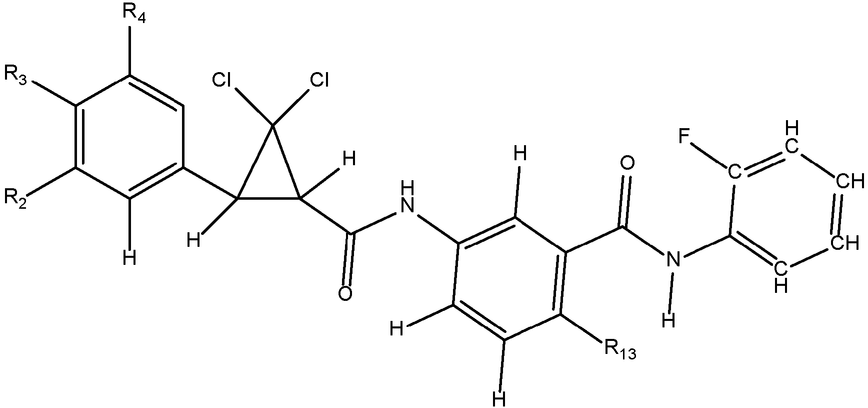
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹³** is Cl or F.

In an embodiment of the invention and/or embodiments thereof, the invention is directed to a compound of formula (XXVII) 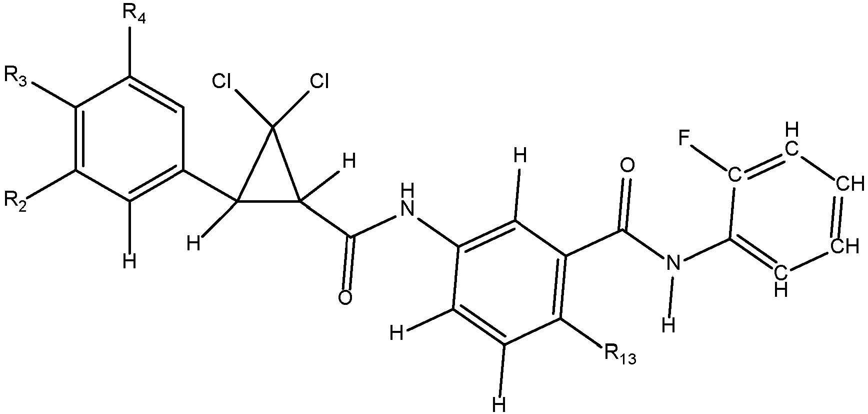
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl.

In an embodiment of the invention and/or embodiments thereof, the invention is directed to a compound of formula (XXVIII) 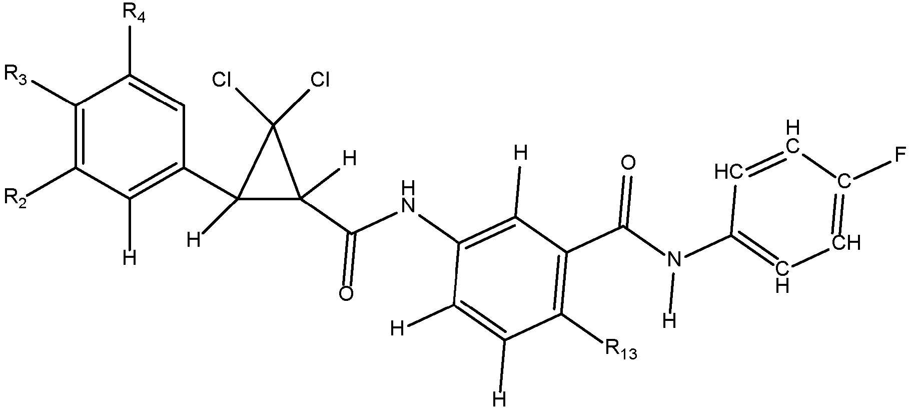
and N-oxides, veterinary acceptable acid addition salts, salt derivatives, solvates, ester derivatives, crystal polymorphs, isotopes, stereoisomers, and tautomers thereof,
wherein R², R³, R⁴, and R¹³ are defined as elsewhere in this description.

In an embodiment of the invention and/or embodiments thereof, the invention is directed to a compound of formula (XXVIII) 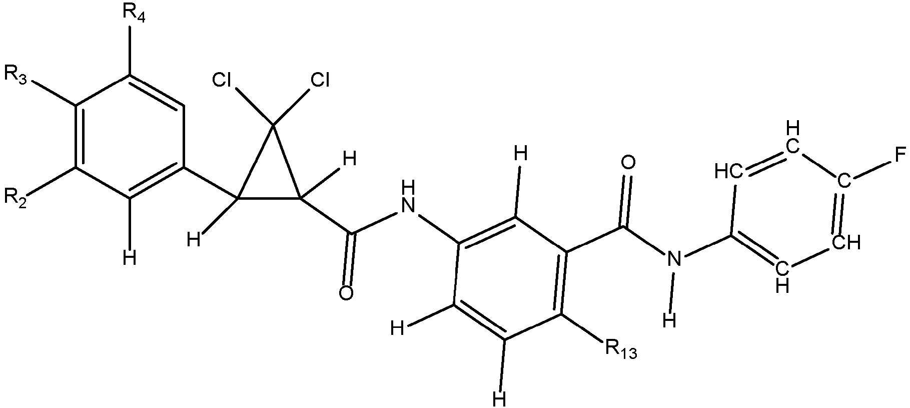
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XXVIII) 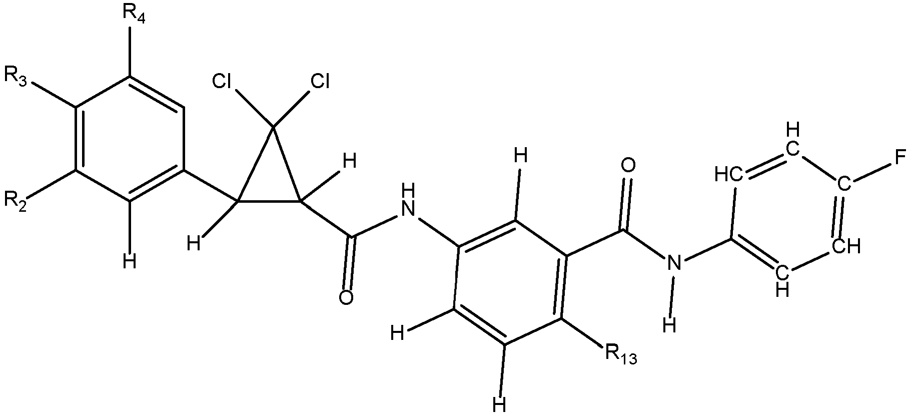
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃.

In an embodiment of the invention and/or embodiments thereof, the invention is directed to a compound of formula (XXVIII) 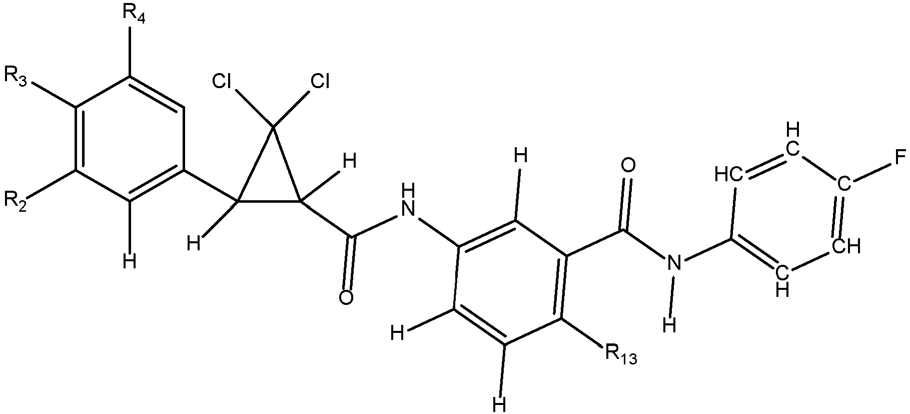
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃.

In an embodiment of the invention and/or embodiments thereof, the invention is directed to a compound of formula (XXVIII) 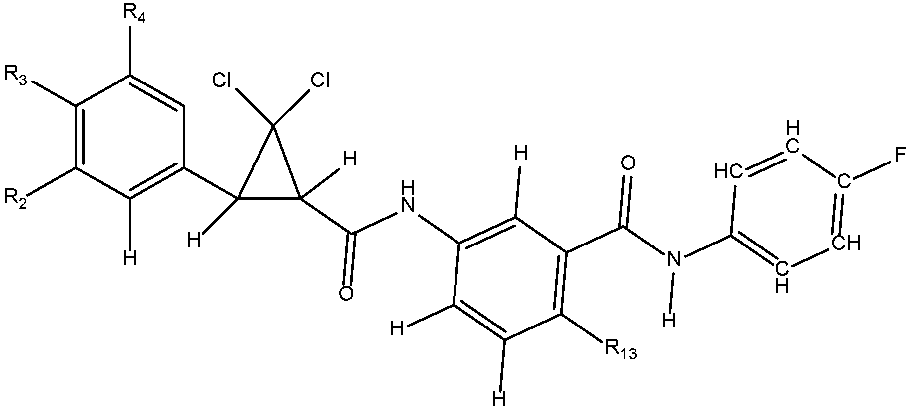
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹³** is Cl or F.

In an embodiment of the invention and/or embodiments thereof, the invention is directed to a compound of formula (XXVIII) 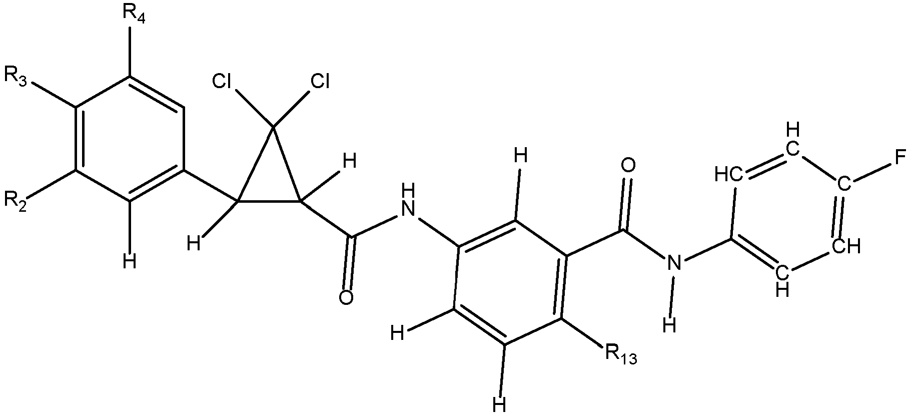
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl.

In an embodiment of the invention and/or embodiments thereof, the invention is directed to a compound of formula (XXIX) 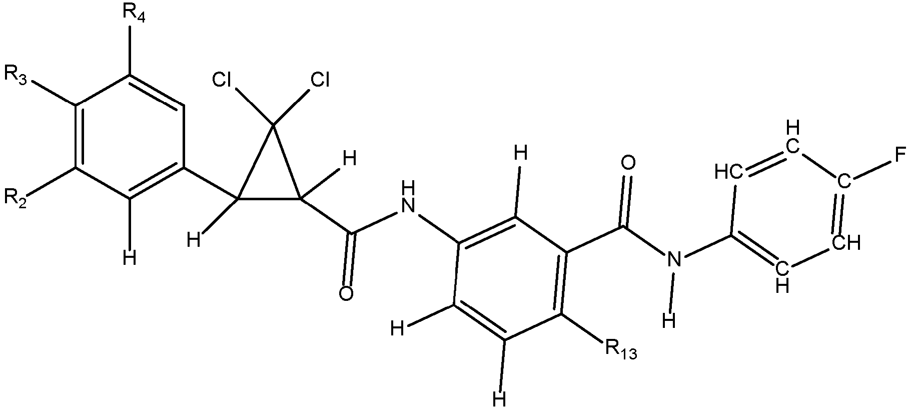
and N-oxides, veterinary acceptable acid addition salts, salt derivatives, solvates, ester derivatives, crystal polymorphs, isotopes, stereoisomers, and tautomers thereof,
wherein R², R³, R⁴, and R¹³ are defined as elsewhere in this description.

In an embodiment of the invention and/or embodiments thereof, the invention is directed to a compound of formula (XXIX) 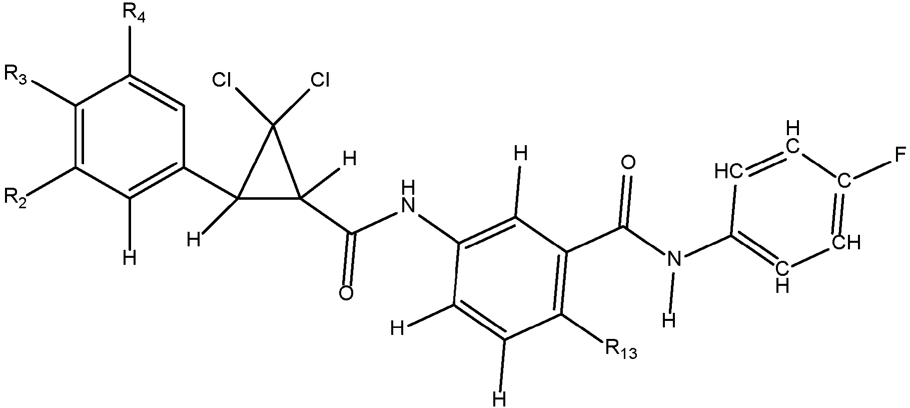
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XXIX) 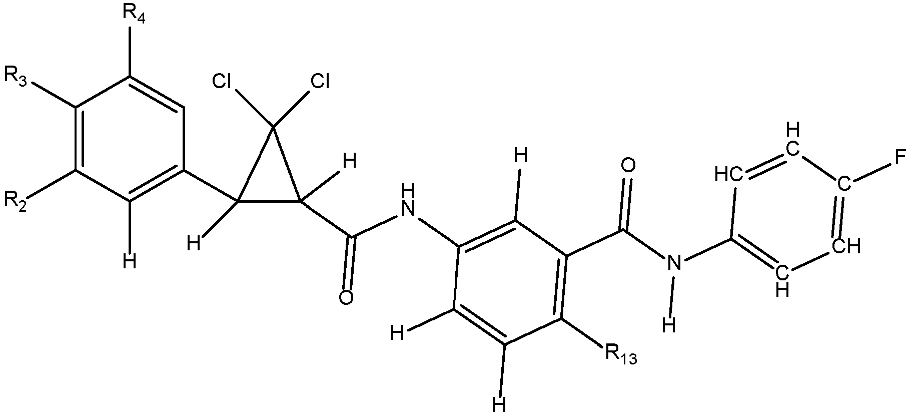
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R¹³** is selected from the group consisting of H, F, Cl, CH₃ and CF₃.

In an embodiment of the invention and/or embodiments thereof, the invention is directed to a compound of formula (XXIX) 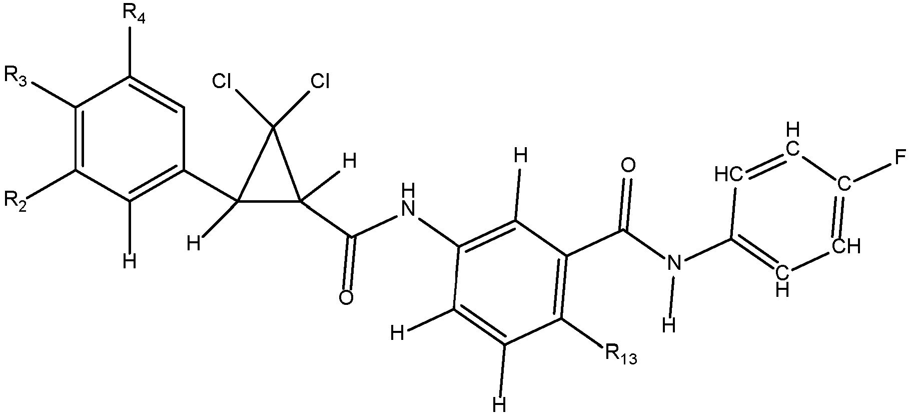
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R¹³** is selected from the group consisting of H, F, Cl, CH₃ and CF₃.

In an embodiment of the invention and/or embodiments thereof, the invention is directed to a compound of formula (XXIX) 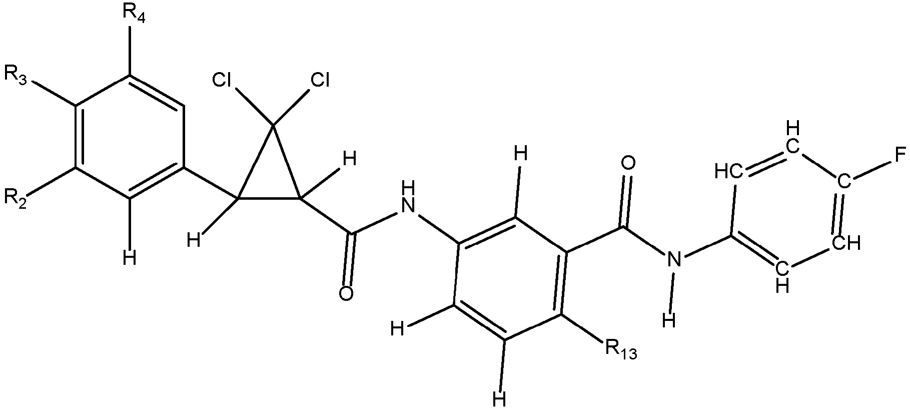
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R¹³** is Cl or F.

In an embodiment of the invention and/or embodiments thereof, the invention is directed to a compound of formula (XXIX) 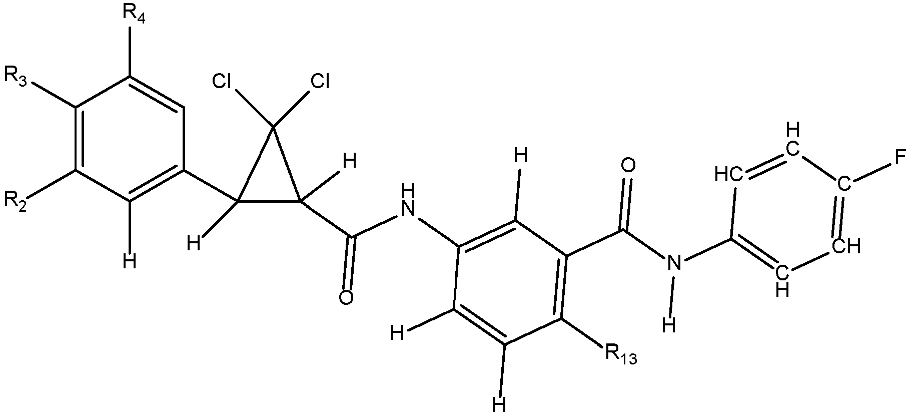
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R¹³** is Cl.

In an embodiment of the invention and/or embodiments thereof, the invention is directed to a compound of formula (XXX) 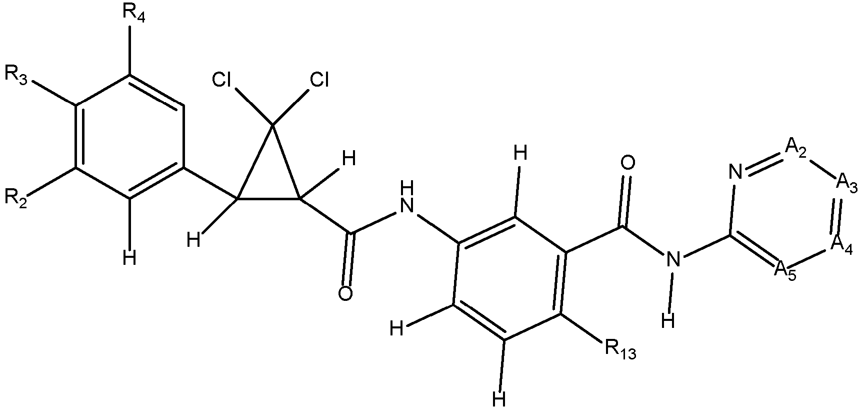
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹³, A₂, A₃, A₄, and A₅ are defined as elsewhere in this description and wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXX) 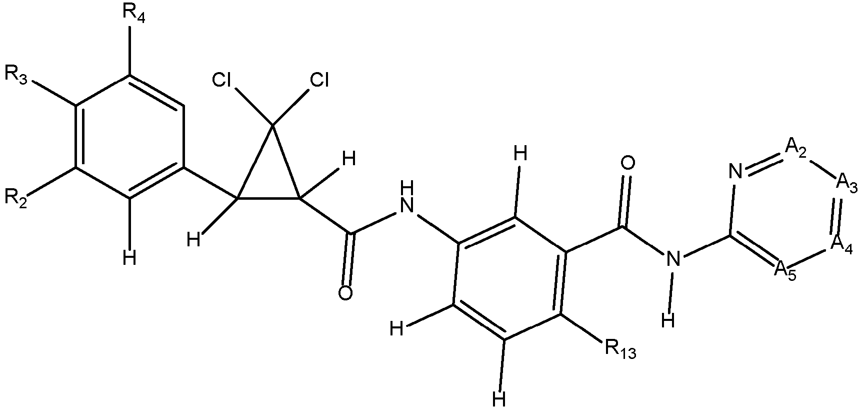
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXX) 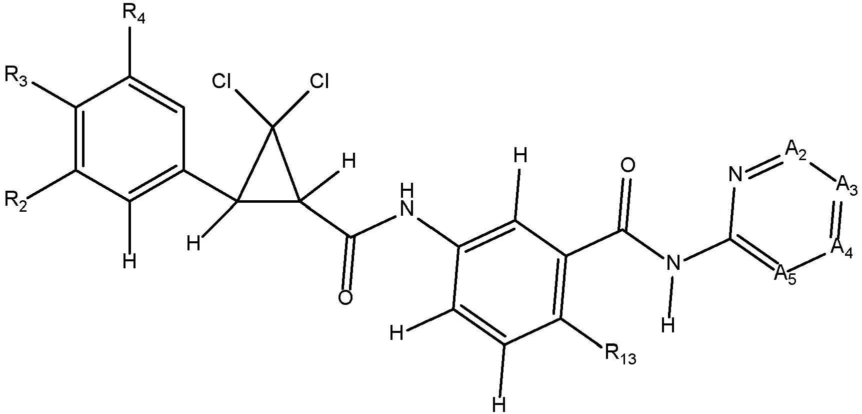
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
wherein at least one of R⁶, R⁷, R⁸, R⁹, is a substituent selected from the group consisting of F, Cl, and Br, and I.

In an embodiment of the invention and/or embodiments thereof, the invention is directed to a compound of formula (XXX) 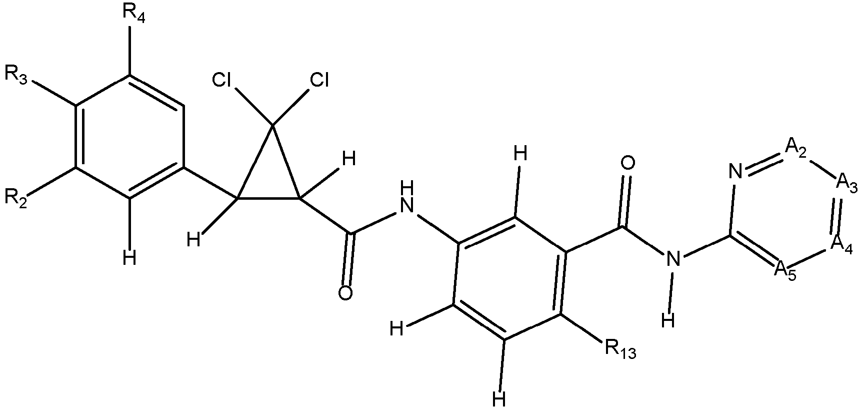
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₃ is CR₇ or N;
A₄ is CR₈ or N;
A₅ is CR₉ or N;
wherein no more than 2 of 2 A₂, A₃, A₄, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
wherein at least one of R⁶, R⁷, R⁸, R⁹, is a substituent selected from the group consisting of F, Cl, Br, and I.

In an embodiment of the invention and/or embodiments thereof, the invention is directed to a compound of formula (XXX) 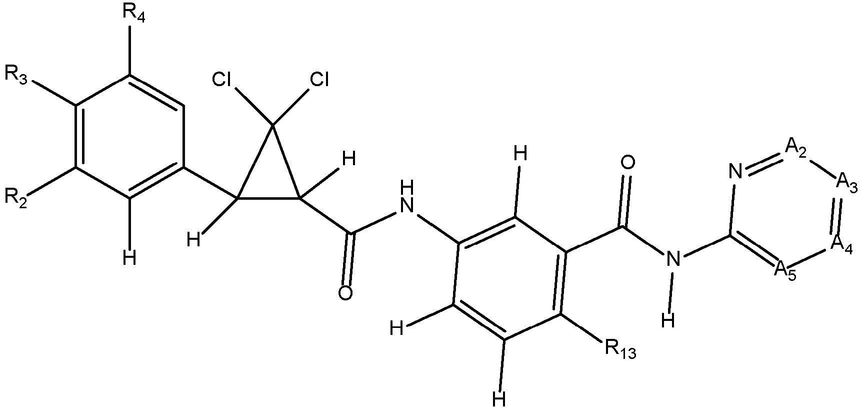
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A2 is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
wherein at least one of R⁶, R⁷, R⁸, R⁹, is a substituent selected from the group consisting of F, and Cl.

In an embodiment of the invention and/or embodiments thereof, the invention is directed to a compound of formula (XXX) 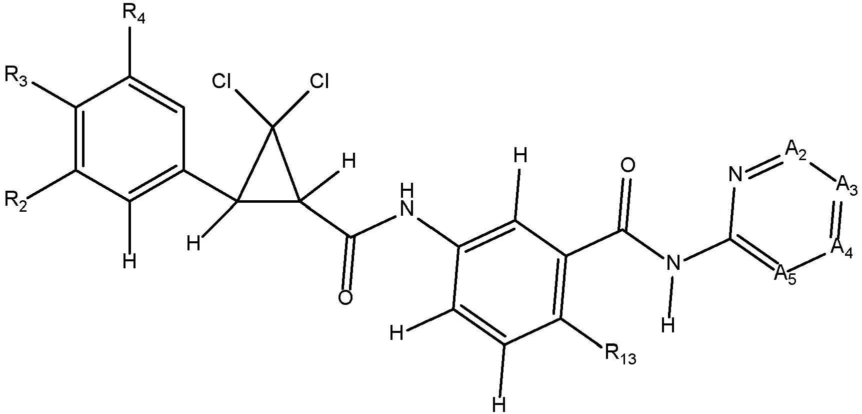
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₂ is CR₆ or N;
A₃ is CR₇;
A₄ is CR₈;
A₅ is CR₉;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
wherein at least one of R⁶, R⁷, R⁸, R⁹, is a substituent selected from the group consisting of F, and Cl.

In an embodiment of the invention and/or embodiments thereof, the invention is directed to a compound of formula (XXXI) 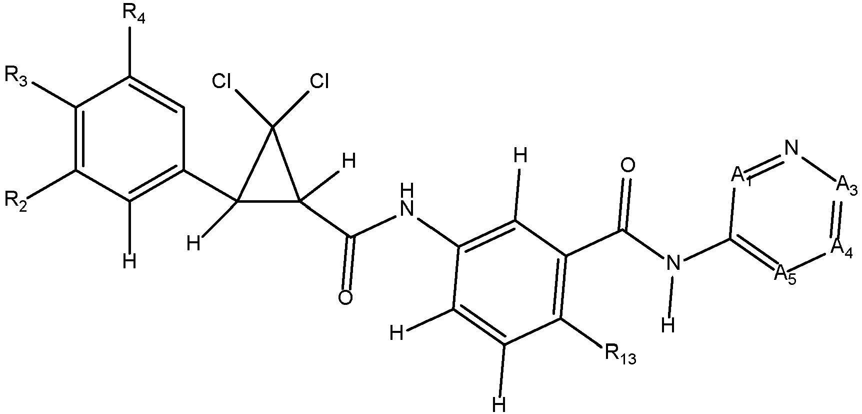
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁷, R⁸, R⁹, R¹³, A₁, A₃, A₄, and A₅ are defined as elsewhere in this description and wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXXI) 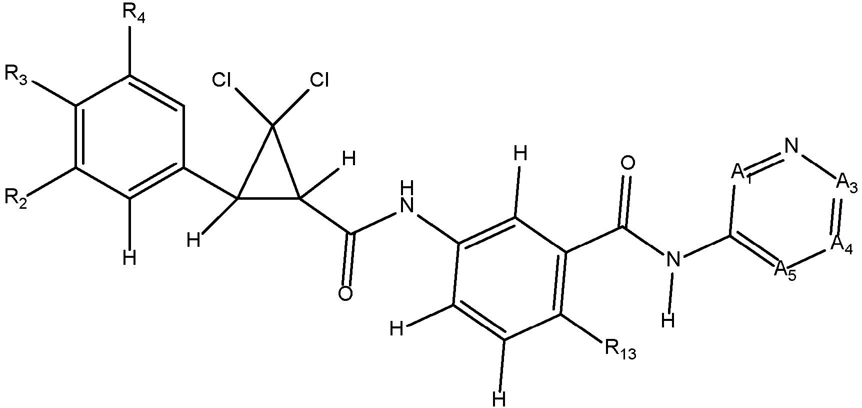
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₃ is CR₇, N=O, or N;
A₄ is CR₈, N=O or N;
A₅ is CR₉, N=O or N ;
wherein no more than 2 of A₁, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁₋C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXXI) 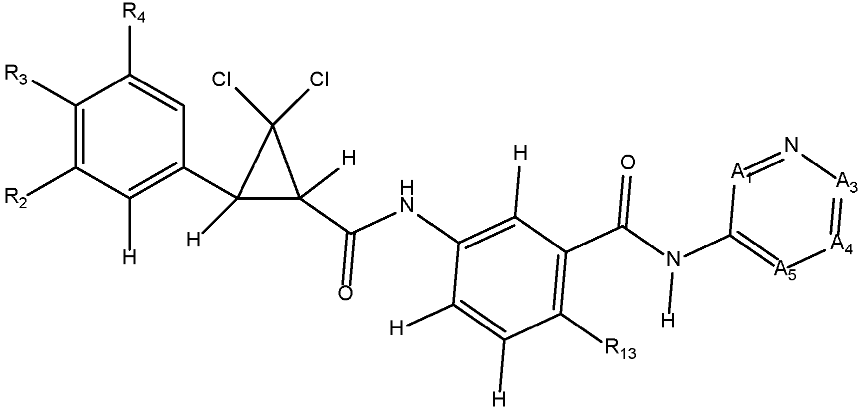
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₃ is CR₇, N=O, or N;
A₄ is CR₈, N=O or N;
A₅ is CR₉, N=O or N;
wherein no more than 2 of A₁, A₃, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
wherein at least one of R⁵, R⁷, R⁸, R⁹, is a substituent selected from the group consisting of F, Cl, and Br, and I.

In an embodiment of the invention and/or embodiments thereof, the invention is directed to a compound of formula (XXXI) 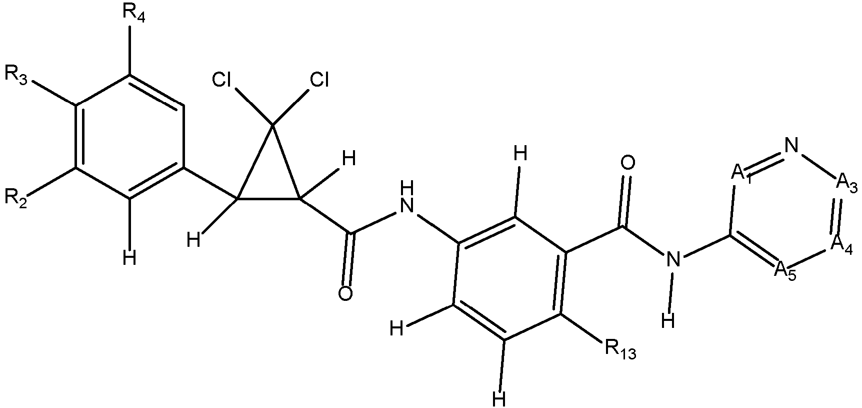
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₃ is CR₇ or N;
A₄ is CR₈ or N;
A₅ is CR₉ or N;
wherein no more than 2 of A₁, A₃, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₁-C₆)cycloalkyl, tetrahydrofuranyl, and phenyl ;
**R¹³ is** selected from the group consisting of H, F, Cl, CH₃ and CF₃ ;
wherein at least one of R⁵, R⁷, R⁸, R⁹, is a substituent selected from the group consisting of F, Cl, Br, and I.

In an embodiment of the invention and/or embodiments thereof, the invention is directed to a compound of formula (XXXI) 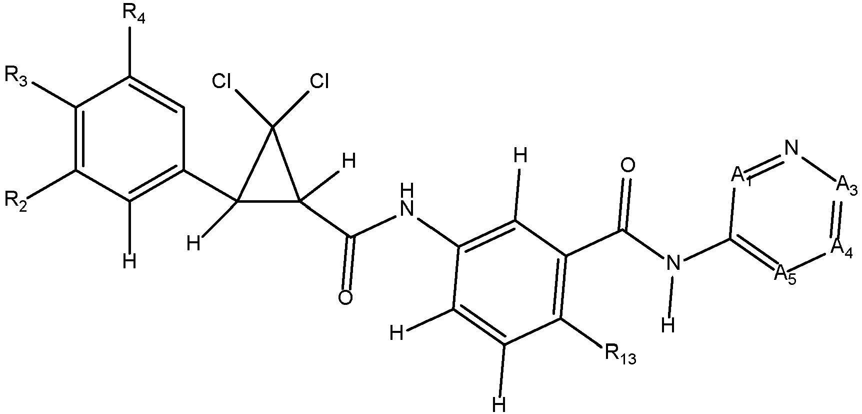
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₃ is CR₇ or N;
A₄ is CR₈ or N;
A₅ is CR₉ or N;
wherein 0 or 1 of A₁, A₃, A₄, A₅ are N;
**R⁵** is selected from the group consisting of H, F, Cl, CN, NO₂, NH(C₁-C₆)alkyl, and (C₁-C₆)alkyl ;
**R⁷** is selected from the group consisting of H, F, and Cl ;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹³** is Cl or F ;
wherein at least one of R⁵, R⁷, R⁸, R⁹, is a substituent selected from the group consisting of F, and Cl.

In an embodiment of the invention and/or embodiments thereof, the invention is directed to a compound of formula (XXXI) 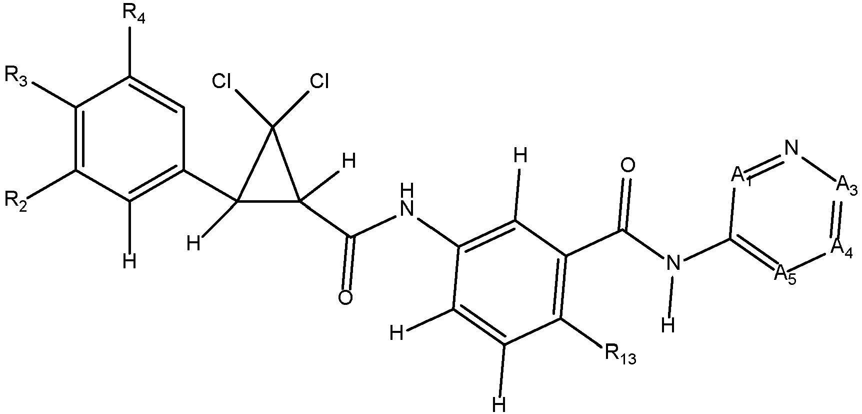
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₃ is CR₇;
A₄ is CR₈;
A₅ is CR₉;
**R⁵** is selected from the group consisting of H, F, Cl, CN and (C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
wherein at least one of R⁵, R⁷, R⁸, R⁹, is a substituent selected from the group consisting of F, and Cl.

In an embodiment of the invention and/or embodiments thereof, the invention is directed to a compound of formula (XXXII) 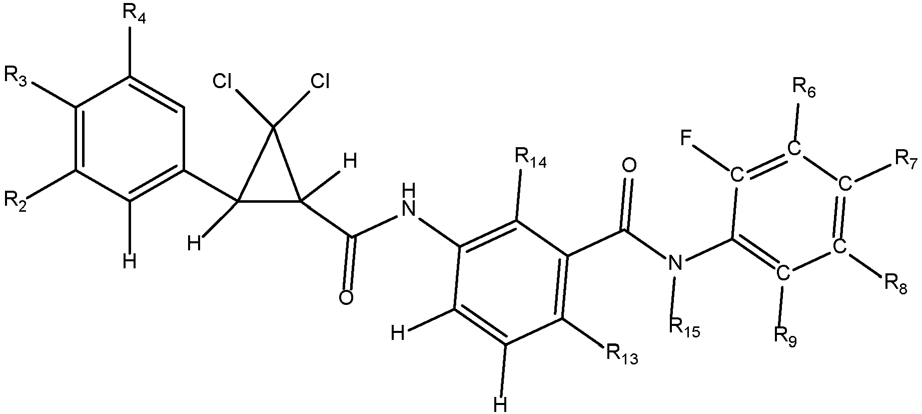
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹³, R¹⁴, R¹⁵are defined as elsewhere in this description.

In an embodiment of the invention and/or embodiments thereof, the invention is directed to a compound of formula (XXXII) 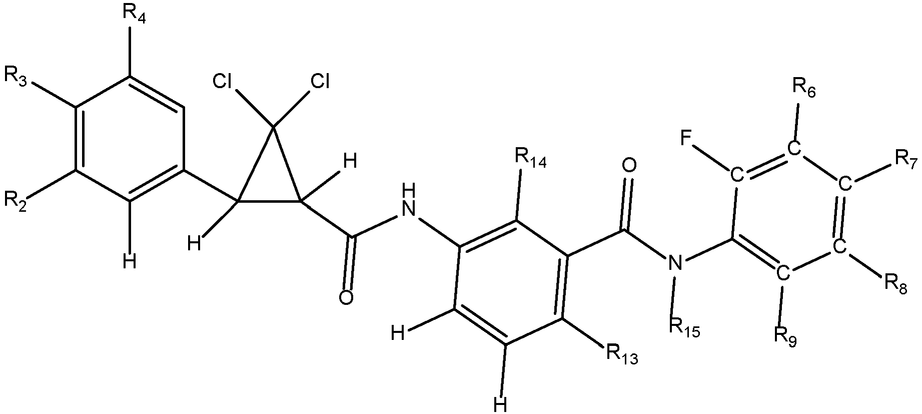
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXXII) 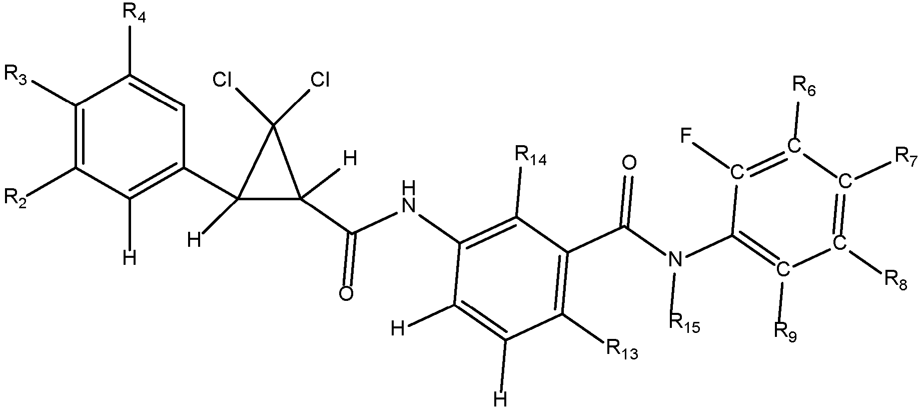
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXII) 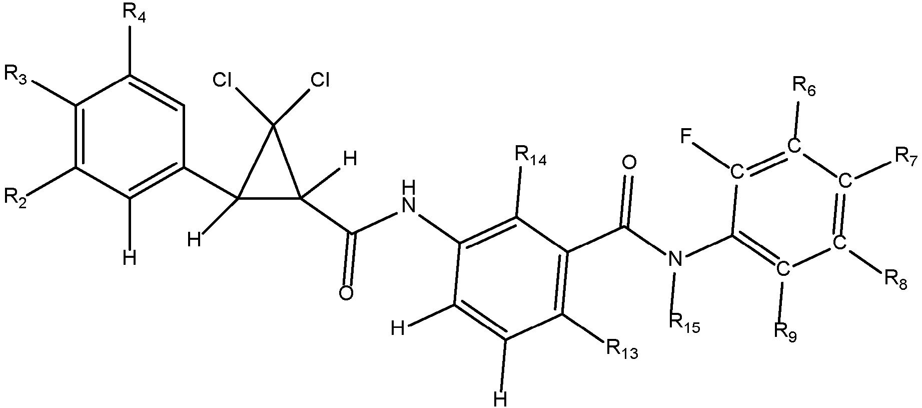
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXII) 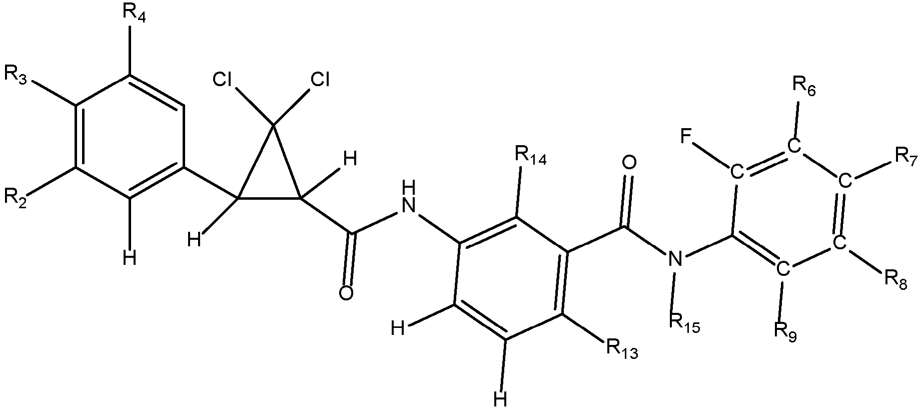
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XXXII) 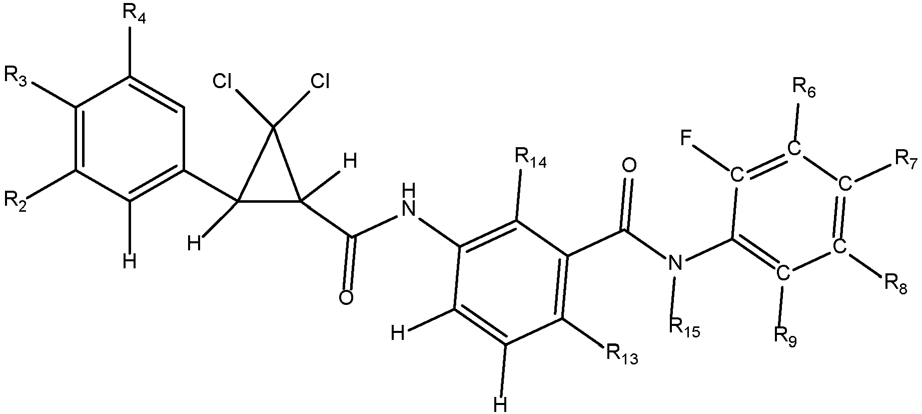
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl;
**R¹⁴** is H;
**R¹⁵** is H.

In an embodiment of the invention and/or embodiments thereof, the invention is directed to a compound of formula (XXXIII) 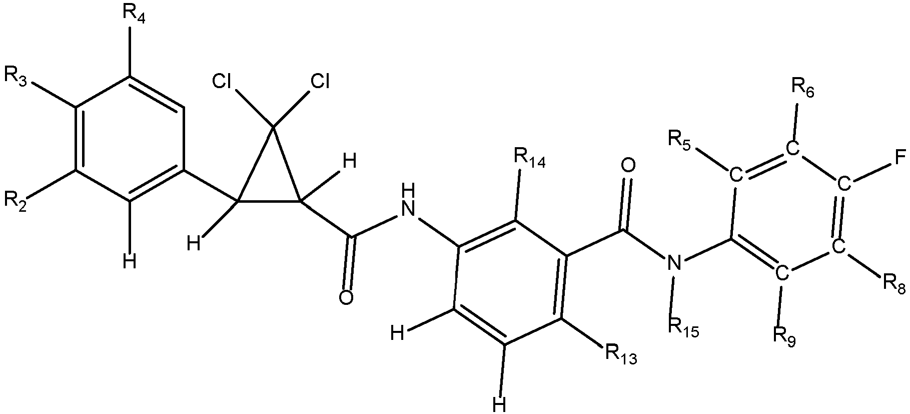
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁸, R⁹, R¹³, R¹⁴, and R¹⁵ are defined as elsewhere in this description.

In an embodiment of the invention and/or embodiments thereof, the invention is directed to a compound of formula (XXXIII) 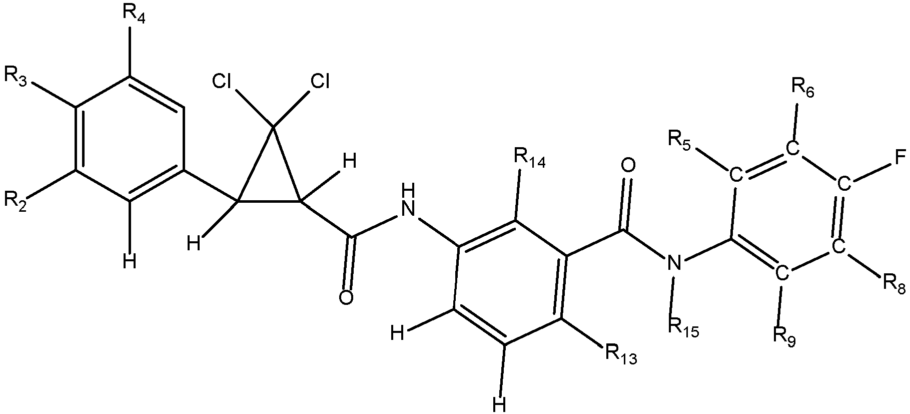
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXXIII) 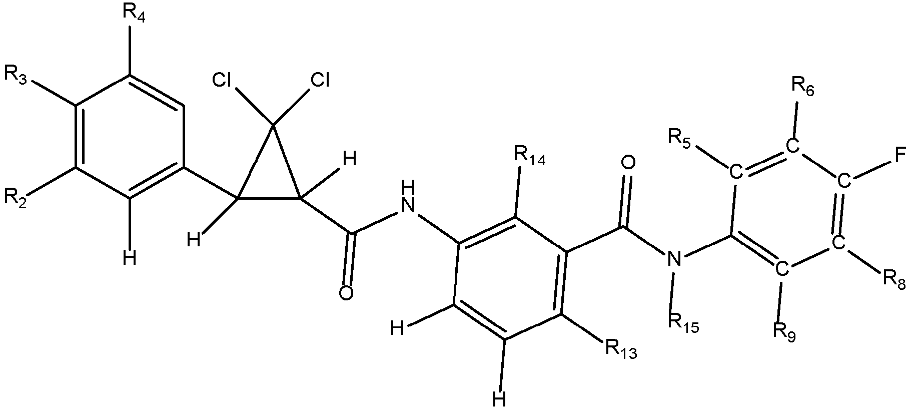
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₈)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIII) 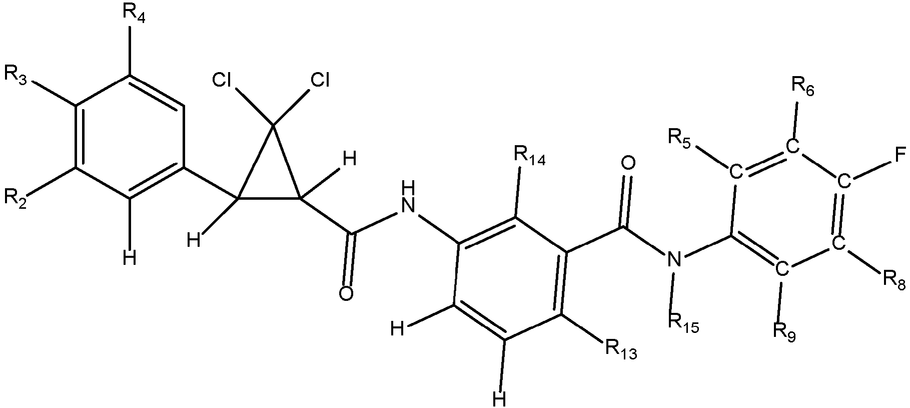
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIII) 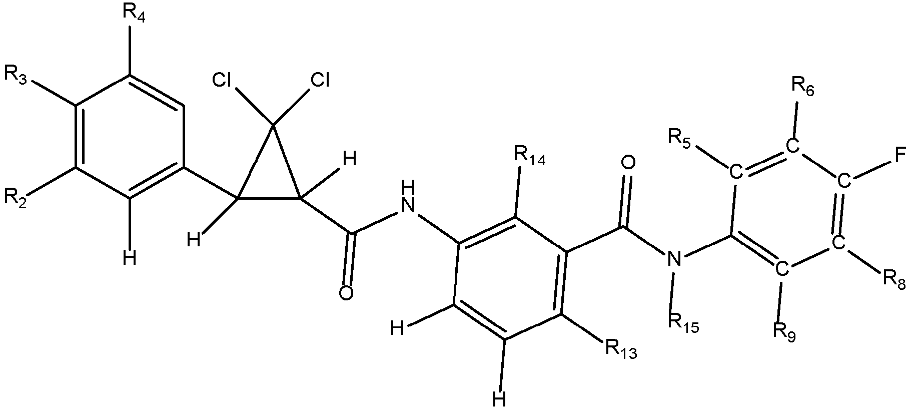
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XXXIII) 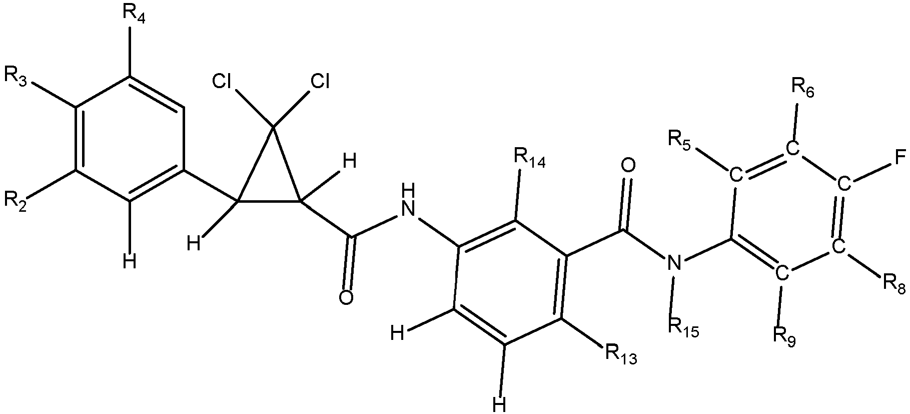
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁵** is selected from the group consisting of H, F, Cl, CN and (C₁-C₆)alkyl ;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹³** is Cl;
**R¹⁴** is H;
**R¹⁵** is H.

In an embodiment of the invention and/or embodiments thereof, the invention is directed to a compound of formula (XXXIV) 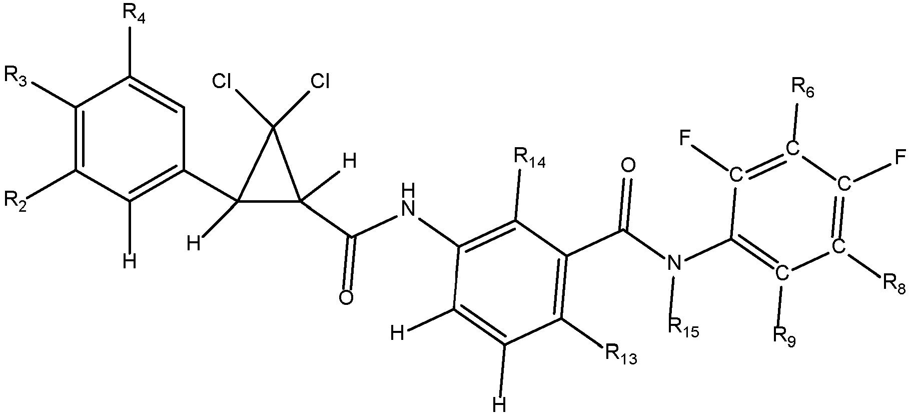
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, R⁹, R¹³, R¹⁴, and R¹⁵ are defined as elsewhere in this description.

In an embodiment of the invention and/or embodiments thereof, the invention is directed to a compound of formula (XXXIV) 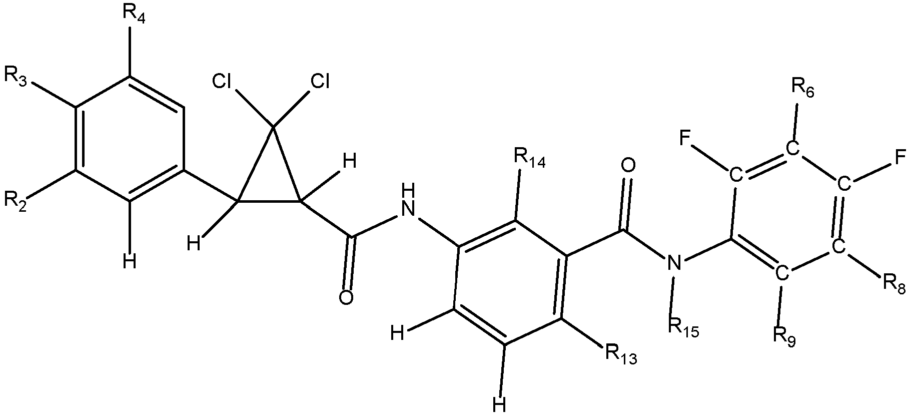
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXXIV) 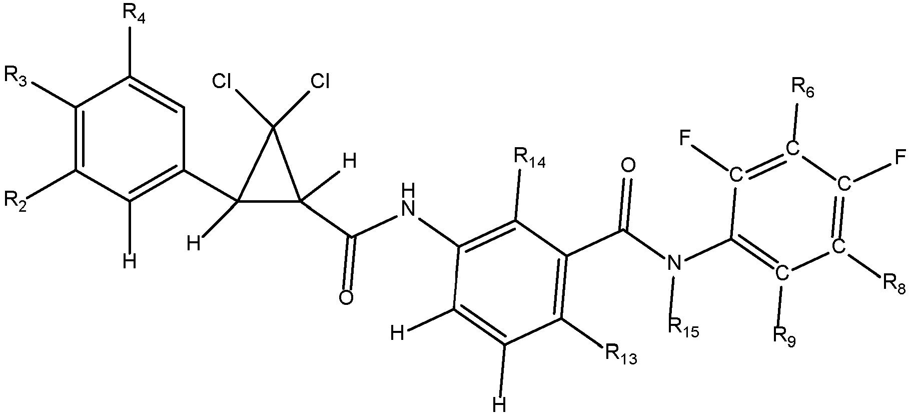
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIV) 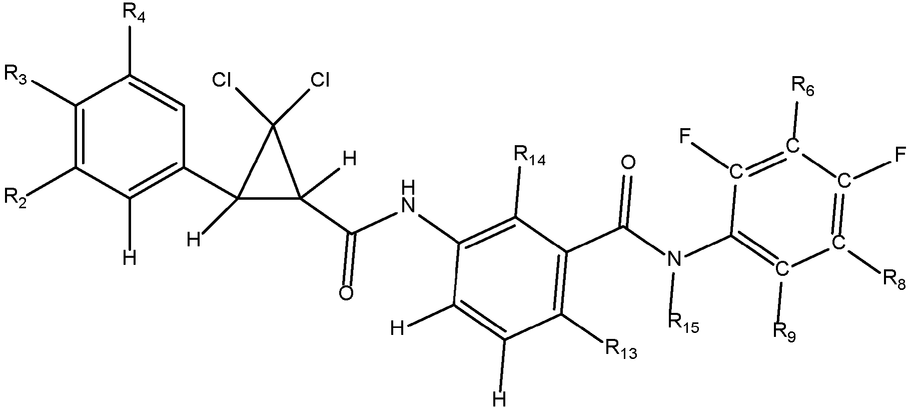
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIV) 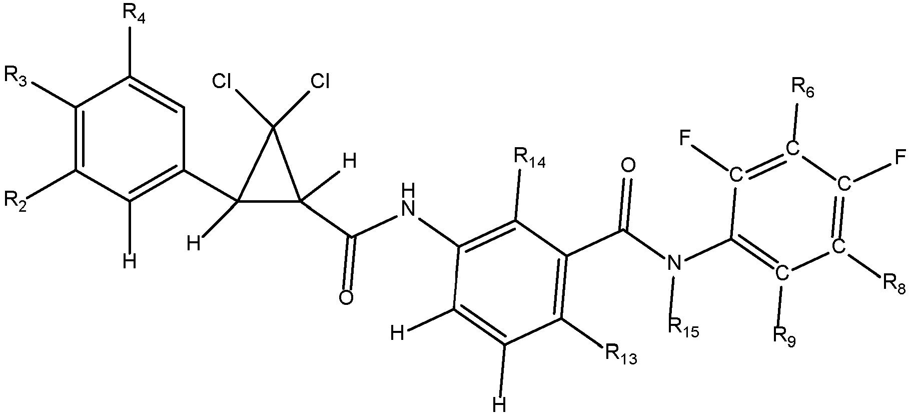
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
R¹³ is Cl or F ;
**R¹⁴** is selected from the group consisting of H and F;
**R¹⁵** is selected from the group consisting of H, and CH₃.

In an embodiment of the invention and/or embodiments thereof, the invention is directed to a compound of formula (XXXIV) 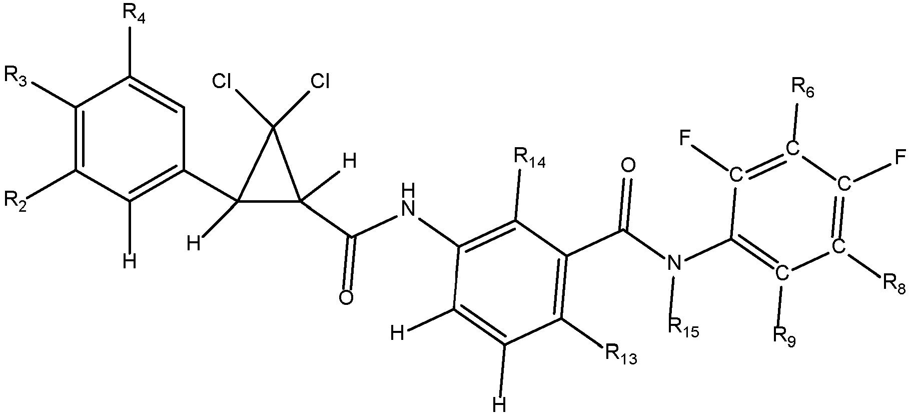
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹³** is Cl;
**R¹⁴** is H;
**R¹⁵** is H.

In an embodiment of the invention and/or embodiments thereof, the invention is directed to a compound of formula (XXXV) 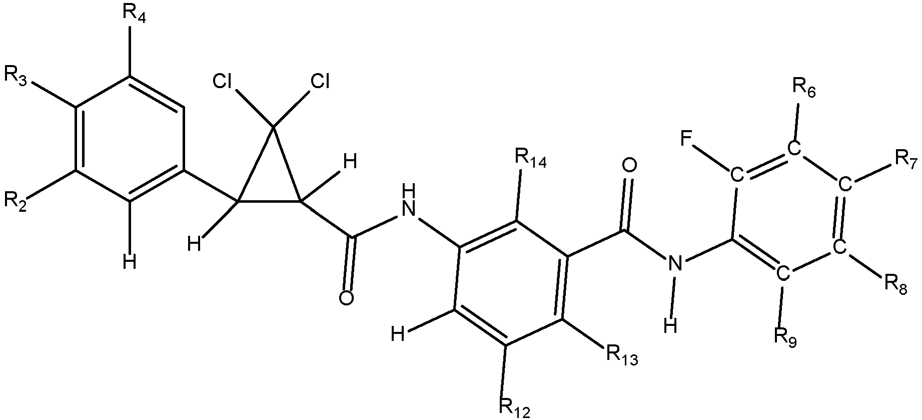
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹², R¹³, and R¹⁴are defined as elsewhere in this description.

In an embodiment of the invention and/or embodiments thereof, the invention is directed to a compound of formula (XXXV) 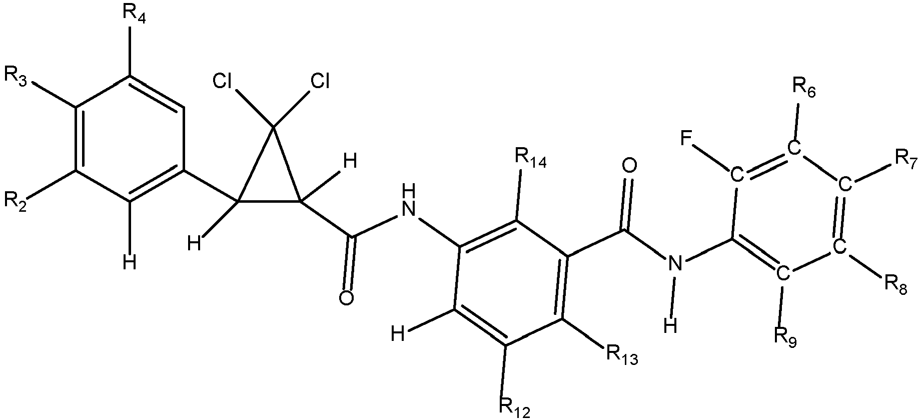
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, CI, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, CI, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XXXV) 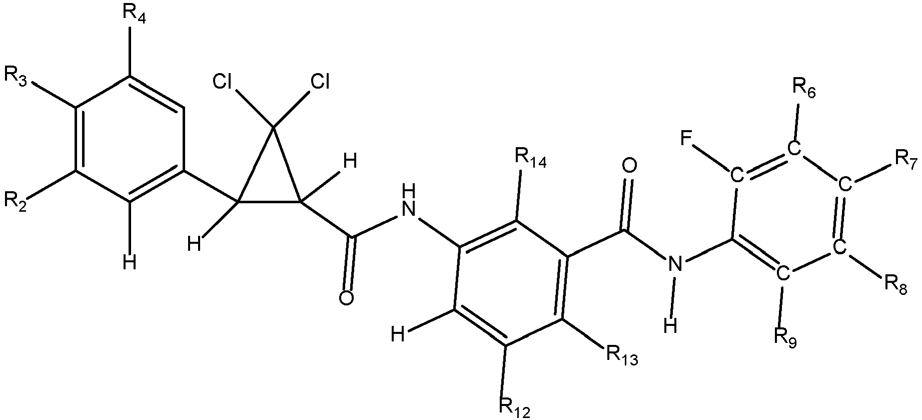
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXV) 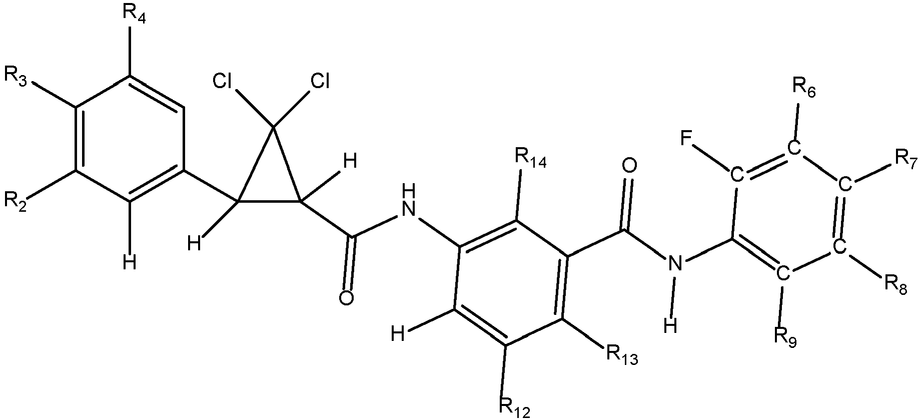
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXV) 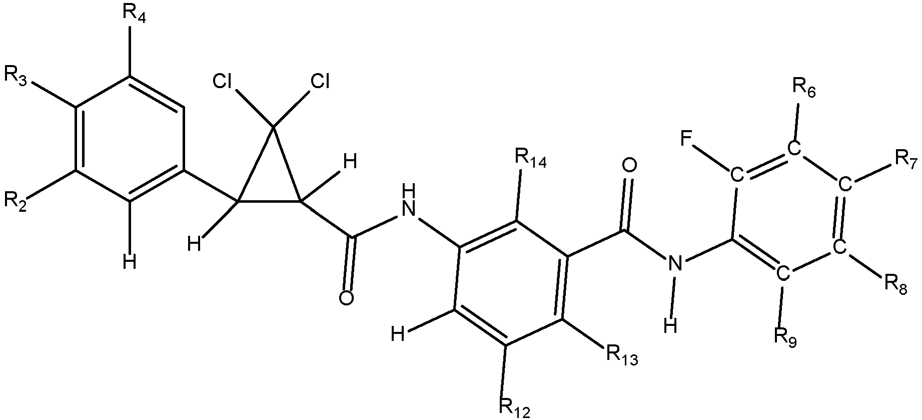
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F.

In an embodiment of the invention and/or embodiments thereof, the invention is directed to a compound of formula (XXXV) 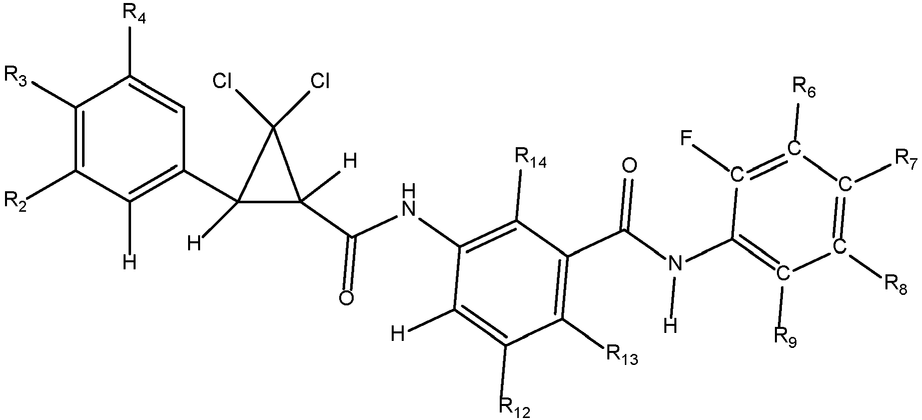
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H.

In an embodiment of the invention and/or embodiments thereof, the invention is directed to a compound of formula (XXXVI) 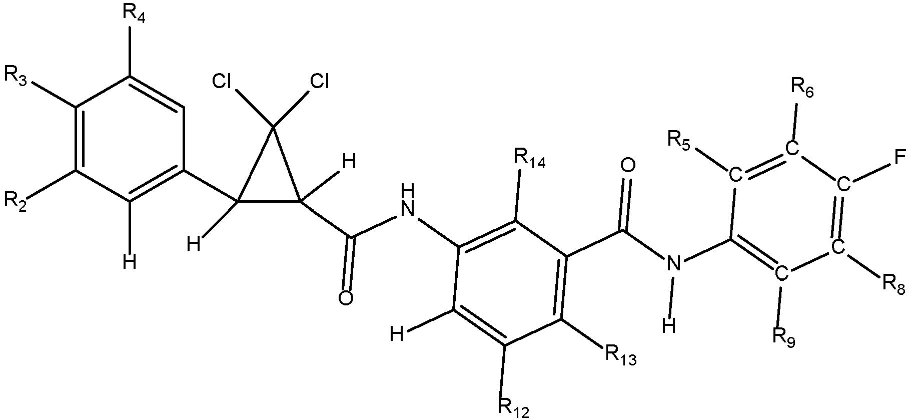
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁸, R⁹, R¹², R¹³, and R¹⁴ are defined as elsewhere in this description.

In an embodiment of the invention and/or embodiments thereof, the invention is directed to a compound of formula (XXXVI) 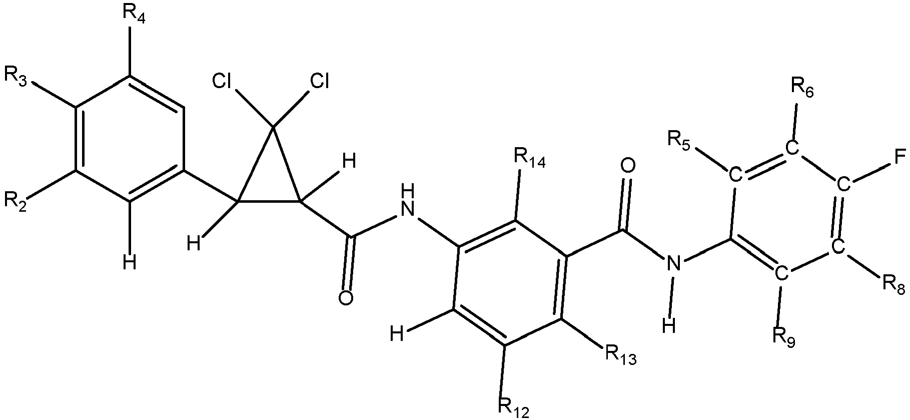
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XXXVI) 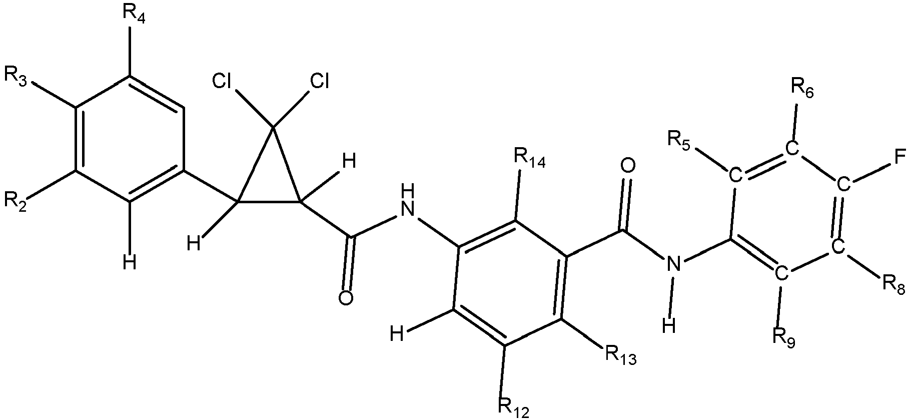
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXVI) 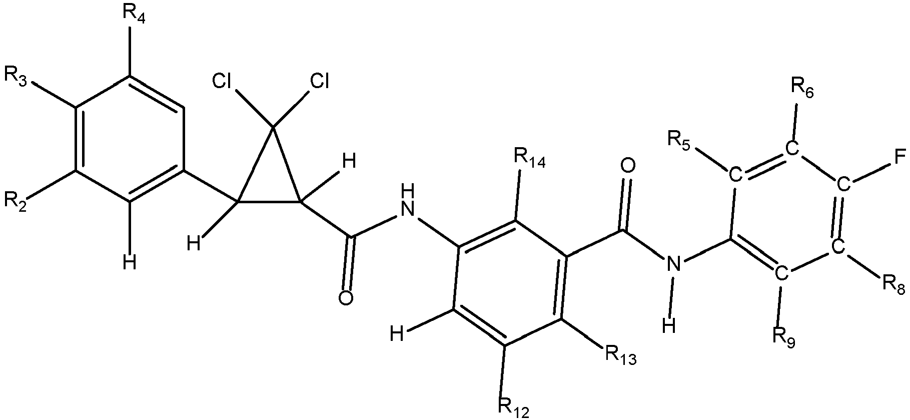
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴ is** selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXVI) 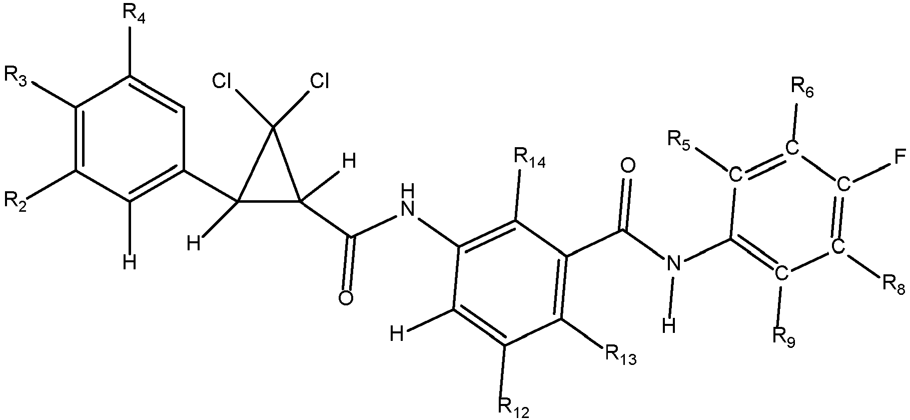
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴ is** selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F.

In an embodiment of the invention and/or embodiments thereof, the invention is directed to a compound of formula (XXXVI) 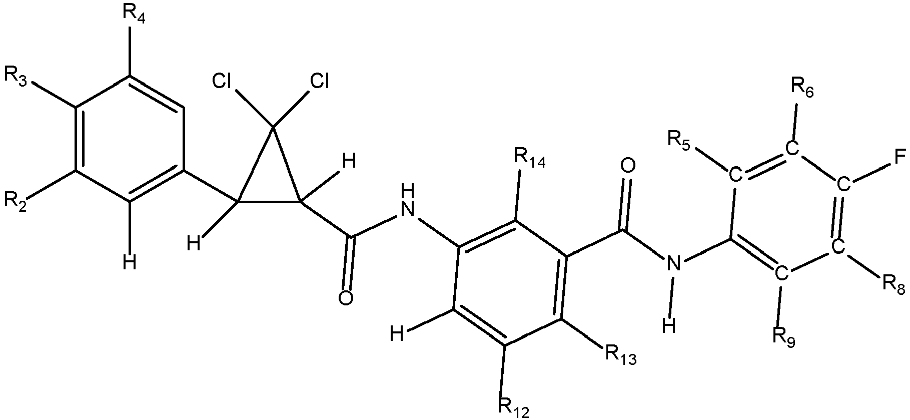
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H.

In an embodiment of the invention and/or embodiments thereof, the invention is directed to a compound of formula (XXXVII) 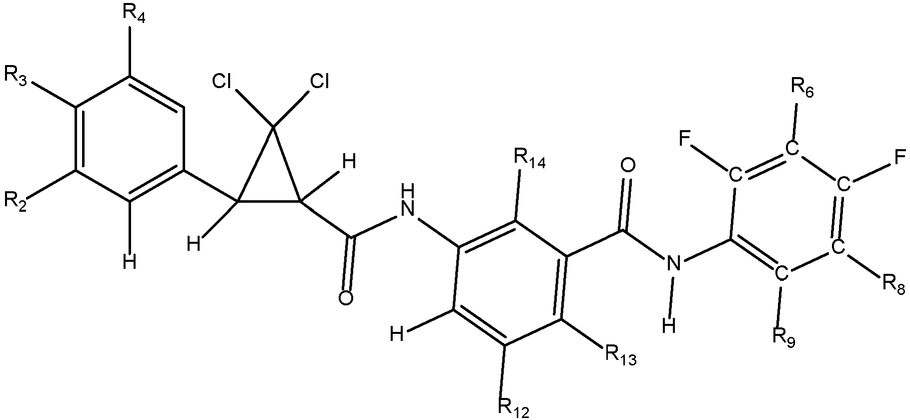
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, R⁹, R¹², R¹³, and R¹⁴ are defined as elsewhere in this description.

In an embodiment of the invention and/or embodiments thereof, the invention is directed to a compound of formula (XXXVII) 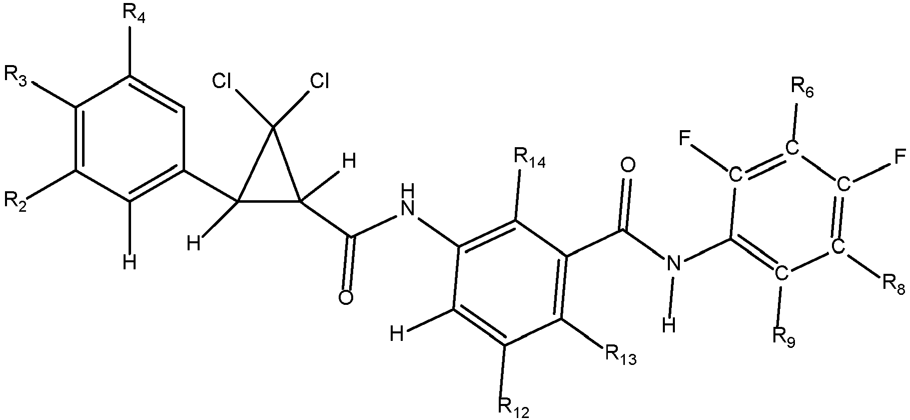
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (XXXVII) 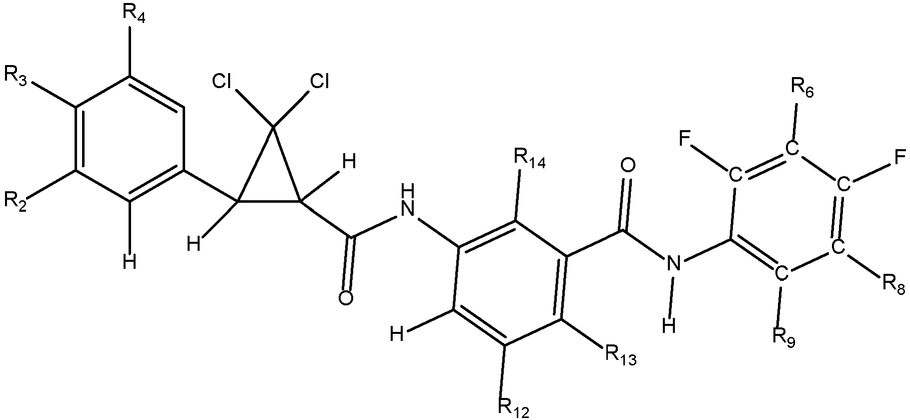
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXVII) 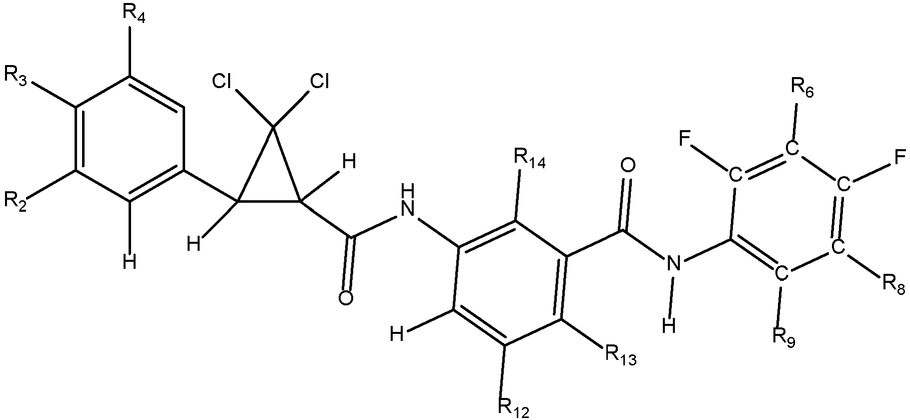
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (XXXVII) 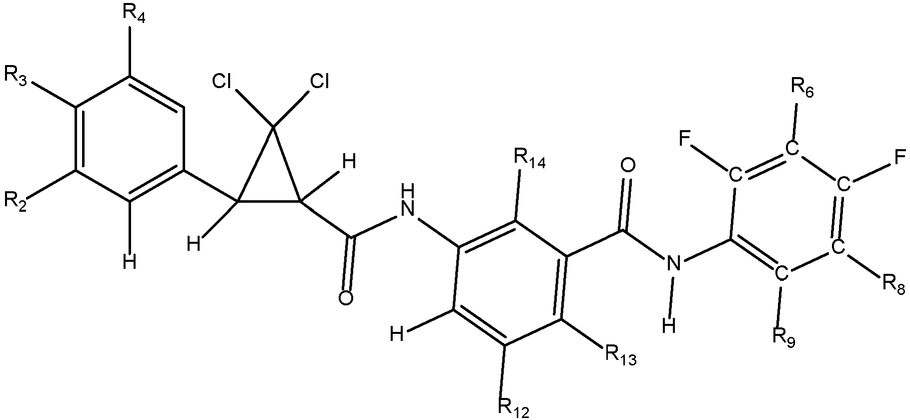
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F.

In an embodiment of the invention and/or embodiments thereof, the invention is directed to a compound of formula (XXXVII) 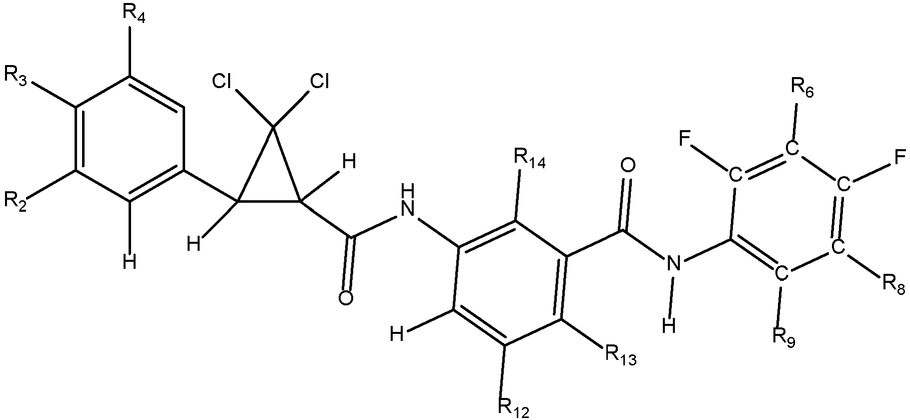
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H.

In an embodiment of the invention and/or embodiments thereof, the invention is directed to a compound of formula (XXXVIII) 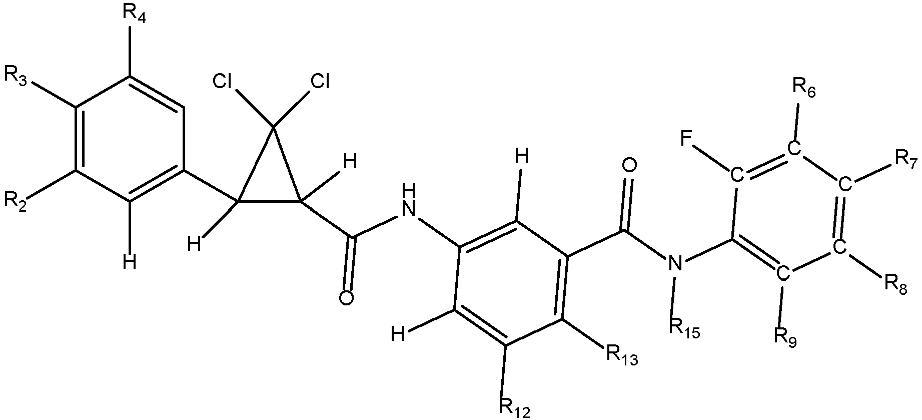
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹², R¹³, R¹³, A₁, A₂, A₃, A₄, and A₅ are defined as elsewhere in this description.

In an embodiment of the invention and/or embodiments thereof, the invention is directed to a compound of formula (XXXVIII) 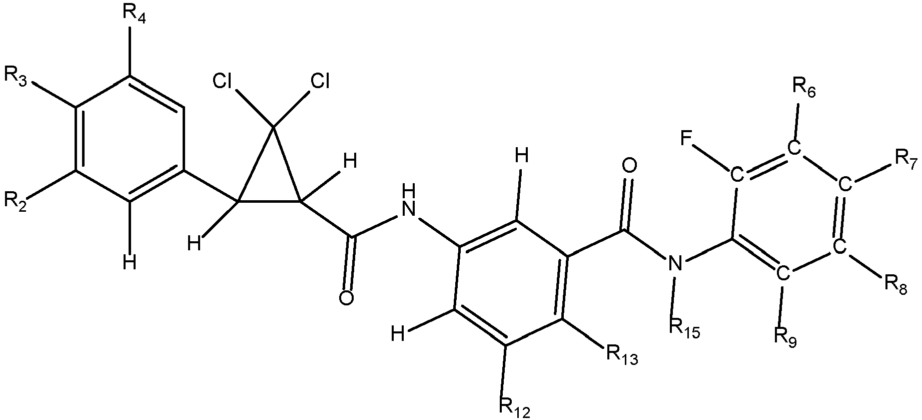
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXXVIII) 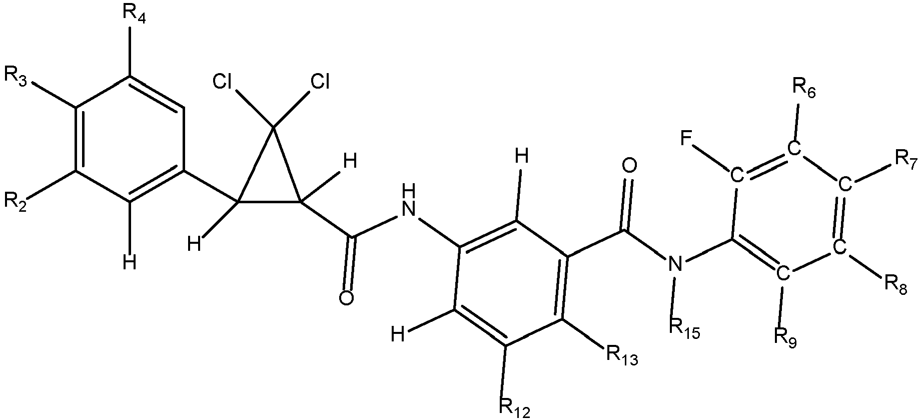
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R**⁶ and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃.

In an embodiment of the invention and/or embodiments thereof, the invention is directed to a compound of formula (XXXVIII) 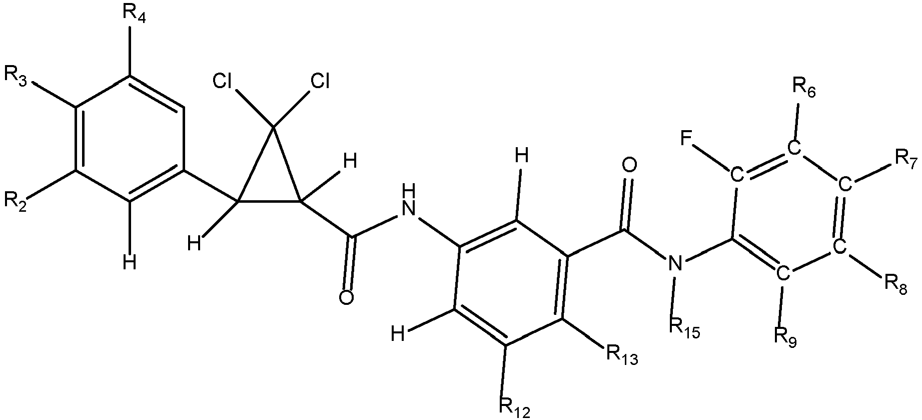
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶ is** selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXVIII) 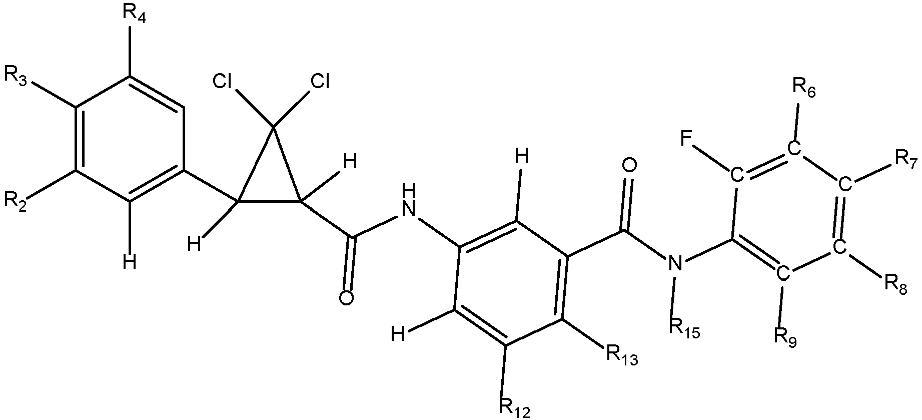
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XXXVIII) 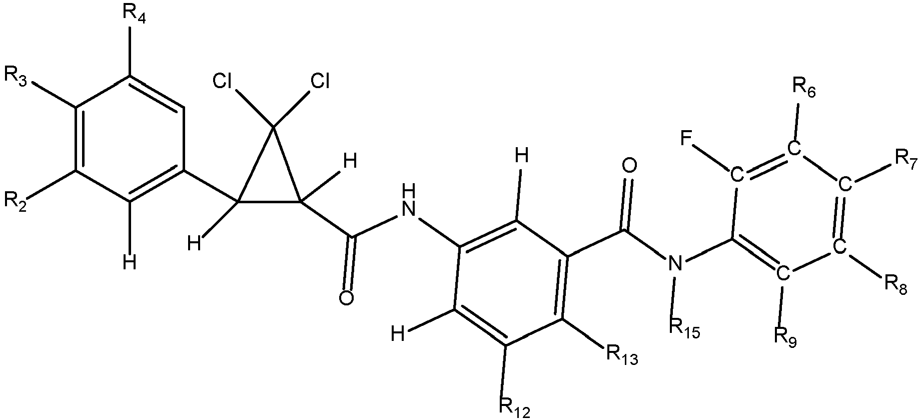
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁵** is H.

In an embodiment of the invention and/or embodiments thereof, the invention is directed to a compound of formula (XXXIX) 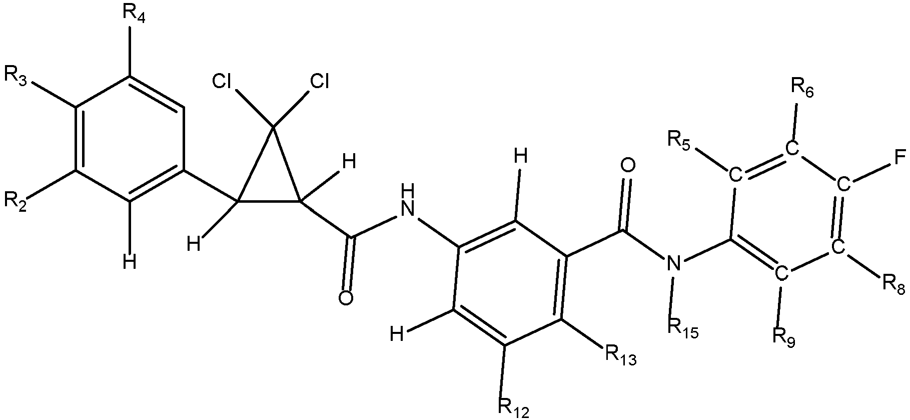
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁸, R⁹, R¹², R¹⁵, and R¹⁵ are defined as elsewhere in this description.

In an embodiment of the invention and/or embodiments thereof, the invention is directed to a compound of formula (XXXIX) 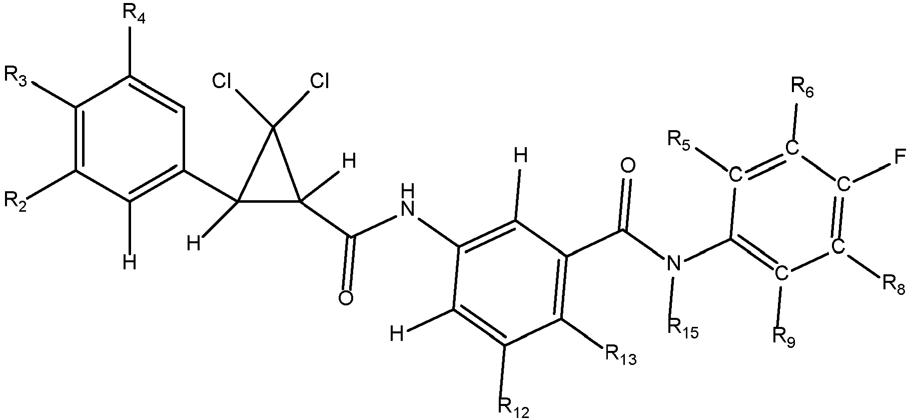
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XXXIX) 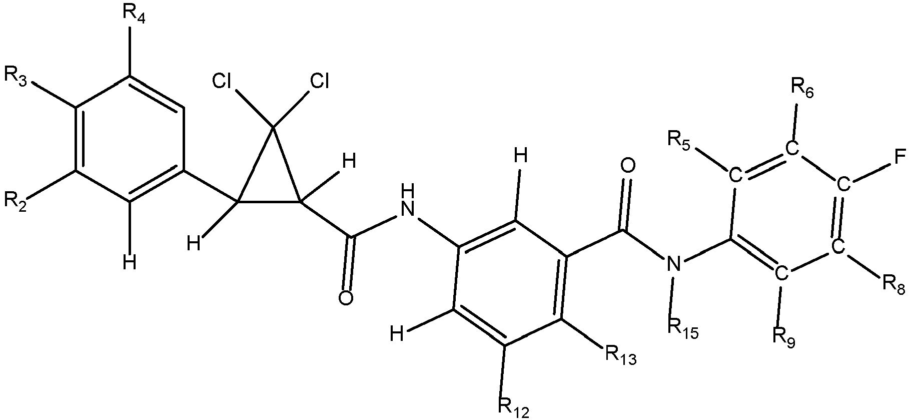
and N-oxides, veterinary acceptable acid addition salts, salt derivatives, solvates, ester derivatives, crystal polymorphs, isotopes, stereoisomers, and tautomers thereof,
wherein
**R² is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸ is** selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIX) 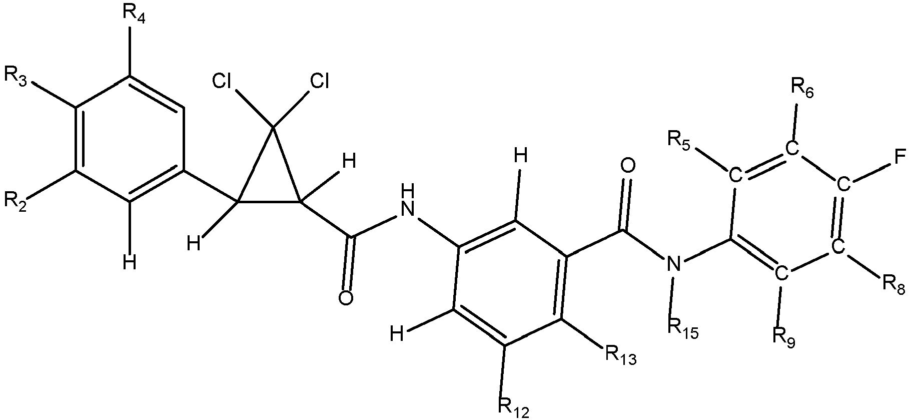
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XXXIX) 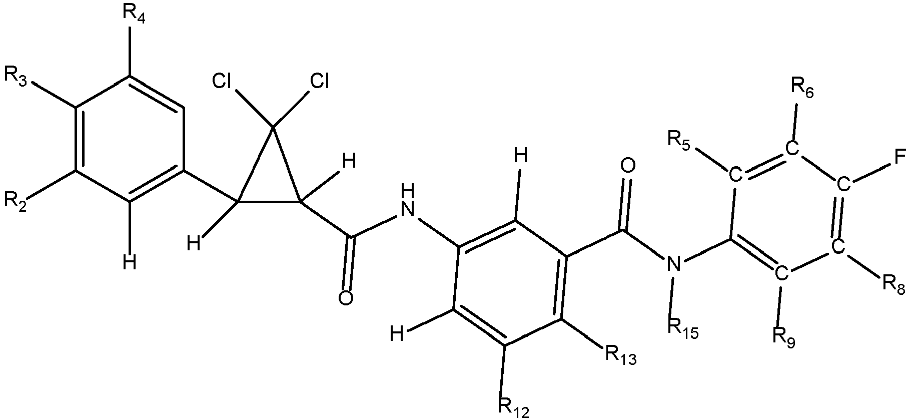
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XXXIX) 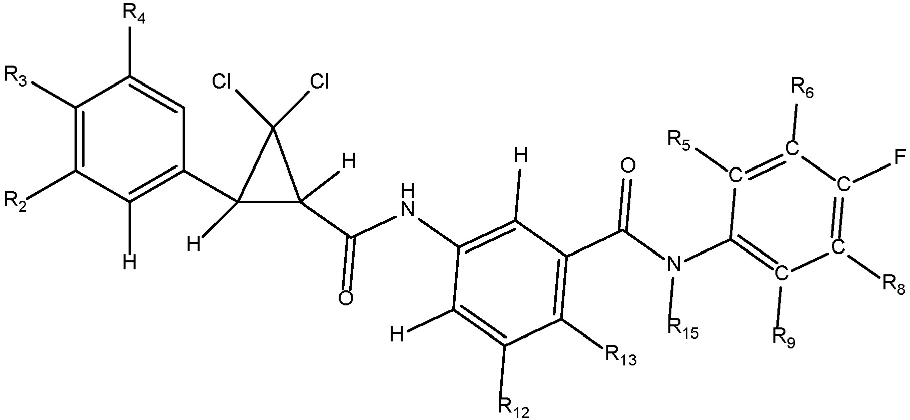
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹²** is H ;
**R¹³** is Cl;
**R¹⁵** is H.

In an embodiment of the invention and/or embodiments thereof, the invention is directed to a compound of formula (XL) 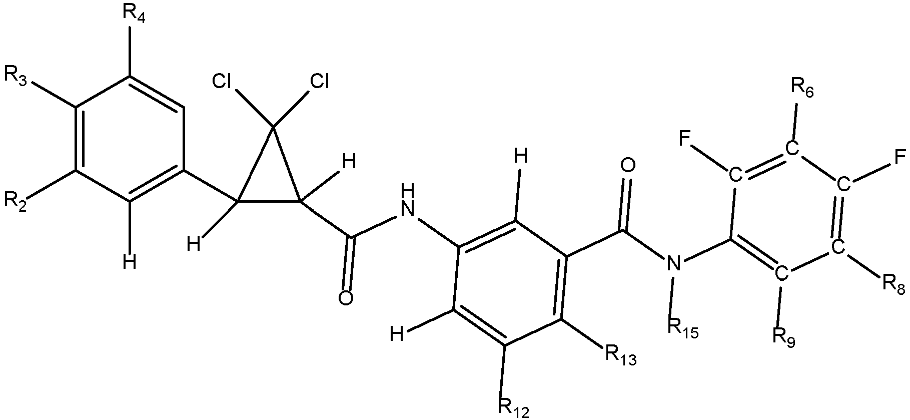
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, R⁹, R¹², R¹³, and R¹⁵ are defined as elsewhere in this description.

In an embodiment of the invention and/or embodiments thereof, the invention is directed to a compound of formula (XL) 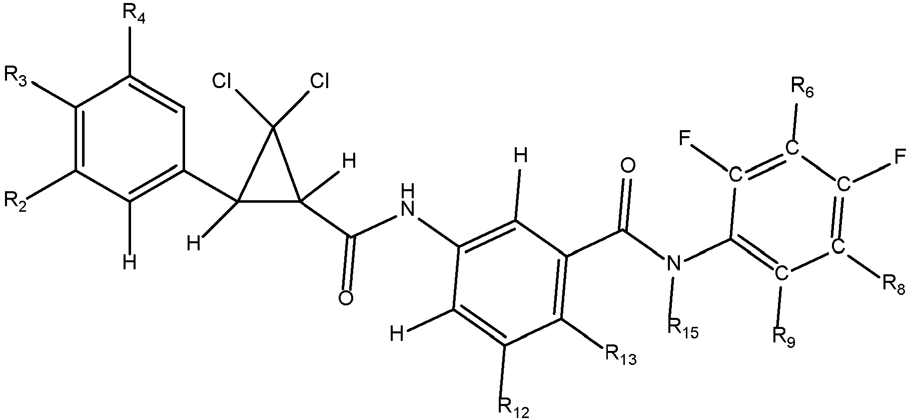
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³ is** selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-Cs)alkyloxy-(C₁-Cs)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XL) 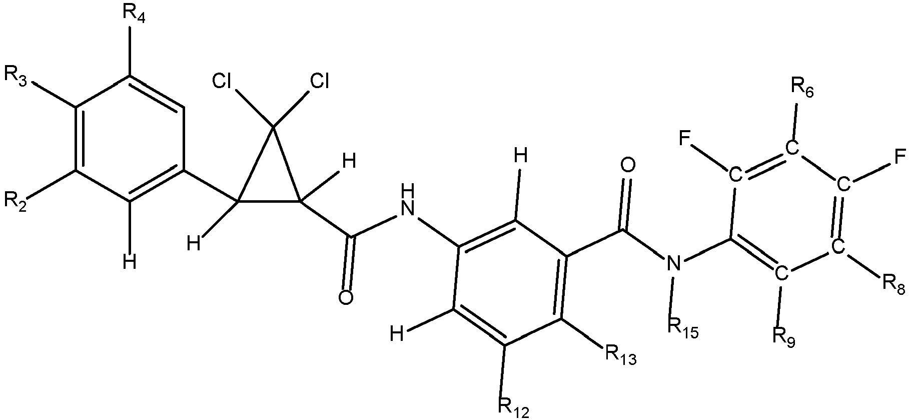
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XL) 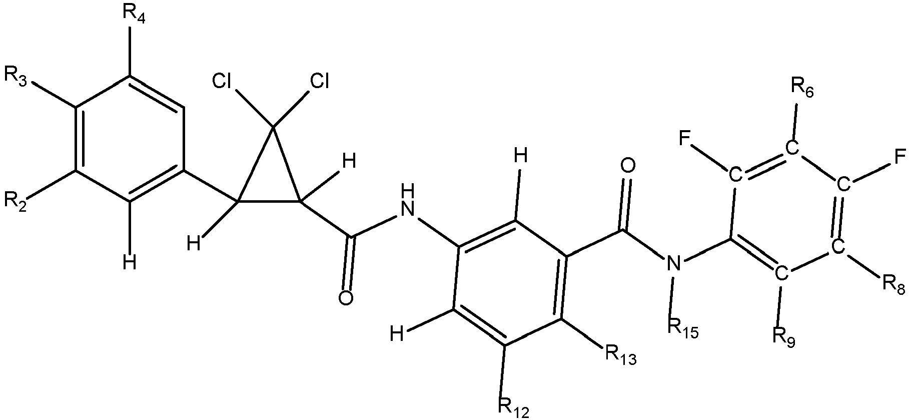
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴ is** selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XL) 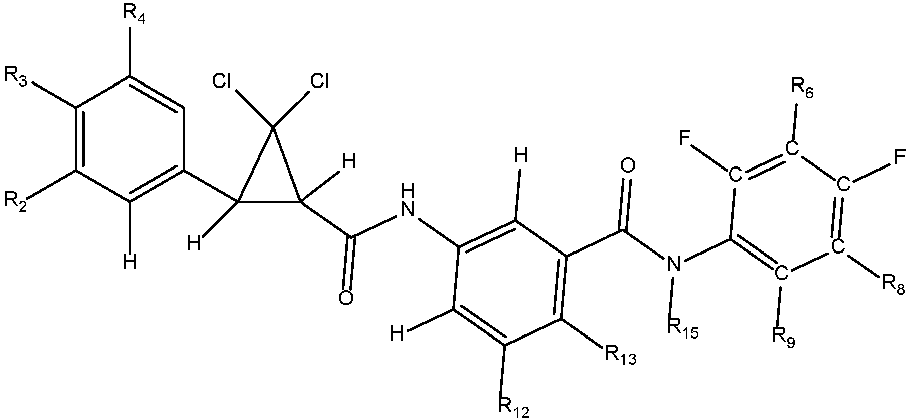
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (XL) 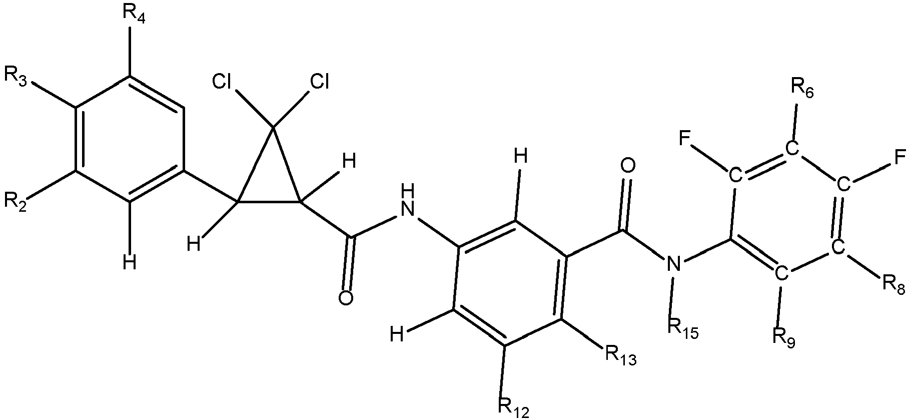
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁶** is selected from the group consisting of H, F, and NH(C₁-C₆)alkyl ;
**R⁸** is selected from the group consisting of H, and F ;
**R⁹** is selected from the group consisting of H and F;
**R¹²** is H ;
**R¹³ is** Cl;
R¹⁵ is H.

In an embodiment of the invention and/or embodiments thereof, the invention is directed to a compound of formula (XLI) 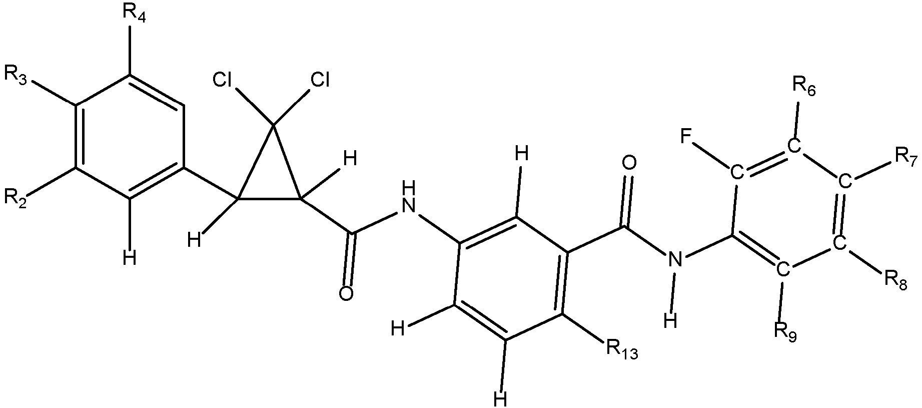
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, and R¹³ are defined as elsewhere in this description.

In an embodiment of the invention and/or embodiments thereof, the invention is directed to a compound of formula (XLI) 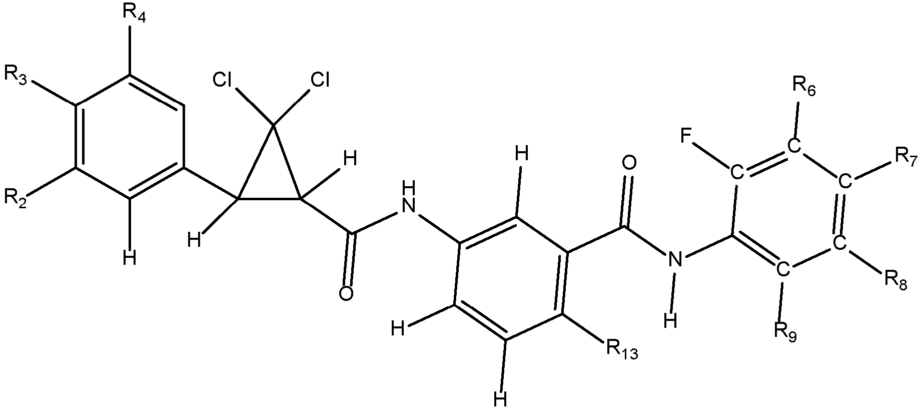
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R**⁶ and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R**⁶ and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R**⁷ and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XLI) 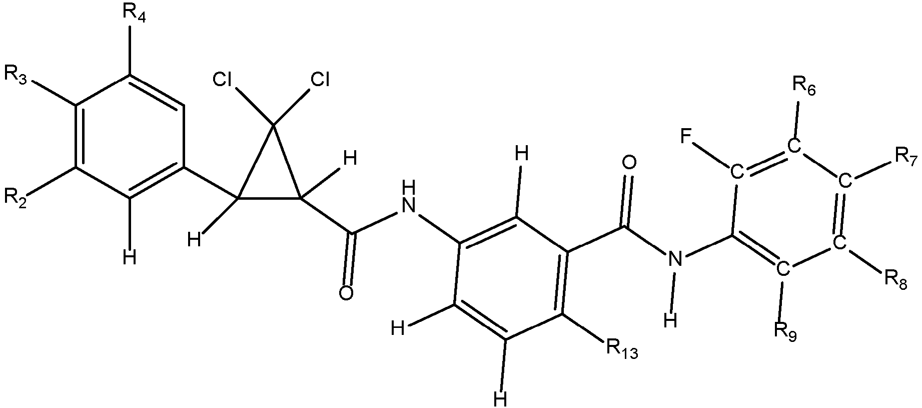
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and R⁸ is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃.

In an embodiment of the invention and/or embodiments thereof, the invention is directed to a compound of formula (XLI) 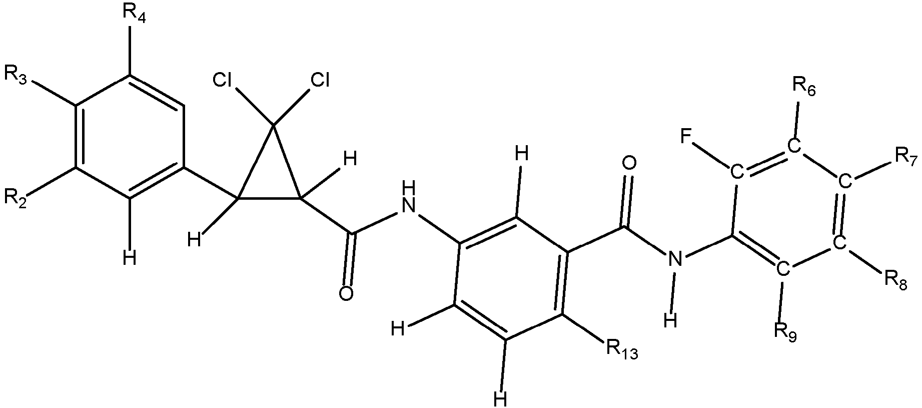
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R**⁶ and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃.

In an embodiment of the invention and/or embodiments thereof, the invention is directed to a compound of formula (XLI) 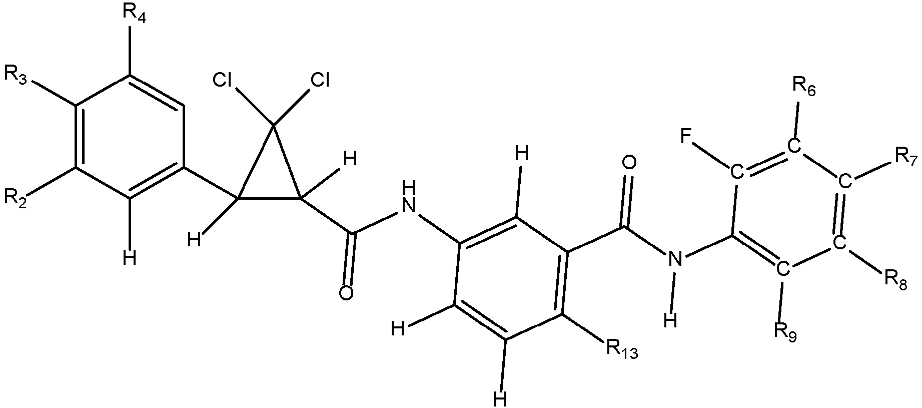
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of CH₃, F, and Cl;
**R⁸** is selected from the group consisting of H, F, and NH(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of CH₃, F, and Cl;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³ is** CI or F.

In an embodiment of the invention and/or embodiments thereof, the invention is directed to a compound of formula (XLI) 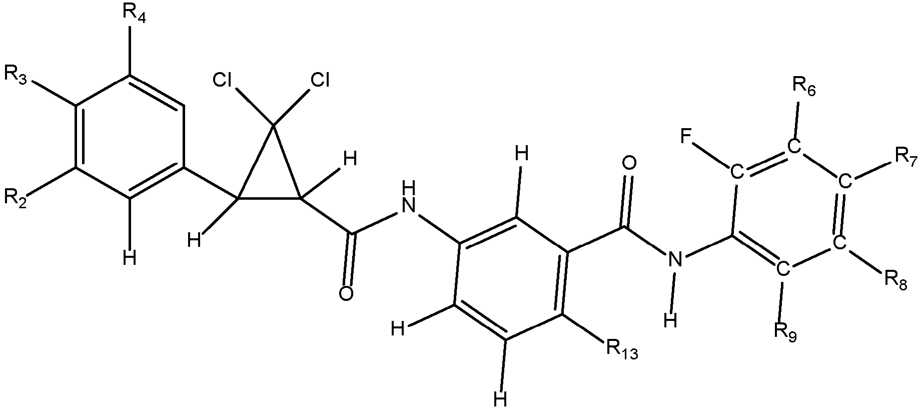
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹³** is Cl.

In an embodiment of the invention and/or embodiments thereof, the invention is directed to a compound of formula (XLII) 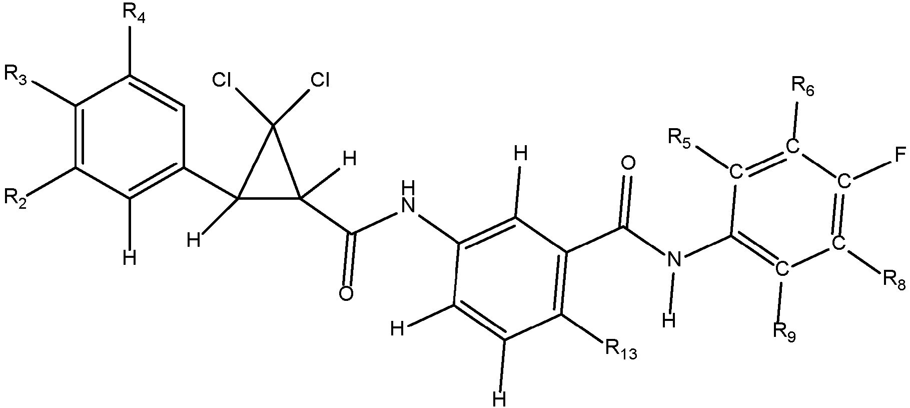
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁸, R⁹, and R¹³ are defined as elsewhere in this description.

In an embodiment of the invention and/or embodiments thereof, the invention is directed to a compound of formula (XLII) 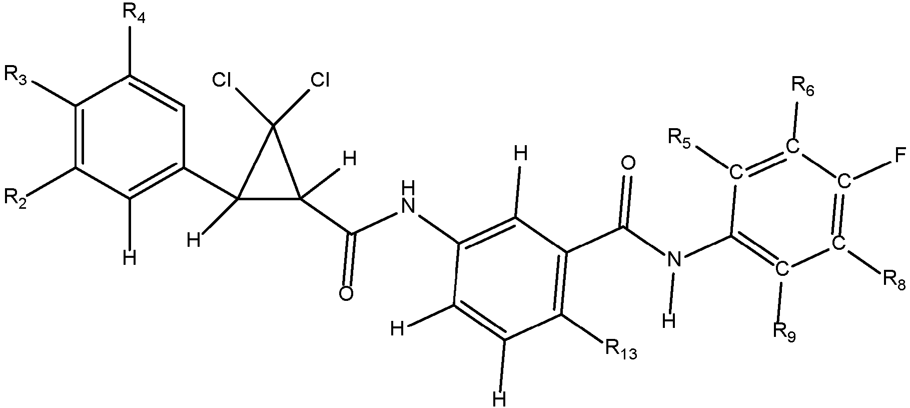
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₁-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XLII) 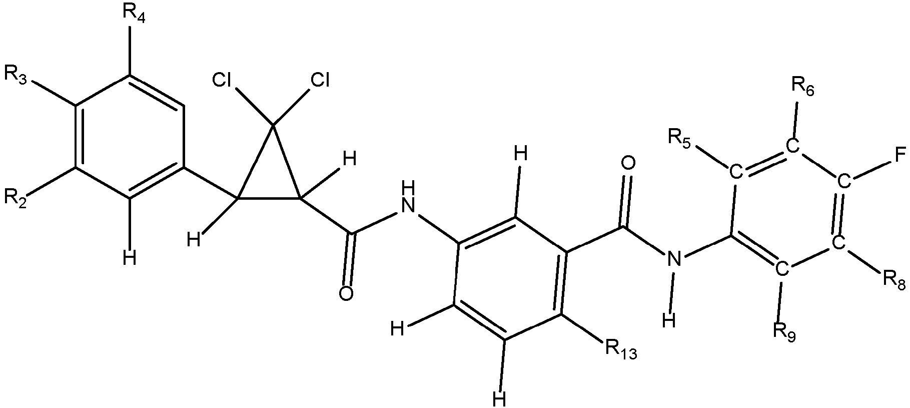
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃.

In an embodiment of the invention and/or embodiments thereof, the invention is directed to a compound of formula (XLII) 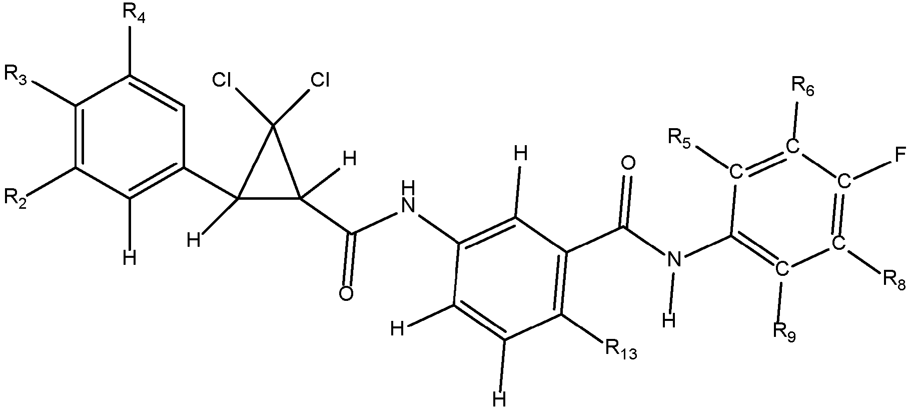
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃.

In an embodiment of the invention and/or embodiments thereof, the invention is directed to a compound of formula (XLII) 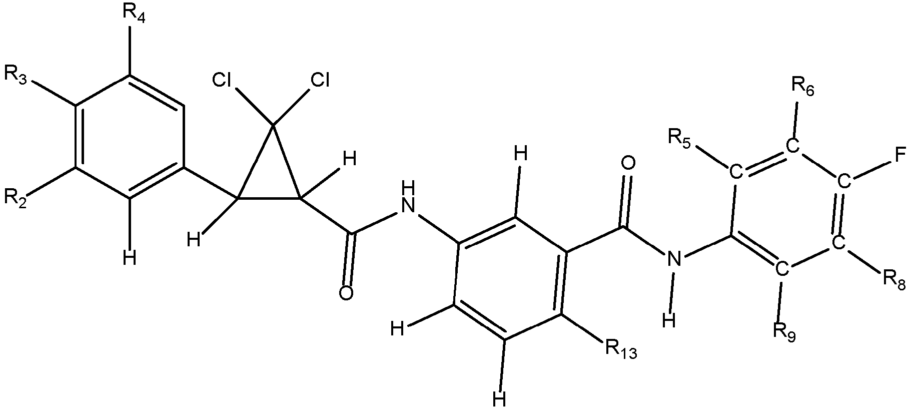
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F.

In an embodiment of the invention and/or embodiments thereof, the invention is directed to a compound of formula (XLII) 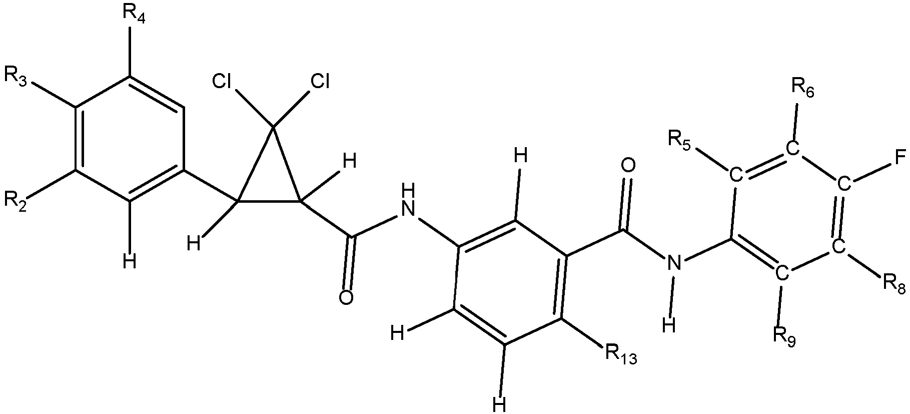
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹³** is Cl.

In an embodiment of the invention and/or embodiments thereof, the invention is directed to a compound of formula (XLIII) 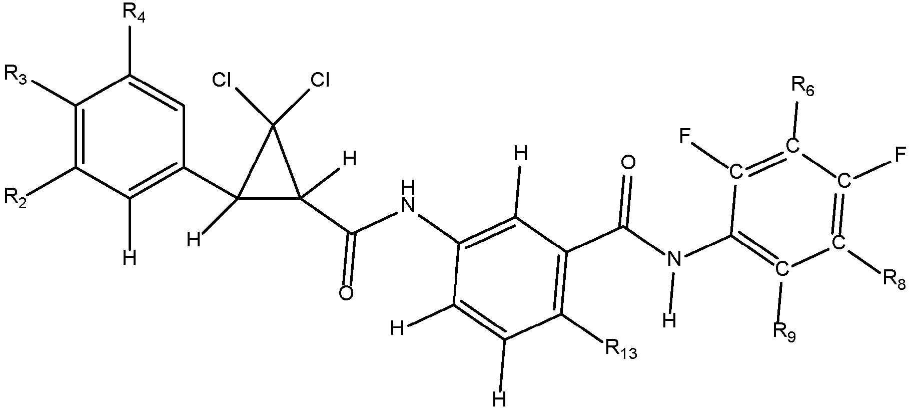
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R³, R⁹, and R¹³ are defined as elsewhere in this description.

In an embodiment of the invention and/or embodiments thereof, the invention is directed to a compound of formula (XLIII) 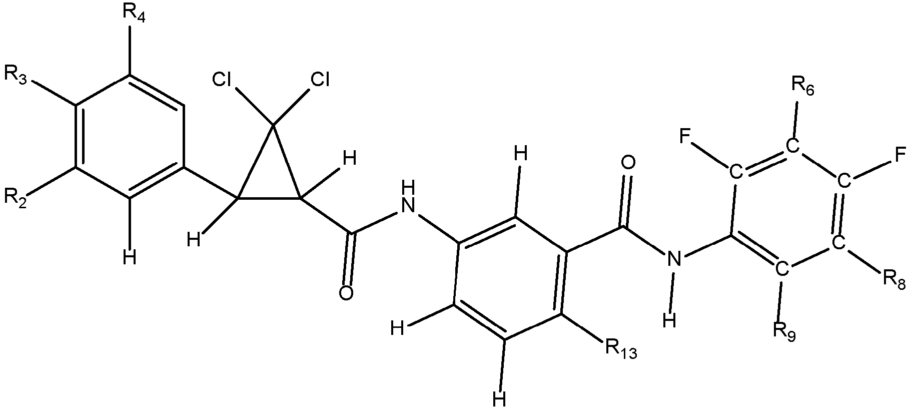
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (XLIII) 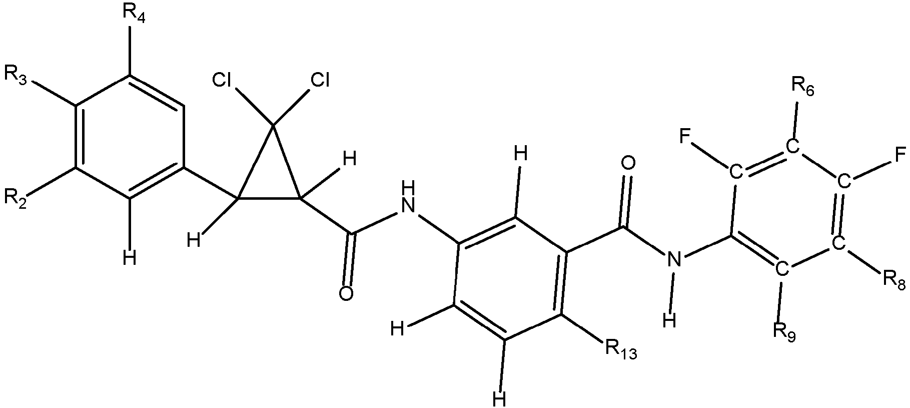
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃.

In an embodiment of the invention and/or embodiments thereof, the invention is directed to a compound of formula (XLIII) 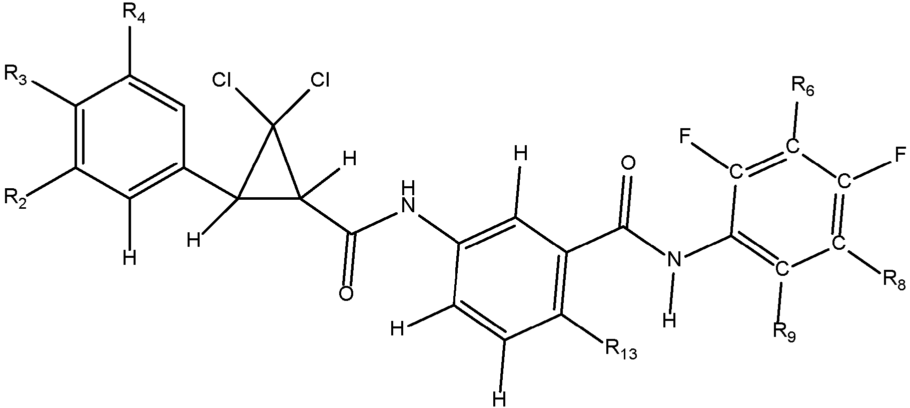
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃.

In an embodiment of the invention and/or embodiments thereof, the invention is directed to a compound of formula (XLIII) 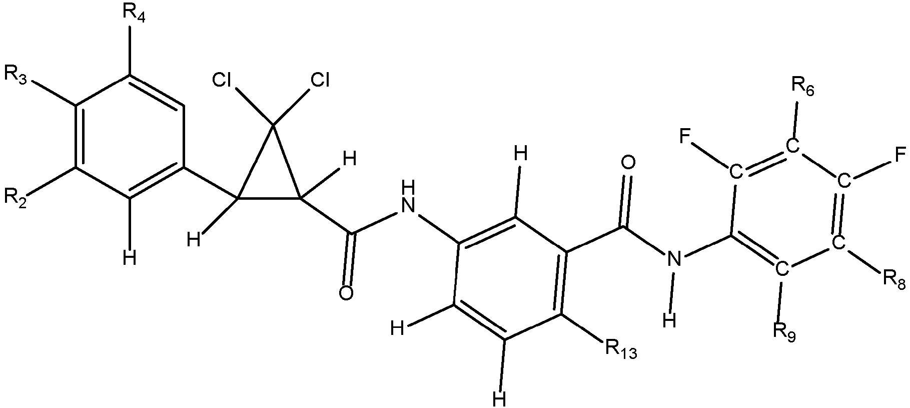
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is CI or F.

In an embodiment of the invention and/or embodiments thereof, the invention is directed to a compound of formula (XLIII) 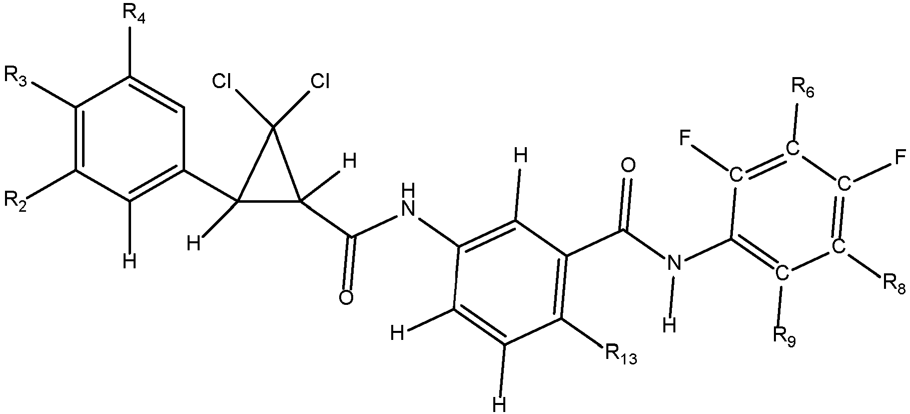
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹³** is Cl.

In an embodiment of the invention and/or embodiments thereof, the invention is directed to a compound of formula (XLIV) 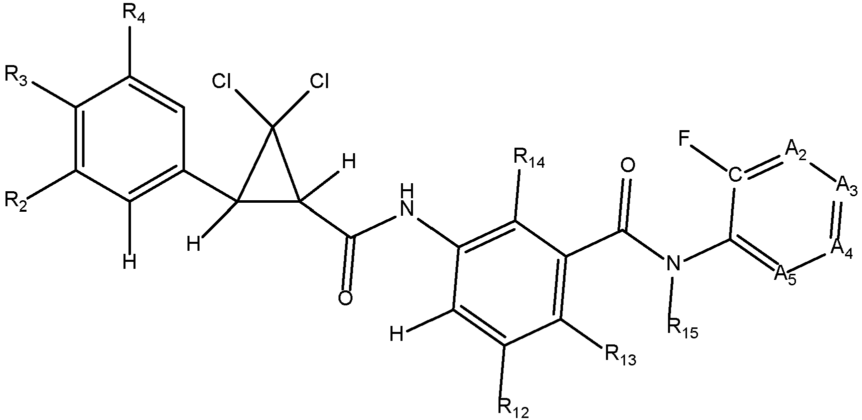
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁷, R⁸, R⁹, R¹², R¹³, R¹⁴, R¹⁵, A₂, A₃, A₄, and A₅ are defined as elsewhere in this description.

In an embodiment of the invention and/or embodiments thereof, the invention is directed to a compound of formula (XLIV) 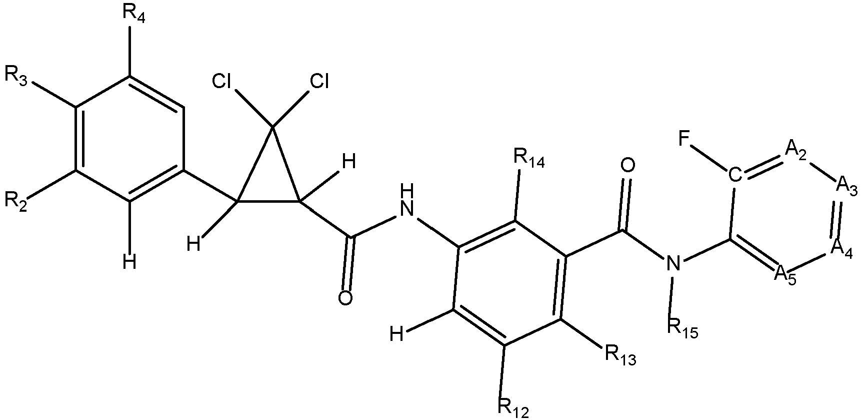
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₃ is CR₇, N=O, or N;
A₄ is CR₈, N=O or N;
A₅ is CR₉, N=O or N ;
wherein no more than 2 of A₂, A₃, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XLIV) 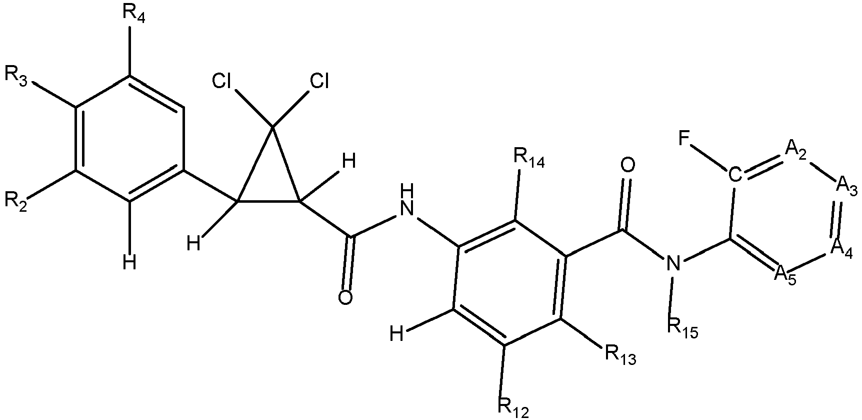
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₃ is CR₇, N=O, or N;
A₄ is CR₈, N=O or N;
A₅ is CR₉, N=O or N;
wherein no more than 2 of A₁, A₂, A₃, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁶** and **R⁷** is optionally substituted with one or more substituents of selected from the group consisting of (C₁-C₃)alkyl, F, Cl, Br, I, CN;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, and -O-, wherein the ring formed by **R⁷** and **R⁸** is optionally substituted with one or more substituents of selected from the group consisting of (C₁-C₃)alkyl, F, Cl, Br, I, and CN;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLIV) 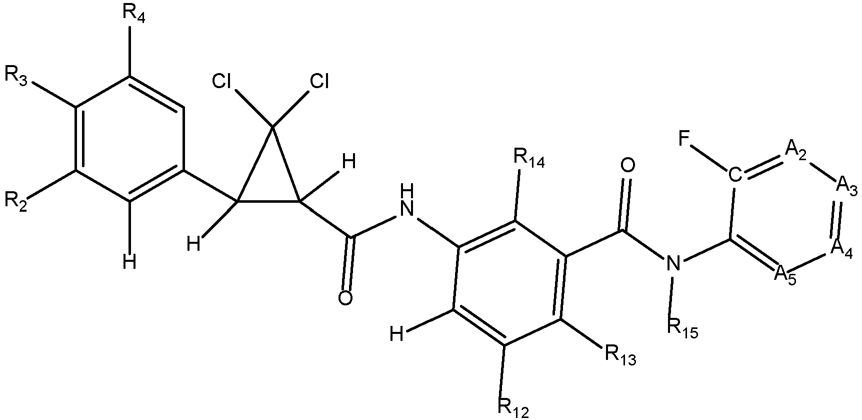
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₃ is CR₇ or N;
A₄ is CR₈ or N;
A₅ is CR₉ or N;
wherein no more than 2 of A₂, A₃, A₄, A₅ are N;
R⁶ is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁷** is selected from the group consisting of H, F, Cl, NH(C₁-C₆)alkyl, N((C₁-C₆)alky)₂, (C₁-C₆)alkyl, (C₁-C₆)alkoxy, and (C₁-C₆)haloalkyl ;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁶** and **R⁷** is optionally substituted with one or more substituents of selected from the group consisting of (C₁-C₃)alkyl, F, and Cl;
**R⁸** is selected from the group consisting of H, F, Cl, NH(C₁-C₆)alkyl, and (C₁-C₆)alkyl ;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by **R⁷** and **R⁸** is optionally substituted with one or more substituents of selected from the group consisting of (C₁-C₃)alkyl, F, and Cl;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLIV) 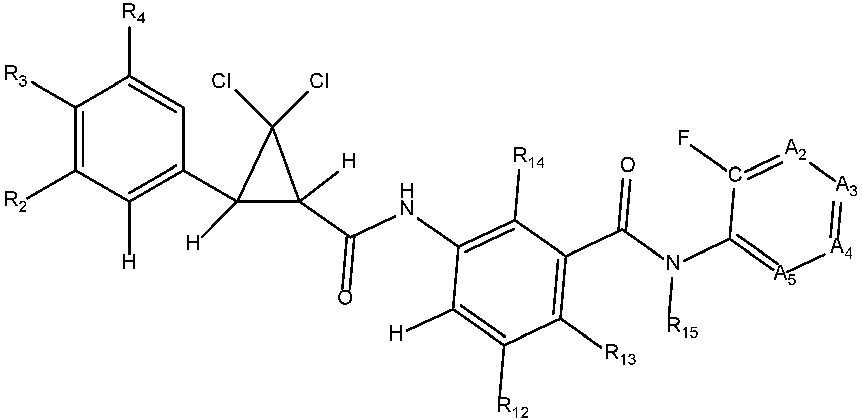
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₂ is CR₆ or N;
A₃ is CR₇ or N;
A₄ is CR₈ or N;
A₅ is CR₉ or N;
wherein 0 or 1 of A₂, A₃, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁷** is selected from the group consisting of H, F, and Cl ;
or R⁶ and R⁷ together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by R⁶ and R⁷ is optionally substituted with one or more substituents of selected from the group consisting of CH₃, F, and Cl;
R⁸ is selected from the group consisting of H, F, and NH(C₁-C₆)alkyl ;
or R⁷ and R⁸ together with the atoms to which they are attached form a 5 or 6- atoms containing ring, wherein the ring-forming atoms are selected from the group consisting of -CH₂-, -CH=, =CH-,-N=, =N-, and -NH-, wherein the ring formed by R⁷ and R⁸ is optionally substituted with one or more substituents of selected from the group consisting of CH₃, F, and Cl;
R⁹ is selected from the group consisting of H, F, and NH(C₁-C₆)alkyl ;
R¹⁰ is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XLIV) 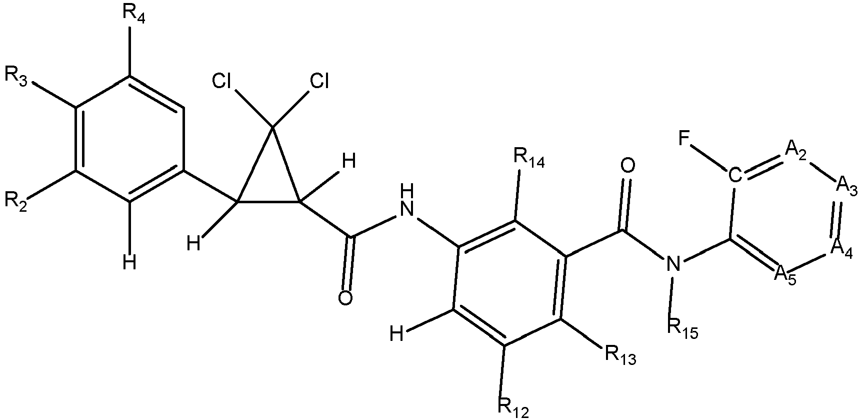
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₂ is CR₆ or N;
A₃ is CR₇;
A₄ is CR₈;
A₅ is CR₉;
**R⁶** is selected from the group consisting of H, F, and NH(C₁-C₆)alkyl ;
**R⁷** is selected from the group consisting of H, and F ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H.

In an embodiment of the invention and/or embodiments thereof, the invention is directed to a compound of formula (XLV) 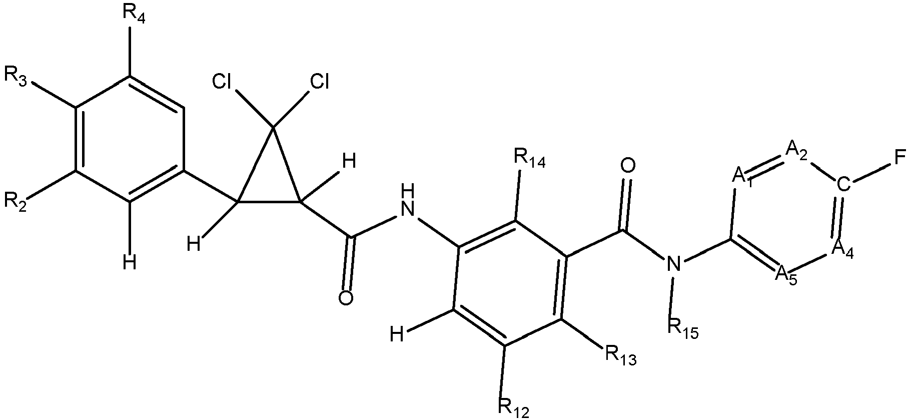
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁸, R⁹, R¹², R¹³, R¹⁴, R¹⁵, A₁, A₂, A₄, and A₅ are defined as elsewhere in this description.

In an embodiment of the invention and/or embodiments thereof, the invention is directed to a compound of formula (XLV) 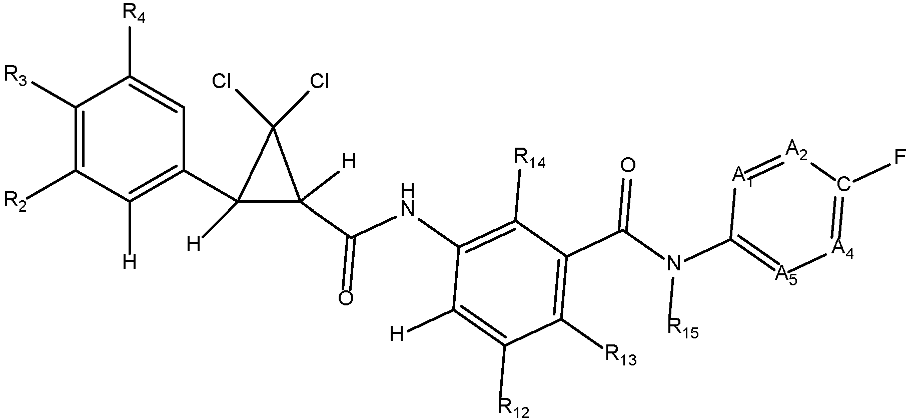
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₄ is CR₈, N=O or N;
A₅ is CR₉, N=O or N ;
wherein no more than 2 of A₁, A₂, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XLV) 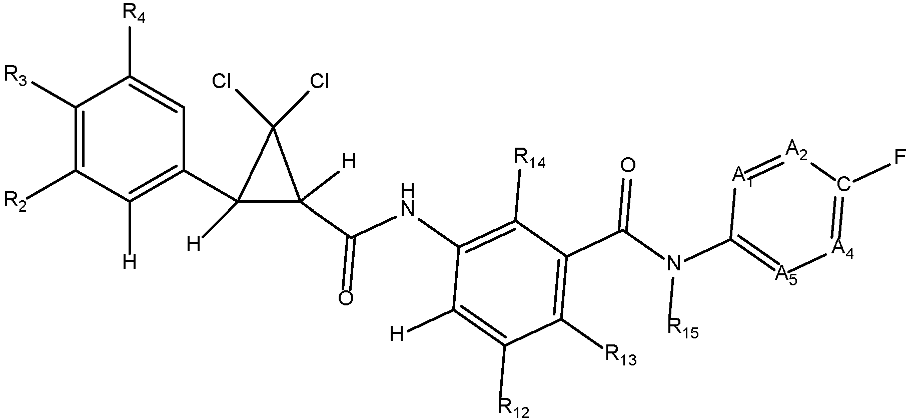
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₄ is CR₈, N=O or N;
A₅ is CR₉, N=O or N;
wherein no more than 2 of A₁, A₂, A₄, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLV) 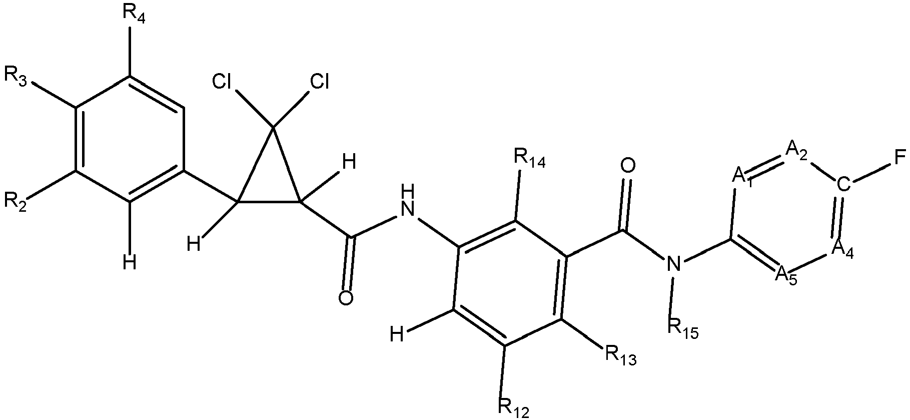
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₄ is CR₈ or N;
A₅ is CR₉ or N;
wherein no more than 2 of A₁, A₂, A₄, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLV) 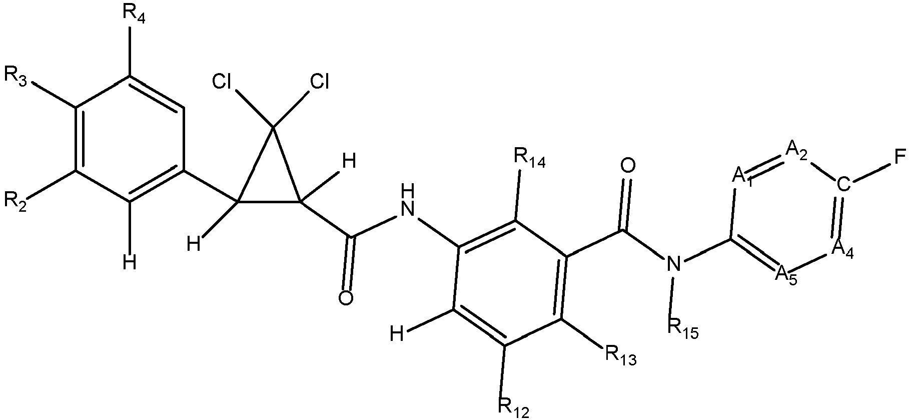
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₄ is CR₈ or N;
A₅ is CR₉ or N;
wherein 0 or 1 of A₁, A₂, A₄, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XLV) 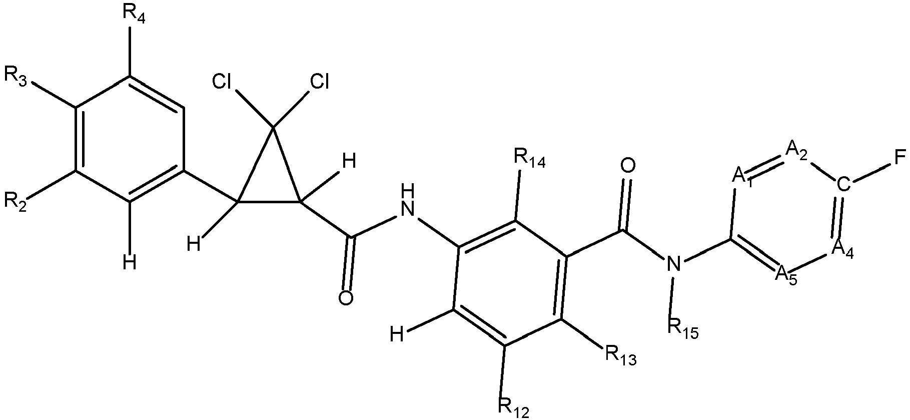
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A₂ is CR₆ or N;
A₄ is CR₈;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H.

In an embodiment of the invention and/or embodiments thereof, the invention is directed to a compound of formula (XLVI) 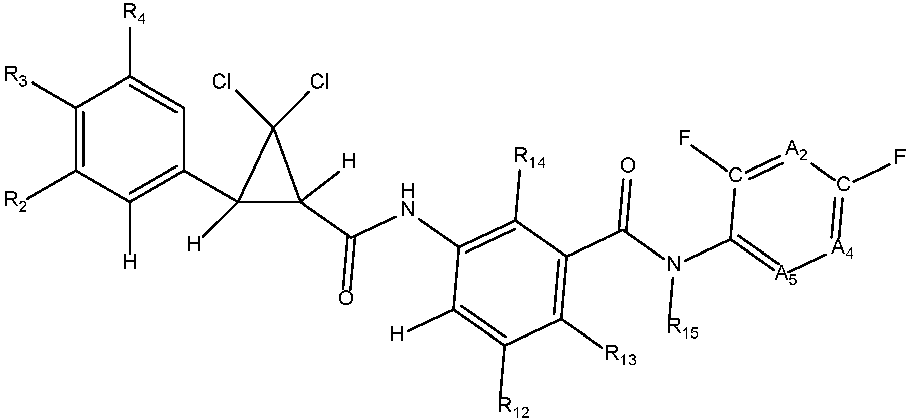
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, R⁹, R¹², R¹³, R¹⁴, R¹⁵, A₂, A₄, and A₅ are defined as elsewhere in this description.

In an embodiment of the invention and/or embodiments thereof, the invention is directed to a compound of formula (XLVI) 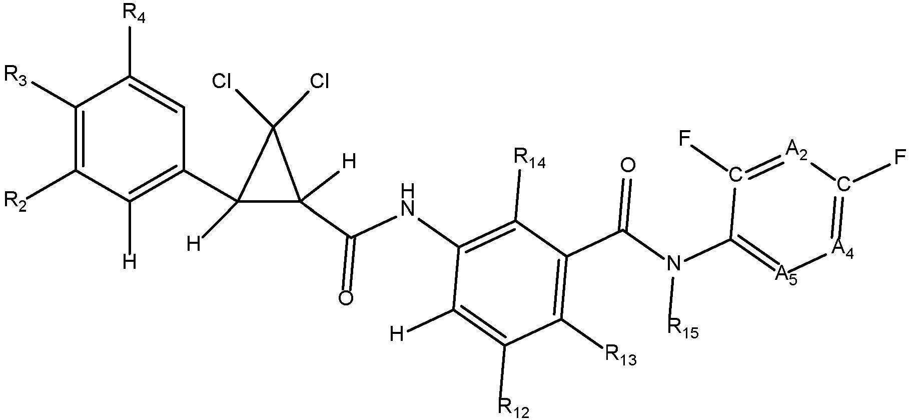
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₄ is CR₈, N=O or N;
A₅ is CR₉, N=O or N ;
wherein no more than 2 of A₂, A₄, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (XLVI) 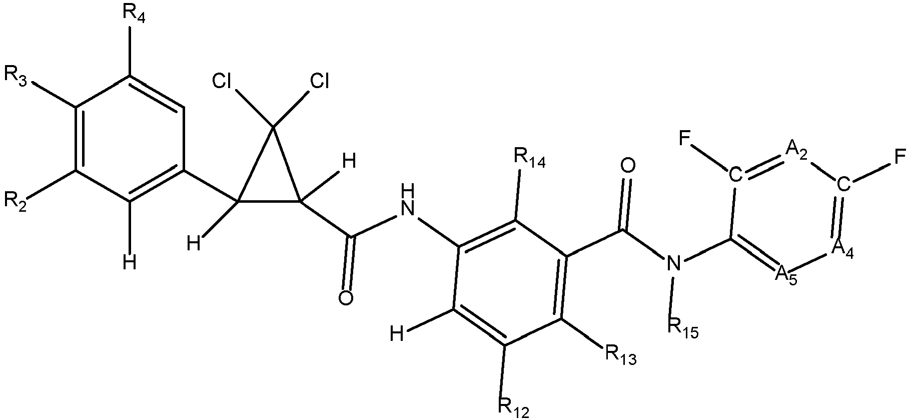
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₄ is CR₈, N=O or N;
A₅ is CR₉, N=O or N;
wherein no more than 2 of A₂, A₄, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLVI) 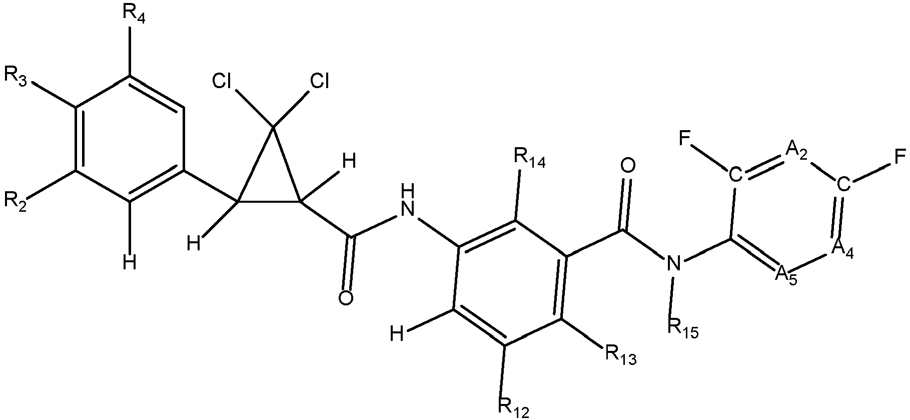
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₄ is CR₈ or N;
A₅ is CR₉ or N;
wherein no more than 2 of A₂, A₄, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (XLVI) 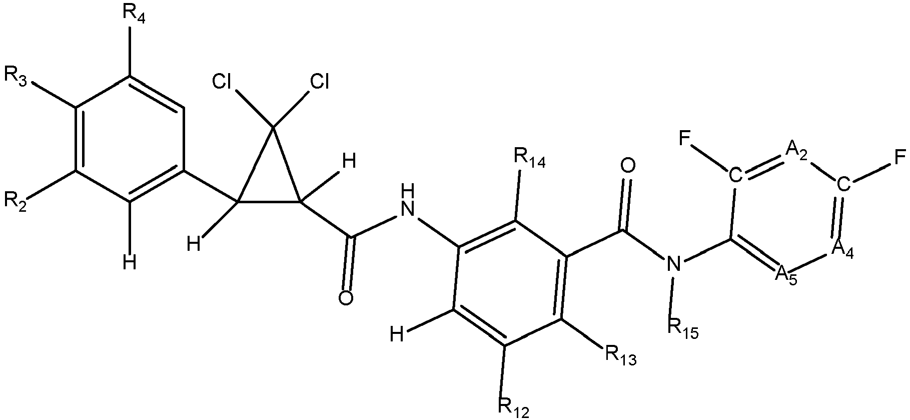
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A2 is CR₆ or N;
A₄ is CR₈ or N;
A₅ is CR₉ or N;
wherein 0 or 1 of A₂, A₄, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (XLVI) 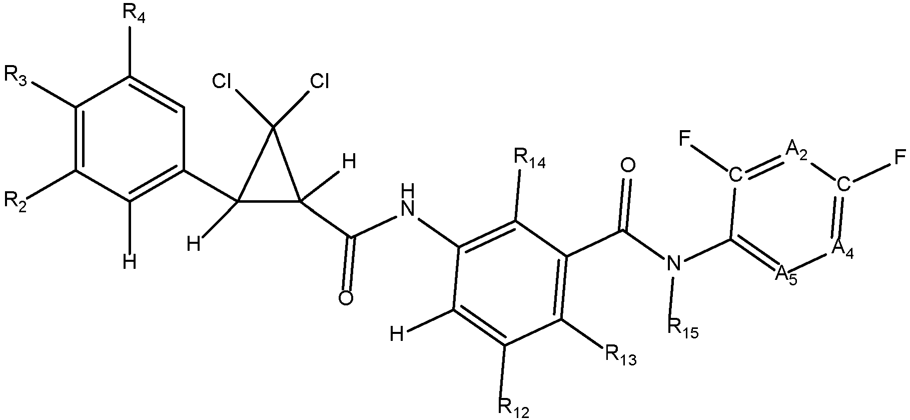
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A2 is CR₆ or N;
A₄ is CR₈;
A₅ is CR₉;
**R⁶** is selected from the group consisting of H, F, and NH(C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H.

In a particular embodiment of the invention and/or embodiments thereof, the invention is directed to a compound of formula (XLVII) 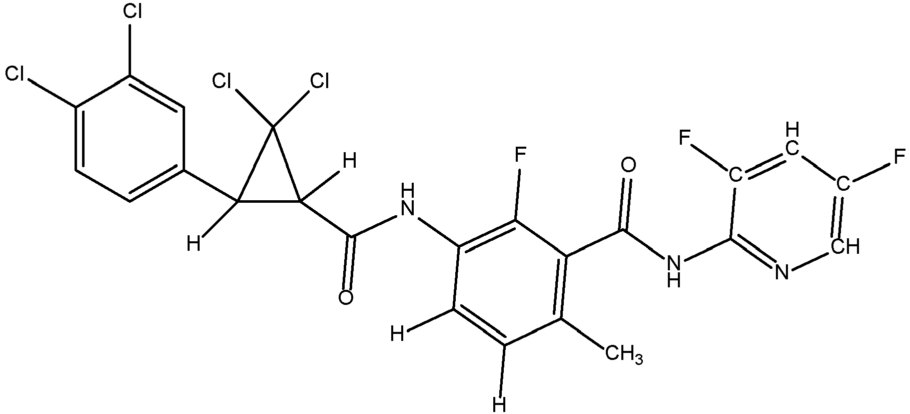 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (XLVlla) 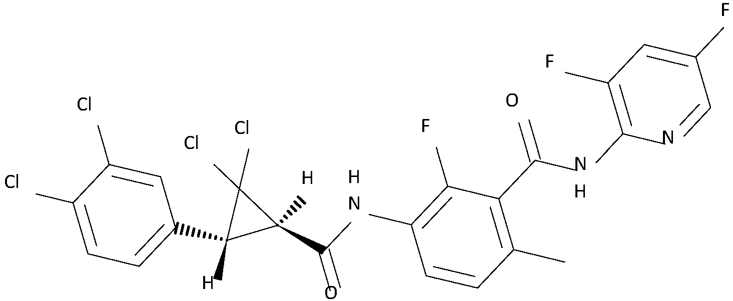 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (XLVIII) 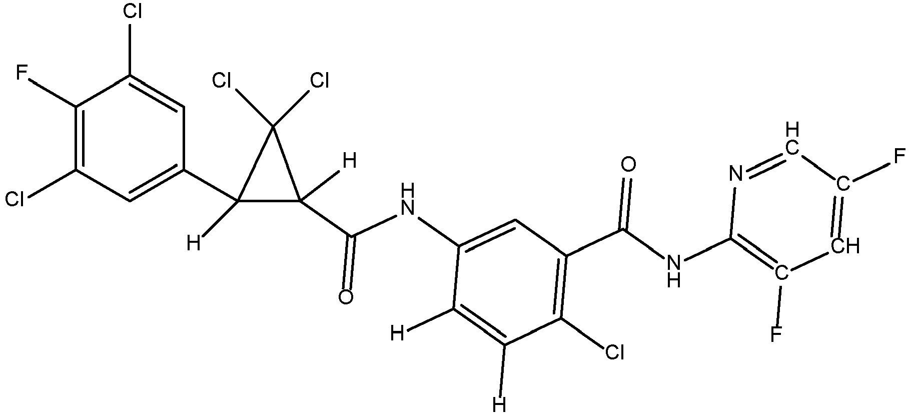 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (XLVIIIa) 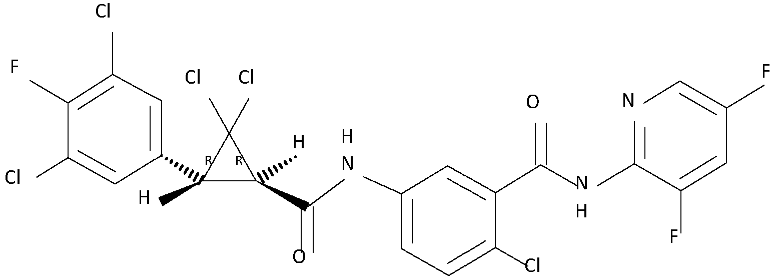 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (XLIX) 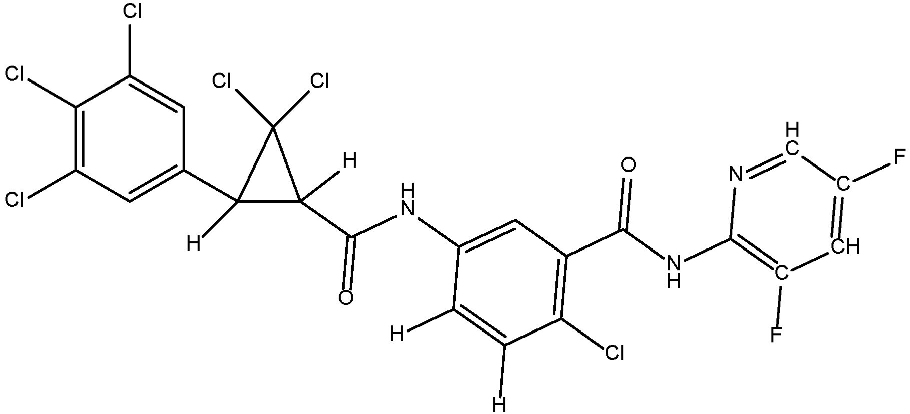 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (XLIXa) 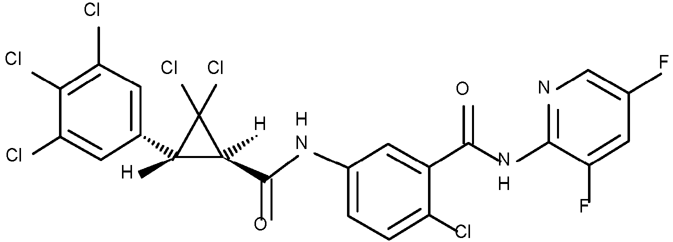 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (L) 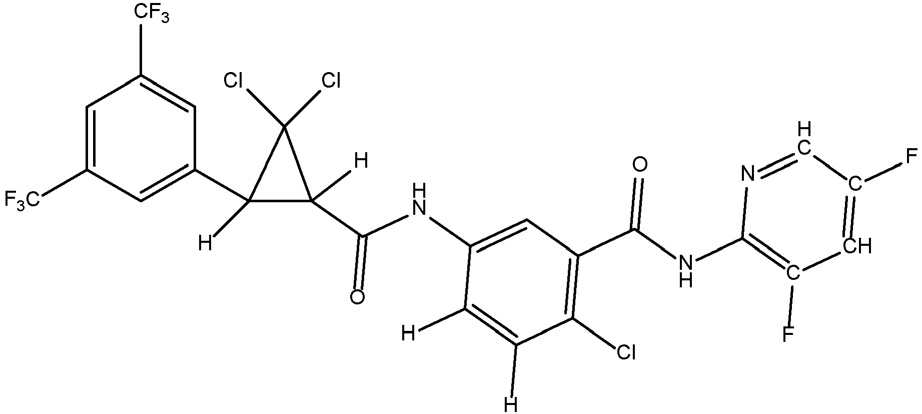 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (La) 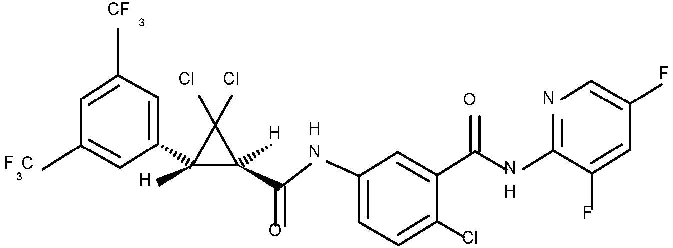 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LI) 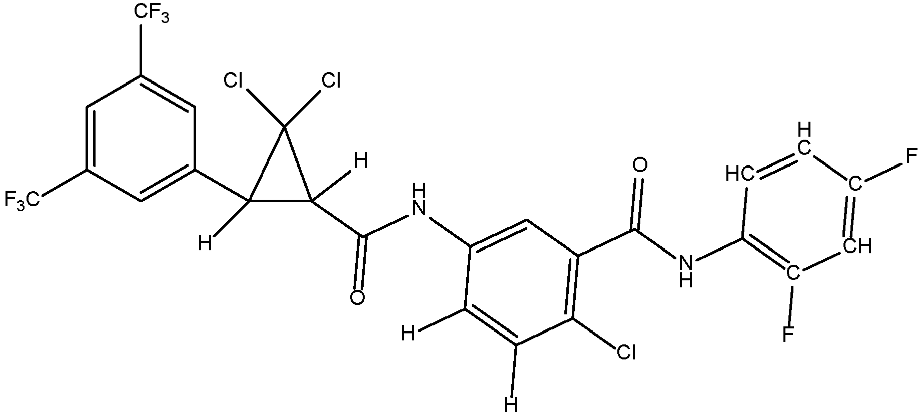 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (Lla) 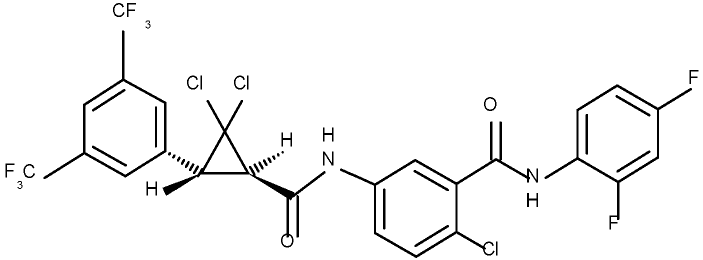 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LII) 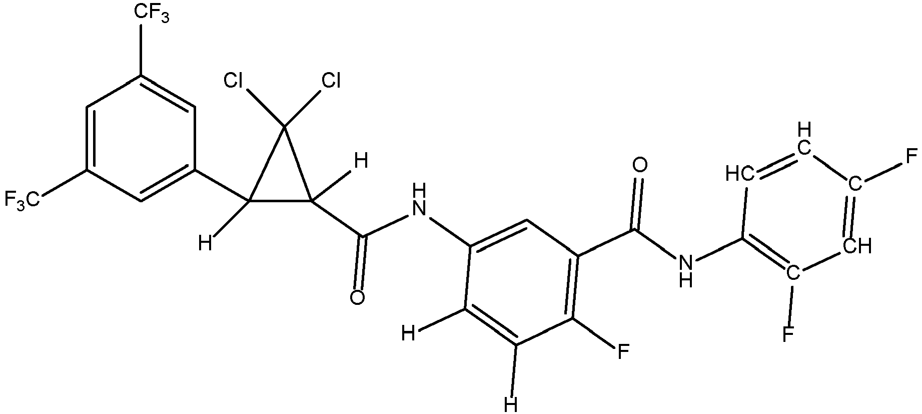 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (Llla) 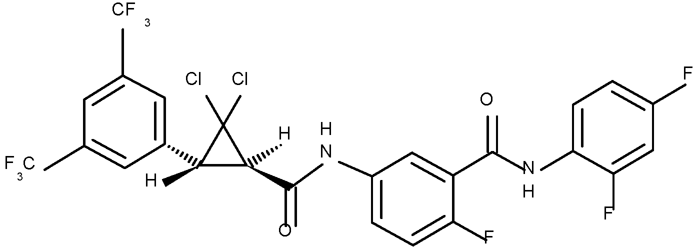 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIII) 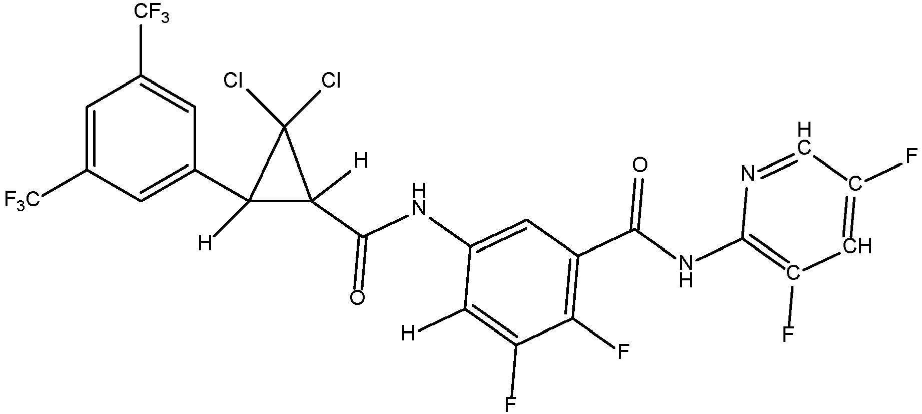 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (Lllla) 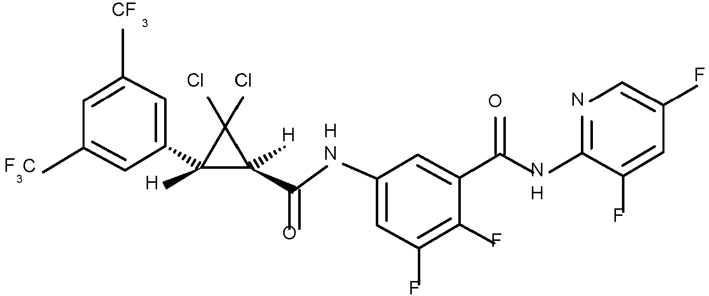 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIV) 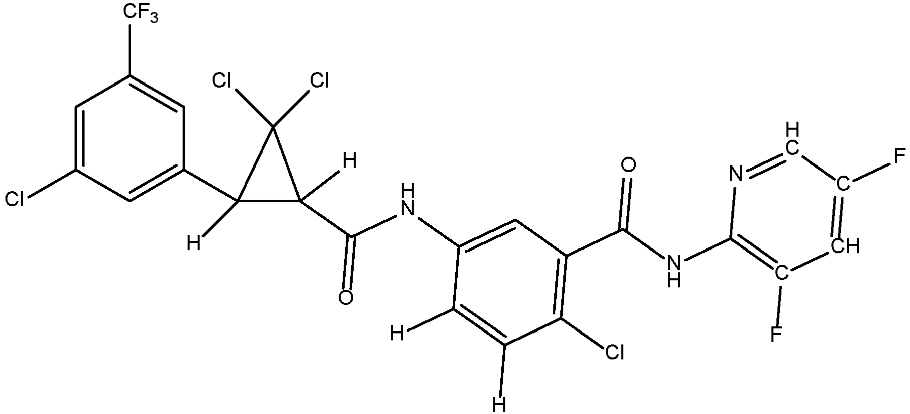 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIVa) 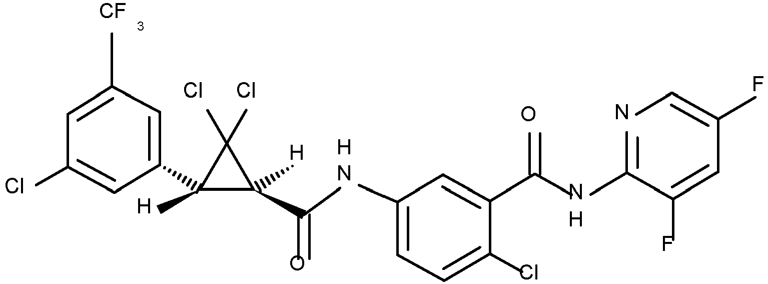 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIVb) 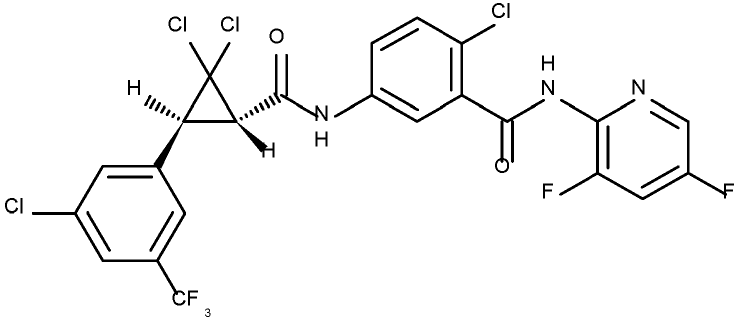 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LV) 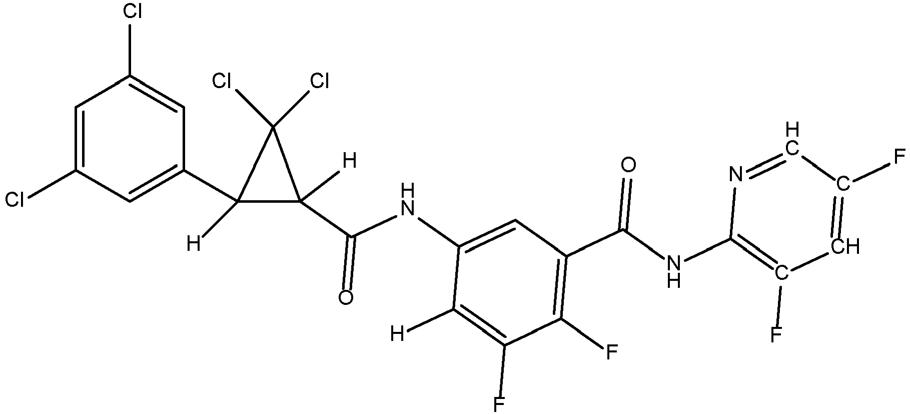 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVa) 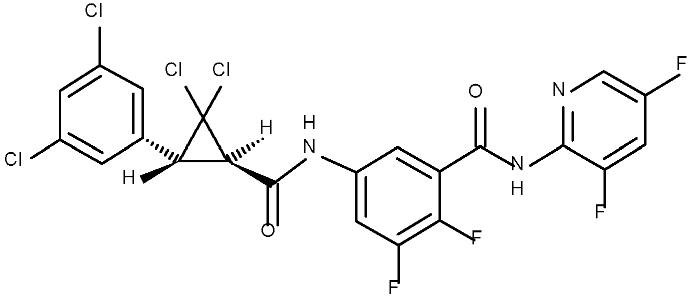 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVI) 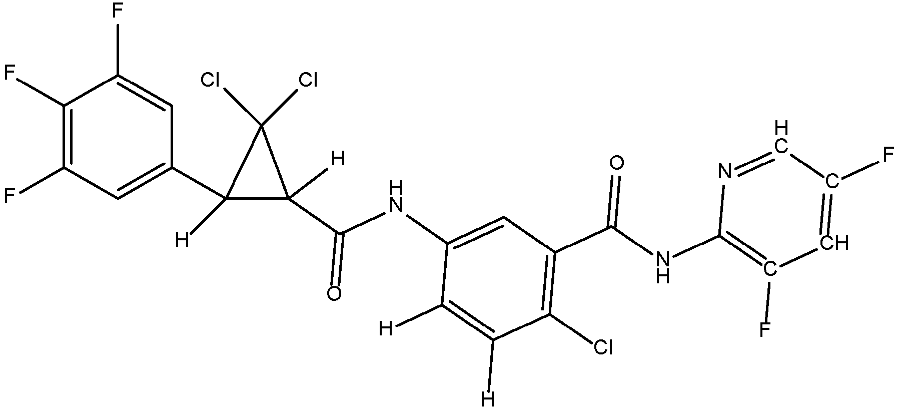 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVIa) 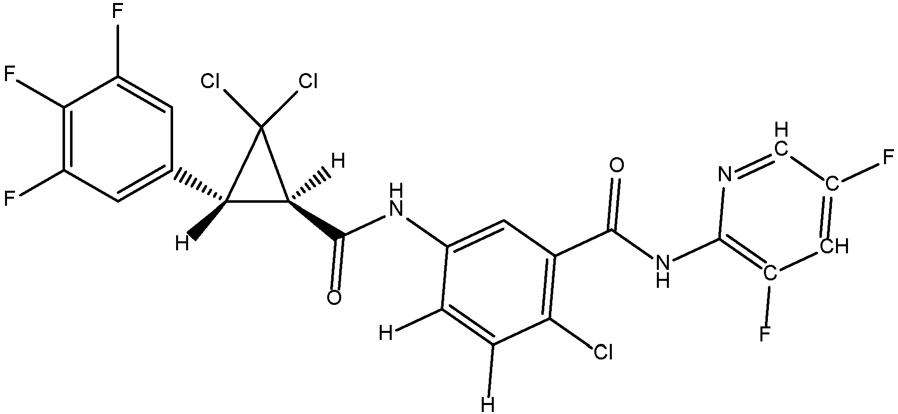 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVII) 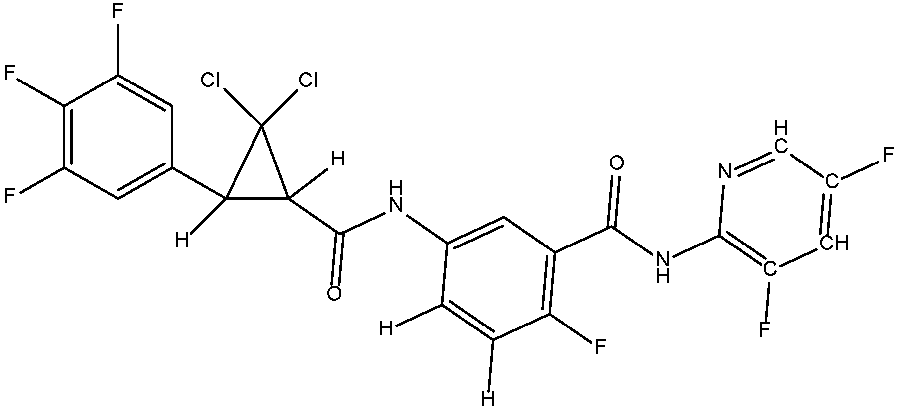 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVlla) 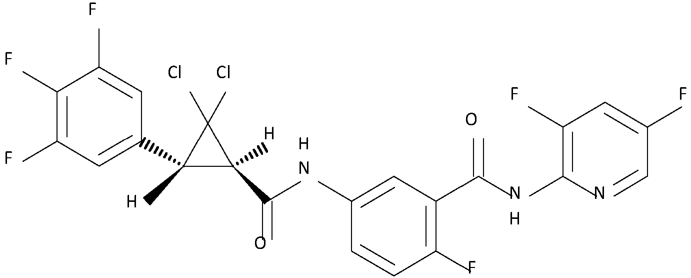 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVIII) 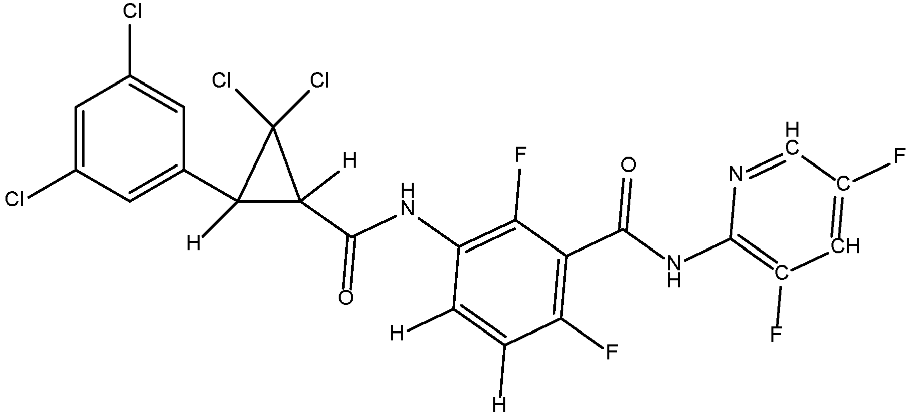 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LVllla) 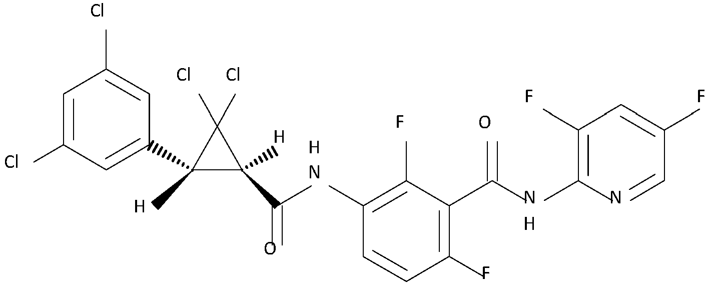 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIX) 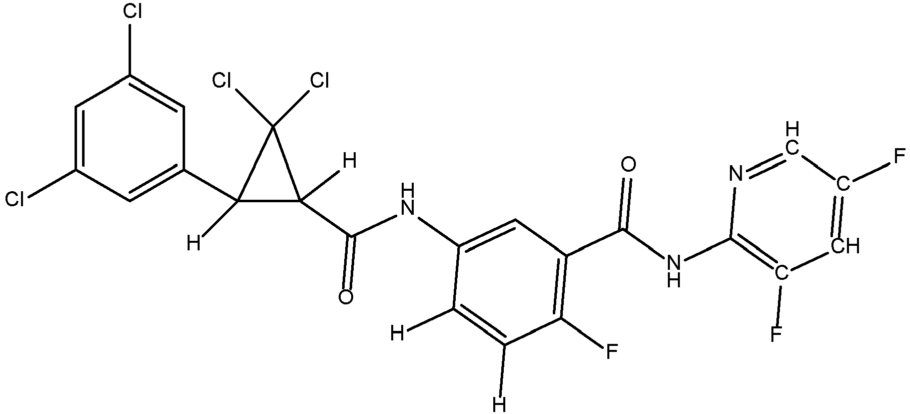 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LIX) 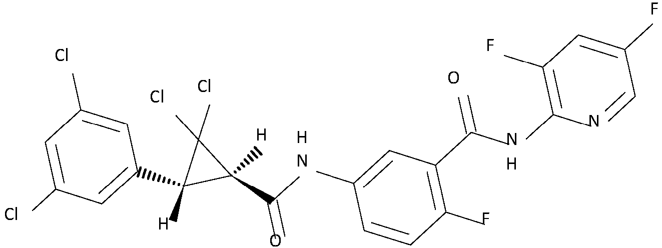 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LX) 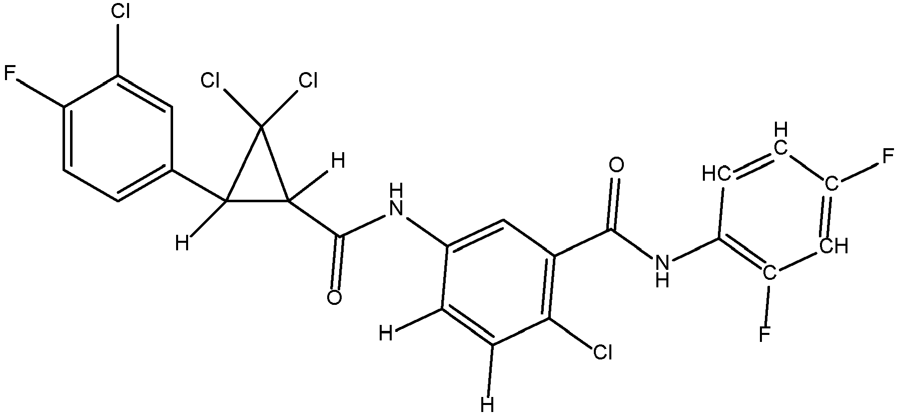 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXa) 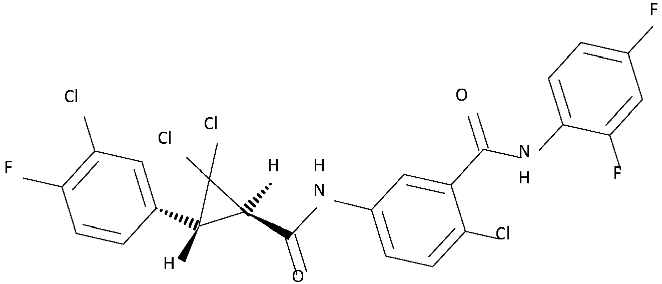 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXI) 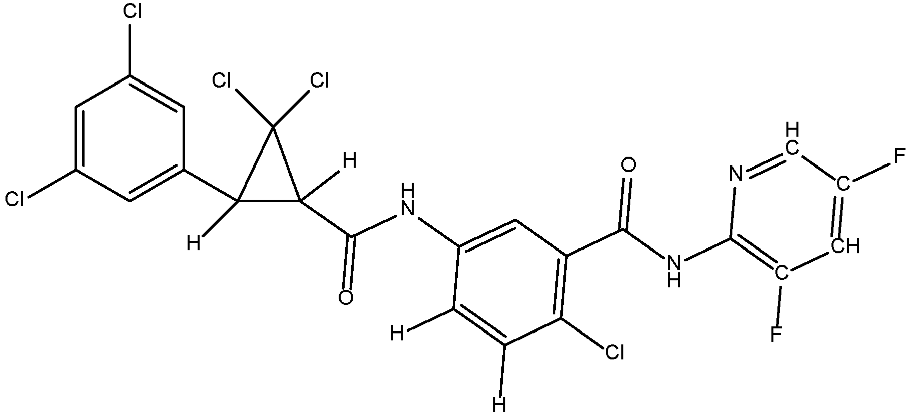 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXla) 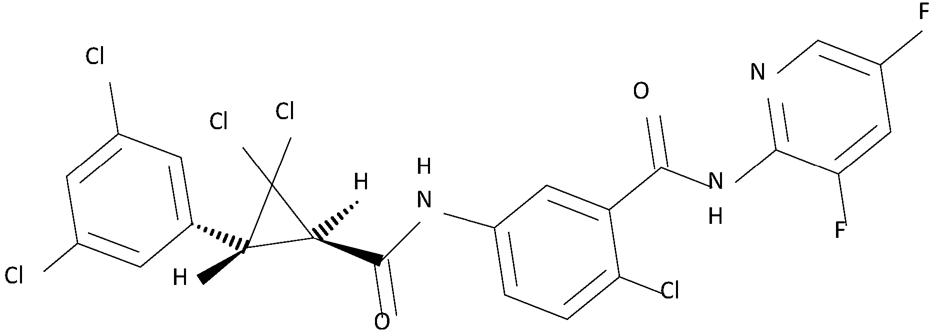 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXII) 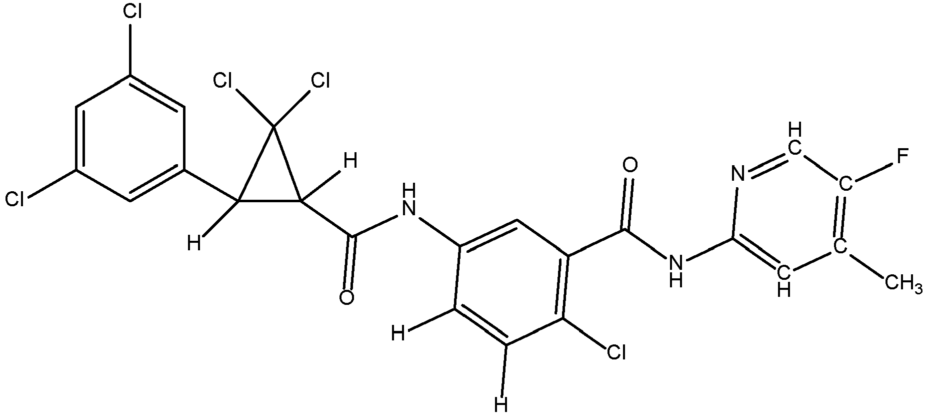 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXlla) 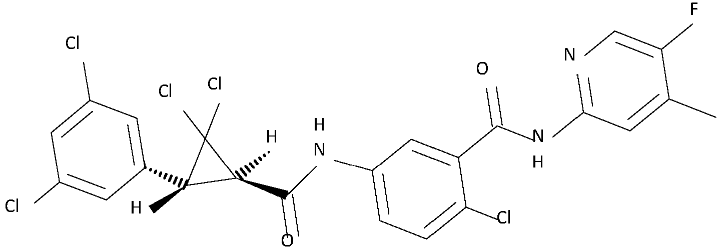 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXIII) 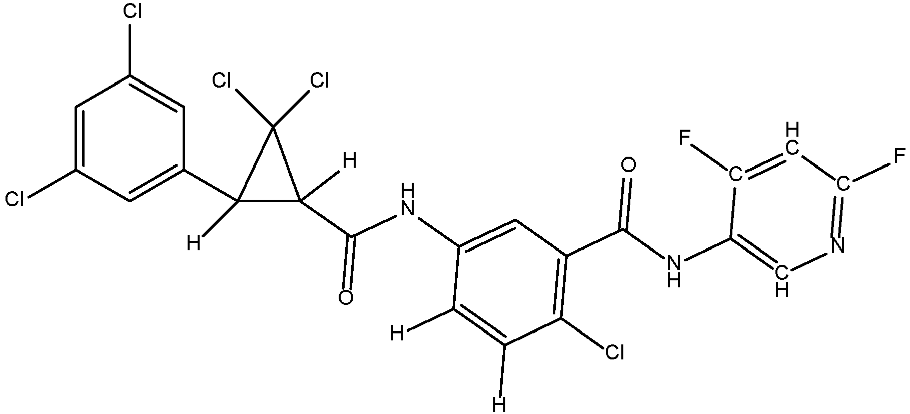 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXllla) 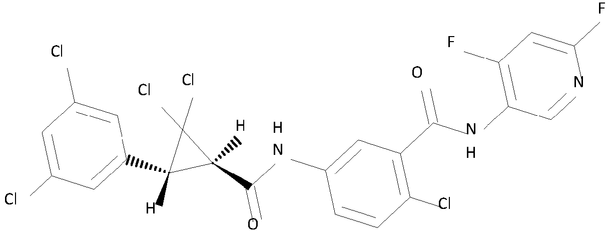 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXIV)
and N-oxides, veterinary acceptable acid addition salts, salt derivatives, solvates, ester derivatives, crystal polymorphs, isotopes, stereoisomers, and tautomers thereof.

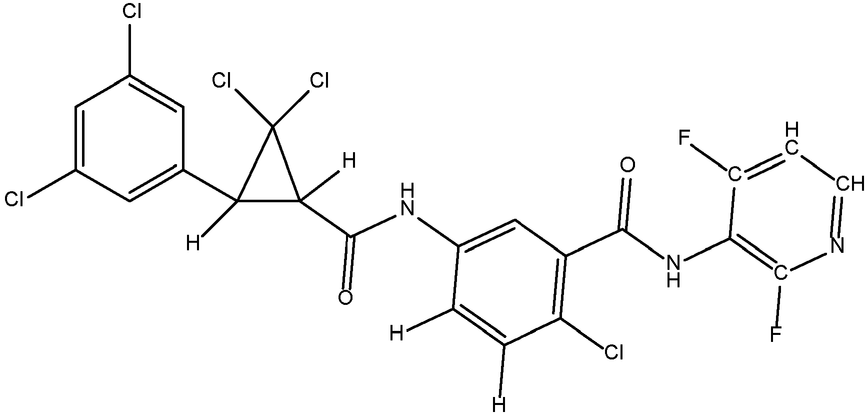

In a particular embodiment of the invention and/or embodiments thereof, the invention is directed to a compound of formula (LXIVa) 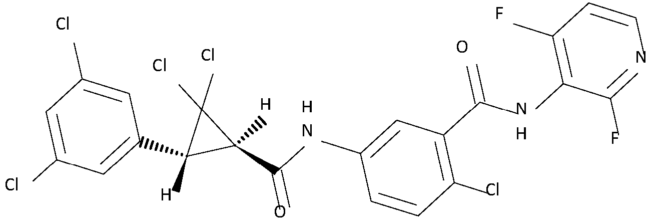 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXV) 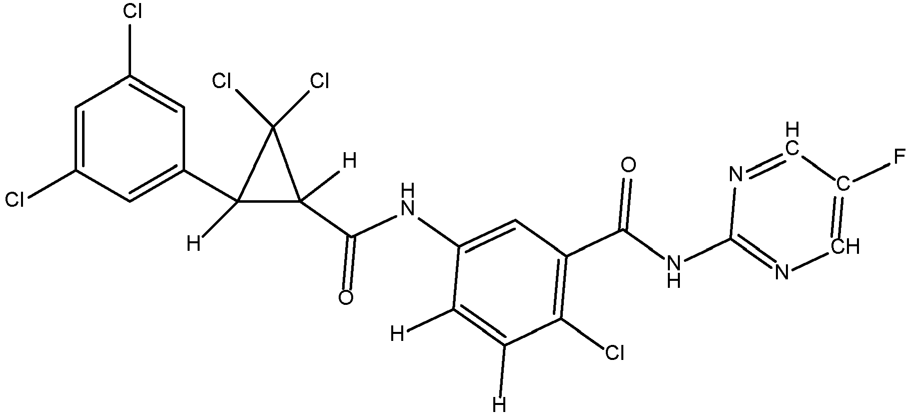 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXVa) 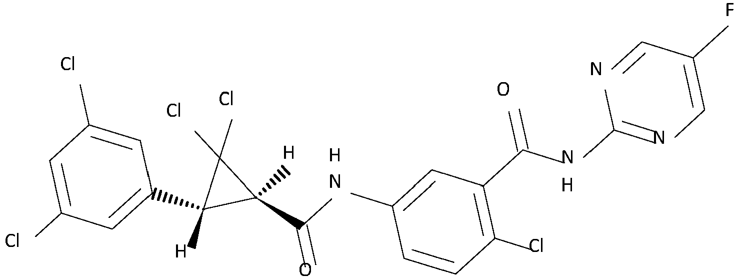 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXVI) 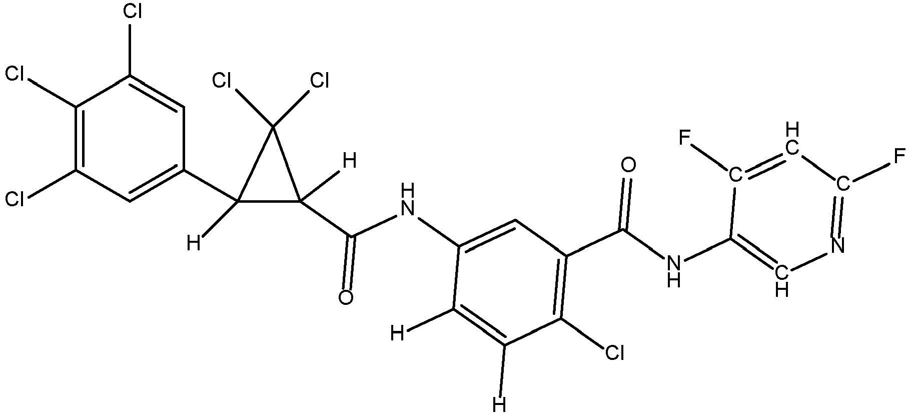 and N-oxides, veterinary acceptable acid addition salts, salt derivatives, solvates, ester derivatives, crystal polymorphs, isotopes, stereoisomers, and tautomers thereof.

In a particular embodiment of the invention and/or embodiments thereof, the invention is directed to a compound of formula (LXVla) 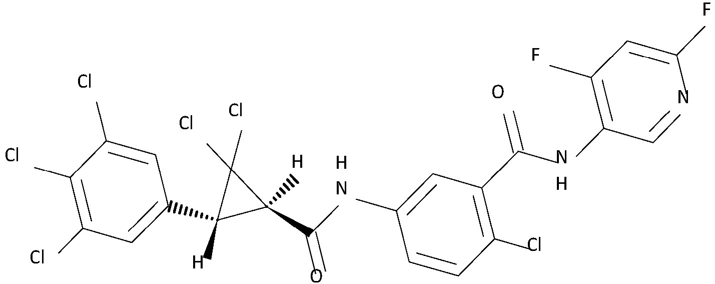 and N-oxides, veterinary acceptable acid addition salts, salt derivatives, solvates, ester derivatives, crystal polymorphs, isotopes, stereoisomers, and tautomers thereof.

In an embodiment of the invention and/or embodiments thereof, the invention is directed to a compound of formula (LXVII) 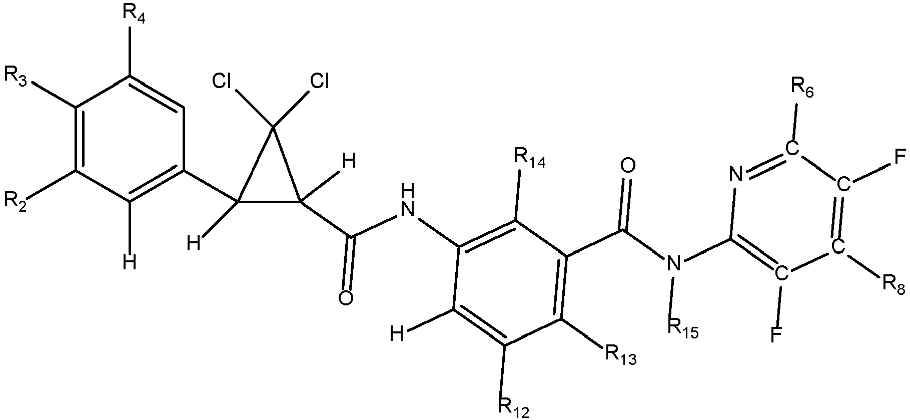
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R³, R¹², R¹³, R¹⁴, and R¹⁵, are defined as elsewhere in this description.

In an embodiment of the invention and/or embodiments thereof, the invention is directed to a compound of formula (LXVII) 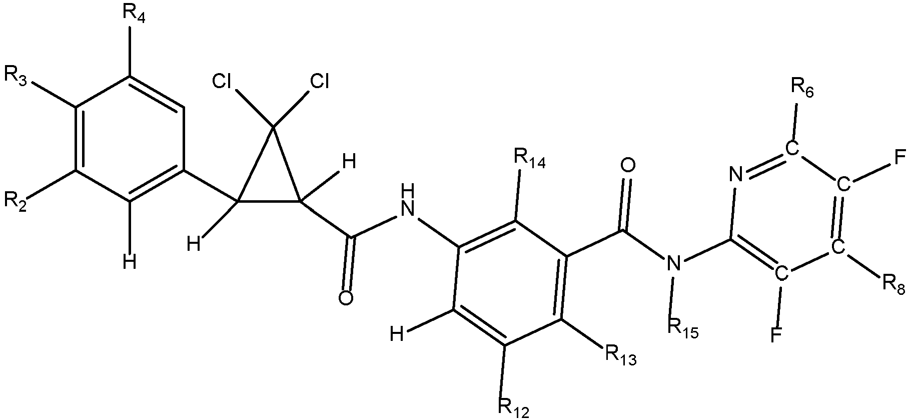
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXVII) 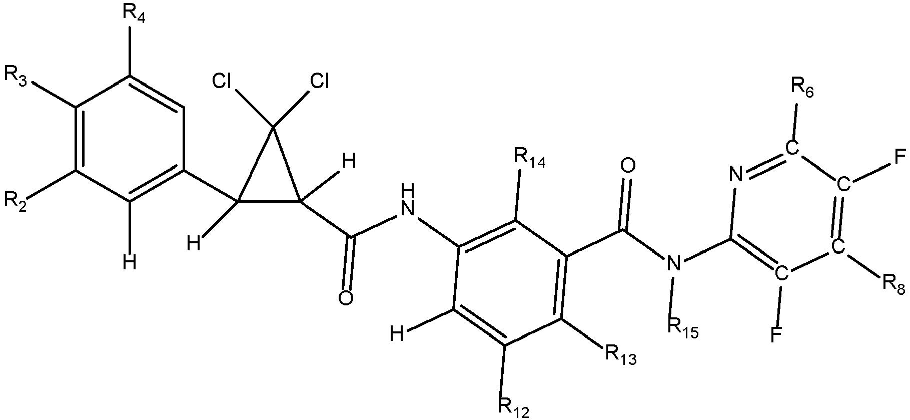
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXVII) 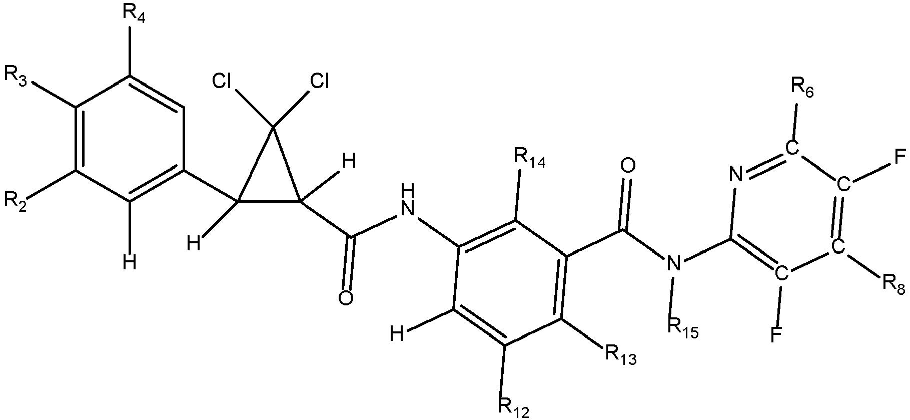
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXVII) 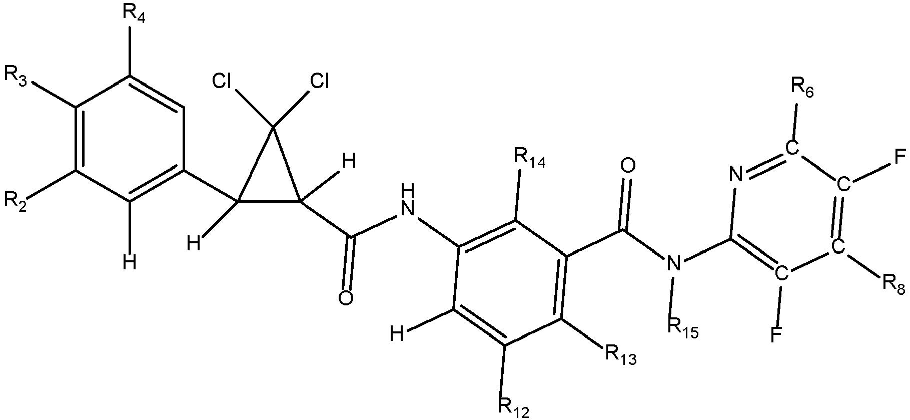
and N-oxides, veterinary acceptable acid addition salts, salt derivatives, solvates, ester derivatives, crystal polymorphs, isotopes, stereoisomers, and tautomers thereof,
wherein,
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (LXVII) 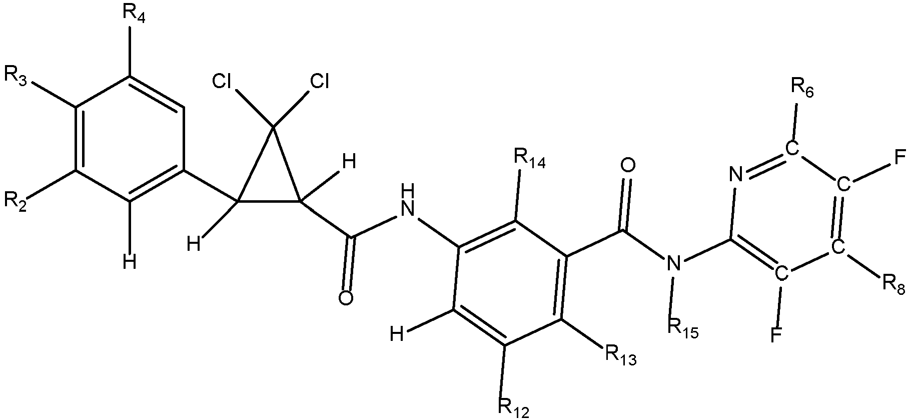
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁶** is selected from the group consisting of H, F, and NH(C₁-C₆)alkyl ;
**R⁸** is selected from the group consisting of H, and F ;
**R¹²** is H ;
**R¹³** is Cl;
**R¹⁴** is H;
**R¹⁵** is H.

In an embodiment of the invention and/or embodiments thereof, the invention is directed to a compound of formula (LXVIII) 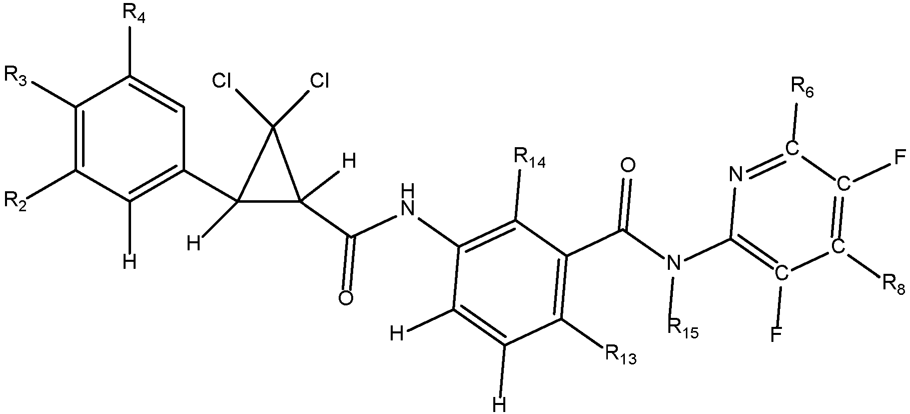
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R³, R¹³, R¹⁴, and R¹⁵ are defined as elsewhere in this description.

In an embodiment of the invention and/or embodiments thereof, the invention is directed to a compound of formula (LXVIII) 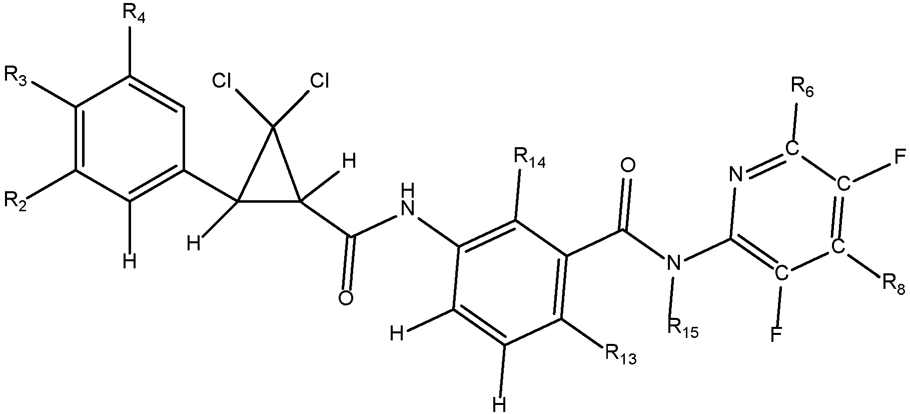
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXVIII) 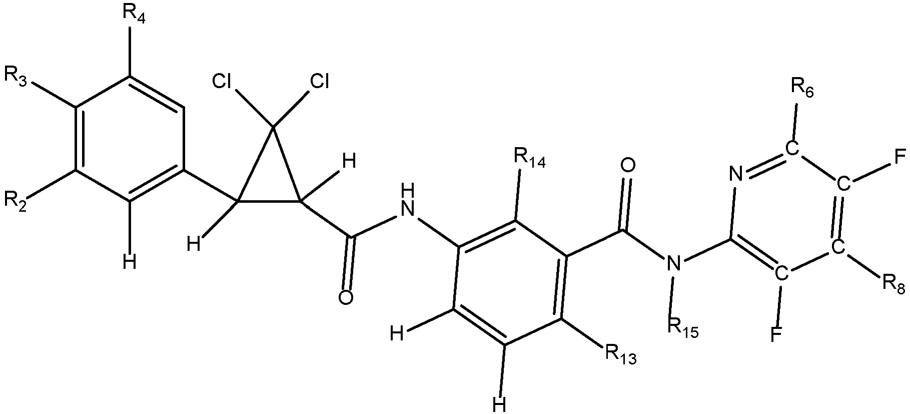
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXVIII) 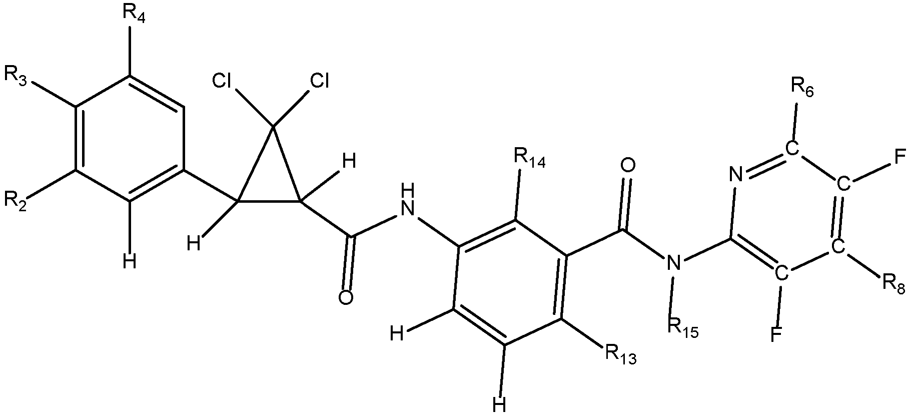
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₁-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXVIII) 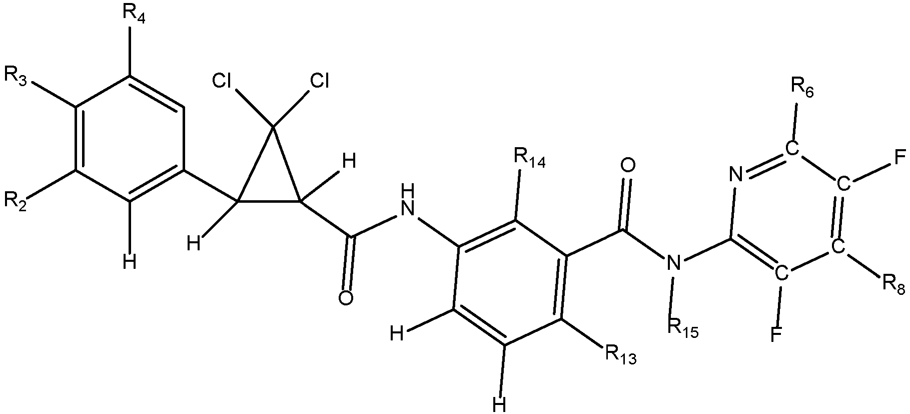
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (LXVIII) 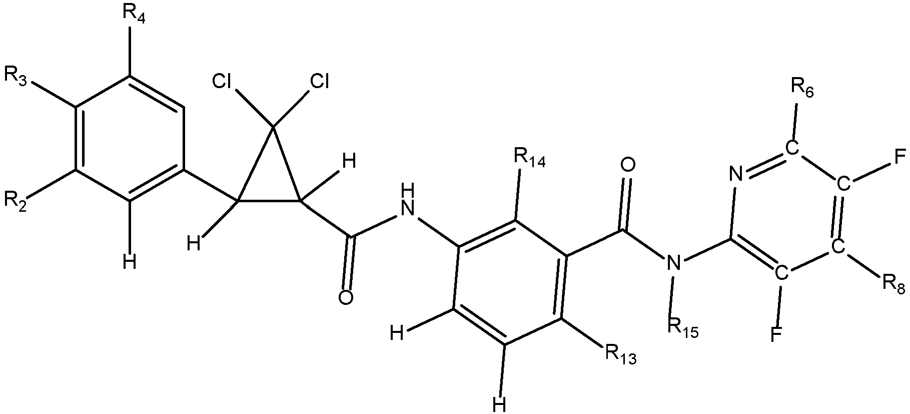
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
R⁶ is selected from the group consisting of H, F, and NH(C₁-C₆)alkyl ;
**R⁸** is selected from the group consisting of H, and F ;
**R¹³** is Cl;
**R¹⁴** is H;
**R¹⁵** is H.

In an embodiment of the invention and/or embodiments thereof, the invention is directed to a compound of formula (LXIX) 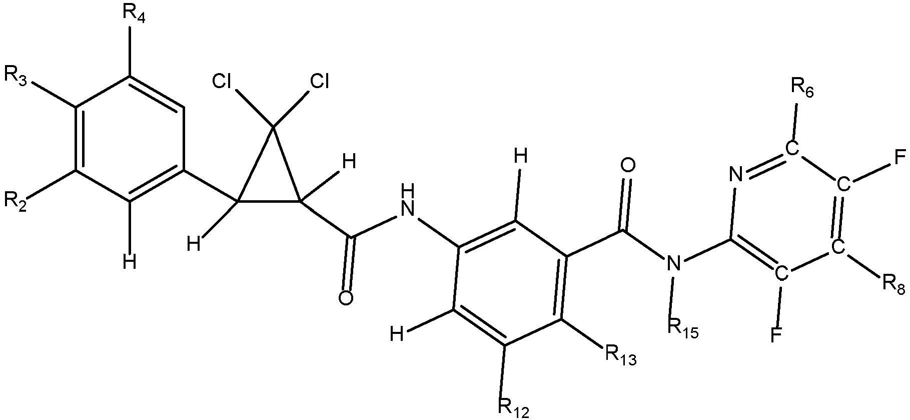
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R³, R¹², R¹³,and R¹⁵ are defined as elsewhere in this description.

In an embodiment of the invention and/or embodiments thereof, the invention is directed to a compound of formula (LXIX) 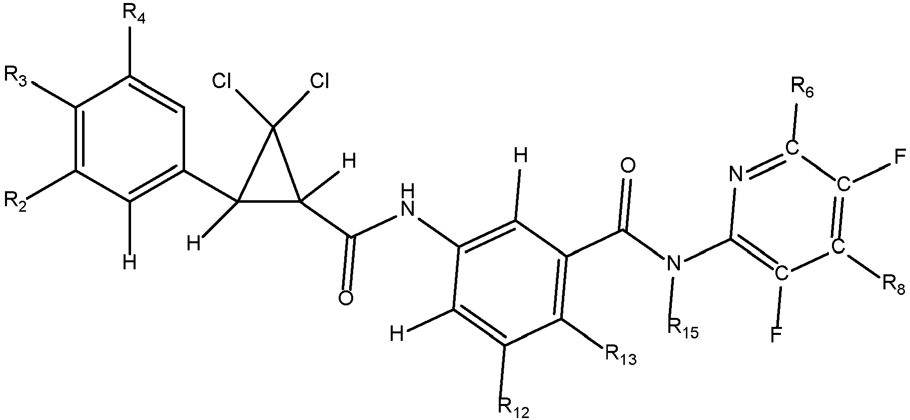
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXIX) 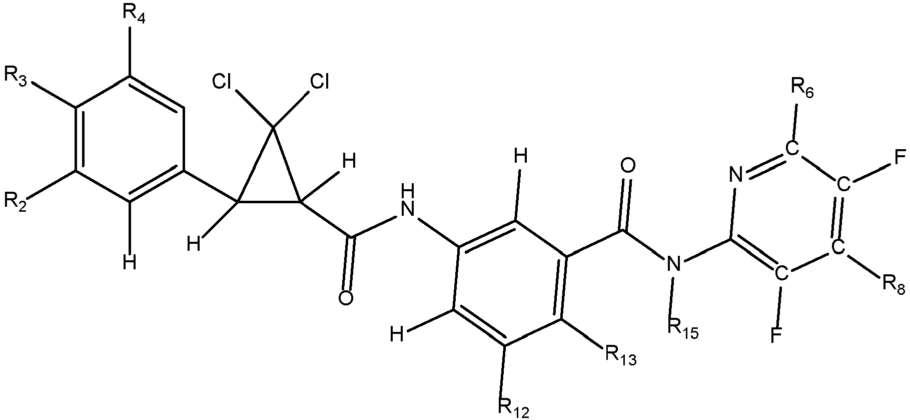
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXIX) 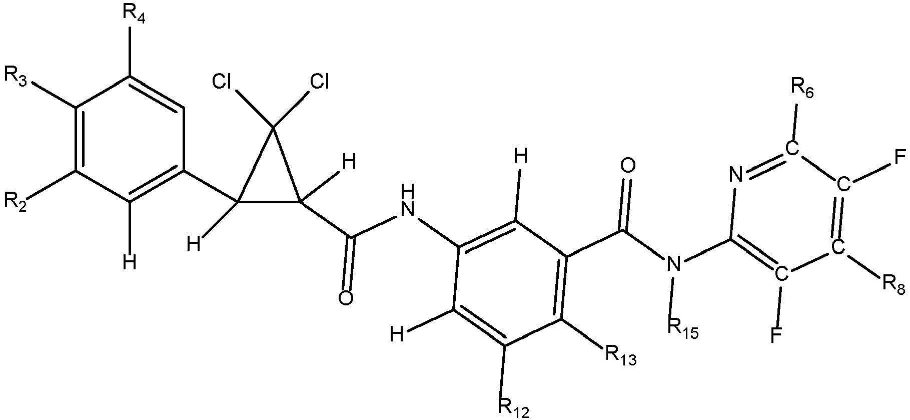
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXIX) 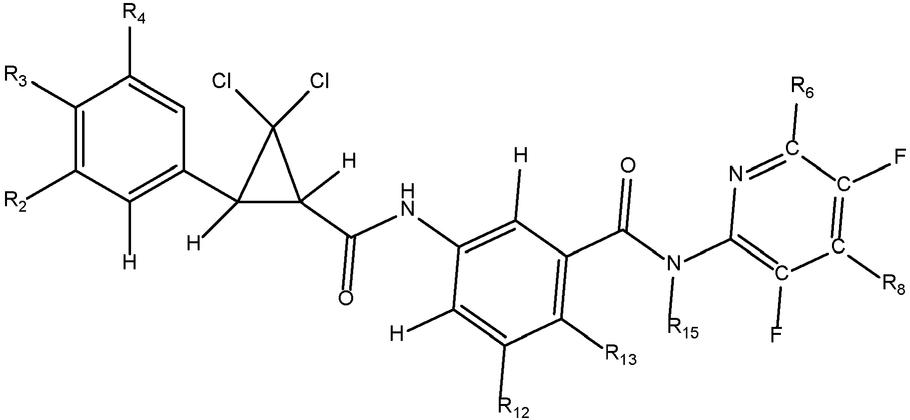
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
R¹⁰ is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (LXIX) 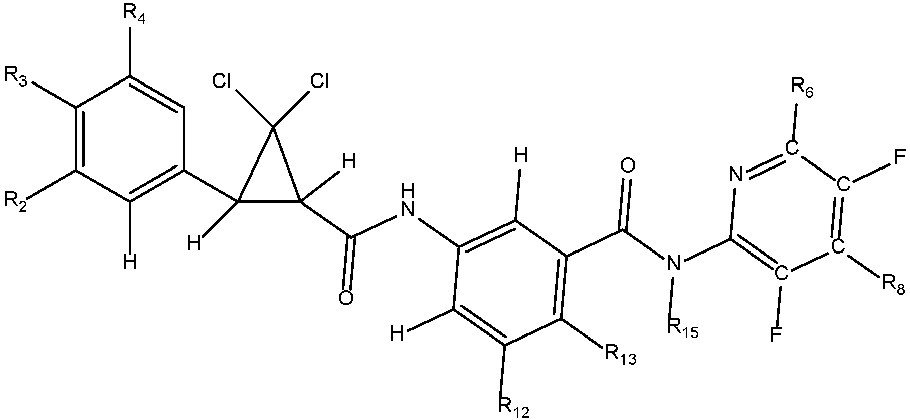
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br;
R³ is selected from the group consisting of H, F, Cl, Br, and I ;
R⁴ is selected from the group consisting of H, F, Cl, Br;
R⁶ is selected from the group consisting of H, F, and NH(C₁-C₆)alkyl ;
R⁸ is selected from the group consisting of H, and F ;
R¹² is H ;
R¹³ is Cl;
R¹⁵ is H.

In an embodiment of the invention and/or embodiments thereof, the invention is directed to a compound of formula (LXX) 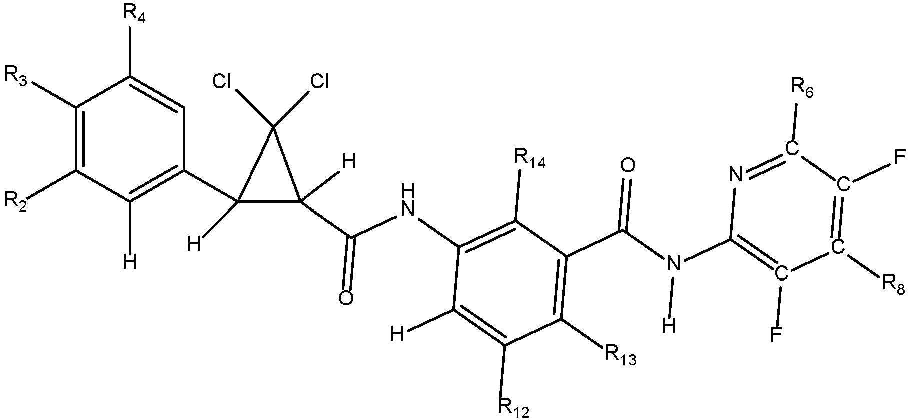
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, R¹², R¹³, and R¹⁴ are defined as elsewhere in this description.

In an embodiment of the invention and/or embodiments thereof, the invention is directed to a compound of formula (LXX) 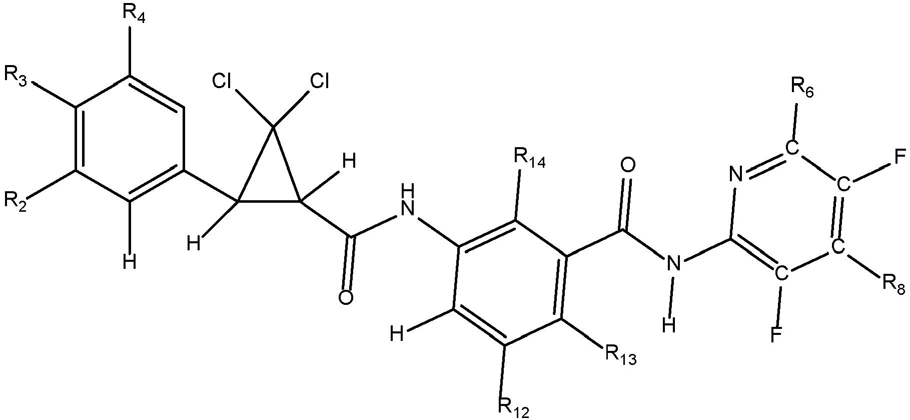
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴ is** selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
R⁸ is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (LXX) 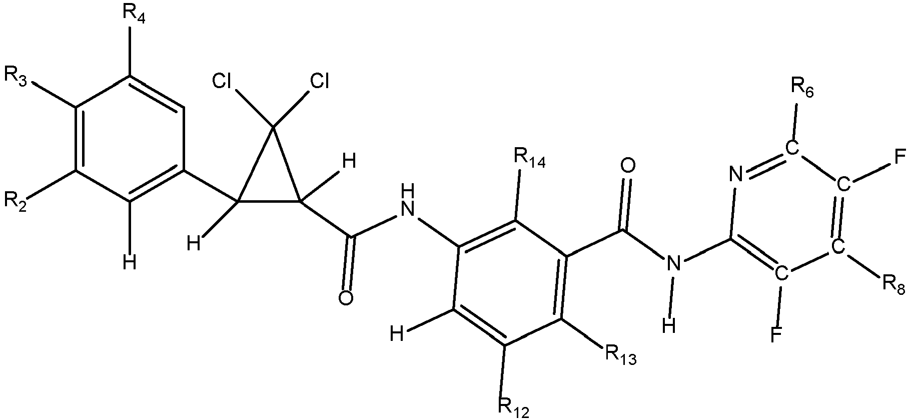
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (LXX) 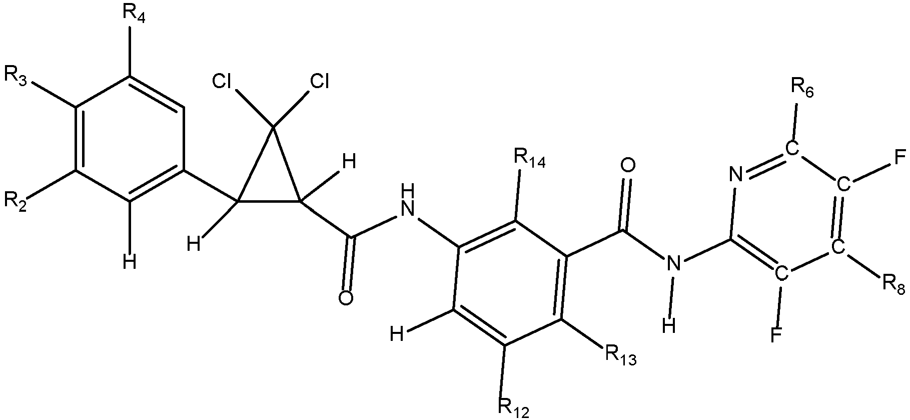
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴ is** selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (LXX) 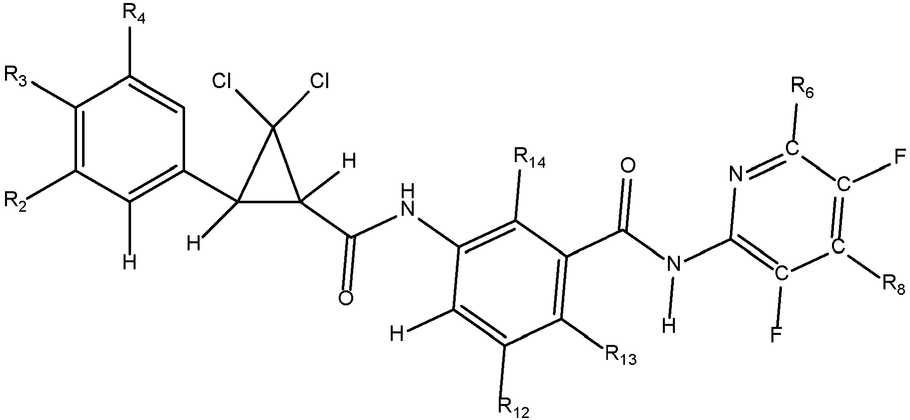
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴ is** selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³ is Cl or F ;**
**R¹⁴** is selected from the group consisting of H and F.

In an embodiment of the invention and/or embodiments thereof, the invention is directed to a compound of formula (LXX) 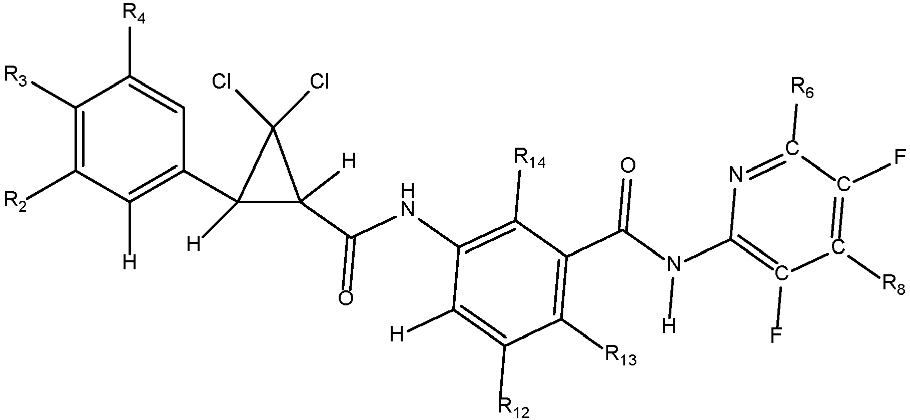
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴ is** selected from the group consisting of H, F, Cl, Br;
**R⁶** is selected from the group consisting of H, F, and NH(C₁-C₆)alkyl ;
**R⁸** is selected from the group consisting of H, and F ;
**R¹² is H ;**
**R¹³** is Cl;
**R¹⁴** is H.

In an embodiment of the invention and/or embodiments thereof, the invention is directed to a compound of formula (LXXI) 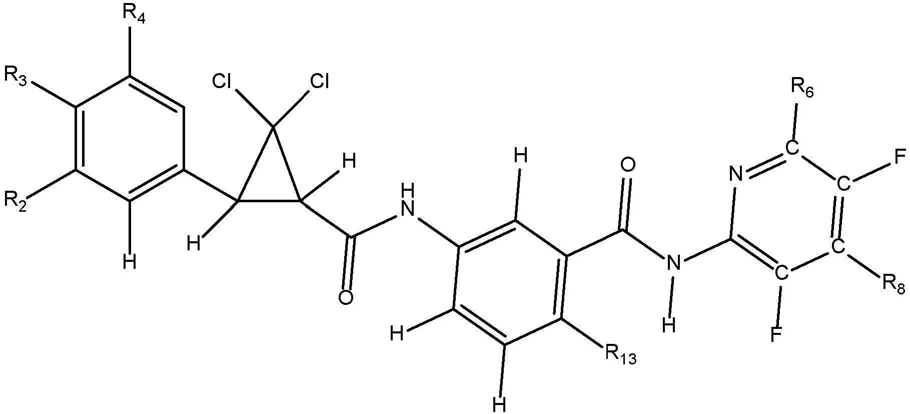
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁸, and R¹³ are defined as elsewhere in this description.

In an embodiment of the invention and/or embodiments thereof, the invention is directed to a compound of formula (LXXI) 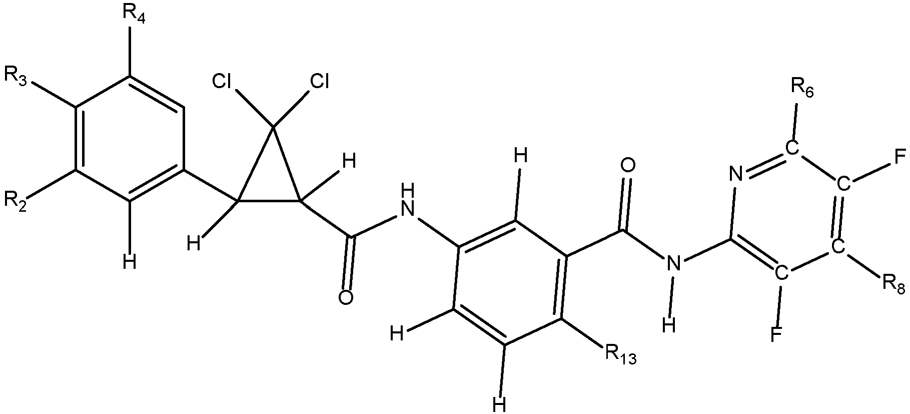
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴ is** selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (LXXI) 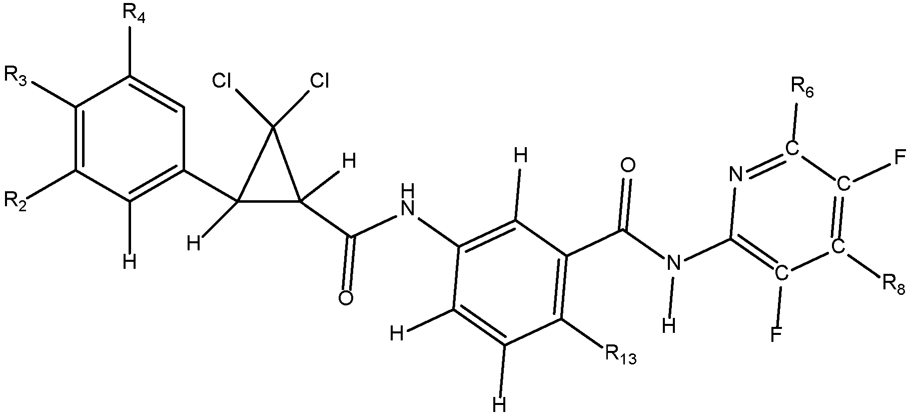
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴ is** selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃.

In an embodiment of the invention and/or embodiments thereof, the invention is directed to a compound of formula (LXXI) 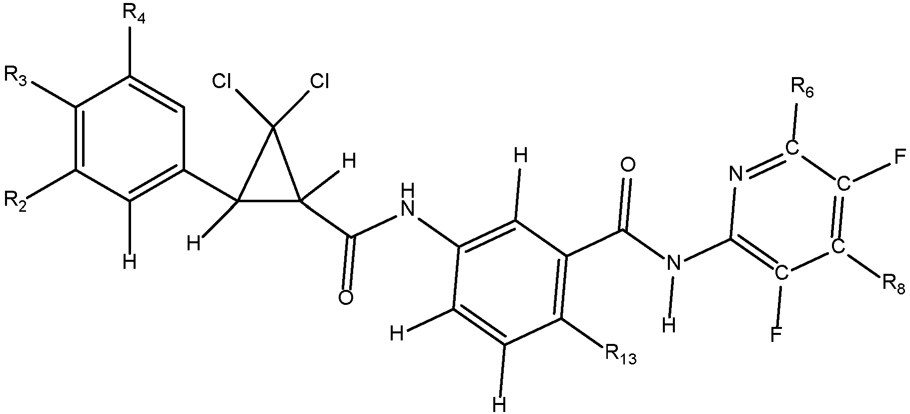
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴ is** selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
R¹⁰ is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃.

In an embodiment of the invention and/or embodiments thereof, the invention is directed to a compound of formula (LXXI) 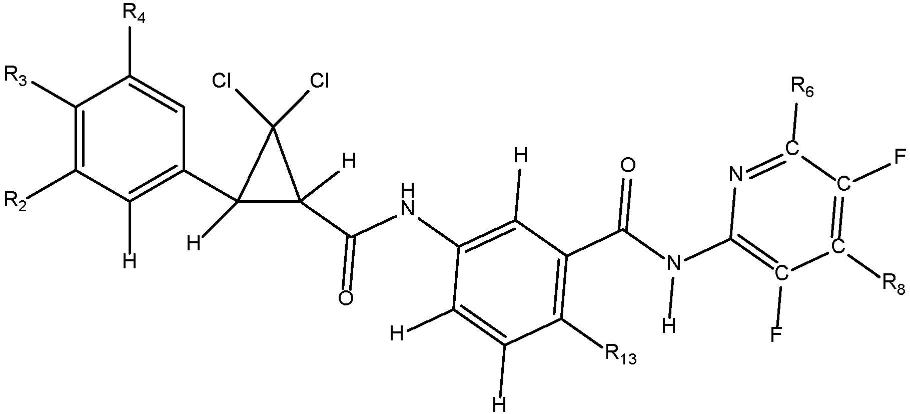
and N-oxides, veterinary acceptable acid addition salts, salt derivatives, solvates, ester derivatives, crystal polymorphs, isotopes, stereoisomers, and tautomers thereof,
wherein
R² is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴ is** selected from the group consisting of H, F, Cl, Br, (C₁-C₆)alkyl, and (C₁-C₆)haloalkyl;
**R⁶** is selected from the group consisting of H, F, NH(C₁-C₆)alkyl, NHC(=O)R¹⁰, and N(C₁-C₆)alkyl-C(=O)IR¹⁰ ;
**R⁸** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is CI or F.

In an embodiment of the invention and/or embodiments thereof, the invention is directed to a compound of formula (LXXI) 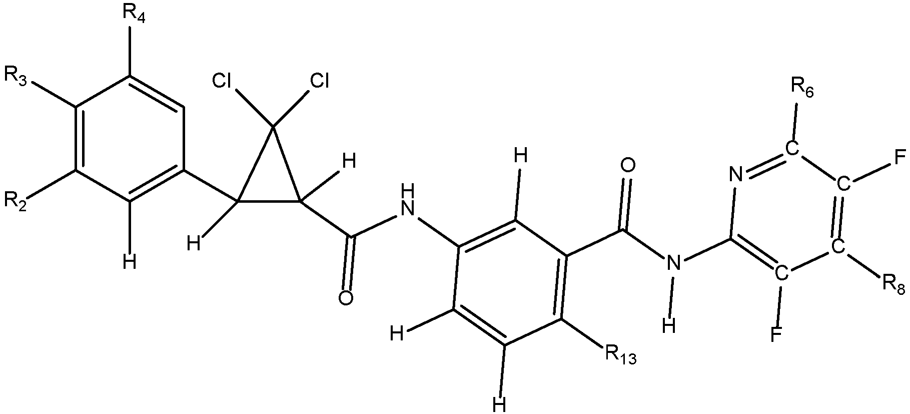
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
R⁴ is selected from the group consisting of H, F, Cl, Br;
**R⁶** is selected from the group consisting of H, F, and NH(C₁-C₆)alkyl ;
**R⁸** is selected from the group consisting of H, and F ;
**R¹³** is Cl.

In an embodiment of the invention and/or embodiments thereof, the invention is directed to a compound of formula (LXXII) 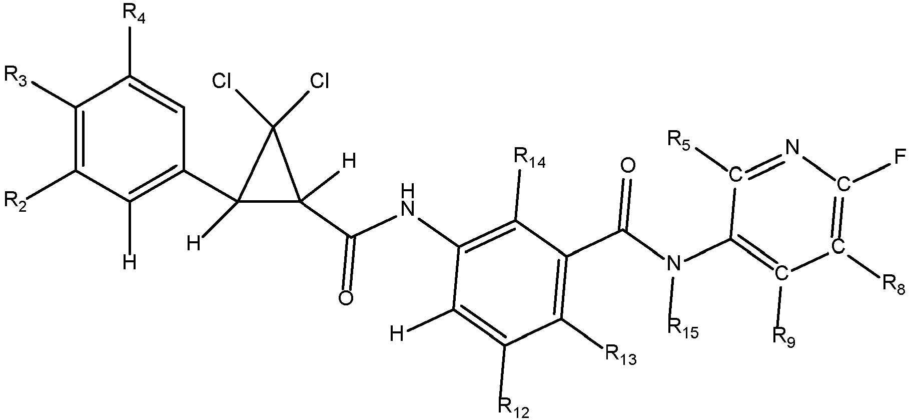
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁸, R⁹, R¹², R¹³, R¹⁴, and R¹⁵are defined as elsewhere in this description.

In an embodiment of the invention and/or embodiments thereof, the invention is directed to a compound of formula (LXXII) 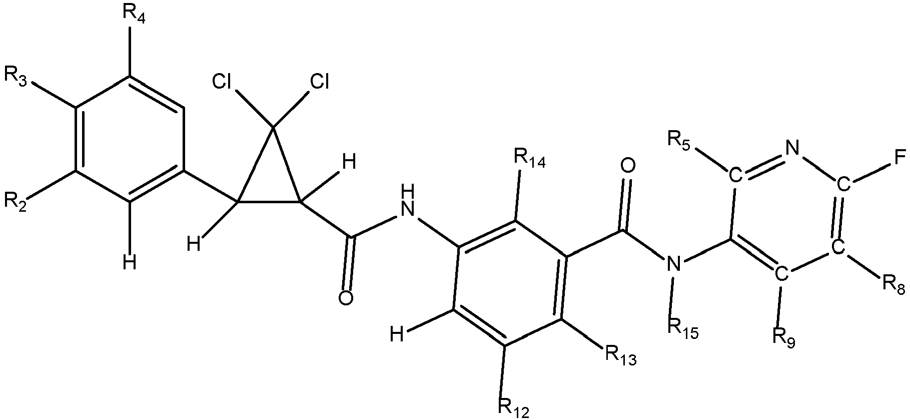
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴ is** selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R**⁵ is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
R⁸ is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXXII) 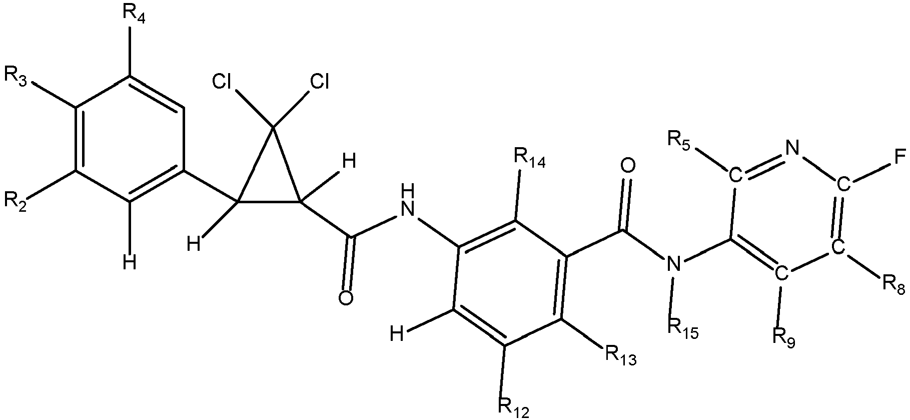
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
R⁵ is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁷** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXII) 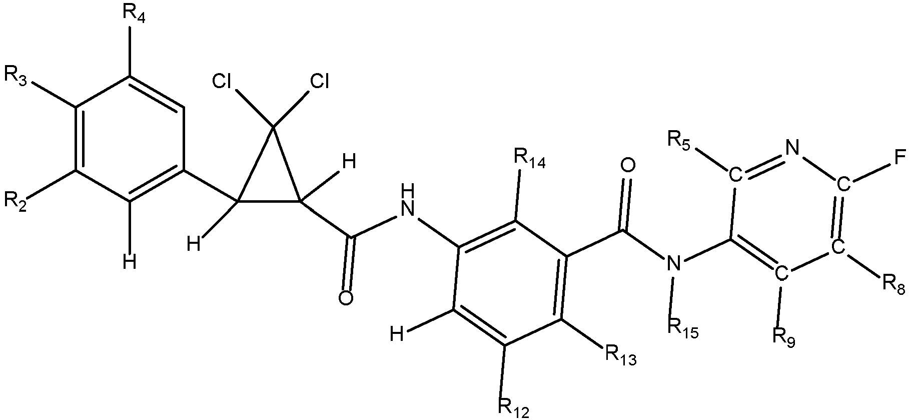
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
R³ is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXII) 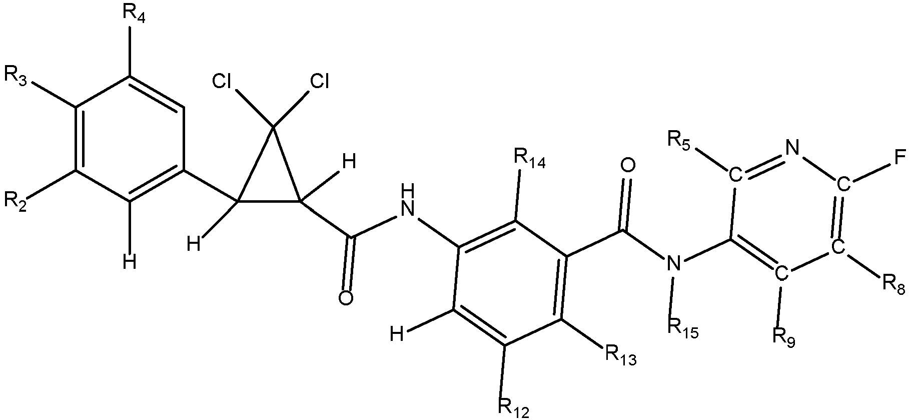
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (LXXII) 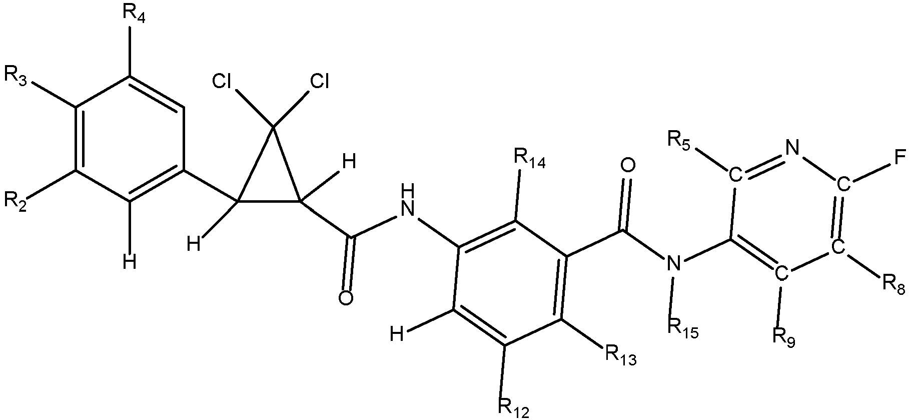
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁵** is selected from the group consisting of H, F, Cl, CN and (C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H;
**R¹⁵** is H.

In an embodiment of the invention and/or embodiments thereof, the invention is directed to a compound of formula (LXXIII) 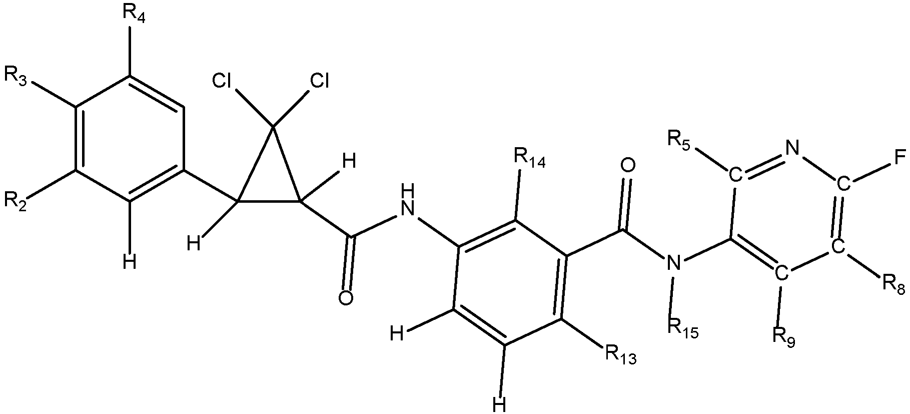
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁸, R⁹, R¹³, R¹⁴, and R¹⁵ are defined as elsewhere in this description.

In an embodiment of the invention and/or embodiments thereof, the invention is directed to a compound of formula (LXXIII) 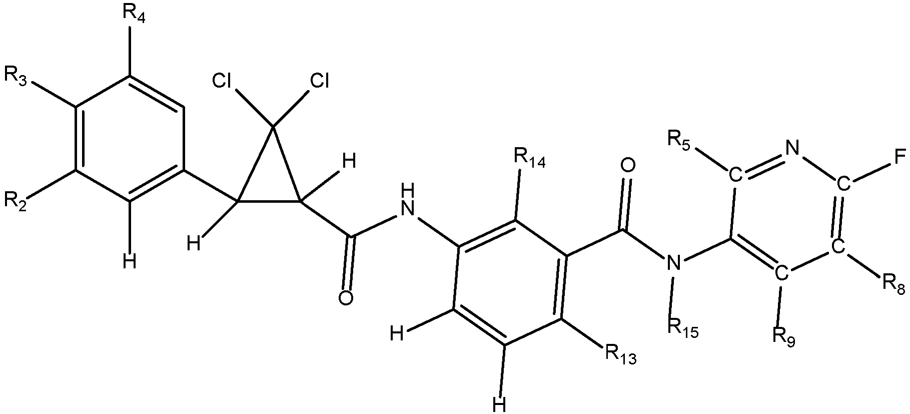
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXXIII) 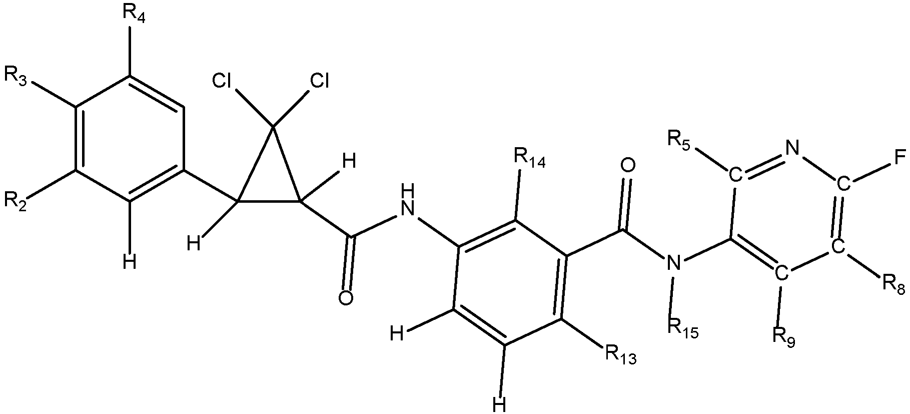
and N-oxides, veterinary acceptable acid addition salts, salt derivatives, solvates, ester derivatives, crystal polymorphs, isotopes, stereoisomers, and tautomers thereof,
whereinl
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
R⁵ is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXIII) 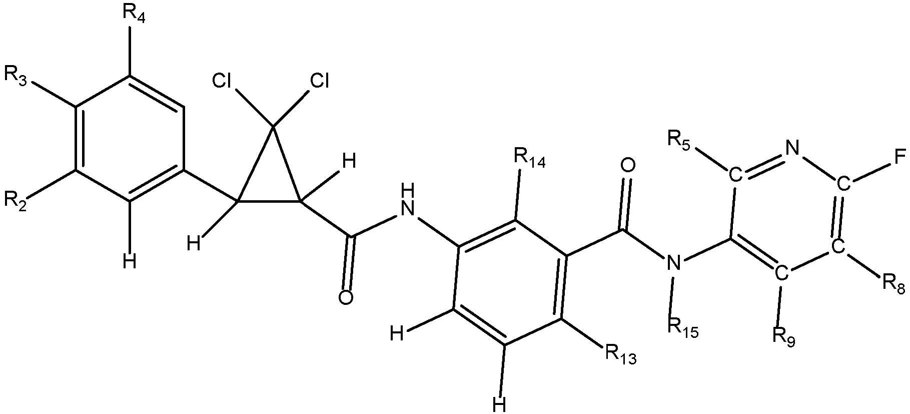
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁴** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXIII) 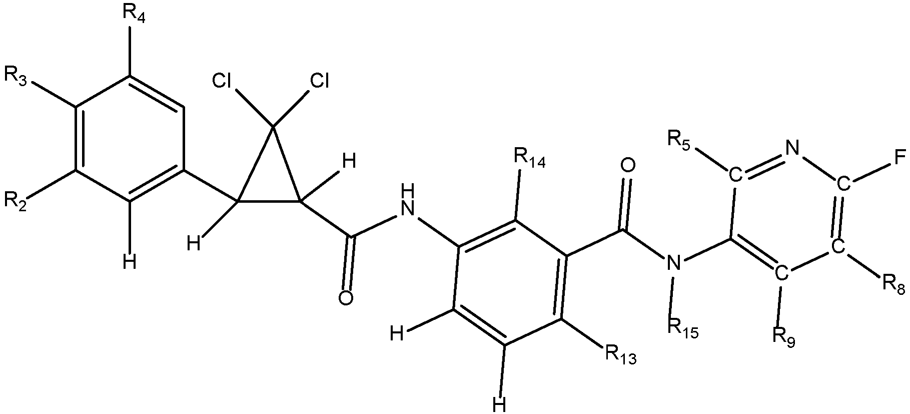
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁴** is selected from the group consisting of H and F ;
**R¹⁵** is selected from the group consisting of H, and CH₃.

In an embodiment of the invention and/or embodiments thereof, the invention is directed to a compound of formula (LXXIII) 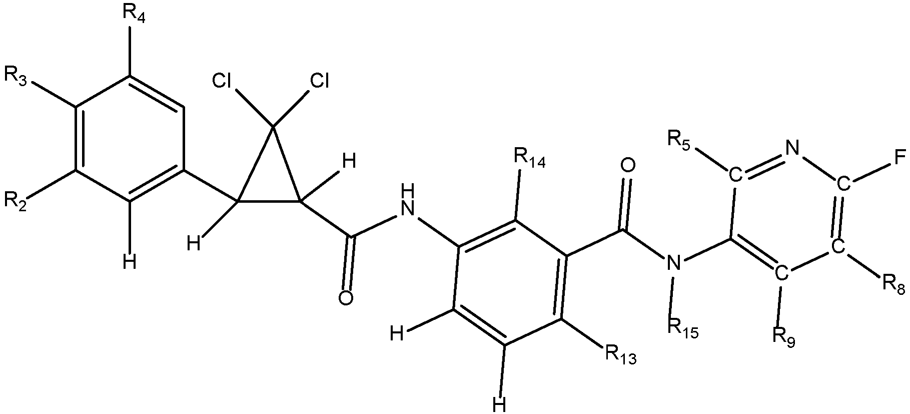
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁸** is selected from the group consisting of H, and F ;
**R⁹** is selected from the group consisting of H and F ;
**R¹³** is Cl;
**R¹⁴** is H;
**R¹⁵** is H.

In an embodiment of the invention and/or embodiments thereof, the invention is directed to a compound of formula (LXXIV) 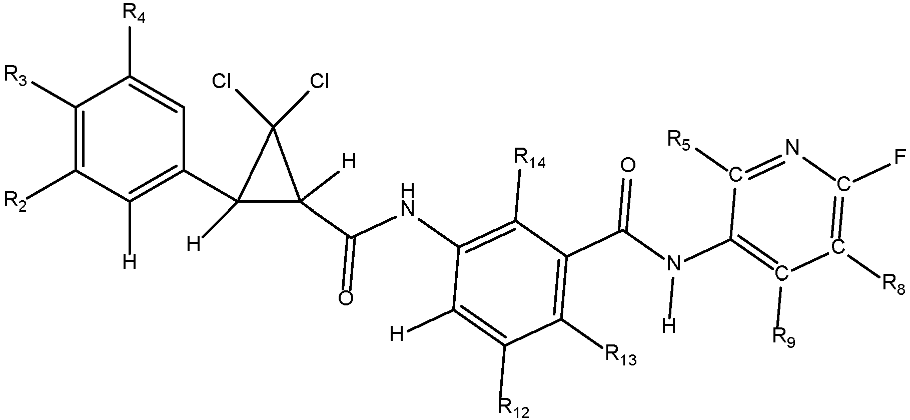
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁸, R⁹, R¹², R¹³, and R¹⁴ are defined as elsewhere in this description.

In an embodiment of the invention and/or embodiments thereof, the invention is directed to a compound of formula (LXXIV) 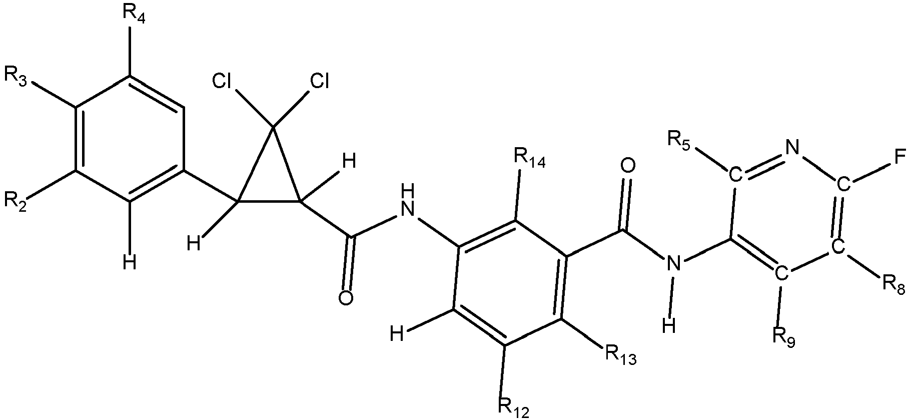
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂.

In an embodiment of the invention and/or embodiments thereof, the invention is directed to a compound of formula (LXXIV) 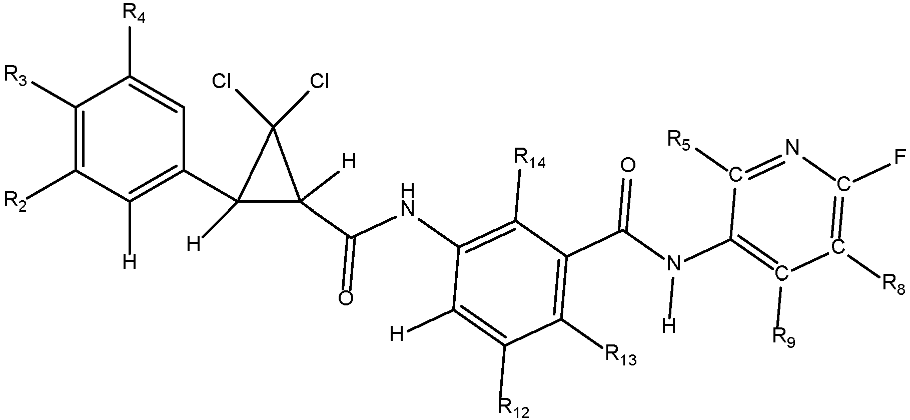
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF_{3;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (LXXIV) 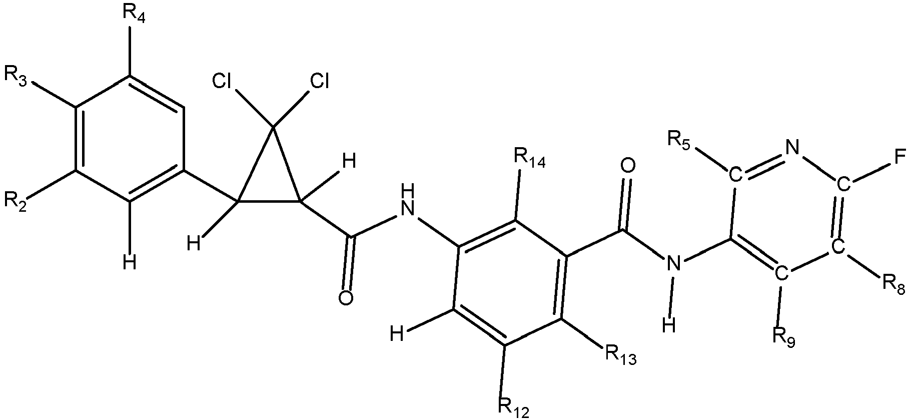
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF_{3 ;}
**R¹³** is selected from the group consisting of H, F, Cl, CH₃ and CF₃ ;
**R¹⁴** is selected from the group consisting of H, F, Cl, CH₃ and CF₃.

In an embodiment of the invention and/or embodiments thereof, the invention is directed to a compound of formula (LXXIV) 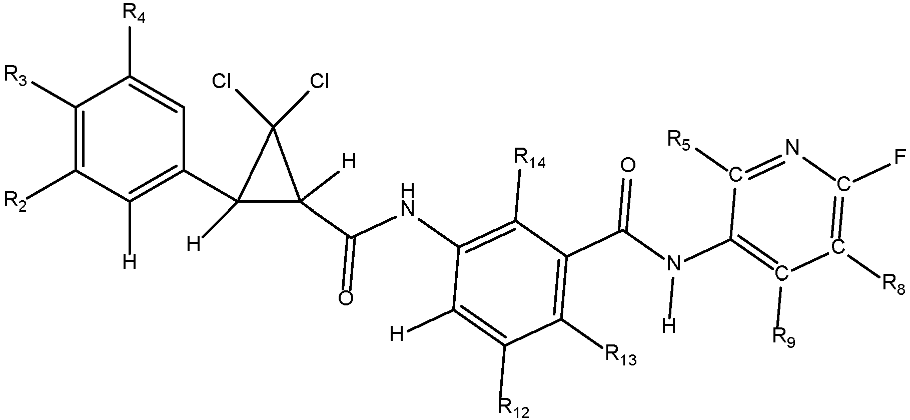
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁴** is selected from the group consisting of H and F.

In an embodiment of the invention and/or embodiments thereof, the invention is directed to a compound of formula (LXXIV) 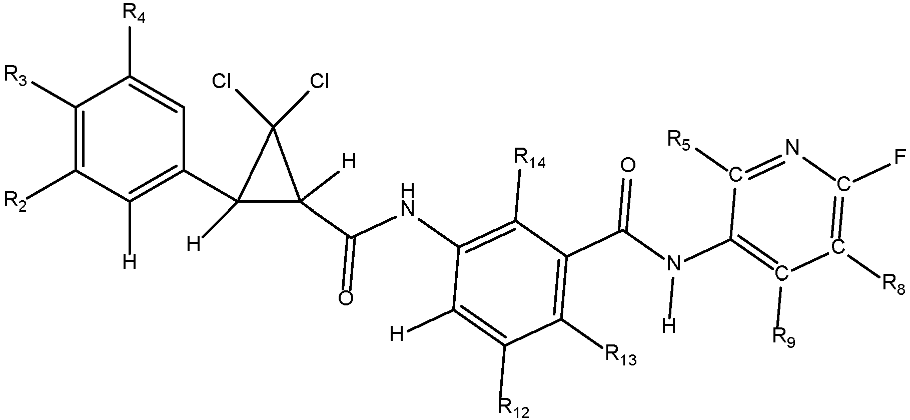
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
**R⁵** is selected from the group consisting of H, F, Cl, CN and (C₁-C₆)alkyl ;
**R⁸** is selected from the group consisting of H, and F ;
**R⁹** is selected from the group consisting of H and F ;
**R¹²** is H ;
**R¹³** is Cl;
**R¹⁴** is H.

In an embodiment of the invention and/or embodiments thereof, the invention is directed to a compound of formula (LXXV) 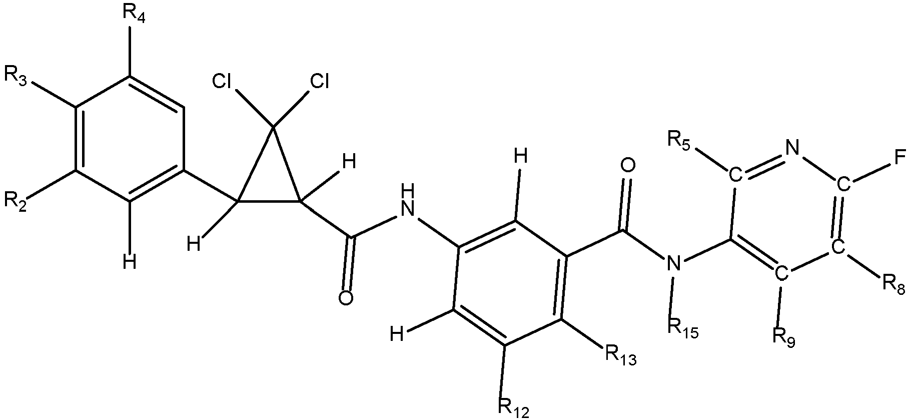
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁸, R⁹, R¹², R¹³, and R¹⁵ are defined as elsewhere in this description.

In an embodiment of the invention and/or embodiments thereof, the invention is directed to a compound of formula (LXXV) 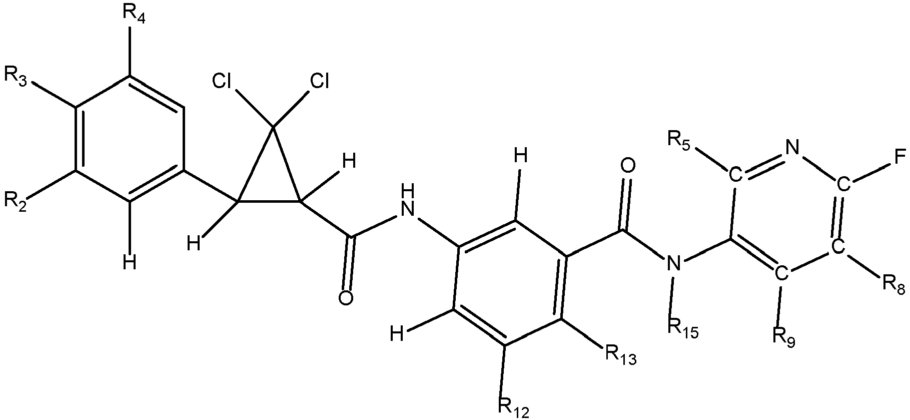
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl.

In an embodiment of the invention and/or embodiments thereof, the invention is directed to a compound of formula (LXXV) 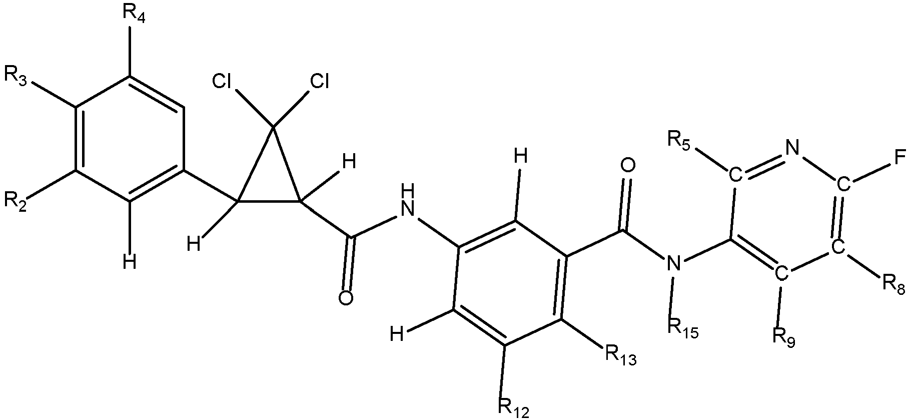
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁₋C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXV) 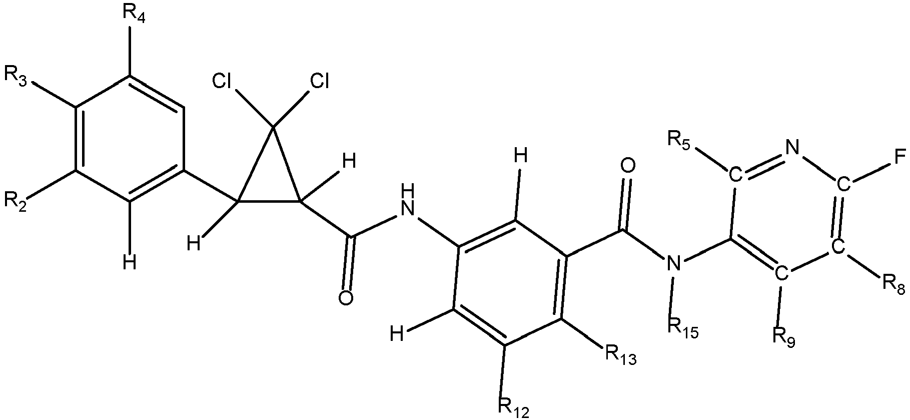
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is selected from the group consisting of H, F, Cl, CH₃ and CF₃;
**R¹³** is selected from the group consisting of H, F, Cl, CH₃ and CF₃ ;
**R¹⁵** is selected from the group consisting of H, CH₃, and CF₃.

In an embodiment of the invention and/or embodiments thereof, the invention is directed to a compound of formula (LXXV) 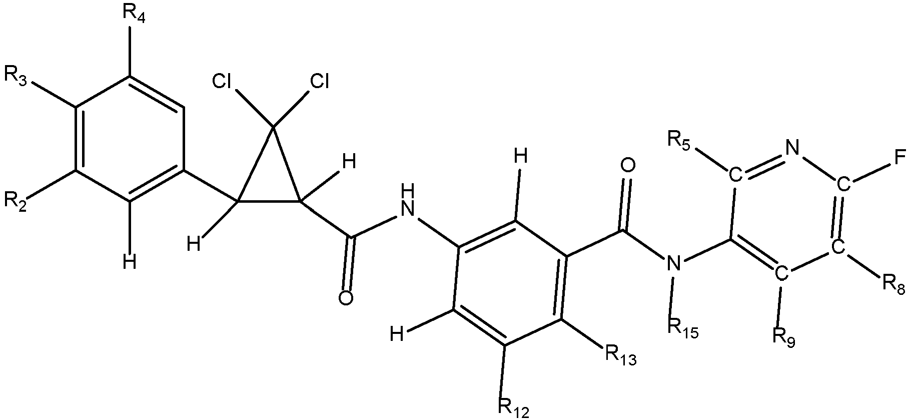
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹²** is H or F ;
**R¹³** is Cl or F ;
**R¹⁵** is selected from the group consisting of H, and CH₃.

In an embodiment of the invention and/or embodiments thereof, the invention is directed to a compound of formula (LXXV) 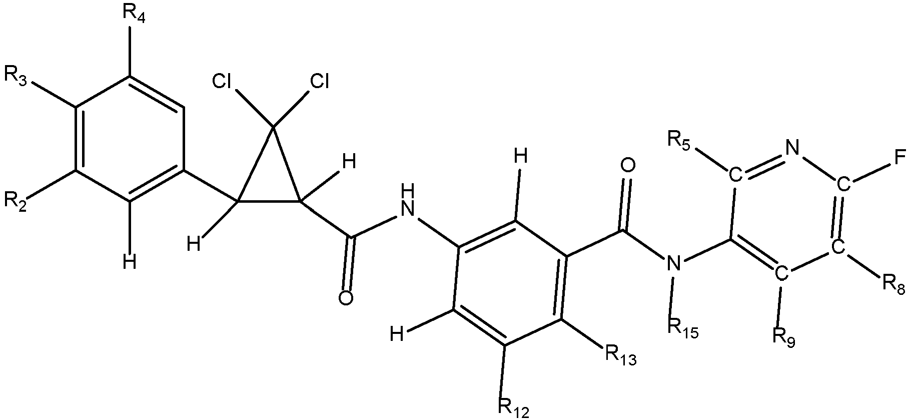
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁸** is selected from the group consisting of H, and F ;
**R⁹** is selected from the group consisting of H and F ;
**R¹²** is H ;
**R¹³** is Cl;
**R¹⁵** is H.

In an embodiment of the invention and/or embodiments thereof, the invention is directed to a compound of formula (LXXVI) 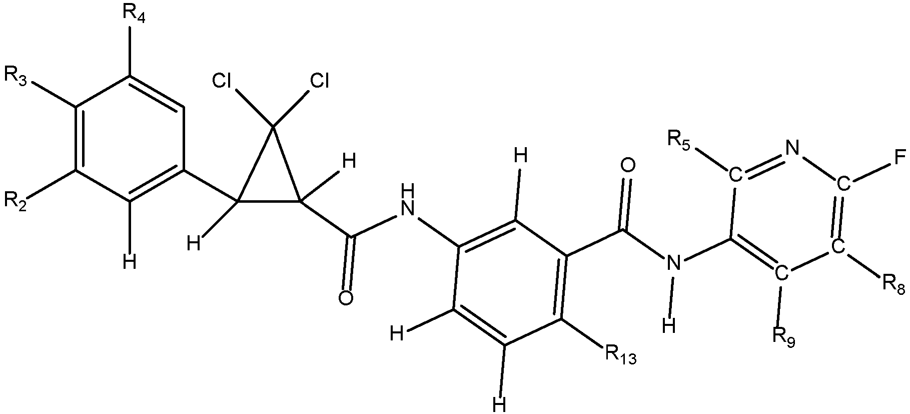
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁸, R⁹, and R¹³ are defined as elsewhere in this description.

In an embodiment of the invention and/or embodiments thereof, the invention is directed to a compound of formula (LXXVI) 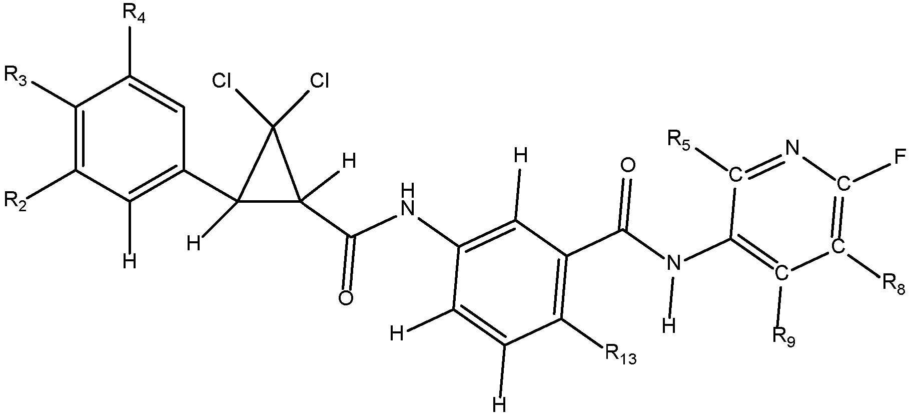
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkyICH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkyICH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl.

In an embodiment of the invention and/or embodiments thereof, the invention is directed to a compound of formula (LXXVI) 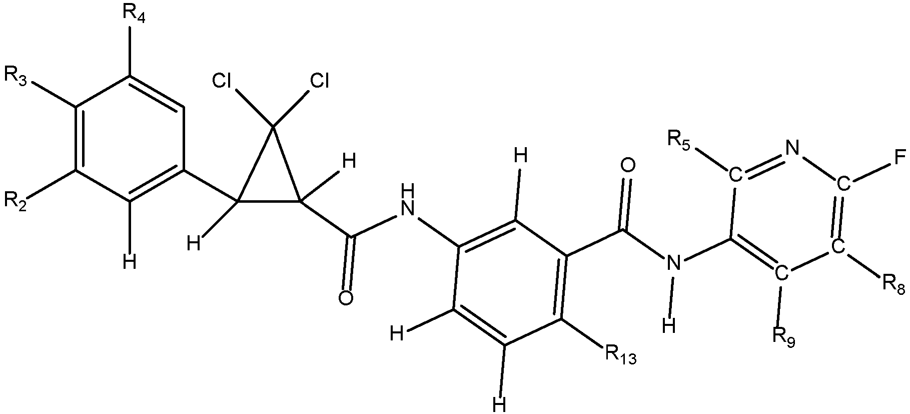
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁸** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃.

In an embodiment of the invention and/or embodiments thereof, the invention is directed to a compound of formula (LXXVI) 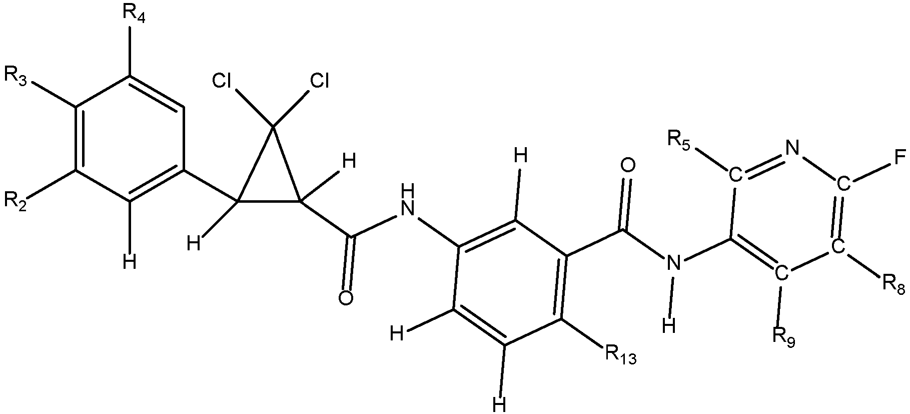
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁸** is selected from the group consisting of H, F, Cl, NH(C₁-C₆)alkyl, and (C₁-C₆)alkyl ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃.

In an embodiment of the invention and/or embodiments thereof, the invention is directed to a compound of formula (LXXVI) 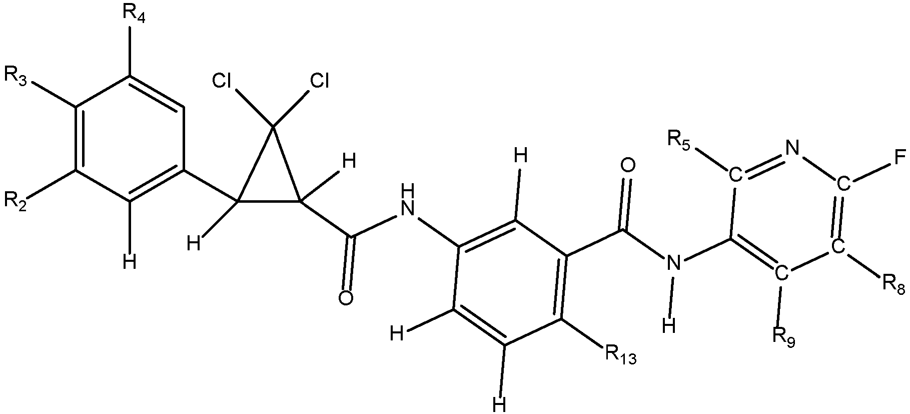
and N-oxides, veterinary acceptable acid addition salts, salt derivatives, solvates, ester derivatives, crystal polymorphs, isotopes, stereoisomers, and tautomers thereof,
wherein
**b** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
**R⁵** is selected from the group consisting of H, F, Cl, CN, NO₂, NH(C₁-C₆)alkyl, and (C₁-C₆)alkyl ;
**R⁸** is selected from the group consisting of H, F, and NH(C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹³** is Cl or F.

In an embodiment of the invention and/or embodiments thereof, the invention is directed to a compound of formula (LXXVI) 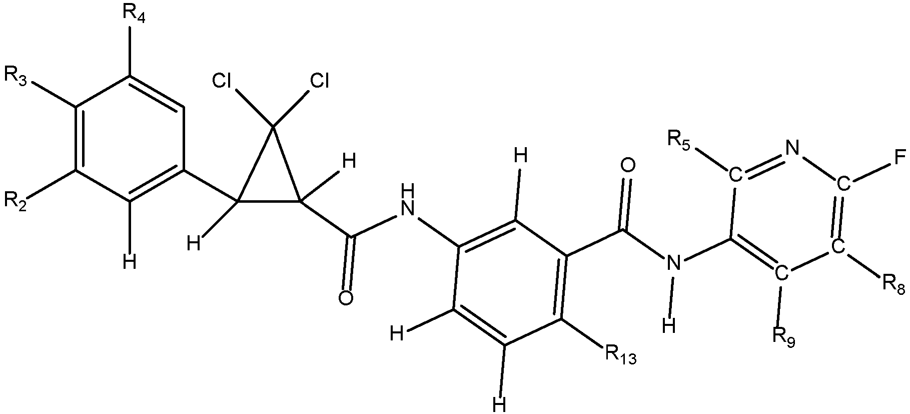
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I;
**R⁴** is selected from the group consisting of H, F, Cl, Br;
**R⁵** is selected from the group consisting of H, F, Cl, CN and (C₁-C₆)alkyl ;
**R⁸** is selected from the group consisting of H, and F ;
**R⁹** is selected from the group consisting of H and F ;
**R¹³** is Cl.

In an embodiment of the invention and/or embodiments thereof, the invention is directed to a compound of formula (LXXVII) 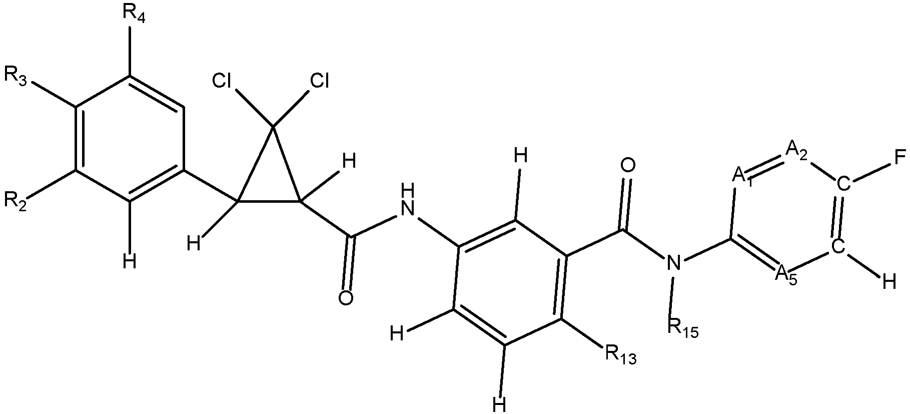
and N-oxides, veterinary acceptable acid addition salts, salt derivatives, solvates, ester derivatives, crystal polymorphs, isotopes, stereoisomers, and tautomers thereof,
wherein R², R³, R⁴, R⁵, R⁶, R⁹, R¹³, R¹⁵, A₁, A₂, and A₅ are defined as elsewhere in this description.

In an embodiment of the invention and/or embodiments thereof, the invention is directed to a compound of formula (LXXVII) 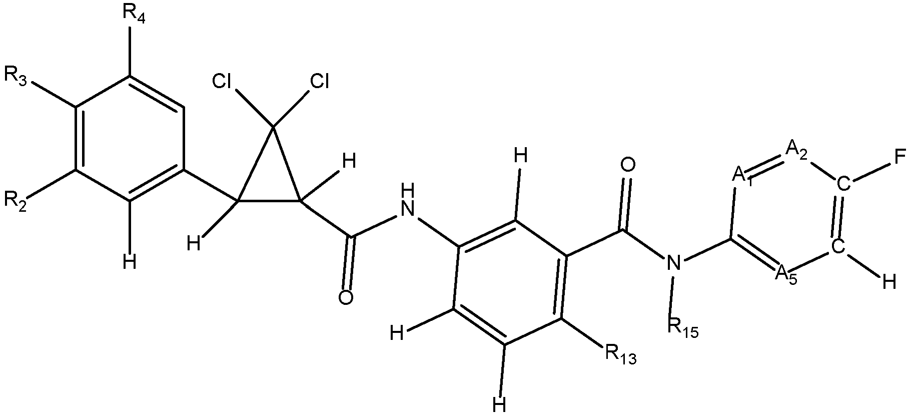
and N-oxides, veterinary acceptable acid addition salts, salt derivatives, solvates, ester derivatives, crystal polymorphs, isotopes, stereoisomers, and tautomers thereof,
wherein A₁ is CR₅, N=O, or N;
A₂ is CR₆, N=O, or N;
A₅ is CR₉, N=O or N ;
wherein no more than 2 of A₁, A₂, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-Cₑ)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXVII) 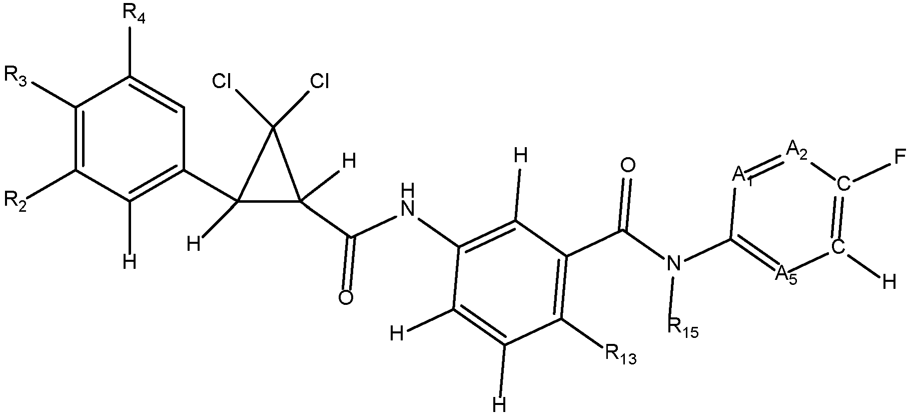
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXVII) 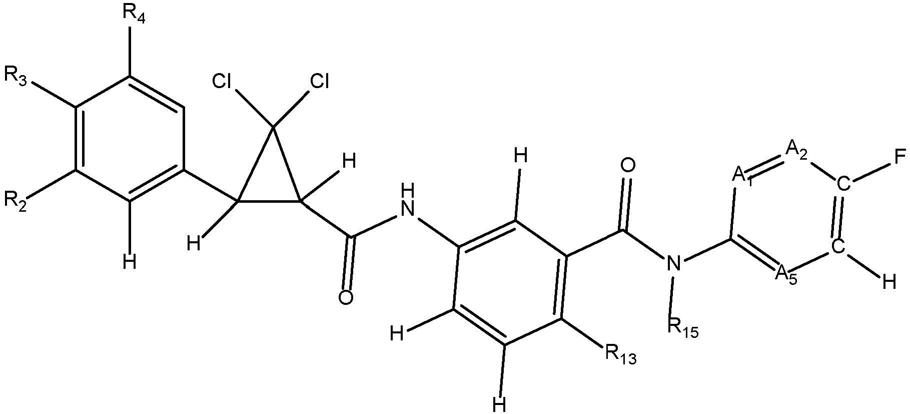
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXVII) 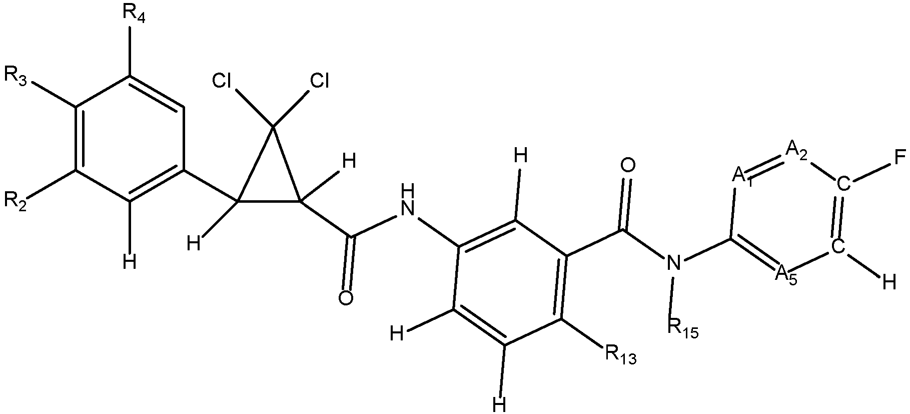
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A₁ is CR₅ or N;
A2 is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXVII) 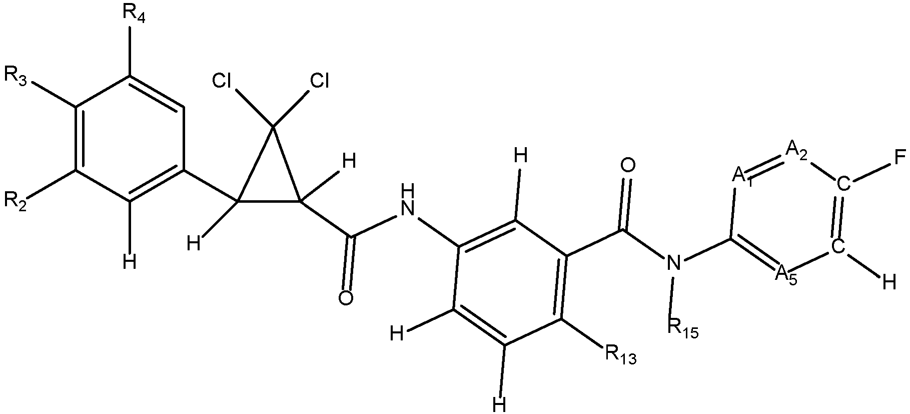
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₁ is CR₅ or N;
A2 is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXVIII) 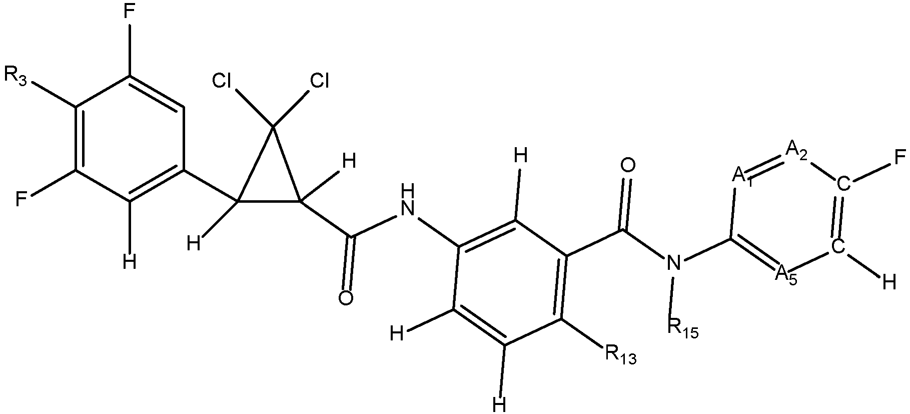
and N-oxides, veterinary acceptable acid addition salts, salt derivatives, solvates, ester derivatives, crystal polymorphs, isotopes, stereoisomers, and tautomers thereof,
wherein R³, R⁵, R⁶, R⁹, R¹³, R¹⁵, A₁, A₂, and A₅ are defined as elsewhere in this description.

In an embodiment of the invention and/or embodiments thereof, the invention is directed to a compound of formula (LXXVIII) 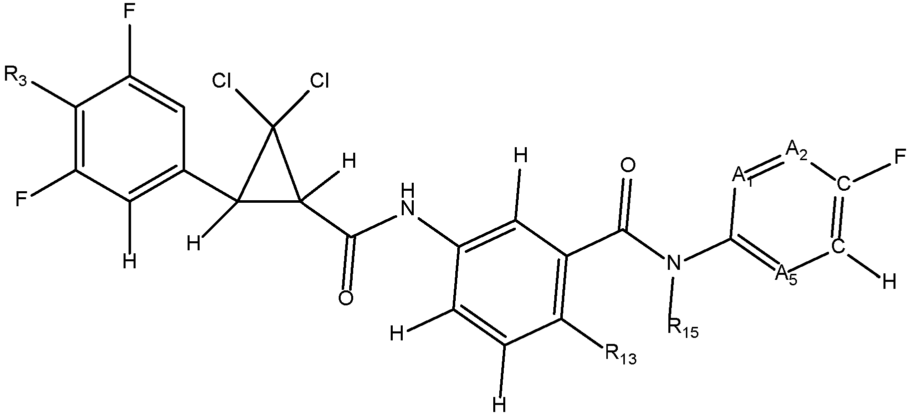
and N-oxides, veterinary acceptable acid addition salts, salt derivatives, solvates, ester derivatives, crystal polymorphs, isotopes, stereoisomers, and tautomers thereof,
wherein A₁ is CR₅, N=O, or N;
A₂ is CR₆, N=O, or N;
A₅ is CR₉, N=O or N ;
wherein no more than 2 of A₁, A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXVIII) 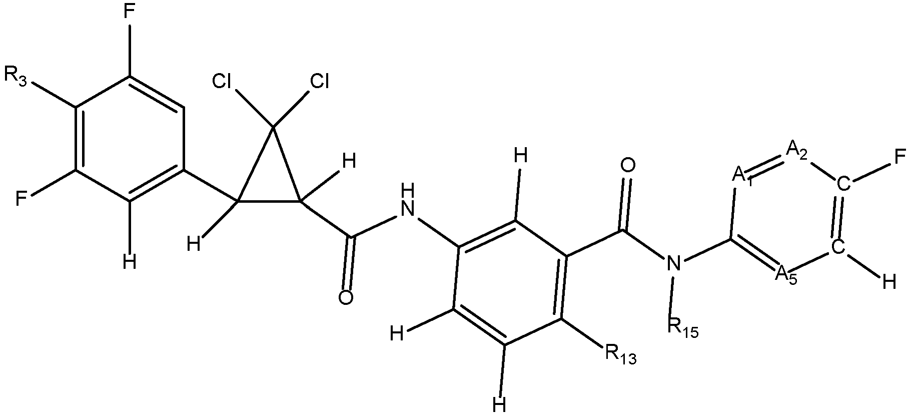
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₁-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXVIII) 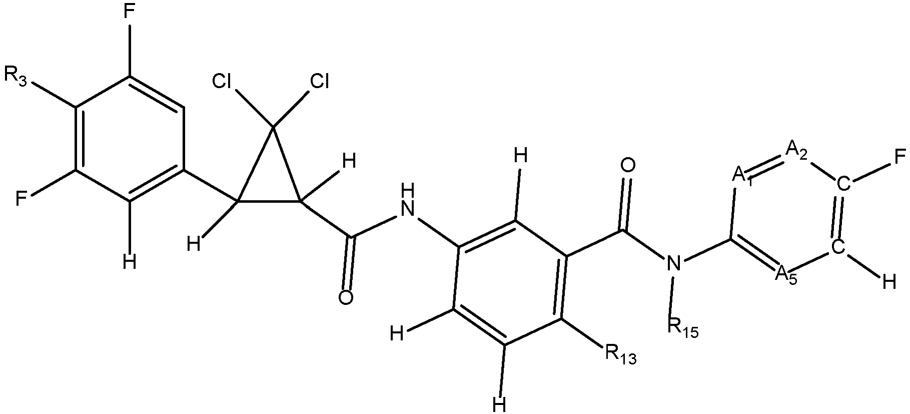
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXVIII) 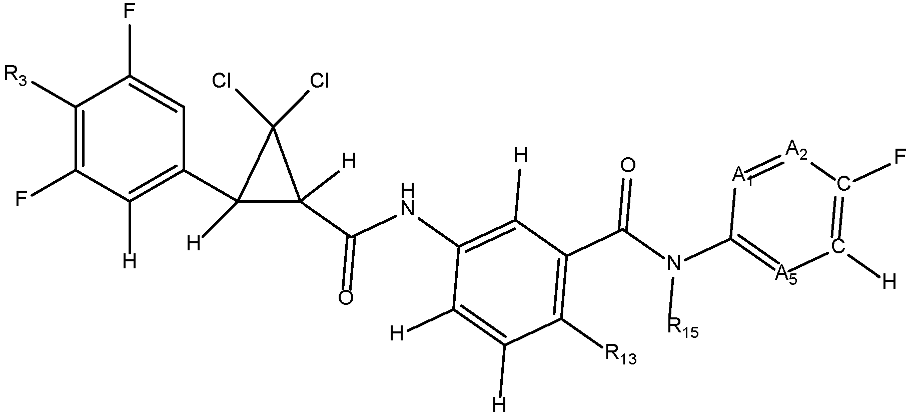
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy ;
A₁ is CR₅ or N;
A₂ is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXVIII) 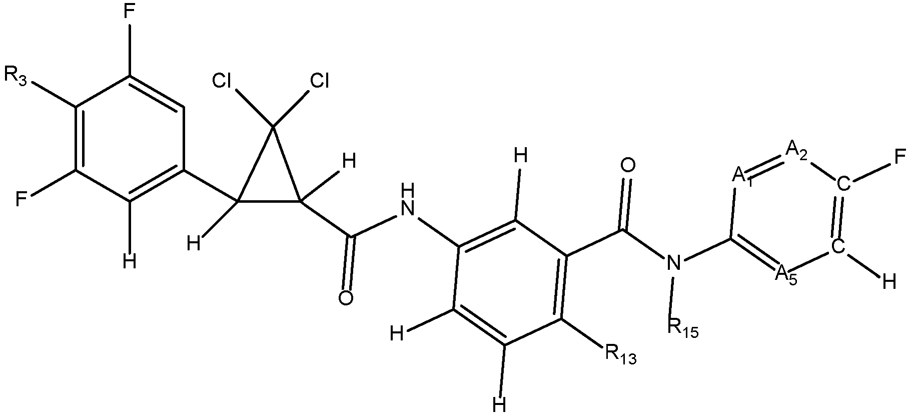
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I ;
A₁ is CR₅ or N;
A₂ is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXIX) 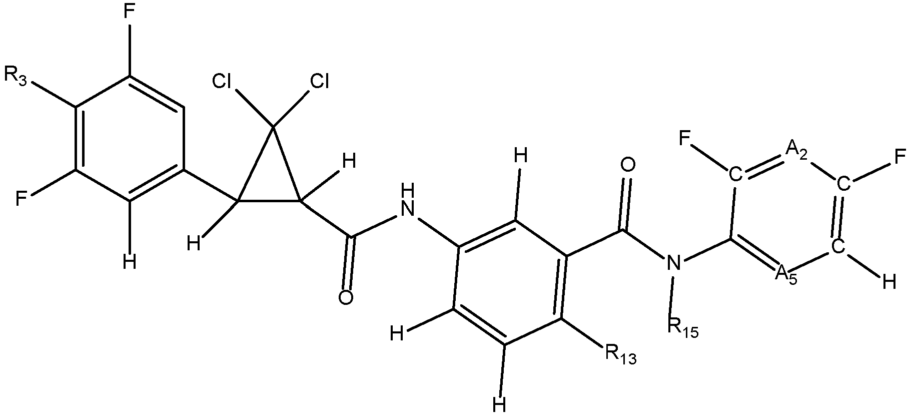
and N-oxides, veterinary acceptable acid addition salts, salt derivatives, solvates, ester derivatives, crystal polymorphs, isotopes, stereoisomers, and tautomers thereof,
wherein R³, R⁶, R⁹, R¹³, R¹⁵, A₂, and A₅ are defined as elsewhere in this description.

In an embodiment of the invention and/or embodiments thereof, the invention is directed to a compound of formula (LXXIX) 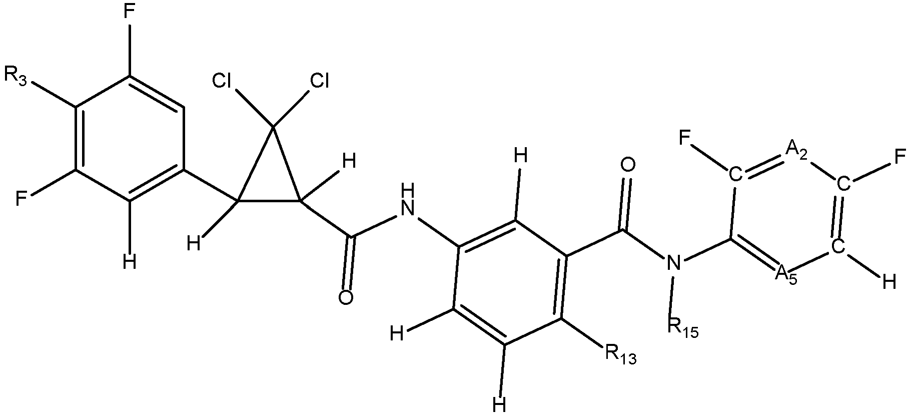
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₅ is CR₉, N=O or N ;
wherein no more than 2 of A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXIX) 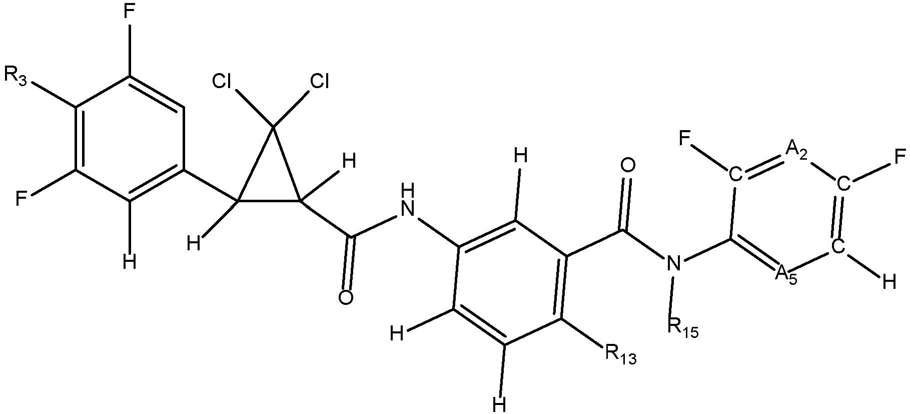
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
A₂ is CR₆, N=O, or N;
A₅ is CR₉, N=O or N;
wherein no more than 2 of A₂, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃;
**R¹⁵** is selected from the group consisting of H, and CH₃.

In an embodiment of the invention and/or embodiments thereof, the invention is directed to a compound of formula (LXXIX) 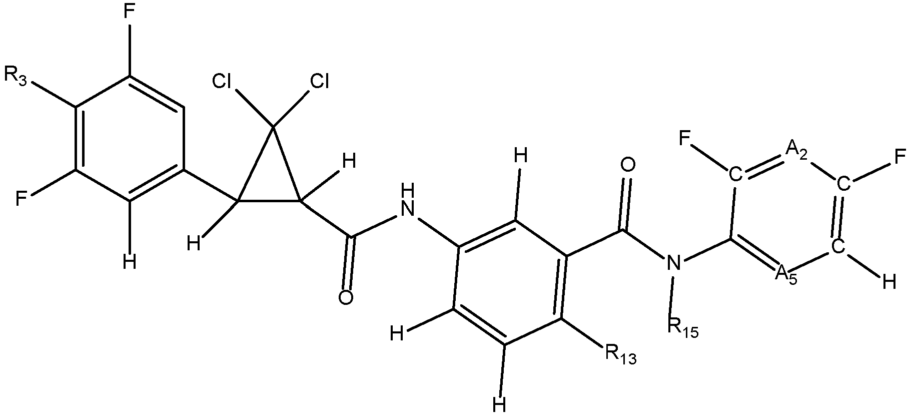
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
A₂ is CR₆ or N;
A₅ is CR₉ or N;
wherein no more than 2 of A₂, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXIX) 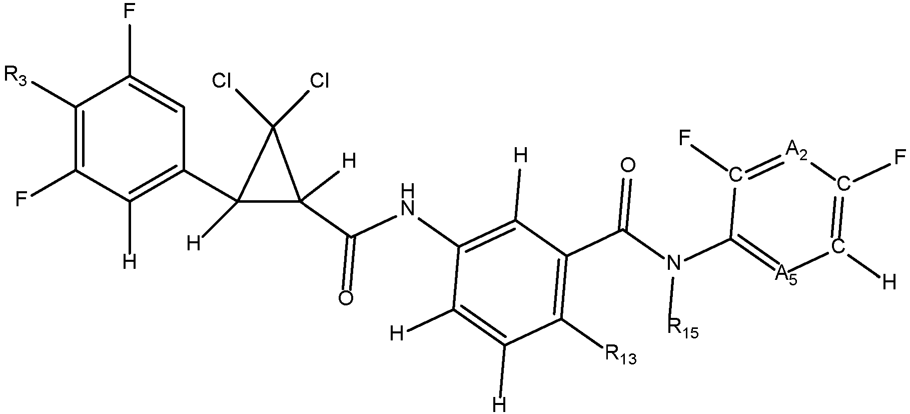
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
A₂ is CR₆ or N;
A₅ is CR₉ or N;
wherein 0 or 1 of A₂, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXIX) 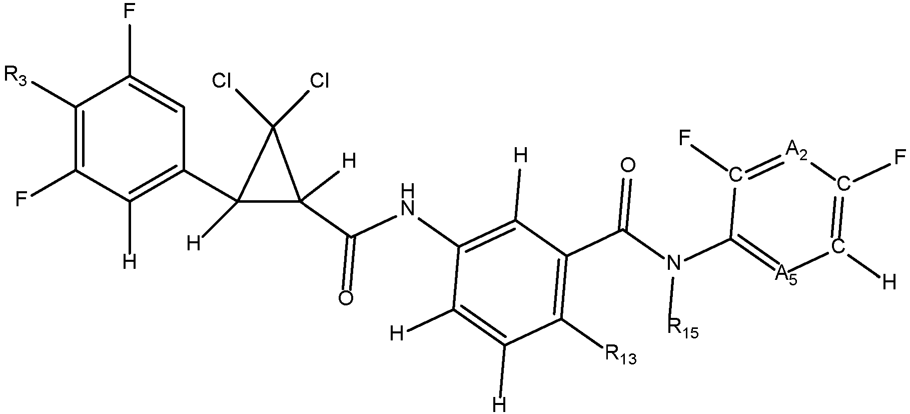
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I;
A₂ is CR₆ or N;
A₅ is CR₉;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXX) 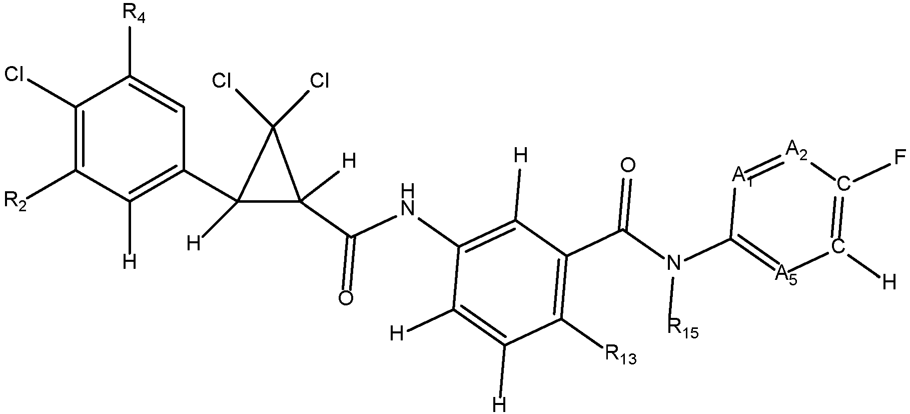
and N-oxides, veterinary acceptable acid addition salts, salt derivatives, solvates, ester derivatives, crystal polymorphs, isotopes, stereoisomers, and tautomers thereof,
wherein R², R⁴, R⁵, R⁶, R⁹, R¹³, R¹⁵, A₁, A₂, and A₅ are defined as elsewhere in this description.

In an embodiment of the invention and/or embodiments thereof, the invention is directed to a compound of formula (LXXX) 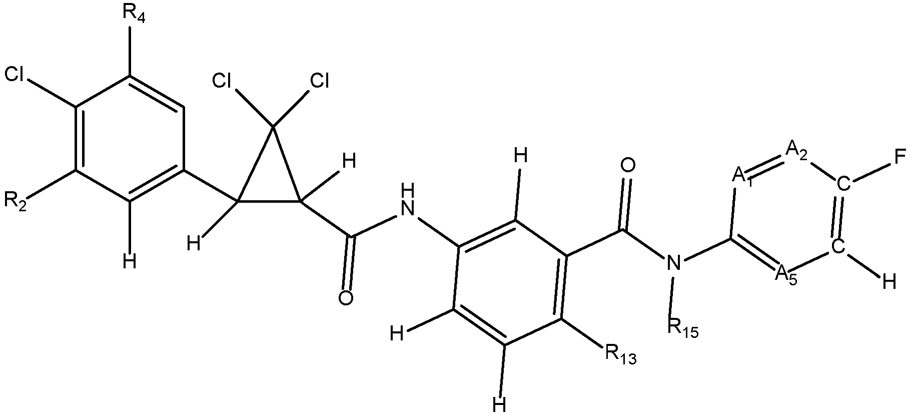
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₅ is CR₉, N=O or N ;
wherein no more than 2 of A₁, A₂, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXX) 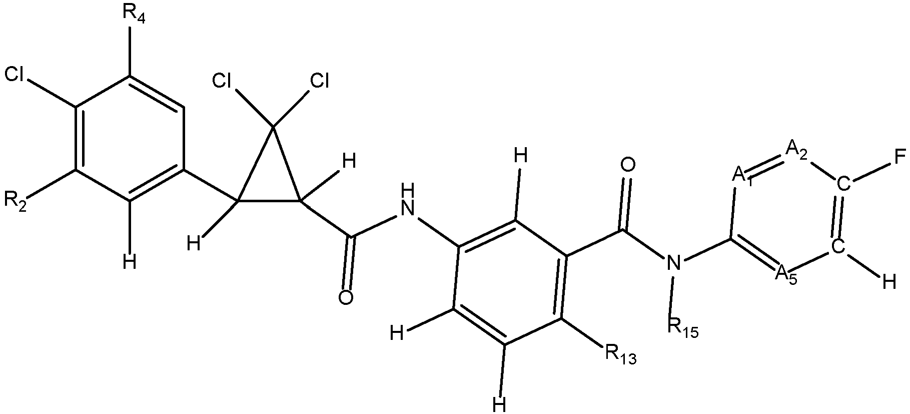
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃;
**R¹⁵** is selected from the group consisting of H, and CH₃.

In an embodiment of the invention and/or embodiments thereof, the invention is directed to a compound of formula (LXXX) 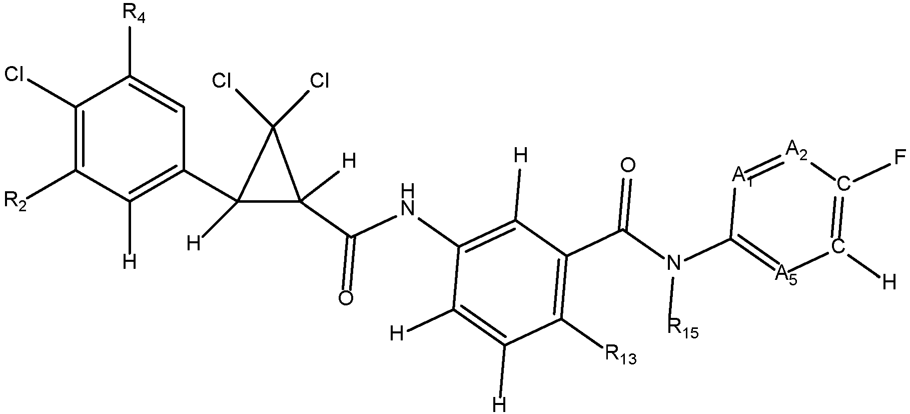
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXX) 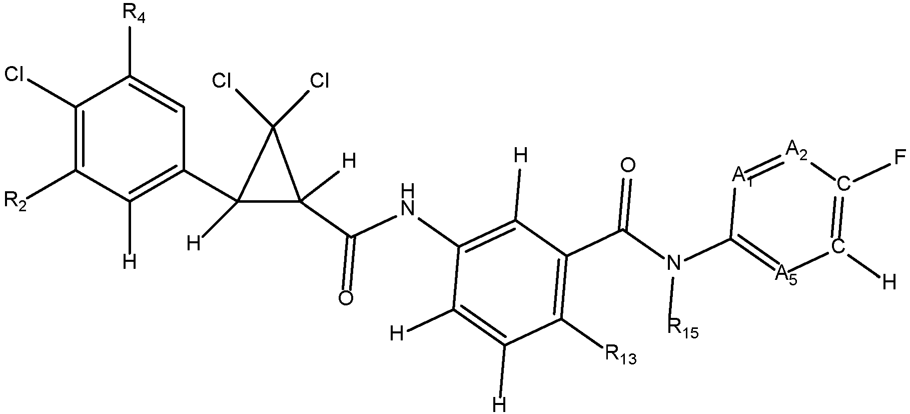
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXX) 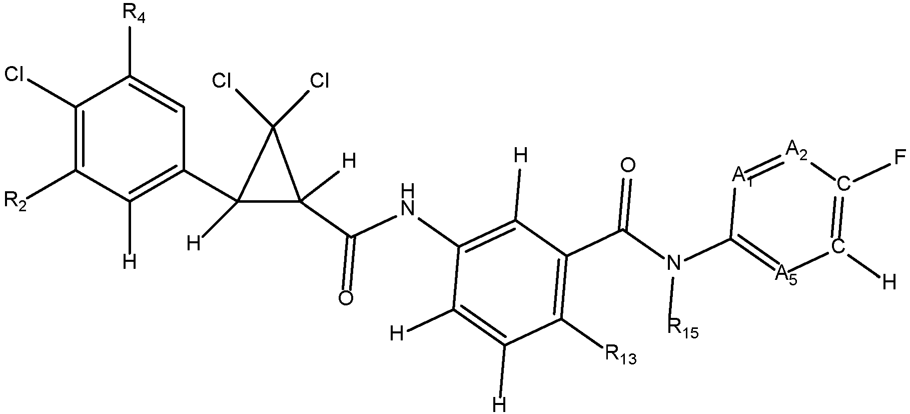
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A₁ is CR₅ or N;
A₂ is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXI) 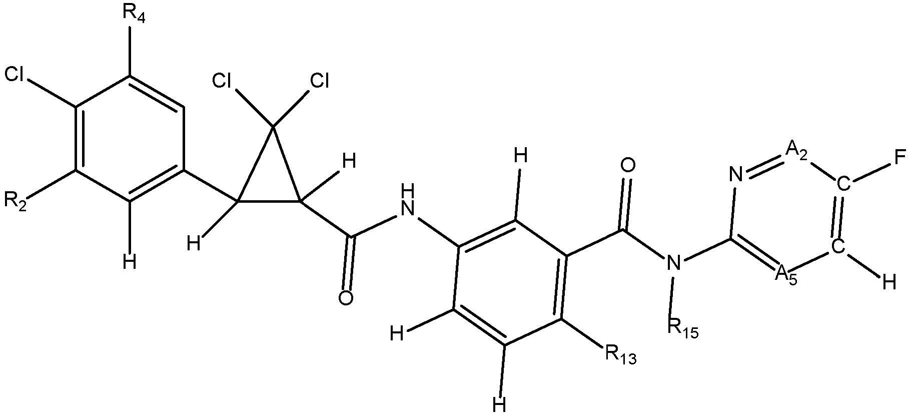
and N-oxides, veterinary acceptable acid addition salts, salt derivatives, solvates, ester derivatives, crystal polymorphs, isotopes, stereoisomers, and tautomers thereof,
wherein R², R⁴, R⁶, R⁹, R¹³, R¹⁵, A₂, and A₅ are defined as elsewhere in this description.

In an embodiment of the invention and/or embodiments thereof, the invention is directed to a compound of formula (LXXXI) 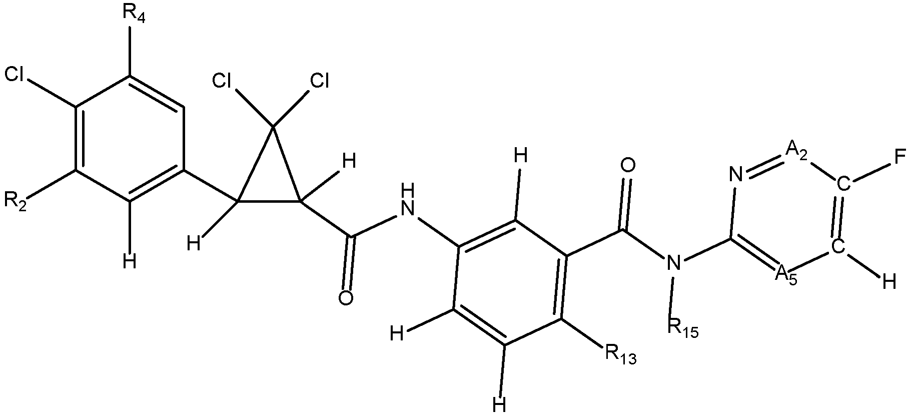
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₅ is CR₉, N=O or N ;
wherein no more than 2 of A₂, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXI) 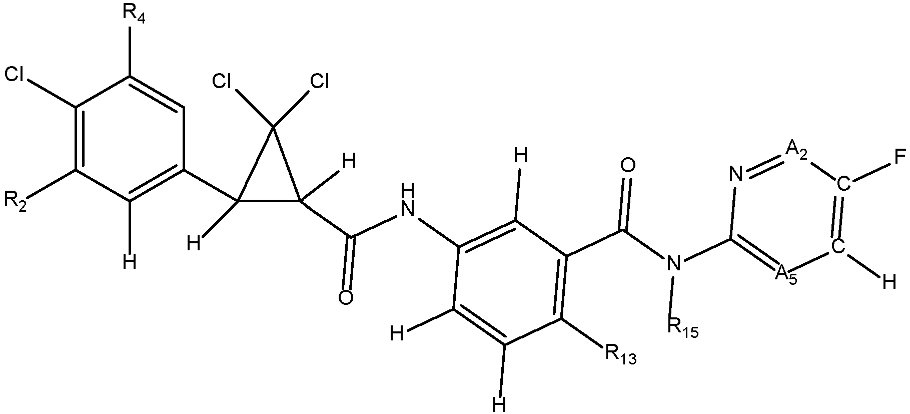
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₅ is CR₉, N=O or N;
wherein no more than 2 of A₂, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXI) 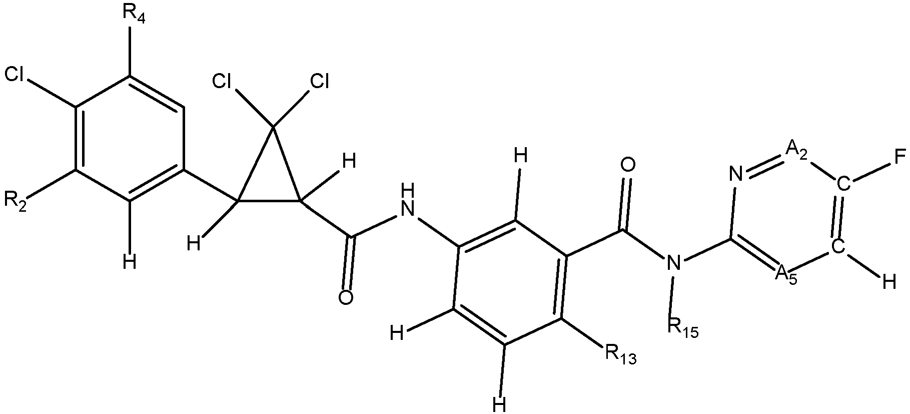
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₅ is CR₉ or N;
wherein no more than 2 of A₂, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXI) 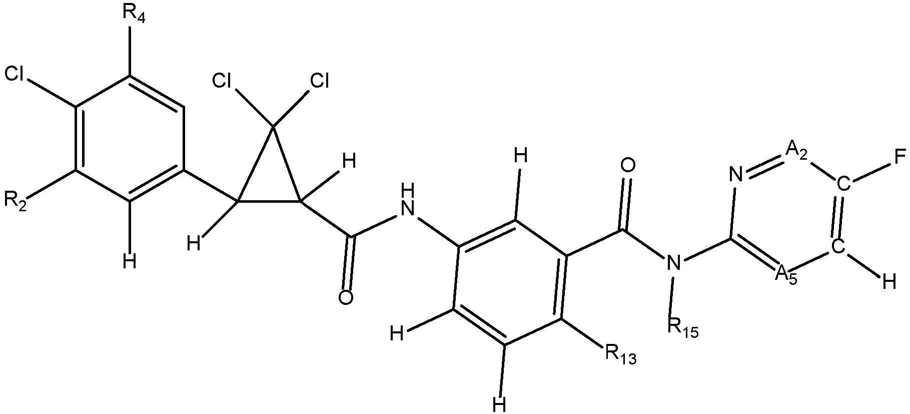
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A2 is CR₆ or N;
A₅ is CR₉ or N;
wherein 0 or 1 of A₂, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXI) 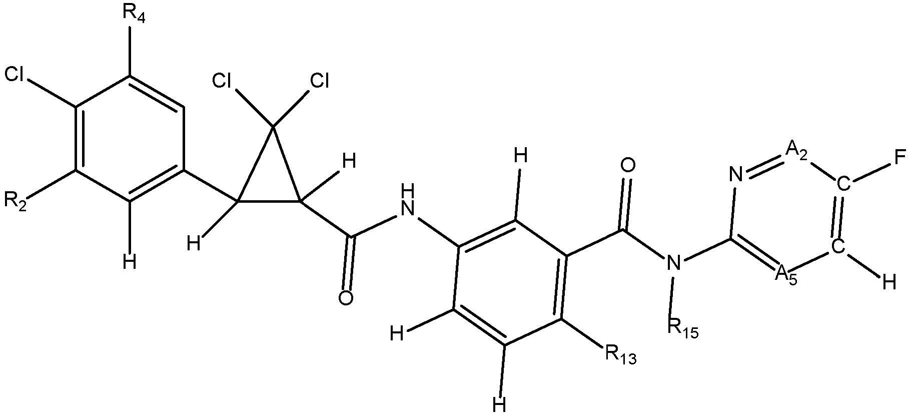
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A₂ is CR₆ or N;
A₅ is CR₉;
**R⁶** is selected from the group consisting of H, F, and NH(C₁-C₆)alkyl ;
**R⁹** is selected from the group consisting of Hand F ;
**R¹³** is Cl or F;
**R¹⁵** is H.

In an embodiment of the invention and/or embodiments thereof, the invention is directed to a compound of formula (LXXXII) 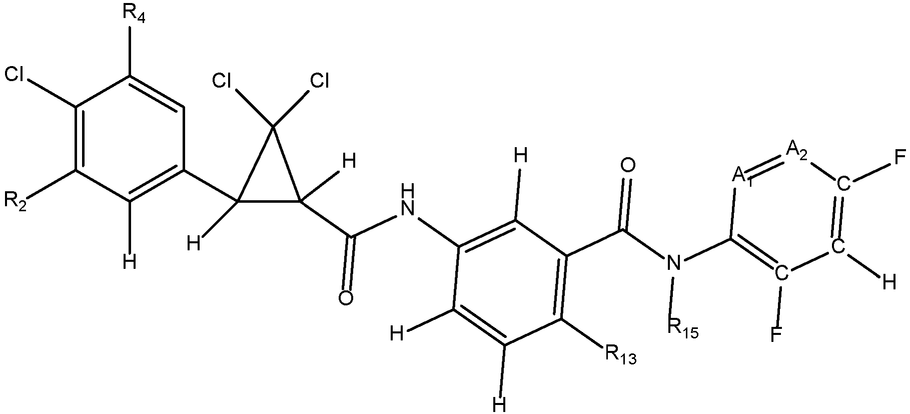
and N-oxides, veterinary acceptable acid addition salts, salt derivatives, solvates, ester derivatives, crystal polymorphs, isotopes, stereoisomers, and tautomers thereof,
wherein R², R⁴, R⁵, R⁶, R¹³, R¹⁵, A₁, and A₂, are defined as elsewhere in this description.

In an embodiment of the invention and/or embodiments thereof, the invention is directed to a compound of formula (LXXXII) 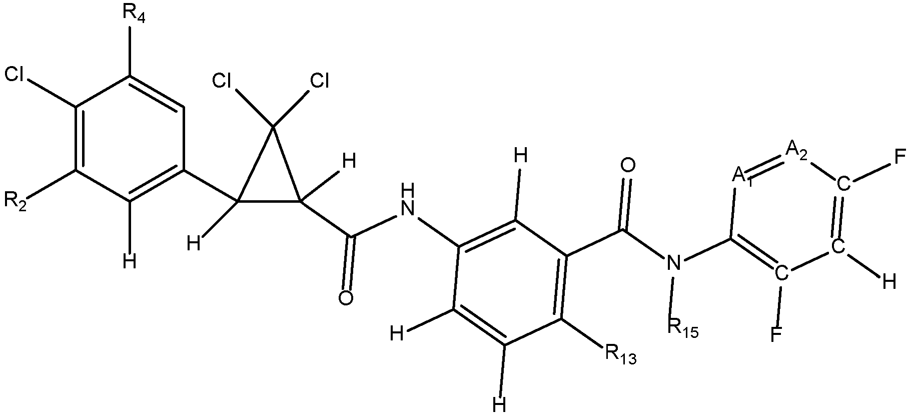
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
wherein no more than 2 of A₁, A₂, are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(Cₗ-C₆)alkyl)(Cₗ-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(Cₗ-C₆)alkyl)(Cₗ-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXII) 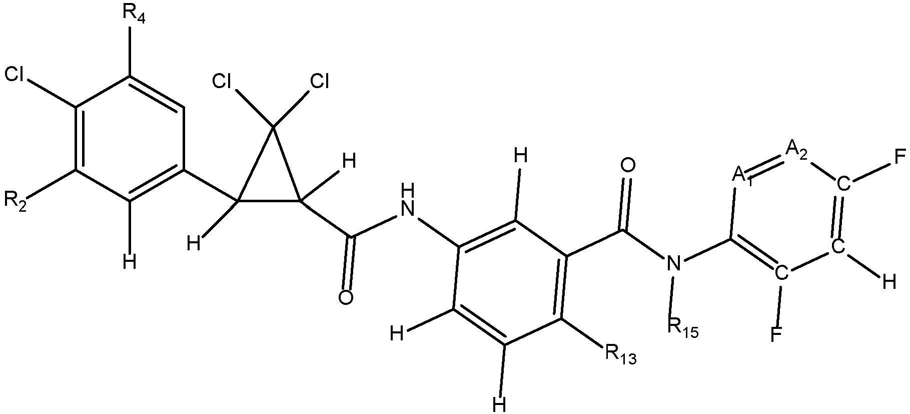
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
wherein no more than 2 of A₁, A₂, are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXII) 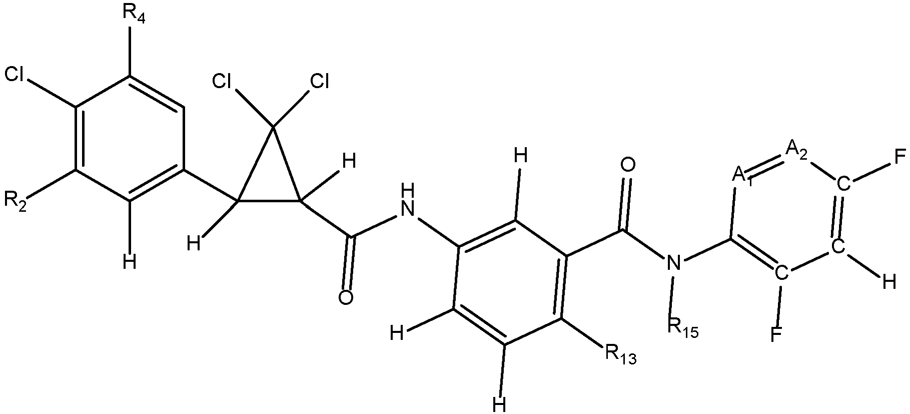
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
wherein no more than 2 of A₁, A₂, are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXII) 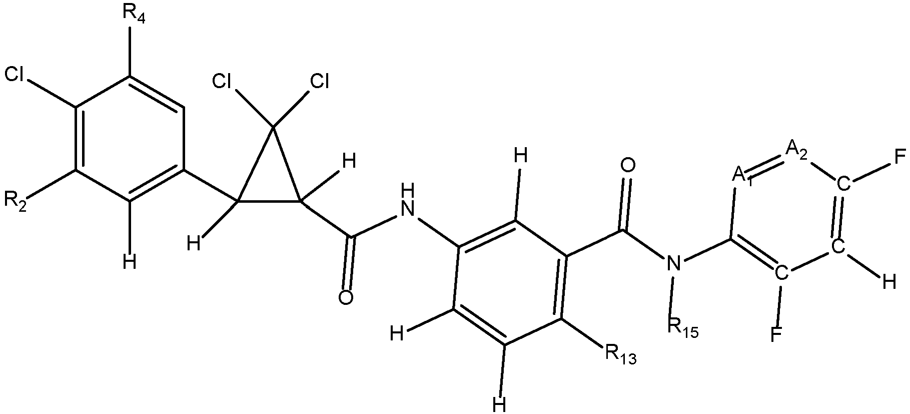
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A₁ is CR₅ or N;
A2 is CR₆ or N;
wherein 0 or 1 of A₁, A₂, are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXII) 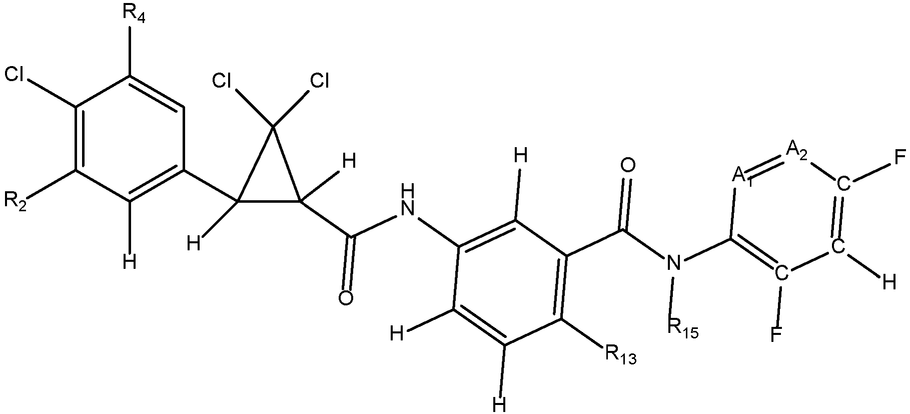
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A₁ is CR₅ or N;
A₂ is CR₆ or N;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R¹³** is Cl or F;
**R¹⁵** is H.

In an embodiment of the invention and/or embodiments thereof, the invention is directed to a compound of formula (LXXXIII) 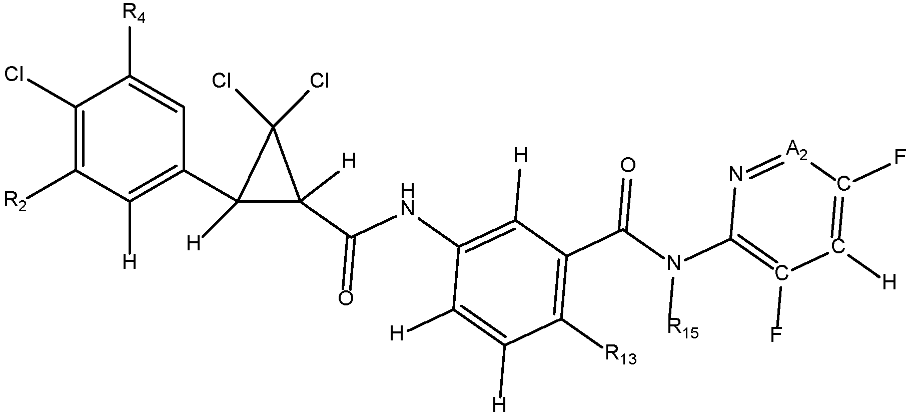
and N-oxides, veterinary acceptable acid addition salts, salt derivatives, solvates, ester derivatives, crystal polymorphs, isotopes, stereoisomers, and tautomers thereof,
wherein R², R⁴, R⁶, R¹³, R¹⁵, and A₂ are defined as elsewhere in this description.

In an embodiment of the invention and/or embodiments thereof, the invention is directed to a compound of formula (LXXXIII) 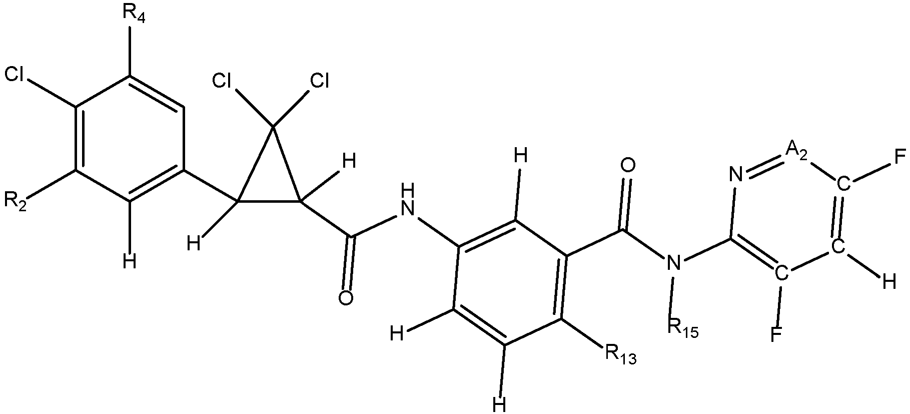
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(Cₗ-C₆)alkyl)(Cₗ-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXIII) 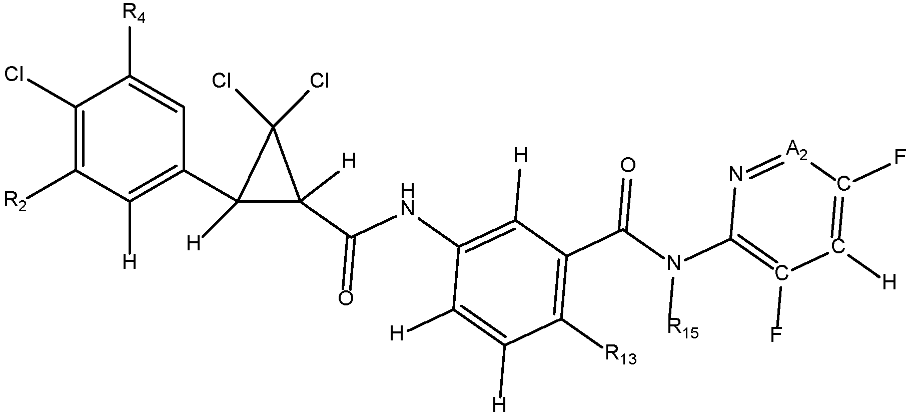
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIII) 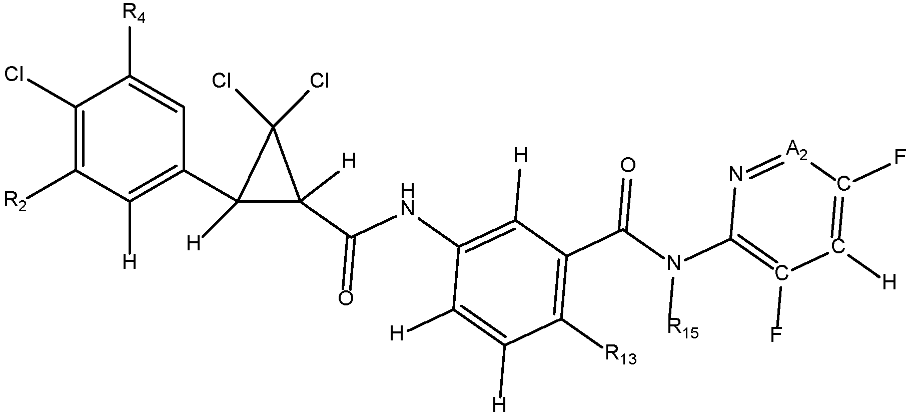
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIII) 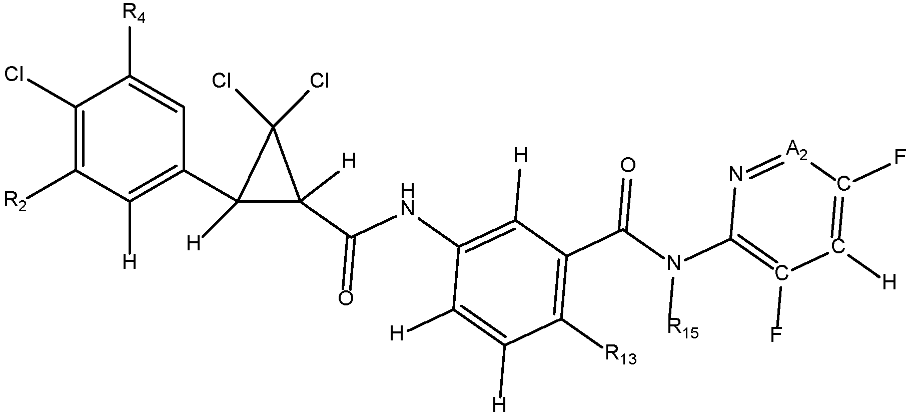
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A2 is CR₆ or N;
**R⁶** is selected from the group consisting of H, F, NH(C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXIII) 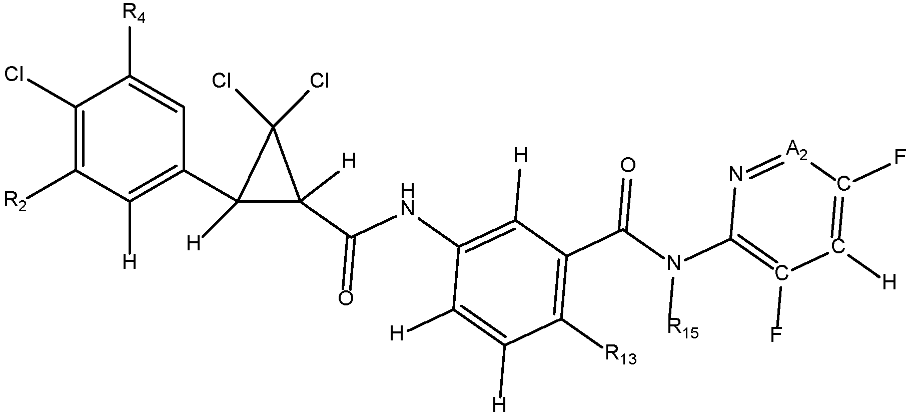
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A2 is CR₆ or N;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXIV) 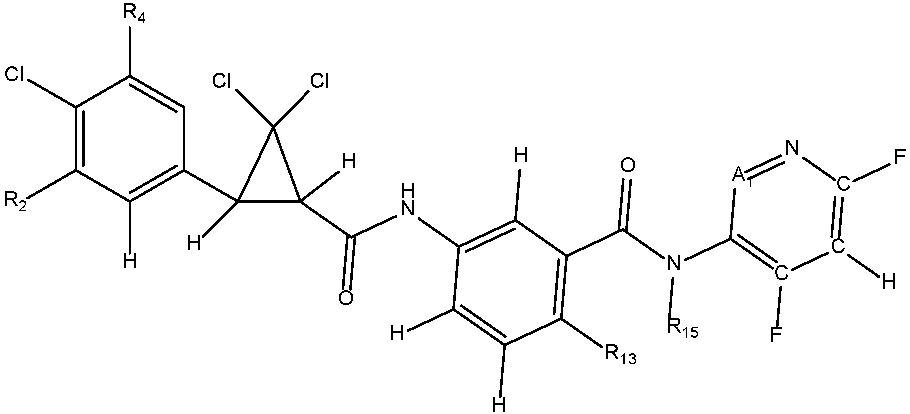
and N-oxides, veterinary acceptable acid addition salts, salt derivatives, solvates, ester derivatives, crystal polymorphs, isotopes, stereoisomers, and tautomers thereof,
wherein R², R⁴, R⁵, R¹³, R¹⁵, and A, are defined as elsewhere in this description.

In an embodiment of the invention and/or embodiments thereof, the invention is directed to a compound of formula (LXXXIV) 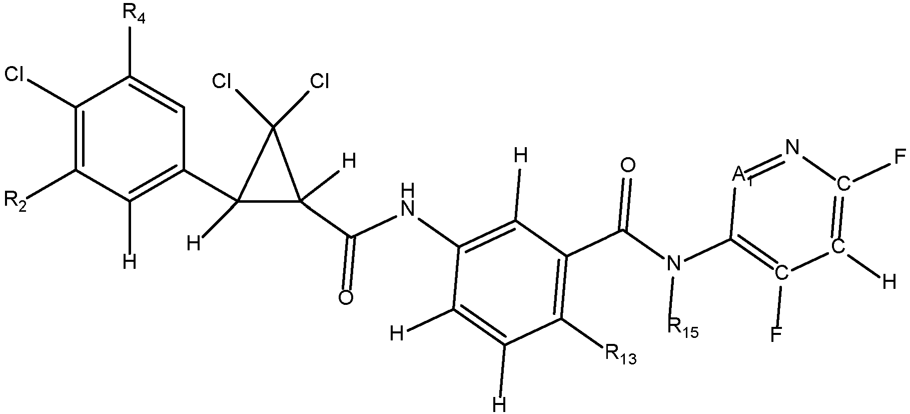
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(Cₗ-C₆)alkyl)(Cₗ-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXIV) 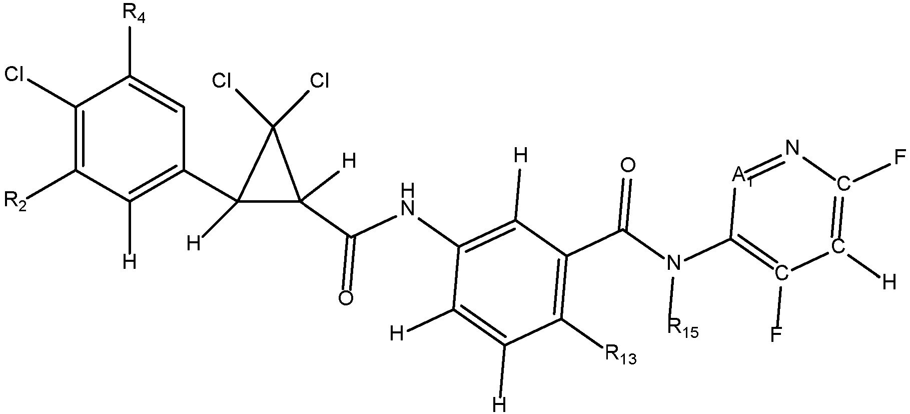
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIV) 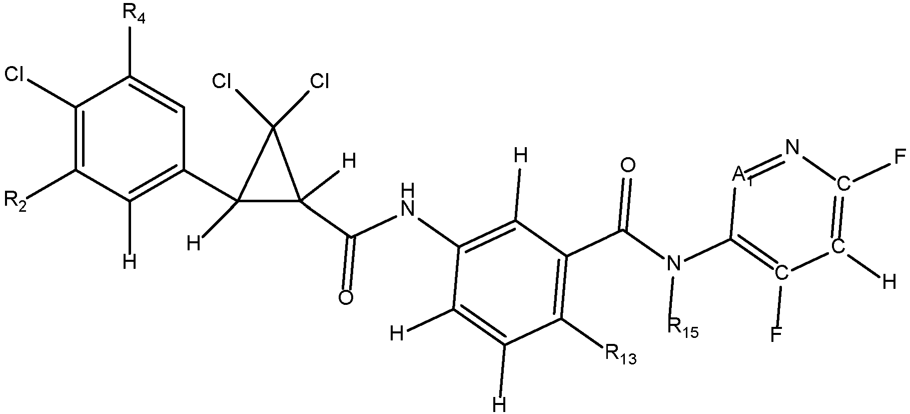
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIV) 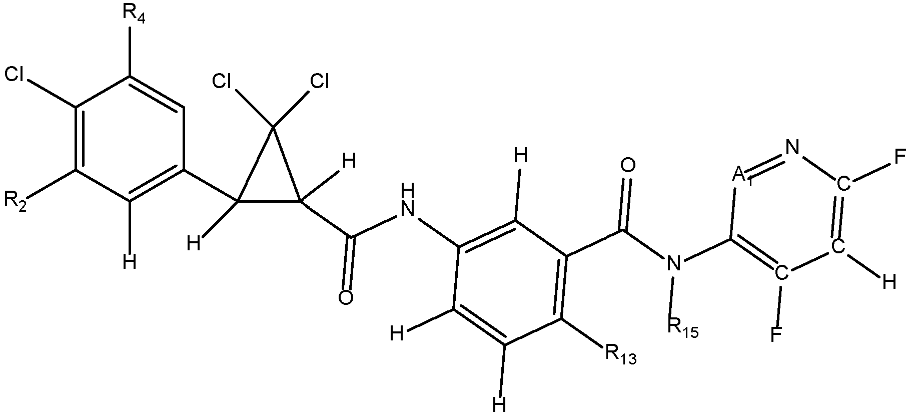
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A₁ is CR₅ or N;
**R⁵** is selected from the group consisting of H, F, Cl, CN, NO₂, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXIV) 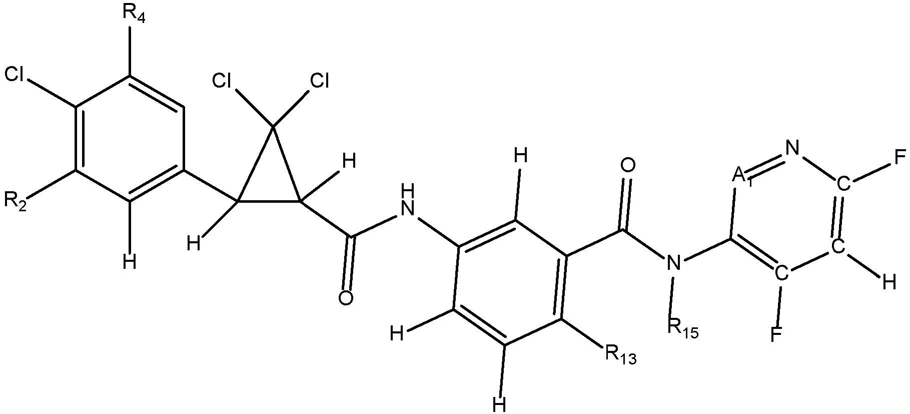
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A₁ is CR₅ or N;
**R⁵** is selected from the group consisting of H, F, Cl, CN and (C₁-C₆)alkyl ;
**R¹³** is Cl or F;
**R¹⁵** is H.

In an embodiment of the invention and/or embodiments thereof, the invention is directed to a compound of formula (LXXXV) 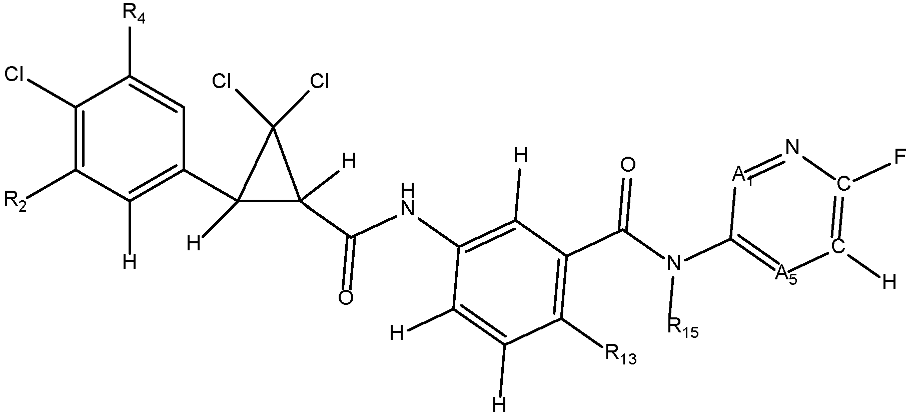
and N-oxides, veterinary acceptable acid addition salts, salt derivatives, solvates, ester derivatives, crystal polymorphs, isotopes, stereoisomers, and tautomers thereof,
wherein R², R⁴, R⁵, R⁹, R¹³, R¹⁵, A₁, and A₅ are defined as elsewhere in this description.

In an embodiment of the invention and/or embodiments thereof, the invention is directed to a compound of formula (LXXXV) 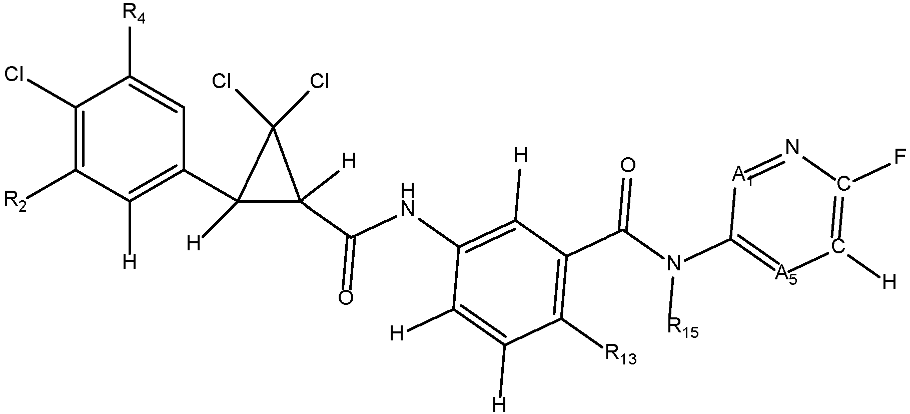
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₅ is CR₉, N=O or N ;
wherein no more than 2 of A₁, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(Cₗ-C₆)alkyl)(Cₗ-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXV) 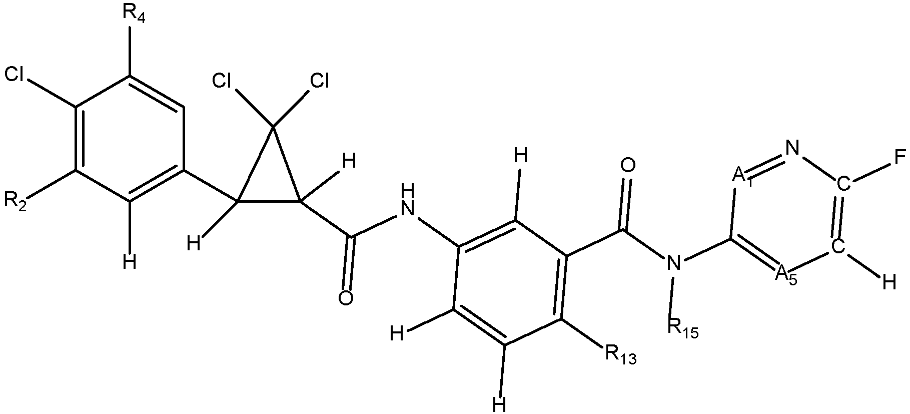
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₅ is CR₉, N=O or N;
wherein no more than 2 of A₁, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXV) 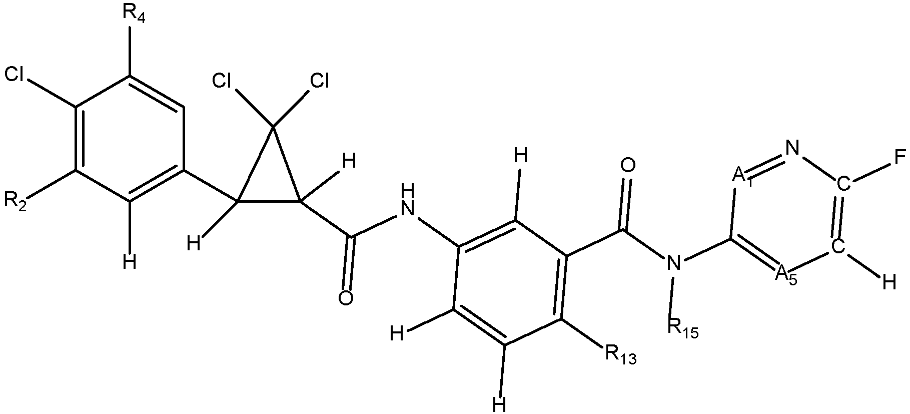
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₅ is CR₉ or N;
wherein no more than 2 of A₁, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXV) 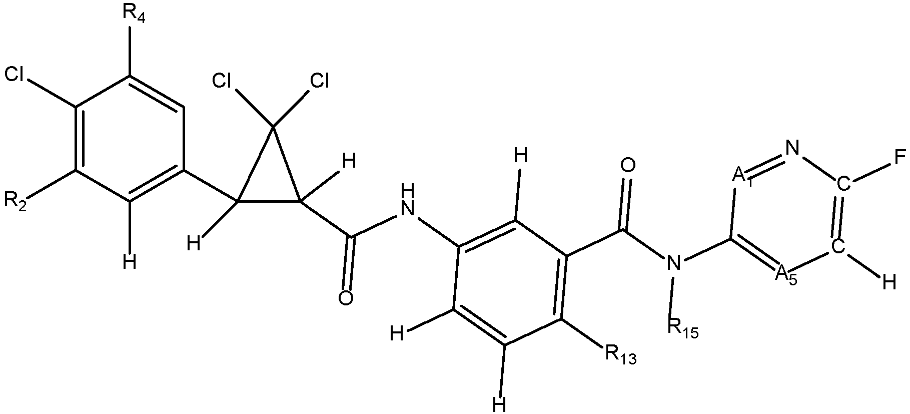
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R⁴** is selected from the group consisting of H, F, Cl, Br, (C₁-C₆)alkyl, and (C₁-C₆)haloalkyl;
A₁ is CR₅ or N;
A₅ is CR₉ or N;
wherein 0 or 1 of A₁, A₅ are N;
**R⁵** is selected from the group consisting of H, F, Cl, CN, NO₂, NH(C₁-C₆)alkyl, and (C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXV) 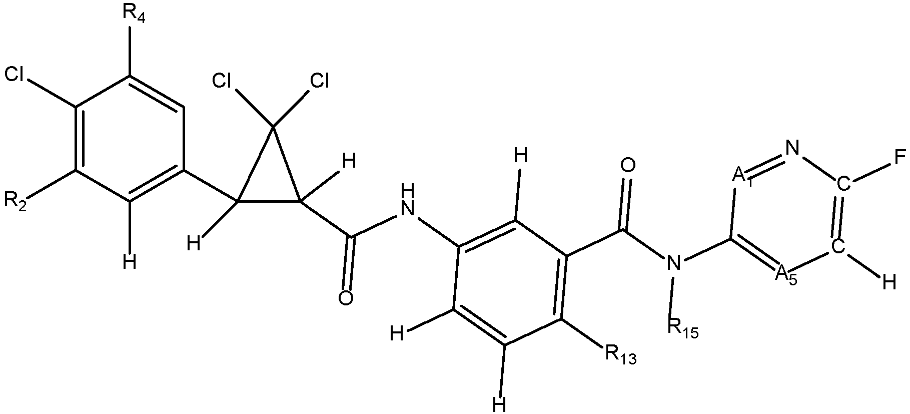
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R⁴** is selected from the group consisting of H, F, Cl, Br;
A₁ is CR₅ or N;
A₅ is CR₉;
**R⁵** is selected from the group consisting of H, F, Cl, CN and (C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXVI) 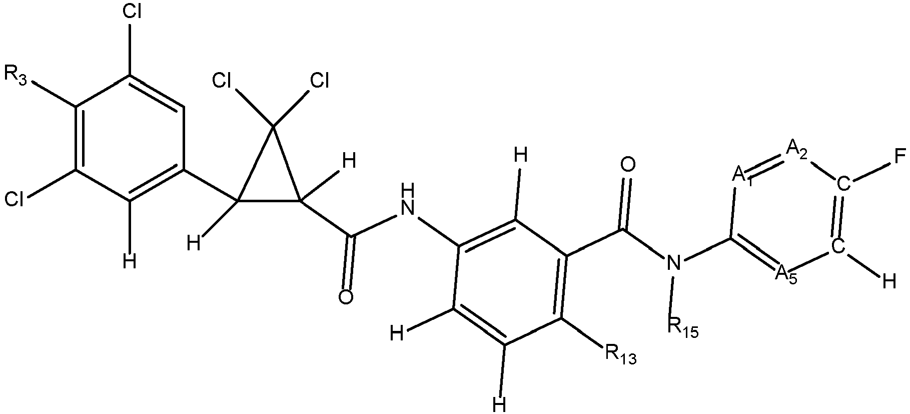
and N-oxides, veterinary acceptable acid addition salts, salt derivatives, solvates, ester derivatives, crystal polymorphs, isotopes, stereoisomers, and tautomers thereof,
wherein R³, R⁵, R⁶, R⁹, R¹³, R¹⁵, A₁, A₂, and A₅ are defined as elsewhere in this description.

In an embodiment of the invention and/or embodiments thereof, the invention is directed to a compound of formula (LXXXVI) 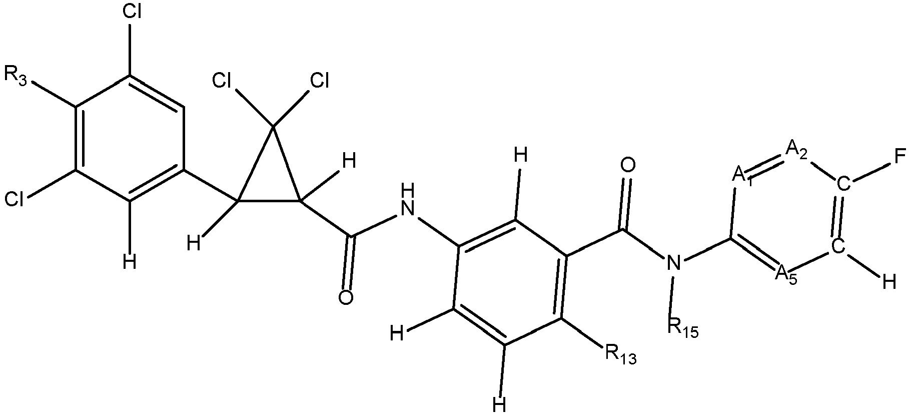
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₅ is CR₉, N=O or N ;
wherein no more than 2 of A₁, A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)_{Z}NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)_{Z}NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXVI) 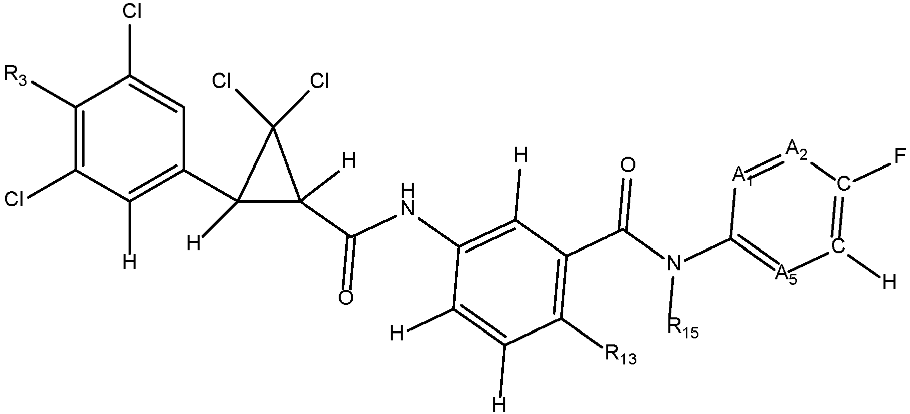
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVI) 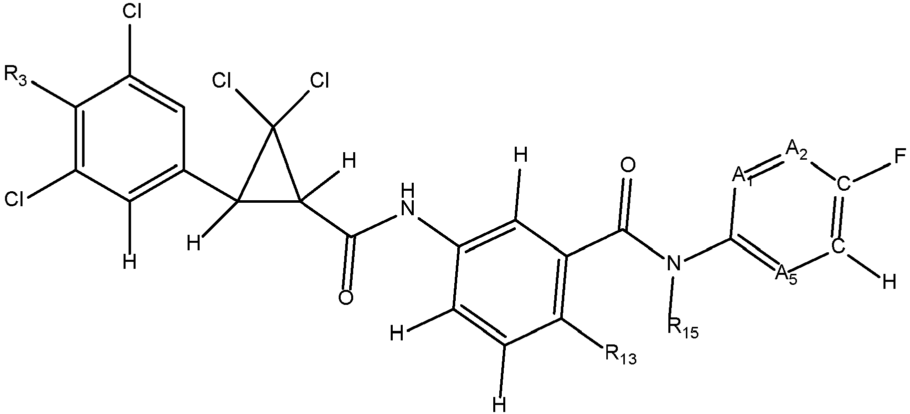
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVI) 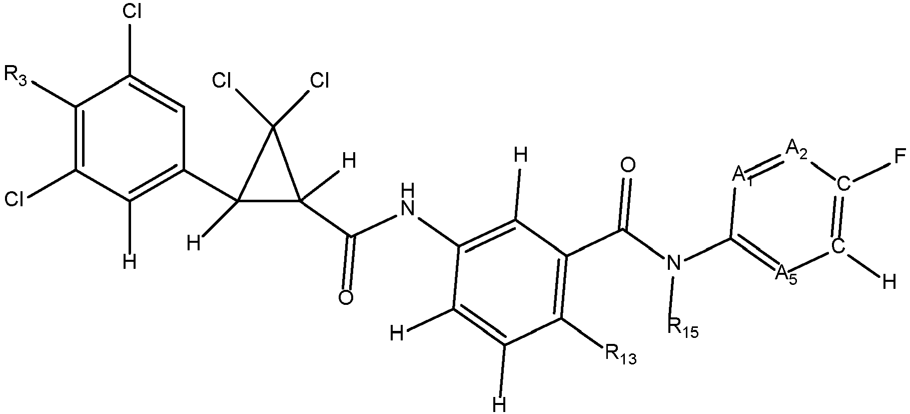
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
A₁ is CR₅ or N;
A₂ is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-( C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXVI) 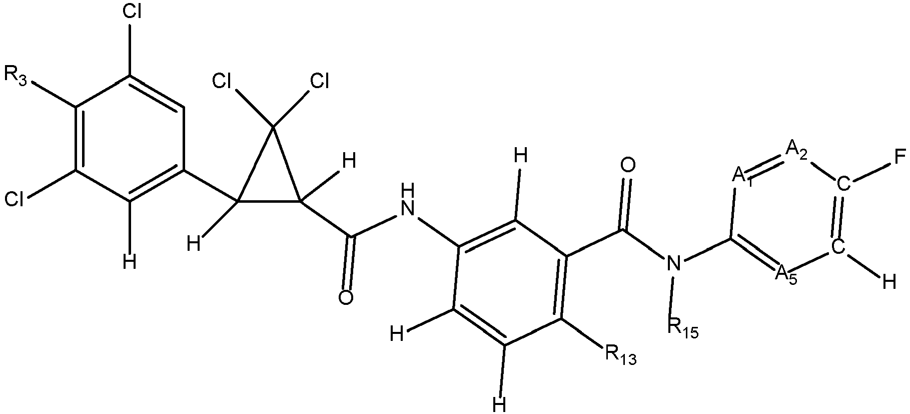
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I;
A₁ is CR₅ or N;
A₂ is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXVII) 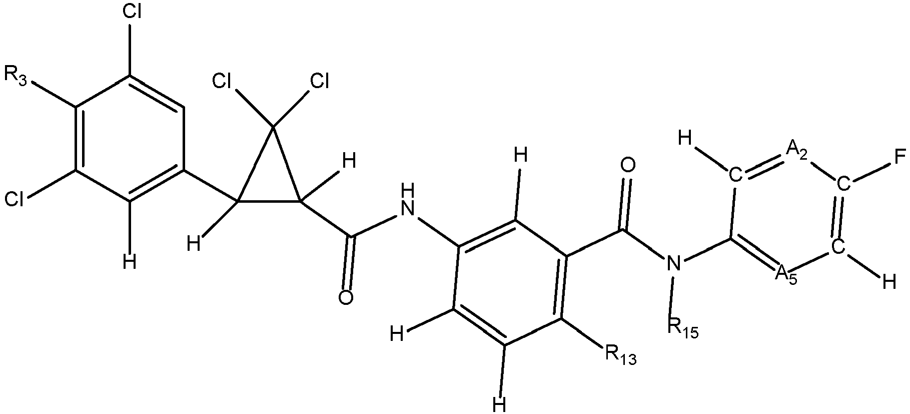
and N-oxides, veterinary acceptable acid addition salts, salt derivatives, solvates, ester derivatives, crystal polymorphs, isotopes, stereoisomers, and tautomers thereof,
wherein R³, R⁵, R⁹, R¹³, R¹⁵, A₂, and A₅ are defined as elsewhere in this description.

In an embodiment of the invention and/or embodiments thereof, the invention is directed to a compound of formula (LXXXVII) 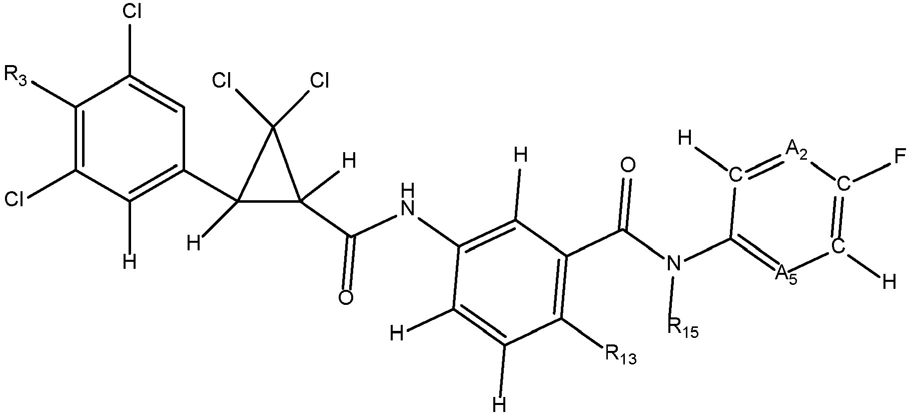
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₅ is CR₉, N=O or N ;
wherein no more than 2 of A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)_{Z}NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)_{Z}NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXVII) 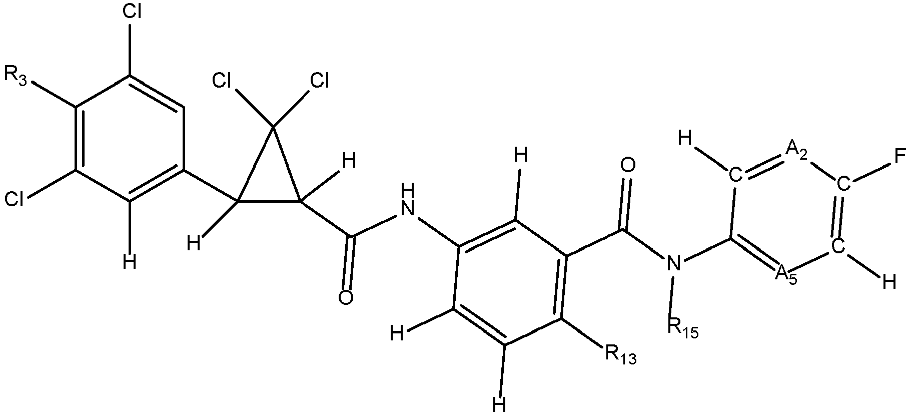
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
A₂ is CR₆, N=O, or N;
A₅ is CR₉, N=O or N;
wherein no more than 2 of A₂, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVII) 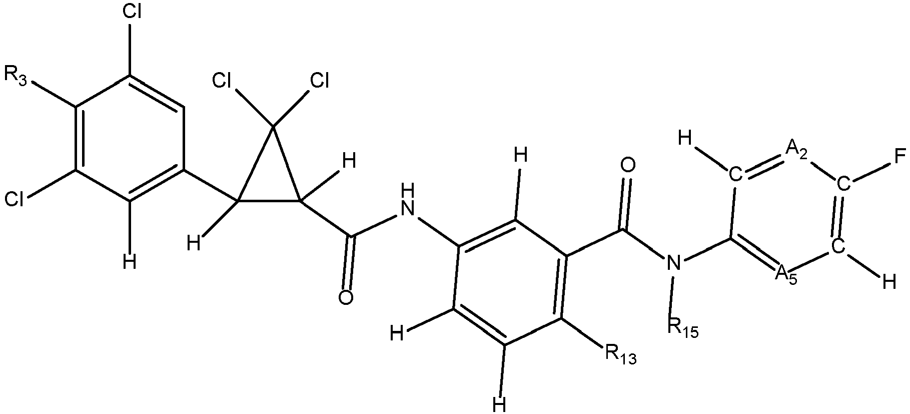
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
A₂ is CR₆ or N;
A₅ is CR₉ or N;
wherein no more than 2 of A₂, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVII) 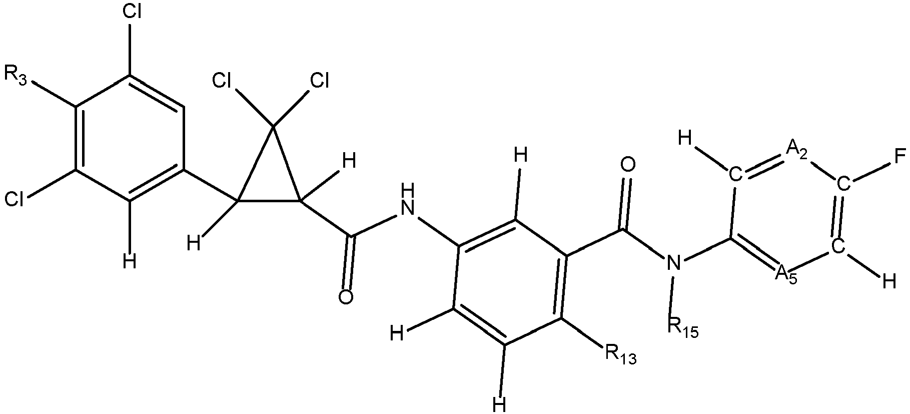
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy ;
A2 is CR₆ or N;
A₅ is CR₉ or N;
wherein 0 or 1 of A₂, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXVII) 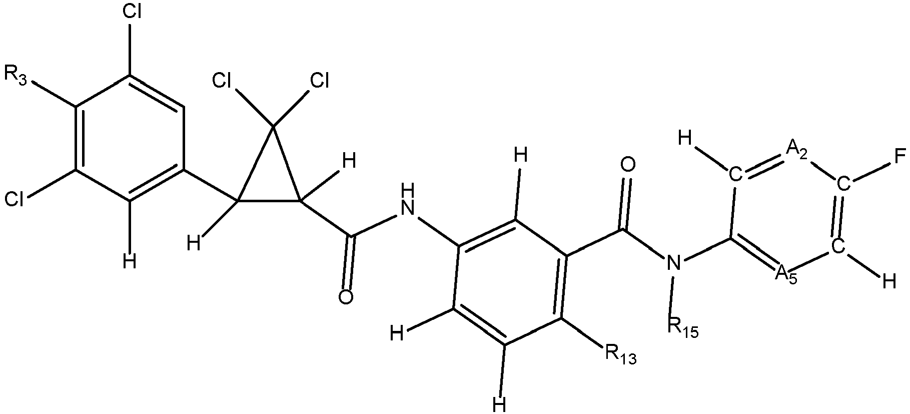
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I ;
A₂ is CR₆ or N;
A₅ is CR₉;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXVIII) 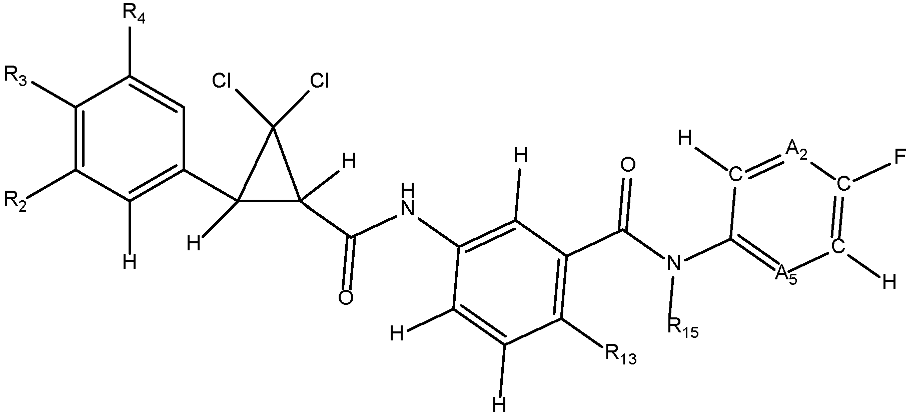
and N-oxides, veterinary acceptable acid addition salts, salt derivatives, solvates, ester derivatives, crystal polymorphs, isotopes, stereoisomers, and tautomers thereof,
wherein R², R³, R⁴, R⁶, R⁹, R¹³, R¹⁵, A₂, and A₅ are defined as elsewhere in this description.

In an embodiment of the invention and/or embodiments thereof, the invention is directed to a compound of formula (LXXXVIII) 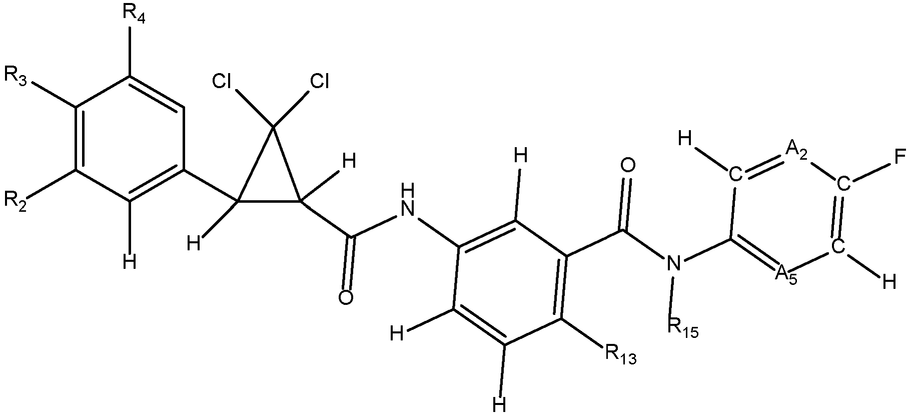
and N-oxides, veterinary acceptable acid addition salts, salt derivatives, solvates, ester derivatives, crystal polymorphs, isotopes, stereoisomers, and tautomers thereof,
wherein
A₂ is CR₆, N=O, or N;
A₅ is CR₉, N=O or N ;
wherein no more than 2 of A₂, A₅ are N or N=O;
**R²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXVIII) 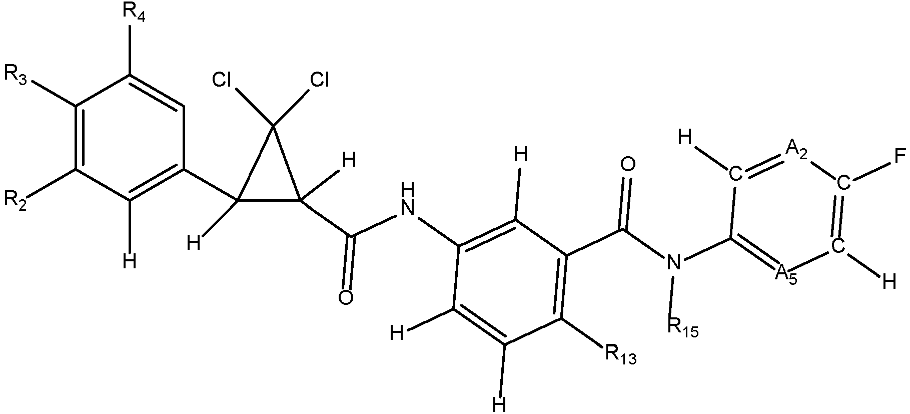
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₂ is CR₆, N=O, or N;
A₅ is CR₉, N=O or N;
wherein no more than 2 of A₂, A₅ are N or N=O;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVIII) 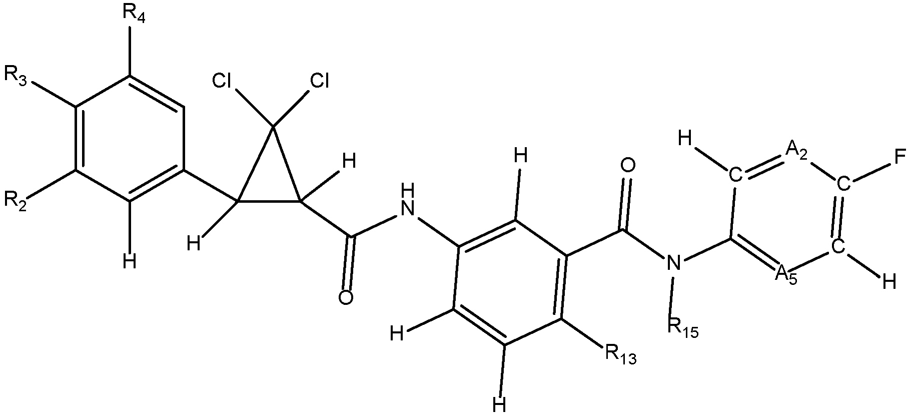
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₂ is CR₆ or N;
A₅ is CR₉ or N;
wherein no more than 2 of A₂, A₅ are N;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXVIII) 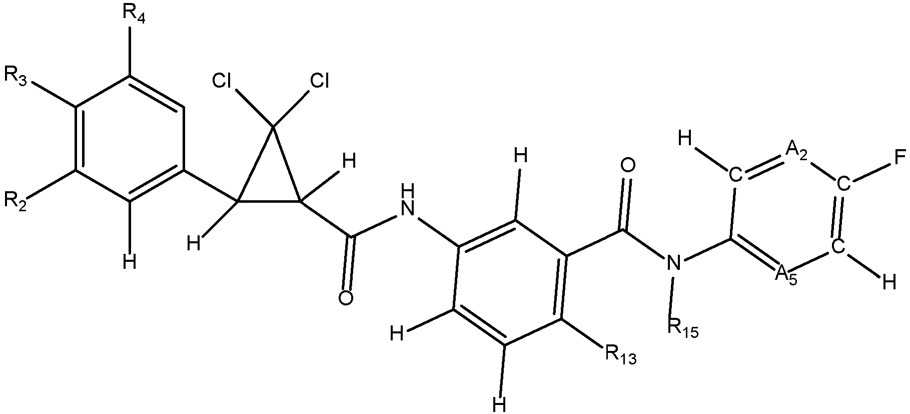
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br, (C₁-C₆)alkyl, and (C₁-C₆)haloalkyl;
**R³** is selected from the group consisting of H, F, Cl, Br, I, (C₁-C₆)haloalkyl, and (C₁-C₆) haloalkoxy ;
**R⁴** is selected from the group consisting of H, F, Cl, Br, (C₁-C₆)alkyl, and (C₁-C₆)haloalkyl;
A2 is CR₆ or N;
A₅ is CR₉ or N;
wherein 0 or 1 of A₂, A₅ are N;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXVIII) 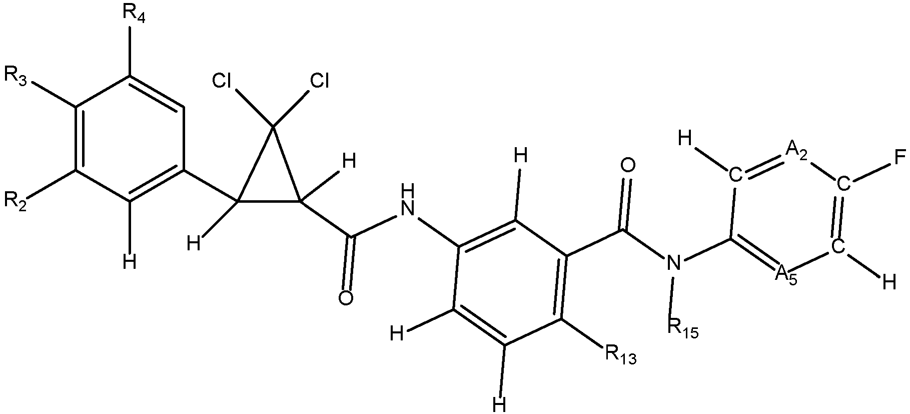
and N-oxides, veterinary acceptable acid addition salts, salt derivatives, solvates, ester derivatives, crystal polymorphs, isotopes, stereoisomers, and tautomers thereof,
wherein
**R²** is selected from the group consisting of H, F, Cl, Br;
**R³** is selected from the group consisting of H, F, Cl, Br, and I ;
**R⁴** is selected from the group consisting of H, F, Cl, Br;
A₂ is CR₆ or N;
A₅ is CR₉;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (LXXXIX) 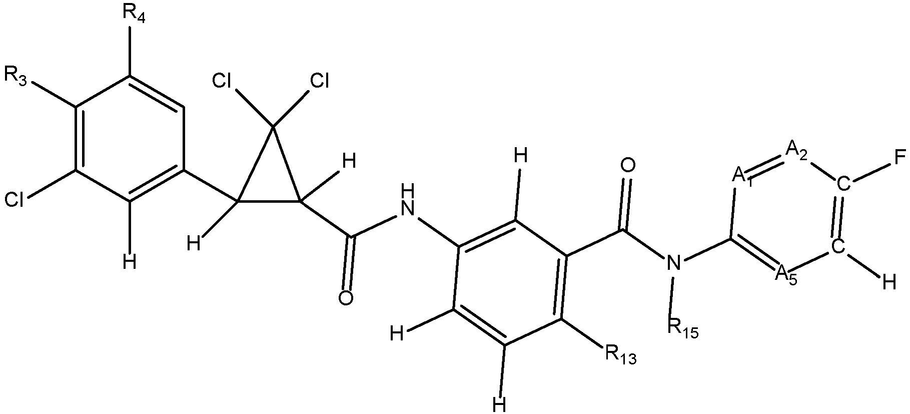
and N-oxides, veterinary acceptable acid addition salts, salt derivatives, solvates, ester derivatives, crystal polymorphs, isotopes, stereoisomers, and tautomers thereof,
wherein R³, R⁴, R⁵, R⁶, R⁹, R¹³, R¹⁵, A₁, A₂, and A₅ are defined as elsewhere in this description.

In an embodiment of the invention and/or embodiments thereof, the invention is directed to a compound of formula (LXXXIX) 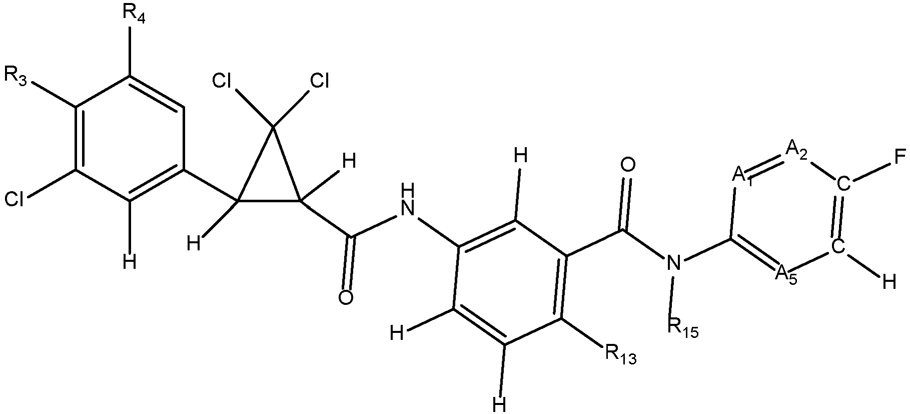
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₅ is CR₉, N=O or N ;
wherein no more than 2 of A₁, A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅ ;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (LXXXIX) 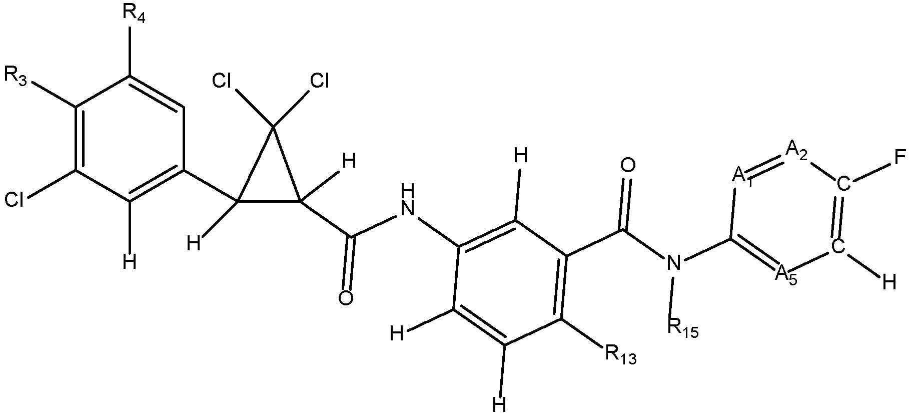
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIX) 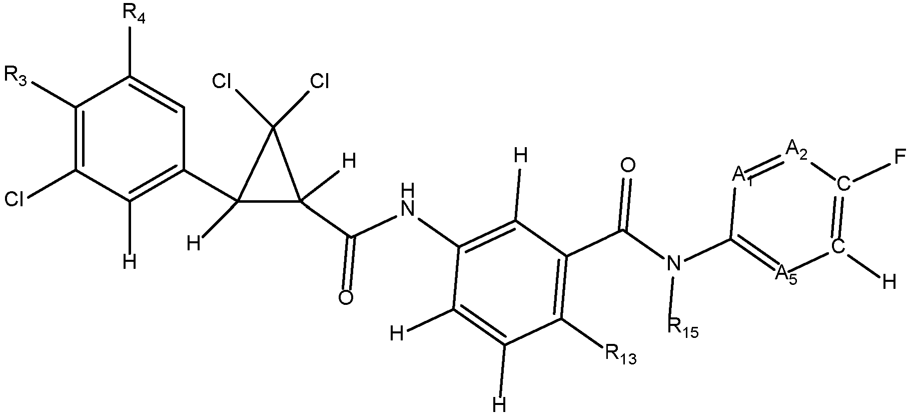
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (LXXXIX) 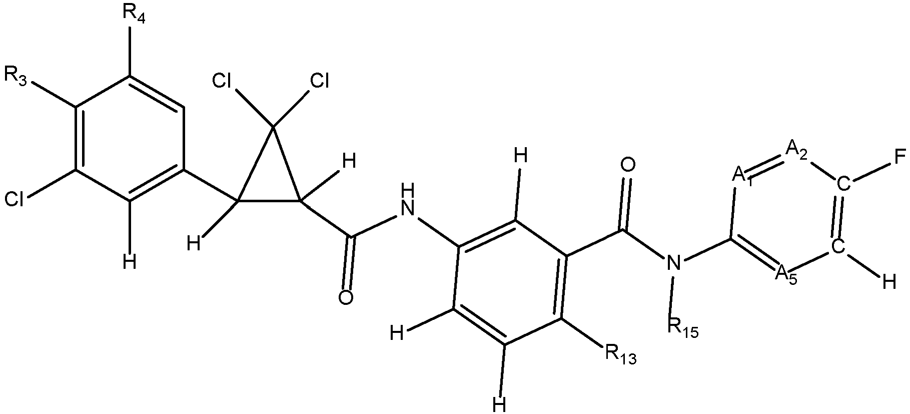
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₁ is CR₅ or N;
A2 is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (LXXXIX) 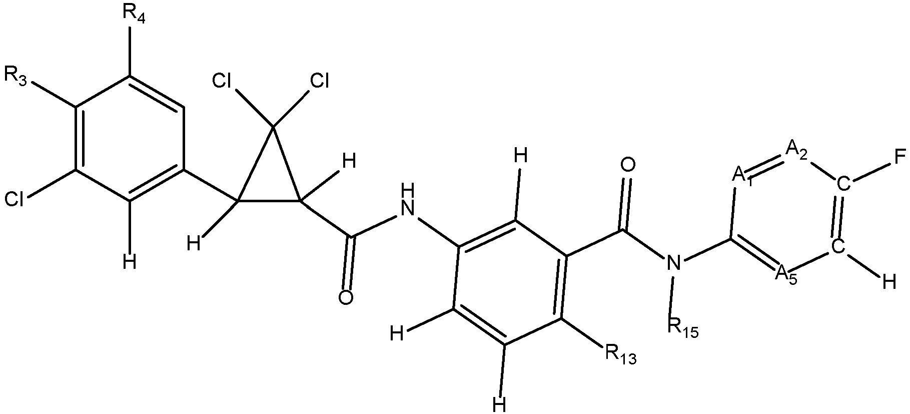
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I ;
**R⁴** is selected from the group consisting of H, F, Cl, Br;
A₁ is CR₅ or N;
A2 is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹³** is Cl or F;
**R¹⁵** is H.

In an embodiment of the invention and/or embodiments thereof, the invention is directed to a compound of formula (XC) 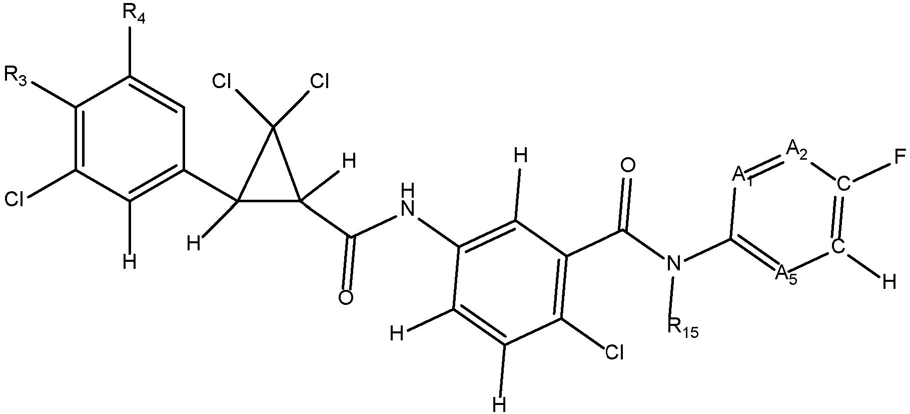
and N-oxides, veterinary acceptable acid addition salts, salt derivatives, solvates, ester derivatives, crystal polymorphs, isotopes, stereoisomers, and tautomers thereof,
wherein R³, R⁴, R⁵, R⁶, R⁹, R¹⁵, A₁, A₂, and A₅ are defined as elsewhere in this description.

In an embodiment of the invention and/or embodiments thereof, the invention is directed to a compound of formula (XC) 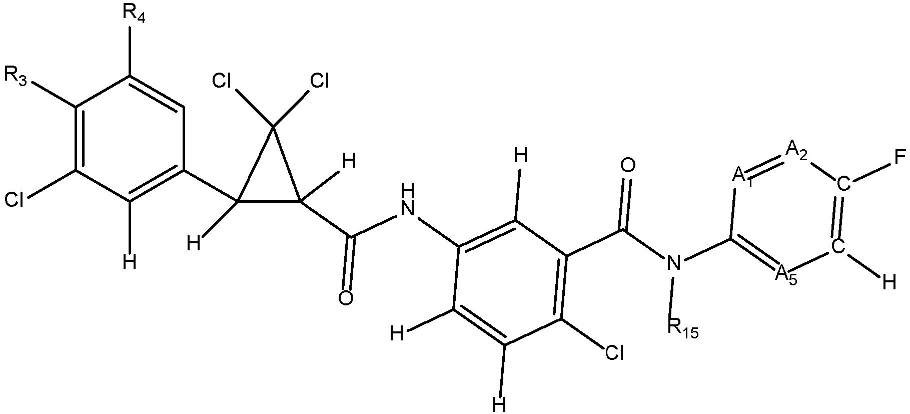
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
A₅ is CR₉, N=O or N ;
wherein no more than 2 of A₁, A₂, A₅ are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, CI, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹⁵** is selected from the group consisting of H, (C₁-C₆)alkyl.

In an embodiment of the invention and/or embodiments thereof, the invention is directed to a compound of formula (XC) 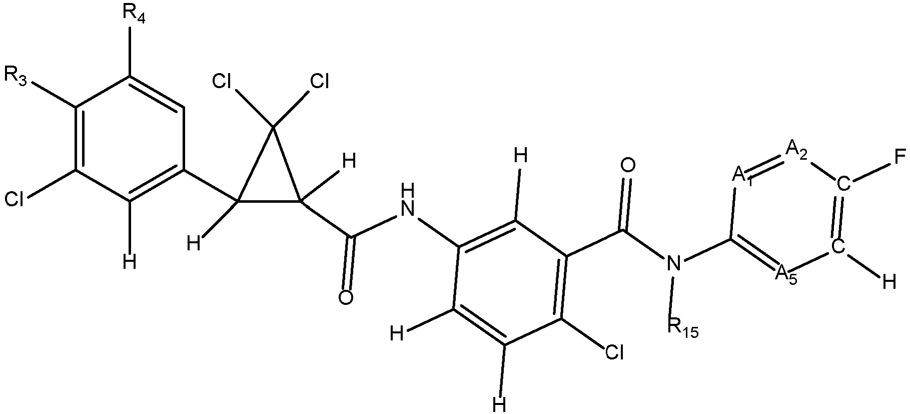
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₂ is CR₆, N=O, or N;
A₅ is CR₉, N=O or N;
wherein no more than 2 of A₁, A₂, A₅ are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹⁵** is selected from the group consisting of H, and CH₃.

In an embodiment of the invention and/or embodiments thereof, the invention is directed to a compound of formula (XC) 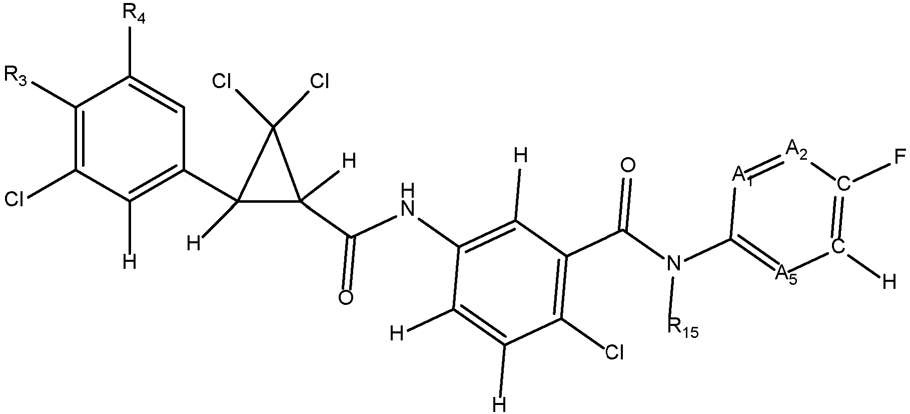
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₂ is CR₆ or N;
A₅ is CR₉ or N;
wherein no more than 2 of A₁, A₂, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹⁵** is selected from the group consisting of H, and CH₃.

In an embodiment of the invention and/or embodiments thereof, the invention is directed to a compound of formula (XC) 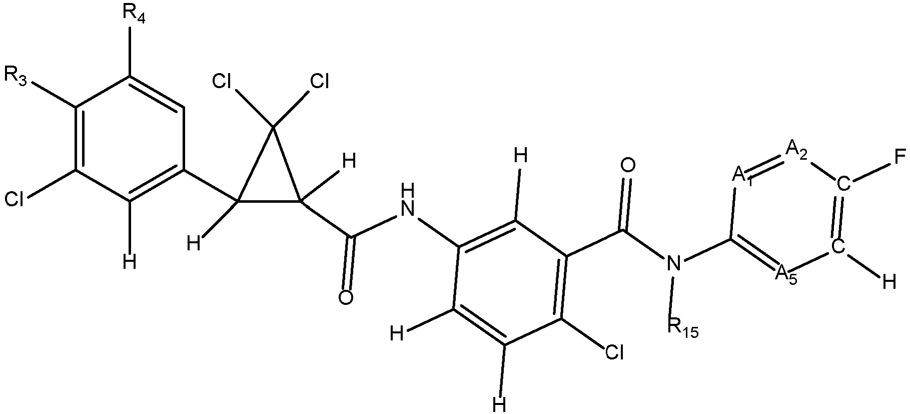
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₁ is CR₅ or N;
A₂ is CR₆ or N;
A₅ is CR₉ or N;
wherein 0 or 1 of A₁, A₂, A₅ are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹⁵** is selected from the group consisting of H, and CH₃.

In an embodiment of the invention and/or embodiments thereof, the invention is directed to a compound of formula (XC) 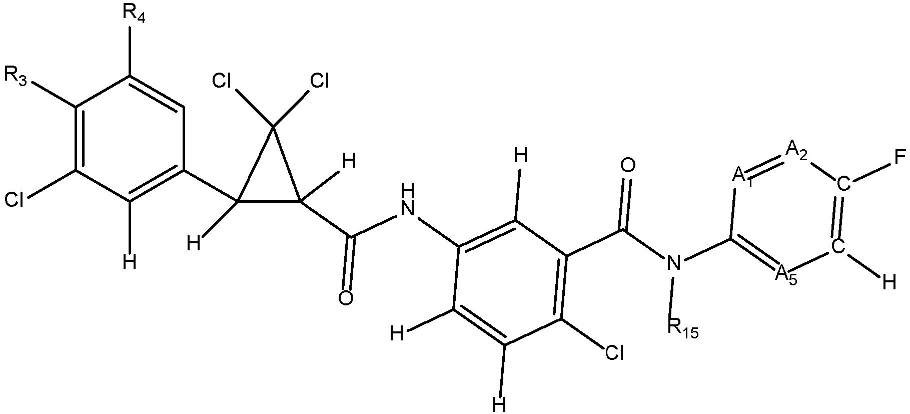
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I ;
**R⁴** is selected from the group consisting of H, F, Cl, Br;
A₁ is CR₅ or N;
A₂ is CR₆ or N;
A₅ is CR₉;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R⁹** is selected from the group consisting of H and F ;
**R¹⁵** is H.

In an embodiment of the invention and/or embodiments thereof, the invention is directed to a compound of formula (XCl) 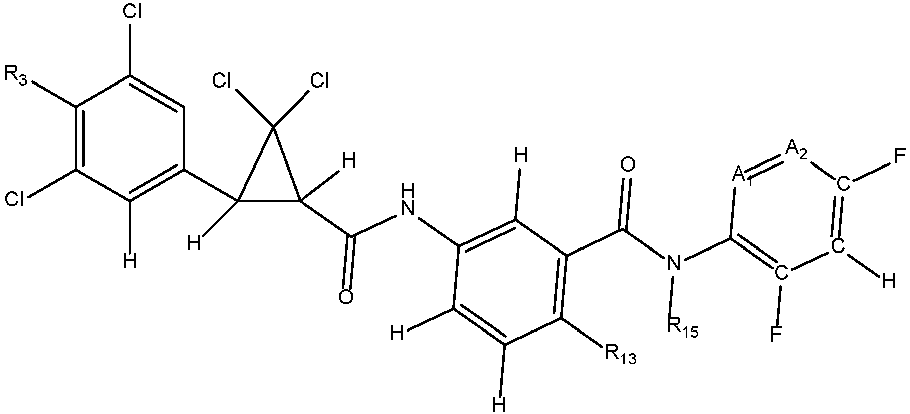
and N-oxides, veterinary acceptable acid addition salts, salt derivatives, solvates, ester derivatives, crystal polymorphs, isotopes, stereoisomers, and tautomers thereof,
wherein R³, R⁵, R⁶, R¹³, R¹⁵, A₁, and A₂, are defined as elsewhere in this description.

In an embodiment of the invention and/or embodiments thereof, the invention is directed to a compound of formula (XCl) 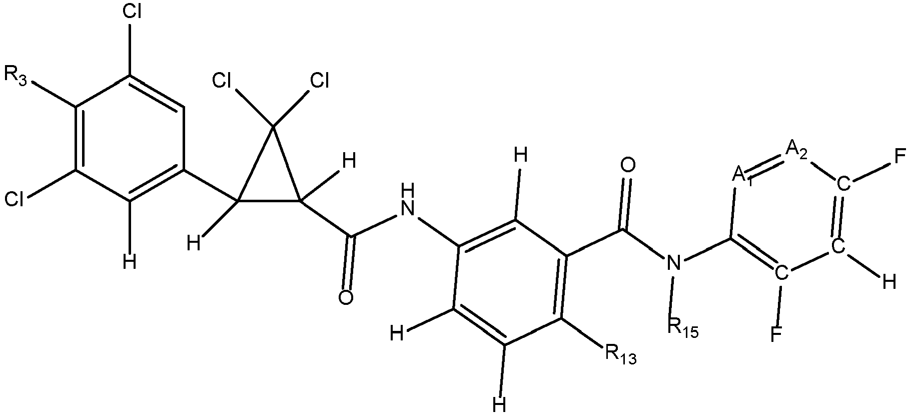
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₂ is CR₆, N=O, or N;
wherein no more than 2 of A₁, A₂, are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (XCl) 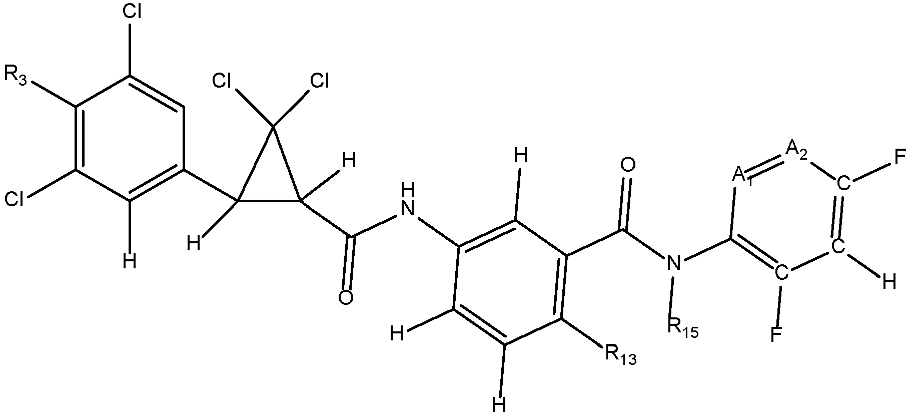
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
A₁ is CR₅, N=O, or N;
A₂ is CR₆, N=O, or N;
wherein no more than 2 of A₁, A₂, are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁶** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (XCl) 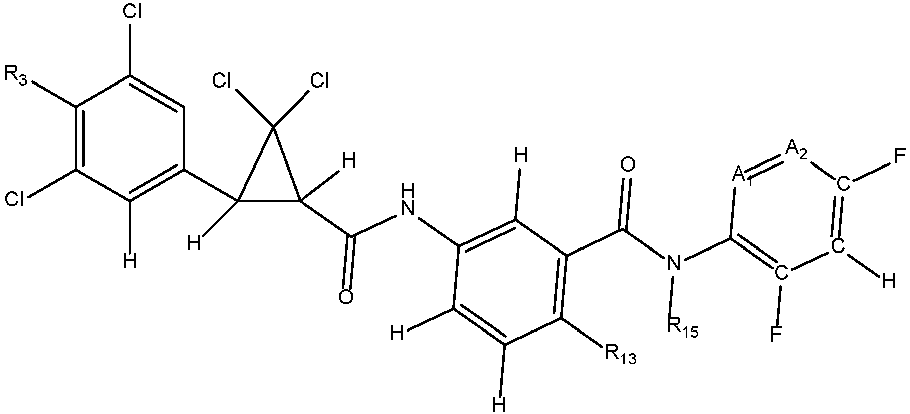
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
A₁ is CR₅ or N;
A₂ is CR₆ or N;
wherein no more than 2 of A₁, A₂, are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁶** is selected from the group consisting of H, F, Cl, NH(C₁-C₆)alkyl, (C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (XCl) 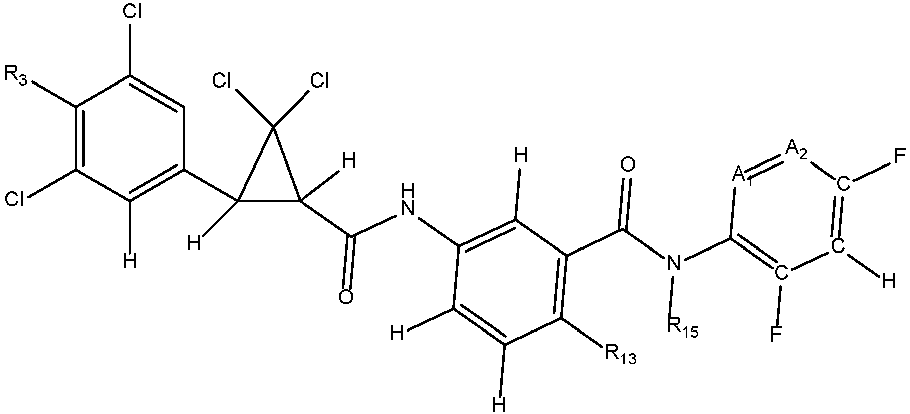
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
A₁ is CR₅ or N;
A₂ is CR₆ or N;
wherein 0 or 1 of A₁, A₂, are N;
**R⁵** is selected from the group consisting of H, F, Cl, CN, NO₂, NH(C₁-C₆)alkyl, and (C₁-C₆)alkyl ;
**R⁶** is selected from the group consisting of H, F, NH(C₁-C₆)alkyl, NHC(=O)R¹⁰, and N(C₁-C₆)alkyl-C(=O)IR¹⁰ ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (XCl) 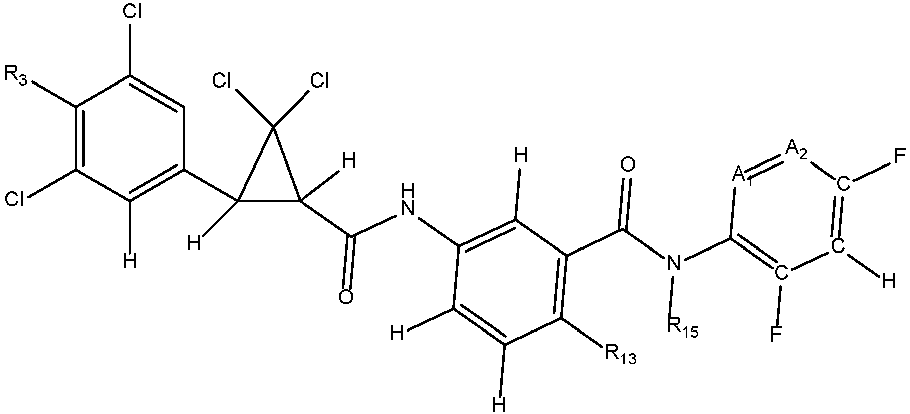
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I ;
A₁ is CR₅ or N;
A₂ is CR₆ or N;
wherein 0 or 1 of A₁, or A₂ are N;
**R⁵** is selected from the group consisting of H, F, Cl, CN and (C₁-C₆)alkyl ;
**R⁶** is selected from the group consisting of H, F, and NH(C₁-C₆)alkyl ;
**R¹³** is Cl or F;
**R¹⁵** is H.

In an embodiment of the invention and/or embodiments thereof, the invention is directed to a compound of formula (XCII) 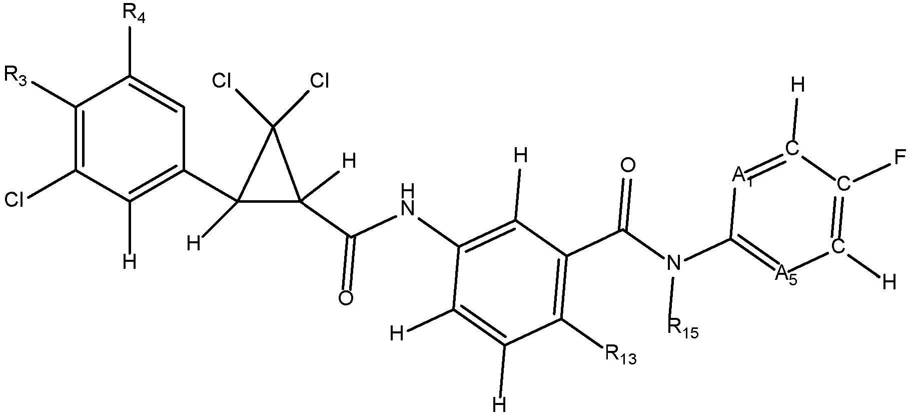
and N-oxides, veterinary acceptable acid addition salts, salt derivatives, solvates, ester derivatives, crystal polymorphs, isotopes, stereoisomers, and tautomers thereof,
wherein R³, R⁴, R⁵, R⁹, R¹³, R¹⁵, A₁, and A₅ are defined as elsewhere in this description.

In an embodiment of the invention and/or embodiments thereof, the invention is directed to a compound of formula (XCII) 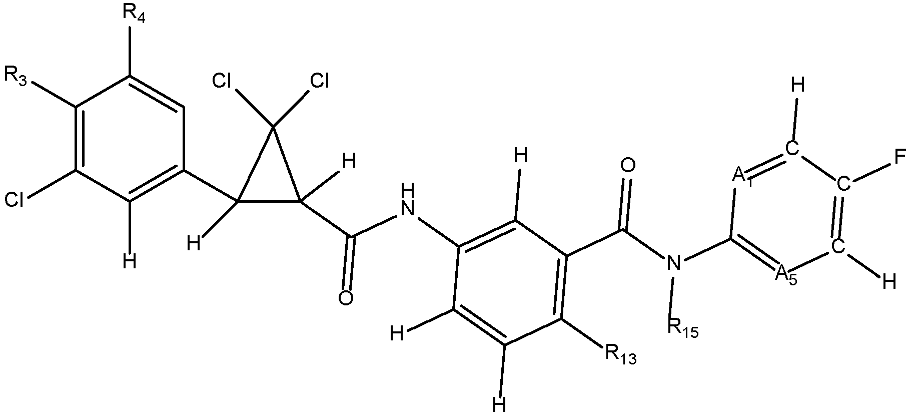
and N-oxides, veterinary acceptable acid addition salts, salt derivatives, solvates, ester derivatives, crystal polymorphs, isotopes, stereoisomers, and tautomers thereof,
wherein
A₁ is CR₅, N=O, or N;
A₅ is CR₉, N=O or N ;
wherein no more than 2 of A₁, As are N or N=O;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹³** is selected from the group consisting of H, F, Cl, Br, I, (C₁-C₆)alkyl, (C₁-C₆)haloalkyl;
**R¹⁵** is selected from the group consisting of H, (C₁-C₆)alkyl.

In an embodiment of the invention and/or embodiments thereof, the invention is directed to a compound of formula (XCII) 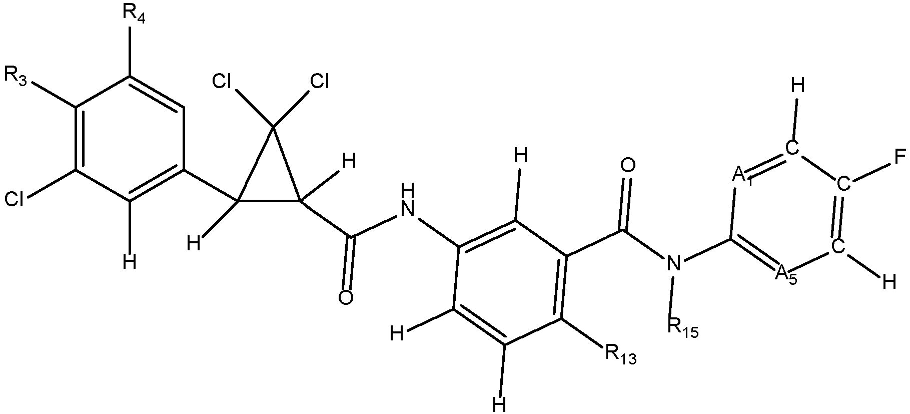
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, and (C₁-C₆) haloalkoxy ;
**R⁴** is selected from the group consisting of H, F, Cl, Br, I, CN, NH₂, NO₂, (C₁-C₆)alkyl, (C₂-C₆)alkenyl, (C₂-C₆)alkynyl, (C₁-C₆)alkoxy, (C₁-C₆)haloalkyl, (C₂-C₆)haloalkenyl, (C₁-C₆)haloalkoxy;
A₁ is CR₅, N=O, or N;
A₅ is CR₉, N=O or N;
wherein no more than 2 of A₁, As are N or N=O;
**R⁵** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R⁹** is selected from the group consisting of H, F, Cl, Br, I, CN, NO₂, NH₂, =O, NH(C₁-C₆)alkyl, N((C₁-C₆)alky)₂, (C₁-C₆)alkyl, (C₁-C₆)alkoxy, (C₁-C₆)haloalkyl, NHC(=O)R¹⁰, N(C₁-C₆)alkyl-C(=O)IR¹⁰, C(=O)(C₁-C₆)alkyl, (C₂-C₆)alkenyl, (C₂-C₆)haloalkenyl, and C(=O)-O-(C₁-C₆)alkyl ;
**R¹⁰** is selected from the group consisting H, (C₁-C₆)alkyl, (C₁-C₆)alkyloxy-(C₁-C₆)-alkyl, (C₂-C₆)alkenyl, (C₂-C₆)alkynyl, ((C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, piperidinyl, pyrrolidinyl, tetrahydrothiophenyl, thianyl, phenyl, and (C₁-C₆)alkylphenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (XCII) 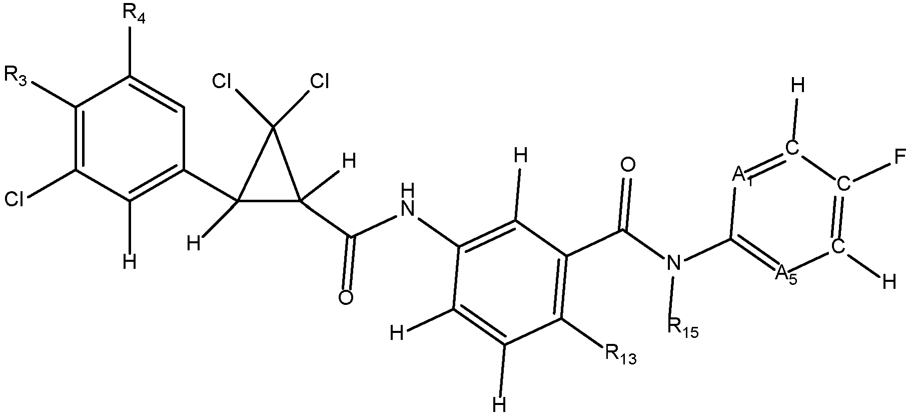
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)alkyl, (C₁-C₆)alkoxy, (C₁-C₆)haloalkyl, and (C₁-C₆) haloalkoxy ;
**R⁴** is selected from the group consisting of H, F, Cl, Br, I, CN, (C₁-C₆)alkyl, (C₂-C₆)alkenyl, (C₂-C₆)alkynyl, (C₁-C₆)alkoxy, (C₁-C₆)haloalkyl, and (C₂-C₆)haloalkenyl;
A₁ is CR₅ or N;
A₅ is CR₉ or N;
wherein no more than 2 of A₁, A₅ are N;
**R⁵** is selected from the group consisting of H, F, Cl, Br, I, CN, NO₂, NH(C₁-C₆)alkyl, (C₁-C₆)alkyl, (C₁-C₆)alkoxy, (C₁-C₆)haloalkyl, NHC(=O)R¹⁰, and N(C₁-C₆)alkyl-C(=O)IR¹⁰ ;
**R⁹** is selected from the group consisting of H, F, Cl, NH(C₁-C₆)alkyl, and (C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is selected from the group consisting of H, F, Cl, CH₃ and CF₃ ;
**R¹⁵** is selected from the group consisting of H, and CH₃.

In an embodiment of the invention and/or embodiments thereof, the invention is directed to a compound of formula (XCII) 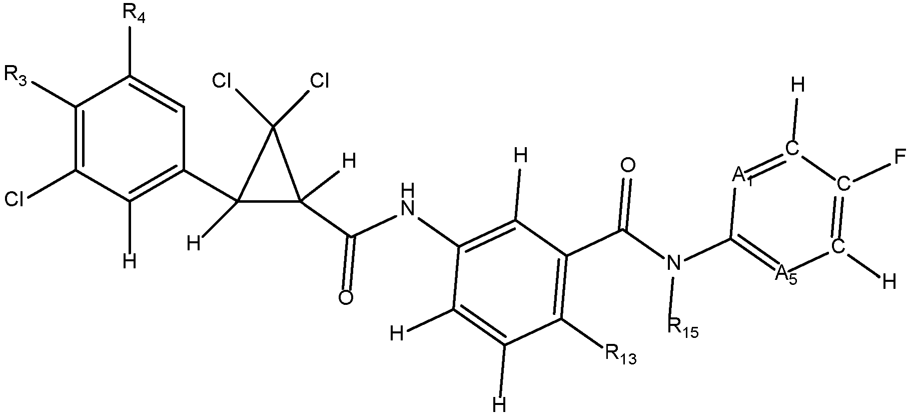
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, I, (C₁-C₆)haloalkyl, and (C₁-C₆) haloalkoxy;
**R⁴** is selected from the group consisting of H, F, Cl, Br, (C₁-C₆)alkyl, and (C₁-C₆)haloalkyl;
A₁ is CR₅ or N;
A₅ is CR₉ or N;
wherein 0 or 1 of A₁, A₅ are N;
**R⁵** is selected from the group consisting of H, F, Cl, CN, NO₂, NH(C₁-C₆)alkyl, and (C₁-C₆)alkyl ;
**R⁹** is selected from the group consisting of H, F, and NH(C₁-C₆)alkyl ;
**R¹⁰** is selected from the group consisting (C₁-C₆)alkyl, (C₁-C₆)alkyloxy-(C₁-C₆)alkyl, (C₂-C₆)alkenyl, (C₂-C₆)alkynyl, (C₁-C₆)haloalkyl, (C₁-C₆)alkyloxy-(C₁-C₆)haloalkyl, (C₁-C₆)alkyl-(C₃-C₆)cycloalkyl, tetrahydrofuranyl, and phenyl ;
**R¹³** is Cl or F ;
**R¹⁵** is selected from the group consisting of H, and CH₃.

In an embodiment of the invention and/or embodiments thereof, the invention is directed to a compound of formula (XCII) 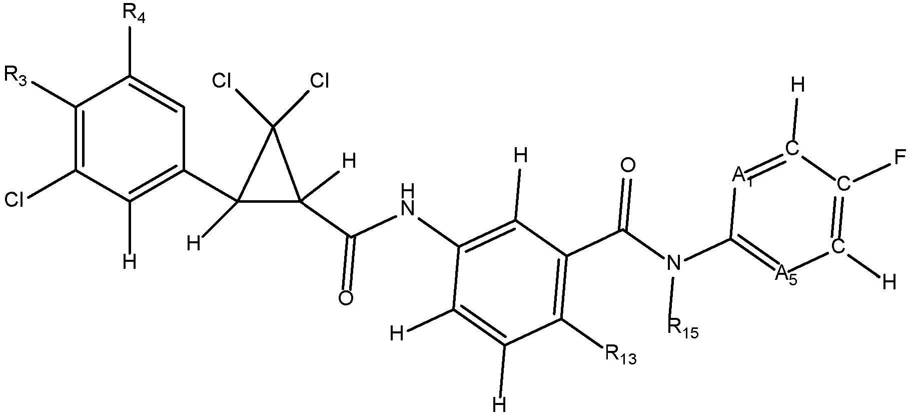
and N-oxides, veterinary acceptable acid addition salts, salt derivatives, solvates, ester derivatives, crystal polymorphs, isotopes, stereoisomers, and tautomers thereof,
wherein
**R³** is selected from the group consisting of H, F, Cl, Br, and I;
**R⁴** is selected from the group consisting of H, F, Cl, Br;
A₁ is CR₅ or N;
A₅ is CR₉;
**R⁵** is selected from the group consisting of H, F, Cl, CN and (C₁-C₆)alkyl ;
**R⁹** is selected from the group consisting of H and F ;
**R¹³** is Cl or F;
**R¹⁵** is H.

In an embodiment of the invention and/or embodiments thereof, the parasitic infestation is an ectoparasite infestation.

In an embodiment of the invention and/or embodiments thereof, the parasitic infestation is a tick infestation and/or flea infestation, preferably a tick infestation.

In an embodiment of the invention and/or embodiments thereof, the parasite is a one host tick.

In an embodiment of the invention and/or embodiments thereof, the parasite is *Rhipicephalus microplus.*

In an embodiment of the invention and/or embodiments thereof, the parasite is a multi-host tick.

In an embodiment of the invention and/or embodiments thereof, the parasite is an acarid parasite.

In an embodiment of the invention and/or embodiments thereof, the animal is a warm-blooded animal, especially a mammal.

In an embodiment of the invention and/or embodiments thereof, the animal is a livestock animal, especially a bovine animal, especially ruminant, especially cattle.

In an embodiment of the invention and/or embodiments thereof, the animal is a fish, and the parasitic infestation is a sea lice infestation.

In an embodiment of the invention and/or embodiments thereof, the animal is a companion animal, especially canine animal or a feline animal, especially a dog or cat.

In one embodiment the compound of the invention and/or embodiments thereof effectively treats exist and protects

Suitably the compounds of the invention and/or embodiments thereof are used in a method, wherein the parasitic infestation is a tick infestation or flea infestation. The compounds are suitably used for a tick infestation.

The compounds of the invention and/or embodiments thereof are suitably used against parasites wherein the parasite is a one host tick or a multi-host tick. Suitably the compounds of the invention are used against *Rhipicephalus microplus.*

In a suitable use or method of the invention and/or any embodiment thereof, the animal is a warm-blooded animal such as a mammal. However, the compounds of the invention and/or embodiments thereof are also active in a livestock animal, such as a ruminant, bovine animal, or cattle. It was also found that the compounds of the invention and/or embodiments thereof are active in fish, against a sea lice infestation. Also, the compounds of the invention and/or embodiments thereof are found active in a companion animal, such as a canine animal or a feline animal, especially a dog or cat.

### Salts, Solvates, N-Oxides and Prodrugs

A salt of the compounds of the Formula (I), or another compound may be advantageous due to one or more of the salt's physical properties, such as pharmaceutical stability in differing temperatures and humidities; crystalline properties; and/or a desirable solubility in water, oil, or other solvent. In some instances, a salt may be used as an aid in the isolation, purification, and/or resolution of the compound. Acid and base salts can typically be formed by, for example, mixing the compound with an acid or base, respectively, using various known methods in the art. To the extent a salt of the compound is intended to be administered *in vivo (i.e.,* to an animal) for a therapeutic benefit, the salt is pharmaceutically acceptable.

Salts may also be of advantage in the synthesis of the compounds according to this invention and/or embodiments thereof. For instance, certain intermediates may advantageously be used in form of their salts in the preparation process of the compounds according to this invention and/or embodiments thereof.

In general, an acid addition salt can be prepared by reacting a free base compound with an approximately stoichiometric amount of an inorganic or organic acid. Non-limiting examples of often suitable inorganic acids for making (pharmaceutically acceptable) salts include hydrochloric, hydrobromic, hydroiodic, nitric, carbonic, sulfuric, and phosphoric acid. Non-limiting examples of often suitable organic acids for making (pharmaceutically acceptable) salts generally include, for example, aliphatic, cycloaliphatic, aromatic, araliphatic, heterocyclic, carboxylic, and sulfonic classes of organic acids. Specific examples of often suitable organic acids include cholic, sorbic, lauric, acetic, trifluoroacetic, formic, propionic, succinic, glycolic, gluconic, digluconic, lactic, malic, tartaric acid, citric, ascorbic, glucuronic, maleic, fumaric, pyruvic, aspartic, glutamic, aryl carboxylic acid (e.g., benzoic), anthranilic acid, mesylic, stearic, salicylic, p-hydroxybenzoic, phenylacetic, mandelic, embonic (pamoic), alkylsulfonic *(e.g.,* ethanesulfonic), arylsulfonic *(e.g.,* benzenesulfonic), pantothenic, 2-hydroxyethanesulfonic, sulfanilic, cyclohexylaminosulfonic, β-hydroxybutyric, galactaric, galacturonic, adipic, alginic, butyric, camphoric, camphorsulfonic, cyclopentanepropionic, dodecylsulfic, glycoheptanoic, glycerophosphic, heptanoic, hexanoic, nicotinic, 2-naphthalesulfonic, oxalic, palmoic, pectinic, 3-phenylpropionic, picric, pivalic, thiocyanic, tosylic, and undecanoic acid. In some such embodiments, for example, the salt comprises a trifluoroacetate, mesylate, or tosylate salt. In other embodiments, the salt comprises a hydrochloric acid salt.

By way of a non-limiting example, an amine function can form salts with hydrochloric, hydrobromic, sulfuric, phosphoric, acetic, benzoic, citric, malonic, salicylic, malic, fumaric, oxalic, succinic, tartaric, lactic, gluconic, ascorbic, maleic, aspartic, benzenesulfonic, methanesulfonic, ethanesulfonic, hydroxyl-methanesulfonic, and hydroxyethanesulfonic acids. Additionally, by way of a non-limiting example, an acid function can form salts including those derived from alkali or alkaline earth metals and those derived from ammonia and amines. Examples of preferred cations include sodium, potassium, and magnesium.

In general, a base addition salt can be prepared by reacting a free acid compound with an approximately stoichiometric amount of an inorganic or organic base. Examples of base addition salts may include, for example, metallic salts and organic salts. Metallic salts, for example, include alkali metal (group la) salts, alkaline earth metal (group Ila) salts, and other physiologically acceptable metal salts. Such salts may be made from aluminum, calcium, lithium, magnesium, potassium, sodium, and zinc. For example, a free acid compound may be mixed with sodium hydroxide to form such a base addition salt. Organic salts may be made from amines, such as trimethylamine, diethylamine, N, N'-dibenzylethylenediamine, chloroprocaine, ethanolamine, diethanolamine, ethylenediamine, meglumine (N-methylglucamine), and procaine. Basic nitrogen-containing groups may be quaternized with agents such as C₁-C₆-alkyl halides (e.g., methyl, ethyl, propyl, and butyl chlorides, bromides, and iodides), dialkyl sulfates (e.g., dimethyl, diethyl, dibuytl, and diamyl sulfates), long chain halides (e.g., decyl, lauryl, myristyl, and stearyl chlorides, bromides, and iodides), arylalkyl halides (e.g., benzyl and phenethyl bromides), and others.

Compounds of Formula (I) may be formulated into stable complexes with a solvent, such that the complex remains intact after the non-complexed solvent is removed. These complexes are often referred to as "solvates." However, it is particularly desirable to form stable hydrates with water as the solvent.

A solvate of a compound of the Formula (I), or another compound may be formed by aggregation of said compound of the Formula (I) with solvent molecules such as water, alcohols, for example ethanol, aromatic solvents such as toluene, ethers, halogenated organic solvents such as dichloromethane, preferably in a definite proportion by weight.

Compounds of Formula (I) containing an acid functionality may be made into ester derivatives. These ester derivatives can then be applied in the same manner as the compounds disclosed in this document are applied.

Compounds of Formula (I) may be made as various crystal polymorphs. Polymorphism is important in the development of veterinary products since different crystal polymorphs or structures of the same molecule can have vastly different physical properties and biological performances.

An N-oxide of a compound of the Formula (I), or another compound may be formed by oxidation of an N-atom in an amine or N-heterocycle such as pyridine and pyrimidine by oxidation agents such as hydrogen peroxide, peracids or inorganic oxidation agents such as potassium peroxymonosulfate (oxone).

This invention also encompasses prodrug derivatives of the compounds of Formula (I). The term prodrug refers to compounds that are transformed to yield the parent compound of Formula (I). Transformation often occurs in vivo. *In vivo* means that in the case of, for example, treatment of a parasitic infestation this transformation can occur in the host organism and/or the parasite. Various forms of prodrugs are well known in the art. For example, if the group of Formula (I) represents a pyridine, it is possible to form pyridinium salts such as, for example, acyloxyalkylpyridinium salts, which can offer advantages in terms of higher solubility for parenteral dosage forms, which are described in S. K. Davidsen et al., J. of Med. Chem. 37 4423-4429 (1994).

Compounds of Formula (I) may be made with different isotopes. Of particular importance are molecules having ²H (also known as deuterium) or ³H (also known as tritium) in place of ¹H. Molecules of Formula One may be made with different radionuclides. Of particular importance are molecules having ¹⁴C (also known as radiocarbon). Molecules of Formula (I) having deuterium, tritium, or ¹⁴C may be used in biological studies allowing tracing in chemical and physiological processes and half-life studies, as well as MoA studies.

### Isomers

The compounds of Formula (I) may exist in different geometric or optical isomeric or different tautomeric forms. One or more centers of chirality may be present in which case compounds of Formula (I) may be present as pure enantiomers, mixtures of enantiomers, pure diastereomers or mixtures of diastereomers. It will be appreciated by those skilled in the art that one stereoisomer may be more active than the other stereoisomers. Individual stereoisomers may be obtained by known selective synthetic procedures, by conventional synthetic procedures using resolved starting materials, or by conventional resolution procedures. There may be double bonds present in the molecule, in which case compounds of Formula (I) may exist as single geometric isomers (*cis* or *trans, E* or *Z*) or mixtures of geometric isomers (*cis* and *trans, E* and Z). Centers of tautomerisation may be present. This disclosure covers all such isomers, tautomers, and mixtures thereof, in all proportions.

In some embodiments, such compounds may have two or more isomers, such as optical isomers or conformational isomers. For example, the compounds can have a (*E*) or (*Z*) configuration at the - CXR³=CR¹R² double bond, such as in alkenyl or alkynyl substituents of the ring formed by A1 to A5. In some preferred embodiments, such compound has the (*E*) configuration, in other embodiments, the compound has the (*Z*) configuration. In a preferred embodiment the compounds have (*E*) configuration.

Unless otherwise stated, a compound structure that does not indicate a particular conformation is intended to encompass all the possible conformational isomers of the compound, as well as comprising fewer than all the possible conformational isomers. Compounds with two chiral centers have four isomers: the RR-, SS-, RS-, and SR-isomers. Such compounds may exist in a number of forms i.e., in the pure RR or SS or RS or SR isomeric forms, or as mixtures, hereinafter called "enantiomeric pairs" of either RR/SS or RS/SR. The compound ***can also exist as racemic mixtures of all four isomers (RR+SS+RS+SR) or in the form of racemic mixtures of the enantiomeric pairs (RR/SS) or (RS/SR). The isomers (RR) and (SS) are mirror images of each other and are therefore enantiomers, which have the same chemical properties and melting points. (RS) and (SR) is similarly an enantiomeric pair. The mirror images of (RR) and (SS) are not, however, super imposable on (RS) and (SR). This relationship is called diastereomerism, and (RR) is a diastereomer to (RS).

Although structurally identical, isomers can have different effects in biological systems: one isomer may have specific therapeutic activity while another isomer may have no therapeutic activity or may have entirely different forms of biological activity.

In the compounds of the invention, the cyclopropane contains 2 chiral centers, one bonded to the phenyl and the other bonded to the carboxamide group.

It has now surprisingly been found that parameters, such as for example efficacy can be greatly improved by administering pure or substantially pure RR-isomer of compounds according to the invention and embodiments as described herein, a pharmaceutically acceptable salt, thereof, while side effects can be substantially avoided. Thus, the applicant has found that by administering a therapeutically effective amount of the pure or substantially pure RR-isomer of compounds according to the invention and embodiments as described in any embodiment, a pharmaceutically acceptable salt, thereof.

Terms like "pure RR- compounds according to the invention and embodiments as described in any embodiment", "pure RR-isomer of compounds according to the invention and embodiments as described in any embodiment" and the like, refer to compounds according to the invention and embodiments as described in any embodiment having an optical purity of RR- compounds according to the invention and embodiments as described in any embodiment that is 98% by weight or better, which means the RR-isomer is present at a concentration of 98% by weight or more, while the total concentration (i.e. the sum) of the corresponding RS-, SR- and SS-isomers is 2% by weight or less, based on the total amount of compounds according to the invention and embodiments as described in any embodiment present.

The terms "substantially pure RR- compounds according to the invention and embodiments as described in any embodiment", "substantially pure RR-isomer of compounds according to the invention and embodiments as described in any embodiment" and the like, refer to an optical purity of RR- compounds according to the invention and embodiments as described in any embodiment that is 80% by weight or better which means a concentration of 80% weight or more of RR-compounds according to the invention and embodiments as described in any embodiment and 20% by weight or less of the sum of the corresponding RS-, SR- and SS-isomers, based on the total amount of compounds according to the invention and embodiments as described in any embodiment present. In a more preferred embodiment, a "substantially pure RR- compounds according to the invention and embodiments as described in any embodiment" contains 90% by weight or more of RR- compounds according to the invention and embodiments as described in any embodiment and 10% or less of the sum of the RS and SR and SS-isomers of compounds according to the invention and embodiments as described in any embodiment.

In a suitable embodiment the compounds of Formula (I), the carboxamido, and the phenyl, which are bonded to the cyclopropane, are in the *R,R* configuration. This embodiment may be used in combination with any of the other embodiments of **R¹, R², R³, R⁴, R⁵, R⁶, R⁷, R⁸, R⁹, R¹⁰, R¹¹, R¹², R¹³, R¹⁴,** and **R¹⁵**.

In an embodiment of the invention and/or embodiments thereof, the parasitic infestation is an ectoparasite infestation.

In an embodiment of the invention and/or embodiments thereof, the parasitic infestation is a tick infestation or flea infestation, preferably a tick infestation.

In an embodiment of the invention and/or embodiments thereof, the parasite is a one host tick.

In an embodiment of the invention and/or embodiments thereof, the parasite is *Rhipicephalus microplus.*

In an embodiment of the invention and/or embodiments thereof, the parasite is a multi-host tick.

In an embodiment of the invention and/or embodiments thereof, the parasite is an acarid parasite.

In an embodiment of the invention and/or embodiments thereof, the animal is a warm-blooded animal, especially a mammal.

In an embodiment of the invention and/or embodiments thereof, the animal is a livestock animal, especially a bovine animal, especially ruminant, especially cattle.

In an embodiment of the invention and/or embodiments thereof, the animal is a fish, and the parasitic infestation is a sea lice infestation.

In an embodiment of the invention and/or embodiments thereof, the animal is a companion animal, especially canine animal or a feline animal, especially a dog or cat.

In an embodiment of the invention and/or embodiments thereof, the animal is protected from a parasite infestation.

In an embodiment of the invention and/or embodiments thereof, an existing parasite infestation of the animal is treated or controlled.

In an embodiment of the invention and/or embodiments thereof, the method comprises administering the compound to an animal weekly, bi-weekly, monthly, every 6 weeks, every 2 months or every 3 months.

In an embodiment of the invention and/or embodiments thereof, the parasite infestation of the animal is prevented for at least one week.

The present invention is also directed to a veterinary composition comprising an effective amount of a compound of Formula (I) as defined in any embodiment described herein and an inert carrier or formulation adjuvant. Suitably the composition is used in the treatment and control of a parasitic infestation of animals as described herein.

In an embodiment of the invention and/or embodiments thereof, the veterinary composition is a topical composition.

In an embodiment of the invention and/or embodiments thereof, the veterinary composition is an oral composition.

In an embodiment of the invention and/or embodiments thereof, the veterinary composition is an injection composition.

### METHODS ACCORDING TO THIS INVENTION

The compounds of the invention and/or embodiments thereof as described in this application and were applicable, pharmaceutically acceptable solvates, N-oxides, salts and prodrugs thereof may generally be used as a medicament for animals wherein one or more, preferably one compound according to this invention and/or embodiments thereof is administered to an animal in order to treat, or control parasitic infestations.

Suitably the compounds of the invention and any embodiments thereof are for use in a method of treatment or control of a parasitic infestation of an animal.

Another aspect of the current invention are compositions, comprising such compounds and veterinary or pharmaceutically acceptable excipients for use in treatment and/or protection animals from parasite infestation.

An "infestation" refers to the presence of parasites in numbers that pose a risk of nuisance or harm to animals. The presence can be in the environment, on the skin or fur of an animal or in the animal's blood, organs or tissues, etc.

The term "(parasitic) infestation" includes conditions associated with or caused by one or more (parasitic) pathogens; said conditions include clinical conditions (parasitoses) and sub-clinical conditions.

The term "treatment of parasitic infestation" thus includes both the treatment of parasitoses and the treatment of sub-clinical conditions. The treatment of a parasite infestation generally implies the suppression of parasite burden of the animal below that level at which economic loss occurs. Treating (parasitic) infestations" means to partially or completely inhibit the development of (parasitic) infestations of an animal susceptible to (parasitic) infestation, reduce or completely eliminate the symptoms of infestations of an animal having infestations, and/or partially or completely cure infestations of an animal having infestations. This can be achieved by alleviating or reducing pathogen numbers such as parasite numbers in or on an animal.

Sub-clinical conditions are typically conditions not directly leading to clinical symptoms in the parasite infested animal but leading to economic losses. Such economic losses can be e.g., by depression of growth in young animals, lower feed efficiency, lower weight gain in meat producing animals, lower milk production in ruminants, or lower wool-production in sheep.

The term 'treatment,' and similar terms such as 'treating' or 'treat' as used herein, refer to the administration of an effective amount of at least one compound as described for use in the invention to an animal which has an infestation -of more or less severity- with parasites of one or more species

The term "control" as used herein with regard to the field of animal health means that the compounds act by reducing the occurrence of the parasite in question in or on an animal, that is infested or is at risk to be infested with such parasites, to a harmless level. The control of parasites is a medical care given to animals that are infested or are at risk to be infested with such parasites. T This means to to reduce or eradicate parasite numbers in and/or on an animal, and/or to partially or completely inhibit the development of parasite infestation in and/or on an animal. This may be achieved by, for example, killing, repelling, expelling, incapacitating, deterring, eliminating, alleviating parasites, or minimizing the parasite infestation.

Therefore, control refers to either ameliorating or eliminating a current parasitic infestation, and/or preventing or diminishing a future infestation or a re-infestation of an animal or an animal population.

Within the context of the invention, the term 'control 'includes, particularly, the preventive treatment of animals against a parasite infestation/infection. The preventive treatment of an animal against a disease or condition designates a treatment made before the animal has been exposed to or in contact with the causative agent of the disease or condition (e.g., a parasite especially infectious stages of parasites) or after said exposure/contact but before development of the detrimental conditions (e.g. before sub-clinical conditions, such as production losses in livestock appear, ( e.g. reduction of milk production, reduced weight gain, wool loss), before appearance of clinical symptoms or at an early stage of development of the disease.

More specifically, "control" as used herein means that the compound kills the parasite in question, retards its growth or inhibits its proliferation.

The effect can be e.g., ovicidal, larvicidal and/or adulticidal or a combination thereof. The effect can manifest itself directly, i.e., killing the parasites either immediately or after some time has elapsed, for example when molting occurs, or by destroying their eggs, or indirectly, e.g., reducing the number of eggs laid and/or the hatching rate.

Therefore, control refers to either ameliorating or eliminating a current parasitic infestation, and/or preventing or diminishing a future infestation or a re-infestation of an animal or an animal population.

Control of the parasites can also contribute to preventing the transmission of infectious agents to animals by parasite (vector control). The compounds of the Formula (I) can also be used in vector control. In the context of the present invention, a vector is especially an arthropod, especially an insect or arachnid or crustacean, capable of transmitting pathogens, for example, viruses, worms, single-cell organisms, and bacteria, from a reservoir (plant, animal, human, etc.) to a host animal.

An example of vectors and the diseases or pathogens they transmit are ticks, that transmit tick-borne encephalitis, Q fever *(Coxiella burnetii),* borreliosis (Lyme disease caused by *Borrelia* spp. *e.g., Borrelia burgdorferi),* babesiosis (or piroplasmosis caused by *Babesia* spp.) and rickettsioses (e.g., Rocky Mountain spotted fever).

The term "parasitoses" relates to clinically manifest pathologic conditions and diseases associated with or caused by an infestation by one or more parasites directly, such as, for example, anemia and flea allergy dermatitis. It also includes pathologic conditions or diseases associated with caused by one or more vector-transmitted pathogens, such as, for example, Lyme disease, ehrlichiosis (particularly canine ehrlichiosis), and Rocky Mountain spotted fever from vector ticks.

The phrase "treatment of parasitoses" means to partially or completely inhibit the development of parasitoses of an animal, reduce or completely eliminate the symptoms of parasitoses of an animal, and/or partially or completely cure parasitoses of an animal.

In general, the control of parasitic infestation including parasitoses is achieved by administering an effective amount of one or more, preferably one compound according to this invention.

The terms "prevent", "prevention" or "prophylaxis" are intended to mean the administration of the compounds of the present invention to the animal before the parasitic infection or infestation has occurred in order to keep said infection or infestation from occurring. Administration of the compound at recommended regular intervals effectively prevents new parasitic infestations or infections in animals by killing new parasites that attack an animal before they can multiply to establish an infestation or infection.

Also, the term 'preventive treatment', in relation to a population of animals, designates the treatment of all members of the population even after the disease or condition (e.g., parasite infestation) has been detected in only certain animals, to limit or avoid spreading of and contamination to the other members of the animal population.

The compounds according to the present invention act against animal parasites, in particular ectoparasites and/or endoparasites. Ectoparasites are parasites that live on the external surface of host animals. Endoparasites are parasites that live in the internal organs or tissues of its host.

Ectoparasites are parasites that live on the external surface of host animals.

Endoparasites are parasites that live in the internal organs or tissues of its host.

Ectoparasites are typically and preferably arthropods, especially insects such as flies (biting and licking), parasitic fly larvae, lice, fleas, and the like; or acarids such as ticks, for example hard ticks or soft ticks, or mites such as scab mites, mange mites or demodex mites and the like. Important ectoparasites of fish are crustacean parasites such as aquatic ectoparasites such as sea lice.

Ectoparasites tend to irritate the animals and can also cause clinical disease and adverse sub-clinical conditions, either by themselves or by carrying vector-transmitted pathogens.

Specifically, ticks parasitize on wild as well as domesticated animals, and are known to be responsible for the transmission of pathogens including bacteria, viruses, and protozoan parasites. Ticks also release toxins which cause inflammation or paralysis in the host. Occasionally, these toxins are fatal to the host.

On the other hand, such ectoparasites seriously impact productivity in the livestock animal industry by reducing weight gain, or milk production, causing inferior quality hide, wool, and meat, and in some cases resulting in mortality. Ectoparasites are also responsible, in part, for the spread of pathogens as vectors and cause discomfort in livestock and companion animals impacting animal welfare.

In one embodiment of the invention and/or embodiments thereof, the ectoparasite is selected from the group consisting of flies, parasitic fly larvae, lice, fleas, ticks, mites, and sea lice.

In one embodiment of the invention and/or embodiments thereof, the ectoparasite is an insect or an acarid. Suitably the insect is not a mosquito. Suitably the ectoparasite is an acarid.

Suitably the acarid ectoparasite is a hard tick, soft tick, or mite.

Suitably the ectoparasite is a sea lice.

For example, a parasite which is prevalent among livestock animals, such as cattle, is the tick of the genus *Rhipicephalus,* especially those of the species *R. microplus* (tropical cattle tick), R. *decoloratus* and *R. annulatus.* Ticks such as *Rhipicephalus microplus* (formerly *Boophilus microplus)* are difficult to control.

This species of ticks is considered a one-host tick and lives as immature and adult stages on one animal before the female engorges and falls off the host to lay eggs in the environment. The life cycle of the tick is approximately three to four weeks. In addition to cattle, *Rhipicephalus microplus* may infest buffalo, horses, donkeys, goats, sheep, deer, pigs, and dogs.

Veterinary relevant ectoparasites of warm- blooded animals (generally insect and acarid pests) include the following:
Insects. These are parasitic stages of flies, fleas, lice, bugs include, for example, migrating diperous larvae, such as, for example, *Hypoderma* sp. in cattle, *Gastrophilus* in horses, and *Cuterebra* spp. in rodents; biting flies, such as, for example, bloodsucking adult flies (*e.g.,* the horn fly (*Haematobia irritans*), horse flies (*Tabanus* spp.), stable flies (*Stomoxys calcitrans*)*,* black flies (*Simulium* spp.), deer flies (*Chrysops* spp.), louse flies (*Melophagus ovinus*), tsetse flies (*Glossina* spp.); parasitic fly maggots, such as, for example, bot flies (*Oestrus ovis* and *Cuterebra* spp.), the blow flies (*Phaenicia* spp.), screwworms (*Cochliomyia hominivorax*), cattle grubs (*Hypoderma* spp.), and fleeceworms; Suitably the insect is not a mosquito.
**Lice.** These include, for example, chewing lice, such as *Menopon* spp. and *Bovicola* spp.; and sucking lice, such as *Haematopinus* spp., *Linognathus* spp., and *Solenopotes* spp.
Fleas. These include, for example, *Ctenocephalides* spp., such as dog fleas (*Ctenocephalides canis*) and cat fleas *(Ctenocephalides felis*); *Xenopsylla* spp., such as oriental rat fleas (*Xenopsylla cheopis*); *Pulex* spp., such as human fleas (*Pulex irritans*); hedgehog fleas (*Archaeopsylla erinacei*); and bird fleas (*Ceratophyllus gallinae*)*.*
**True bugs.** These include, for example, *Cimicidae* or the common bed bug (*Cimex lectularius*); and *Triatominae* spp., such as triatomid bugs (also known as kissing bugs) (*e.g., Rhodnius prolixus* and *Triatoma* spp.).

**Mites.** These include:
i. *Mesostigmata* spp., such as mesostigmatids, which include chicken mites (*Dermanyssus gallinae*)*.*
ii. li. *Astigmata* spp., such as itch or scab mites, which include *Sarcoptidae* spp. (*e.g., Sarcoptes scabiei*); and mange mites, which include *Psoroptidae* spp. (*e.g., Chorioptes bovis* and *Psoroptes ovis*).
iii. lii. *P rostigmata* spp, such as chiggers, which include *Trombiculidae* spp. *(e.g.,* North American chiggers, *Trombicula alfreddugesi*)*.*

### Iv. Demodex spp.

Ticks. These include, for example, soft-bodied ticks, such as *Argasidae* spp. (*e.g., Argas* spp. and *Ornithodoros* spp.); and hard-bodied ticks, such as *Ixodidae* spp. (*e.g., Ixodes ricinus, Rhipicephalus sanguineus, Haemaphysalis* spp, *Dermacentor reticulatus, Dermacentor variabilis, Amblyomma americanum,* and *Rhipicephalus* (*Boophilus)* spp.).

In one embodiment the ectoparasite is a tick from the genera *Rhipicephalus (Boophilus), Dermacentor, Ixodes, Ambylomma, Haemaphysalis, Hyalomma* especially *Rhipicephalus* (*Boophilus*), especially those of the cattle tick species *R. microplus, R. decoloratus* and *R. annulatus.*

*Rhipicephalus microplus, R. decoloratus* and *R. annulatus* are single host ticks, this means all three stages of the lifecycle live on the same animal.

Multi-host ticks drop to the ground after each stage has fed on an animal and re-attach to another host after molting. The species of host between the distinct stages may differ. Representatives of multi-host ticks are e.g., *Amblyomma cajennense, Ixodes holocyclus,* i.e., paralysis tick: *H. longicornis, Rhipicephalus appendiculatus* and *Amblyomma hebraeum, Dermacentor albipictus, Amblyomma maculatum, Amblyomma andersoni, Ixodes ricinus, Dermacentor marginatus.*

The main reasons for tick control in livestock animals are to protect hosts from irritation and production losses, formation of lesions that can become secondarily infected, damage to hides and udders, toxicosis, paralysis, and of greatest importance, infection with a wide variety of disease agents.

The ectoparasite infestations of animals treated by the compounds include, but are not limited to fleas, ticks, mites, flies, lice, blowfly, and co- infestations with more than one species thereof.

In one preferred embodiment, the compounds of the invention are used for the treatment of or protection of animals from parasitic infections and infestations in companion animals, especially dogs and cat, especially to control infestations with fleas and/or ticks, or mites especially *Ctenocephalides felis, Rhipicephalus sanguineus, Ixodes ricinus, Dermacenter variabilis, Amblyomma americanum, Ixodes scapularis, Dermacentor reticulatus, Hyalomma spp.* and/or *Ixodes holocyclus.*

In another preferred embodiment, the compounds of the invention are used for the treatment of or protection from parasitic infections and infestations in dogs and cats, especially to control infestations with *Demodex* spp. mites.

The compound is suited for the treatment of tick infestations *(Dermacentor reticulatus, Ixodes hexagonus, Ixodes ricinus* and *Rhipicephalus sanguineus)* in dogs and/or cats. The compound has immediate and persistent tick killing activity for at least 5 weeks. For the treatment of flea infestations (*Ctenocephalides felis* and *Ctenocephalides canis*) the compound has immediate and persistent flea killing activity against new infestations for at least 5 weeks. The compound can be used as part of a treatment strategy for the control of Flea Allergy Dermatitis (FAD). The compound can be used for the treatment of sarcoptic mange (*Sarcoptes scabiei*). The compound is suited for the treatment of ear mite infestations (*Otodectes cynotis*). The compound is suited for the treatment of demodicosis (*Demodex canis*).

The compound is suited to kill adult fleas and is indicated for the treatment and prevention of flea infestations (*Ctenocephalides felis*) and the treatment and control of Blacklegged tick (*Ixodes scapularis*)*,* American Dog tick (Dermacentor variabilis), Lone Star tick (*Amblyomma americanum*)*,* and Brown Dog tick (Rhipicephalus sanguineus) infestations in companion animals , in dogs and/or cats.

In another preferred embodiment, the compounds of the invention are used for the treatment of or protection from parasitic infections and infestations in livestock, especially ruminants such as cattle or sheep. When treating livestock animals such as cattle or sheep, the compounds are particularly effective against *Rhipicephalus (Boophilus) microplus, Haematobia irritans* (horn fly), *Stomoxys calcitrans* (stable fly), and myiases such as *Lucilia sericata,* (European green blowfly), *Lucilia cuprina* (Green blowfly or Australian sheep blowfly known as blowfly strike in Australia, New Zealand and South Africa), *Chrysomya rufifacies* (Hairy maggot fly), *Chrysomya varipes* (Small green blowfly), *Calliphora stygia* (Common brown blowfly), *Calliphora augur* (Lesser brown blowfly (eastern), *Calliphora novicia* (Lesser brown blowfly (western).

Sucking lice consume a blood meal from their host and are more important in transmitting pathogens. Chewing or biting lice ingest fur and skin and sometimes blood from their host; important lice parasites are the cattle biting louse (*Bovicola bovis*), the longnosed cattle louse (*Linognathus vituli*), the little blue cattle louse (*Solenopotes capillatus*)*,* the shortnosed cattle louse (*Haematopinus eurysternus*), and the cattle tail louse (*Haematopinus quadripertusus*) and the sheep biting louse of sheep and goats (*Bovicola ovis*).

In one embodiment the compounds of the invention are used for the treatment of or protection from parasitic infections and infestations by mange mites like *Chorioptes bovis, Sarcoptes scabiei* and *Psoroptes ovis.*

The above list is not exhaustive and other ectoparasites are well known in the art to be harmful to animals, especially livestock animals, especially ruminants, more especially cattle or sheep. These include, for example migrating dipterous larvae.

It has been surprisingly found that the compounds or composition of the invention are effective to control one or more of these parasites.

Important ectoparasites of cold-blooded animals like fish are sea lice. Sea lice are ectoparasites which feed on the mucus, epidermal tissue, and blood of host marine fish, in particular salmon. Sea lice belong to the sub-class of copepods. As used herein, the term "sea lice" refers to all members of the family *Caligidae;* in particular, it includes *Lepeophtheirus salmonis, Caligus rogercresseyi , C. elongatus* and *Caligus clemensi* or the family *Lernanthropidae;* it includes *Lernanthropus kroyeri.*

Sea lice can cause significant harm to host fish, in particular they can cause serious fin damage, skin erosion, bleeding, and open wounds. Additionally, sea lice can cause chronic stress response in fish, which in turn can make them susceptible to other diseases. Sea lice infestations are a major problem in fish farming and can result in significant economic loss.

The term "endoparasites" includes especially helminths such as cestodes, nematodes or trematodes, and protozoa such as coccidia. Endoparasites include nematode pests which commonly infect animals, and include the egg, larval, and adult stages thereof. Such pests include helminths (roundworms, hookworms, tapeworms, heartworms), and are commercially important because they cause serious diseases in animals.

In some embodiments of the invention, especially in case the compound is combined with another active ingredient, the composition can also be used to treat against endoparasites such as those caused by one or more helminths selected from the group consisting of a) cestodes: e.g., *Anaplocephala* spp.; *Dipylidium* spp.; *Diphyllobothrium* spp.; *Echinococcus* spp.; *Moniezia* spp.; *Taenia* spp.; b) trematodes e.g. *Dicrocoelium* spp.; *Fasciola* spp.; *Paramphistomum* spp.; *Schistosoma* spp.; or c) nematodes, e.g. *; Ancylostoma* spp.; *Anecator* spp.; *Ascaridia* spp.; *Ascaris* spp.; *Brugia* spp.; *Bunostomum* spp.; *Capillaria* spp.; *Chabertia* spp.; *Cooperia* spp.; *Cyathostomum* spp.; *Cylicocyclus* spp.; *Cylicodontophorus* spp.; *Cylicostephanus* spp.; *Craterostomum* spp.; *Dictyocaulus* spp.; *Dipetalonema* spp; *Dirofilaria* spp.; *Dracunculus* spp.; *Enterobius* spp.; *Filaroides* spp.; *Habronema* spp.; *Haemonchus* spp.; *Heterakis* spp.; *Hyostrongylus* spp.; *Metastrongylus* spp.; *Meullerius* spp. *Necator* spp.; *Nematodirus* spp.; *Nippostrongylus* spp.; *Oesophagostomum* spp.; *Onchocerca* spp.; *Ostertagia* spp.; *Oxyuris* spp.; *Parascaris spp.;Stephanurus* spp.; *Strongylus* spp.; *Syngamus* spp.; *Toxocara* spp.; *Strongyloides* spp.; *Teladorsagia* spp.; *Toxascaris* spp.; *Trichinella* spp.; *Trichuris* spp.; *Trichostrongylus* spp.; *Triodontophorous* spp.; *Uncinaria* spp., and/or *Wuchereria* spp..

In a suitable use of the invention and/or any embodiment thereof, the animal is protected from a parasite infestation. In one embodiment an existing parasite infestation of the animal is treated or controlled. In another embodiment an existing parasite infestation of the animal is treated or controlled, and the animal is protected from a parasite infestation

Another embodiment is a method of protecting animals that are at risk to be infested by parasites comprising administering an effective amount of at least one compound of the invention and/or embodiments thereof to the animals using a single administration.

In one embodiment the invention provides a composition comprising an effective amount of a compound of the invention and/or embodiments thereof when used for the treatment of parasite infestations of animals and/ or for the protection of animals from infestation by parasites, wherein the composition is administered to an animal that has been diagnosed or is suspected to be infested by parasites. In one embodiment the composition is administered to an animal that is at risk to be infested by parasites to PROTECT it from such infestation.

In a suitable use of the invention and/or any embodiment thereof, the method comprises administering a compound of the invention and/or embodiments thereof to an animal weekly, bi-weekly, monthly, every 6 weeks, every 2 months, every 3 months, every 4 months, every 6 months, or every 9 months.

Surprisingly a number of compounds of the invention and/or embodiments thereof were also able to protect various species of animals from parasite infestation.

In an embodiment of the invention and/or embodiments thereof, the animal is protected from a parasite infestation.

In an embodiment of the invention and/or embodiments thereof, a ruminant animal, preferably a cattle anima is protected, preferably from an infestation with ticks is. In one embodiment the tick is a one host tick. In another embodiment the tick is a multi-host tick.

In an embodiment of the invention and/or embodiments thereof, a companion animal, preferably dog or cat is protected, preferably from an infestation with ticks and/or fleas, preferably ticks, preferably ticks and fleas.

In an embodiment of the invention and/or embodiments thereof, the method comprises administering the compound to an animal weekly, bi-weekly, monthly, every 6 weeks, every 2 months, every 3 months, every 6 months or every nine months.

Another aspect of the current invention are compositions, comprising compounds of the invention and/or embodiments thereof and veterinary or pharmaceutically acceptable excipients for use in treatment and/or protection of animals from parasite infestation.

"Protection or protect " means that a new infestation or re-infestation of an animal, or a mob or flock or herd with parasites is prevented by killing adult parasites and any development/larval stages, that are able to infest the host, before infestation of the host or, by killing or inhibiting the parasites when they infest an animal that has been treated with a compound or preventng generation of offspring of the parasites e.g. reducing the number of eggs laid and/or the hatching rate.

For use in the invention "protection from re-infestation" covers the situation where the animal is exposed to a new infestation by the parasite because it either lives in an environment were living parasites are present or is in close contact with parasite infested animals (or humans)- is therefore "at risk to be infested."

Re-infestation is a second or subsequent infestation by parasites. This means an animal that is at a certain point (substantially) free from a parasite infestation is infested (again) with parasites from external sources. The compounds or composition of the invention kills such new emerging parasites before they can establish an infestation of the animal, or alternatively reduce the number of parasites to limit the irritation and damage to the animal by allowing a non-significant number of parasites.

"Protection period" means the time, expressed in days or weeks after the treatment, that a veterinary test product will preventthe ectoparasite re-infestation of the animal hosts. Sometimes referred to as the prophylactic period or the persistent efficacy period (WAAVP guidelines 2006)

Protective periods may be e.g., useful when treated animals contact untreated animals, or when treated and untreated animals become mixed.

The frequency of the administration will be dependent upon several factors and can be a single dose administered once a day, or depending on the protection period once a week, once a month, once every two, three, four, or six months, or nine months, or once every year. In some embodiments, the frequency of administration may be, for example, weekly, bi-weekly, monthly, bi-monthly, every 3 month, every 4 month, every 5 month, every 6 month, every 9 months or every 12 month or the equivalent administration frequency expressed in days or weeks that approximate such frequency.

In one embodiment, the composition comprising a compound of the invention and/or embodiments thereof exhibits long lasting efficacy and provides protection against parasites in animals, especially livestock animals for at least one month.

In another embodiment, compounds of the invention and/or embodiments thereof exhibit very long-lasting efficacy of at least 50% against ticks that for a period of at least 1 month, at least 2 months, at least 3 months, or at least 4 months or at least 5 months, at least 6 months or 9 months.

In another embodiment, the compounds of the invention and/or embodiments thereof exhibit very long-lasting efficacy of at least 70% against ticks that for a period of at least 1 month, at least 2 months, at least 3 months, or at least 4 months or at least 5 months, at least 6 months or 9 months.

In another embodiment, the compounds of the invention and/or embodiments thereof exhibit very long-lasting efficacy of at least 90% against parasites such as e.g., ticks that for a period of at least 1 month, at least 2 months, at least 3 months, or at least 4 months, or at least 5 months, at least 6 months or 9 months.

In another embodiment, the compounds of the invention and/or embodiments thereof exhibit very long-lasting efficacy of at least 90% against parasites such as fleas that for a period of at least 1 month, at least 2 months, at least 3 months, or at least 4 months, or at least 5 months, at least 6 months or 9 months.

In an embodiment of the invention and/or embodiments thereof, the parasite infestation of the animal is prevented for at least one week, preferably at lest 10 days, at least two weeks, 3 weeks, 4 weeks, 6 weeks, 8 weeks, 10 weeks, 12 weeks, 16 weeks, 26 weeks or 36 weeks.

In an embodiment of the invention and/or embodiments thereof, the parasite infestation of the animal is prevented or controlled for at least two weeks, 3 weeks, 4 weeks, 6 weeks, 8 weeks, 10 weeks, 12 weeks, 16 weeks, 26 weeks or 36 weeks.

In an embodiment of the invention and/or embodiments thereof, the parasite infestation of the animal is prevented or controlled for at least one months, 2 months, 3 months, 6 months, or 9 months.

A single administration of a compound of the invention and/or embodiments thereof is typically sufficient to protect animals from a parasitic infestation. Although such a single dose is typically preferred, it is contemplated that multiple doses can be used for a single treatment.

Another embodiment is a method of treating animals that have been diagnosed or are suspected to be infested by parasites comprising administering an effective amount of at least one compound of the invention and/or embodiments thereof to the animals using a single administration.

In an embodiment of the invention and/or embodiments thereof, an existing parasite infestation of the animal is treated or controlled.

In an embodiment of the invention and/or embodiments thereof, an existing parasite infestation of a companion animal, preferably dog or cat, preferably an infestation with ticks and/or fleas, preferably ticks, preferably ticks and fleas is treated.

In an embodiment of the invention and/or embodiments thereof, an existing parasite infestation of a ruminant animal, preferably a cattle animal, preferably an infestation with ticks is treated. In one embodiment the tick is a one host tick. In another embodiment the tick is a multi-host tick.

A single administration of a compound of the invention and/or embodiments thereof is typically sufficient to treat a parasitic infestation. Although such a single dose is typically preferred, it is contemplated that multiple doses can be used for a single treatment.

The compounds according to this invention are useful in a medicament, especially a medicament for treating and controlling such parasitic infestations of animals. In such medicament a concentration of a compound of the invention and/or embodiments thereof is present that is administered as an effective amount to an animal in need thereof.

An "effective amount," is the amount or quantity of a compound that is required to cause a measurable reduction in the ectoparasite population infesting an animal, and/or to inhibit the development of parasite infestations of an animal, in whole or in part. Alternatively, this may refer to an amount, dose, or quantity that can effectively control and/or reduce presence of parasites in an animal's housing or its surroundings, e.g., the house, building, farm, etc.

This amount is readily determined by observation or detection of the parasite numbers both before and after contacting the animals with the compound. When the compound shows an effect via feeding/ingestion by the parasite, an effective amount generally constitutes an amount that results in tissue and/or blood concentrations generally toxic when ingested by the target parasite.

To establish that an effective reduction of infestation of an animal, or an effective control and/or reduction of parasites in the surroundings has occurred, and thus: what constitutes such an effective amount, is readily determined by comparing the parasite numbers either on the animal or in an animal's environment, before and after administering a compound as described herein.

Detection can be e.g., done by counting of the number of parasites visible on an animal, or by counting parasites using a trap or other detection-device in the surroundings.

The difference in numbers from such counts made before and after treatment indicates the efficacy of the dose applied e.g., the parasite count is reduced, after a first administration, by an amount ranging from 5% to about 100%.

So, although reductions at- or close to 100 % are aimed for, however a reduction in parasite numbers of about 50 % may already constitute a significant reduction. This because even such a modest reduction already alleviates certain symptoms for affected animals, and /or may restore the animals to an improved economic production level.

In one embodiment, an effective amount of the active agent achieves at least about 80%, or at least about 90% efficacy against the target parasites. In a specific preferred embodiment, the efficacy is at least about 95%.

In a preferred embodiment, the reduction of infestation on an animal, or the control and/or reduction achieved, regards a reduction in the number of a particular type of parasite by at least 50, 60, 70, 75, 80, 85, 90, 91, 92, 93, 94, 95, 96, 97, 98, 99, or even 100 %, in that order of preference.

The duration of activity or efficacy (i.e., "knockdown," onset of activity and/ or sustained effects) can be the primary concern in antiparasitic product choices. Products are evaluated based on both their immediate and residual speed of kill and duration of efficacy. A rapid residual speed of kill, sometimes called onset of activity/ efficacy is critically important when attempting to reduce the chances of a (tick) parasite transmitting a pathogen. The durations over which the compounds are effective against various ectoparasites is important for the required treatment interval. This is particularly important, especially considering that such long activities are obtained when using low doses that are non-toxic to the target animal.

In another embodiment, the compounds of the invention provide an efficacy against ectoparasites of at least 95.0% or higher for about 3 months or longer.

In other embodiments, the compounds of the invention have an efficacy of at least about 90% against ectoparasites including, but not limited to, fleas, ticks, mites and parasitic flies, of about 7 months or longer, about 8 months or longer or about 9 months or longer.

In other embodiments, the compounds of the invention have an efficacy of at least about 90% against ectoparasites including, but not limited to, fleas, ticks, mites and parasitic flies, of about 10 months or longer, about 11 months or longer or even about 12 months or longer.

Therefore, one embodiment of the invention is a method wherein the compound is administered every month.

Therefore, in another embodiment of the invention the compound is administered every 6 weeks.

Therefore, in another embodiment of the invention the compound is administered every 2 months.

Therefore, in another embodiment of the invention the compound is administered every 10 weeks.

Therefore, in another embodiment of the invention the compound is administered every 3 months.

Therefore, in another embodiment of the invention the compound is administered every 6 months.

Therefore, in another embodiment of the invention the compound is administered every 9 months.

In one embodiment an effective dose of the compound protects animals, especially cattle from infestation or re-infestation by parasites such as e.g., ticks, mites, lice and/or biting flies for at least 48 days.

Therefore, in one embodiment of the invention the compound is administered every 6 weeks (about 1 and a half months) 7 weeks, 8 weeks (about 2 months), 9 weeks, or 10 weeks (about 2 and a half months).

In another embodiment of the invention, it was surprisingly discovered that the compounds of the invention provide 80% efficacy as early as 3 days after administration and provide long lasting efficacy. For a number of compounds such early onset of activity against ticks was observed even after topical administration.

In yet other embodiments of the invention, the compounds provide an early onset of activity, this means 80 % or more efficacy as early as 3 days or, less than 3 days, e.g., 2 days or 1 day after administration.

The fast acting and long-lasting control provided by the compounds of the current invention is unexpected.

In another aspect the present invention thus provides a pharmaceutical composition comprising an effective amount of one or more, preferably one compound according to the invention and one or more pharmaceutically acceptable excipients.

The compounds according to this invention are useful in controlling parasitic infestations of animals. One of ordinary skill in the art typically can determine an "effective" dose by, for example, observing or detecting changes in a clinical condition or behavior of a host animal, as well as by observing or detecting relative changes in parasite numbers after such treatment.

An "effective amount," is the amount or quantity of a compound that is required to cause a measurable reduction in the ectoparasite population infesting an animal, and/or to inhibit the development of parasite infestations of an animal, in whole or in part. Alternatively, this may refer to an amount, dose, or quantity that can effectively control and/or reduce presence of parasites in an animal's housing or its surroundings, e.g., the house, building, farm, fields, etc.

This amount is readily determined by observation or detection of the pathogen numbers such as parasite numbers both before and after contacting the sample of pathogens such as parasites including their stages with the compound according to this invention, directly and/or indirectly, e.g., by contacting articles, surfaces, foliage, or animals with the compound. When the compound shows an effect via feeding/ingestion by the parasite, an effective amount generally constitutes an amount that results in tissue and/or blood concentrations generally toxic when ingested by the target parasite.

To establish that an effective reduction of infestation of an animal, or an effective control and/or reduction of parasites in the surroundings has occurred, and thus: what constitutes such an effective amount, is readily determined by comparing the parasite numbers either on the animal or in an animal's environment, before and after administering a compound as described herein.

Detection can be e.g., done by counting of the number of parasites visible on an animal, or by counting parasites using a trap or other detection-device in the surroundings.

The difference in numbers from such counts made before and after treatment indicates the efficacy of the dose applied e.g., the parasite count is reduced, after a first administration, by an amount ranging from 5% to about 100%.

So, although reductions at- or close to 100 % are aimed for, however a reduction in parasite numbers of about 50 % may already constitute a significant reduction. This because even such a modest reduction already alleviates certain symptoms for affected animals, and /or may restore the animals to an improved economic production level.

In one embodiment, an effective amount of the active agent achieves at least about 80%, or at least about 90% efficacy against the target parasites. In a specific preferred embodiment, the efficacy is at least about 95%.

In a preferred embodiment, the reduction of infestation on an animal, or the control and/or reduction achieved, regards a reduction in the number of a particular type of parasite by at least 50, 60, 70, 75, 80, 85, 90, 91, 92, 93, 94, 95, 96, 97, 98, 99, or even 100 %, in that order of preference.

### Animals to be treated

The compounds or composition may be used for controlling parasite infestations in a range of animals, especially domestic animals.

As used herein, the term "domestic animal" includes any animal that is kept by humans as a companion animal, pet, working animal or as livestock for food, fur, leather, wool or other animal product; or an animal that is found in association with humans such that control of ectoparasites on such animal is desirable.

In one embodiment such animals are warm-blooded animals. Such warm-blooded animals include poultry animals such as chickens and turkey and mammals, for example, mammals. Mammals include, for example, farm or livestock mammals (e.g., swine, bovines, sheep, goats, etc.), laboratory mammals (e.g., mice, rats, jirds, etc.), companion mammals (e.g., dogs, cats, equines, etc.), fur-bearing animals (e.g., minks, foxes, chinchillas, rabbits, etc.), and wild and zoo mammals (e.g., buffalo, deer, etc.).

The compounds according to the present invention are particularly useful for livestock animals, kept for meat and milk or wool or alternatively as working animals, especially ruminants such as cattle, sheep, goat, deer, camels, lamas.

In one embodiment the animals are bovine animals such as cattle. In this specification, bovine animals are ruminant mammals of the genus *Bos* and include, but are not limited to, cattle, such as steers, heifers, cows (lactating and non-lactating), calves, bulls, and also buffalo. Especially preferred are beef cattle, i.e., cattle animals kept for meat or alternatively dairy cows. In another embodiment the animals are sheep. In another embodiment the animals are goats.

In another preferred embodiment, the methods and compositions of the invention are used for the treatment prevention of parasitic infections and infestations in ruminants, especially cattle or sheep. When treating livestock animals such as cattle or sheep, the methods and compositions are particularly effective against one host ticks, especially *Rhipicephalus (Boophilus) microplus,*

In some embodiments, the compounds are used to treat companion animals, especially canine or feline animals. In one embodiment are used to treat canines (e.g., domestic dogs). In other embodiments, the compounds are used to treat felines (e.g., domestic cats).

In a preferred embodiment, the invention provides methods and compositions for the treatment or prevention of parasitic infections and infestations in companion animals including, but not limited to, cats and dogs. The methods and compositions are particularly effective for preventing or treating parasitic infestations of cats and dogs with fleas and/or ticks.

The compounds also are suitable to treat warm-blooded non-mammals, such as poultry (e.g., turkeys, chickens, geese, ducks, parrots, etc.).

In one embodiment the compounds are used to treat cold-blooded animals, such as fish (e.g., salmon, trout, sea bass, sea bream or ornamental fish such as koi, etc.). Therefore, in one embodiment such animals are fish.

In a preferred embodiment the treated fish is salmon. The term "salmon" within the scope of this invention will be understood as comprising all representatives of the family Salmonidae, especially of the subfamily *Salmoninae,* and preferably, the Atlantic salmon (*Salmo salar*)*,* rainbow trout (*Oncorhynchus mykiss),* brown or sea trout (*S. trutta*)*.*

The compounds for use in the invention can be made available during a treatment period to a single animal.

Especially for livestock animals more advantageous is the treatment at the same time of a group of animals, or to all animals in a single stable, pen, net, group, house, or farm. For companion animals preferably all animals at risk of a parasite infestation are treated at the same time.

A single administration of a compound according to this invention or a compound corresponding to the use according to the invention is typically sufficient to treat a parasitic infestation. Although such a single dose is typically preferred, it is contemplated that multiple doses can be used.

The frequency of the administration will be dependent upon several factors and can be a single dose administered once a day, once a week, once a month, once every two, three, four, or six months, or one every year. In some embodiments, the frequency of administration may be, for example, weekly, bi-weekly, monthly, bi-monthly, every 3 month, every 4 month, every 5 month, every 6 month or every 12 month or the equivalent administration frequency expressed in days or weeks that approximate such frequency.

The duration of activity or efficacy (i.e., "knockdown," onset of activity and/ or sustained effects) can be the primary concern in antiparasitic product choices. Products are evaluated based on both their immediate and residual speed of kill and duration of efficacy. A rapid residual speed of kill , sometimes called onset of activity/ efficacy is critically important when attempting to reduce the chances of a tick parasite transmitting a pathogen. The durations over which the compounds are effective against various ectoparasites is important for the required treatment interval. This is particularly important, especially considering that such long activities are obtained when using low doses that are non-toxic to the target animal.

In one embodiment, the composition exhibits long lasting efficacy and provides protection against parasites in livestock animals for at least one month.

In another embodiment, the compounds of the invention exhibit very long-lasting efficacy of at least 50% against ticks that for a period of at least 1 month, at least 2 months, at least 3 months, or at least 4 months.

In another embodiment, the compounds of the invention exhibit very long-lasting efficacy of at least 70% against ticks that for a period of at least 1 month, at least 2 months, at least 3 months, or at least 4 months.

In another embodiment, the compounds of the invention exhibit very long-lasting efficacy of at least 90% against parasites such as e.g. ticks that for a period of at least 1 month, at least 2 months, at least 3 months, or at least 4 months.

Therefore, one embodiment of the invention is a method wherein the compound is administered every month.

Therefore, in another embodiment of the invention the compound is administered every 6 weeks.

Therefore, in another embodiment of the invention the compound is administered every 2 months.

Therefore, in another embodiment of the invention the compound is administered every 3 months.

In one embodiment an effective dose of the compound protects animals, especially cattle from infestation or re-infestation by parasites such as e.g., ticks, mites, lice and/or biting flies for at least 48 days.

Therefore, in one embodiment of the invention the compound is administered every 6 weeks (about 1 and a half months) 7 weeks, 8 weeks (about 2 months), 9 weeks, or 10 weeks (about 2 and a half months).

In another embodiment of the invention, it was surprisingly discovered that the compounds of the invention provide 80% efficacy as early as 2 to 6 days after administration and provide long lasting efficacy. For a number of compounds such early onset of activity against ticks was observed even after topical administration.

In yet other embodiments of the invention, the compounds provide an early onset of activity, this means 80 % or more efficacy as early as 3 days or 2 days or 1 day after administration.

The fast acting and long-lasting control provided by the compounds of the current invention is unexpected.

The compounds for use in the invention can be made available during a treatment period to a single animal.

Especially for livestock animals more advantageous is the treatment at the same time of a group of animals, or to all animals in a single stable, pen, net, group, house, or farm. For companion animals preferably all animals at risk of a parasite infestation are treated at the same time.

Generally, a dose of from about 0.001 to about 100 mg per kg of body weight given as a single dose will be used but, of course, there can be instances where higher or lower dosage ranges are indicated, and such are within the scope of this invention.

In some embodiments, for example, from about 0.01 to about 100 mg/kg body weight is administered.

In other embodiments, for example, from about 0.01 to about 50 mg/kg body weight is administered.

In some such embodiments, for example, from about 0.1 to about 40 mg/kg body weight is administered.

In other such embodiments, from about 1 to about 30 mg/kg body weight is administered. Greater dosages tend to provide for greater duration of activity / efficacy.

Factors affecting the preferred dosage may include, for example, the parasite species infestation to be treated and the development stages of the parasites, the type (e.g., species and breed), age, size, sex, diet, activity, and condition of the infected animal; the dosage route; pharmacological considerations, such as the activity, efficacy, pharmacokinetic, and toxicology profiles of the particular composition administered; and whether the compound according to this invention being administered as part of a combination of active ingredients. Thus, the preferred amount of the compound according to this invention can vary, and, therefore, can deviate from the typical dosages set forth above.

For many animals, the dose and composition comprising the compounds are chosen to maintain a blood level of at least about 30 ng/ml, more preferably about 40 ng/ml, about 50 ng/ml, or 60 ng/ml.

In some embodiments, the concentration of the compounds in the plasma that is sufficient to obtain at least 80% efficacy against ticks is less than or equal to about 60 ng/ml.

Generally, a dose of from about 0.001 to about 100 mg per kg of body weight given as a single dose or in divided doses for a period of from 1 to 5 days will be satisfactory but, of course, there can be instances where higher or lower dosage ranges are indicated, and such are within the scope of this invention.

When compounds according to this invention is orally administered, the total dose is generally greater than about 0.01 mg/kg (i.e., milligram of compound according to this invention per kilogram body weight of the treated animal). The oral administration of compounds has been surprisingly shown to protect animals from parasite infestation, especially dogs from flea and/or tick infestation for an exceptionally long period.

Therefore, in one embodiment the compound is administered orally to the animal, especially dog to protect the animal from parasite infestation, especially from fleas and or ticks, preferably from fleas and ticks.

In some such embodiments, the total dose is from about 0.01 to about 100 mg/kg, from about 0.01 to about 50 mg/kg, from about 0.1 to about 25 mg/kg, or from about 1 to about 20. For sheep, for example, the dose is generally from about 0.5 to about 15 mg/kg, from about 1 to about 10 mg/kg. The same dose range may be suitable for other dosage routes.

For example, in some embodiments, a higher dose range is used for topical pour on administration.

In one embodiment the compound is administered by topical pour on administration to the animal, especially cattle to protect the animal from parasite infestation, especially from acaride parasites, preferably from ticks.

For example, in some embodiments, the same dose range is used for subcutaneous administration.

In one embodiment the compound is administered by injection, especially subcutaneous administration to the animal, especially cattle to protect the animal from parasite infestation, especially from acaride parasites, preferably from ticks.

If the compound according to this invention is administered parenterally via an injection, especially via subcutaneous administration the concentration of the compound according to this invention in the dosage form preferably is sufficient to provide the desired effective amount of the compound according to this invention in a volume that is acceptable for parenteral administration. Furthermore, no severe injection site reaction should be resulting from such injection.

In some embodiments, one or more, preferably one compound according to this invention is used to control an infestation by a parasite, especially ectoparasite, especially tick that is resistant to one or more commercially available and known existing parasiticide compounds.

Therefore, the compounds of the current invention can be used on animals that are infested or at risk of infestation with parasites, especially ticks, that are resistant to any one of organophosphates, synthetic pyrethroids, or benzoyl phenyl urea compounds and will still provide effective treatment/ protection.

Factors affecting the preferred dosage may include, for example, the parasite species infestation to be treated and the development stages of the parasites, the type (e.g., species and breed), age, size, sex, diet, activity, and condition of the infected animal; the dosage route; pharmacological considerations, such as the activity, efficacy, pharmacokinetic, and toxicology profiles of the particular composition administered; and whether the compound according to this invention being administered as part of a combination of active ingredients. Thus, the preferred amount of the compound according to this invention can vary, and, therefore, can deviate from the typical dosages set forth above.

For many animals, the dose and composition comprising the compounds are chosen to maintain a blood level of at least about 30 ng/ml, more preferably about 40 ng/ml, about 50 ng/ml, or 60 ng/ml.

In some embodiments, the concentration of the compounds in the plasma that is sufficient to obtain at least 80% efficacy against ticks is less than or equal to about 60 ng/ml.

### Administration routes

When compounds according to this invention is orally administered, the total dose is generally greater than about 0.01 mg/kg (i.e., milligram of compound according to this invention per kilogram body weight of the treated animal).

In some such embodiments, the total dose is from about 0.01 to about 100 mg/kg, from about 0.01 to about 50 mg/kg, from about 0.1 to about 25 mg/kg, or from about 1 to about 20. For sheep, for example, the dose is generally from about 0.5 to about 15 mg/kg, from about 1 to about 10 mg/kg. The same dose range may be suitable for other dosage routes.

For example, in some embodiments, a higher dose range is used for topical pour on administration.

For example, in some embodiments, the same dose range is used for subcutaneous administration.

If the compound according to this invention is administered parenterally via an injection, especially via subcutaneous administration the concentration of the compound according to this invention in the dosage form preferably is sufficient to provide the desired therapeutically effective amount of the compound according to this invention in a volume that is acceptable for parenteral administration. Furthermore, no severe injection site reaction should be resulting from such injection.

In some embodiments, one or more, preferably one compound according to this invention is used to control an infestation by a parasite, especially ectoparasite, especially tick that is resistant to one or more commercially available and known existing parasiticide compounds.

Therefore, the compounds of the current invention can be used on animals that are infested or at risk of infestation with parasites, especially ticks, that are resistant to any one of organophosphates, synthetic pyrethroids, or benzoyl phenyl urea compounds and will still provide effective treatment/ protection.

### DOSAGE FORM

The compounds according to this invention may be administered in various dosage forms.

The term "dosage form" means that the compounds according to this invention are formulated into a product suitable for administering to the animal via the envisaged administration route. Such dosage forms are sometimes referred to herein as formulations or pharmaceutical composition.

The formulation type chosen for a dosage form in any instance will depend upon the particular purpose envisaged and the physical, chemical, and biological properties of the compound according to this invention.

The invention is also directed to a veterinary composition comprising an effective amount of a compound of Formula (I) as defined in any embodiment described herein and an inert carrier or formulation adjuvant. The composition is suitable for use in the treatment and control of a parasitic infestation of animals such as described herein.

Suitably the veterinary composition is a topical composition, an oral composition, or an injection composition.

The present invention is also directed to a veterinary composition comprising an effective amount of a compound of Formula (I) as defined in any embodiment described herein and an inert carrier or formulation adjuvant. Suitably the composition is used in the treatment and control of a parasitic infestation of animals as described herein.

In an embodiment of the invention and/or embodiments thereof, the veterinary composition is a topical composition.

In an embodiment of the invention and/or embodiments thereof, the veterinary composition is an oral composition.

In an embodiment of the invention and/or embodiments thereof, the veterinary composition is an injection composition.

In another aspect the present invention thus provides a pharmaceutical composition comprising an effective amount of one or more, preferably one compound according to the invention and one or more pharmaceutically acceptable excipients.

The term "excipient," "diluent" or "carrier" is used herein to describe any ingredient other than the Formula (I) compounds or any additional antiparasitic agent in a dosage form. The choice of excipient, diluent, or carrier will to a considerable extent depend on factors such as the particular route and mode of administration, the effect of the excipient, carrier, or diluent on solubility and stability, and the nature of the dosage form.

Pharmaceutically acceptable as used in this application, for example with reference to salts and composition components such as carriers, includes "dermatologically acceptable" for topical dosage forms, and "veterinary acceptable," and thus includes both human and animal applications independently.

Dosage forms useful in the current invention can be liquid, semi-solid or solid dosage forms.

Liquid dosage forms of the compounds are generally solutions, suspensions, or emulsions. A solution is a mixture of two or more components that form a single phase that is homogeneous down to the molecular level. A suspension consists of insoluble solid particles dispersed in a liquid medium, with the solid particles accounting for about 0.5% to about 30% of the suspension. The liquid may be aqueous, oily, or both. An emulsion is a heterogeneous dispersion of one immiscible liquid in another; it relies on an emulsifying agent for stability.

A dry powder (or granule) for reconstitution is reconstituted as a solution or as a suspension immediately prior to injection. The principal advantage of this dosage form is that it overcomes the problem of instability in solution or suspension.

One possible administration route is the oral route, wherein the compound according to this invention is administered via the mouth. Oral dosage forms suitable for oral administration comprise liquids (e.g., drench or drinking water formulations), semi-solids (e.g., pastes, gels), and solids (e.g., tablets, capsules, powders, granules, chewable treats, premixes, and medicated blocks).

A number of veterinary compositions are known to be suitable for oral administration to animals, but they vary for the different animal species. For sheep conventionally pharmaceutically active ingredients are administered orally as solids (e.g., tablets or boluses) or liquids, and via their feed or drinking water. In large sheep and cattle flocks the use of oral drenches is the most common oral dosage form, especially when administering anthelmintic compounds.

Drenching means that a liquid, potentially slightly viscous composition comprising the compound and excipients is applied via the mouth with a specific drenching gun that dispenses a compound into the sheep's throat.

A drench is a liquid oral formulation that is administered directly into the mouth/throat of an animal, especially a livestock animal, by means of a "drench gun" or syringe or another suitable device. When the composition is administered in the animal recipient's drinking water or as a drench, it may be convenient to use a solution or suspension formulation. This formulation can be, for example, a concentrated suspension that is mixed with water or a dry preparation that is mixed and suspended in the water.

Semi-solid oral formulations (pastes or gels) are generally administered via an applicator directly into the mouth of an animal or mixed with the feed.

Solid oral formulations are either administered directly to an animal (tablet, capsule, bolus) or mixed with the feed or via medicated feed blocks.

When the oral formulation is administered via a non-human animal's feed, it may, for example, be fed as a discrete feed or as a chewable treat. Alternatively (or additionally), it may, for example, be intimately dispersed in the animal recipient's regular feed, used as a top dressing, or in the form of solid pellets, paste or liquid that is added to the finished feed. When the oral formulation is administered as a feed additive, it may be convenient to prepare a "premix" in which the oral formulation is dispersed in a liquid or solid carrier. This "premix" is, in turn, dispersed in the animal's feed using, for example, a conventional mixer in order to ensure a more homogeneous distribution of the compound in the feed.

Several modified-release delivery systems have been developed, that take advantage of the unique anatomy of the ruminant forestomach, i.e., for intra-ruminal administration. An intraruminal bolus is a specific formulation for ruminants (cattle, sheep, goats, buffalos, camelids, deer etc.). It is a veterinary delayed release delivery system which remains in the rumeno-reticular sac of a ruminant animal over an extended period of time and in which the therapeutically active substance has a predictable and delayed release pattern. Such intraruminal boluses are usually administered using a balling gun or another suitable device.

Compounds according to this invention may alternatively be administered via non-oral dosage routes, such as topically (e.g., via a spot-on, pour-on, spray), or parenterally (e.g., subcutaneous injection, intravenous injection, intramuscular injection, etc.).

For instance, the compounds according to this invention may be administered topically using a transdermal formulation (i.e., a formulation that passes through the skin).

Topical dosage forms suitable for topical administration comprise liquids (e.g., bath, spray, spot-on), semi-solids (e.g., creams, gels), and solids (e.g., patches, powders, collars). Typical topical formulations for animals are liquid or semi-liquid dosage forms.

Typical formulations for topical and transdermal administration include, for example, pour-ons, spot-ons, dips, sprays, mousses, shampoos, powders, gels, hydrogels, lotions, solutions, creams, ointments, dusting powders, dressings, foams, films, skin patches, limb bands, collars, ear tags, wafers, sponges, fibers, bandages, and microemulsions. When a liquid formulation is used topically on skin, it can be administered by, for example, pouring on (pour-on or spot-on), spreading, rubbing, atomizing, spraying, dipping, bathing, or washing.

The pour-on or spot-on methods, for example, comprise applying the formulation to a specific location of the skin or coat, such as on the neck or backbone of the animal. This may be achieved by, for example, applying a swab or drop of the pour-on or spot-on formulation to a relatively small area of the recipient animal's skin or coat (i.e., generally no greater than about 10% of the animal recipient's skin or coat). In some embodiments, the compound according to this invention is dispersed from the application site to wide areas of the fur due to the spreading nature of the components in the formulation and the animal's movements while, in parallel, being absorbed through the skin and distributed via the animal recipient's fluids and/or tissues.

Parenteral formulations and delivery systems for non-oral routes comprise liquids (e.g., solutions, suspensions, emulsions, and dry powders for reconstitution), semi-solids and solids (e.g., implants). The majority of implants that are used in veterinary medicine are compressed tablets or dispersed matrix systems in which the drug is uniformly dispersed within a biodegradable or nondegradable polymer or are extrusion products.

### PHARMACEUTICAL COMPOSITIONS

This invention also is directed to pharmaceutical compositions (or medicaments) comprising one or more, preferably one compound according to this invention. The compositions also may (and preferably will) comprise one or more pharmaceutically acceptable excipients. The following subject matter about pharmaceutical compositions is also applicable to pharmaceutical compositions comprising compounds corresponding to the use according to this invention.

Pharmaceutical compositions of the present invention may be manufactured by, for example, processes known in the art. These processes include, for example, a variety of known mixing, dissolving, granulating, emulsifying, encapsulating, entrapping, and lyophilizing processes. Optimal formulation depends on, for example, the dosage route (e.g., oral, parenteral by injection, topical).

Solid dosage forms, for example, may be prepared by, for example, intimately and uniformly mixing the compounds with fillers, binders, lubricants, glidants, disintegrants, flavoring agents (e.g., sweeteners), buffers, preservatives, pharmaceutical-grade dyes or pigments, and controlled release agents.

Oral dosage forms other than solids may be prepared by mixing the compounds with, for example, one or more solvents, viscosity-enhancing agents, surfactants, preservatives, stabilizers, resins, fillers, binders, lubricants, glidants, disintegrants, co-solvents, sweeteners, flavorings, buffers, suspending agents, and pharmaceutical-grade dyes or pigments.

Contemplated binders include, for example, starch, gelatin, acacia, and carboxymethyl cellulose.

Contemplated lubricants include, for example, magnesium stearate, stearic acid, and talc.

Contemplated disintegrants include, for example, corn starch, alginic acid, sodium carboxymethylcellulose, and sodium croscarmellose.

Contemplated buffers include, for example, sodium citrate, and magnesium and calcium carbonate and bicarbonate.

Contemplated solvents include, for example, water, pyrrolidone solvents, vegetable oils, mineral oil, and synthetic oil such as medium chain triglycerides e.g., Miglyol 812 or 840. Physiological saline solution or glycols (e.g., propylene glycol, or polyethylene glycol) also may be included. The solvent preferably has sufficient chemical properties and quantity to keep the compounds solubilized at temperatures in which the composition is stored and used.

Contemplated viscosity-enhancing agents include, for example, polyethylene, methylcellulose, sodium carboxymethylcellulose, hydroxypropyl-methylcellulose, hydroxypropylcellulose, sodium alginate, carbomer, povidone, acacia, guar gum, xanthan gum, tragacanth, methylcellulose, carbomer, xanthan gum, guar gum, povidone, sodium carboxymethylcellulose, magnesium aluminum silicate, carboxyvinyl polymers, carrageenan, hydroxyethyl cellulose, laponite, water-soluble salts of cellulose ethers, natural gums, colloidal magnesium aluminum silicate or finely divided silica, homopolymers of acrylic acid crosslinked with an alkyl ether of pentaerythritol or an alkyl ether of sucrose, and carbomers.

Contemplated surfactants include, for example, sodium lauryl sulfate, polyoxyethylene sorbitan fatty acid esters; polyoxyethylene monoalkyl ethers; sucrose monoesters; lanolin esters and ethers; alkyl sulfate salts; and sodium, potassium, and ammonium salts of fatty acids.

Contemplated preservatives include, for example, phenol, alkyl esters of parahydroxybenzoic acid (e.g., methyl p-hydroxybenzoate (or "methylparaben") and propyl p-hydroxybenzoate (or "propylparaben")), sorbic acid, o-phenylphenol benzoic acid and the salts thereof, chlorobutanol, benzyl alcohol, thimerosal, phenylmercuric acetate and nitrate, nitromersol, benzalkonium chloride, and cetylpyridinium chloride.

Contemplated stabilizers include, for example, chelating agents and antioxidants.

Solid dosage forms also may comprise, for example, one or more excipients to control the release of the compounds. For example, it is contemplated that the compounds may be dispersed in, for example, hydroxypropylmethyl cellulose. Some oral dosage forms (e.g., tablets and pills) also may be prepared with enteric coatings.

Topical dosage route uses, for example, a concentrated liquid or semi-liquid solution, suspension (aqueous or non-aqueous), emulsion (water-in-oil or oil-in-water), or microemulsion comprising a compound dissolved, suspended, or emulgated in a pharmaceutically acceptable liquid vehicle. In such embodiments, a crystallization inhibitor optionally may generally be present.

Such a pour-on or spot-on formulation can be prepared by dissolving, suspending, or emulsifying the compounds in a suitable skin-fitted solvent or solvent mixture. Other excipients may be included as well, such as, for example, a surfactant, colorant, antioxidant, stabilizer, adhesive, etc. Contemplated solvents include, for example, water, alkanol, glycol, polyethylene glycol, polypropylene glycol, glycerin, benzyl alcohol, phenylethanol, phenoxyethanol, ethyl acetate, butyl acetate, benzyl benzoate, dipropylene glycol monomethyl ether, diethylene glycol monobutyl ether, acetone, methyl ethyl ketone, aromatic and/or aliphatic hydrocarbons, vegetable or synthetic oil, DMF, liquid paraffin, silicone, dimethylacetamide, N-methylpyrrolidone, or 2,2-dimethyl-4-oxy-methylene-1,3-dioxolane.

In some embodiments, a topical formulation (particularly a pour-on or spot-on formulation) comprises a carrier that promotes the absorption or penetration of the compounds through the skin into the blood stream, other bodily fluids (lymph), and/or body tissue (fat tissue). Contemplated examples of dermal penetration enhancers include, for example, dimethylsulfoxide, isopropyl myristate, dipropylene glycol pelargonate, silicone oil, aliphatic esters, triglycerides, and fatty alcohols.

Topical formulations also (or alternatively) may comprise, for example, one or more spreading agents. These substances act as carriers that assist in distributing an active ingredient over the animal recipient's coat or skin. They may include, for example, isopropyl myristate, dipropylene glycol pelargonate, silicone oils, fatty acid esters, triglycerides, and/or fatty alcohols. Various spreading oil/solvent combinations also may be suitable, such as, for example, oily solutions, alcoholic and isopropanolic solutions (e.g., solutions of 2-octyl dodecanol or oleyl alcohol), solutions of esters of monocarboxylic acids (e.g., isopropyl myristate, isopropyl palmitate, lauric acid oxalic ester, oleic acid oleyl ester, oleic acid decyl ester, hexyl laurate, oleyl oleate, decyl oleate, and caproic acid esters of saturated fatty alcohols having a carbon chain of 12 to 18 carbons), solutions of esters of dicarboxylic acids (e.g., dibutyl phthalate, diisopropyl isophthalate, adipic acid diisopropyl ester, and di-n-butyl adipate), or solutions of esters of aliphatic acids (e.g., glycols). When the formulation comprises a spreading agent, it also may be advantageous to include a dispersant, such as, for example, pyrrolidin-2-one, N-alkylpyrrolidin-2-one, acetone, polyethylene glycol or ether or ester thereof, propylene glycol, or synthetic triglycerides.

Injectable formulations may be prepared according to, for example, the known art using suitable solvents, solubilizing agents, protecting agents, dispersing agents, wetting agents, and/or suspending agents. Contemplated carrier materials include, for example, water, ethanol, butanol, benzyl alcohol, glycerin, 1,3-butanediol, Ringer's solution, isotonic sodium chloride solution, bland fixed oils (e.g., synthetic mono- or diglycerides), vegetable oil (e.g., corn oil), fatty acids (e.g., oleic acid), dimethyl acetamide, surfactants (e.g., ionic, and non-ionic detergents), N-methylpyrrolidone, propylene glycol, and/or polyethylene glycols (e.g., PEG 400). Contemplated solubilizing agents include, for example, polyvinyl pyrrolidone, polyoxyethylated castor oil, polyoxyethylated sorbitan ester, and the like. Contemplated protecting agents include, for example, benzyl alcohol, trichlorobutanol, p-hydroxybenzoic acid ester, n-butanol, and the like.

In some embodiments, a parenteral formulation is, for example, prepared from sterile powders or granules having one or more of the carrier materials discussed above for other formulations. The compound is, for example, dissolved or suspended in a liquid comprising water, polyethylene glycol, propylene glycol, ethanol, corn oil, cottonseed oil, peanut oil, sesame oil, benzyl alcohol, sodium chloride, and/or various buffers. The pH generally may be adjusted, if necessary, with a suitable acid, base, or buffer.

Further aspects regarding formulation of drugs and various excipients are found in, for example, Gennaro, A.R., et al., eds., Remington: The Science and Practice of Pharmacy (Lippincott Williams & Wilkins, 20th Ed., 2000).

The concentration of the compounds according to this invention in the applied dosage form may vary widely depending on, for example, the dosage route. In general, the concentration in the dosage form is from about 1 to about 70% (by weight). In some such embodiments, for example, the concentration is from about 1 to about 50% (by weight), or from about 10 to about 50% (by weight). In other embodiments, the concentration is from about 35 to about 65% (by weight), from about 40 to about 60% (by weight), from about 45 to about 55% (by weight), or about 50% (by weight).

In another aspect the present invention thus provides a pharmaceutical composition comprising an effective amount of one or more, preferably one compound according to this invention and one or more pharmaceutically acceptable excipients.

The formulation type chosen for a dosage form in any instance will depend upon the particular purpose envisaged and the physical, chemical, and biological properties of the compound according to this invention.

### TOPICAL PHARMACEUTICAL COMPOSITIONS

In one embodiment the compound of Formula (I)-(XCII) according to the invention and/or embodiments thereof is administered topically to an animal, preferably by pour-on or spot-on administration.

In another aspect of the invention and/or embodiments thereof, a use or method for protecting an animal from or treating a parasite infestation/infection in an animal is provided, comprising administering to the animal a topical composition comprising an effective amount of at least one compound of the invention together with a pharmaceutically acceptable carrier that is suitable for application to the skin of the animal.

In some embodiments of the invention, the topical compositions are formulated to control the rate of permeation of the compound in order to maintain efficacious levels of the active in the plasma for a prolonged period of time and significantly extend the duration of efficacy.

In a specific embodiment the current invention is directed to a veterinary pharmaceutical composition comprising a compound of Formula (I) according to the invention and/or embodiments thereof and a pharmaceutically acceptable oily carrier.

The topical composition is administered to the animal in an amount of about 0.5 mg to about 10 mg, even more typically about 1 mg to about 2.5 mg, of the compound of Formula (I)-(XCII) according to the invention and/or any embodiment thereof, especially of compound according to formula (LIX), formula (LIXa), formula (LXI) or formula (LXla) per kg bodyweight of the treated animal.

### Compound of formula (LIX)

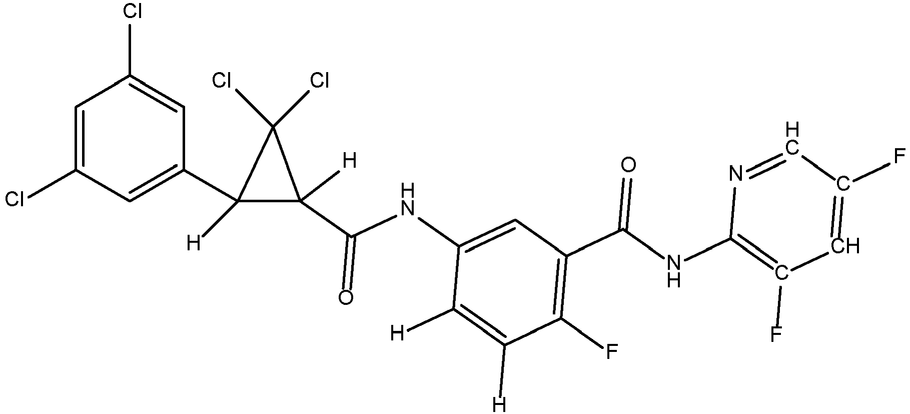

and N-oxides, veterinary acceptable acid addition salts, salt derivatives, solvates, ester derivatives, crystal polymorphs, isotopes, stereoisomers, and tautomers thereof.

Compound of formula (LIXa) 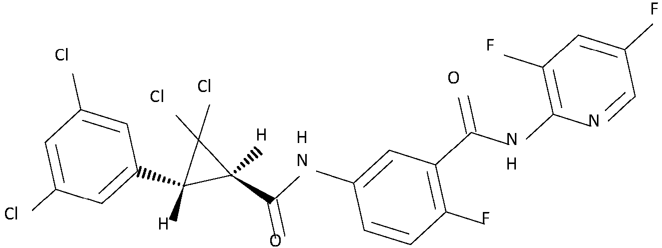 and N-oxides, veterinary acceptable acid addition salts, salt derivatives, solvates, ester derivatives, crystal polymorphs, isotopes, stereoisomers, and tautomers thereof.

Compound of formula (LXI) 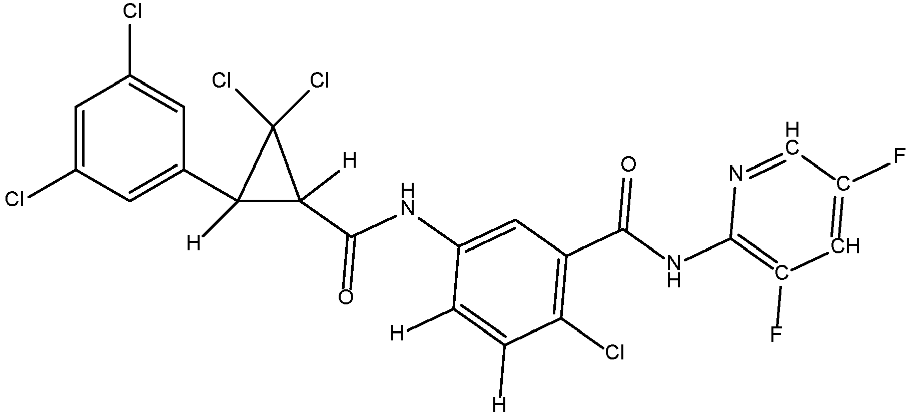 and N-oxides, veterinary acceptable acid addition salts, salt derivatives, solvates, ester derivatives, crystal polymorphs, isotopes, stereoisomers, and tautomers thereof.

Compound of formula (LXla) 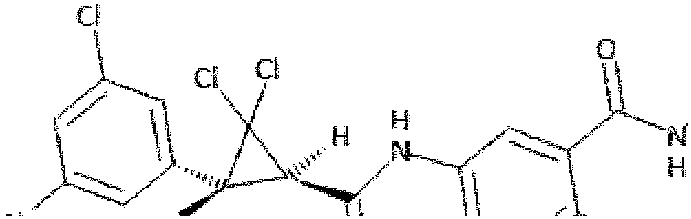 and N-oxides, veterinary acceptable acid addition salts, salt derivatives, solvates, ester derivatives, crystal polymorphs, isotopes, stereoisomers, and tautomers thereof.

However, as will be apparent to a person skilled in the art, the effective amount to control particular parasites of an animal will vary depending on a number of factors including, for example, the particular compound of Formula (I) in the composition, the species of animal, the age, sex and health of the animal, the particular parasites infesting the animal and the severity of parasitic infestation. For any given case, a person skilled in the art could readily determine an effective amount using standard techniques and routine experimentation.

The composition of the present invention includes as a pharmaceutically effective oily carrier.

"Pharmaceutically or veterinarily acceptable carrier" means a carrier that is useful in preparing a pharmaceutical composition that is generally safe, non-toxic, and neither biologically nor otherwise undesirable, and includes a carrier that is acceptable for veterinary use or pharmaceutical use.

As used herein, by an "oily carrier" means a carrier that used an oil as a solvent for dissolving the compound or, otherwise, adopts oil as a medium for dispersing or suspending the compound in a powder form, which is insoluble in the oil. It is meant that an oil constitutes at least 50% by weight of the total amount of the carrier and may include additional nonvolatile co-solvent or co-solvents.

In a preferred embodiment an oil constitutes at least 99% to 99.5 % by weight of the total amount of the carrier.

According to the present invention, the oil may be a solvent in which the compound of Formula (I)-(XCII) and/or embodiments thereof, is dissolved, dispersed and/or suspended and may include, for example; monoglyceride, diglyceride, triglyceride, medium chain triglyceride (MCT), sesame oil, arachis oil (peanut oil), castor oil, olive oil, corn oil, cotton seed oil, soybean oil, peppermint oil, coconut oil, palm seed oil, safflower oil, etc., without being particularly limited thereto. These substances may be used alone or as a mixture of two or more thereof.

In a preferred embodiment the carrier consists of a fatty acid ester. Fatty acid esters (FAEs) are a type of ester that result from the combination of a fatty acid with an alcohol. When the alcohol component is glycerol, the fatty acid esters produced can be monoglycerides, diglycerides, or triglycerides.

In an alternative embodiment the oily carrier comprises a fatty acid ester.

Preferably the fatty acid ester is a medium chain triglyceride. Medium chain triglycerides (MCT) have fatty acid chains of 6 - 12 carbon atoms and for the medically refined grades of MCT oil each chain has 8 - 10 carbon atoms.

The MCT oil may comprise either triglycerides of the C₈-C₁₀ fatty acids, or propylene glycoldiesters of these fatty acids or a mixture of both triglycerides and propylene glycol diesters. Preferably these C8 -C10 fatty acids are fully saturated, such as n-caprylic and n-capric acids. These are conveniently prepared by the commercial fractionating of naturally occurring vegetable (e.g. coconut) oil to give mainly C8-10 fatty acids followed by esterification of these acids with a chosen alcohol.

Fractionated vegetable oil having the desired composition is commercially available. Proprietary examples of such MCT oils are Miglyol^{®} 812 as capric/caprylic triglycerides and Miglyol^{®} 840 as propylene glycol dicaprylate/caprate. Most preferred is propylene glycol dicaprylate/caprate such as e.g., Miglyol 840.

A preferred additional component is Glycerol Monocaprylate, e.g. commercially available as Imwitor^{®}988.

It has been found that a particular group of MCT oils has the combined properties of low irritancy and appropriate solvency and is particularly suitable for use in compositions for localised topical application of the compound.

These medium chain fatty acid ester have the additional advantages of having low melting points and it has been surprisingly found that Miglyol 840 results in a very high level of transdermal penetration of the compound of Formula (I) according to the invention and/or any embodiment thereof.

Specifically, the invention relates to a composition according to the invention comprising of compound according to formula (LIX), formula (LIXa), formula (LXI) or formula (LXla) as described above dissolved in a mixture of glycerol and propylene glycol esters of fatty acids.

One example of a composition according to the invention consists of 1%w/v of compound according to formula (I) of the invention and/or embodiments therofand 99% w/v propylene glycol dicaprylocaprate.

Another example of a composition according to the invention consists of 1% w/v of compound according to formula (I) according to the invention and/or embodiments thereof, 20% w/v glycerol monocaprylate, and 79%w/v propylene glycol dicaprylocaprate.

One example of a composition according to the invention consists of 1%w/v of compound according to formula (LIX), formula (LIXa), formula (LXI) or formula (LXla), preferably compound of formula (LXI) or formula (LXIa), and 99% w/v propylene glycol dicaprylocaprate.

Another example of a composition according to the invention consists of 1% w/v of compound according to formula (LIX), formula (LIXa), formula (LXI) or formula (LXla), preferably compound of formula (LXI) or formula (LXla), 20% w/v glycerol monocaprylate, and 79%w/v propylene glycol dicaprylocaprate.

The examples show how, contrary to many topical solutions (such as e.g., the commercial pour- on product Dectomax from Zoetis) the composition of the invention does not require rapid solvent evaporation on application to have good maximum absorbance (Cₘₐₓ) and total absorbance (AUC).

In particular compositions of the invention, the glycol or glycerol ester of a C₈-₁₀ fatty acid in the carrier is present in an amount from about 50 to about 99.9% by weight of the composition, particularly, from about 79 to about 99.5%, from about 85 to about 99%.

Typically, the composition comprises from about 0.5% to about 10% w/v, preferably 1% of the compound of Formula (I), b) from about 10% to about 40% w/v, preferably 20% of glycerol monocaprylate; and c) optionally from about 2% to about 50% w/v, preferably 79% of propylene glycol dicaprylocaprate.

In accordance with this invention, it has been discovered that the compositions according to the invention generally show one or more beneficial technical features including desirable transdermal bioavailability.

It has been discovered that a single administration of such compositions generally provides potent activity against one or more ectoparasites, while also tending to provide fast onset of activity, long duration of activity, and/or desirable safety profiles toward the host animals and animal caretaker. Furthermore, the compositions are easy to administer to the animals and have s desirable transdermal bioavailability and spectrum of coverage. Preferably effective blood levels are obtained by the administration of the composition according to the invention.

An example of a species where this invention would be useful in the prevention of parasites is on cattle. Cattle skin is known to be especially difficult for transdermal administration and surprisingly this oily carrier of a compound of Formula (I) is highly effective.

The transdermal liquid preparation according to the invention has a desirable absolute systemic bioavailability to facilitate this excellent and long duration efficacy against ectoparasites.

It was surprisingly found that the topical compositions of the invention comprising a compound provide excellent efficacy against ticks. In some embodiments, the topical compositions of the invention comprising selected solvents and excipients result in effective levels of the active compound over a prolonged period of time.

Conventionally pour-on compositions that show systemic bioavailability require the presence of penetration enhancersin the composition (also called permeation enhancer, sorption promoters or accelerants) which penetrate into skin to reversibly decrease the barrier resistance. Numerous compounds have been evaluated for penetration enhancing activity, including sulphoxides (such as dimethylsulphoxide, DMSO), azones (e.g., laurocapram), pyrrolidones (for example 2-pyrrolidone), alcohols and alkanols (ethanol, or decanol), glycols (for example propylene glycol, PG, a common excipient in topically applied dosage forms), surfactants and terpene or fatty acids.

Therefore, it was unexpected to obtain effective blood levels after pour-on administration of the composition of the invention without the need to include any such permeation or penetration enhancer.

It has been observed that the addition of a permeation/penetration enhancer does not provide any significant advantage as to the effectiveness of the composition according to the invention.

Unexpectedly, the pharmaceutical composition resulted in both bioavailability after transdermal administration and therapeutic and prophylactic efficacy against an infestation of against artificially acquired burdens of cattle tick *Rhipicephalus microplus* ticks.

Such effective blood concentrations are already reached 2 days after administration and were further maintained for a prolonged period of time, especially more than 70 days, at least 11 weeks, or more than 2.5 months.

In another embodiment the composition exhibits long lasting efficacy of at least 90% against ticks, mites or lice for a period of at least 6 weeks.

One embodiment of the invention is a method wherein the composition is administered every month. In another embodiment of the invention the composition is administered every six weeks. In another embodiment of the invention the composition is administered every 2 months. In another embodiment of the invention the composition is administered every 3 months.

The compositions according to the invention are outstandingly suitable for use in parasite control or parasite prophylaxis. Thus, the pharmaceutical compositions described here serve for use in the treatment of or prophylaxis of parasite infestation, especially in the treatment of or prophylaxis of ectoparasites.

The compositions according to the invention are particularly suitable in the control of or prophylaxis of ectoparasites, preferably of ticks and/or mites, and/or flies or fly larvae, and/or lice, on non-human animals, particularly warm-blooded animals, preferably mammals. In one preferred embodiment the non-human animal is a ruminant e.g., animals such as cattle sheep and goats, especially cattle animal, such as a dairy cow, beef cattle or breeding stock.

According to a particularly preferred embodiment the present compositions are used in the control of and/or prophylaxis of ectoparasites of cattle such as infestation or re-infestation by ticks, mites, lice and/or biting flies. In a preferred embodiment the composition of the invention comprising compounds of Formula (I) is administered by pour- on administration to cattle animal to control ticks, especially *R. microplus.*

The pour-on composition according to the invention is a liquid and has a sufficient viscosity that prevents run-off from the animals' fur or skin by sufficient adherence to the fur/skin of the animal after administration, but can be easy administered, e.g., expelled the correct amount from the container at various temperatures.

The pour on composition dry within a reasonable period of time without impairment of the animal's appearance, is gentle on the animal's coat, has acceptable irritancy to the animal's skin and maintain its effectiveness on the animal through normal activities of the animal, such as exposure to sun and water.

The inventors have also found that compositions according to the current invention are also advantageously "rainfast", that is, the composition is effective in controlling parasites of an animal even when applied to a wet animal and is effective in controlling parasites even when the animal is exposed to rain shortly after topical application of the composition of the invention.

This means that the therapeutic efficacy for parasites is not affected by heavy rainfall (such as e.g., 25 mm) either before (20 minutes) or after (20 and 40 minutes) administration to the animal.

Consequently, in one aspect the current invention is directed to the veterinary pharmaceutical composition according to the current invention wherein the composition is rainfast by showing therapeutic efficacy for parasites is not affected by heavy rainfall either before or after treatment.

The volume of the topical composition as a spot-on or pour-on applied should be practical and shown to be safe and effective. Typically, the volume applied depends on the size and weight of the animal as well as the concentration of active. It is preferred to apply a volume that allows accurate dosing but does not result in run-off of the composition, especially if the animal moves.

For the pour-on form of the composition, the volume applied can be of the order of about 0.3 to about 100 ml. In other embodiments, volume applied of the pour-on compositions may be about 1 ml to about 100 ml or about 1 ml to about 50 ml. In still other embodiments, the volume may be about 5 ml to about 50 ml or about 10 ml to about 100 ml.

Preferably for cattle and sheep animals a volume of 10 ml or less per animal of the pour-on-on composition is administered. Generally, pour-on on compositions have a higher viscosity than compositions for spot-on applications and in one embodiment comprise a viscosity increasing component.

In one embodiment the application volume is 1 ml of the composition per 10 kg of the animal bodyweight. In another embodiment the application volume is 1 ml of the composition per 20 kg of the animal bodyweight.

The topical localized spot-on composition is applied as a low volume of about 0.01 to 1 ml per kg, preferably about 0.05 to 0.1 ml per kg, with a total volume from 0.3 to 100 ml per animal depending on the target species.

The pour-on or spot-on compositions according to the invention may be applied using any means known per se, e.g., using an applicator gun or a metering flask, pipette, syringes, roll on, droppers, capsules, foil packages, vials, twist tip containers, metered-dose aerosols or sprays and other single dose and multi-dose containers.

The compositions according to the invention can be prepared by customary methods.

### EXAMPLES OF CONTEMPLATED COMBINATION THERAPIES

The methods and pharmaceutical compositions of this invention encompass methods wherein a compound according to this invention is the sole active ingredient administered to the recipient animal. It is contemplated, however, that the methods and pharmaceutical compositions also encompass combination therapies wherein a compound is administered in combination with one or more other pharmaceutically acceptable active ingredients. The other active ingredient(s) may be, for example, one or more other compounds according to this invention or one or more other compounds corresponding to the use according to the invention. Alternatively (or additionally), the other active ingredient(s) may be one or more pharmaceutically acceptable compounds that are not compounds according to this invention or compounds corresponding to the use according to the invention. The other active ingredient(s) may target the same and/or different parasites and conditions.

Contemplated active ingredient(s) that may be administered in combination with the compounds include, for example, pharmaceutically acceptable anthelmintics, insecticides and acaricides, insect growth regulators, anti-inflammatories, anti-infectives, anti-protozoals, hormones, dermatological preparations (e.g., antiseptics and disinfectants), and immunobiologicals (e.g., vaccines and antisera) for disease prevention.

In one embodiment of the description, arylpyrazole compounds such as phenylpyrazoles may be included in the veterinary compositions of the description. Arylpyrazoles are known in the art and may be suitable for combination with the compound of Formula (I) in the compositions of the description. Examples of such arylpyrazole compounds include but are not limited to those described in U.S. Pat. Nos. 6,001,384; 6,010,710; 6,083,519; 6,096,329; 6,174, 540; 6,685,954, 6,998,131 and 7,759,381 (all of which are incorporated herein by reference). A particularly preferred arylpyrazole active agent is fipronil.

In another embodiment, the compositions of the description may advantageously include one or more isoxazoline compounds known in the art. Isoxazoline active agents are highly effective against a variety of ectoparasites and combination with the compound of Formula (I) would expand the scope of efficacy against these parasites.

Particularly useful isoxazoline active agents that can be combined with the compound include afoxolaner (including substantially pure active enantiomer), sarolaner, fluralaner (including substantially pure active enantiomer) and lotilaner.

These active agents are described in U.S. Pat. No. 7,964,204, US 2010/0254960 A1, US2011/0159107, US2012/0309620, US2012/0030841, US2010/0069247, WO 2007/125984, WO 2012/086462, U.S. Pat. Nos. 8,318, 757, 8,466,115, 8,618,126, 8,822,466, 8,383,659, 8,853,186, 9,221,835, US 2011/0144349, U.S. Pat. No. 8,053,452; US 2010/0137612, U.S. Pat. No. 8,410,153, US 2011/152081, WO 2012/089623, WO 2012/089622, U.S. Pat. Nos. 8,119, 671; 7,947,715; WO 2102/120135, WO 2012/107533, WO 2011/157748, US 2011/0245274, US 2011/0245239, US 2012/0232026, US 2012/0077765, US 2012/0035122, US 2011/0251247, WO 2011/154433, WO 2011/154434, US 2012/0238517, US 2011/0166193, WO 2011/104088, WO 2011/104087, WO 2011/104089, US 2012/015946, US 2009/0143410, WO 2007/123855 A2, US 2011/0118212, U.S. Pat. No. 7,951,828 & U.S. Pat. No. 7,662,972, US 2010/0137372 A1, US 2010/0179194 A2, US 2011/0086886 A2, US 2011/0059988 A1, US 2010/0179195 A1, US 2015/ 0126523, WO 2010/003923, WO 2010/003877, WO 2010/ 072602, WO 2014/134236, WO 2017/147352, U.S. Pat. Nos. 7,897,630, and 7,951,828, all of which are incorporated herein by reference in their entirety.

Another combination partner is 2-chloro-N-(1-cyanocyclopropyl)-5-[1-[2-methyl-5-(1,1,2,2,2-pentafluoroethyl)-4-(trifluoromethyl)pyrazol-3-yl]pyrazol-4-yl]benzamide (CAS RN 1621436). This compound is known as tigolaner.

In another embodiment, the compositions of the description may advantageously include one or more SLO- 1 modulator compounds known in the art. SLO-1 modulator active agents are highly effective against a variety of parasites and combination with the compound of Formula (I) would expand the scope of efficacy against these parasites. Particularly useful SLO-1 modulator active agents that can be combined with the compound include compounds disclosed in WO2017/178416, WO2018/087036, WO2018/197401, WO2019/002132, WO2019/025341, WO2019115768, WO2019/215182, WO2020/14068, WO2020/131629, WO2020/131631, and WO2020/083971 all of which are incorporated herein by reference in their entirety.

In another embodiment, the compositions of the description may advantageously include one or more SLO- 1 depsipeptide compounds known in the art. SLO-1 depsipeptide active agents are highly effective against a variety of parasites and combination with the compound of Formula (I) would expand the scope of efficacy against these parasites. Particularly useful SLO-1 depsipeptide active agents that can be combined with the compound include compounds disclosed in WO2019/108591, WO2019/217449, WO2016/187534, WO2017/116702, WO2018/093920, WO1996/011945, WO1995/007272, WO1994/019334, WO1993/019053, WO2014/089053, WO2018/187173, WO2013/187480, WO2015/093558, WO2012/077783, WO1997/002256, WO1997/011064, U.S. Pat. No 5624897, WO1997/020547, AU2002/226415, JP03207870, DE594082812 all of which are incorporated herein by reference in their entirety.

Therefore, this invention is also directed to the use as a medicament of combinations comprising a) one or more compounds of Formula (I) according to this invention or one with b) one or more pharmaceutically acceptable active compounds which differ in structure from component a).

The active compounds b) are preferably anthelmintic compounds, more preferably selected from the group consisting of SLO- 1 depsipeptide compounds and SLO -1 modulators as described above, avermectins (e.g., ivermectin, selamectin, doramectin, abamectin, and eprinomectin); milbemycins (moxidectin and milbemycin oxime); pro-benzimidazoles (e.g., febantel, netobimin, and thiophanate); benzimidazole derivatives, such as triclabendazole or a thiazole benzimidazole derivative (e.g., thiabendazole and cambendazole) or a carbamate benzimidazole derivatives (e.g., fenbendazole, albendazole (oxide), mebendazole, oxfendazole, parbendazole, oxibendazole, flubendazole); an imidazothiazoles (e.g., levamisole and tetramisole); a tetrahydropyrimidine (morantel and pyrantel), organophosphates (e.g., trichlorphon, haloxon, dichlorvos, and naphthalophos); salicylanilides (e.g., closantel, oxyclozanide, rafoxanide, and niclosamide); nitrophenolic compounds (e.g., nitroxynil and nitroscanate); benzenedisulphonamides (e.g., clorsulon); pyrazineisoquinolines (e.g., praziquantel and epsiprantel); heterocyclic compounds (e.g., piperazine, diethylcarbamazine, dichlorophen, and phenothiazine); arsenicals (e.g., thiacetarsamide, melorsamine, and arsenamide); cyclooctadepsipeptides (e.g., emodepside); paraherquamides ( e.g. derquantel); and amino-acetonitrile compounds (e.g. monepantel, AAD 1566); tribendimidine (amidine compound); amidine compounds (e.g., amidantel and tribendimidin), including all pharmaceutically acceptable forms, such as salts, solvates or N-oxides.

Preferred combinations are comprising a) one compound selected from the group of compounds according to formula (I) as defined elsewhere in the description, suitably compound 1 to 480 of the table (or enantiomers, salts, solvates, N-oxides or prodrugs thereof) and b) one compound selected from the group consisting of anthelmintic avermectins (e.g., ivermectin, selamectin, doramectin, abamectin, emamectin and eprinomectin); milbemycins (moxidectin and milbemycin oxime); pro-benzimidazoles (e.g., febantel, netobimin, and thiophanate); benzimidazole derivatives, such as thiazole benzimidazole derivatives (e.g., thiabendazole and cambendazole), carbamate benzimidazole derivatives (e.g., fenbendazole, albendazole (oxide), mebendazole, oxfendazole, parbendazole, oxibendazole, flubendazole, and triclabendazole); imidazothiazoles (e.g., levamisole and tetramisole); tetrahydropyrimidines (morantel and pyrantel), organophosphates (e.g., trichlorphon, haloxon, dichlorvos, and naphthalophos); salicylanilides (e.g., closantel, oxyclozanide, rafoxanide, and niclosamide); nitrophenolic compounds (e.g., nitroxynil and nitroscanate); benzenedisulphonamides (e.g., clorsulon); pyrazineisoquinolines (e.g., praziquantel and epsiprantel); heterocyclic compounds (e.g., piperazine, diethylcarbamazine, dichlorophen, and phenothiazine); arsenicals (e.g., thiacetarsamide, melorsamine, and arsenamide); cyclooctadepsipeptides (e.g., emodepside); paraherquamides (e.g. derquantel); amino-acetonitrile compounds (e.g. monepantel,

AAD 1566); tribendimidine (amidine compound); and amidantel (amidine compound); including all pharmaceutically acceptable forms, such as salts.

Preferred combinations comprise at least one compound selected from the group of compounds according to formula (I) as defined elsewhere in the description, suitably compound 1 to 480 of the table and
- abamectin, ivermectin, emamectin, eprinomectin, doramectin, moxidectin, milbemycin oxime; or
- closantel, oxyclozanide, rafoxanide, niclosamide; or
- nitroxynil, nitroscanate, clorsulon; or
- praziquantel, epsiprantel; or
- emodepside, derquantel, monepantel.

The compounds as described herein can be combined with pharmaceutically acceptable insecticides or acaricides. Such pharmaceutically acceptable insecticides and acaricides include, for example, acetamiprid, acetoprole, amitraz, amidoflumet, avermectin, azadirachtin, bifenthrin, bifenazate, buprofezin, bistrifluron, chlorfenapyr, chlorfluazuron, chlorantraniliprole, chlorpyrifos, chromafenozide, clothianidin, cyantraniliprole, cyflumetofen, β-cyfluthrin, cyhalothrin, λ-cyhalothrin, cymiazole cypermethrin, cyromazine, deltamethrin, demiditraz, diafenthiuron, diazinon, diflubenzuron, dimefluthrin, dinotefuran, emamectin, esfenvalerate, ethiprole, fenoxycarb, fenpropathrin, fenvalerate, fipronil, flonicamid, flubendiamide, flucythrinate, tau-fluvalinate, flufenoxuron, halofenozide, hexaflumuron, imidacloprid, indoxacarb, lufenuron, metaflumizone, methoprene, metofluthrin, methoxyfenozide, nitenpyram, novaluron, noviflumuron, permethrin,phosmet, profluthrin, protrifenbute, pymetrozine, pyrafluprole, pyrethrin, pyridalyl, pyrifluquinazon, pyriprole, pyriproxyfen, rotenone, ryanodine, spinetoram, spinosad, spirodiclofen, spiromesifen, spirotetramat, sulfoxaflor, tebufenozide, tebufenpyrad, teflubenzuron, tefluthrin, tetrachlorvinphos, tetramethylfluthrin, thiacloprid, thiamethoxam, tolfenpyrad, tralomethrin, isoxazolines as described above and triflumuron. General references discussing antiparasitic agents, such as insecticides and acaricides, include, for example, The Pesticide Manual, 13th Edition, C. D. S. Tomlin, Ed., British Crop Protection Council, Farnham, Surrey, U.K. (2003).

The compounds as described herein can be combined with pharmaceutically acceptable insect growth regulators. Such pharmaceutically acceptable insect growth regulators include, for example, methoprene, pyriproxyfen, tetrahydroazadirachtin, chlorfluazuron, cyromazine, diflubenzuron, fluazuron, flucycloxuron, flufenoxuron, hexaflumuron, lufenuron, ifenuron, tebufenozide, and triflumuron. These compounds tend to provide both initial and sustained treatment of parasite infestations at all stages of insect development, including eggs, on the animal subject, as well as within the environment of the animal subject.

The compounds as described herein can be combined with pharmaceutically acceptable anti-protozoals. Such pharmaceutically acceptable anti-protozoals include, for example, triazintriones like toltrazuril and ponazuril and triazindiones such as clazuril, diclazuril and letrazuril.

In some contemplated embodiments, the compounds are administered with pyridylmethylamine derivatives, such as, for example, pyridylmethylamine derivatives discussed in European Patent Appl. EP0539588 or Int'l Patent Appl. Publ. WO2007/115643.

In some contemplated embodiments, the compounds are administered with nodulisporic acids and derivatives thereof, such as, for example, compounds discussed in US Patent 5,399,582; 5,945,317; 5,962,499; 5,834,260; 6,221,894; or 5,595,991; or WO 1996/29073.

In some contemplated embodiments, the compounds are administered with dihydroazole compounds, such as, for example, compounds discussed in WO 2010/75591.

Other antiparasitic compounds contemplated to be useful in combination therapies with the compounds include, for example, imidazo[1,2-b] pyridazine compounds discussed in US Patent Appl. Publ. No. 2005-0182059; 1-(4-Mono and di-halomethylsulphonylphenyl)-2-acylamino-3-fluoropropanol compounds discussed US Patent 7,361,689; trifluoromethanesulfonanilide oxime ether compounds discussed in US Patent 7,312,248; n-[(phenyloxy)phenyl]-1,1,1-trifluoromethanesulfonamide and n-[(phenylsulfanyl)phenyl]-1,1,1-trifluoromethanesulfonamide compounds discussed in US Patent Appl. Publ. 2006-0281695; and 2-phenyl-3-(1H-pyrrol-2-yl)acrylonitrile compounds discussed in US Appl. Publ. 2006/0128779; isoxazoline compounds discussed in WO Patent Appl, Publ. 2005-085216, WO 2007/026965, WO 2007/070606, WO 2007/075459, WO 2007/079162, WO 2007/105814, WO 2007/125984, WO 2008/019760, WO 2008/122375, WO 2008/150393, WO 2009/002809, WO 2009/003075, WO 2009/022746, WO 2009/035004, WO 2009/045999, WO 2009/051956, WO 2009/035004.

In the contemplated combination therapies, the compounds according to this invention may be administered before, simultaneously, and/or after the other active ingredient(s). In addition, the compounds according to this invention may be administered in the same composition as the other active ingredient(s) and/or in separate compositions from the other active ingredient(s). Further, the compounds according to this invention and other active ingredient(s) may be administered via the same and/or different dosage route.

When the compounds according to this invention are administered in a combination therapy, the weight ratio of the active ingredients may vary widely. Factors influencing this ratio include, for example, the particular compounds; the identity of the other active ingredient(s) be administered in the combination therapy; the dosage route of the compounds and other active ingredient(s); the target condition and pathogen; the type (e.g., species and breed), age, size, sex, diet, activity, and condition of the animal; and pharmacological considerations, such as the activity, efficacy, pharmacokinetic, and toxicology profiles of the compounds and other active ingredient(s). In some contemplated embodiments, for example, the weight ratio of the compounds to the other active ingredient(s) is, for example, is from about 1:3000 to about 3000:1. In some such instances, the weight ratio is from about 1:300 to about 300:1. In other such instances, the weight ratio is from about 1:30 and about 30:1.

In addition to other active ingredients, it is contemplated that the compounds may be administered with one or more other compounds that beneficially affects (e.g., enhances or prolongs) the activity (or other characteristic, such as safety) of the compounds. For example, it is contemplated that the compounds may be administered with one or more synergists, such as, for example, piperonyl butoxide (PBO) and triphenyl phosphate (TPP).

This invention also is directed to kits that are, for example, suitable for use in performing the methods of treatment described above. The kit comprises a therapeutically effective amount of one or more compounds of this invention, and an additional component. The additional component(s) may be, for example, one or more of the following: another ingredient (e.g., an excipient or active ingredient), an apparatus for combining the compound of this invention with another ingredient and/or for administering the compound of this invention, or a diagnostic tool.

The compounds used according to this invention show an excellent activity in treating parasite infestations and in addition are acceptable for the animals treated.

The following table presents suitable compounds of the invention. In case racemate is indicated but only one of the stereoisomer is drawn, also the other stereoisomer is included.

The following compounds are very suitable for use in the method of the invention:

| # | Compound | Stereo |
|---|---|---|
| 4 | 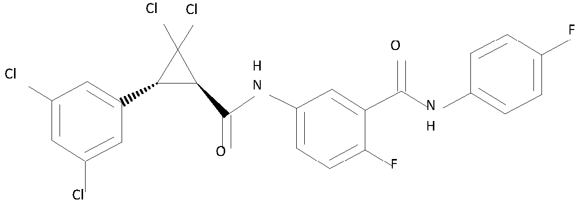 | racemate |
| 51 | 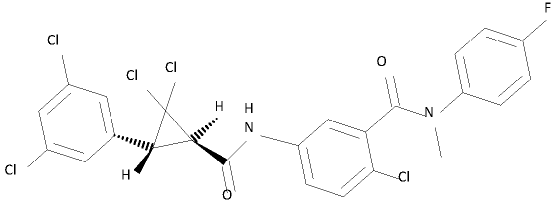 | 1R, 3R |
| 86 | 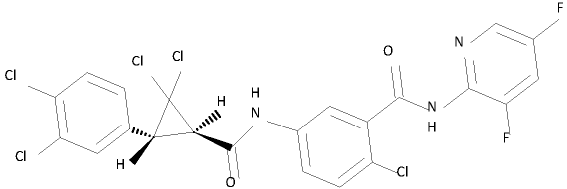 | 1R, 3R |
| 89 | 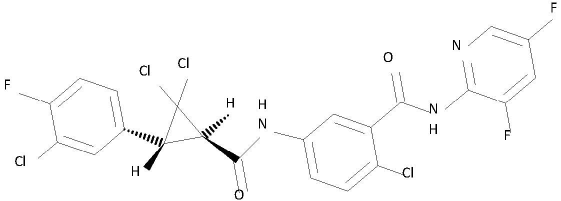 | 1R, 3R |
| 197 | 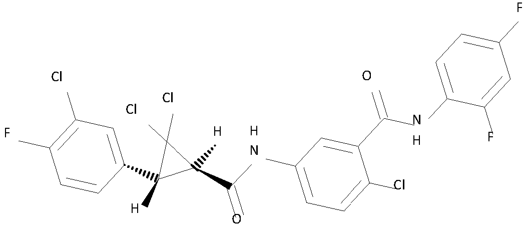 | 1R, 3R |
| 446 | 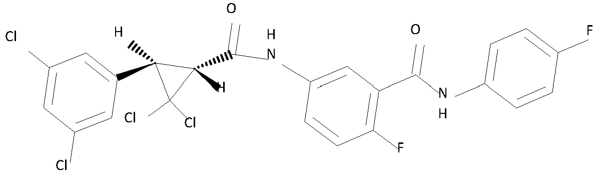 | 1R, 3R |
| 447 | 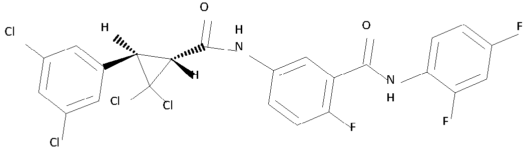 | 1R, 3R |
| 449 | 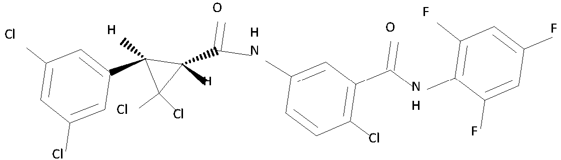 | 1R, 3R |
| 451 | 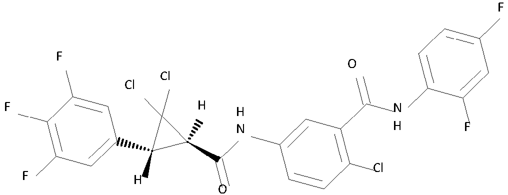 | 1R, 3R |
| 453 | 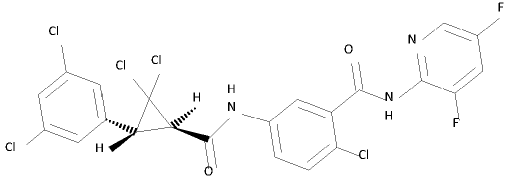 | 1R, 3R |
| 454 | 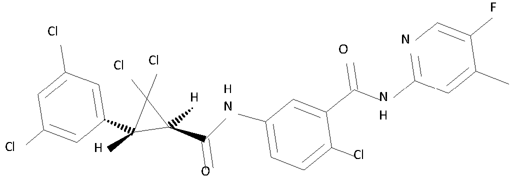 | 1R, 3R |
| 455 | 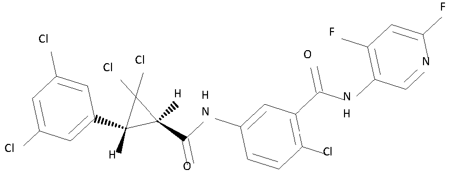 | 1R, 3R |
| 456 | 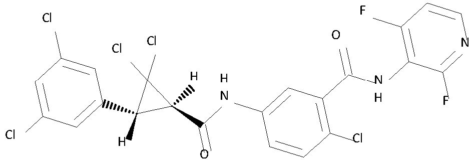 | 1R, 3R |
| 460 | 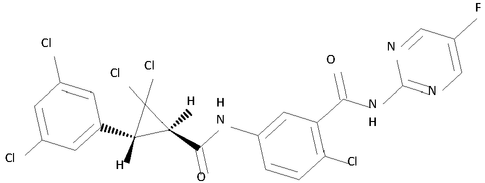 | 1R, 3R |
| 466 | 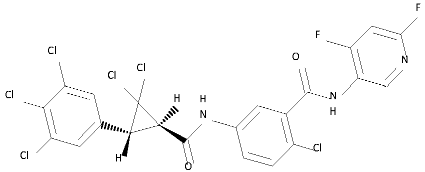 | 1R, 3R |

### EXAMPLES

The following compounds have been made.

Compounds were synthesized according to methods as described in WO2016168059 and WO2018073127

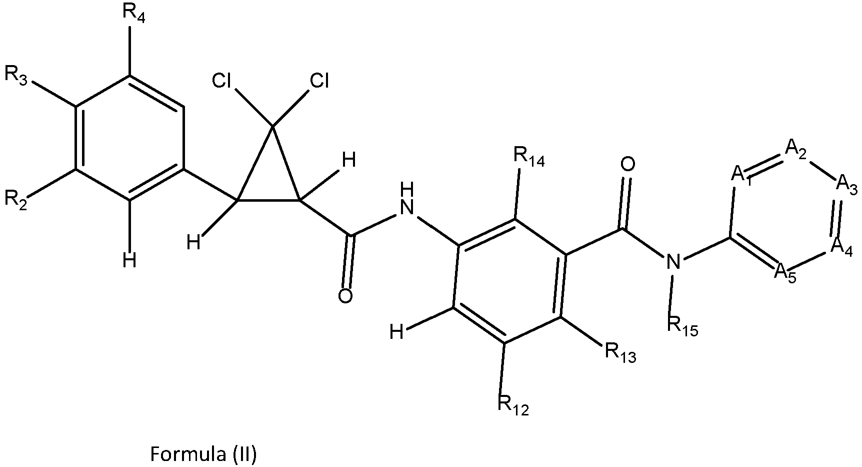

See table 1 on the following page

**Table I**

| **#** | **R₂** | **R₃** | **R₄** | **R₁₂** | **R₁₃** | **R₁₄** | **R₁₅** | **A₁** | **A₂** | **A₃** | **A₄** | **A₅** |
|---|---|---|---|---|---|---|---|---|---|---|---|---|
| 1 | Cl | H | Cl | H | Me | H | H | CH | CH | CF | CH | CH |
| 2 | Cl | H | Cl | H | Br | H | H | CH | CH | CF | CH | CH |
| 3 | H | Cl | Cl | H | Cl | H | H | CH | CH | CF | CH | CH |
| 4 | Cl | H | Cl | H | F | H | H | CH | CH | CF | CH | CH |
| 5 | Cl | H | Cl | H | H | F | H | CH | CH | CF | CH | CH |
| 6 | Cl | H | Cl | H | Cl | H | H | CF | CF | CH | CH | CH |
| 7 | Cl | H | Cl | H | Cl | H | H | CH | CF | CF | CH | CH |
| 8 | Cl | H | Cl | H | Cl | H | H | CF | CH | CF | CH | CF |
| 9 | Cl | H | Cl | H | Cl | H | H | CF | CH | CH | CH | CF |
| 10 | Cl | H | Cl | H | Cl | H | H | CCL | N | CH | CH | CH |
| 11 | Cl | H | Cl | H | Cl | H | H | CH | N | CCL | CH | CH |
| 12 | Cl | H | Cl | H | Cl | H | Me | CH | CF | CH | CH | CH |
| 13 | Cl | H | Cl | H | Cl | H | H | C-Me | CH | CF | CH | CH |
| 14 | Cl | Cl | Cl | H | Cl | H | H | CF | CH | CF | CH | CH |
| 15 | Cl | H | Cl | H | F | H | Me | CH | CF | CH | CH | CH |
| 16 | Cl | H | Cl | H | H | F | Me | CH | CH | CF | CH | CH |
| 17 | Cl | Cl | H | H | Cl | H | H | CH | CF | CH | CH | CH |
| 18 | CF₃ | H | H | H | Cl | H | Me | CH | CH | CF | CH | CH |
| 19 | Cl | H | Cl | H | F | H | H | CF | CH | CF | CH | CH |
| 20 | Cl | H | Cl | H | F | H | Me | CF | CH | CF | CH | CH |
| 21 | Cl | Cl | Cl | H | F | H | Me | CF | CH | CF | CH | CH |
| 22 | Cl | OCF₃ | H | H | Cl | H | H | CH | CH | CF | CH | CH |
| 23 | H | CF₃ | H | H | Cl | H | H | CH | CH | CF | CH | CH |
| 24 | CF₃ | H | Cl | H | Cl | H | H | CH | CH | CF | CH | CH |
| 25 | Cl | H | Cl | H | Cl | H | 2,2,2-Trifluoro-ethyl | CH | CH | CF | CH | CH |
| 26 | Cl | H | Cl | H | Cl | H | n-Propyl | CH | CH | CF | CH | CH |
| 27 | Me | H | Cl | H | Cl | H | H | CH | CH | CF | CH | CH |
| 28 | Me | H | Cl | H | Cl | H | Me | CH | CH | CF | CH | CH |
| 29 | Me | H | Cl | H | Cl | H | H | CF | CH | CH | CH | CF |
| 30 | Cl | Cl | Me | H | Cl | H | H | CH | CH | CF | CH | CH |
| 31 | H | Cl | F | H | Cl | H | H | CH | CH | CF | CH | CH |
| 32 | Br | H | Cl | H | Cl | H | H | CH | CH | CF | CH | CH |
| 33 | Br | H | Cl | H | Cl | H | Me | CH | CH | CF | CH | CH |
| 34 | Cl | H | Cl | H | Cl | H | H | CH | C-Me | CF | CH | CH |
| 35 | Cl | Cl | H | H | Cl | H | H | C-Me | CH | CF | CH | CH |
| 36 | Cl | Cl | Cl | H | Cl | H | H | C-Me | CH | CF | CH | CH |
| 37 | H | F | Cl | H | Cl | H | H | C-Me | CH | CF | CH | CH |
| 38 | Cl | H | Cl | H | Cl | H | Me | C-Me | CH | CF | CH | CH |
| 39 | H | F | Cl | H | Cl | H | Me | C-Me | CH | CF | CH | CH |
| 40 | Cl | H | Cl | H | Cl | H | H | CH | C-O-Me | CF | CH | CH |
| 41 | Cl | H | Cl | H | Cl | H | H | C-Me | C-Me | CF | CH | CH |
| 42 | Cl | H | Cl | H | Cl | H | H | CH | C-O-Me | CCL | CH | CH |
| 43 | Cl | H | Cl | H | Cl | H | H | CH | CF | C-Br | CH | CH |
| 44 | Cl | H | Cl | H | Cl | H | H | CH | C-Me | CCL | CH | CH |
| 45 | Cl | H | Cl | H | Cl | H | H | CF | CH | CH | CF | CH |
| 46 | Cl | H | Cl | H | Cl | H | H | CF | CH | CCL | CH | CH |
| 47 | Cl | H | Cl | H | Cl | H | H | CH | CCL | C-Me | CH | CH |
| 48 | Cl | H | Cl | H | Cl | H | H | CH | CH | CF | CH | CH |
| 49 | Cl | H | Cl | H | Cl | H | H | C-CN | CH | CF | CH | CH |
| 50 | Cl | H | Cl | H | Cl | H | H | CF | CF | CF | CH | CH |
| 51 | Cl | H | Cl | H | Cl | H | Me | CH | CH | CF | CH | CH |
| 52 | Cl | H | Cl | H | Cl | H | H | CH | CF | C-NH-Me | CH | CH |
| 53 | Cl | H | Cl | H | Cl | H | H | N | CH | CCL | CH | CH |
| 54 | Cl | OMe | Cl | H | Cl | H | H | CF | CH | CF | CH | CH |
| 55 | Cl | H | Cl | H | Cl | H | H | CF | CH | CF | CH | CH |
| 56 | Cl | H | Cl | H | H | H | H | CF | CH | CF | CH | CH |
| 57 | CN | H | Cl | H | Cl | H | H | CF | CH | CF | CH | CH |
| 58 | CN | H | Cl | H | Cl | H | Me | CH | CH | CF | CH | CH |
| 59 | H | Cl | Cl | H | Cl | H | H | C-CN | CH | CF | CH | CH |
| 60 | Cl | Cl | Cl | H | Cl | H | H | C-CN | CH | CF | CH | CH |
| 61 | H | F | Cl | H | Cl | H | H | C-CN | CH | CF | CH | CH |
| 62 | Cl | H | Cl | H | Cl | H | H | CF | CH | C-I | CH | CH |
| 63 | Cl | H | Cl | H | Cl | H | H | C-CN | CH | CF | CH | CH |
| 64 | Cl | Cl | H | H | Cl | H | H | CH | CH | CF | CH | CH |
| 65 | Cl | Cl | H | H | Cl | H | H | CF | CH | CF | CH | CH |
| 66 | Cl | H | Cl | H | Cl | H | H | CF | CH | C-NH-Me | CH | CH |
| 67 | Cl | H | Cl | H | Cl | H | H | CH | C-NH₂ | CF | CH | CH |
| 68 | Cl | H | Cl | H | Cl | H | H | N | CF | CH | CH | CH |
| 69 | Cl | H | Cl | H | Cl | H | H | N | CH | CF | CH | CF |
| 70 | Cl | H | Cl | H | Cl | H | H | N | CH | CF | CH | C-Me |
| 71 | Cl | H | Cl | H | Cl | H | Me | C-CN | CH | CF | CH | CH |
| 72 | Cl | Cl | Cl | H | Cl | H | Me | C-CN | CH | CF | CH | CH |
| 73 | Cl | H | Cl | H | Cl | H | H | CF | CH | C-CO-Me | CH | CH |
| 74 | Cl | Cl | Cl | H | H | H | H | C-CN | CH | CF | CH | CH |
| 75 | Cl | Cl | H | H | H | H | H | C-CN | CH | CF | CH | CH |
| 76 | H | Br | Cl | H | Cl | H | H | CH | CH | CF | CH | CH |
| 77 | Cl | H | Cl | H | H | H | Me | CH | CH | CF | CH | CH |
| 78 | Cl | H | Cl | H | H | H | H | CCL | CH | CF | CH | CH |
| 79 | Cl | Cl | Cl | H | Cl | H | H | CH | CH | CF | CH | CH |
| 80 | Cl | Cl | Cl | H | Cl | H | H | CF | CH | CF | CH | CH |
| 81 | Cl | H | Cl | H | Cl | H | H | N | CH | CF | C-Me | CH |
| 82 | Cl | H | Cl | H | Cl | H | H | C-CN | CH | C-Me | CH | CF |
| 83 | Cl | H | Cl | H | Cl | H | H | C-CN | CH | CCL | CH | CH |
| 84 | Cl | H | Cl | H | Cl | H | H | C-CN | CH | CH | C-Br | CH |
| 85 | CF₃ | H | CF₃ | H | Cl | H | H | C-CN | CH | CF | CH | CH |
| 86 | H | Cl | Cl | H | Cl | H | H | N | CH | CF | CH | CF |
| 87 | Cl | H | Cl | H | Cl | H | H | CF | C-NH₂ | CF | CH | CH |
| 88 | Cl | Cl | Cl | H | Cl | H | H | CF | C-NH₂ | CF | CH | CH |
| 89 | H | F | Cl | H | Cl | H | H | N | CH | CF | CH | CF |
| 90 | Cl | F | H | H | Cl | H | H | CF | C-NH₂ | CF | CH | CH |
| 91 | H | F | Cl | H | Cl | H | H | CF | CH | CF | CH | CH |
| 92 | H | Br | Cl | H | Cl | H | H | C-CN | CH | CF | CH | CH |
| 93 | H | Cl | Br | H | Cl | H | H | CH | CH | CF | CH | CH |
| 94 | CF₃ | H | H | H | Cl | H | H | C-CN | CH | CF | CH | CH |
| 95 | CF₃ | H | H | H | Cl | H | H | CF | C-NH₂ | CF | CH | CH |
| 96 | CN | H | Cl | H | Cl | H | H | N | CH | CF | CH | CF |
| 97 | CN | H | Cl | H | Cl | H | H | CF | C-NH₂ | CF | CH | CH |
| 98 | Cl | H | Cl | H | Cl | H | H | N | CH | CF | C-NH₂ | CF |
| 99 | H | F | F | H | Cl | H | H | CF | CH | CF | CH | CH |
| 100 | Br | Br | H | H | Cl | H | H | CF | CH | CF | CH | CH |
| 101 | F | CF₃ | H | H | Cl | H | H | CF | CH | CF | CH | CH |
| 102 | Cl | H | Cl | H | Cl | H | H | C-NO₂ | CH | CF | CH | CH |
| 103 | Cl | H | Cl | H | Cl | H | H | CF | CH | CF | C-NH₂ | CH |
| 104 | Cl | H | Cl | H | Cl | H | H | CF | CH | C-NH-CH=CH-C | | CH |
| 105 | F | Br | H | H | Cl | H | H | CF | CH | CF | CH | CH |
| 106 | Br | CF₃ | H | H | Cl | H | H | CF | CH | CF | CH | CH |
| 107 | CF₃ | Br | H | H | Cl | H | H | CF | CH | CF | CH | CH |
| 108 | Cl | CF₃ | H | H | Cl | H | H | CF | CH | CF | CH | CH |
| 109 | Cl | H | Cl | H | Cl | H | H | CH | CCL | N | CH | CH |
| 110 | Cl | Cl | H | H | Cl | H | H | CH | CCL | N | CH | CH |
| 111 | Cl | H | Cl | H | Cl | H | H | N | CH | CF | C-NH₂ | CF |
| 112 | Cl | Cl | H | H | Cl | H | H | N | CH | CF | C-NH₂ | CF |
| 113 | CF₃ | Cl | H | H | Cl | H | H | CF | CH | CF | CH | CH |
| 114 | Br | F | H | H | Cl | H | H | CF | CH | CF | CH | CH |
| 115 | CF₃ | F | H | H | Cl | H | H | CF | CH | CF | CH | CH |
| 116 | Cl | H | Br | H | Cl | H | H | C-CN | CH | CF | CH | CH |
| 117 | Cl | H | Cl | H | Cl | H | H | CH | CF | C-NH₂ | CF | CH |
| 118 | Cl | Cl | Cl | H | Cl | H | H | CH | CF | C-NH₂ | CF | CH |
| 119 | Cl | F | H | H | Cl | H | H | CH | CF | C-NH₂ | CF | CH |
| 120 | H | 1-Fluorovinyl | Cl | H | Cl | H | H | CF | CH | CF | CH | CH |
| 121 | H | Cl | 1-fluorovinyl | H | Cl | H | H | CH | CH | CF | CH | CH |
| 122 | Cl | H | 1-fluorovinyl | H | Cl | H | H | CF | CH | CF | CH | CH |
| 123 | Cl | H | 1-fluorovinyl | H | Cl | H | H | C-CN | CH | CF | CH | CH |
| 124 | Cl | H | Cl | H | Cl | H | H | N | CH | CF | C-I | CF |
| 125 | H | Vinyl | F | H | Cl | H | H | CF | CH | CF | CH | CH |
| 126 | H | F | Vinyl | H | Cl | H | H | CF | CH | CF | CH | CH |
| 127 | H | Vinyl | CF₃ | H | Cl | H | H | CF | CH | CF | CH | CH |
| 128 | H | CF₃ | Vinyl | H | Cl | H | H | CF | CH | CF | CH | CH |
| 129 | Cl | H | Cl | H | Cl | H | H | N | CH | CF | C-CH=CH₂ | CF |
| 130 | Cl | H | Cl | H | Cl | H | H | N | CH | CF | C-CFCH₂ | CF |
| 131 | Cl | H | Cl | H | Cl | H | H | CH | N | CF | C-Br | CH |
| 132 | Cl | H | Cl | H | Cl | H | H | CH | N | CF | C-Me | CH |
| 133 | Cl | H | Cl | H | Cl | H | H | N | CF | C-CH=CH₂ | CH | CH |
| 134 | Cl | H | Cl | H | Cl | H | H | N | CF | C-CF=CH₂ | CH | CH |
| 135 | Cl | H | Cl | H | Cl | H | H | CH | N | CF | C-CH=CH₂ | CH |
| 136 | H | Cl | Br | H | Cl | H | H | CF | CH | CF | CH | CH |
| 137 | H | Br | Cl | H | Cl | H | H | CF | C-NH₂ | CF | CH | CH |
| 138 | H | Cl | Br | H | Cl | H | H | CF | C-NH₂ | CF | CH | CH |
| 139 | H | Ethynyl | Cl | H | Cl | H | H | C-CN | CH | CF | CH | CH |
| 140 | Cl | Cl | Cl | H | Cl | H | H | CF | CH | CF | C-NH₂ | CH |
| 141 | Cl | Cl | Cl | H | Cl | H | H | C-NH₂ | CF | CH | CF | CH |
| 142 | Cl | H | Cl | H | Cl | H | H | C-NH₂ | CH | CF | CH | CF |
| 143 | Cl | Cl | H | H | Cl | H | H | C-NH₂ | CH | CF | CH | CF |
| 144 | H | Cl | Ethynyl | H | Cl | H | H | CH | CH | CF | CH | CH |
| 145 | F | Cl | H | H | Cl | H | H | CF | CH | CF | CH | CH |
| 146 | F | H | F | H | Cl | H | H | CF | CH | CF | CH | CH |
| 147 | F | H | CF₃ | H | Cl | H | H | CF | CH | CF | CH | CH |
| 148 | Cl | H | Cl | H | Cl | H | prop-2-ynyl | CH | CH | CF | CH | CH |
| 149 | Cl | F | H | H | Cl | H | prop-2-ynyl | CF | CH | CF | CH | CH |
| 150 | Cl | F | H | H | Cl | H | H | C-NH₂ | CF | CH | CF | CH |
| 151 | F | H | Br | H | Cl | H | H | CF | CH | CF | CH | CH |
| 152 | Br | H | CF₃ | H | Cl | H | H | CF | CH | CF | CH | CH |
| 153 | Cl | Cl | H | H | Cl | H | H | CF | CH | CH | C-NH₂ | CH |
| 154 | Cl | F | H | H | Cl | H | H | CF | CH | CH | C-NH₂ | CH |
| 155 | Cl | Cl | H | H | Cl | H | H | CH | C-NH₂ | CH | CF | CH |
| 156 | Cl | F | H | H | Cl | H | H | CH | C-NH₂ | CH | CF | CH |
| 157 | F | OMe | F | H | Cl | H | H | CF | CH | CF | CH | CH |
| 158 | F | Br | F | H | Cl | H | H | CH | CH | CF | CH | CH |
| 159 | F | Br | OMe | H | Cl | H | H | CH | CH | CF | CH | CH |
| 160 | F | Br | F | H | Cl | H | H | CF | CH | CF | CH | CH |
| 161 | F | Br | OMe | H | Cl | H | H | CF | CH | CF | CH | CH |
| 162 | Br | OMe | F | H | Cl | H | H | CF | CH | CF | CH | CH |
| 163 | H | F | Cl | H | Cl | H | H | CCL | C-O- Me | N | CH | CH |
| 164 | H | F | Cl | H | Cl | H | H | C-Br | CH | N | C-O- Me | CH |
| 165 | Cl | H | Cl | H | Cl | H | H | CCL | N | C-O- Me | CH | CH |
| 166 | H | F | Cl | H | Cl | H | H | CCL | N | C-O- Me | CH | CH |
| 167 | Cl | H | Cl | H | Cl | H | H | C-NO₂ | CH | CF | CH | CH |
| 168 | Cl | Cl | H | H | Cl | H | H | C-NO₂ | CH | CF | CH | CH |
| 169 | Cl | Cl | Cl | H | Cl | H | H | C-NO₂ | CH | CF | CH | CH |
| 170 | Cl | F | H | H | Cl | H | H | C-NO₂ | CH | CF | CH | CH |
| 171 | CHF₂ | Cl | H | H | Cl | H | H | CF | CH | CF | CH | CH |
| 172 | CHF₂ | H | F | H | Cl | H | H | CF | CH | CF | CH | CH |
| 173 | CHF₂ | F | H | H | Cl | H | H | CF | CH | CF | CH | CH |
| 174 | F | CHF₂ | H | H | Cl | H | H | CF | CH | CF | CH | CH |
| 175 | Cl | H | Cl | H | Cl | H | H | CF | CH | C-NH₂ | CF | CH |
| 176 | Cl | Cl | H | H | Cl | H | H | CF | CH | C-NH₂ | CF | CH |
| 177 | Cl | Cl | Cl | H | Cl | H | H | CF | CH | C-NH₂ | CF | CH |
| 178 | Cl | F | H | H | Cl | H | H | CF | CH | C-NH₂ | CF | CH |
| 179 | Br | Cl | H | H | Cl | H | H | CF | CH | CF | CH | CH |
| 180 | Cl | H | Cl | H | Cl | H | H | C-NH₂ | CH | CF | CH | CH |
| 181 | Cl | Cl | Cl | H | Cl | H | H | C-NH₂ | CH | CF | CH | CH |
| 182 | Cl | F | H | H | Cl | H | H | C-NH₂ | CH | CF | CH | CH |
| 183 | Br | Cl | H | H | Cl | H | H | CF | C-NH₂ | CF | CH | CH |
| 184 | Br | Cl | H | H | Cl | H | H | CF | CH | CF | C-NH₂ | CH |
| 185 | F | F | F | H | Cl | H | H | CF | CH | CF | CH | CH |
| 186 | Cl | H | Cl | H | Cl | H | H | CH | C-Br | CF | CF | CH |
| 187 | CHF₂ | H | Cl | H | Cl | H | H | N | CH | CF | CH | CF |
| 188 | CHF₂ | Cl | H | H | Cl | H | H | N | CH | CF | CH | CF |
| 189 | CHF₂ | H | F | H | Cl | H | H | N | CH | CF | CH | CF |
| 190 | CHF₂ | F | H | H | Cl | H | H | N | CH | CF | CH | CF |
| 191 | F | CHF₂ | H | H | Cl | H | H | N | CH | CF | CH | CF |
| 192 | H | CHF₂ | H | H | Cl | H | H | N | CH | CF | CH | CF |
| 193 | CHF₂ | H | Cl | H | Cl | H | Me | CH | CH | CF | CH | CH |
| 194 | CHF₂ | Cl | H | H | Cl | H | Me | CH | CH | CF | CH | CH |
| 195 | CHF₂ | F | H | H | Cl | H | Me | CH | CH | CF | CH | CH |
| 196 | F | CHF₂ | H | H | Cl | H | Me | CH | CH | CF | CH | CH |
| 197 | Cl | F | H | H | Cl | H | H | CF | CH | CF | CH | CH |
| 198 | H | Cl | F | H | Cl | H | H | CF | CH | CH | CH | CF |
| 199 | Br | H | Cl | H | Cl | H | H | CF | C-NH₂ | CF | CH | CH |
| 200 | Cl | Cl | H | H | Cl | H | H | CF | CH | CF | C-NH₂ | CH |
| 201 | Cl | Cl | H | H | Cl | H | H | C-NH₂ | CF | CH | CF | CH |
| 202 | Cl | Cl | H | H | Cl | H | H | CH | CF | C-NH₂ | CF | CH |
| 203 | Cl | F | H | H | Cl | H | H | CF | CH | CF | C-NH₂ | CH |
| 204 | Cl | H | Cl | H | Cl | H | H | CH | C-NH₂ | CH | CF | CH |
| 205 | Cl | H | Cl | H | Cl | H | H | CF | C-NH₂ | CH | CH | CH |
| 206 | Cl | Cl | H | H | Cl | H | H | C-NH₂ | CH | CF | CH | CH |
| 207 | Cl | H | Cl | H | Cl | H | H | CF | C-NH₂ | CF | CH | CH |
| 208 | CF₃ | F | H | F | Cl | H | H | CF | C-NH-CO-CF₃ | CF | CH | CH |
| 209 | Cl | F | H | Me | F | H | H | CF | C-NH₂ | CF | CH | CH |
| 210 | Cl | Cl | Cl | H | Cl | H | H | CF | C-NH₂ | CH | CH | CF |
| 211 | Cl | H | Cl | H | Cl | H | H | C-Br | CH | CF | CH | CF |
| 212 | Cl | F | H | F | F | H | H | CH | CH | CF | CH | CH |
| 213 | H | Cl | Cl | H | Cl | H | H | CF | C-NH-CO-CH₂-O-Me | CF | CH | CH |
| 214 | Cl | Cl | Cl | F | Cl | H | H | CF | C-NH-CO-CHF₂ | CF | CH | CH |
| 215 | F | H | CF₃ | H | Cl | H | H | CF | C-NH2 | CF | CH | CH |
| 216 | Cl | F | H | H | Cl | H | H | CF | CH | CF | CH | CH |
| 217 | Cl | H | Cl | H | Cl | H | H | CF | CH | CF | C-Br | CH |
| 218 | Cl | H | Cl | H | Cl | H | H | CF | C-NH-CO-CHF₂ | CH | CH | CF |
| 219 | Cl | H | Cl | H | Cl | H | Et | C-CN | CH | CF | CH | CH |
| 220 | Cl | Cl | Cl | H | Cl | H | H | CF | C-NH-CO-CHF₂ | CF | CH | CH |
| 221 | Cl | F | H | F | Cl | H | H | CF | C-NH2 | CF | CH | CH |
| 222 | Cl | F | H | Me | Cl | H | H | CH | CH | CF | CH | CH |
| 223 | H | Cl | CF₃ | H | Cl | H | H | CF | C-NH2 | CF | CH | CH |
| 224 | Cl | Cl | H | H | Cl | H | H | CF | CH | CF | C-NH-CO-CF₃ | CH |
| 225 | H | I | H | H | Cl | H | H | CF | CH | CF | CH | CH |
| 226 | F | Cl | Cl | H | Cl | H | H | CF | C-NH₂ | CF | CH | CH |
| 227 | Cl | F | H | F | F | H | H | CF | CH | CF | CH | CH |
| 228 | Cl | H | Cl | H | Cl | H | H | CF | C-NH-CO-CHF₂ | CF | CH | CH |
| 229 | CF₃ | F | H | F | Cl | H | H | CF | C-NH-CO-CHF₂ | CF | CH | CH |
| 230 | Cl | F | H | Me | Cl | H | H | CF | CH | CF | CH | CH |
| 231 | Cl | F | H | Cl | Me | H | H | CH | CH | CF | CH | CH |
| 232 | Cl | F | H | Cl | Me | H | H | CF | C-NH₂ | CF | CH | CH |
| 233 | SF₅ | H | Cl | H | Cl | H | H | CF | CH | CF | CH | CH |
| 234 | Cl | F | H | CF₃ | Cl | H | H | CH | CH | CF | CH | CH |
| 235 | Br | H | SF₅ | H | Cl | H | H | CF | C-NH₂ | CF | CH | CH |
| 236 | Cl | H | Cl | F | Cl | H | H | CF | C-NH-CO-CHF₂ | CF | CH | CH |
| 237 | Cl | Cl | H | F | Cl | H | H | CF | C-NH-CO-CF₃ | CF | CH | CH |
| 238 | Cl | Cl | Cl | H | Cl | H | H | CF | C-NH-CO-CHF₂ | CH | CH | CF |
| 239 | Cl | H | Cl | H | Cl | H | H | CCL | C-NH-CO-Me | CF | CH | CH |
| 240 | Cl | F | H | Cl | Me | H | H | CF | CH | CF | CH | CH |
| 241 | Cl | Cl | H | H | Cl | H | H | CF | C-NH-CO-Phenyl | CF | CH | CH |
| 242 | Cl | H | Cl | H | Cl | H | H | CF | C-CO-O-Me | CF | CH | CH |
| 243 | CF₃ | F | H | H | Cl | H | Me | CF | C-NH₂ | CF | CH | CH |
| 244 | I | H | H | H | Cl | H | H | CF | CH | CF | CH | CH |
| 245 | I | H | H | H | Cl | H | H | CF | C-NH₂ | CF | CH | CH |
| 246 | CF₃ | H | Br | H | Cl | H | H | CF | C-NH₂ | CF | CH | CH |
| 247 | Cl | F | H | H | Cl | H | H | CF | C-NH-CO-CHF₂ | CH | CH | CF |
| 248 | Cl | F | Cl | H | Cl | H | H | CF | CH | CF | CH | CH |
| 249 | Cl | Cl | H | H | Cl | H | H | CF | C-NMe-CO-CHF₂ | CF | CH | CH |
| 250 | Cl | F | Cl | H | Cl | H | H | CF | C-NH2 | CF | CH | CH |
| 251 | F | Cl | CF₃ | H | Cl | H | H | CF | CH | CF | CH | CH |
| 252 | Cl | F | H | H | Cl | H | H | CF | C-NH-CO-CHF₂ | CF | CH | CH |
| 253 | CF₃ | F | H | H | Cl | H | prop-2-ynyl | CF | C-NH₂ | CF | CH | CH |
| 254 | OCHF₂ | H | Cl | H | Cl | H | H | CF | CH | CF | CH | CH |
| 255 | Cl | H | Cl | H | Cl | H | H | CF | C-Br | CF | CH | CH |
| 256 | Cl | H | Cl | F | Cl | H | H | CF | C-NH-CO-CF₃ | CF | CH | CH |
| 257 | SF₅ | H | Cl | H | Cl | H | H | CF | C-NH₂ | CF | CH | CH |
| 258 | Cl | Cl | H | F | Cl | H | H | CF | C-NH-CO-CHF₂ | CF | CH | CH |
| 259 | Cl | H | Cl | H | Cl | H | H | CF | CH | C-NH₂ | CH | CF |
| 260 | Cl | F | H | H | Cl | H | H | CF | C-NH₂ | CH | CH | CF |
| 261 | Cl | Cl | CF₃ | H | Cl | H | H | CF | C-NH₂ | CF | CH | CH |
| 262 | H | F | CF₃ | H | Cl | H | H | CF | C-NH-CO-CBrF₂ | CF | CH | CH |
| 263 | Cl | Cl | Br | H | Cl | H | H | CF | CH | CF | CH | CH |
| 264 | Cl | F | H | Me | F | H | H | CF | CH | CF | CH | CH |
| 265 | Cl | F | H | F | Cl | H | H | CH | CH | CF | CH | CH |
| 266 | Cl | Cl | CF₃ | H | Cl | H | H | CF | CH | CF | CH | CH |
| 267 | H | F | CF₃ | H | Cl | H | H | CF | C-NH-CO-CClF₂ | CF | CH | CH |
| 268 | Cl | Cl | H | H | Cl | H | H | CF | CH | C-NH-CO-CF₃ | CH | CF |
| 269 | Cl | F | H | H | Cl | H | H | CF | CH | C-NH₂ | CH | CF |
| 270 | SF₅ | H | H | H | Cl | H | H | CF | CH | CF | CH | CH |
| 271 | H | I | H | H | Cl | H | H | CF | C-NH₂ | CF | CH | CH |
| 272 | CHF₂ | H | Cl | H | Cl | H | H | CF | C-NH₂ | CF | CH | CH |
| 273 | F | Cl | CF₃ | H | Cl | H | H | CF | C-NH2 | CF | CH | CH |
| 274 | Cl | F | H | F | Cl | H | H | CF | C-NH-CO-CF₃ | CF | CH | CH |
| 275 | Cl | Cl | H | H | Cl | H | H | CF | C-NH₂ | CH | CH | CF |
| 276 | Cl | H | Cl | H | Cl | H | H | C-Br | CH | CF | CH | CH |
| 277 | SF₅ | H | H | H | Cl | H | H | CF | C-NH₂ | CF | CH | CH |
| 278 | H | F | CF₃ | H | Cl | H | H | CF | C-NH₂ | CF | CH | CH |
| 279 | Cl | Cl | H | H | Cl | H | H | CF | CH | CF | C-NH-CO-CCIF₂ | CH |
| 280 | H | Br | CF₃ | H | Cl | H | H | CF | C-NH₂ | CF | CH | CH |
| 281 | CHF₂ | F | H | H | Cl | H | H | CF | C-NH₂ | CF | CH | CH |
| 282 | F | F | F | H | Cl | H | H | CF | C-NH₂ | CF | CH | CH |
| 283 | F | Cl | Cl | H | Cl | H | H | CF | CH | CF | CH | CH |
| 284 | H | CF₃ | F | H | Cl | H | H | CF | C-NH-CO-CF₃ | CF | CH | CH |
| 285 | Cl | Cl | H | H | Cl | H | H | CF | C-NH-CO-CCl₃ | CF | CH | CH |
| 286 | Cl | H | Cl | H | Cl | H | H | CF | C-O-Me | CF | CH | CH |
| 287 | Cl | H | CF₃ | H | Cl | H | H | CF | CH | CF | CH | CH |
| 288 | Cl | F | H | Me | Cl | H | H | CF | C-NH₂ | CF | CH | CH |
| 289 | Cl | F | H | H | F | F | H | CH | CH | CF | CH | CH |
| 290 | Cl | Cl | H | H | Cl | H | H | C-NH-CO-CHF₂ | CH | CF | CH | CH |
| 291 | Cl | Cl | H | H | Cl | H | H | CF | C-NH-CO-CCIF₂ | CF | CH | CH |
| 292 | F | F | F | H | Cl | H | H | CF | CH | CF | CH | CH |
| 293 | Cl | H | Cl | H | Cl | H | H | CH | C-Br | CF | CH | CH |
| 294 | H | H | CF₃ | H | Cl | H | H | CF | CH | CF | CH | CH |
| 295 | Cl | Cl | H | H | Cl | H | H | CF | C-NH-CO-CHCl₂ | CF | CH | CH |
| 296 | Cl | H | Cl | H | Cl | H | H | CF | CH | CF | C-CH=CH₂ | CH |
| 297 | Cl | H | Cl | H | Cl | H | H | C-CF=CH₂ | CH | CF | CH | CF |
| 298 | Cl | H | Cl | H | Cl | H | H | CF | CH | C-CH=CH₂ | CH | CH |
| 299 | Cl | H | Cl | H | Cl | H | H | CF | C-CH=CH₂ | CF | CH | CH |
| 300 | Cl | H | Cl | H | Cl | H | H | C-CH=CH₂ | CH | CF | CH | CH |
| 301 | H | F | CF₃ | H | Cl | H | H | CF | C-NH-CO-CHCl₂ | CF | CH | CH |
| 302 | Me | F | CF₃ | H | Cl | H | H | CF | CH | CF | CH | CH |
| 303 | Me | F | CF₃ | H | Cl | H | H | CF | C-NH₂ | CF | CH | CH |
| 304 | CHF₂ | Cl | H | H | Cl | H | H | CF | C-NH₂ | CF | CH | CH |
| 305 | H | F | CF₃ | H | Cl | H | H | CF | C-NH₂ | CF | CH | CH |
| 306 | Cl | Br | Cl | H | Cl | H | H | CF | CH | CF | CH | CH |
| 307 | Cl | Cl | Br | H | Cl | H | H | CH | CH | CF | CH | CH |
| 308 | Cl | Cl | H | H | Cl | H | Me | CF | C-NH₂ | CF | CH | CH |
| 309 | H | F | Br | H | Cl | H | H | CF | C-NH₂ | CF | CH | CH |
| 310 | Cl | F | H | F | Cl | H | H | CF | CH | CF | CH | CH |
| 311 | F | H | Br | H | Cl | H | H | CF | C-NH₂ | CF | CH | CH |
| 312 | Cl | Cl | Br | H | Cl | H | H | CF | C-NH₂ | CF | CH | CH |
| 313 | Cl | F | H | Me | F | H | H | CH | CH | CF | CH | CH |
| 314 | Cl | H | Cl | H | Cl | H | H | CF | CH | C-Br | CH | CH |
| 315 | Cl | F | H | F | Me | H | H | CH | CH | CF | CH | CH |
| 316 | Cl | H | Cl | H | Cl | H | H | CF | CH | C-CF=CH₂ | CH | CH |
| 317 | Cl | H | Cl | H | Cl | H | H | CH | C-Br | CF | CF | CH |
| 318 | Cl | H | Cl | H | Cl | H | H | CF | CH | CF | CH | C-CH=CH₂ |
| 319 | Cl | Cl | Cl | F | Cl | H | H | CF | C-NH-CO-CF₃ | CF | CH | CH |
| 320 | Cl | Cl | H | H | Cl | H | H | CF | C-NH-CO-CF2-Me | CF | CH | CH |
| 321 | Cl | F | H | Cl | F | H | H | CH | CH | CF | CH | CH |
| 322 | F | F | Br | H | Cl | H | H | CF | C-NH₂ | CF | CH | CH |
| 323 | Cl | Cl | H | H | Cl | H | H | CF | C-NH-CO-CHF₂ | CH | CH | CF |
| 324 | Cl | H | Cl | H | Cl | H | H | C-CF=CH₂ | CH | CF | CH | CH |
| 325 | Cl | F | H | H | Cl | H | H | CF | C-NH-CO-CH2-O-CF3 | CF | CH | CH |
| 326 | CF₃ | Cl | H | H | Cl | H | H | CF | C-NH-CO-CF₃ | CF | CH | CH |
| 327 | Cl | Cl | Cl | H | Cl | H | H | CF | CH | CF | C-NH-CO-CF₃ | CH |
| 328 | Cl | H | Cl | F | Cl | H | H | CF | CH | CF | C-NH-CO-CHF₂ | CH |
| 329 | H | F | Cl | H | Cl | H | H | CH | C-NH2 | CCL | CH | CH |
| 330 | H | Cl | Cl | Me | Cl | H | H | CF | C-NH-CO-CHF₂ | CF | CH | CH |
| 331 | Cl | F | H | H | Cl | H | H | CF | C-NMe-CO-CHF₂ | CF | CH | CH |
| 332 | Cl | Cl | H | H | Cl | H | H | CF | C-NH-CO-CH₂-O-CF₃ | CF | CH | CH |
| 333 | CF₃ | F | H | H | F | H | H | CF | C-NH-CO-CF3 | CF | CH | CH |
| 334 | CF₃ | F | H | H | Cl | H | H | CF | CH | CF | C-NH-CO-CF₂-CHF₂ | CH |
| 335 | F | H | CF₃ | H | Cl | H | H | CF | C-NH2 | CF | CH | CH |
| 336 | Cl | Cl | Cl | H | Cl | H | H | CF | C-NMe-CO-CHF₂ | CF | CH | CH |
| 337 | CF₃ | F | H | F | F | H | H | CF | C-NH-CO-CF₃ | CF | CH | CH |
| 338 | H | F | CF₃ | H | Cl | H | H | CF | C-Me | CF | C-NH₂ | CH |
| 339 | CF₃ | F | H | H | F | H | H | CF | C-NH-CO-CHF₂ | CF | CH | CH |
| 340 | Br | Cl | H | H | Cl | H | H | CF | C-NH-CO-CHF₂ | CF | CH | CH |
| 341 | Cl | Cl | H | H | Cl | H | Me | CF | C-NH-CO-CH2-O-CF₃ | CF | CH | CH |
| 342 | Cl | H | Cl | H | Cl | H | H | CF | C-NMe-CO-CHF₂ | CF | CH | CH |
| 343 | Cl | Cl | H | H | F | H | H | CF | CH | CF | C-NH-CO-CF₃ | CH |
| 344 | CF₃ | F | H | F | Cl | H | H | CF | CH | CF | C-NH-CO-CF₃ | CH |
| 345 | Cl | Cl | H | H | F | H | H | CF | C-NH-CO-CHF₂ | CF | CH | CH |
| 346 | Cl | Cl | H | H | Cl | H | H | CF | C-NH-CO-CH(Me)-O-C₂H₅ | CF | CH | CH |
| 347 | Cl | F | H | H | Cl | H | H | CH | C-NH-CO-CF₃ | CF | CH | CH |
| 348 | Cl | Cl | H | F | F | H | H | CF | C-NH-CO-CHF₂ | CF | CH | CH |
| 349 | Cl | Cl | Cl | H | F | H | H | CF | C-NH-CO-CF₃ | CF | CH | CH |
| 350 | H | F | Cl | H | Cl | F | H | CH | CH | CF | CH | CH |
| 351 | Cl | Cl | H | H | Cl | H | H | CF | C-NH-CO-tetrahydrofuran-2-yl | CF | CH | CH |
| 352 | Cl | Cl | H | F | F | H | H | CF | C-NH-CO-CF₃ | CF | CH | CH |
| 353 | Cl | F | H | H | Cl | H | H | CF | C-NH-CO-CH(Me)-O-CF₃ | CF | CH | CH |
| 354 | Cl | Cl | Cl | H | Cl | H | H | CF | C-NH-CO-CH₂-cProp | CF | CH | CH |
| 355 | H | F | CF₃ | H | Cl | H | H | CF | C-NH-CO-CHF₂ | CH | CH | CF |
| 356 | H | Cl | H | H | Cl | H | H | CF | C-NH₂ | CF | CH | CH |
| 357 | CF₃ | F | H | H | F | H | H | CF | C-NH-CO-CF₃ | CH | CH | CF |
| 358 | Cl | Cl | H | H | Cl | H | H | CF | C-NH-CO-C₂H₅ | CF | CH | CH |
| 359 | H | F | H | H | Cl | H | H | CF | C-NH-CO-CHF₂ | CF | CH | CH |
| 360 | H | F | CF₃ | H | Cl | H | H | CF | C-NH₂ | CH | CH | CF |
| 361 | Cl | Cl | H | H | Cl | H | H | CF | C-NH-CO-CH(Me)-O-CH₂H₅ | CF | CH | CH |
| 362 | H | F | CF₃ | H | Cl | H | H | CF | CH | CF | C-NH-CO-CF₃ | CH |
| 363 | F | H | CF₃ | H | Cl | H | H | CF | C-NH-CO-tetrahydrofuran-2-yl | CF | CH | CH |
| 364 | Cl | H | Cl | F | F | H | H | CF | C-NH-CO-CHF₂ | CF | CH | CH |
| 365 | H | Cl | H | H | Cl | H | H | CF | C-NH-CO-CHF₂ | CF | CH | CH |
| 366 | Cl | F | H | F | Cl | H | H | CF | CH | CF | C-NH-CO-CHF2 | CH |
| 367 | H | F | Br | H | Cl | H | H | CF | **C-NH-CO-CH(Me)-O-**CF₃ | CF | CH | CH |
| 368 | Cl | Cl | H | H | Cl | H | H | CF | C-NMe-CO-CF₃ | CF | CH | CH |
| 369 | Cl | F | H | H | Cl | H | H | CF | C-NH-CO-tetrahydrofuran-2-yl | CF | CH | CH |
| 370 | Cl | Cl | H | H | Cl | H | H | CH | C-NH-CO-CF₃ | CF | CH | CH |
| 371 | CF₃ | Cl | H | H | Cl | H | H | CF | C-NH-CO-CHF₂ | CF | CH | CH |
| 372 | Cl | Cl | H | H | F | H | H | CF | CH | CF | C-NH-CO-CHF₂ | CH |
| 373 | Cl | H | Cl | F | Cl | H | H | CF | CH | CF | C-NH-CO-CF₃ | CH |
| 374 | Cl | Cl | H | H | F | H | H | CF | C-NH-CO-CHF₂ | CH | CH | CF |
| 375 | Cl | F | H | F | Cl | H | H | CF | CH | CF | C-NH-CO-CF₃ | CH |
| 376 | Cl | F | H | H | Cl | H | H | CF | C-NH-CO-CH₂-CH=CH₂ | CF | CH | CH |
| 377 | H | H | F | H | Cl | H | H | CF | C-NH₂ | CF | CH | CH |
| 378 | Cl | Cl | H | F | Cl | H | H | CF | CH | CF | C-NH-CO-CHF₂ | CH |
| 379 | Cl | Cl | H | H | F | H | H | CF | C-NH-CO-CF₃ | CF | CH | CH |
| 380 | H | Cl | Br | H | Cl | H | H | CF | C-NH-CO-CF₃ | CF | CH | CH |
| 381 | H | H | CF₃ | H | Cl | H | H | CF | C-NH-CO-CHF₂ | CF | CH | CH |
| 382 | H | F | CF₃ | H | Cl | H | H | C-Me | C-NH₂ | C-Br | CH | CH |
| 383 | Cl | Cl | H | H | Cl | H | H | CF | C-NH-CO-C₂H₄-O-C₂H₅ | CF | CH | CH |
| 384 | Cl | Cl | Cl | F | F | H | H | CF | C-NH-CO-CF₃ | CF | CH | CH |
| 385 | F | H | CF₃ | H | Cl | H | H | CF | C-NH-CO-CH₂-CH=CH₂ | CF | CH | CH |
| 386 | H | H | CF₃ | H | Cl | H | H | CF | C-NH₂ | CF | CH | CH |
| 387 | H | Cl | Cl | Me | Cl | H | H | CF | C-NH-CO-CF₃ | CH | CH | CF |
| 388 | Cl | Cl | Cl | H | F | H | H | CF | C-NH-CO-CHF₂ | CF | CH | CH |
| 389 | Cl | F | H | H | Cl | H | H | CF | C-NH-CO-C₃H₇ | CF | CH | CH |
| 390 | Cl | Cl | Cl | H | Cl | H | H | CF | C-NH-CO-CCIF₂ | CF | CH | CH |
| 391 | CF₃ | F | H | F | F | H | H | CF | CH | CF | C-NH-CO-CHF₂ | CH |
| 392 | Cl | F | H | H | Cl | H | H | CF | C-NH-CO-C₂H₅ | CF | CH | CH |
| 393 | H | F | Br | H | Cl | H | H | CF | C-NH-CO-CH₂-O-CF₃ | CF | CH | CH |
| 394 | Cl | Cl | Cl | H | Cl | H | H | CF | C-NH-CO-CH₂-O-Me | CF | CH | CH |
| 395 | Cl | Cl | Cl | H | F | H | H | CF | CH | CF | C-NH-CO-CHF₂ | CH |
| 396 | Cl | Cl | Cl | H | Cl | H | H | CF | CH | CF | C-NH-CO-CHF₂ | CH |
| 397 | CF₃ | F | H | F | F | H | H | CF | CH | CF | C-NH-CO-CF₃ | CH |
| 398 | H | H | CF₃ | H | Cl | H | H | CF | C-NH-CO-tetrahydrofuran-2-yl | CF | CH | CH |
| 399 | F | H | CF₃ | H | Cl | H | H | CF | C-NH-CO-C₂H₅ | CF | CH | CH |
| 400 | Cl | Cl | Cl | H | F | H | H | CF | CH | CF | C-NH-CO-Me | CH |
| 401 | F | H | CF₃ | H | Cl | H | H | CF | C-NH-CO-CF₂CHF₂ | CF | CH | CH |
| 402 | Cl | Cl | H | H | Cl | H | Me | CF | C-NH-CO-CF₃ | CF | CH | CH |
| 403 | Cl | F | H | H | Cl | H | H | CF | C-NH-CO-Me | CF | CH | CH |
| 404 | H | F | Cl | H | Cl | H | H | CH | C-NH-CO-CF₃ | CCL | CH | CH |
| 405 | Cl | H | Cl | H | Cl | H | H | CH | C-NH-CO-CF₃ | CF | CH | CH |
| 406 | H | H | CF₃ | H | Cl | H | H | CF | C-NH-CO-Me | CF | CH | CH |
| 407 | Cl | Cl | H | F | F | H | H | CF | CH | CF | C-NH-CO-CF₃ | CH |
| 408 | Cl | Cl | Cl | H | Cl | H | H | CF | C-NH-CO-CF₃ | CF | CH | CH |
| 409 | H | H | CF₃ | H | Cl | H | H | CF | C-NH-CO-CH₂-CH=CH₂ | CF | CH | CH |
| 410 | Cl | F | H | H | Cl | H | H | CF | C-NH-CO-CH₂-O-CH₂-CF₃ | CF | CH | CH |
| 411 | Cl | Cl | Cl | F | F | H | H | CF | CH | CF | C-NH-CO-CHF₂ | CH |
| 412 | Cl | Cl | H | H | Cl | H | H | CF | C-NH-CO-CH2-cProp | CF | CH | CH |
| 413 | F | H | CF₃ | H | Cl | H | H | CF | C-NH-CO-CHF₂ | CF | CH | CH |
| 414 | H | F | CF₃ | Me | Cl | H | H | CF | C-NH-CO-CF₃ | CH | CH | CF |
| 415 | Cl | Cl | Cl | H | Cl | H | H | CF | C-NH-CO-CF₂CHF₂ | CF | CH | CH |
| 416 | CF₃ | F | H | H | F | H | H | CF | CH | CF | C-NH-CO-CF₃ | CH |
| 417 | CF₃ | F | H | H | Cl | H | H | CH | C-NH-CO-CF₃ | CF | CH | CH |
| 418 | Cl | Cl | H | H | Cl | H | Me | CF | C-NH-CO-CH(Me)-O-C₂H₅ | CF | CH | CH |
| 419 | H | Br | H | H | Cl | H | H | CF | C-NH₂ | CF | CH | CH |
| 420 | H | Cl | Cl | Me | Cl | H | H | CF | C-NH-CO-CF₃ | CF | CH | CH |
| 421 | CF₃ | F | H | F | F | H | H | CF | C-NH-CO-CHF₂ | CF | CH | CH |
| 422 | CF₃ | Cl | H | H | Cl | H | H | CF | C-NH-CO-CCIF₂ | CF | CH | CH |
| 423 | Cl | Cl | H | H | Cl | H | H | CF | C-NH-Me | CF | CH | CH |
| 424 | Cl | Cl | H | H | F | H | H | CF | C-NH-CO-CF₃ | CH | CH | CF |
| 425 | Cl | Cl | H | F | F | H | H | CF | CH | CF | C-NH-CO-CHF₂ | CH |
| 426 | Cl | Cl | Cl | H | F | H | H | CF | CH | CF | C-NH-CO-CF₃ | CH |
| 427 | Cl | Cl | Cl | F | F | H | H | CF | CH | CF | C-NH-CO-CF₃ | CH |
| 428 | Cl | Cl | Cl | H | Cl | H | H | CF | CH | CF | C-NH-CO-CF₂-CF₂H | CH |
| 429 | H | F | CF₃ | H | Cl | H | H | CF | CH | CF | C-NH-CO-CHF₂ | CH |
| 430 | H | H | CF₃ | H | Cl | H | H | CF | C-NH-CO-CF₂CHF₂ | CF | CH | CH |
| 431 | CF₃ | F | H | F | Cl | H | H | CF | CH | CF | C-NH-CO-CHF₂ | CH |
| 432 | Cl | F | H | H | Cl | H | H | CF | C-NH-CO-CF₂CHF₂ | CF | CH | CH |
| 433 | H | H | CF₃ | H | Cl | H | H | CF | C-NH-CO-C₂H₅ | CF | CH | CH |
| 434 | Cl | Cl | H | H | Cl | H | H | CF | C-NH-CO-CF₂CHF₂ | CF | CH | CH |
| 435 | Cl | F | H | H | Cl | H | H | CF | C-NH-CO-CCIF₂ | CF | CH | CH |
| 436 | H | F | Br | H | Cl | H | H | CF | C-NH-CO-CH₂-CH=CH₂ | CF | CH | CH |
| 437 | CF₃ | F | H | H | F | H | H | CF | CH | CF | C-NH-CO-CHF₂ | CH |
| 438 | H | F | CF₃ | H | Cl | H | H | CF | CF | CF | C-NH-CO-CHF₂ | CH |
| 439 | H | F | CF₃ | Me | Cl | H | H | CF | CH | CF | C-NH-CO-CF₃ | CH |
| 440 | H | H | CF₃ | H | Cl | H | H | CF | C-NH-CO-CH2-O-CF₃ | CF | CH | CH |
| 441 | H | H | CF₃ | H | Cl | H | H | CF | C-NH-CO-CH(Me)-O-CF₃ | CF | CH | CH |
| 442 | Cl | F | H | H | Cl | H | H | CF | C-NH-CO-C₂F₅ | CF | CH | CH |
| 443 | H | F | CF₃ | H | Cl | H | H | CF | C-NH-CO-CF₂-CHF₂ | CH | CH | CF |
| 444 | Cl | Cl | H | F | F | H | H | CF | CH | CF | C-NH-CO-Me | CH |
| 445 | CF₃ | F | H | H | F | H | H | CF | C-NH-CO-CHF₂ | CH | CH | CF |
| 446 | Cl | H | Cl | H | F | H | H | CH | CH | CF | CH | CH |
| 447 | Cl | H | Cl | H | F | H | H | CF | CH | CF | CH | CH |
| 448 | Cl | H | Cl | H | F | H | H | CF | CH | CF | CH | CH |
| 449 | Cl | H | Cl | H | Cl | H | H | CF | CH | CF | CH | CF |
| 450 | Cl | H | Cl | H | Cl | H | H | CF | CH | CF | CH | CF |
| 451 | F | F | F | H | Cl | H | H | CF | CH | CF | CH | CH |
| 452 | F | F | H | H | Cl | H | H | CF | CH | CF | CH | CH |
| 453 | Cl | H | Cl | H | Cl | H | H | N | CH | CF | CH | CF |
| 454 | Cl | H | Cl | H | Cl | H | H | N | CH | CF | C-Me | CH |
| 455 | Cl | H | Cl | H | Cl | H | H | CH | N | CF | CH | CF |
| 456 | Cl | H | Cl | H | Cl | H | H | CF | N | CH | CH | CF |
| 457 | Cl | H | Cl | F | Me | H | H | N | CH | CF | CH | CF |
| 458 | Cl | H | Cl | H | Cl | H | H | CF | CH | CH | CH | CF |
| 460 | Cl | H | Cl | H | Cl | H | H | N | CH | CF | CH | N |
| 462 | Cl | Cl | H | H | Cl | H | H | N | CH | CF | CH | CF |
| 463 | Cl | H | Cl | H | F | H | H | N | CH | CF | CH | CF |
| 464 | Cl | H | Cl | F | Cl | H | H | N | CH | CF | CH | CF |
| 465 | Cl | H | Cl | H | H | F | H | N | CH | CF | CH | CF |
| 466 | Cl | Cl | Cl | H | Cl | H | H | CH | N | CF | CH | CF |
| 467 | Cl | H | Cl | H | Cl | F | H | N | CH | CF | CH | CF |
| 468 | H | Cl | Cl | H | Me | F | H | N | CH | CF | CH | CF |
| 469 | Cl | F | Cl | H | Cl | H | H | N | CH | CF | CH | CF |
| 470 | Cl | Cl | Cl | H | Cl | H | H | N | CH | CF | CH | CF |
| 471 | CF3 | H | CF3 | H | Cl | H | H | N | CH | CF | CH | CF |
| 472 | CF3 | H | CF3 | H | Cl | H | H | CF | CH | CF | CH | CH |
| 473 | CF3 | H | CF3 | H | F | H | H | CF | CH | CF | CH | CH |
| 474 | CF3 | H | CF3 | F | F | H | H | N | CH | CF | CH | CF |
| 475 | Cl | H | CF3 | H | Cl | H | H | N | CH | CF | CH | CF |
| 476 | Cl | H | CF3 | H | Cl | H | H | N | CH | CF | CH | CF |
| 477 | Cl | H | Cl | F | F | H | H | N | CH | CF | CH | CF |
| 478 | F | F | F | H | Cl | H | H | N | CH | CF | CH | CF |
| 479 | F | F | F | H | F | H | H | N | CH | CF | CH | CF |
| 480 | Cl | H | Cl | H | F | F | H | N | CH | CF | CH | CF |

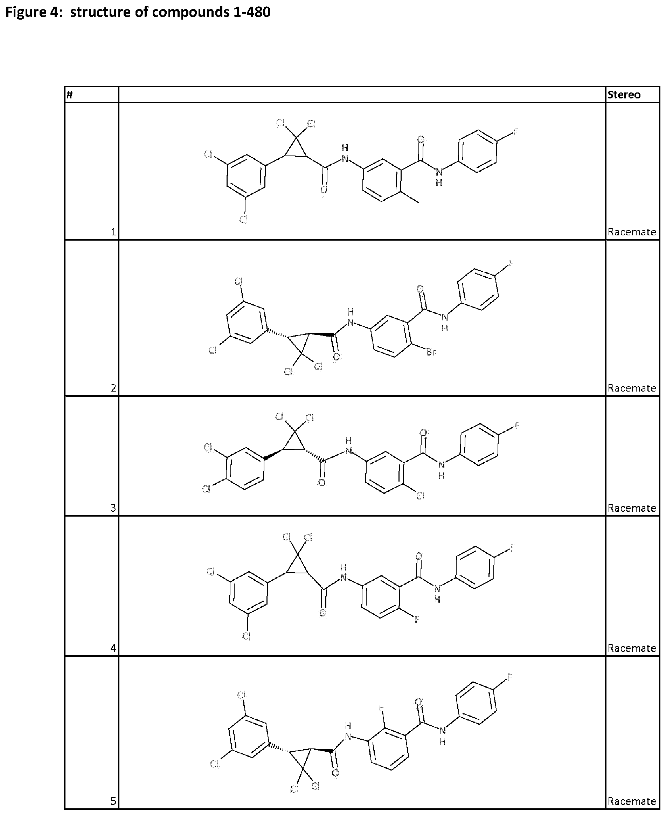

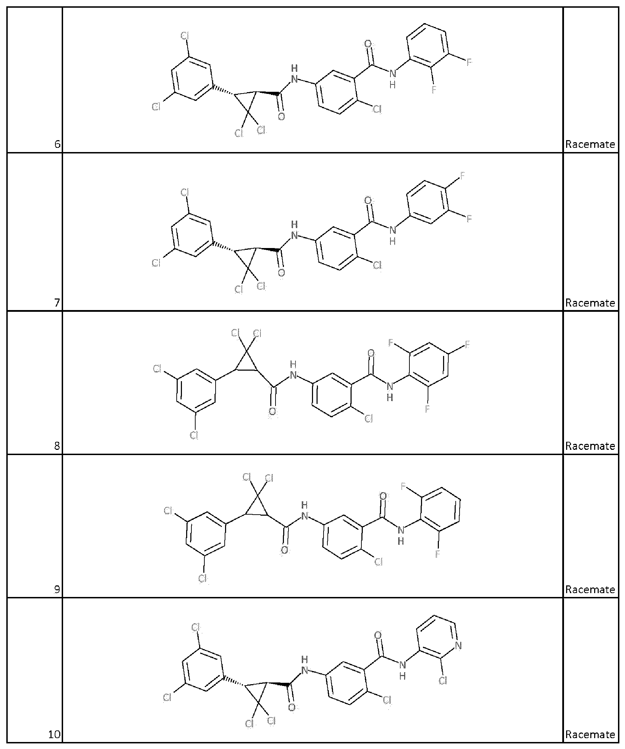

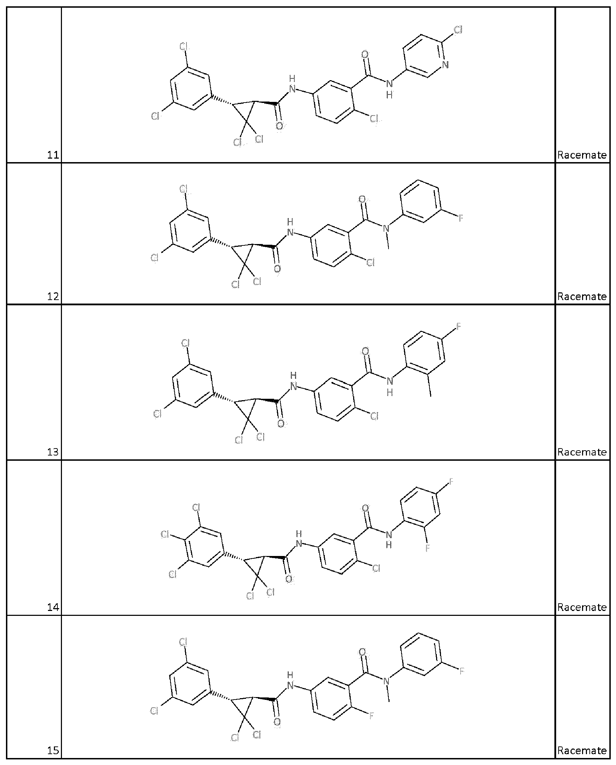

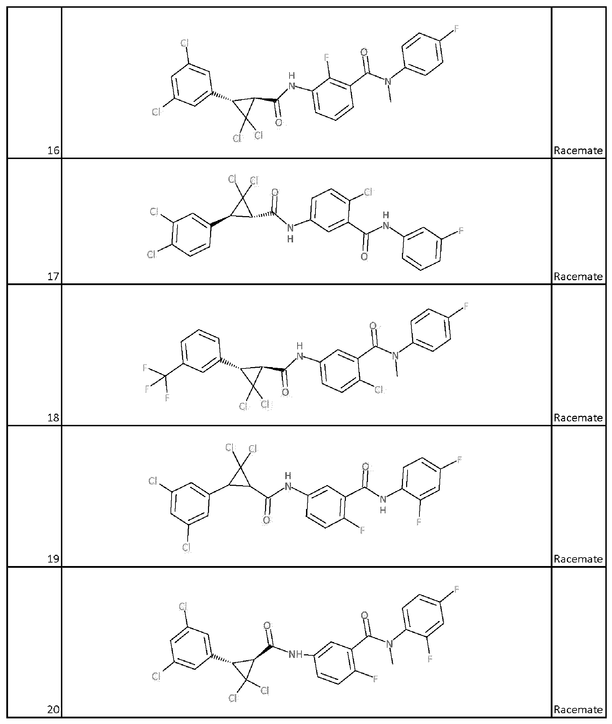

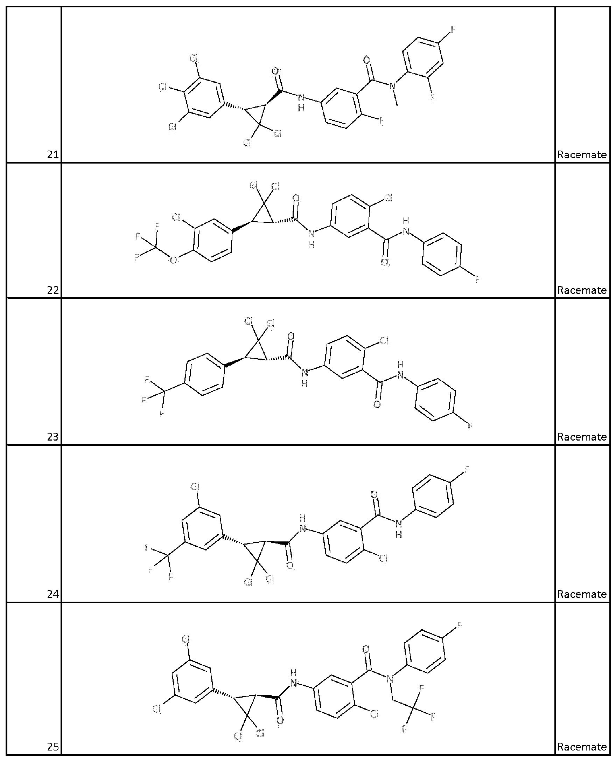

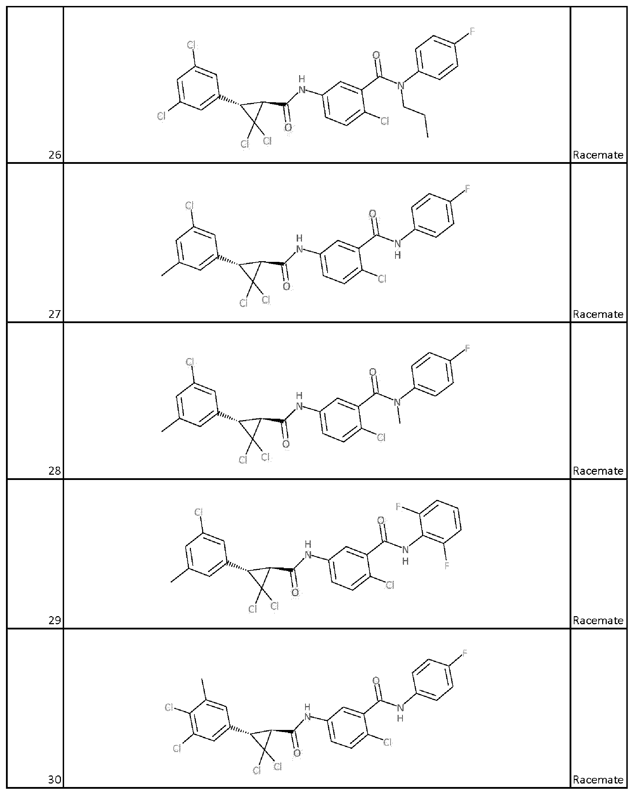

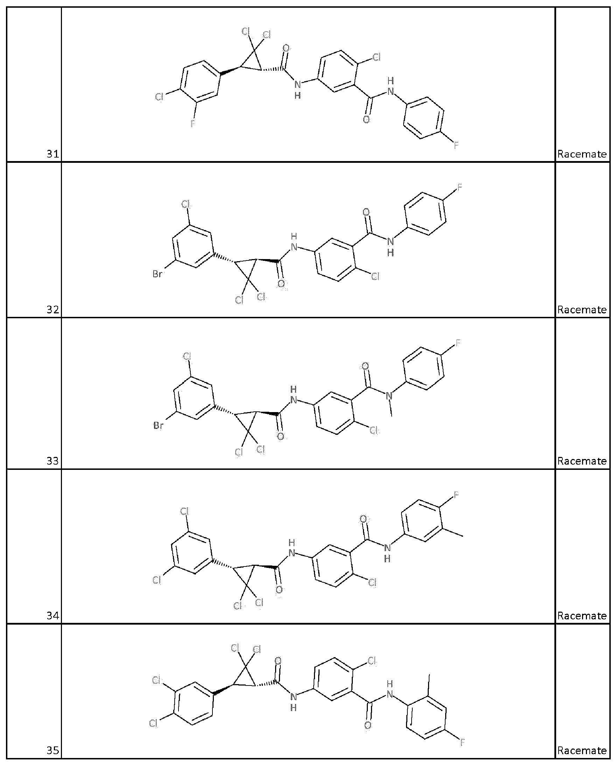

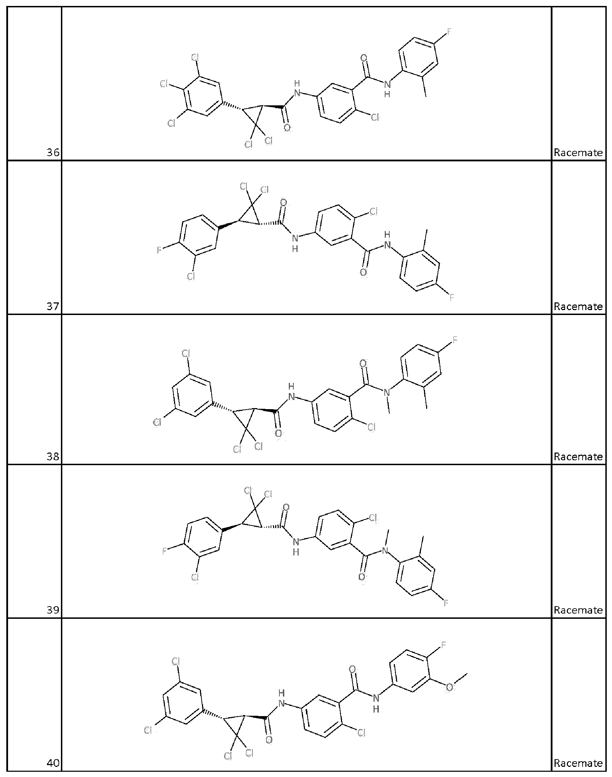

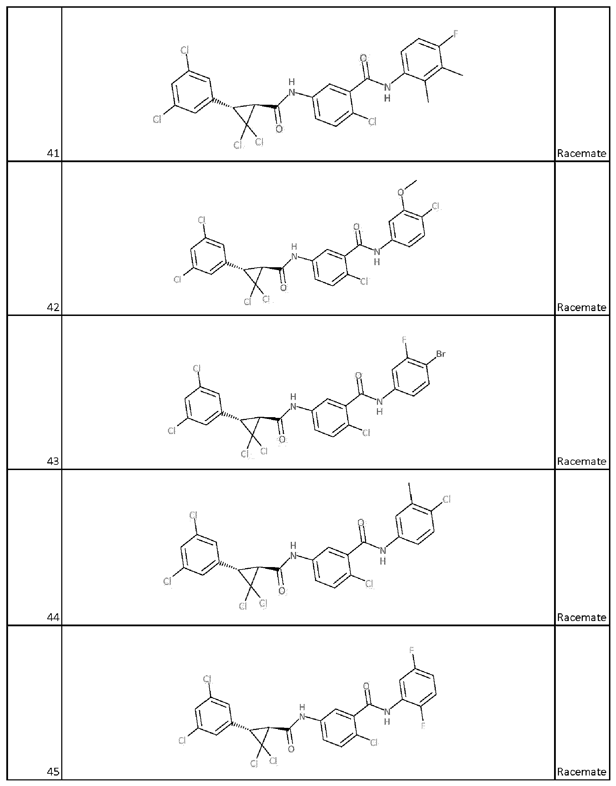

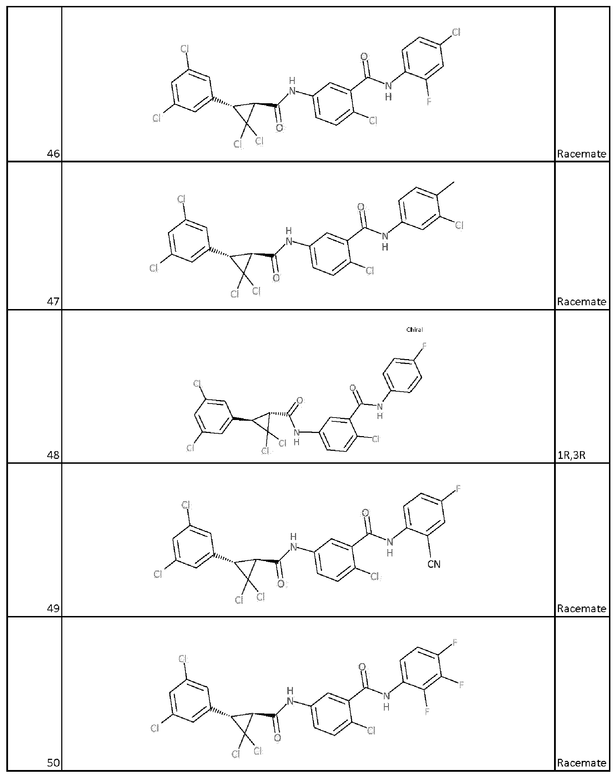

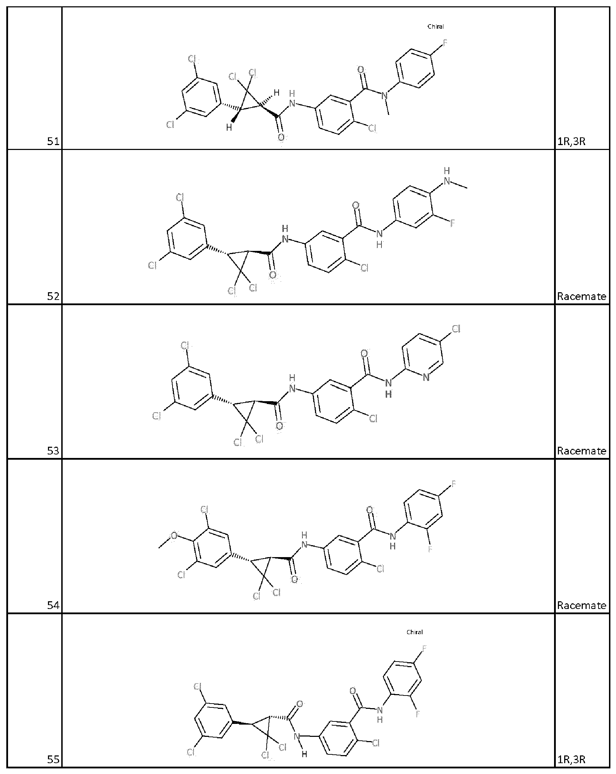

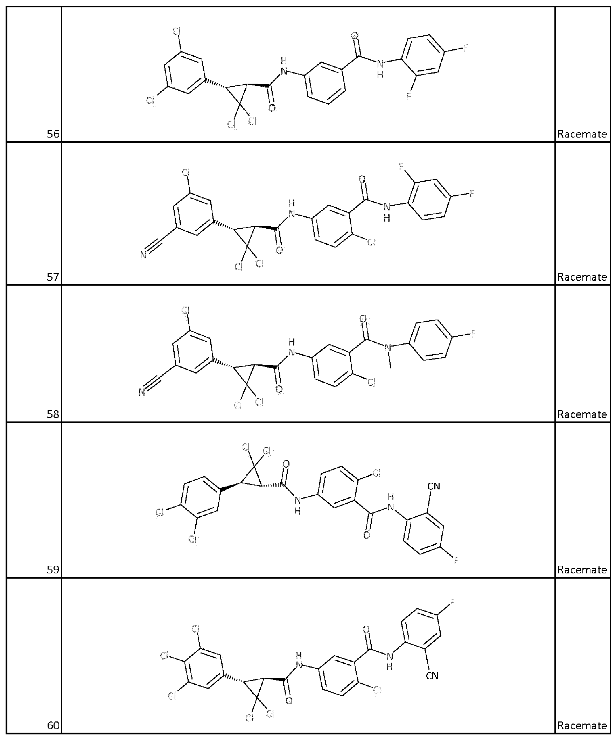

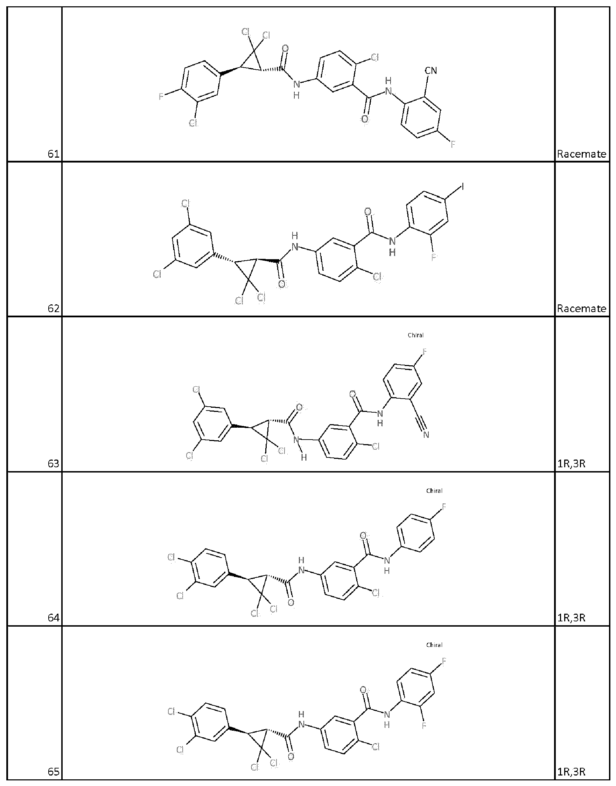

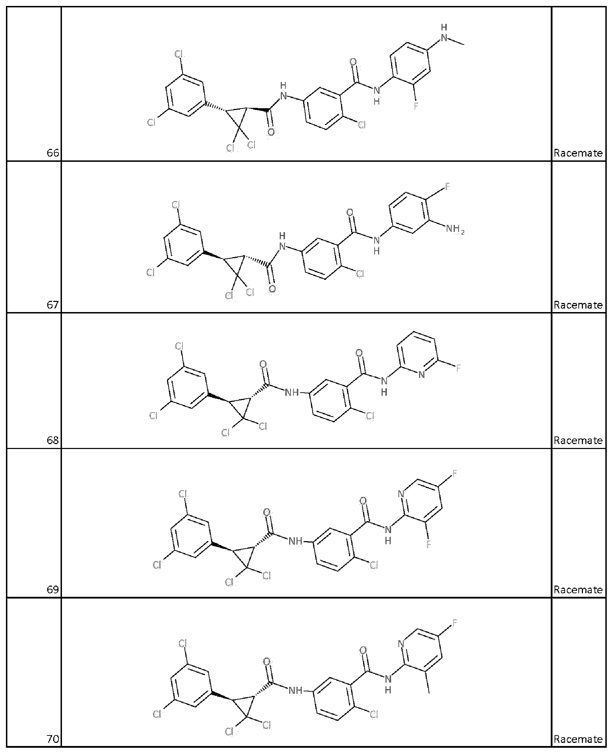

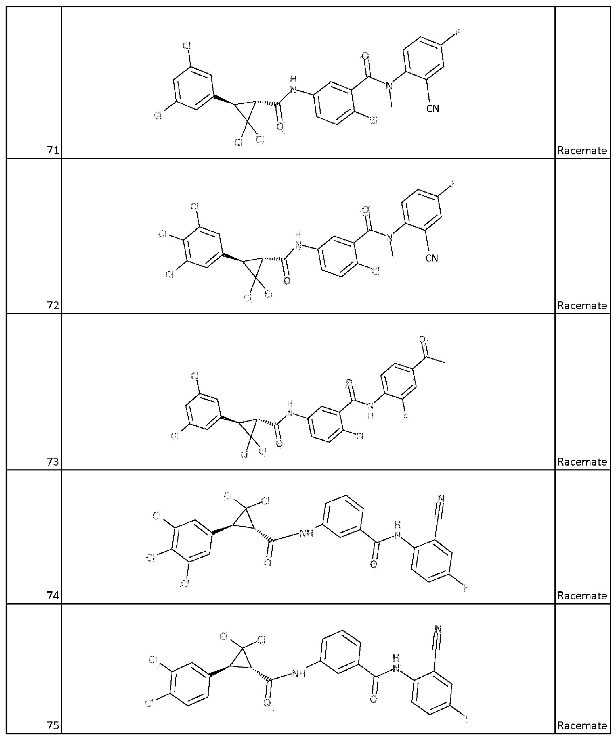

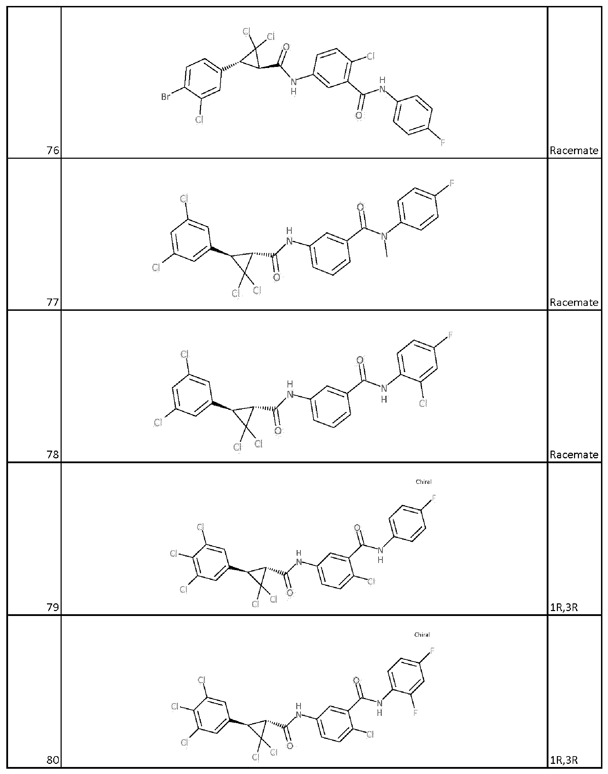

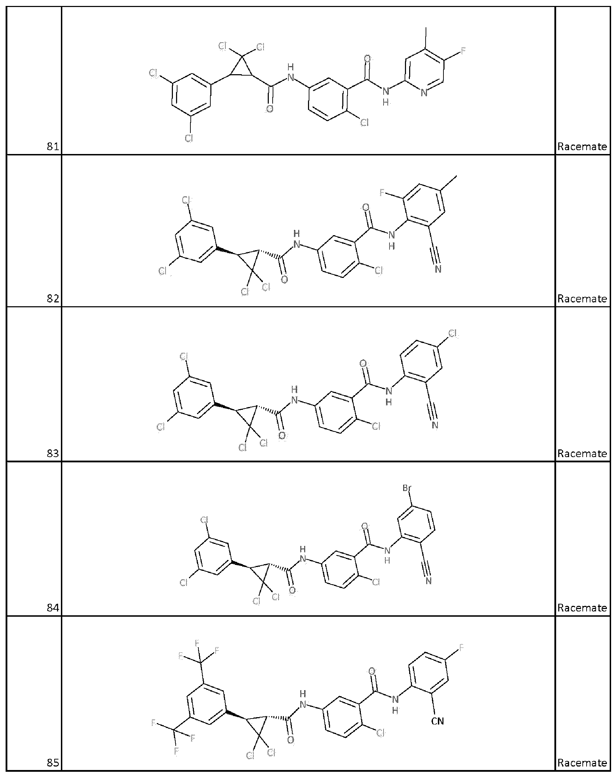

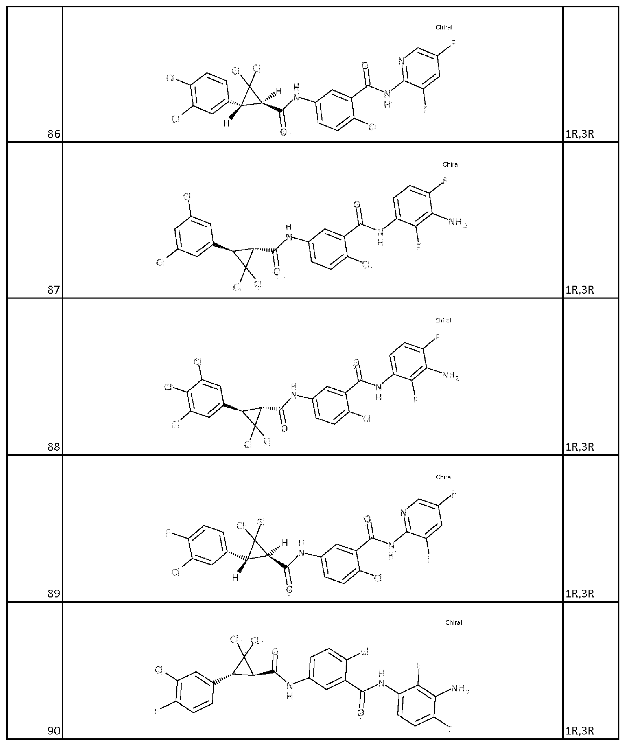

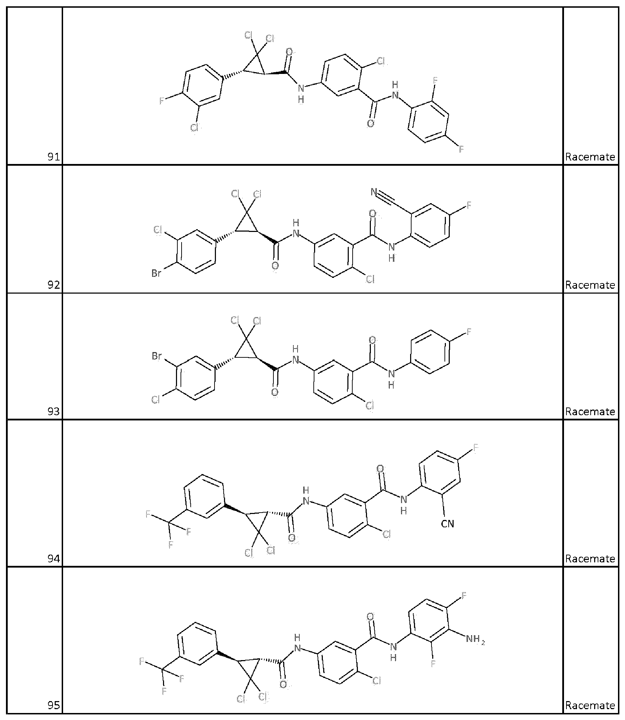

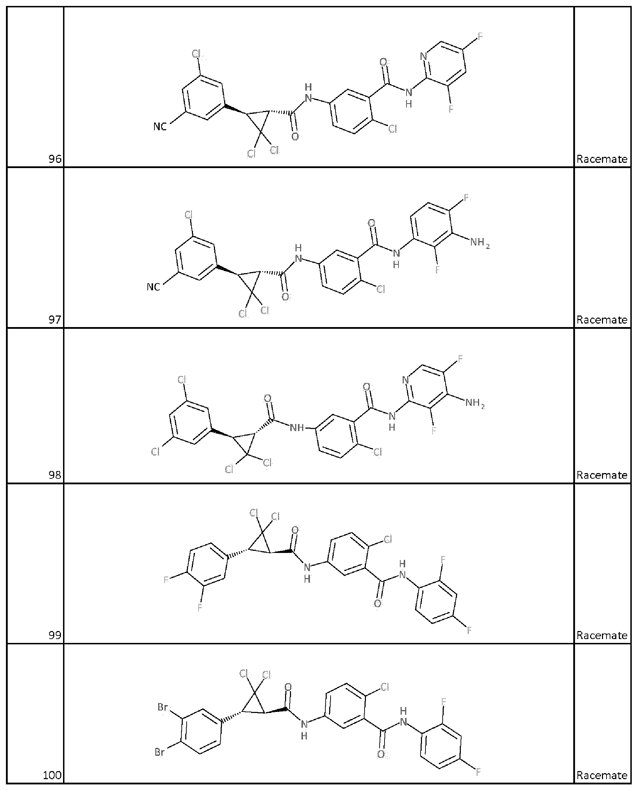

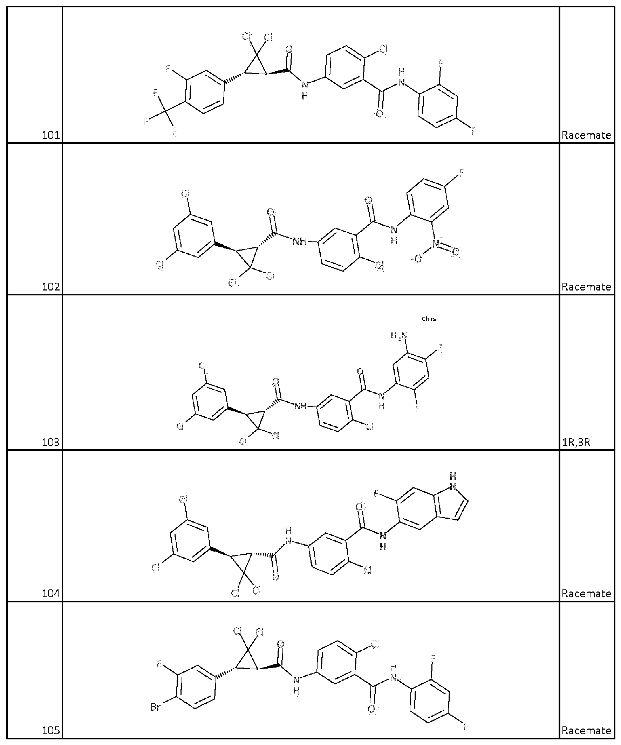

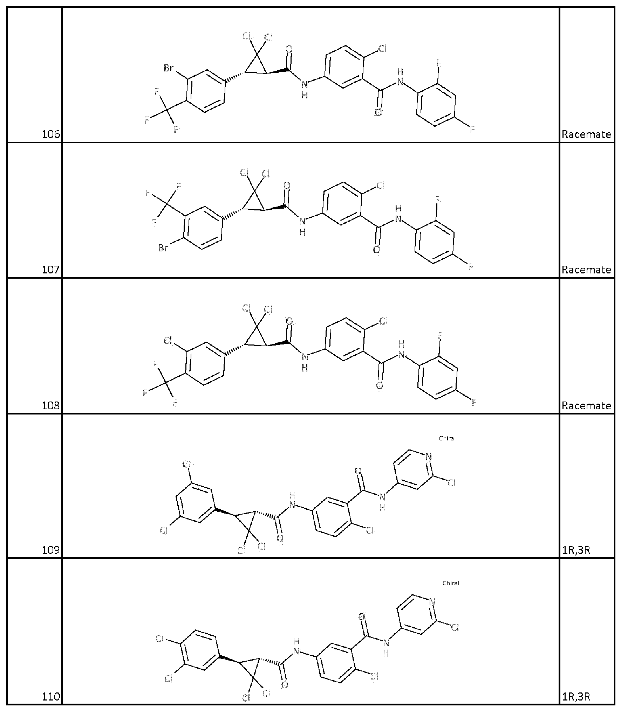

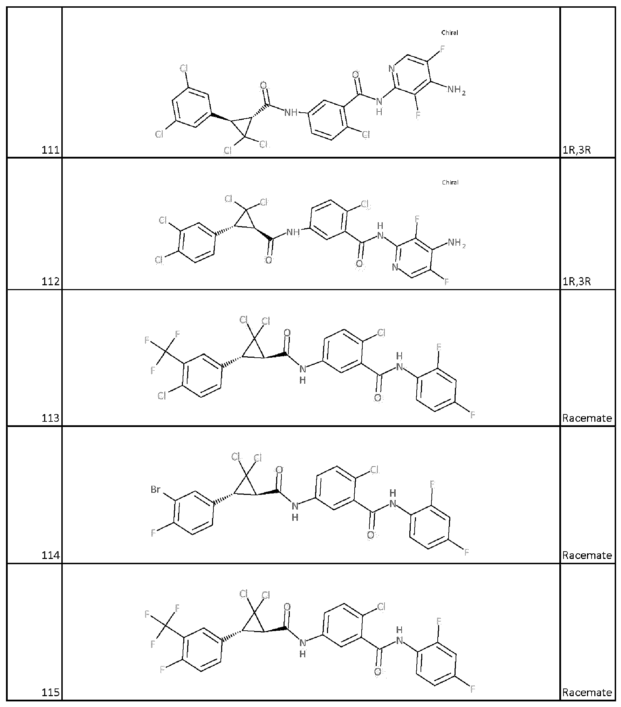

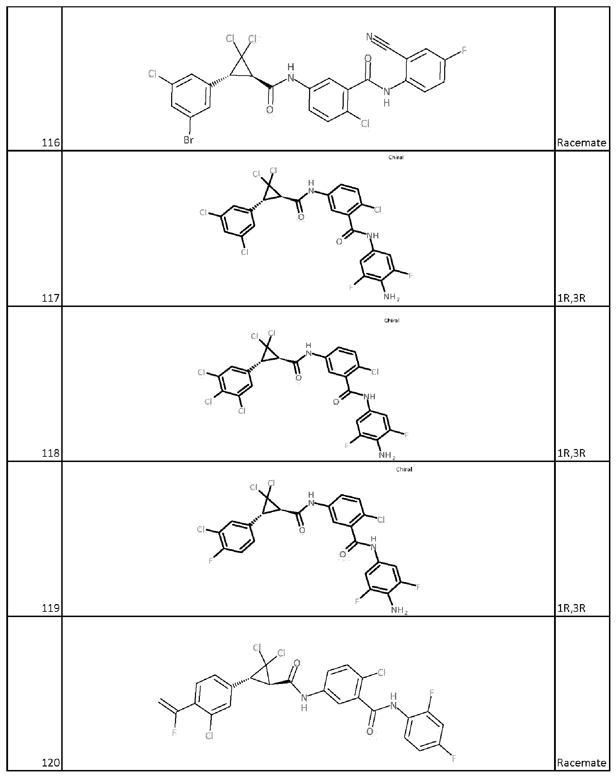

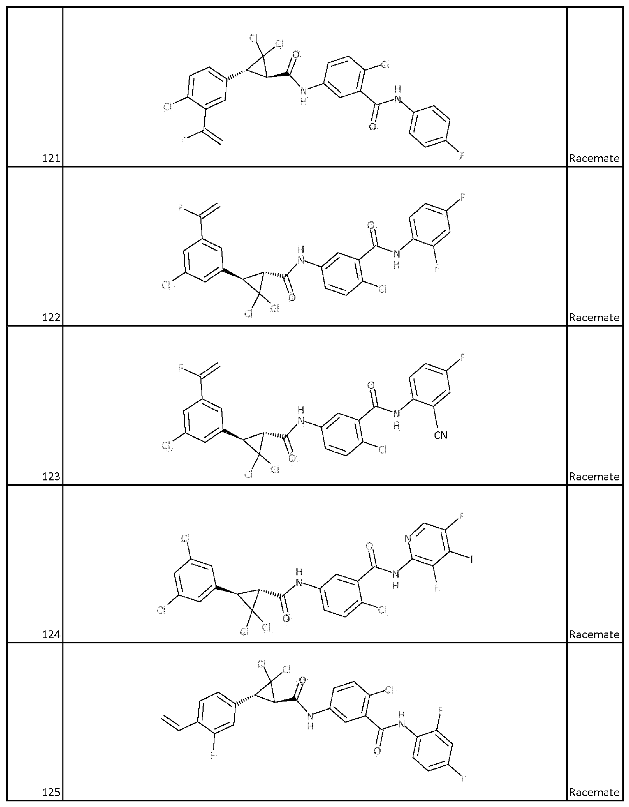

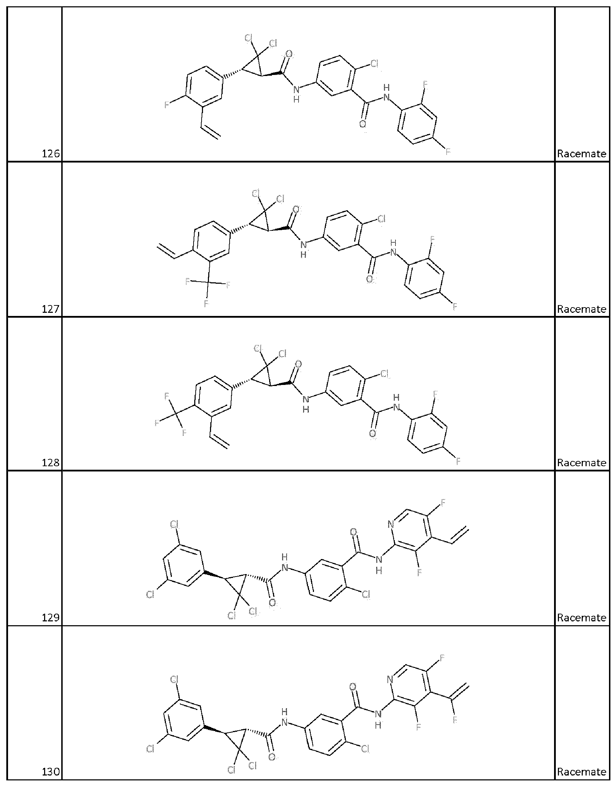

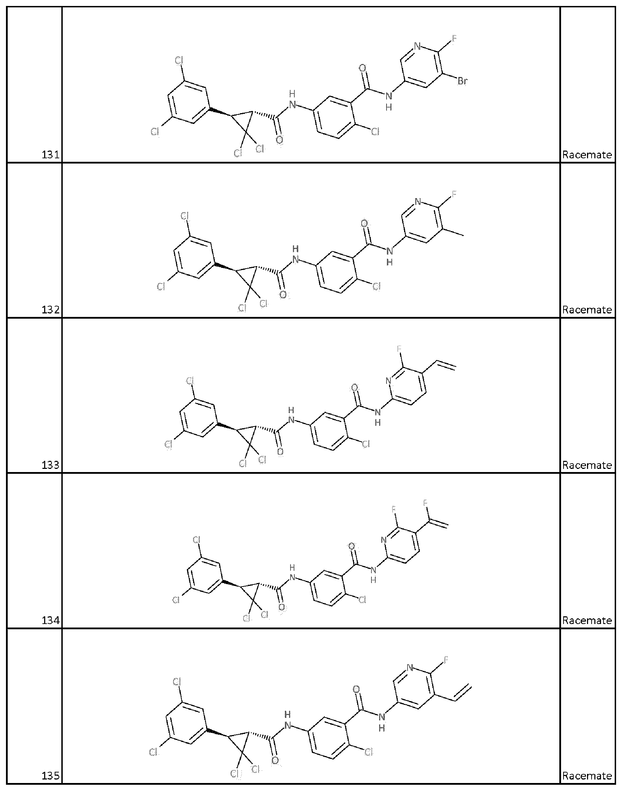

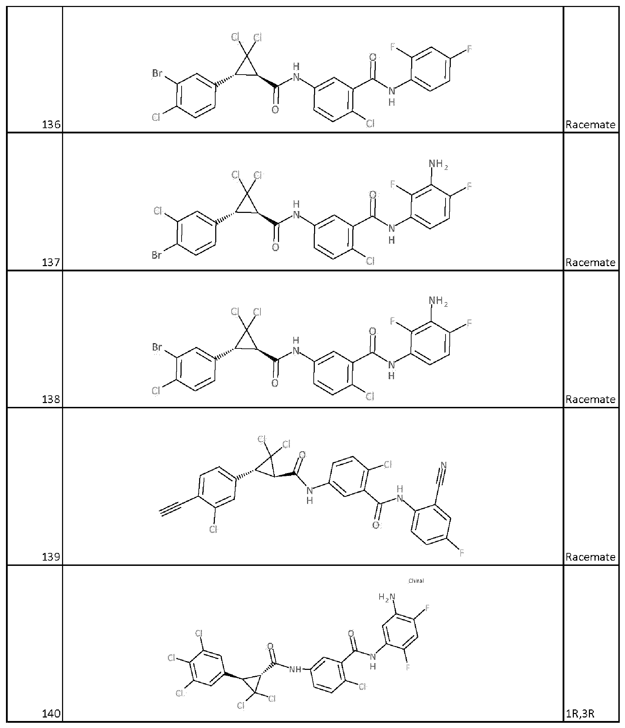

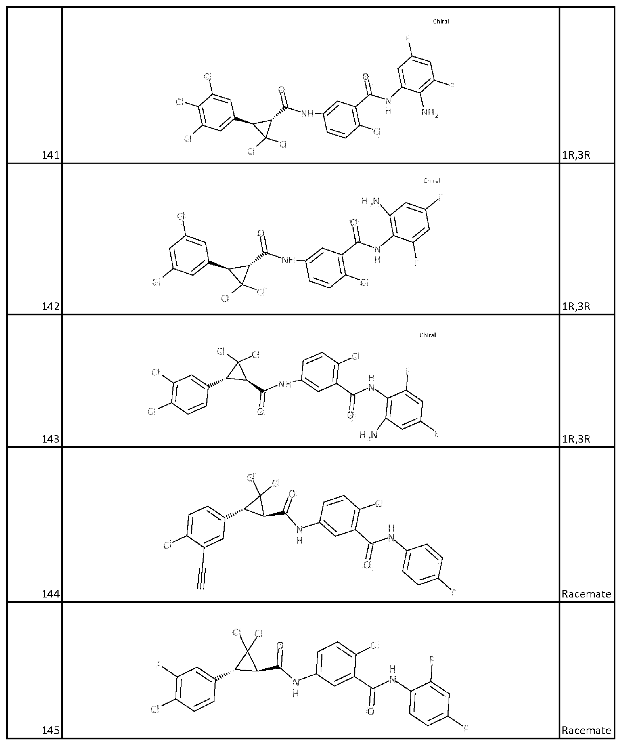

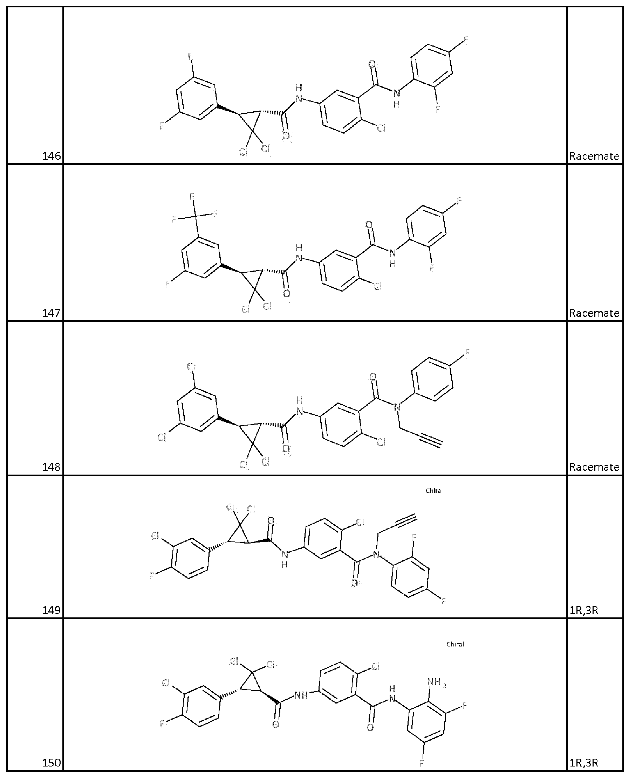

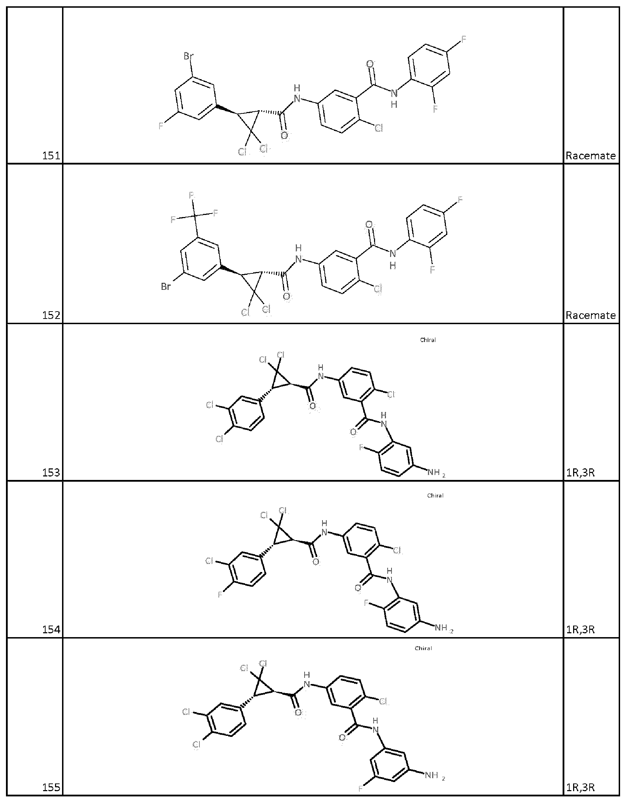

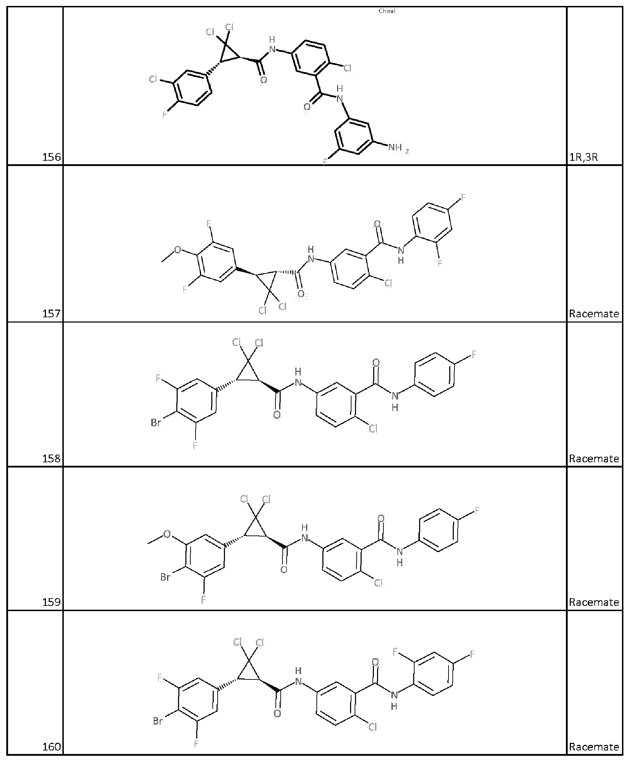

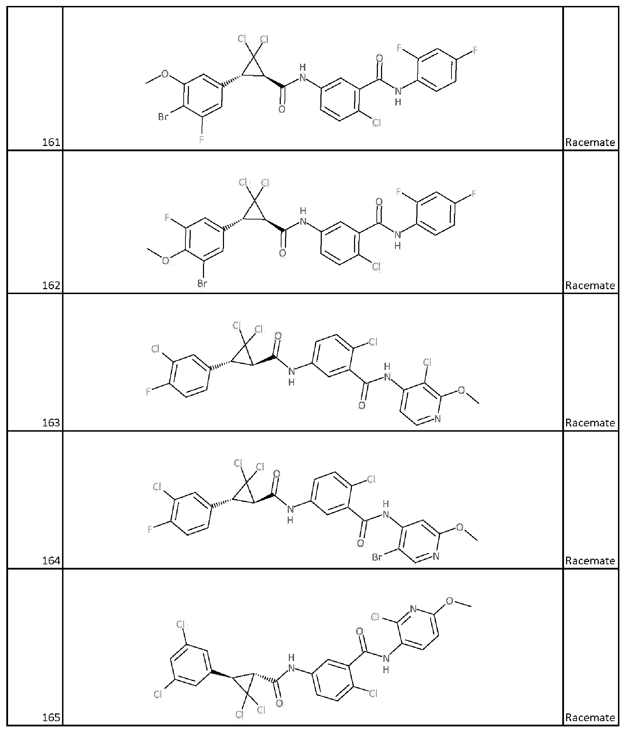

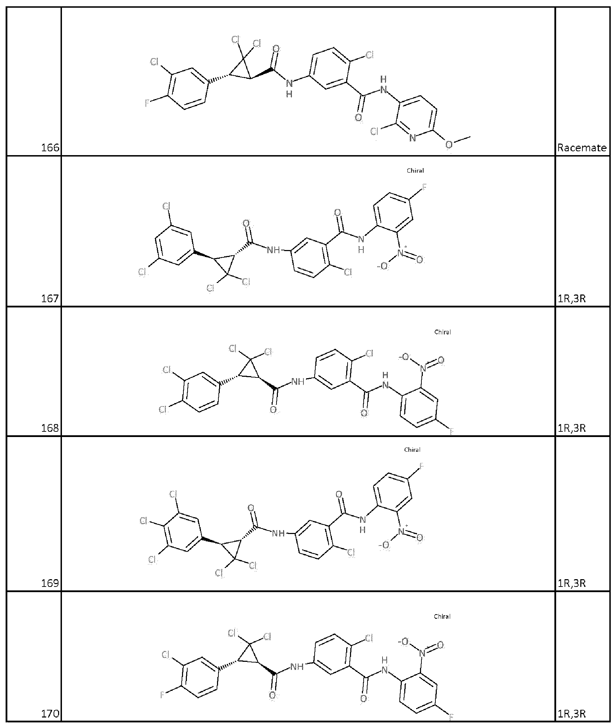

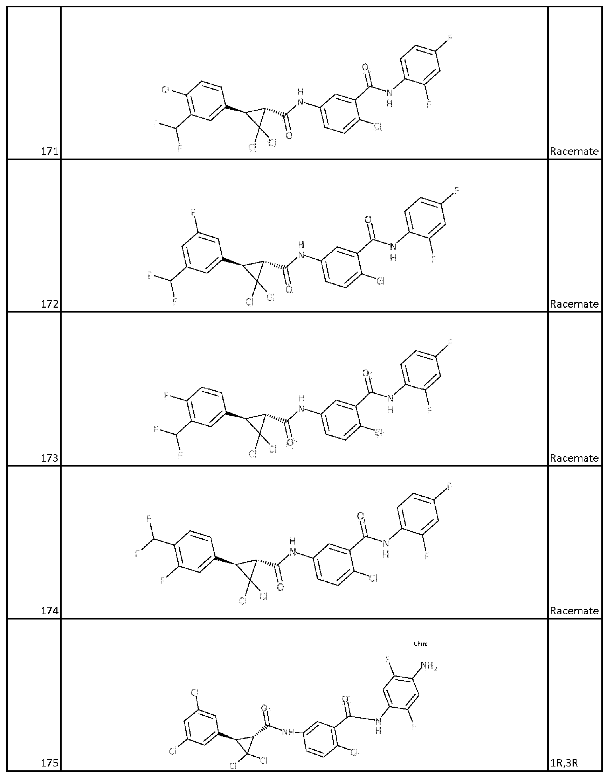

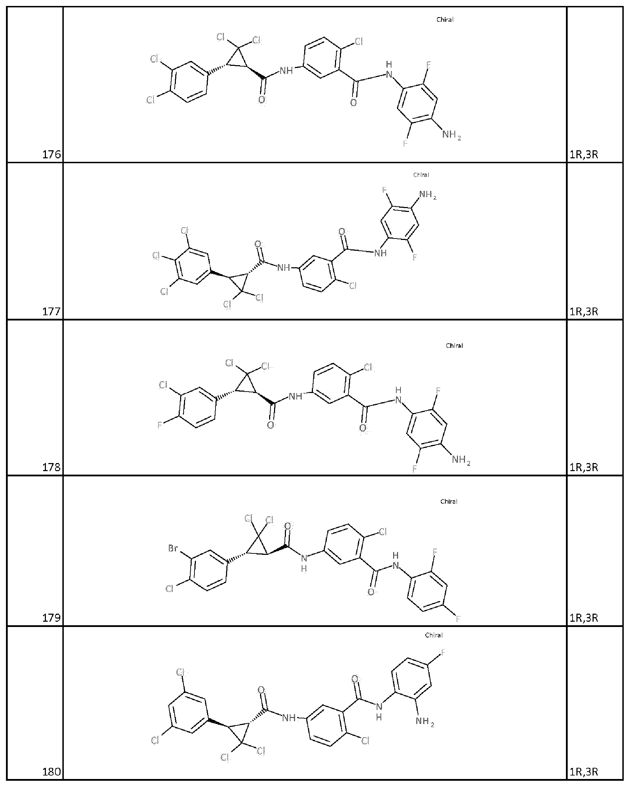

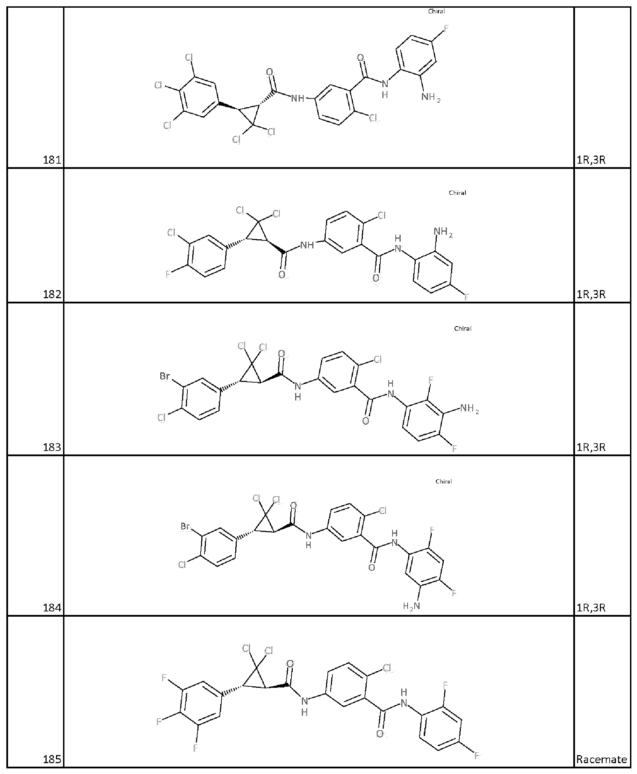

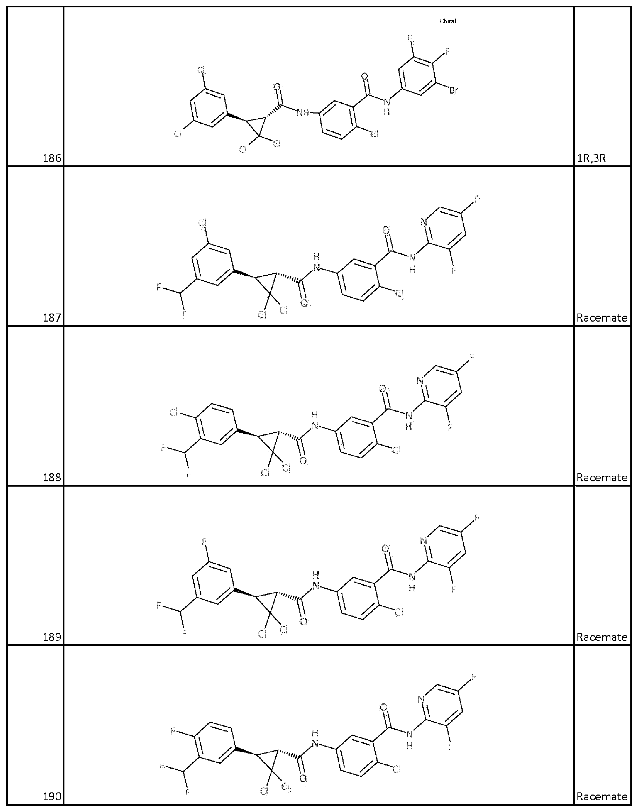

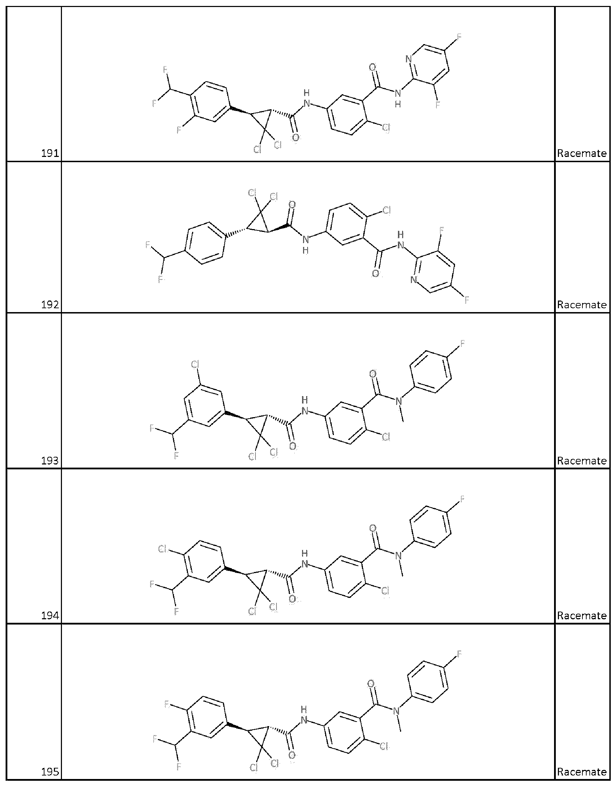

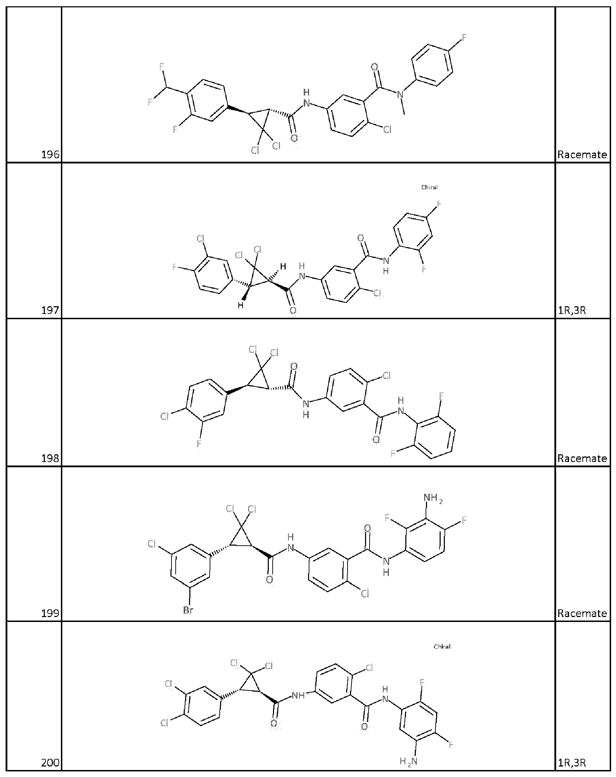

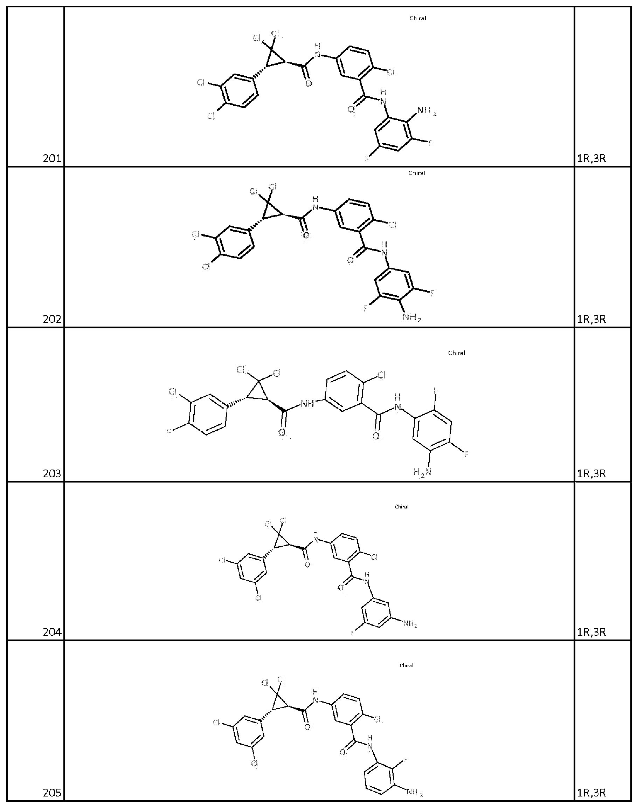

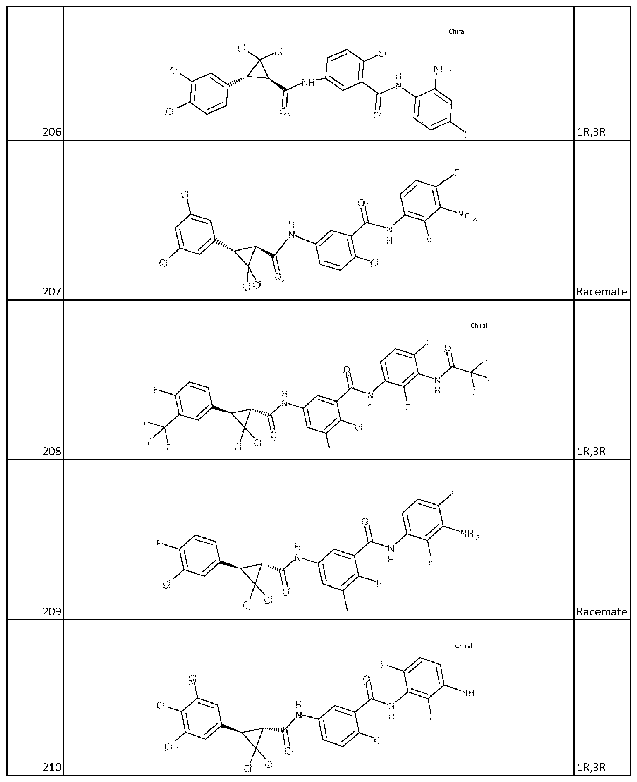

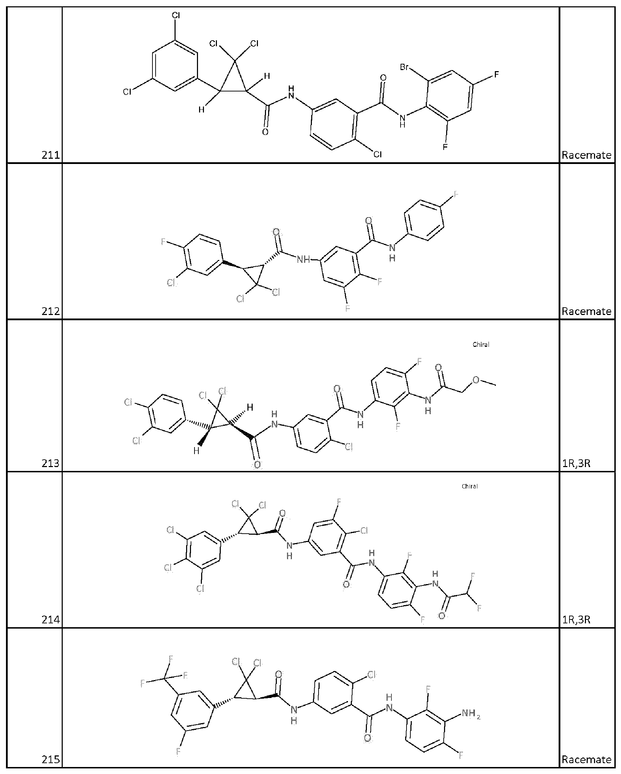

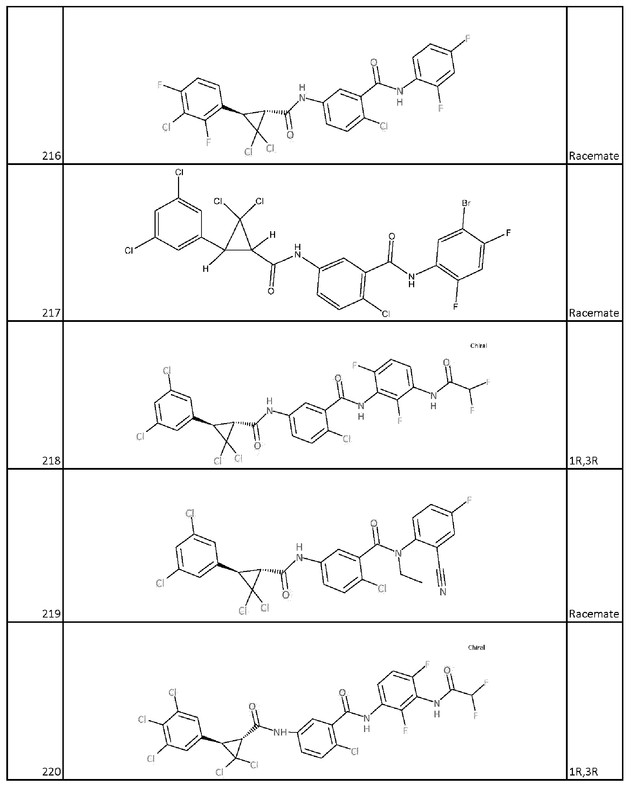

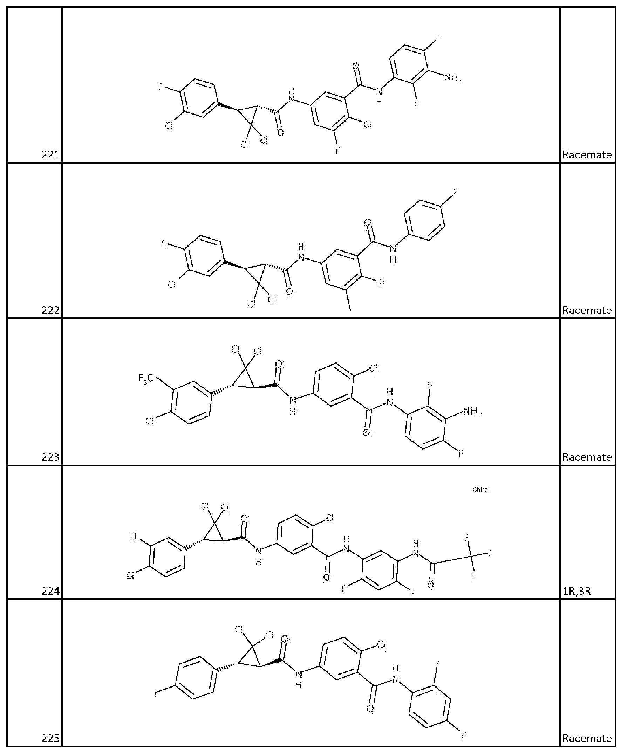

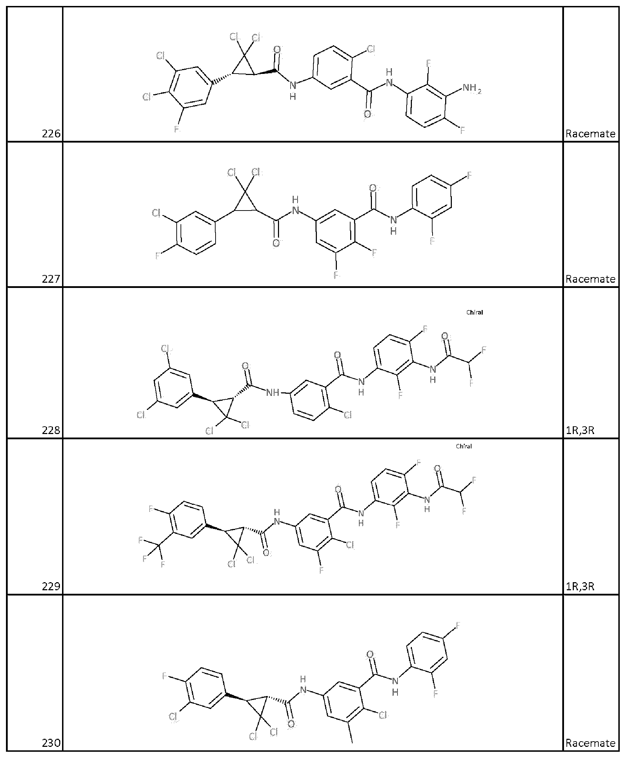

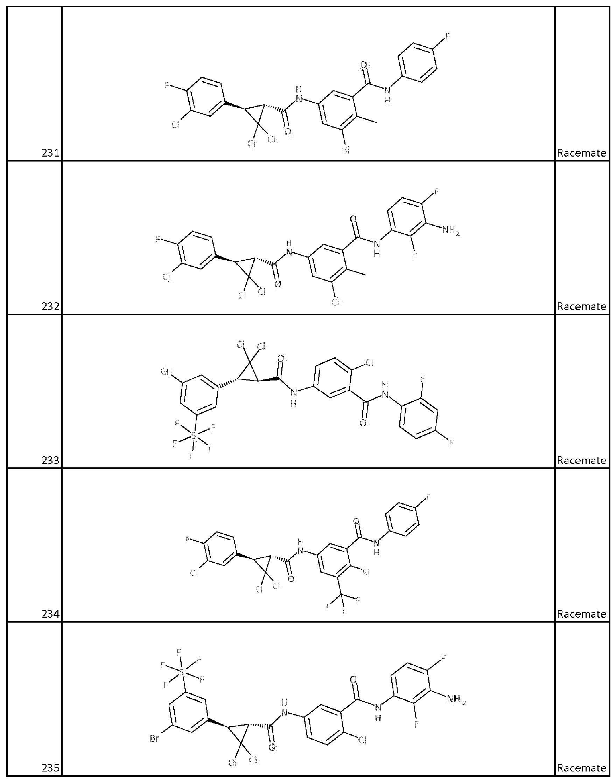

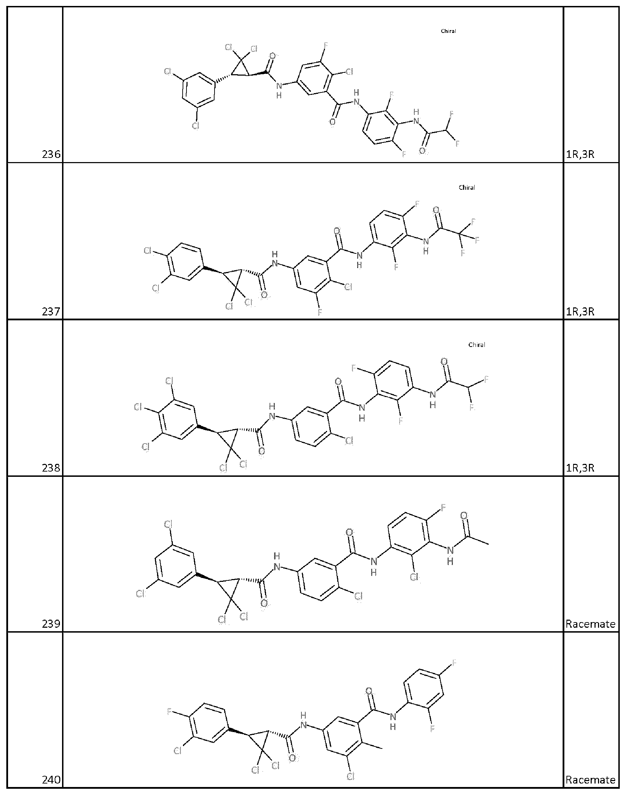

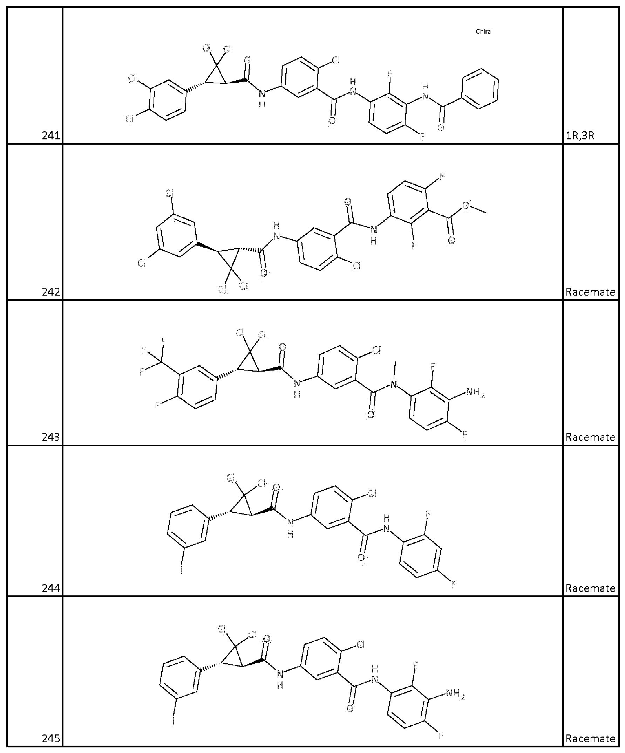

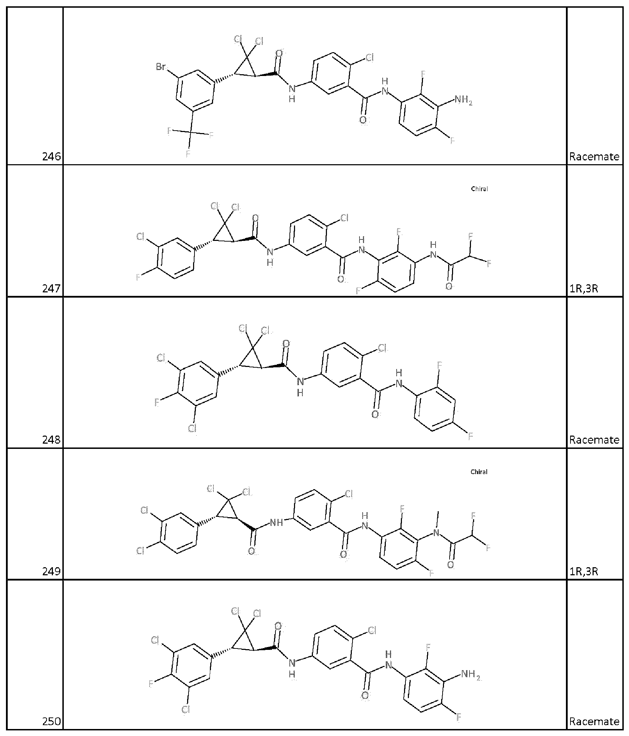

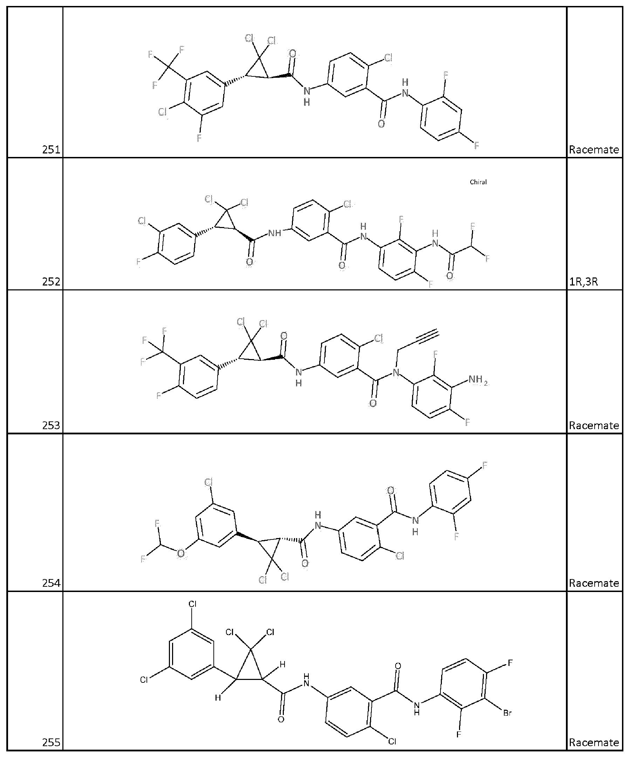

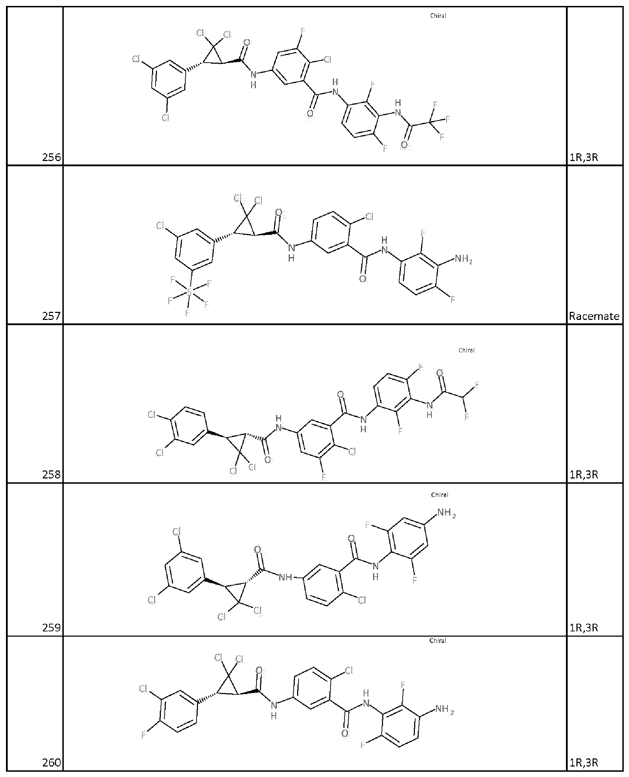

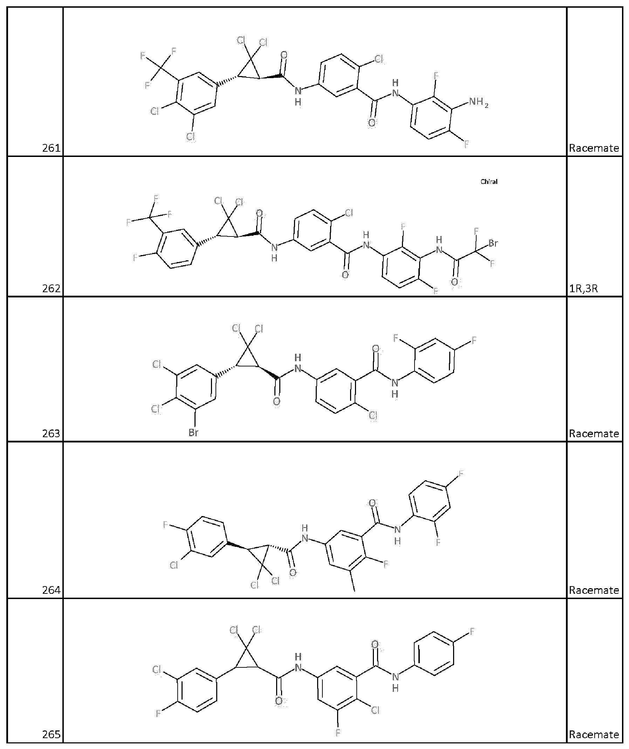

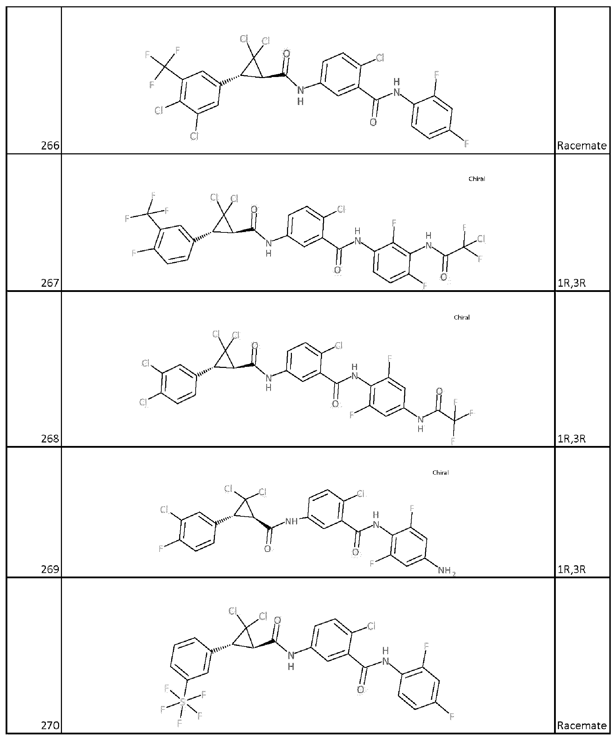

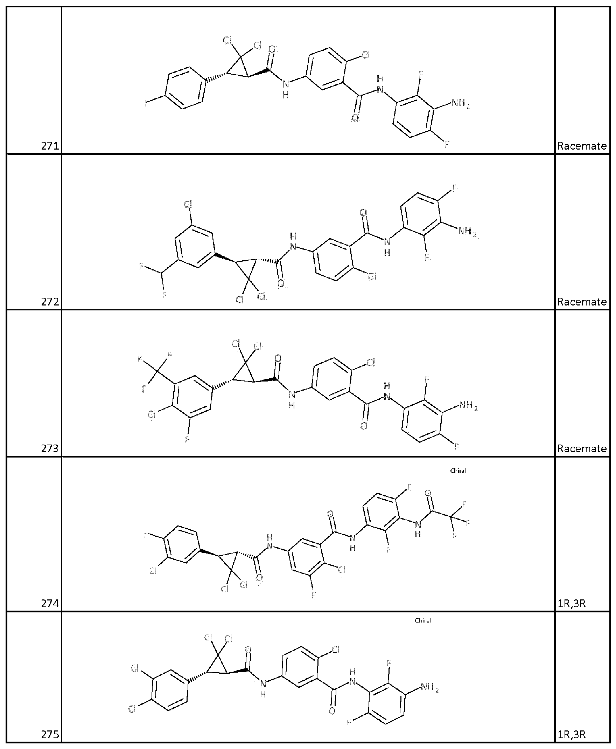

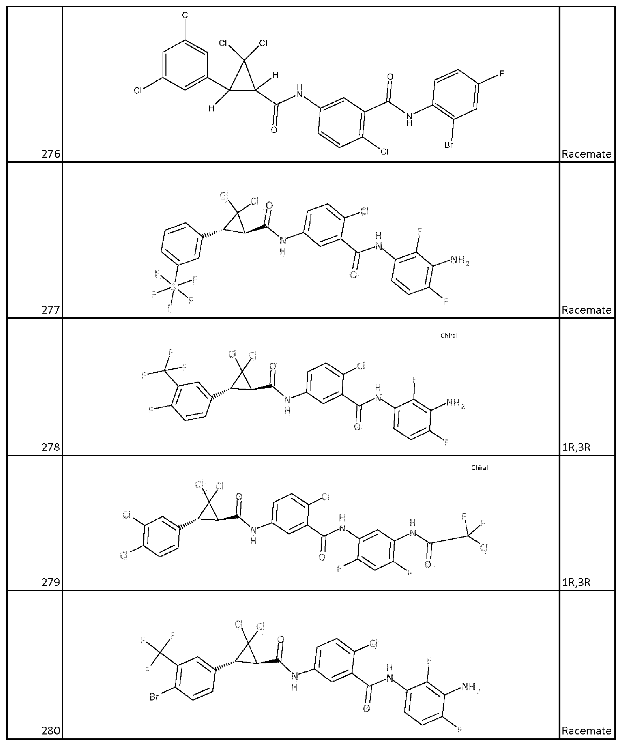

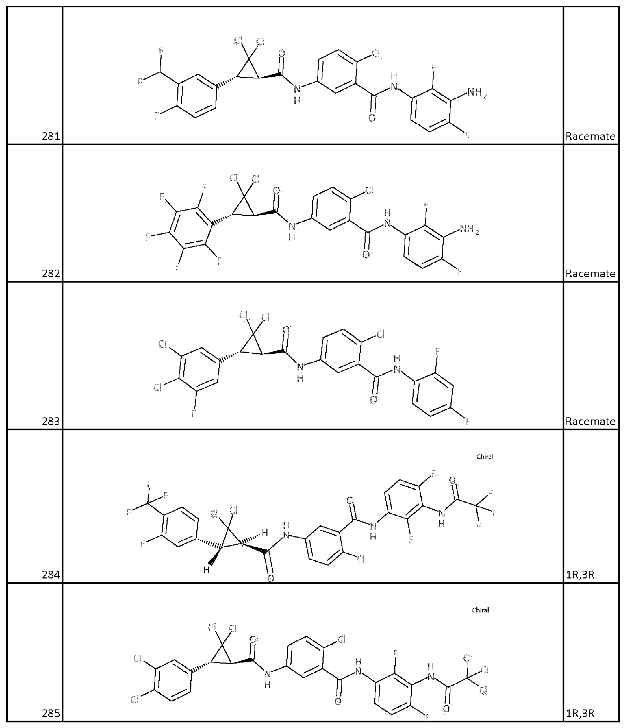

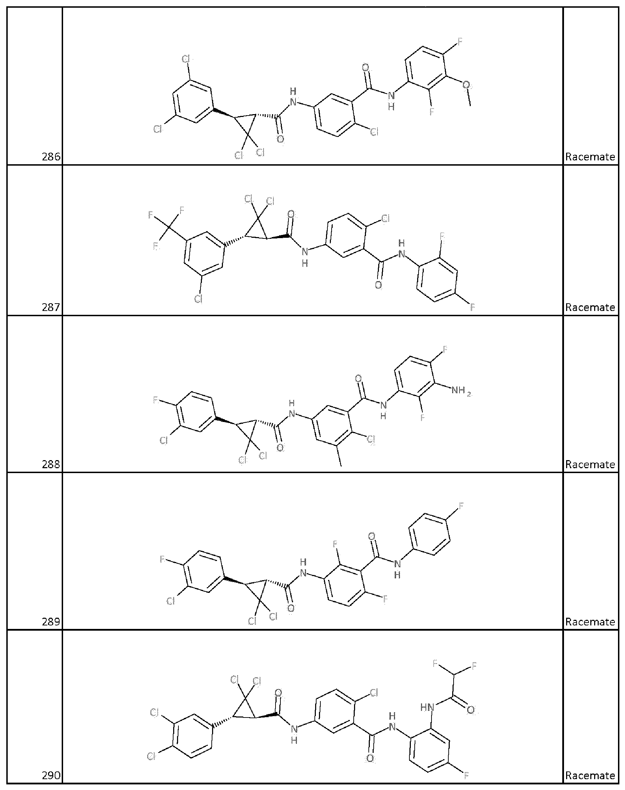

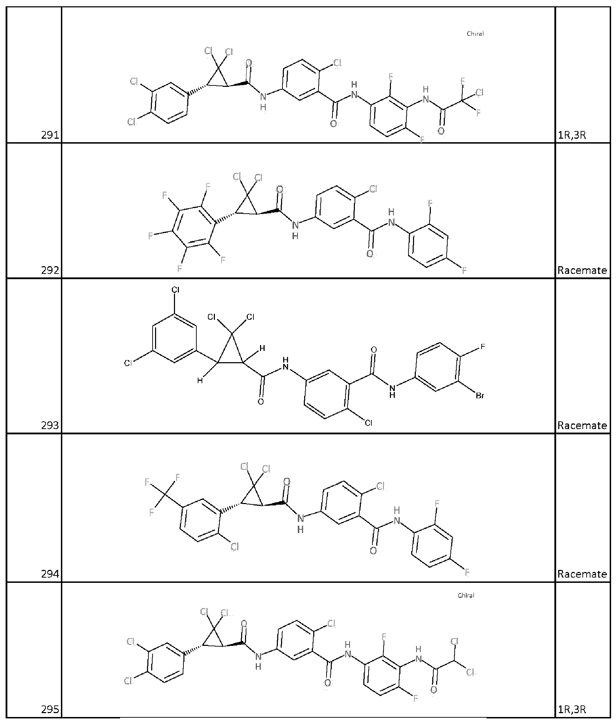

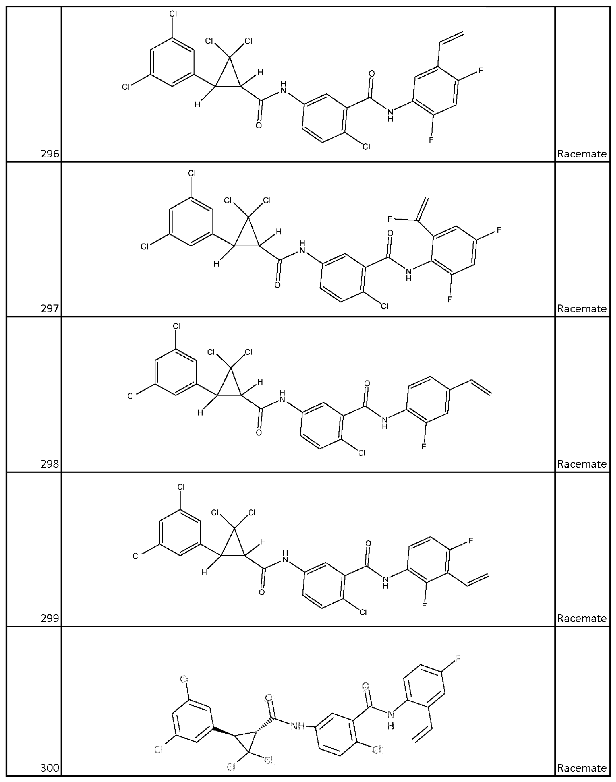

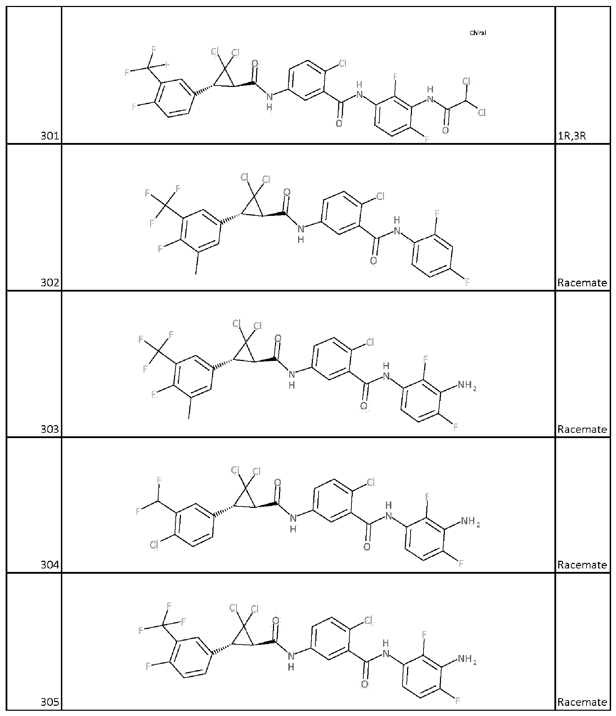

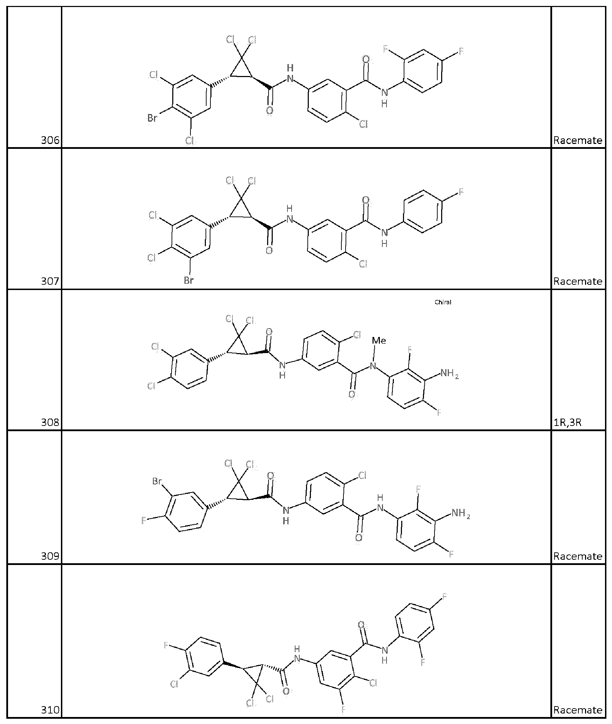

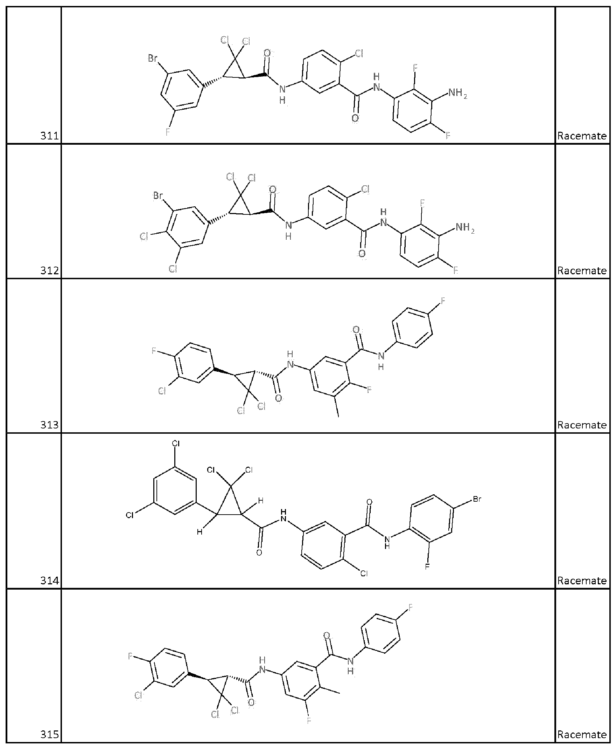

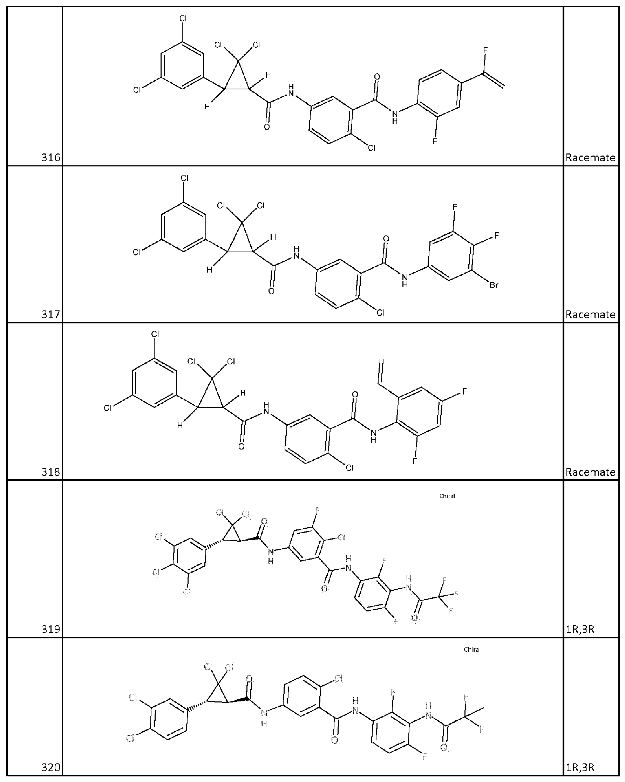

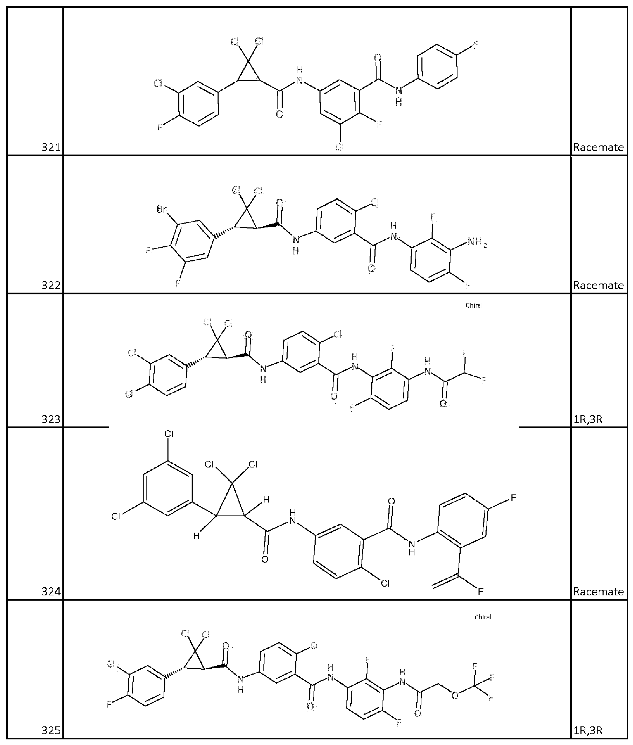

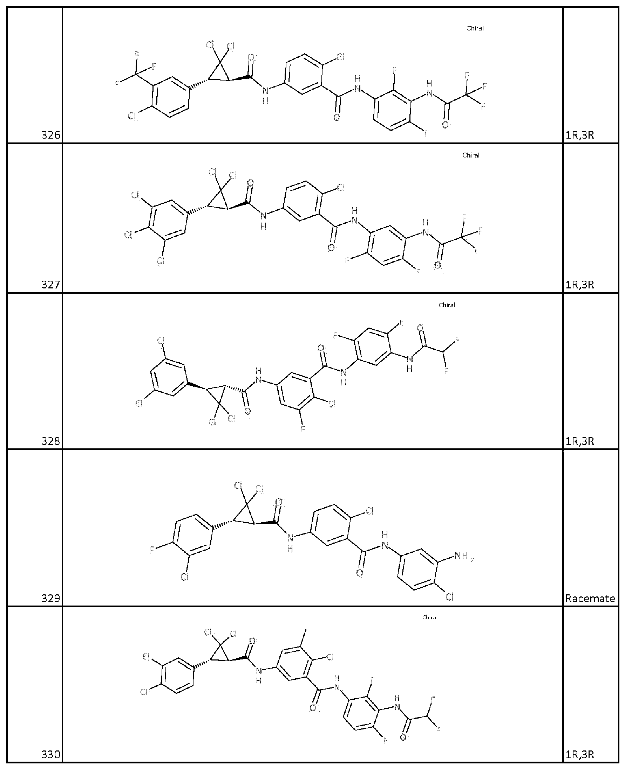

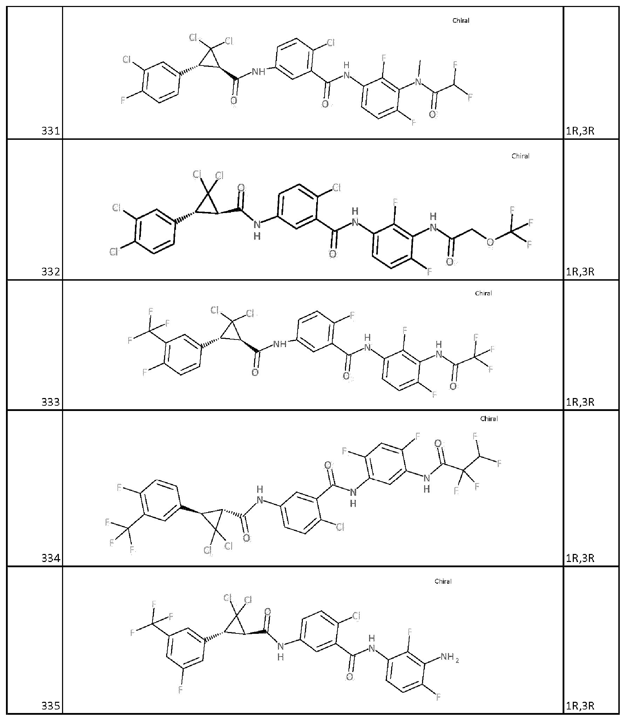

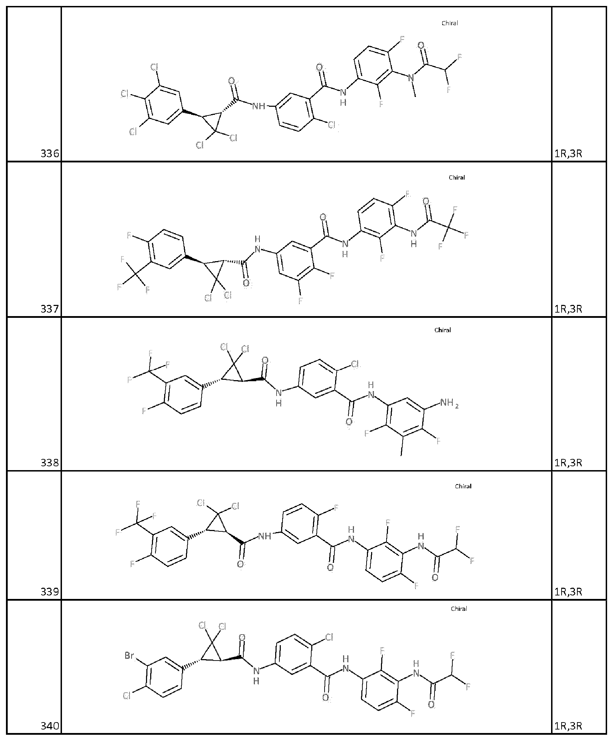

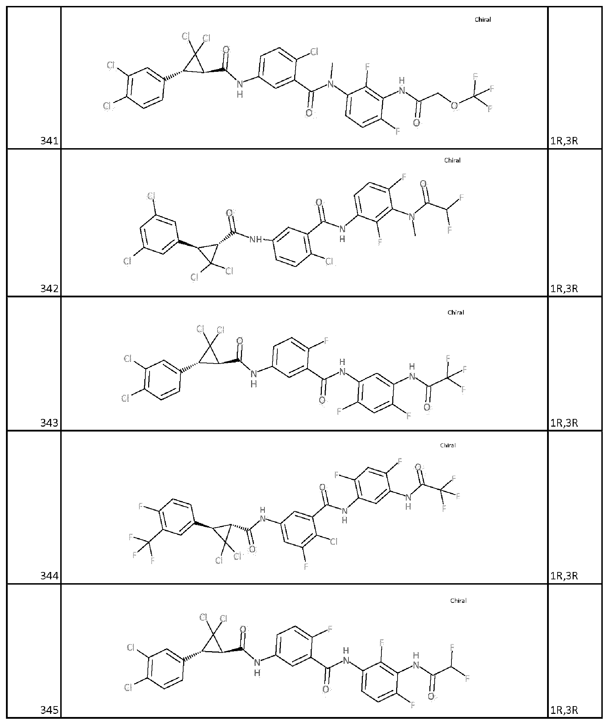

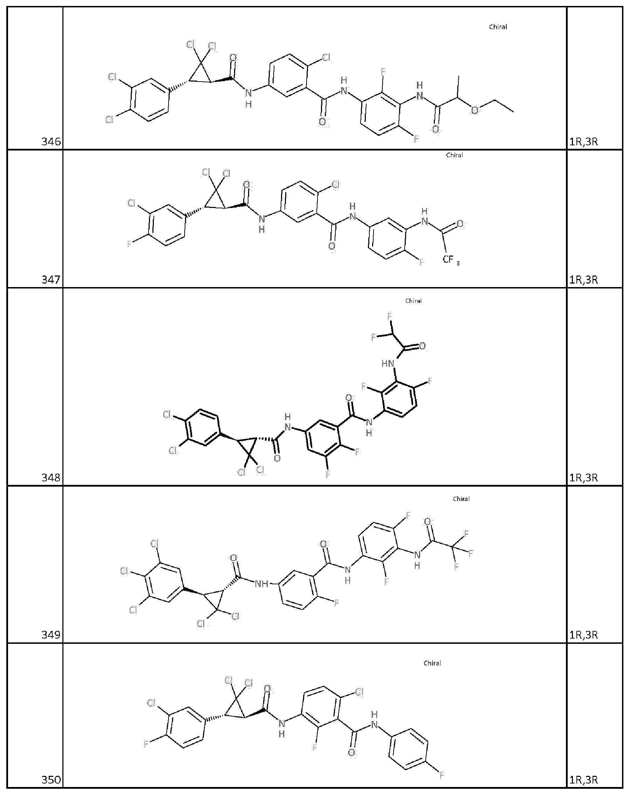

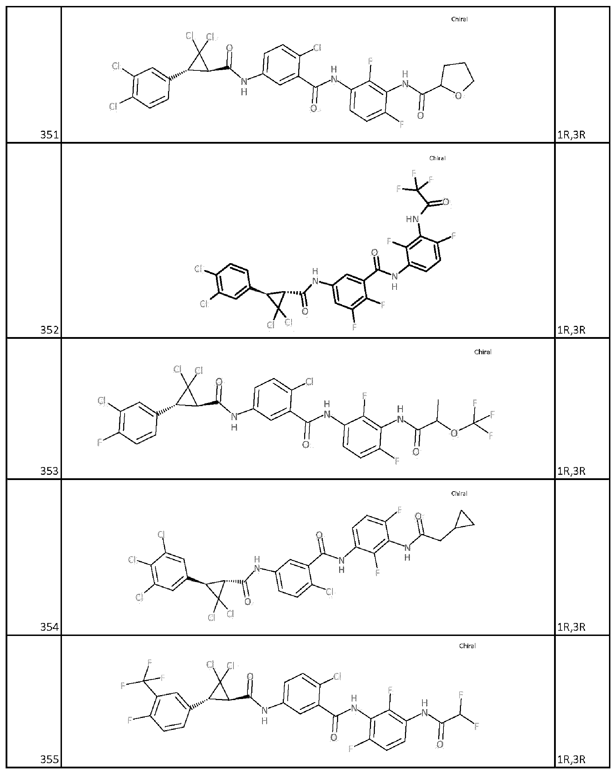

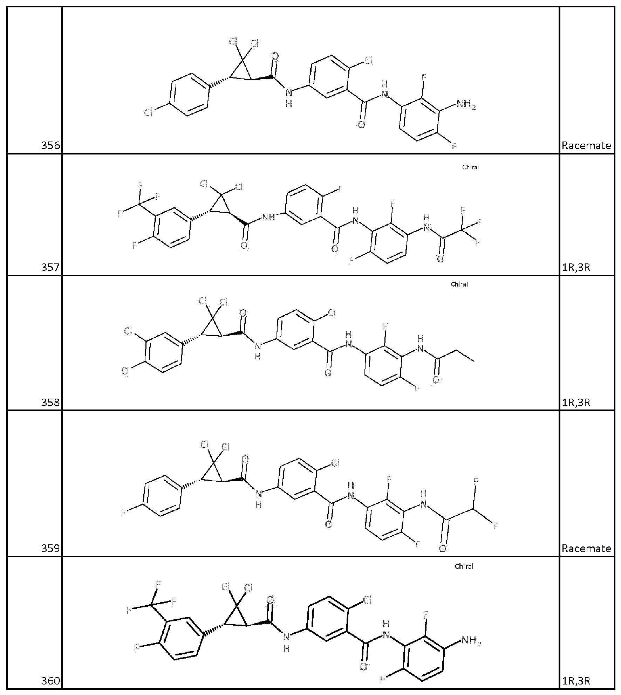

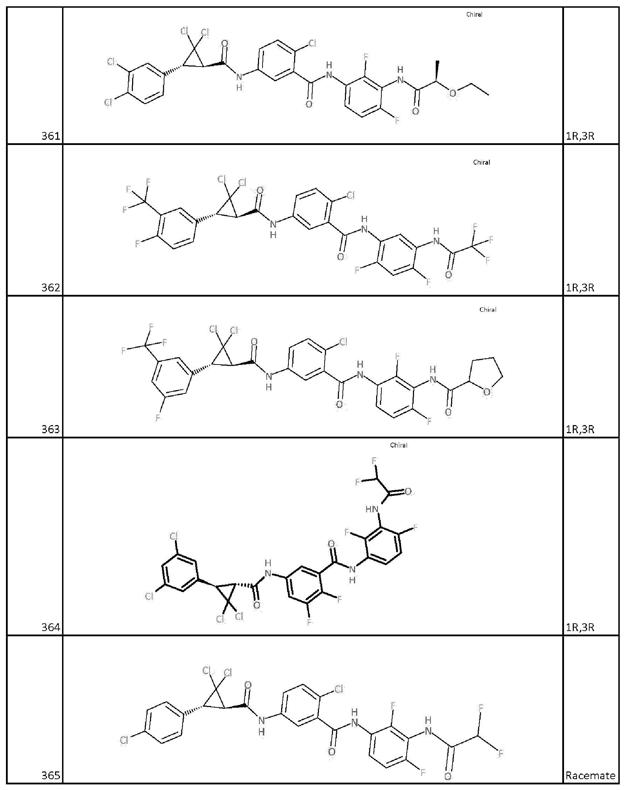

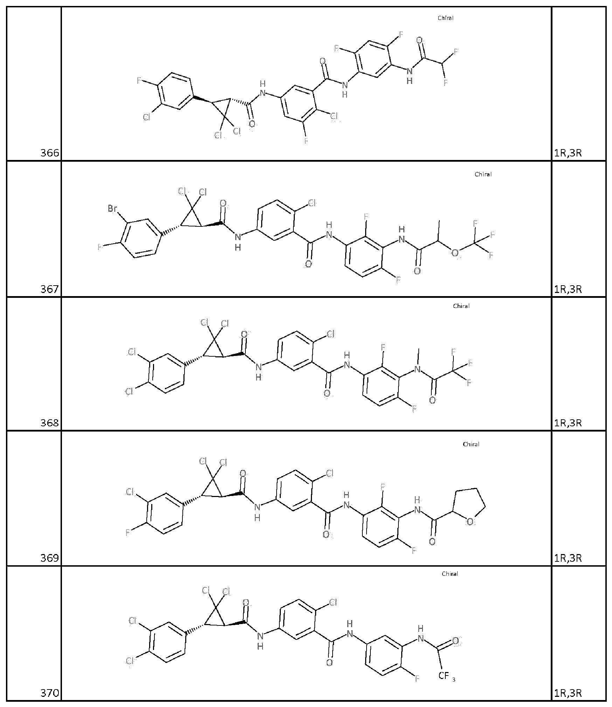

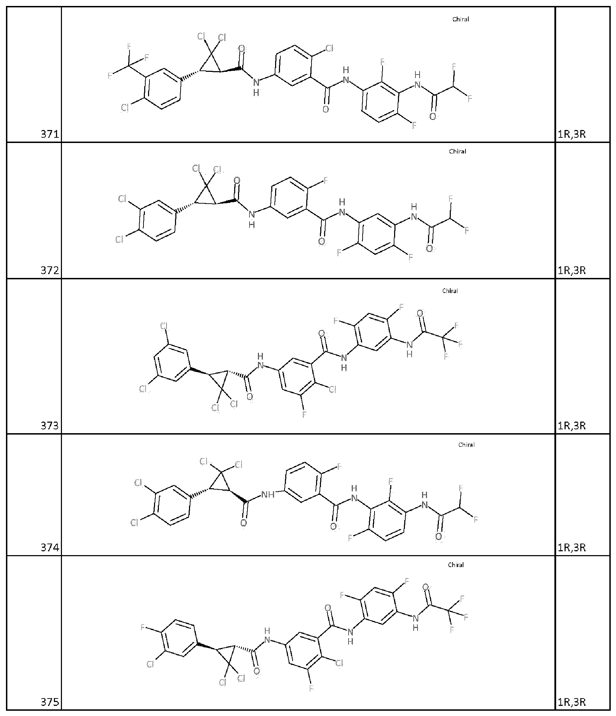

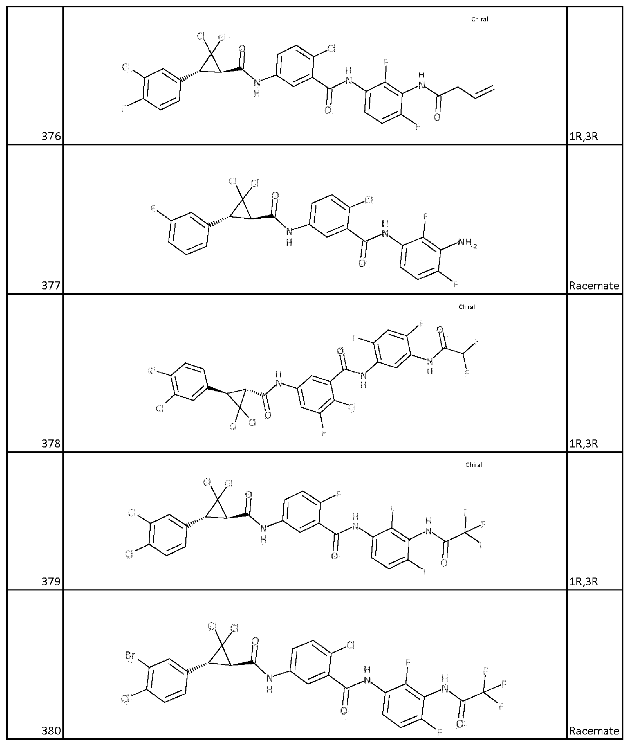

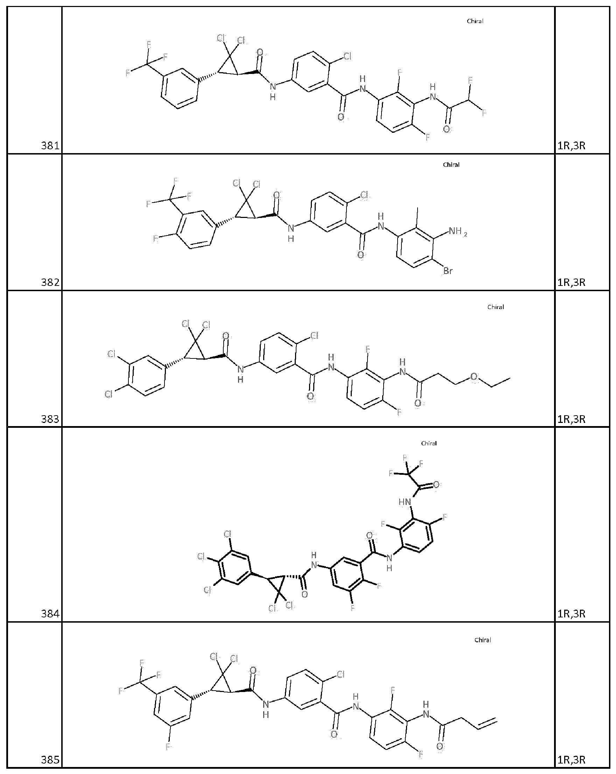

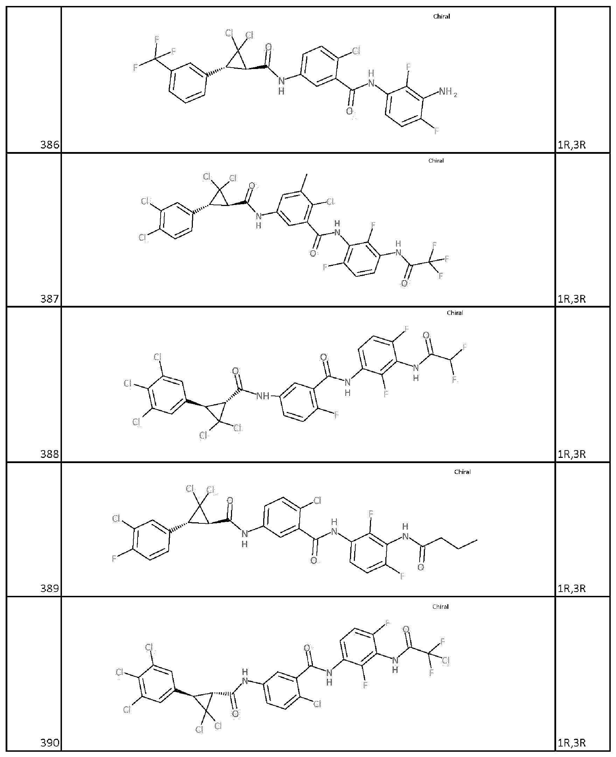

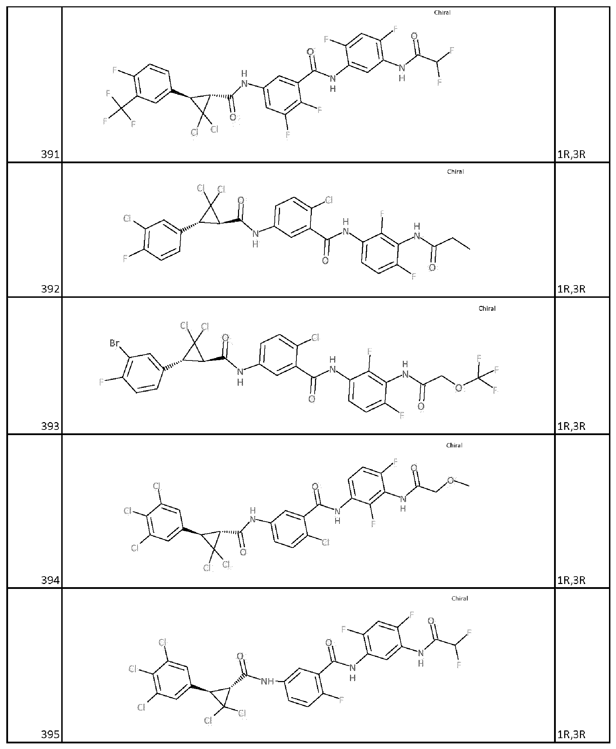

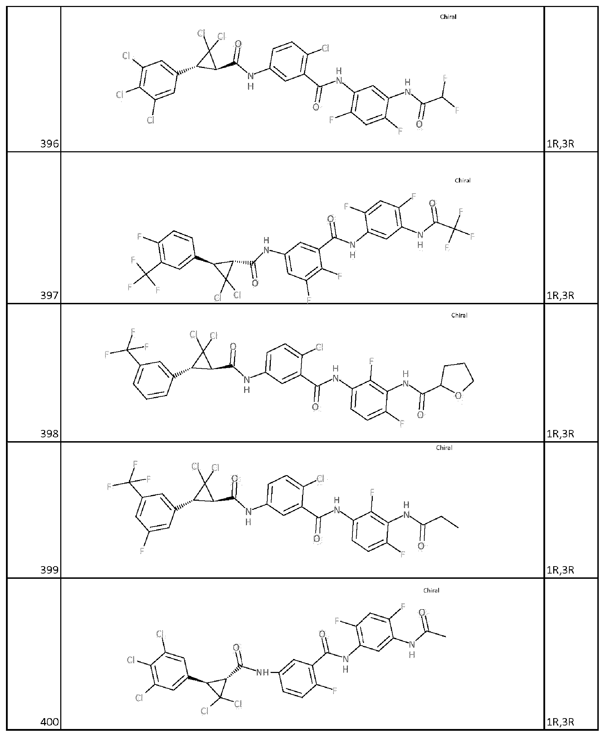

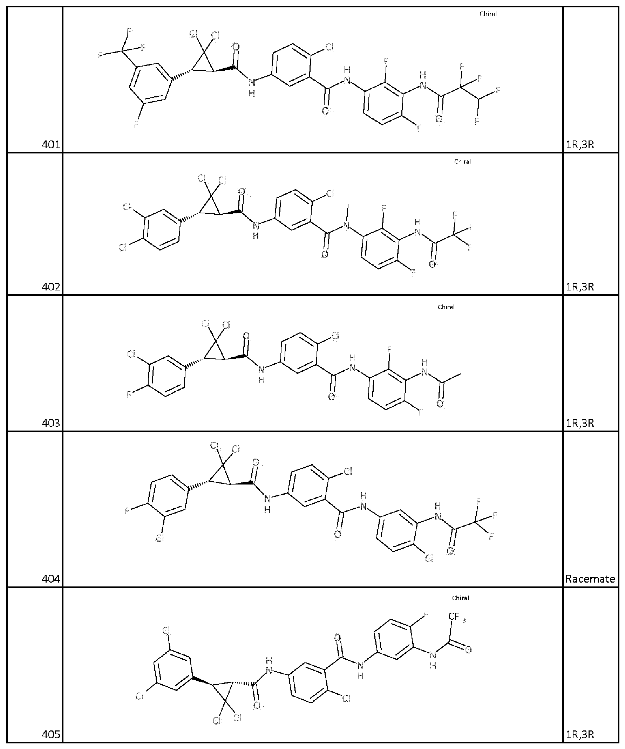

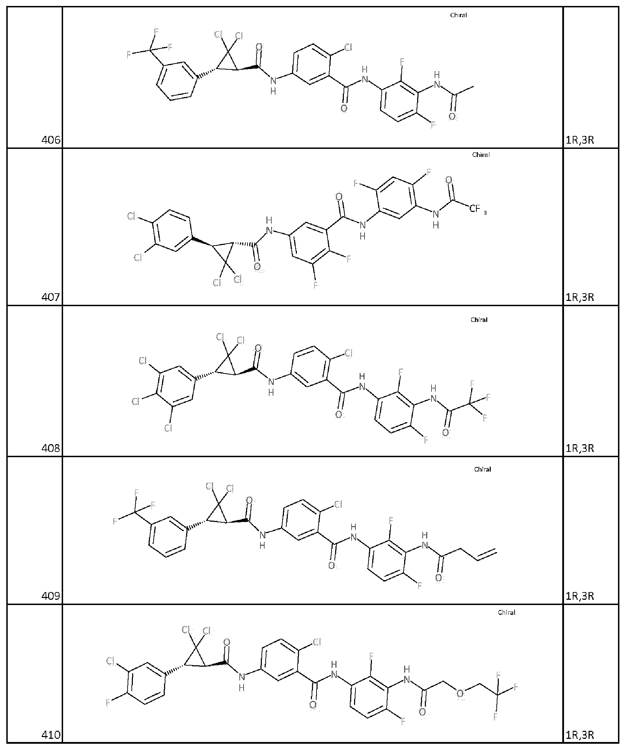

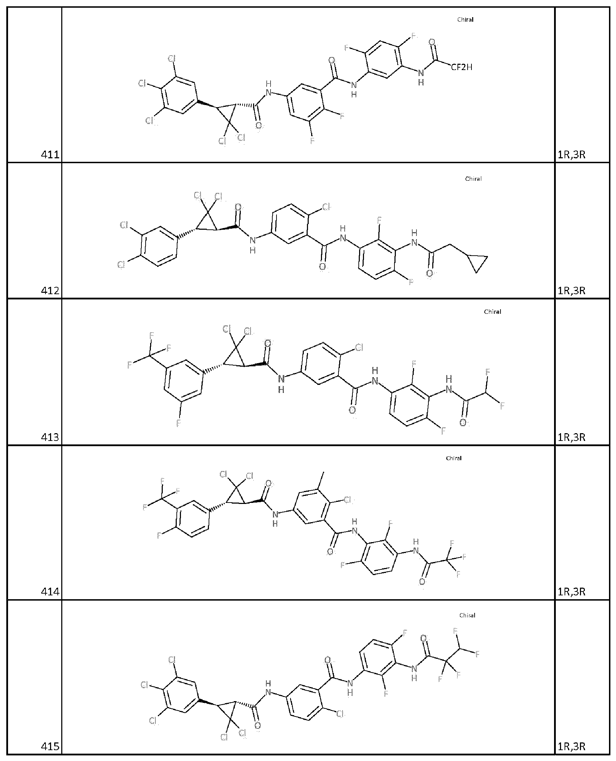

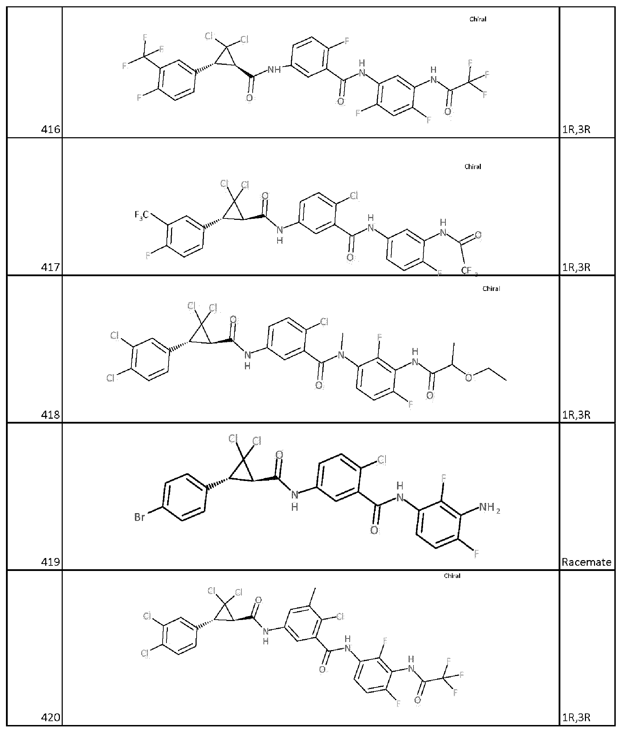

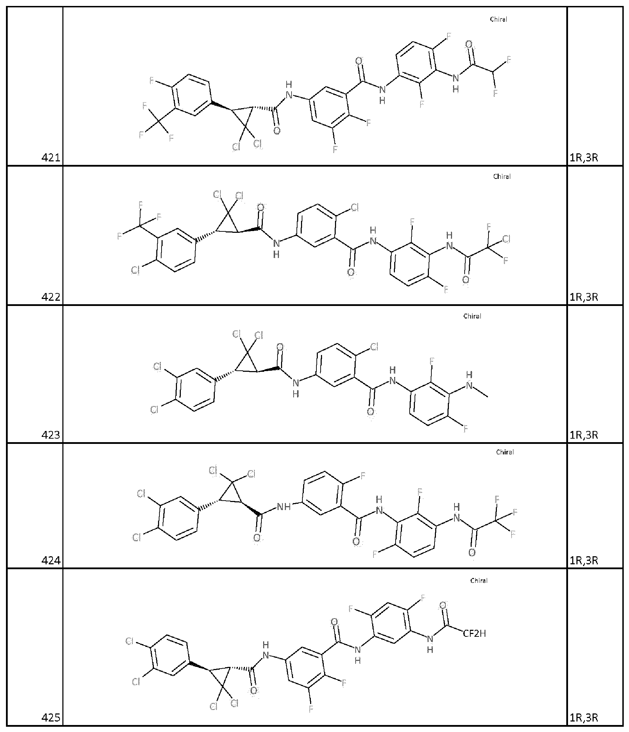

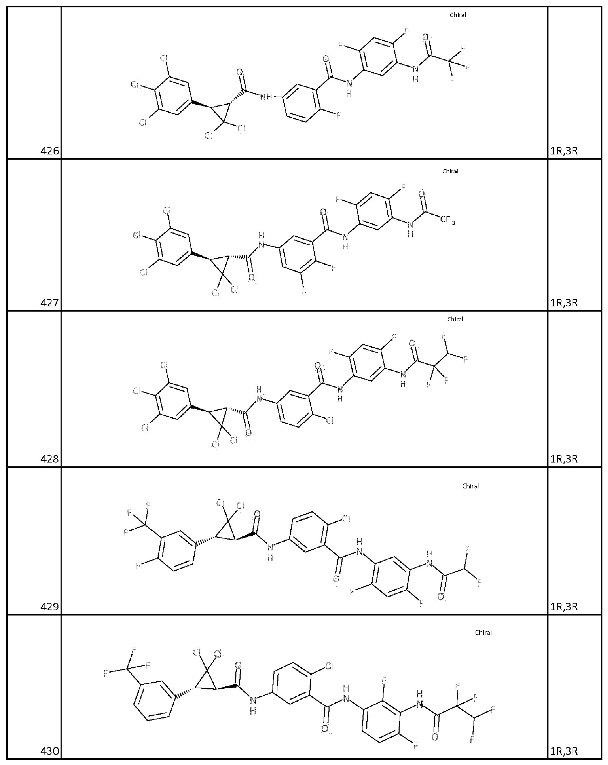

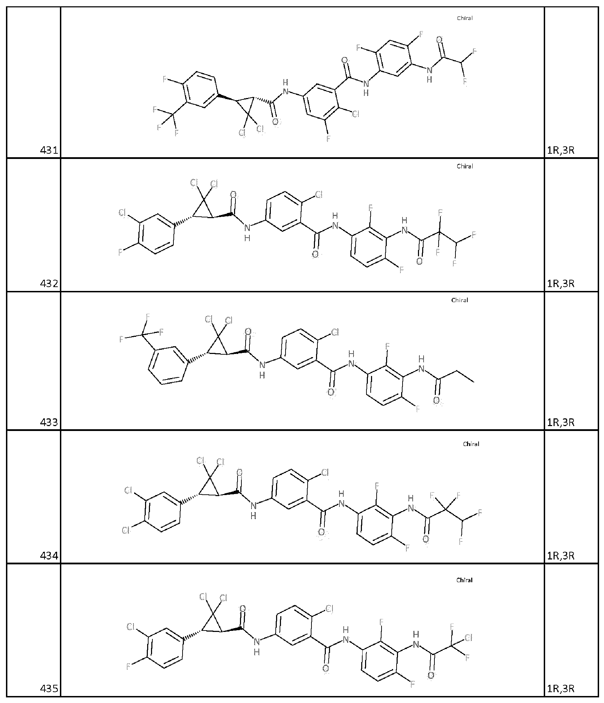

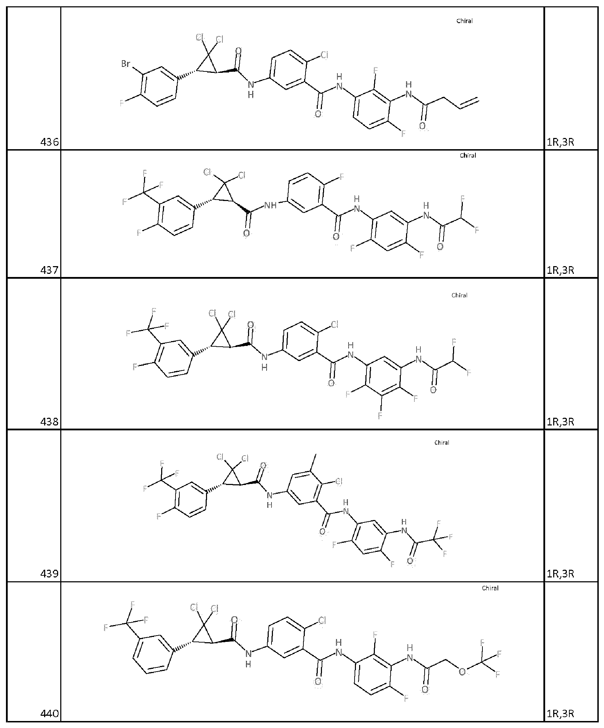

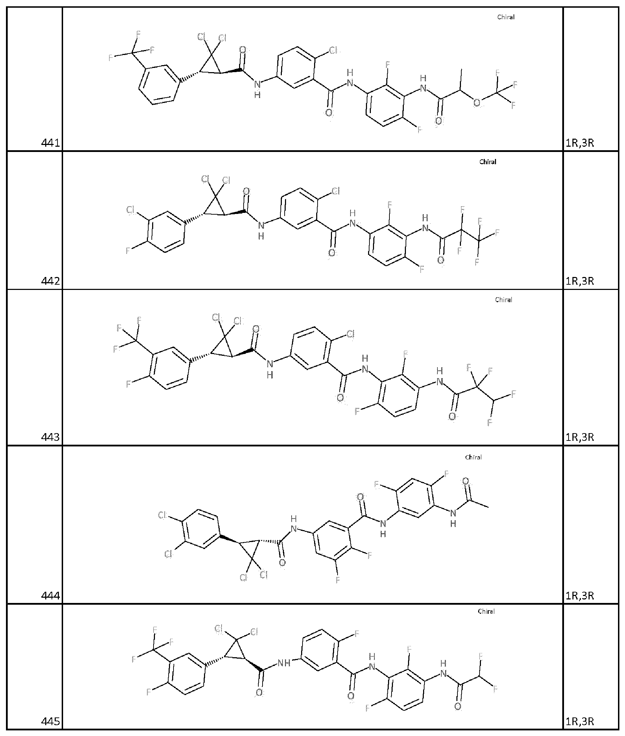

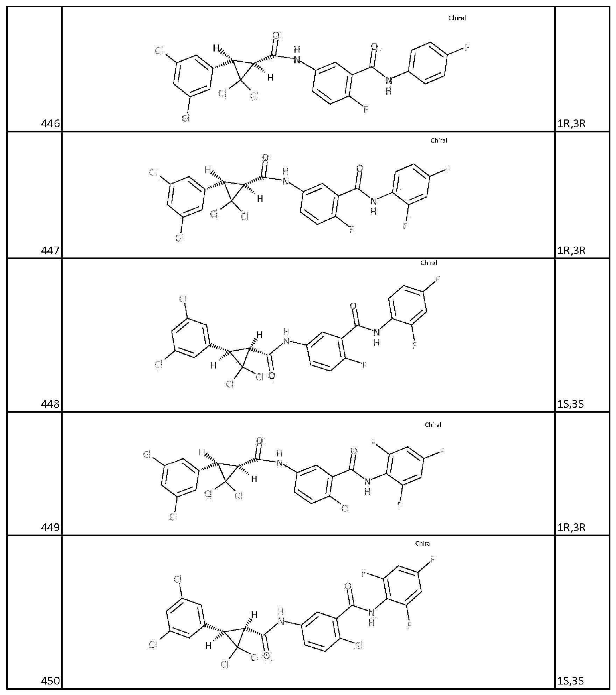

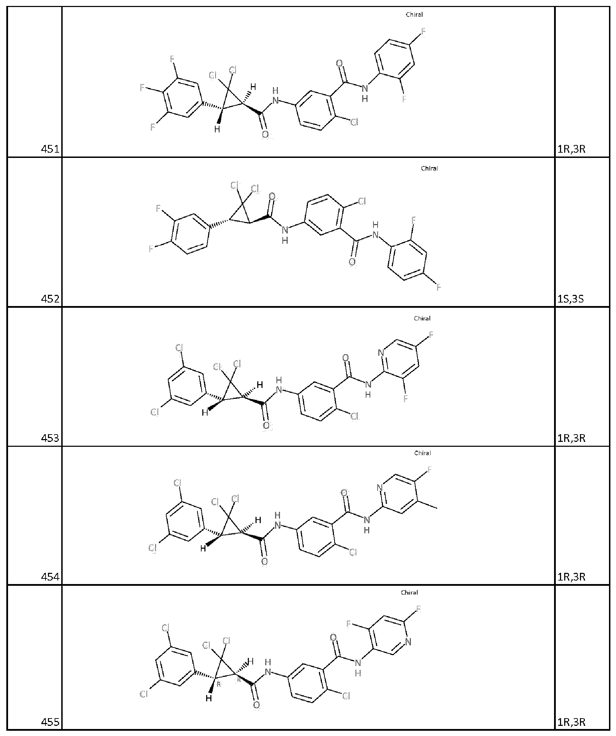

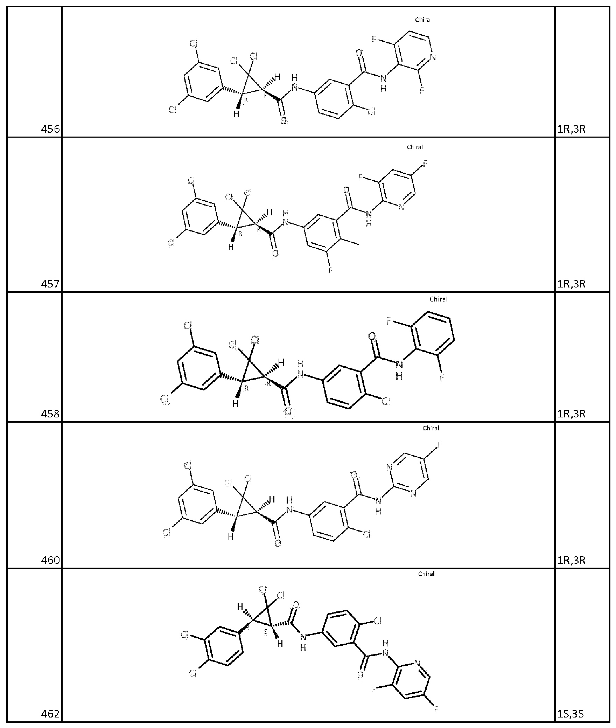

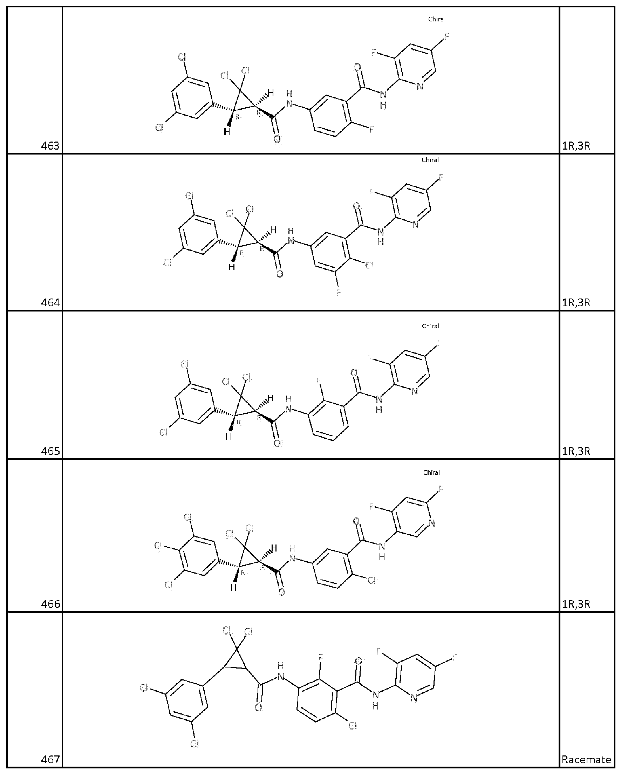

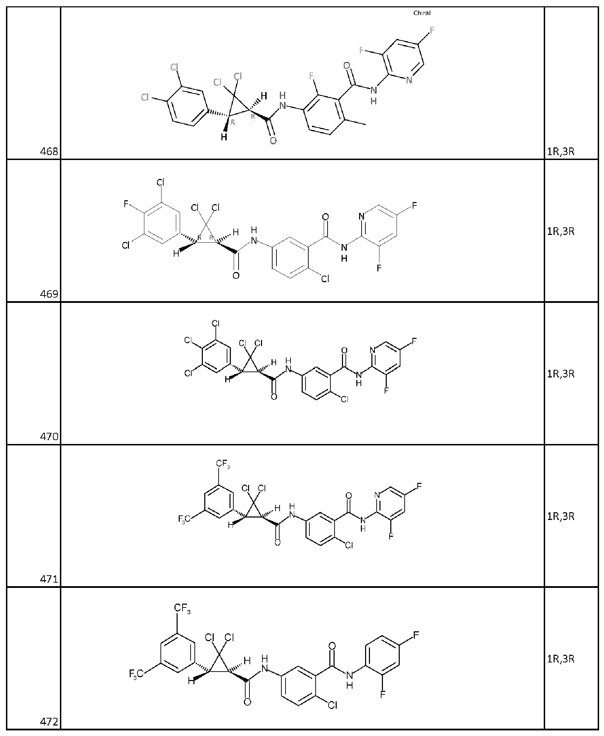

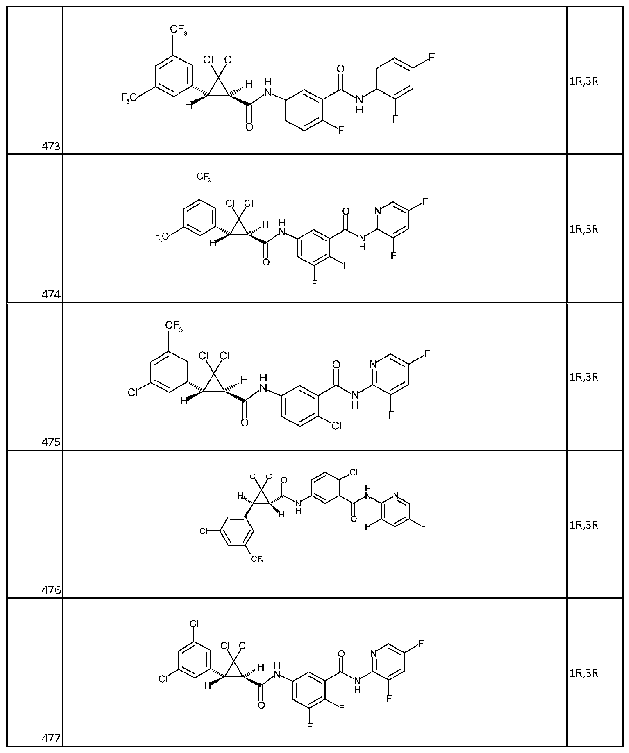

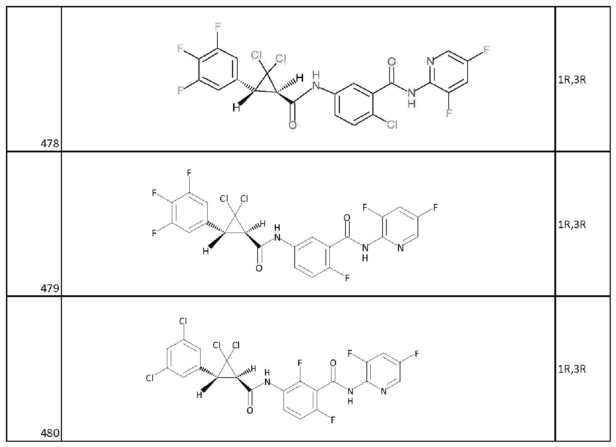

Structures of the compounds 1-480 are presented in figure 4. In case racemate is indicated but only one of the stereoisomer is drawn, also the other stereoisomer is intended.

### In vitro assays - ectoparasites

### Example 1: Tick larvae contact assay

Tick larvae (*Rhipicephalus microplus*) were immersed for approximately 3 minutes in declining concentrations of a test compound (dissolved in a DMSO-emulsifier-deionized water mixture). Tick inhibition (% of damage + dead ticks per test concentration) was assessed approximately 24 hours after contact exposure. The vitality of used parasites was confirmed in negative control groups (i.e. an untreated control and a solvent control).

Results: Compounds with activity of ≥ 80% inhibition at test concentration of 388 µM are active. (*indicates compounds with activity of ≥ 80% inhibition at test concentration of 49µM)

Compounds 1, 3*, 5*, 6*, 7, 8*, 9, 10, 11, 12, 13*, 14*, 15*, 16*, 17, 18*, 19*, 20*, 21, 22, 23*, 24, 25, 26*, 27, 28, 30*, 35*, 39*, 50*, 51*, 55*, 59*, 61*, 63*, 64*, 65*, 80*, 87*, 88*, 89*, 90*, 91*, 92, 94, 95*, 99*, 101*, 107*, 113*, 114*, 115*, 117, 120, 126*, 136*, 137*, 140, 145*, 147*, 151, 152, 160*, 165*, 168*, 170*, 173*, 179*, 183*, 185*, 197*, 199*, 201, 203* and 207*.

Compounds with tick activity of ≥ 80% inhibition at test concentration of 25 µM are active, i.e.:
Compounds 4, 19, 37, 51, 55, 56, 59, 63, 64, 65, 80, 87, 90, 91, 95, 99, 100, 114, 136, 145, 147, 149, 150, 160, 170, 173, 179, 185, 197, 209, 212, 215, 216, 221, 225, 227, 230, 252, 264, 265, 278, 281, 283, 284, 289, 292, 294, 302, 309, 310, 313, 321, 331, 333, 335, 352, 376, 379, 386, 423 and 432.

Compounds with tick activity of ≥ 80% inhibition at test concentration of 100 ppm are active, i.e.:
Compounds 1, 2, 3, 4, 5, 6, 7, 8, 10, 12, 13, 14, 15, 16, 17, 18, 19, 20, 21, 23, 24, 25, 26, 27, 35, 37, 46, 49, 50, 51, 55, 56, 59, 63, 64, 65, 69, 80, 81, 86, 87, 88, 89, 90, 91, 95, 99, 100, 101, 107, 113, 114, 115, 126, 136, 145, 147, 149, 150, 160, 169, 171, 173, 179, 183, 184, 185, 197, 200, 203, 209, 212, 213, 215, 216, 220, 221, 223, 225, 227, 230, 233, 252, 264, 265, 278, 281, 283, 284, 289, 292, 294, 302, 309, 310, 313, 321, 331, 335, 352, 376, 379, 386, 423, 432, 469, 470, 471, 472, 473, 474, 475, 477, 478, 479, and 480.

### Example 2: Flea contact assay

Adult fleas (*Ctenocephalides felis*) were exposed to filter paper impregnated with declining concentrations of a test compound (dissolved in a DMSO-emulsifier-deionized water mixture). After approximately 48 hours of continuous contact exposure, flea inhibition (% of dead + damaged fleas per test concentration) was assessed. The vitality of used parasites was confirmed in negative control groups (i.e., an untreated control and a solvent control).

Results: Compounds with flea activity of ≥ 80% inhibition at test concentration of 1000 ppm are active, i.e.: Compounds 55, 65, 90, 91, 99, 100, 114, 115, 145, 147, 179, 185, 197 289, 469, 470, 471, 472, 473, 474, 477, and 479.

### Example 3: Tick feeding assay

Tick nymphs (stage III, *Ornithodoros moubata*) were fed on artificial membranes with blood spiked with declining concentrations of a test compound (dissolved in a DMSO-emulsifier-blood mixture). Fed nymphs were incubated for around 2-3 weeks to enable tick molting into the next stage. Tick inhibition (i.e, % of damaged + dead ticks at molting-assessment per test concentration) was assessed. The vitality of used parasites was confirmed in negative control groups (i.e., an untreated control and a solvent control).

Results: Compounds with tick activity of ≥ 80% inhibition at test concentration of 1 ppm are active, i.e.: Compounds 1, 2, 3, 4, 5, 6, 7, 8, 10, 12, 13, 14, 15, 16, 17, 18, 19, 20, 21, 23, 24, 25, 26, 27, 469, 470, 471, 472, 473, 474, 475, 477, 478, 479 and 480.

### Example 4: Flea feeding assay

Adult fleas (*Ctenocephalides felis*) of defined age were fed on artificial membranes with blood spiked with declining concentrations of a test compound (dissolved in a DMSO-emulsifier-blood mixture). After approximately 48 hours of continuous feeding, flea inhibition (i.e., % of dead + damaged fleas per test concentration) was assessed. The vitality of used parasites was confirmed in negative control groups (i.e., an untreated control and a solvent control).

Results: Compounds with flea activity of ≥ 80% inhibition at test concentration of 1 ppm were found active, i.e.: Compounds 4, 15, 18, 19, 20, 21, 23, 24, 25, 64, 65, 80, 81, 86, 87, 88, 89, 90, 91, 95, 99, 100, 115, 145, 149, 150, 160, 173, 179, 183, 184, 185, 197, 200, 203, 209, 212, 213, 220, 221, 223, 227, 230, 252, 265, 278, 281, 289, 309, 313, 321, 331, 333, 335, 352, 376, 379, 386, 423, 432, 469, 470, 471, 472, 473, 474, 475, 477, 478, 479 and 480.

### Example 5: Fly larvae contact/feeding assay

Fly larvae I (*Lucilia cuprina*) were grown in medium spiked with declining concentrations of a test compound (dissolved in a DMSO-emulsifier-deionized water mixture). Inhibition of fly larvae development (%) was assessed after approximately 48 hours of continuous exposure. The vitality of used parasites was confirmed in negative control groups (i.e., an untreated control and a solvent control).

Results: Compounds with fly larvae activity of ≥ 80% inhibition at test concentration of 100 ppm were found active, i.e.: Compounds 3, 4, 5, 6, 8, 14, 18, 19, 20, 21, 26, 46, 49, 50, 51, 55, 56, 59, 63, 64, 65, 69, 80, 81, 86, 87, 88, 90, 91, 95, 99, 100, 101, 107, 113, 114, 115, 136, 145, 147, 149, 150, 169, 171, 173, 179, 183, 184, 185, 197, 200, 203, 209, 212, 213, 215, 216, 220, 221, 223, 227, 230, 233, 252, 264, 265, 278, 281, 284, 289, 294, 309, 310, 313, 321, 331, 335, 352, 376, 379, 386, 423, 432, 469, 470, 471, 472, 473, 474, 475, 476, 477, 478, 479 and 480.

### Example 6: Juvenile Sea lice contact assay

Copepodids (juvenile sea lice stage of *Lepeophtheirus salmonis)* were exposed to seawater spiked with declining concentrations of a test compound (dissolved in a DMSO sea-water mixture). Sea lice inhibition (% of dead + damaged copepodids) was assessed after approximately 24 hours of continuous exposure. The vitality of used parasites was confirmed in a negative (solvent) control group.

Results: The following compounds showed ≥80% inhibition at a test concentration of 100 nM (* denotes 100% inhibition): Compounds 1*, 4, 11*, 27*, 29*, 48*, 51, 63*, 68, 70*, 96*, 98*, 109, 111*, 112*, 124*, 132, 187*, 188*, 189*, 190*, 218*, 454*, 328*, 331*, 335*, 342*, 347*, 355, 364*, 367*, 369*, 370*, 371*, 373*, 375*, 377*, 386*, 405*, 432, 439, 461*, 465*, 466*, 467*, 468*, 470*, 473*, 474*, 480*.

Additionally, the following compounds showed ≥80% inhibition at a test concentration of 10 nM (* denotes 100% inhibition): Compounds 9, 86*, 89*, 447*, 449*, 455*, 456*, 457*, 458*, 459*, 460*, 464*, 469*, 471*, 472, 475*, 477, 478*, 479*.

Additionally, the following compounds showed ≥80% inhibition at a test concentration of 1 nM (* denotes 100% inhibition): Compounds 197*, 446*, 451*, 453*, 463*.

### Example 7: Adult sea lice contact assay

Mobile stages of sea lice (i.e., pre-adults, adults of *Lepeophtheirus salmonis*) were exposed to seawater spiked with declining concentrations of a test compound (dissolved in a PEG sea-water mixture). Sea lice inhibition (% of dead +damaged sea lice) was assessed after approximately 1 and 24 hours of continuous exposure in comparison to a negative (solvent) control group.

Results: The following compounds had an EC₅₀ >10 nM ≤ 25 nM: Compounds 4, 9, 197, 449, 464, 465.

Additionally, the following compound had an EC₅₀ ≤ 10 nM: Compounds 8, 86, 89, 446, 447, 451, 453, 454, 455, 456, 457, 458, 459, 460, 463, 466, 469, 471, 473, 474, 475, 477, 478, 479, 480.

### Example 8: Adult Caligus rogercresseyi sea lice contact assay

Mobile stages of sea lice (i.e., pre-adults, adults of *Caligus rogercresseyi*) were exposed to seawater spiked with declining concentrations of a test compound (dissolved in a PEG sea-water mixture). Sea lice inhibition (% of dead + damaged sea lice) was assessed after approximately 1, 24 and 48 hours of continuous exposure in comparison to a negative (solvent) and untreated control group.

Results: Compound 453 had an EC₅₀ of 0.072 ppb after 24 hours and 0.035 ppb after 48 hours.

### Example 9: Tick larvae resistance assay

A tick resistance assay (i.e., larval immersion test, LIT) was carried out using larvae of several *Rhipicephalus microplus* isolates with known resistance profiles (i.e.: fully susceptible; resistant to dieldrin, coumaphos, DDT, chlorpyriphos, flumethrin, cypermethrin, amitraz, fluazuron).

Tick larvae (*Rhipicephalus microplus*) were immersed for approximately 3 minutes in test compound solution. Tick mortality was assessed approximately 24 hours after contact exposure. The vitality of used parasites was confirmed in negative control groups (i.e., an untreated control and a solvent control).

| | **Compound 446** | **Compound 197** |
|---|---|---|
| **Isolate** | **LC50 (ppm)** | **LC50 (ppm)** |
| Fully susceptible | 0.344 | 0.288 |
| DDT, Dieldrin, Coumaphos | 0.31 | 0.346 |
| DDT, Dieldrin, Coumaphos, Chlorpyriphos | 0.463 | 0.335 |
| Coumaphos, Flumethrin, Cypermethrin | 0.353 | 0.343 |
| Coumaphos, Flumethrin, Cypermethrin, Amitraz | 0.48 | 0.375 |
| Dieldrin, Coumaphos, Fluazuron | 0.322 | 0.270 |

Results: Compounds 446 and 197 were equally effective against the sensitive isolate in comparison to all tested multi-resistant isolates

### Example 10: Sea lice resistance assay

A sea lice resistance assay was carried out using mobile sea lice stages (i.e., pre-adults, adults) of several *Lepeophtheirus* salmonis-isolates with known resistance profiles (i.e.: fully susceptible, resistant to azametiphos (OP), emamectin benzoate (ML), deltamethrin (SP). Sea lice were exposed to seawater spiked with declining concentrations of a test compound (dissolved in a PEG300 / sea-water mixture). Sea lice inhibition (% of dead+ damaged sea lice) was assessed after approximately 1, 24 and 48 hour/s of continuous exposure in comparison to a negative (solvent) control group.

Results: Compound 453 was equally effective against the sensitive isolate (EC₅₀ 1.20 ppb after 24 hours and 0.29 ppb after 48 hours) and the multi-resistant isolate (EC₅₀ 1.96 ppb after 24 hours and 0.64 ppb after 48 hours).

### In vivo assays - ectoparasites

### Example 11: Guinea pig - efficacy against argaside tick/bed bug

Guinea pigs were locally co-infested (using an infestation device on a shaved skin area) with juvenile soft ticks (*Ornithodoros moubata*) and bed bugs (*Cimex lectularius*) before the treatment day (Day 0) to provide a baseline-negative control value. Guinea pigs were treated orally (by gavage) or subcutaneously at a dose of 5 mg/kg bodyweight of a test compound in a formulation of 10% DMF/Emulsifier + 90% Aqua ad injectabilia.

Repeated local infestations with both parasites were performed at different time points after treatment. Within approximately 45 min after each infestation ten engorged ticks and ten engorged bed bugs were
collected and the percentage of dead and damaged parasites per species were assessed approximately 24 hours following collection. The efficacy was
calculated as % of inhibition 'killed + damaged' parasites in a treated group in comparison to the baseline-negative control (using a modified Schneider Orelli's formula).

Results: Activity of the test compounds at different evaluation time-points (≥80% inhibition were found active):

### Tick nymphs - oral administration

Compounds 4, 51, 86, 89, 197, 446, 447, 449, 451, 453, 454, 455, 456, 460, 466, 469, 470, 471, 472 and 475 showed immediate activity on Day 0.

Compounds 4, 51, 86, 197, 447, 451, 453, 466, 469, 470, 471 and 475 showed activity after 7 days.

Compounds 4, 86, 451, 453, 466, 469, 470, 471 and 475 showed activity after 10 days.

Compounds 4, 466, 470, 471, and 475 showed activity after 14 days.

Compounds 470, 471, and 475 showed activity after 17 days.

Compounds 471 showed activity after 21, 24, 28, 31 and 35 days.

### Tick nymphs - subcutaneous administration

Compounds 4, 51, 86, 89, 197, 446, 447, 449, 451, 453, 454, 455, 456, 460, 466, 469, 470, 471, 472 and 475 showed immediate activity on Day 0.

Compounds 4, 51, 86, 197, 446, 447, 449, 451, 453, 454, 466, 469, 470, 471 and 475 showed activity after 7 days.

Compounds 4, 51, 86, 197, 446, 447, 451, 453, 454, 469, 470, 471 and 475 showed activity after 10 days.

Compounds 4, 51, 446, 447, 469, 470, 471 and 475 showed activity after 14 and 17 days.

Compounds 470, 471 and 475 showed activity after 21, 24, 28 and 31 days.

Compounds 471 and 475 showed activity after 35 days.

### Bedbugs - oral administration

Compounds 4, 51, 86, 89, 197, 446, 447, 449, 451, 453, 454, 455, 456, 460, 466, 469, 470, 471, 472 and 475 showed immediate activity on Day 0.

Compounds 4, 51, 86, 197, 446, 447, 451, 453, 460, 466, 469, 470, 471 and 475 showed activity after 7 days.

Compounds 4, 86, 451, 453, 466, 469, 470, 471 and 475 showed activity after 10 days.

Compounds 4, 466, 470, 471 and 475 showed activity after 14 days.

Compound 466, 470, 471 and 475 showed activity after 17 days.

Compounds 470, 471 and 475 showed activity after 21, 24 and 28 days.

Compounds 471 showed activity after 31 and 35 days.

### Bed bugs - subcutaneous administration

Compounds 4, 51, 86, 89, 197, 446, 447, 449, 451, 453, 454, 455, 456, 460, 466, 469, 470, 471, 472 and 475 showed immediate activity on Day 0.

Compounds 4, 51, 86, 197, 446, 447, 451, 453, 460, 466, 469, 470, 471 and 475 showed activity after 7 days.

Compounds 4, 51, 86, 197, 446, 447, 451, 453, 466, 469, 470, 471 and 475 showed activity after 10 days.

Compounds 4, 51, 446, 466, 469, 470, 471 and 475 showed activity after 14 and 17 days.

Compounds 446, 469, 470, 471 and 475 showed activity after 21 days.

Compounds 446, 470, 471 and 475 showed activity after 24 days.

Compounds 470, 471 and 475 showed activity after 28 and 31 days.

Compounds 470 and 471 showed activity after 35 days.

Conclusion: The compounds showed efficacy against both parasites and were generally well tolerated in guinea pig.

Compounds 4, 86, 451, 453, 466 and 469 were 100% effective to control ticks and bed bugs at least until Day 10 using both administration routes.

Compounds 4, 470, 471, 475 were 100% effective to control ticks and bed bugs for at least one week using both administration routes.

Subcutaneous administration resulted in a longer duration of efficacy for the compounds 4, 51, 197, 446, 447, 454 and 455.

### Example 12: Dog - efficacy against tick/flea

Beagle dogs were infested (whole body) with 80 *Ctenocephalides felis* fleas and *50 Rhipicephalus sanguineus* or *Haemaphysalis elliptica* ticks (sex ratio 1:1) two days before the treatment day (Day 0), and repeatedly thereafter. Dogs of treatment groups were treated with 10 mg/kg bodyweight of a test compound orally (compound weighed in gelatin capsule) , topically (spot-on, DMA 92%/DEET 8%) or subcutaneously (PEG 200 90%/water for injection 10%).Dogs of the negative control group received only an empty gelatin capsule orally and the spot-on formulation excipients topically.

The efficacy was assessed approximately 48 hours after treatment (assessment of **therapeutic efficacy)** or 48 hours after each post-treatment infestation (assessment of **prophylactic efficacy)** by counting live ticks/fleas on dogs in a treatment group in comparison to live parasite counts in the negative control group (efficacy calculation using the Abbott's formula). Compounds with efficacy ≥80 percent were found active.

### Ticks - oral administration

Compounds 4, 197, 453 and 466 were active on D2 (therapeutic efficacy).

Compound 466 was active up to D16. Compound 453 was active up to D70. Compound 197 was active up to D198.

### Ticks - topical administration

Compounds 453 and 466 were active on D2 (therapeutic efficacy).

Compound 466 was active up to D30. Compounds 197 and 453 were active up to D70/72.

### Ticks - subcutaneous administration

Compound 4 was active on D2 (therapeutic efficacy).

Compound 4 was active up to D16.

### Fleas - oral administration

Compounds 4, 197, 453 and 466 were active on D2 (therapeutic efficacy).

Compounds 453 and 466 were active up to D70. Compound 197 was active up to D227.

### Fleas - topical administration

Compounds 197, 453 and 466 were active on D2 (therapeutic efficacy).

Compounds 453 and 466 were active up to D70. Compound 197 was active up to D100.

### Fleas - subcutaneous administration

Compound 4 was active on D2 (therapeutic efficacy).

Compound 4 was active up to D 86.

Conclusion: Compounds showed both therapeutic and prophylactic efficacy against fleas and ticks. They were generally well tolerated in dogs. Compounds 4, 197, 453 and 466 showed therapeutic efficacy against fleas and ticks in dogs.

Compounds 466, 197 and 453 showed prophylactic efficacy against fleas for at least 10 weeks after topical and oral administration.

Compounds 197 and 453 showed prophylactic efficacy against fleas and ticks for at least 10 weeks after topical and oral administration.

### Example 13: Cattle - efficacy against one-host tick

Cattle were infested (whole body) with up to 3000 *Rhipicephalus microplus* larvae two or three times per week before and after the treatment day (Day 0). Cattle of treatment groups were treated subcutaneously or topically (spray or pour-on) at a dose of 1 mg/kg bodyweight of a test compound. As subcutaneous formulation either 2-pyrrolidon 50%/Tween 80 50% or NMP 60%/PEG300 40% was used. As topical formulation either a spray solution (DMF/Emulsifier 2%/tap water 98%) (Compound 4) or a DMA 92%/DEET 8% pour-on solution (all other compounds) or a pour-on solution containing Imwitor 988 (20%) + Miglyol 840 (QS) (Compound 453 and Compound 466) or a pour-on solution containing Miglyol 840 (QS) (Compound 453) was used. Cattle of the negative control group received only the formulation excipients of the respective administration route.

Fully engorged female ticks dropping from cattle were collected daily after treatment and counted in treatment and control groups. Efficacy was expressed using the standard ADEQ analysis method (ADEQ calculation in accordance with WAAVP guidelines for evaluating the efficacy of acaricides against ticks on ruminants; Veterinary parasitology 136 (2006) 29-43). Compounds with efficacy ≥80 percent were found active/effective. Onset of efficacy means the first Day with efficacy ≥80 percent. Duration of efficacy means the last Day with efficacy ≥80 percent.

Results: Efficacy of the test compounds at different evaluation time-points was as follows:
Compound 4 - onset of efficacy on Day 3 after topical administration and on Day 2 after subcutaneous administration.

Compound 446 - onset of efficacy on Day 3 after topical administration.

Compound 447 - onset of efficacy on Day 4 after topical administration.

Compound 197 - onset of efficacy on Day 2 after topical administration and Day 3 after subcutaneous administration.

Compound 453 - onset of efficacy after topical (DMA 92%/DEET 8% pour-on solution) and subcutaneous administration on Day 3.

Compound 453 formulated as pour on in Imwitor 988 + Miglyol 840 - onset of efficacy on Day 1 after topical formulation.

Compound 453 formulated in as pour on in Miglyol 840 - onset of efficacy on Day 2 after topical formulation

Compound 466 formulated as pour on in Imwitor 988 + Miglyol 840 - onset of efficacy on Day 1 after topical administration

Compound 4 - duration of efficacy until Day 58 after topical administration (spray solution (DMF/Emulsifier 2%/tap water 98%) and until Day 38 after subcutaneous administration.

Compound 446 - duration of efficacy until Day 47 after topical administration.

Compound 447 - duration of efficacy at least until Day 57 after topical administration.

Compound 197 - duration of efficacy until Day 47 after topical administration and at least until Day 120 after subcutaneous administration.

Compound 453 - duration of efficacy until Day 63 after topical administration and until Day 82 after subcutaneous administration.

Compound 453 formulated as pour on in Imwitor 988 + Miglyol 840 - duration of efficacy until Day 77.

Compound 453 formulated in as pour on in Miglyol 840 - duration of efficacy until Day 84.

Compound 466 formulated as pour on in Imwitor 988 + Miglyol 840 - duration of efficacy until Day 87.

Conclusion: Compounds showed therapeutic efficacy (against existing infestation) and prophylactic efficacy (against re-infestation) against one-host ticks on cattle. They were generally well tolerated in cattle.

### Example 14: Cattle - efficacy against multi-host ticks

Cattle were treated subcutaneously or topically (pour-on) at a dose of 5 mg/kg bodyweight of a test compound (Day 0). As subcutaneous formulation NMP 60%/PEG300 40% was used, and as pour-on formulation DMA 92%/DEET 8% was used. Cattle of the negative control group received only the formulation excipients of both formulations (SC, pour-on).

Cattle were locally infested with 30 to 40 unfed adult ticks each
of *Rhipicephalus appendiculatus* and *Dermacenter variabilis* (for both species sex ratio 1:1), by placing the ticks in an ear sleeve (*R. appendiculatus*) or a stockinet patch attached to the body side (*D. variabilis*) once before treatment, and at different time points after treatment. Free (i.e., non-attached ticks) were removed from ear sleeves/stockinet patches two days after each infestation and were excluded from the efficacy calculation.

The efficacy (for both tick species) was assessed two days after treatment (assessment of therapeutic efficacy) or three days after each post-treatment infestation (assessment of prophylactic efficacy) by counting of live ticks collected from cattle in a treatment group in comparison to live parasite counts in the negative control group (efficacy calculation using the Abbott's formula). Compounds with efficacy ≥80 percent were found active.

### Results: Dermacenter variabilis - topical administration

Compounds 197, 446, 447 and 453 showed therapeutic efficacy on Day 2.

Compounds 197, 446, 447 and 453 showed prophylactic efficacy when re-infested after 7 days.

Compounds 197, 446 and 453 showed prophylactic efficacy when re-infested after 14 days.

Compounds 197, 446 and 453 showed prophylactic efficacy when re-infested after 21 days.

Compounds 446, 447 and 453 showed prophylactic efficacy when re-infested after 28 days.

Compounds 446 and 453 showed prophylactic efficacy when re-infested after 35 days.

### Dermacenter variabilis - subcutaneous administration

Compounds 197, 446, 447 and 453 showed therapeutic efficacy on Day 2.

Compounds 197, 446, 447 and 453 showed prophylactic efficacy when re-infested after 7 days.

Compounds 197, 446 and 453 showed prophylactic efficacy when re-infested after 14 days.

Compounds 197, 446 and 453 showed prophylactic efficacy when re-infested after 21 days.

Compounds 197, 446, 447 and 453 showed prophylactic efficacy when re-infested after 28 days.

Compound 453 showed prophylactic efficacy when re-infested after 35 days.

### Rhipicephalus appendiculatus - topical administration

Compounds 197, 447 and 453 showed therapeutic efficacy on Day 2.

Compounds 197, 446, 447 and 453 showed prophylactic efficacy when re-infested after 7 days.

Compounds 197, 446, 447 and 453 showed prophylactic efficacy when re-infested after 14 days.

Compounds 197, 446 and 453 showed prophylactic efficacy when re-infested after 21 days.

Compounds 197, 446, 447 and 453 showed prophylactic efficacy when re-infested after 28 days.

Compounds 197, 447 and 453 showed prophylactic efficacy when re-infested after 35 days.

### Rhipicephalus appendiculatus - subcutaneous administration

Compounds 197, 446, 447 and 453 showed therapeutic efficacy on Day 2.

Compounds 197, 446, and 453 showed prophylactic efficacy when re-infested after 7 days.

Compounds 197, 446 and 453 showed prophylactic efficacy when re-infested after 14 days.

Compounds 197, 446, 447 and 453 showed prophylactic efficacy when re-infested after 21 days.

Compounds 197 and 453 showed prophylactic efficacy when re-infested after 28 days.

Compounds 197 and 453 showed prophylactic efficacy when re-infested after 35 days.

Conclusion: Compounds showed both therapeutic and prophylactic efficacy against multi- host ticks on cattle. They were generally well tolerated in cattle.

Compounds 197, 447 and 453 were 100% effective in treating existing tick infestations using both administration routes.

### Example 15: Salmon - in vivo efficacy against sea lice using gavage administration:

Salmon (*Salmo salar*) were treated once orally by gavage at a dose of 5 mg/kg bodyweight per fish (Day 0). As formulation DMSO 5%/fish oil 95% was used. Salmon of the negative control group received only the excipients of the formulation.

The fish were infested with *Lepeophtheirus salmonis* copepodids twice before treatment (i.e., around day -28 and day -3) and once after treatment (i.e., day 17).

At day 7, fish were examined under sedation and (pre-)adult sea lice on each fish were counted and removed (assessment of **therapeutic efficacy against (pre-)adult sea lice** from day -28 infestation).

Around day 28 (end of animal phase), fish were euthanized, and lice on each fish were classified ((pre-)adult or juvenile stage) and counted. The number of (pre-) adult and juvenile parasite stages corresponding to the respective infestation time point were used for efficacy calculation, i.e., assessment of **therapeutic efficacy against juvenile sea lice** from day -3 infestation and assessment of **prophylactic efficacy** from day 17 re-infestation. Efficacy is expressed as a % reduction of sea lice in treated groups versus a control group, using the Abbott's formula.

Results: The following compounds had a therapeutic efficacy of ≥ 80 % against (pre-)adult sea lice (* denotes 100% efficacy): Compounds 86*, 89*, 197, 449, 451, 453* 459*, 460*.
86*, 89*, 197, 449, 451, 453*, 455*, 456, 457, 458*, 459*, 460*, 463*, 464*, 466, 478*, 479*, 480*.

The following compounds had a therapeutic efficacy of ≥ 80% against juvenile sea lice (* denotes 100% efficacy): Compounds 86*, 89*, 449, 459*, 460*.
86*, 89*, 449, 455*, 456*, 457*, 458*, 459*, 460*, 463*, 464*, 466, 478, 479, 480*.

The following compounds had a prophylactic efficacy of ≥ 80% at re-infestation on Day 17 (* denotes 100% efficacy): Compounds 453*, 456, 458, 464*.

### Example 16: Salmon - in vivo efficacy against sea lice using in-feed administration

Four study groups of fish (one group for assessment of therapeutic efficacy, one for assessment of prophylactic efficacy and their respective controls) were used. Commercial fish feed pellets were coated with formulated test compound. As formulation DMSO 5%/fish oil 95% was used. Salmon (*Salmo salar*) were treated orally with 1 mg/kg fish biomass via coated feed for 7 consecutive days. Salmon of the negative control groups received only feed coated with the excipients of the formulation.

Fish of the therapeutic groups were infested with *Lepeophtheirus salmonis* copepodids twice before treatment (around day -28 and day -3). Around day 8, fish were examined under sedation and (pre) adult sea lice on each fish were counted and removed (assessment of **therapeutic efficacy against (pre-)adult sea lice** from around day -28 infestation). Around day 25, fish were euthanized and (pre-) adult sea lice on each fish were counted (assessment of **therapeutic efficacy against juvenile sea lice** from around day -3 infestation.

Fish of the prophylactic groups were infested at several time points after treatment in 2-week intervals. The fish were assessed for (pre-)adult sea lice approximately 3 weeks after each re-infestation. At each assessment, all (pre-)adult sea lice were removed from the fish. The number of sea lice on fish corresponding to the respective re-infestation time point were used for **prophylactic efficacy** calculation. Efficacy is expressed as a % reduction of sea lice in treated groups versus a control group using the Abbott's formula.

Results: For Compound 453 100% therapeutic efficacy against (pre-)adult and juvenile sea lice was achieved. Against re-infestation after treatment, prophylactic efficacies were 100% (D14), 99.9% (D28) and 86.0% (D42).

### Example 17 Topical Pour-on

All formulations were prepared by weighing the desired amount of either Compound 453 or Compound 466 into a volumetric flask and adding approximately 80% of solvent 1 and all of any additional solvents used or other additives used. The flask was then heated while stirring to a temperature between 50 and 80 °C until the solution was free of any visible solids and then allowed to cool to room temperature. Finally the remainder of solvent 1 was added to the fill line on the volumetric flask.

The table below shows all of the compositions used in the Examples 18 to 20 :

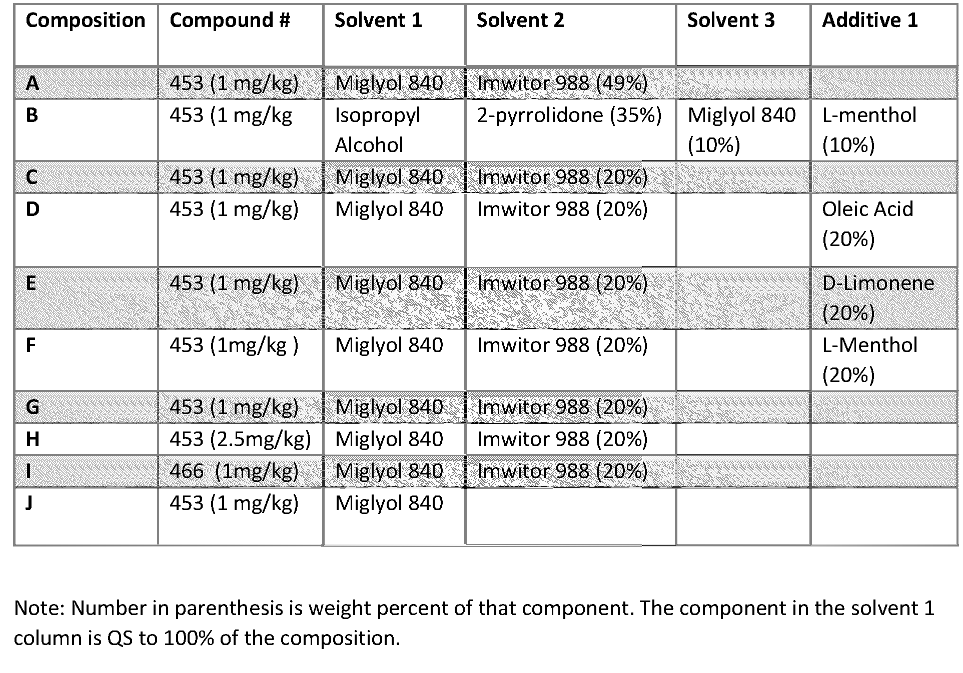

### Example 18

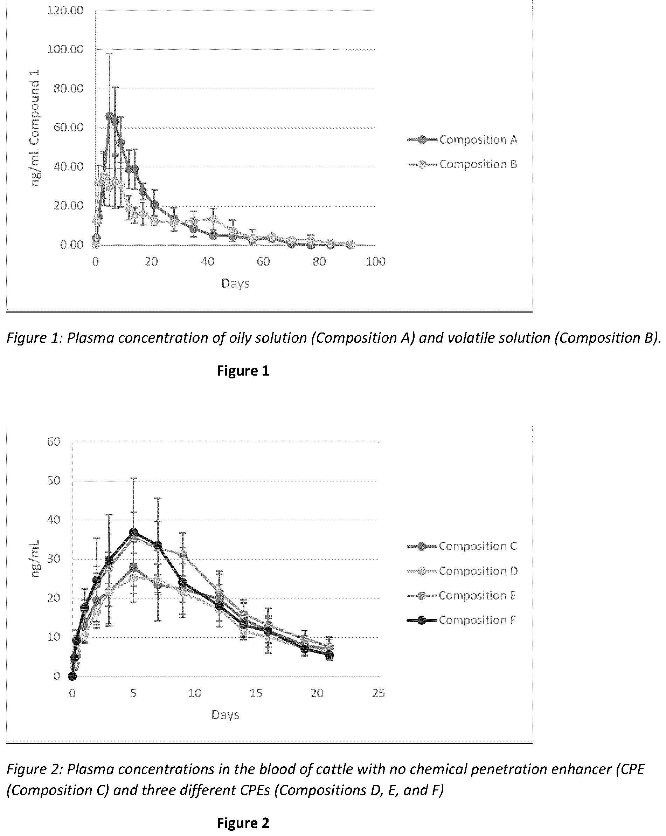

Composition A and composition B were each applied to 4 cattle and the concentration of compound 1(Compound 453) in their blood was monitored over 91 days. Figure 1 shows how, contrary to many commercially available topical solutions, Compound 1 (453) does not require rapid solvent evaporation on application to have good maximum absorbance (Cmax) and total absorbance (AUC).

*Figure 1* *shows that an oily solution (Composition A) had higher Cmax and AUC values of than a volatile solution (Composition B)*.

### Example 19

Compositions C, D, E, and F were each applied to 4 cattle and the concentration of compound 453 in their blood was monitored over 2 days.The data in Figure 2 below support the claim that no chemical penetration enhancer (CPE) is needed. It is clear that while several of these have slightly higher absorbance and Cmax values, these are not significant improvements and are not enough to impact the overall performance of the compound.

*Figure 2* shows a *comparison of concentrations of Compound 453 in the blood of cattle with no CPE (Composition C) and three different CPEs (Compositions D, E, and F) showing no significant improvement with the inclusion of any CPE.*

### Example 20

Cattle were infested (whole body) with up to 3000 *Rhipicephalus microplus* larvae two or three times per week before and after the treatment day (Day 0). Cattle of treatment groups were treated topically (pour-on) with the compositions G, H, I and J as disclosed in Example 1. Cattle of the negative control group received only the formulation excipients of the respective administration route.

Fully engorged female ticks dropping from cattle were collected daily after treatment and counted in treatment and control groups. Efficacy was expressed using the standard ADEQ analysis method (ADEQ calculation in accordance with WAAVP guidelines for evaluating the efficacy of acaricides against ticks on ruminants; Veterinary parasitology 136 (2006) 29-43). Compositions with efficacy ≥80 percent were found active/effective. Onset of efficacy means the first Day with efficacy ≥80 percent. Duration of efficacy means the last Day with efficacy ≥80 percent.

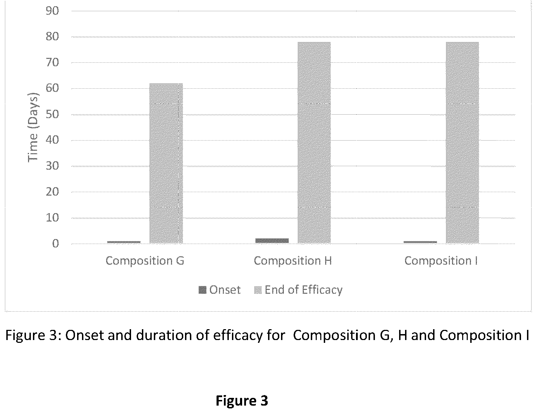

Figure 3 shows the duration of efficacy >90% against *R. microplus* and the onset of efficacy>90% against *R.microplus.*

### Example 21

### In vitro test of rain fastness

In addition to good absorbance, the composition according to the invention has a high resistance to water. After applying a 5% solution of Compound 453 in Miglyol 840 to rabbit hide and allowing to sit for 4 hours, the sample was soaked in water for 30 minutes. After this there was 4% weight loss and the assaying the water for the presence Compound 453 showed that it was below the detection limit.

### COMPARATIVE EXAMPLES:

### Compounds that showed activity in prior art plant protection bioassays but not in animal health parasite assays

Compounds have been tested in prior art documents WO2016/168059= D1 and WO2018/071327 =D2 against a range of plant protection agrochemical relevant parasites. Surprisingly it has been found that a number of compounds from D1 and D2 that were active in plant protection assays did not show activity against animal health parasites in Example 1 described above which is the most sensitive of the assays. The Table below shows the compounds that were not active in against animal health parasites in example 1 with the reference number in such prior art documents and the activity of these compounds in the plant protection bioassays as described below. In particular, these comparative compounds as can be seen, do not have a halogen on the ring formed by A₁-A₅.

### Plant protection assays

Bioassays against Beet Armyworm (*Spodoptera exigua*), Cabbage Looper (*Trichoplusia ni*) are in the plant protection are a two good indicator species for a broad range of chewing pests. Yellow Fever Mosquito (*Aedes aegypti*) bioassay is an indicator for sucking pests, including blood sucking pests.

### Bioassays on Beet Armyworm BAW (Spodoptera exigua) ("Test A"), and Cabbage Looper CL (Trichoplusia ni) ("Test B")

Bioassays on BAW: Bioassays on BAW were conducted using a 128-well diet tray assay. One to five second instar BAW larvae were placed in each well (3 mL) of the diet tray that had been previously filled with approximately 1.5 mL of artificial diet to which 50 µg/cm² of the test molecule (dissolved in 50 µL of 90:10 acetone-water mixture) had been applied (to each of eight wells) and then allowed to dry. Trays were covered with a clear self-adhesive cover, vented to allow gas exchange, and held at 25 °C, 14:10 light-dark for five to seven days. Percent mortality was recorded for the larvae in each well; activity in the eight wells was then averaged. The results are indicated in the table below.

Bioassays on CL: Bioassays on CL were conducted using a 128-well diet tray assay. one to five second instar CL larvae were placed in each well (3 mL) of the diet tray that had been previously filled with 1 mL of artificial diet to which 50 µg/cm² of the test molecule (dissolved in 50 µL of 90:10 acetone-water mixture) had been applied (to each of eight wells) and then allowed to dry. Trays were covered with a clear self-adhesive cover, vented to allow gas exchange, and held at 25 °C, 14:10 light-dark for five to seven days. Percent mortality was recorded for the larvae in each well; activity in the eight wells was then averaged. The results are indicated in the table below using the following Test A & B Rating table.

| **Test A & B Rating Table** | |
|---|---|
| **% Control (or Mortality)** | **Rating** |
| **50-100** | **A** |
| **More than 0 - Less than 50** | **B** |
| **Not Tested** | **C** |
| **No activity noticed in this bioassay** | **D** |

### Bioassays on Yellow Fever Mosquito (Aedes aegypti, AEDSAE) ("Test C").

Master plates containing 400 µg of a molecule dissolved in 100 µL of dimethyl sulfoxide (DMSO) (equivalent to a 4000-ppm solution) are used. A master plate of assembled molecules contains 15 µL per well. To this plate, 135 µL of a 90:10 water/acetone mixture is added to each well. A robot is programmed to dispense 15 µL aspirations from the master plate into an empty 96-well shallow plate ("daughter" plate). There are 6 reps ("daughter" plates) created per master. The created "daughter" plates are then immediately infested with YFM larvae.

The day before plates are to be treated, mosquito eggs are placed in Millipore water containing liver powder to begin hatching (4 g. into 400 mL). After the "daughter" plates are created using the robot, they are infested with 220 µL of the liver powder/larval mosquito mixture (about 1 day-old larvae). After plates are infested with mosquito larvae, a non-evaporative lid is used to cover the plate to reduce drying. Plates are held at room temperature for 3 days prior to grading. After 3 days, each well is observed and scored based on mortality. The results using the following Test C Rating table are indicated in the table below.

| **Test C Rating Table** | |
|---|---|
| **% Control (or Mortality)** | |
| **80-100** | **Rating A** |
| **More than 0 - Less than 80** | **B** |
| **Not Tested** | **C** |
| **No activity noticed in this bioassay** | **D** |

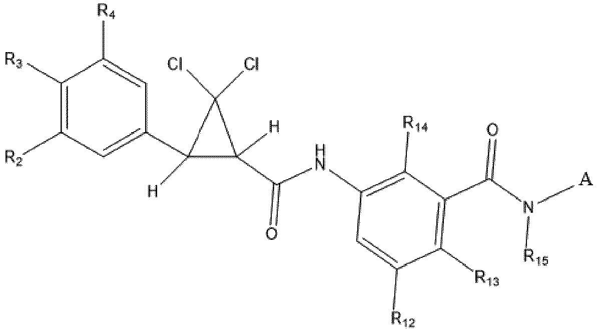

WO2016/168059= D1
WO2018/071327 =D2

| **#** | **R₂** | **R₃** | **R₄** | **R₁₂** | **R₁₃** | **R₁₄** | **R₁₅** | **A** | **A** | **B** | **C** | **WO** | **comp ref** |
|---|---|---|---|---|---|---|---|---|---|---|---|---|---|
| c1 | Cl | H | Cl | H | Cl | H | H | 4-imidazol-1-ylphenyl | A | A | A | D1 | PF56 |
| **c2** | Cl | H | Cl | H | Cl | H | H | 4-(1,2,4-triazol-1-yl)phenyl | A | A | A | D1 | PF57 |
| **c3** | Cl | H | Cl | H | Cl | H | H | 2,6-dimethyl-4-[1,2,2,2-tetrafluoro-1-(trifluoromethyl)ethyl] phenyl | A | A | D | D1 | PF60 |
| **c4** | Cl | H | Cl | H | Cl | H | H | 4-(cyclopropanecarbonyl amino)phenyl | A | A | A | D1 | PF107 |
| **c5** | Cl | H | Cl | H | Cl | H | H | 4-[(2-cyclopropylacetyl)amin o]phenyl | A | A | A | D1 | PF163 |
| **c6** | Cl | H | Cl | H | Cl | H | H | 4-[[1-(methoxymethyl)cyclo propanecarbonyl]amin o]phenyl | A | A | A | D1 | PF161 |
| **c7** | Cl | H | Cl | H | Cl | H | H | 4-[(2,2-difluorocyclopropanec arbonyl)amino]phenyl | A | A | A | D1 | PF108 |
| **c8** | Cl | H | Cl | H | Cl | H | H | 4-(3,3-dimethylbutanoylamin o)phenyl | A | A | D | D1 | PF164 |
| **c9** | Cl | H | Cl | H | Cl | H | H | 4-(1,2,4-triazol-4-yl)phenyl | A | A | A | D1 | PF102 |
| **c10** | Cl | H | Cl | H | Cl | H | H | 4-acetamido-2-methyl-phenyl | A | A | A | D1 | PF158 |
| **c11** | Cl | H | Cl | H | Cl | H | H | 1,3-benzothiazol-6-yl | A | A | A | D1 | F434 |
| **c12** | Cl | H | Cl | H | Cl | H | H | 1H-indol-5-yl | A | A | A | D1 | F435 |
| **c13** | Cl | H | Cl | H | Cl | H | H | 7-quinolyl | A | A | B | D1 | F436 |
| **c14** | Cl | H | Cl | H | Cl | H | H | 1H-pyrrolo[3,2-b]pyridin-6-yl | A | A | B | D1 | F437 |
| **c15** | Cl | H | Cl | H | Cl | H | H | 2-chloro-3H-benzimidazol-5-yl | A | A | B | D1 | F438 |
| **c16** | Cl | H | Cl | H | Cl | H | H | 4-(methoxycarbonylamin o)phenyl | A | A | B | D1 | PF110 |
| **c17** | Cl | H | Cl | H | Cl | H | H | 5-(trifluoromethyl)-1H-pyrazol-3-yl | A | A | D | D1 | PF138 |
| **c18** | Cl | H | Cl | H | Cl | H | H | 4-pyrazol-1-ylphenyl | A | A | A | D1 | F440 |
| **c19** | Cl | H | Cl | H | Cl | H | H | 2-Benzthiazolyl | A | A | B | D1 | F441 |
| **c20** | Cl | OMe | Cl | H | Cl | H | H | 4-acetamidophenyl | A | A | D | D1 | F181 |
| **c21** | Cl | OMe | Cl | H | Cl | H | H | 4-(methylamino)phenyl | A | A | D | D1 | F182 |
| **c22** | Cl | H | Cl | H | Cl | H | H | 1H-tetrazol-5-yl | A | A | B | D1 | PF130 |
| **c23** | Cl | H | Cl | H | Cl | H | H | 4-oxazol-2-ylphenyl | A | A | D | D1 | F446 |
| **c24** | Cl | H | Cl | H | Cl | H | H | 4-(p-tolyl)thiazol-2-yl | A | A | D | D1 | PF136 |
| **c25** | Cl | H | Cl | H | Cl | H | H | 2,4-dioxo-1H-pyrimidin-5-yl | A | A | B | D1 | PF139 |
| **c26** | Cl | H | Cl | H | Cl | H | H | 2-Benzoxazolyl | A | A | A | D1 | F447 |
| **c27** | Cl | H | Cl | H | Cl | H | H | 1,3-benzoxazol-6-yl | A | A | A | D1 | F450 |
| **c28** | Cl | H | Cl | H | Cl | H | H | benzothiophen-5-yl | A | A | A | D1 | F452 |
| **c29** | Cl | H | Cl | H | Cl | H | H | 3-aminophenyl | A | A | A | D1 | F201 |
| **c30** | Cl | H | Cl | H | Cl | H | H | 3-(methylamino)phenyl | A | A | A | D1 | F202 |
| **c31** | Cl | H | Cl | H | Cl | H | H | 3-amino-4-methyl-phenyl | A | A | B | D1 | F205 |
| **c32** | Cl | H | Cl | H | Cl | H | H | 2-methyl-1H-indol-5-yl | A | A | A | D1 | F454 |
| **c33** | Cl | H | Cl | H | Cl | H | H | 2-methyl-1,3-benzothiazol-5-yl | A | A | A | D1 | F455 |
| **c34** | Cl | H | Cl | H | Cl | H | H | 1H-benzimidazol-5-yl | A | A | B | D1 | F456 |
| **c35** | Cl | H | Cl | H | Cl | H | H | 2-methyl-4-(methylamino)phenyl | A | A | A | D1 | F196 |
| **c36** | Cl | H | Cl | H | Cl | H | H | 1-methylindol-5-yl | A | A | A | D1 | F457 |
| **c37** | Cl | H | Cl | H | Cl | H | H | 1-benzylindol-5-yl | A | A | D | D1 | F458 |
| **c38** | Cl | H | Cl | H | Cl | H | H | 1-methyl-2-oxo-indolin-5-yl | A | A | A | D1 | F459 |
| **c39** | Cl | H | Cl | H | Cl | H | H | 1-methyl-5-(trifluoromethyl)indazo l-3-yl | A | A | A | D1 | F460 |
| **c40** | Cl | H | Cl | H | Cl | H | H | 4-(triazol-1-yl)phenyl | A | A | B | D1 | F217 |
| **c41** | Cl | H | Cl | H | Cl | H | H | 4-thiazol-2-ylphenyl | A | A | A | D1 | F461 |
| **c42** | H | F | Cl | H | Cl | H | H | 4-[tert-butoxycarbonyl(methyl )amino]-2-methyl-phenyl | A | D | D | D1 | F219 |
| **c43** | Cl | H | Cl | H | Cl | H | Me | 4-[tert-butoxycarbonyl(methyl )amino]-2-methyl-phenyl | A | A | D | D1 | F220 |
| **c44** | Cl | Cl | Cl | H | Cl | H | Me | 4-[tert-butoxycarbonyl(methyl )amino]-2-methyl-pheny | A | D | D | D1 | F221 |
| **c45** | Cl | H | Cl | H | Cl | H | H | 4-(2-thienyl)thiazol-2-yl | A | A | A | D1 | PF135 |
| **c46** | Cl | H | Cl | H | Cl | H | H | 2-(trifluoromethyl)-1H-indol-5-yl | A | A | D | D1 | F465 |
| **c47** | Cl | Cl | Cl | H | Cl | H | H | 2-methyl-4-(methylamino)phenyl | A | A | A | D1 | F223 |
| **c48** | Cl | H | Cl | H | Cl | H | Me | 2-methyl-4-(methylamino)phenyl | A | A | D | D1 | F225 |
| **c49** | Cl | Cl | Cl | H | Cl | H | Me | 2-methyl-4-(methylamino)phenyl | A | A | A | D1 | F226 |
| **c50** | H | F | Cl | H | Cl | H | H | 4-amino-2-methyl-phenyl | A | A | A | D1 | F228 |
| **c51** | Cl | H | Cl | H | Cl | H | H | 2-cyano-4-nitro-phenyl | A | A | A | D1 | F229 |
| **c52** | Cl | H | Cl | H | Cl | H | H | 4-methyl-2-nitrophenyl | A | A | A | D1 | F231 |
| **c53** | Cl | H | Cl | H | Cl | H | H | 2-cyano-4-(trifluoromethyl)phenyl | A | A | A | D1 | F232 |
| **c54** | Cl | H | Cl | H | Cl | H | H | 4-cyano-3-(trifluoromethyl)phenyl | A | A | A | D1 | F235 |
| **c55** | Cl | H | Cl | H | Cl | H | H | 1,3-dioxoisoindolin-5-yl | A | A | D | D1 | F439 |
| **c56** | Cl | H | Cl | H | Cl | H | H | 4-amino-2-cyanophenyl | A | A | A | D1 | F236 |
| **c57** | Cl | H | Cl | H | Cl | H | H | 2-cyano-4-(diacetylamino)phenyl | A | A | A | D1 | F469 |
| **c58** | Cl | H | Cl | H | Cl | H | H | 2-cyano-4-(methylamino)phenyl | A | A | A | D1 | F237 |
| **c59** | Cl | H | Cl | H | Cl | H | H | 4-amino-2-methyl-phenyl | A | A | A | D1 | F195 |
| **c60** | Cl | H | Cl | H | Cl | H | H | 2-oxoindolin-5-yl | A | A | A | D1 | F472 |
| **c61** | Cl | H | Cl | H | Cl | H | H | 1H-pyrrolo[2,3-c]pyridin-5-yl | A | A | A | D1 | F474 |
| **c62** | Cl | H | Cl | H | Cl | H | H | imidazo[1,2-a]pyridin-6-yl | A | A | A | D1 | F475 |
| **c63** | Cl | H | Cl | H | Cl | H | H | 1-methylpyrazolo[3,4-b]pyridin-5-yl | A | A | A | D1 | F476 |
| **c64** | Cl | H | Cl | H | Cl | H | H | 6-methyl-1H-indazol-3-yl | A | A | A | D1 | F478 |
| **c65** | Cl | H | Cl | H | Cl | H | H | 4-(2,5-dioxopyrrol-1-yl)-2-methyl-phenyl | A | A | A | D1 | F480 |
| **c66** | Cl | H | Cl | H | Cl | H | H | 1H-pyrrolo[2,3-b]pyridin-5-yl | A | A | A | D1 | F481 |
| **c67** | Cl | H | Cl | H | Cl | H | H | 2-ethoxycarbonyl-1H-indol-5-yl | A | A | A | D1 | F483 |
| **c68** | Cl | H | Cl | H | Cl | H | H | 1-tert-butoxycarbonylindol-5-yl | A | A | D | D1 | F484 |
| **c69** | Cl | H | Cl | H | Cl | H | H | 4-methyl-1H-indol-5-yl | A | A | A | D1 | F485 |
| **c70** | Cl | H | Cl | H | Cl | H | H | 2-tert-butyl-1H-indol-5-yl | A | A | A | D1 | F486 |
| **c71** | Cl | H | Cl | H | Cl | H | H | 2-methyl-4-(2,2,2-trifluoro-1-hydroxy-1-methoxycarbonylethyl)phenyl | A | A | A | D1 | F257 |
| **c72** | Cl | H | Cl | H | Cl | H | H | 2-methyl-4-[1-(trifluoromethyl)vinyl]p henyl | A | A | B | D1 | F258 |
| **c73** | Cl | H | Cl | H | Cl | H | H | 1-ethylindol-5-yl | A | A | A | D1 | F488 |
| **c74** | Cl | Cl | Cl | H | H | H | H | 4-(tert-butoxycarbonylamino)-2-methyl-phenyl | A | A | B | D1 | F262 |
| **c75** | Cl | H | Cl | H | H | H | H | 4-(tert-butoxycarbonylamino)-2-methyl-phenyl | A | A | B | D1 | F263 |
| **c76** | Cl | Cl | Cl | H | H | H | H | 4-amino-2-methyl-phenyl | A | A | A | D1 | F264 |
| **c77** | Cl | Cl | H | H | H | H | H | 4-amino-2-methyl-phenyl | A | A | A | D1 | F265 |
| **c78** | Cl | H | Cl | H | H | H | H | 4-amino-2-methyl-phenyl | A | A | A | D1 | F266 |
| **c79** | Cl | H | Cl | Cl | Cl | H | H | Phenyl | A | A | A | D1 | F246 |
| **c80** | Cl | H | Cl | Cl | Cl | H | H | 4-(methylamino)phenyl | A | A | A | D1 | F249 |
| **c81** | Cl | H | Cl | H | Cl | H | H | 4-(tert-butoxycarbonylamino)-2-methyl-phenyl | A | A | A | D1 | F275 |
| **c82** | Cl | H | Cl | H | Cl | H | H | 4-amino-2-methyl-phenyl | A | A | A | D1 | F283 |
| **c83** | Cl | H | Cl | H | Cl | H | H | 4-amino-2-methyl-phenyl | A | A | A | D1 | F284 |
| **c84** | Cl | Cl | Cl | H | Cl | H | H | 4-amino-2-methyl-phenyl | A | A | A | D1 | F286 |
| **c85** | Cl | H | Cl | H | Cl | H | H | 2,4-dicyanophenyl | A | A | A | D1 | F287 |
| **c86** | Cl | H | Cl | H | Cl | H | H | 1-methyl-2-oxo-3,4-dihydroquinolin-6-yl | A | A | A | D1 | F490 |
| **c87** | Cl | H | Cl | H | Cl | H | H | 1-methyl-3,4-dihydro-2H-quinolin-6-yl | A | A | A | D1 | F491 |
| **c88** | Cl | H | Cl | H | Cl | H | H | 1,2,3,4-tetrahydroquinolin-6-yl | A | A | A | D1 | F492 |
| **c89** | Cl | F | H | H | Cl | H | H | 4-amino-2-methyl-phenyl | A | A | A | D1 | F291 |
| **c90** | Cl | H | Cl | H | Cl | H | H | 6-acetamido-2-pyridyl | A | A | A | D1 | F494 |
| **c91** | Cl | H | Cl | H | Cl | H | H | 6-amino-5-cyano-2-pyridyl | A | A | D | D1 | F292 |
| **c92** | Cl | H | Cl | H | Cl | H | H | 4-(N-cyano-S-methyl-sulfinimidoyl)phenyl | A | A | A | D1 | F495 |
| **c93** | Cl | H | Cl | H | Cl | H | H | 1-(2-fluoroethyl)indol-5-yl | A | A | A | D1 | F496 |
| **c94** | Cl | H | Cl | H | Cl | H | H | 2-phenyl-1H-indol-5-yl | A | A | A | D1 | F497 |
| **c95** | Cl | H | Cl | H | Cl | H | H | 2-cyano-3-methyl-phenyl | A | A | A | D1 | F304 |
| **c96** | Cl | H | Cl | H | Cl | H | H | 2-methylisoindolin-5-yl | A | A | A | D1 | F498 |
| **c97** | Cl | H | Cl | H | Cl | H | H | 6-(tert-butoxycarbonylamino)-2-pyridyl | A | A | D | D1 | F505 |
| **c98** | Cl | F | H | H | Cl | H | H | 6-(tert-butoxycarbonylamino)-2-pyridyl | A | A | A | D1 | F506 |
| **c99** | Cl | H | Cl | H | Cl | H | H | indolin-5-yl | A | A | A | D1 | F511 |
| **c100** | H | Br | Cl | H | Cl | H | H | 2-methyl-4-(methylamino)phenyl | A | A | A | D1 | F323 |
| **c101** | H | Br | Cl | H | Cl | H | H | 4-amino-2-methyl-phenyl | A | A | A | D1 | F324 |
| **c102** | Cl | H | Br | H | Cl | H | H | 4-(tert-butoxycarbonylamino)-2-methyl-phenyl | A | A | D | D1 | F335 |
| **c103** | Br | H | Cl | H | Cl | H | H | 4-amino-2-methyl-phenyl | A | A | A | D1 | F337 |
| **c104** | Cl | H | Cl | H | Cl | H | H | 2-methyl-3-oxo-isoindolin-5-yl | A | A | A | D1 | F517 |
| **c105** | Cl | H | Cl | H | Cl | H | H | 2-methyl-1-oxo-isoindolin-5-yl | A | A | A | D1 | F518 |
| **c106** | Cl | H | Cl | H | Cl | H | H | 5-nitro-3-pyridyl | A | A | A | D1 | F358 |
| **c107** | Cl | Cl | H | H | Cl | H | H | 5-nitro-3-pyridyl | A | A | A | D1 | F359 |
| **c108** | Cl | H | Cl | H | Cl | H | H | 5-(hydroxyamino)-3-pyridyl | A | A | A | D1 | F525 |
| **c109** | Cl | H | Cl | H | Cl | H | H | 5-amino-3-pyridyl | A | A | A | D1 | F362 |
| **c110** | Cl | Cl | H | H | Cl | H | H | 5-(hydroxyamino)-3-pyridyl | A | A | A | D1 | F526 |
| **c111** | Cl | Cl | H | H | Cl | H | H | 5-amino-3-pyridyl | A | A | A | D1 | F363 |
| **c112** | Cl | H | Cl | H | Cl | H | H | 1-methylindol-4-yl | A | A | A | D1 | F530 |
| **c113** | Cl | H | Cl | H | Cl | H | H | 1-methylindol-7-yl | A | A | A | D1 | F531 |
| **c114** | Cl | Cl | H | H | Cl | H | H | 4-amino-2-methyl-phenyl | A | A | A | D1 | F574 |
| **c115** | Cl | H | Cl | H | Cl | H | H | 4-[(2-methoxyacetyl)-methyl-amino]phenyl | A | A | B | D1 | PF31 |
| **c116** | Cl | H | Cl | H | Cl | H | H | 3-methyl-4-(methylamino)phenyl | A | A | A | D1 | F206 |
| **c117** | H | F | Cl | H | Cl | H | H | 4-acetamidophenyl | A | A | A | D1 | F260 |
| **c118** | Cl | F | H | Cl | M e | H | H | 4-acetamidophenyl | A | A | A | D2 | F1191 |
| **c119** | CF₃ | F | H | H | Cl | H | H | 4-(2-acetoxyethylamino)-2-methyl-phenyl | A | A | C | D2 | F1144 |
| **c120** | CF₃ | F | H | H | Cl | H | H | 4-(2-hydroxyethylamino)-2-methyl-phenyl | A | A | C | D2 | F1145 |
| **c121** | Cl | F | H | CF₃ | Cl | H | H | Phenyl | A | A | A | D2 | F1212 |
| **c122** | Cl | F | H | H | Cl | Cl | H | Phenyl | A | A | A | D2 | F1104 |
| **c123** | H | F | CF₃ | H | Cl | H | H | 4-amino-2,3-dimethylphenyl | A | A | A | D2 | F1389 |
| **c124** | H | F | CF₃ | H | Cl | H | H | 2,3-dimethyl-4-[(2,2,2-trifluoroacetyl)amino]p henyl | A | A | A | D2 | F1392 |
| **c125** | H | F | CF₃ | H | Cl | H | H | 2-methyl-3-[(2,2,2-trifluoroacetyl)amino]p henyl | A | A | A | D2 | F1451 |

List of embodiments according to the present invention:
1. A Compound of Formula (I) 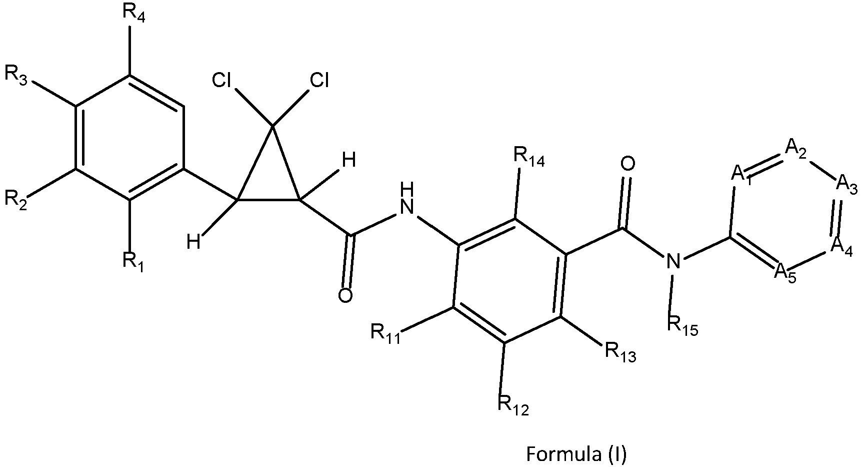
and N-oxides, veterinary acceptable acid addition salts, salt derivatives, solvates, ester derivatives, crystal polymorphs, isotopes, stereoisomers, and tautomers thereof,
for use in a method of treatment or control of a parasitic infestation of an animal, wherein
A₁ is CR₅, N=O, or N;
A₂ is CR₆, N=O, or N;
A₃ is CR₇, N=O, or N;
A₄ is CR₈, N=O or N;
A₅ is CR₉, N=O or N;
wherein no more than 3 of A₁, A₂, A₃, A₄, As are N or N=O;
**R¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R₂** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R³** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂ and S-(Halo)₅;
**R⁵** is selected from the group consisting of H, F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁶** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁷** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁸** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
**R⁹** is selected from the group consisting of F, Cl, Br, I, H, CN, CHO, NHOH, NO, NO₂, OH, NH₂, =O, (C₁-C₆)alkoxy, (C₁-C₆)alkyl, (C₁-C₆)alkylphenyl, (C₁-C₆)alkyl-S(O)₂NH₂, (C₁-C₆)haloalkoxy, (C₁-C₆)haloalkyl, (C₁-C₆)haloalkyl-S(O)₂NH₂, (C₂-C₆)alkenyl, (C₂-C₆)alkynyl, (C₂-C₆)haloalkenyl, (C₃-C₆)cycloalkenyl, (C₃-C₆)cycloalkyl, (C₃-C₆)halocycloalkenyl, (C₃-C₆)halocycloalkyl, (C₁-C₆)alkyl((C₁-C₆)alkyl)(=NO(C₁-C₆)alkyl), C(=NO(C₁-C₆)alkyl)(C₁-C₆)alkyl, C(=O)(C₁-C₆)alkyl, C(=O)NH(C₁-C₆)alkyl, C(=O)NHphenyl, C(=O)O(C₁-C₆)alkyl, CH(=NO(C₁-C₆)alkyl), imidazolyl, N((C₁-C₆)alkyl)(C(=O)(C₁-C₆)alkyl), N((C₁-C₆)alkyl)(C(=O)(C₁-C₆)alkyl-O(C₁-C₆)alkyl), N((C₁-C₆)alkyl)(C(=O)(C₁-C₆)haloalkyl), N((C₁-C₆)alkyl)(C(=O)O(C₁-C₆)alkyl), N((C₁-C₆)alkyl)₂, N(C(=O)O(C₁-C₆)alkyl)₂, N=CH-phenyl, NH((C₁-C₆)alkylC(=O)(C₁-C₆)alkyl), NHC(=O)R¹⁰, N(C₁-C₆)alkyl-C(=O)IR¹⁰, NH(C₁-C₆)alkyl, NH(C₁-C₆)alkenyl, NH(C₁-C₆)alkynyl, NH(C₁-C₆)alkylphenyl, NH(S(O)₂(C₁-C₆)alkyl), NH-C(=O)O(C₁-C₆)alkyl, oxazolyl, phenyl, pyrazolyl, pyridinyl, S(=NCN)((C₁-C₆)alkyl), S(C₁-C₆)alkyl, S(C₁-C₆)haloalkyl, S(O)(=NCN)((C₁-C₆)alkyl), S(O)(C₁-C₆)alkyl, S(O)(C₁-C₆)haloalkyl, S(O)₂(C₁-C₆)alkyl, S(O)₂(C₁-C₆)haloalkyl, SCN, thiazolyl, thienyl, and triazolyl,
   wherein each alkoxy, alkyl, haloalkoxy, haloalkyl, alkenyl, alkynyl, haloalkenyl, cycloalkenyl, cycloalkyl, halocycloalkenyl, halocycloalkyl, imidazolyl, phenyl, pyrazolyl, pyridinyl, thiazolyl, thienyl, and triazolyl, may be optionally substituted with one or more substituents selected from the group consisting of F, Cl, Br, I, CN, OH, NH(C₁-C₆)alkyl, NH(C₃-C₆)cycloalkylCH₂O(C₁-C₆)alkyl, NH(C₃-C₆)cycloalkylCH₂O(C₁-C₆)haloalkyl, NHCH₂(C₃-C₆)cycloalkyl, NH₂, NO₂, oxo, (C₁-C₆)alkyl, (C₁-C₆)alkyl, (C₁-C₆)alkoxy, and C(=O)O-(C₁-C₆)alkyl;
or **R⁶** and **R⁷** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁶** and **R⁷** is optionally substituted with one or more substituents of selected from the group consisting of (C₁-C₃)alkyl, F, Cl, Br, I, CN, NO₂, and =O;
or **R⁷** and **R⁸** together with the atoms to which they are attached form a 5 or 6- atoms containing ring, wherein the ring-forming atoms are selected from the group consisting of -CH₂-, -CH=, =CH-,-N=, =N-, -NH-, -O-, -S(O)-, -S(O)₂- and -S-, wherein the ring formed by **R⁷** and **R⁸** is optionally substituted with one or more substituents of selected from the group consisting of (C₁-C₃)alkyl, F, Cl, Br, I, CN, NO₂, and =O;
**R¹⁰** is selected from the group consisting of H, (C₁-C₆)alkyl, C₃-C₆)cycloalkyl), (C₁-C₆)alkyloxy-(C₁-C₆)-alkyl, (C₂-C₆)alkenyl, (C₂-C₆)alkynyl, (C₁-C₆)haloalkyl, (C₁-C₆)alkyloxy-(C₁-C₆)haloalkyl, C₁-C₆)alkyloxy-(C₂-C₆)-alkenyl, (C₁-C₆)alkyl-(C₃-C₆)cycloalkyl, heterocycle, aryl and (C₁-C₆)alkylphenyl;
**R¹¹** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹²** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹³** is selected from the group consisting of H, F, Cl, Br, I, CN, NH₂, NO₂, CHO, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkylS(O)₂NH₂, (C₁-C₆)haloalkyl-S(O)₂NH₂, and triazolyl;
**R¹⁴** is selected from the group consisting of H, F, Cl, Br, I, CN, NH₂, NO₂, (C₁-C₆)alkyl, (C₃-C₆)cycloalkyl, (C₂-C₆)alkenyl, (C₃-C₆)cycloalkenyl, (C₂-C₆)alkynyl, (C₁-C₆)alkoxy, (C₁-C₆)haloalkyl, (C₃-C₆)halocycloalkyl, (C₂-C₆)haloalkenyl, (C₃-C₆)halocycloalkenyl, (C₁-C₆)haloalkoxy, S(C₁-C₆)alkyl, S(O)(C₁-C₆)alkyl, S(O)₂(C₁-C₆)alkyl, S(C₁-C₆)haloalkyl, S(O)(C₁-C₆)haloalkyl, S(O)₂(C₁-C₆)haloalkyl, (C₁-C₆)alkyl-S(O)₂NH₂, and (C₁-C₆)haloalkyl-S(O)₂NH₂;
**R¹⁵** is selected from the group consisting of H, (C₁-C₆)alkyl, (C₂-C₆)alkenyl, (C₂-C₆)alkynyl, (C₁-C₆)haloalkyl, (C₁-C₆)alkoxy(C₁-C₆)alkyl, C(=O)(C₁-C₆)alkyl, and (C₁-C₆)alkoxyC(=O)(C₁-C₆)alkyl H, (C₁-C₆)alkyl, (C₂-C₆)alkenyl, (C₂-C₆)alkynyl, (C₁-C₆)haloalkyl;
wherein at least one of R⁵, R⁶, R⁷, R⁸, R⁹, is a substituent selected from the group consisting of F, Cl, Br, and I.
2. A compound or use according to any of the preceding embodiments, wherein at least two of R⁵, R⁶, R⁷, R⁸, R⁹, is a substituent selected from the group consisting of F, Cl, Br, and I, more preferably F or Cl.
3. A compound or use according to any of the preceding embodiments, wherein 1 of A₁, A₂, A₃, A₄, A₅ is N, preferably A₁ or A₂ is N.
4. A compound or use according to any of the preceding embodiments, wherein **R¹²** is selected from the group consisting H, F, CL, CH₃, CF₃.
5. A compound or use according to any of the preceding embodiments, wherein **R¹³** is selected from the group consisting of H, F, Cl, CF₃ and CH₃.
6. A compound or use according to any of the preceding embodiments, wherein **R¹⁵** is selected from the group consisting of H and CH₃.
7. A compound or use according to any of the preceding embodiments, wherein at least one of the following selections is made:
**R¹** is selected from the group consisting of H F, and Cl; and/or
**R²** is selected from the group consisting of H, F, and Cl; and/or
**R³** is selected from the group consisting of H, F, and Cl; and/
**R⁴** is selected from the group consisting of H, F, and Cl.
8. A compound or use according to any of the preceding embodiments, wherein at least one of the following selections is made:
**R⁵** is selected from the group consisting of H F, Cl, Br, and I; and/or
**R⁶** is selected from the group consisting of H, F, and Cl, Br, and CH₃; and/or
**R⁷** is selected from the group consisting of H F, Cl, Br, and I; and/or
**R⁸** is selected from the group consisting of H, F, Cl, and Br, and CH₃; and/or
**R⁹** is selected from the group consisting of H F, Cl, Br, and I.
9. A compound or use according to any of the preceding embodiments, wherein:
**R¹** is selected from the group consisting of H F, and Cl;
**R²** is selected from the group consisting of H, F, and Cl;
**R³** is selected from the group consisting of H, F, and Cl;
**R⁴** is selected from the group consisting of H, F, and Cl;
**R⁵** is selected from the group consisting of H F, Cl, Br, and I;
**R⁶** is selected from the group consisting of H, F, and Cl, Br, and CH₃;
**R⁷** is selected from the group consisting of H F, Cl, Br, and I;
**R⁸** is selected from the group consisting of H, F, Cl, and Br, and CH₃;
**R⁹** is selected from the group consisting of H F, Cl, Br, and I;
**R¹¹** is H;
**R¹²** is selected from the group consisting H, F, CL, CH₃, CF₃;
**R¹³** is selected from the group consisting of H, F, Cl, CF₃ and CH₃;
**R¹⁴** is H or F;
**R¹⁵** is selected from the group consisting of H and CH₃; and
wherein at least one of R⁵, R⁶, R⁷, R⁸, R⁹, is a substituent selected from the group consisting of F, Cl, Br, and I.
10. A compound and use according to any of the preceding embodiments, wherein:
**R¹** is H;
**R²** is selected from the group consisting of H, F, and Cl;
**R³** is selected from the group consisting of H, Cl, F, and Br;
**R⁴** is H, F, Cl;
**R⁵** is selected from the group consisting of H F, and Cl;
**R⁶** is selected from the group consisting of H, F, and Cl, and CH₃;
**R⁷** is selected from the group consisting of H F, and Cl;
**R⁸** is selected from the group consisting of H, F, Cl, and CH₃;
**R⁹** is selected from the group consisting of H F, and Cl;
**R¹¹** is H;
**R¹²** is selected from the group consisting H, F and Cl;
**R¹³** is selected from the group consisting of H, F, and Cl;
**R¹⁴** is H;
**R¹⁵** is selected from the group consisting of H and CH₃; and
wherein at least one of R⁵, R⁶, R⁷, R⁸, R⁹, is a substituent selected from the group consisting of F, Cl, Br, and I.
11. Compound for use according to any of the preceding embodiments, wherein the parasitic infestation is an ectoparasite infestation.
12. Compound for use according to any of the preceding embodiments, wherein the parasitic infestation is an acaracide infestation.
13. Compound for use according to any of the preceding embodiments, wherein the parasitic infestation is an infestation selected from tick, flea and/or fly, preferably a tick infestation.
14. Compound for use according to any of the preceding embodiments, wherein the parasite is a one host tick or a multi-host tick, preferably the parasite is *Rhipicephalus microplus.*
15. Compound for use according to any of the preceding embodiments, wherein the animal is a warm-blooded animal, especially a mammal.
16. Compound for use according to any of the embodiments 1-15, wherein the animal is a livestock animal, especially ruminant, especially a bovine animal, especially cattle.
17. Compound for use according to any of the embodiments 1-15, wherein the animal is a fish, and the parasitic infestation is a sea lice infestation.
18. Compound for use according to any of embodiments 1-15, wherein the animal is a companion animal, especially canine animal or a feline animal, especially a dog or cat.
19. Compound for use according to any of the preceding embodiments, wherein the animal is protected from a parasite infestation.
20. Compound for use according to any of the preceding embodiments, wherein an existing parasite infestation of the animal is treated or controlled.
21. Compound for use according to any of the preceding embodiments, wherein the method comprises administering the compound to an animal weekly, bi-weekly, monthly, every 6 weeks, every 2 months , every 3 months, 6 months or 9 months.
22. A Compound of Formula 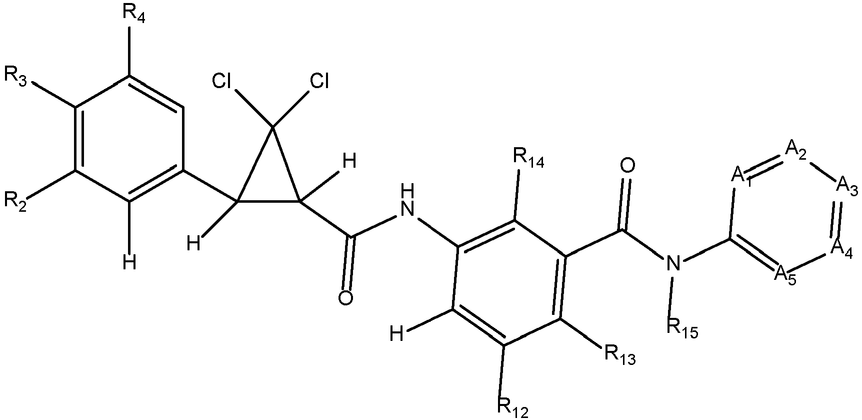 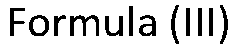 and N-oxides, veterinary acceptable acid addition salts, salt derivatives, solvates, ester derivatives, crystal polymorphs, isotopes, stereoisomers, and tautomers thereof, wherein the compound is selected from the group consisting of

| # | R₂ | R₃ | R₄ | R₁₂ | R₁₃ | R₁₄ | R₁₅ | A₁ | A₂ | A₃ | A₄ | A₅ |
|---|---|---|---|---|---|---|---|---|---|---|---|---|
| 254 | OCHF₂ | H | Cl | H | Cl | H | H | CF | CH | CF | CH | CH |
| 302 | Me | F | CF₃ | H | Cl | H | H | CF | CH | CF | CH | CH |
| 303 | Me | F | CF₃ | H | Cl | H | H | CF | CNH₂ | CF | CH | CH |
| 317 | Cl | H | Cl | H | Cl | H | H | CH | CBr | CF | CF | CH |
| 454 | Cl | H | Cl | H | Cl | H | H | N | CH | CF | CCH₃ | CH |
| 455 | Cl | H | Cl | H | Cl | H | H | CH | N | CF | CH | CF |
| 456 | Cl | H | Cl | H | Cl | H | H | CF | N | CH | CH | CF |
| 457 | Cl | H | Cl | F | Me | H | H | N | CH | CF | CH | CF |
| 458 | Cl | H | Cl | H | Cl | H | H | CF | CH | CH | CH | CF |
| 460 | Cl | H | Cl | H | Cl | H | H | N | CH | CF | CH | N |
| 462 | CI | Cl | H | H | Cl | H | H | N | CH | CF | CH | CF |
| 463 | Cl | H | Cl | H | F | H | H | N | CH | CF | CH | CF |
| 464 | Cl | H | Cl | F | Cl | H | H | N | CH | CF | CH | CF |
| 465 | Cl | H | Cl | H | H | F | H | N | CH | CF | CH | CF |
| 466 | Cl | Cl | Cl | H | Cl | H | H | CH | N | CF | CH | CF |
| 467 | Cl | H | Cl | H | Cl | F | H | N | CH | CF | CH | CF |
| 468 | H | Cl | Cl | H | Me | F | H | N | CH | CF | CH | CF |
| 469 | Cl | F | CI | H | Cl | H | H | N | CH | CF | CH | CF |
| 471 | CF₃ | H | CF₃ | H | Cl | H | H | N | CH | CF | CH | CF |
| 472 | CF₃ | H | CF₃ | H | Cl | H | H | CF | CH | CF | CH | CH |
| 473 | CF₃ | H | CF₃ | H | F | H | H | CF | CH | CF | CH | CH |
| 474 | CF₃ | H | CF₃ | F | F | H | H | N | CH | CF | CH | CF |
| 475 | Cl | H | CF₃ | H | Cl | H | H | N | CH | CF | CH | CF |
| 476 | Cl | H | CF₃ | H | Cl | H | H | N | CH | CF | CH | CF |
| 477 | Cl | H | Cl | F | F | H | H | N | CH | CF | CH | CF |
| 478 | F | F | F | H | Cl | H | H | N | CH | CF | CH | CF |
| 479 | F | F | F | H | F | H | H | N | CH | CF | CH | CF |
| 480 | Cl | H | Cl | H | F | F | H | N | CH | CF | CH | CF |

23. A veterinary composition comprising an effective amount of a compound of Formula (I) as defined in any of embodiments 1-10 or 22 and an inert carrier or formulation adjuvant.
24. The veterinary composition according to embodiment 23, wherein the veterinary composition is a topical composition, an oral composition, or an injection composition.
25. The veterinary composition according to embodiment 23 or 24 and a pharmaceutically acceptable oily carrier.
26. The veterinary composition according to embodiment 25 wherein the oily carrier comprises a fatty acid ester, preferably essentially consist of a fatty acid ester.
27. The veterinary pharmaceutical composition according to embodiment 26, wherein the fatty acid ester is a glycol diester or a glycerol triester of a C₈-₁₀ fatty acid, preferably propylene glycol octanoate decanoate (e.g. Miglyol 840).
28. The veterinary pharmaceutical composition according to embodiment 25, wherein the oily carrier comprises a combination of glycerol monocaprylate and propylene glycol dicaprylocaprate.
29. The veterinary pharmaceutical composition according to any one of embodiments 23 to 28, wherein the composition does not comprise a penetration enhancer.
30. The veterinary pharmaceutical composition according to any one of embodiments 23 to 29, comprising:
a) from about 0.5% to about 10% w/v, preferably 1% of the compound of Formula (I);
b1) from about 90% to 99.9% w/v of preferably propylene glycol octanoate decanoate; or alternatively
b2) from about 10% to about 40% w/v, preferably 20% of glycerol monocaprylate; and c) optionally, from about 2% to about 50% w/v, preferably 99% of propylene glycol dicaprylocaprate.
31. A compound of Formula (III) 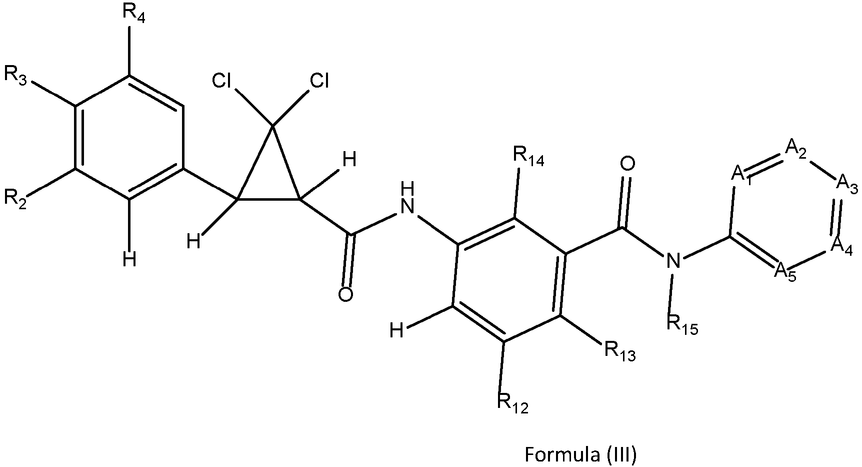 and N-oxides, veterinary acceptable acid addition salts, salt derivatives, solvates, ester derivatives, crystal polymorphs, isotopes, stereoisomers, and tautomers thereof, wherein the compound is selected from the group consisting of

| **#** | **R₂** | **R₃** | **R₄** | **R₁₂** | **R₁₃** | **R₁₄** | **R₁₅** | **A₁** | **A₂** | **A₃** | **A₄** | **A₅** |
|---|---|---|---|---|---|---|---|---|---|---|---|---|
| 254 | OCHF₂ | H | Cl | H | Cl | H | H | CF | CH | CF | CH | CH |
| 302 | Me | F | CF₃ | H | Cl | H | H | CF | CH | CF | CH | CH |
| 303 | Me | F | CF₃ | H | Cl | H | H | CF | CNH₂ | CF | CH | CH |
| 317 | Cl | H | Cl | H | Cl | H | H | CH | CBr | CF | CF | CH |
| 454 | Cl | H | Cl | H | Cl | H | H | N | CH | CF | CCH₃ | CH |

## Claims

1. A compound of Formula (III) 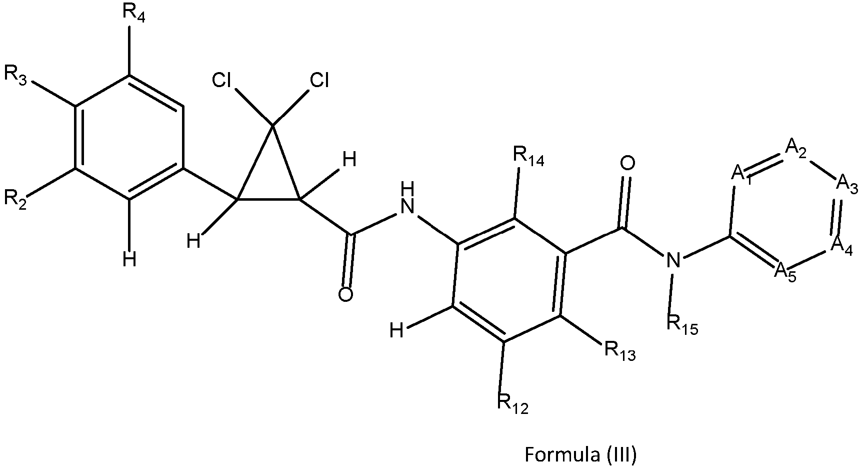 and N-oxides, veterinary acceptable acid addition salts, salt derivatives, solvates, ester derivatives, crystal polymorphs, isotopes, stereoisomers, and tautomers thereof, wherein the compound is selected from the group consisting of
| # | R₂ | R₃ | R₄ | R₁₂ | R₁₃ | R₁₄ | R₁₅ | A₁ | A₂ | A₃ | A₄ | A₅ |
|---|---|---|---|---|---|---|---|---|---|---|---|---|
| 254 | OCHF₂ | H | Cl | H | Cl | H | H | CF | CH | CF | CH | CH |
| 302 | Me | F | CF₃ | H | Cl | H | H | CF | CH | CF | CH | CH |
| 303 | Me | F | CF₃ | H | Cl | H | H | CF | CNH₂ | CF | CH | CH |
| 317 | Cl | H | Cl | H | Cl | H | H | CH | CBr | CF | CF | CH |
| 454 | Cl | H | Cl | H | Cl | H | H | N | CH | CF | CCH₃ | CH |
